(19) Europäisches Patentamt
European Patent Office
Office européen des brevets

(11) EP 4 431 490 A1

(12) **EUROPEAN PATENT APPLICATION**
published in accordance with Art. 153(4) EPC

(43) Date of publication:
**18.09.2024 Bulletin 2024/38**

(21) Application number: **22890077.5**

(22) Date of filing: **08.11.2022**

(51) International Patent Classification (IPC):
***C07C 263/04*** (2006.01) ***C07C 209/86*** (2006.01)
***C07C 211/49*** (2006.01) ***C07C 263/18*** (2006.01)
***C07C 265/14*** (2006.01) ***C07C 269/04*** (2006.01)
***C07C 271/28*** (2006.01) ***C07C 271/44*** (2006.01)
***C07C 271/56*** (2006.01) ***C07B 61/00*** (2006.01)

(52) Cooperative Patent Classification (CPC):
(C-Sets available)
**C07C 269/04; C07C 269/06; C07C 269/08;**
**C07C 273/189;** C07B 61/00; C07C 2601/14;
Y02P 20/10 (Cont.)

(86) International application number:
**PCT/JP2022/041611**

(87) International publication number:
**WO 2023/080257 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.11.2021 JP 2021182191**
**08.11.2021 JP 2021182194**
**08.11.2021 JP 2021182195**
**08.12.2021 JP 2021199645**

(71) Applicant: **Asahi Kasei Kabushiki Kaisha**
**Tokyo 1000006 (JP)**

(72) Inventors:
- **TAKAGAKI Kazuhiro**
  **Tokyo 100-0006 (JP)**
- **IWATA Yusuke**
  **Tokyo 100-0006 (JP)**
- **NAKAOKA Koichi**
  **Tokyo 100-0006 (JP)**
- **SAKURAI Yusuke**
  **Tokyo 100-0006 (JP)**
- **NAGAMOTO Midori**
  **Tokyo 100-0006 (JP)**

(74) Representative: **D Young & Co LLP**
**3 Noble Street**
**London EC2V 7BQ (GB)**

# (54) METHOD FOR PRODUCING ISOCYANATE COMPOUNDS, METHOD FOR PRODUCING CARBAMATE COMPOUNDS, METHOD FOR RECOVERING AMINE COMPOUNDS, AND ISOCYANATE COMPOSITION

(57) Provided is a method for producing an isocyanate compound, including a step (1) of reacting a primary amine compound with a carbonic acid derivative to obtain an N-substituted carbamate compound while extracting a by-product compound having a boiling point lower than a boiling point of the N-substituted carbamate compound, a step (2) of performing thermal decomposition of the N-substituted carbamate compound on a reaction solution obtained in the step (1) in the presence of an aprotic solvent to obtain an isocyanate compound while extracting a by-product hydroxy compound, a step (3) of separating the isocyanate compound and the aprotic solvent from a reaction solution obtained in the step (2), and a step (4) of removing a component having a boiling point higher than a boiling point of the isocyanate compound from a fraction obtained in the step (3) to purify the isocyanate compound.

EP 4 431 490 A1

FIG. 1

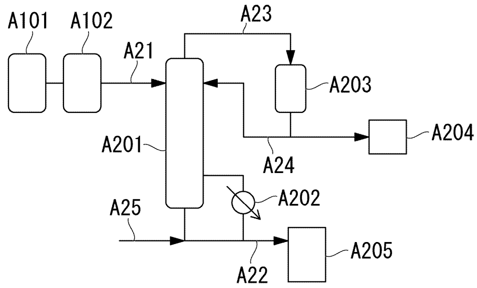


(52) Cooperative Patent Classification (CPC): (Cont.)

C-Sets
**C07C 269/04, C07C 271/20;**
**C07C 269/04, C07C 271/52;**
**C07C 269/06, C07C 271/66;**
**C07C 269/08, C07C 271/28;**
**C07C 269/08, C07C 271/52;**
**C07C 273/189, C07C 275/14;**
**C07C 273/189, C07C 275/18;**
**C07C 273/189, C07C 275/26;**
**C07C 273/189, C07C 275/40;**
**C07C 273/189, C07C 275/62**

## Description

[Technical Field]

The present invention relates to a method for producing an isocyanate compound, a method for producing a carbamate compound, a method for recovering an amine compound, and an isocyanate composition.

Priority is claimed on Japanese Patent Application Nos. 2021-182191, 2021-182194, and 2021-182195, filed November 8, 2021, and Japanese Patent Application No. 2021-199645 filed December 8, 2021, the contents of which are incorporated herein by reference.

[Background Art]

Isocyanate has been widely used as a raw material for manufacturing polyurethane foam, polyurea, paint, adhesives, and the like. A main industrial method for producing the isocyanate is a reaction between an amine compound and phosgene (phosgene method), and almost all of the production amount of isocyanate in the world is produced by the phosgene method. However, the phosgene method has many problems.

First, a large amount of phosgene is used as a raw material. The phosgene is extremely highly toxic, and thus it requires special attention in handling in order to prevent exposure to workers, and also requires a special device for detoxifying waste. Second, in the phosgene method, since a large amount of highly corrosive hydrogen chloride is produced as a by-product, a process for removing the hydrogen chloride is required, and the produced isocyanate is often hydrolyzable chlorine. Therefore, in a case where the isocyanate produced by the phosgene method is used, weather resistance and heat resistance of the polyurethane product may be adversely affected.

In view of such a background, a method for producing an isocyanate compound without using the phosgene is desired. As one of methods for producing the isocyanate compound without using the phosgene, a method (urea method) in which an amine, at least any of urea and N-unsubstituted carbamic acid ester, and an alcohol are reacted (carbamylation reaction) to obtain a carbamate compound, and the carbamate compound is thermally decomposed has been proposed (for example, refer to Patent Document 1 and the like). It has been known for a long time that isocyanate and a hydroxy compound are obtained by the thermal decomposition of the carbamate compound (for example, refer to Non Patent Document 1). The basic reaction is mentioned by Formula (A).

$$R(NHCOOR')a \rightarrow R(NCO)a + a\ R'OH \qquad (A)$$

(in Formula (A), R is an a-valent organic group, R' is a monovalent organic group, and a is an integer of 1 or more)

On the other hand, in the thermal decomposition reaction of the carbamate compound, various irreversible side reactions such as an undesirable thermal depolymerization reaction of the carbamate compound and a condensation reaction of isocyanate generated by the thermal decomposition are likely to occur. As the side reaction, for example, a reaction for forming an isocyanurate group represented by Formula (B), a reaction for generating carbodiimides represented by Formula (C), a reaction for forming a uretonimine group represented by Formula (D), and a reaction for forming an allophanate group represented by Formula (E) are exemplary examples (for example, refer to Non Patent Documents 1 to 4 and the like).

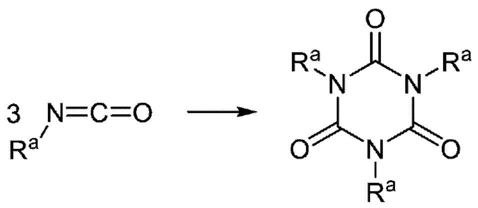
(B)

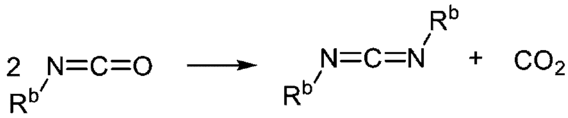
(C)

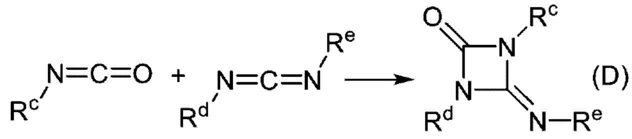
(D)

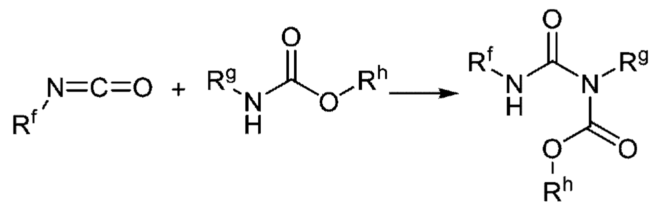
(E)

(in Formulae (B) to (E), $R^a$ to $R^h$ are each independently a monovalent organic group)

These side reactions not only cause a decrease in yield and selectivity of the desired isocyanate, but also a problem that, in the production of the isocyanate, a polymer-like solid material is precipitated, the reactor is blocked, and the like which may make it difficult to operate for a long period of time.

Various methods have been proposed as a method for producing an isocyanate using a carbamate compound as a raw material. For example, Patent Documents 2 and 3 disclose a technique for continuously producing a polyisocyanate by thermally decomposing a carbamate compound in the presence of a high-boiling-point solvent, without a catalyst.

In addition, Patent Document 4 discloses that monocarbamate can be decomposed at a relatively low temperature with a favorable yield without using a solvent in the presence or absence of at least any of a catalyst and a stabilizer under reduced pressure. The decomposition products (monoisocyanate and alcohol) are removed by distillation from the boiling reaction mixture, and separately collected by fractional condensation. A method for partially removing the reaction mixture is disclosed in a general form in order to remove the by-product formed by the thermal decomposition.

In addition, in the carbamylation reaction, it is necessary to react a large excess of hydroxy compounds at a high temperature in order to obtain a sufficient carbamate yield.

A method of using a catalyst is an exemplary example of a method of increasing the reaction rate. For example, Patent Document 1 discloses, as the catalyst, basic catalysts such as methylates, ethylates, and butylates (each isomer) of lithium, sodium, potassium, calcium, and barium; simple substances of rare earth elements, antimony, bismuth, or oxides, sulfides, and salts of these elements; simple substances of boron or boron compounds; metals of the copper group, zinc group, aluminum group, carbon group, and titanium group in the periodic table, or oxides and sulfides of these metals; and carbides and nitrides of the carbon group excluding carbon, titanium group, vanadium group, and chromium group elements in the periodic table.

In addition, Patent Document 5 discloses a method for producing carbamate using zinc toluenesulfonate as a catalyst.

In addition, as a catalyst used for the production of carbamate, Patent Document 6 discloses two kinds of metal compounds, Patent Document 7 discloses a metal compound containing a non-coordinating anion, and Patent Document 8 discloses a compound containing one or more cations of a metal from Group IIB, Group VB, Group VIIB, and Group VIII in the periodic table.

In addition, Patent Documents 9 to 11 disclose a method for producing carbamate, in which various metal compounds may be used as a catalyst.

In addition, it is known that, in the above-described phosgene method, the isocyanate is produced by a reaction between an amine and phosgene, but an unsatisfactory polymerization reaction such as reaction of isocyanates occurs, and as a result, a high-boiling-point compound having a boiling point higher than that of isocyanate such as a polymer is produced. However, it is also known that the high-boiling-point compound having a boiling point higher than that of

isocyanate is generated after the isocyanate is recovered in the production of isocyanate by an urea method. The composition containing the high-boiling-point compound may become a highly viscous liquid or solid near room temperature, and may cause clogging or the like in the continuous production of the isocyanate compound. In addition, the high-boiling-point compound is a by-product derived from the isocyanate compound and an organic amine compound which is a raw material of the isocyanate compound, and it is industrially advantageous in a case where the high-boiling point compound can be recovered as an active ingredient.

For example, Patent Document 12 discloses a method for separating isocyanate from a diisocyanate-containing organic residue under specific temperature and pressure conditions and forcibly transporting the residue.

Patent Documents 13 to 17 disclose a post-treatment method for residues generated in the production of isocyanate.

Patent Document 18 discloses a method for absorbing a by-product gas component as a total amount of carbonate with an alkali metal in a post-treatment method for residues generated in the production of isocyanate.

Patent Document 19 discloses a method of performing post-treatment by reacting a distillation residue generated in the synthesis of triaryl isocyanate with water, in which the distillation residue is reacted with the water in the presence of a hydrolyzate in a reverse-phase reaction vessel in a continuous or semi-continuous manner.

Patent Document 20 discloses a decomposition recovery method in which an isocyanate-based compound is brought into contact with high-temperature and high-pressure water containing one or more compounds selected from the group consisting of ammonia and an aliphatic amine to recover the isocyanate-based compound as a raw material.

Patent Document 21 discloses a method of decomposing residues generated in the production of isocyanate in the presence of high-temperature and high-pressure water and an alkali metal or alkaline earth metal hydroxide.

[Citation List]

[Patent Documents]

[Patent Document 1]
PCT International Publication No. WO2014/157636
[Patent Document 2]
Japanese Unexamined Patent Application, First Publication No. S52-71443
[Patent Document 3]
Japanese Unexamined Patent Application, First Publication No. H5-255227
[Patent Document 4]
United States Patent No. 4386033
[Patent Document 5]
PCT International Publication No. WO2011/089932
[Patent Document 6]
PCT International Publication No. WO2010/116871
[Patent Document 7]
PCT International Publication No. WO2010/110142
[Patent Document 8]
Japanese Unexamined Patent Application, First Publication No. S64-038055
[Patent Document 9]
Japanese Unexamined Patent Application, First Publication No. H8-217744
[Patent Document 10]
Japanese Unexamined Patent Application, First Publication No. H6-025136
[Patent Document 11]
Japanese Unexamined Patent Application, First Publication No. S59-205352
[Patent Document 12]
PCT International Publication No. WO2007/036479
[Patent Document 13]
PCT International Publication No. WO2009/127591
[Patent Document 14]
PCT International Publication No. WO2019/131855
[Patent Document 15]
Japanese Patent No. 5563886
[Patent Document 16]

Japanese Patent No. 5240678
[Patent Document 17]
PCT International Publication No. WO2009/130842
[Patent Document 18]
Japanese Examined Patent Application, Second Publication No. S58-048538
[Patent Document 19]
Published Japanese Translation No. 2002-518369 of the PCT International Publication
[Patent Document 20]
Japanese Unexamined Patent Application, First Publication No. 2002-173471
[Patent Document 21]
Japanese Examined Patent Application, Second Publication No. S58-008380

[Non Patent Documents]

[Non Patent Document 1]
Drifter Jahrgang, "186. A. W. Hofmann: Ueber die aromatischen Cyanate.", Berichte der Deutschen Chemischen Gesellschaft, Vol. 3, pp. 653-658, 1870.
[Non Patent Document 2]
Dyer E et al., "Thermal Degradation of Alkyl N-Phenylcarbomates.", Journal of American Chemical Society, Vol. 81, pp. 2138-2143, 1959.
[Non Patent Document 3]
Ulrich H et al., "[2+2] Cycloaddition Reactions of Unsymmetrically substituted Carbodiimides.", Journal of Heterocyclic Chemistry, Vol. 24, pp. 1121-1123, 1987.
[Non Patent Document 4]
Schwetlick K et al., "Kinetics and Catalysis of Consecutive Isocyanate Reactions. Formation of Carbamates, Allophanates and Isocyanurates.", Journal of the Chemical Society, Perkin transactions II, Vol. 2, pp. 395-402, 1995.

[Summary of Invention]

[Technical Problem]

However, in the method disclosed in Patent Document 2, since the isocyanate is evaporated and recovered, in a case of producing a high-boiling point isocyanate, severe temperature and pressure conditions are required. In addition, under such severe conditions, there is also a problem that the amount of high-boiling-point by-products incorporated increases. Furthermore, in the method for producing the isocyanate compound in the related art, which does not use phosgene, the isocyanate compound is produced from the amine compound through a ureido compound and a phenylcarbamate compound, but there is a problem that a reaction equilibrium constant K of each reaction step is biased to the raw material side to approximately $10^{-3}$ or more and $10^{-1}$ or less, and the yield of the isocyanate compound finally obtained is low.
In the method disclosed in Patent Document 1, high-temperature conditions are required to obtain the carbamate in a high yield. In addition, a large excess of hydroxy compounds is used to suppress the modification of carbamate. Therefore, a large device is required, a large amount of energy is consumed, productivity is poor, and production cost is high.
As a method for improving the productivity of carbamate, a method of using a metal or a metal compound as a catalyst is disclosed in many patent documents including Patent Document 1. In a case where the carbamate is used as a raw material for isocyanate, it is desirable to separate the metal or the compound thereof from the isocyanate because the metal or the compound thereof modifies the isocyanate. However, the metal or the compound thereof is very difficult to volatilize, and there are cases where a process design is restricted.
In the method of Patent Document 12, in a case where the diisocyanate-containing organic residues are transported to the device for separating the diisocyanate, since the diisocyanate-containing organic residues tend to have a relatively high viscosity, the transportation itself is often difficult, which makes it difficult to say that the method is useful.
In the method disclosed in Patent Documents 13 to 18, a sufficient isocyanate compound cannot be obtained, and it is necessary to further thermally decompose and isocyanate the carbamate after the treatment step, and even in this additional thermally decomposed step in terms of the reaction principle, there is a problem that by-products are produced. In addition, the method of adding a carbonic acid ester has an essential problem of generating carbamates that promote side reactions by reacting the organic amine compound generated by the treatment of the isocyanate

compound with the carbonic acid ester. Furthermore, in a case where the component containing the high-boiling-point compound after recovering the isocyanate is a solid, it is industrially difficult to treat the component in a continuous process. Therefore, it is necessary to rely on a batch process, and it is difficult to say that the method is useful in terms of poor economic efficiency.

The method disclosed in Patent Document 19 has a problem that the reaction efficiency is low and a long time is required for the reaction to be completed.

The method disclosed in Patent Document 20 has a problem that the reaction efficiency depends on an interfacial contact efficiency between the water phase and the organic phase, and liquid-liquid phase separation occurs in a portion where there is no stirring power, and the reaction efficiency is low.

The method disclosed in Patent Document 21 has a problem that the carbonate of the alkali metal or the alkaline earth metal, added to the reaction solution, is precipitated as a solid content and is fixed in the device during the separation operation such as distillation.

As described above, various studies have been made on the isocyanate production method by thermal decomposition of a carbamate compound. However, there are problems that it is difficult to continuously produce the isocyanate compound for a long period of time due to the generation of by-products having a boiling point higher than that of the target isocyanate compound or due to the adhesion of the by-products to the reactor. Therefore, it is a fact that the methods are hardly implemented industrially.

The present invention has been made in consideration of the above circumstances, and provides a method for producing a novel isocyanate compound, in which the yield of the isocyanate compound is favorable, stable continuous operation can be performed, and an amount of by-products incorporated is suppressed.

In addition, the present invention has been made in consideration of the above circumstances, and provides a method for producing a carbamate compound, which does not use a metal catalyst, is inexpensive, and has a favorable yield; and a method for producing an isocyanate compound using the carbamate compound obtained by the production method.

In addition, the present invention has been made in consideration of the above circumstances, and provides a method for recovering an amine compound, which can efficiently regenerate a useful component including an amine compound from a liquid-phase component remaining after production of an isocyanate compound.

In addition, the present invention has been made in consideration of the above circumstances, and provides an isocyanate composition which can prevent fixation of by-products to a device and improve the yield of an isocyanate compound in a case of producing an isocyanate compound without using phosgene; and a method for producing an isocyanate compound using the isocyanate composition.

[Solution to Problem]

That is, the present invention includes the following aspects.

<1> A method for producing an isocyanate compound, including:

a step (1) of reacting a primary amine compound with a carbonic acid derivative to obtain an N-substituted carbamate compound while extracting a by-product compound having a boiling point lower than a boiling point of the N-substituted carbamate compound;
a step (2) of performing thermal decomposition of the N-substituted carbamate compound on a reaction solution containing the N-substituted carbamate compound, which is obtained in the step (1), in the presence of an aprotic solvent to obtain an isocyanate compound while extracting a by-product hydroxy compound;
a step (3) of separating the isocyanate compound and the aprotic solvent from a reaction solution containing the isocyanate compound, which is obtained in the step (2); and
a step (4) of removing a component having a boiling point higher than a boiling point of the isocyanate compound from a fraction containing the isocyanate compound obtained in the step (3) to purify the isocyanate compound.

<2> The method for producing an isocyanate compound according to <1>, further including:
a step (5) of hydrolyzing a fraction containing the aprotic solvent separated in the step (3) or a component having a boiling point higher than the boiling point of the isocyanate compound removed in the step (4) in the presence of an alkali and water to obtain the primary amine compound and the hydroxy compound.
<3> The method for producing an isocyanate compound according to <1> or <2>,

in which, in the step (1), the primary amine compound is reacted with urea or a urea derivative as the carbonic acid derivative in the presence of a hydroxy compound to obtain the N-substituted carbamate compound while extracting the by-product compound having a boiling point lower than the boiling point of the N-substituted

carbamate compound, or
the primary amine compound is reacted with a carbonic acid ester as the carbonic acid derivative to obtain the N-substituted carbamate compound while extracting a hydroxy compound as the by-product compound having a boiling point lower than the boiling point of the N-substituted carbamate compound.

<4> The method for producing an isocyanate compound according to any one of <1> to <3>,
in which, in the step (2), a liquid phase containing the isocyanate compound is obtained while extracting the by-product hydroxy compound into a gas phase.
<5> The method for producing an isocyanate compound according to any one of <1> to <4>,
in which the step (1) and the step (2) are performed using one or more reactors selected from the group consisting of a tank-type reactor, a distillation column, a tubular evaporator, a thin film evaporator, and a falling film-type evaporator.
<6> The method for producing an isocyanate compound according to any one of <1> to <5>,
in which at least one of the step (1) or the step (2) is performed by a reactive distillation method.
<7> The method for producing an isocyanate compound according to any one of <1> to <6>,
in which the hydroxy compound extracted in the step (2) is re-used by circulating the hydroxy compound to the step (1).
<8> The method for producing an isocyanate compound according to any one of <1> to <7>,
in which the aprotic solvent separated in the step (3) is re-used by circulating the aprotic solvent to the step (2).
<9> The method for producing an isocyanate compound according to any one of <1> to <8>,
in which the aprotic solvent is a carbonic acid ester.
<10> The method for producing an isocyanate compound according to any one of <1> to <9>,

in which, in the step (4), the isocyanate compound is purified in the presence of a carbonyl compound represented by General Formula (I) in an amount of 1 ppm by mass or more and 50% by mass or less with respect to a mass of the fraction containing the isocyanate compound.

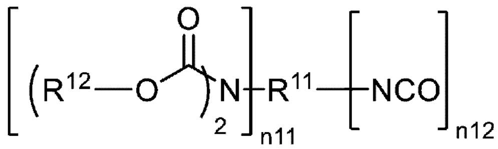 (I)

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less)

<11> The method for producing an isocyanate compound according to any one of <1> to <10>,

in which the primary amine compound is a compound represented by General Formula (II).

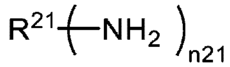 (II)

(in General Formula (II), $R^{21}$ is an n21-valent organic group, and n21 is an integer of 2 or more)

<12> The method for producing an isocyanate compound according to <11>, in which n21 is 3 or more.
<13> A method for producing a carbamate compound, including:
a reaction step of reacting a primary amine compound, a carbonic acid derivative, and a hydroxy compound in the presence of, as a catalyst, an amine compound having no active hydrogen to obtain a carbamate compound.
<14> The method for producing a carbamate compound according to <13>,
in which the amine compound having no active hydrogen has one or more functional groups selected from the group consisting of a tertiary amino group, a nitrogen-containing aromatic group, an amidine group, and a guanidine group.
<15> The method for producing a carbamate compound according to <14>,
in which the amine compound having no active hydrogen is a compound having 1 or more tertiary amino groups and having 3 or more and 85 or less carbon atoms.
<16> The method for producing a carbamate compound according to <15>,
in which the amine compound having no active hydrogen is a compound having 1 or more and 6 or less tertiary

amino groups and having 3 or more and 30 or less carbon atoms.

<17> The method for producing a carbamate compound according to <16>,

in which the amine compound having no active hydrogen is N,N'-dimethylaniline, N,N'-diethylaniline, N-methyl-N'-ethylaniline, N,N'-dimethylaminopyridine, N,N,N',N'-tetramethylphenylenediamine, methylenebis(N,N'-dimethylaniline), triethylamine, ethyldiisopropylamine, N-methylmorpholine, N-methylpiperidine, quinuclidine, N,N'-dimethylpiperazine, triethylenediamine, N,N,N',N'-tetramethylethylenediamine, N,N,N',N'-tetramethylhexanediamine, N,N,N',N'-tetramethylxylylenediamine, pentamethyldiethylenetriamine, bis(2-morpholinoethyl)ether, hexahydro-1,3,5-tris(3-dimethylaminopropyl)-1,3,5-triazine, or hexamethylenetetramine.

<18> The method for producing a carbamate compound according to <14>,

in which the amine compound having no active hydrogen is a compound having 1 or more nitrogen-containing aromatic groups and having 3 or more and 85 or less carbon atoms.

<19> The method for producing a carbamate compound according to <18>,

in which the nitrogen-containing aromatic group is a substituted or unsubstituted pyridyl group, imidazolyl group, pyrazolyl group, quinolyl group, isoquinolyl group, oxazolyl group, thiazolyl group, pyridazyl group, pyrimidyl group, or pyrazyl group.

<20> The method for producing a carbamate compound according to <19>,

in which the amine compound having no active hydrogen is pyridine, picoline, lutidine, collidine, 1-methylimidazole, 1-methylpyrazole, quinoline, isoquinoline, methylquinoline, oxazole, thiazole, pyridazine, pyrimidine, or pyrazine.

<21> The method for producing a carbamate compound according to <14>,

in which the amine compound having no active hydrogen is an amidine group-containing compound represented by General Formula (VIII-1).

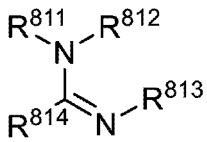

( VIII-1 )

(in General Formula (V1I1-1), $R^{811}$, $R^{812}$, $R^{813}$, and $R^{814}$ are each independently a monovalent organic group, $R^{811}$ and $R^{812}$, $R^{812}$ and $R^{813}$, $R^{813}$ and $R^{814}$, or $R^{814}$ and $R^{811}$ may be each independently bonded to each other to form a ring structure, and a total number of carbon atoms in $R^{811}$, $R^{812}$, $R^{813}$, and $R^{814}$ is 5 or more and 85 or less)

<22> The method for producing a carbamate compound according to <21>,

in which the amidine group-containing compound has one or more ring structures, and
the total number of carbon atoms in $R^{811}$, $R^{812}$, $R^{813}$, and $R^{814}$ is 5 or more and 30 or less.

<23> The method for producing a carbamate compound according to <22>,

in which the amidine group-containing compound is 1,2-dimethyl-1,4,5,6-tetrahydropyrimidine, 1,8-diazabicyclo-[5.4.0]undeca-7-ene, or 1,5-diazabicyclo-[4.3.0]nona-5-ene.

<24> The method for producing a carbamate compound according to <14>,

in which the amine compound having no active hydrogen is a guanidine group-containing compound represented by General Formula (VIII-2).

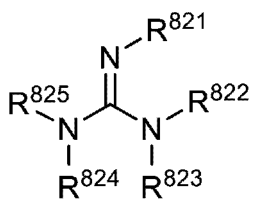

( VIII-2 )

(in General Formula (VIII-2), $R^{821}$, $R^{822}$, $R^{823}$, $R^{824}$, and $R^{825}$ are each independently a monovalent organic group, $R^{821}$ and $R^{822}$, $R^{822}$ and $R^{823}$, $R^{823}$ and $R^{824}$, $R^{824}$ and $R^{825}$, or $R^{825}$ and $R^{821}$ may be each independently bonded to each other to form a ring structure, and a total number of carbon atoms in $R^{821}$, $R^{822}$, $R^{823}$, $R^{824}$,

and $R^{825}$ is 5 or more and 85 or less)

<25> The method for producing a carbamate compound according to <24>,
in which the total number of carbon atoms in $R^{821}$, $R^{822}$, $R^{823}$, $R^{824}$, and $R^{825}$ is 5 or more and 30 or less.
<26> The method for producing a carbamate compound according to <25>,
in which the guanidine group-containing compound is pentamethylguanidine or 7-methyl-1,5,7-triazabicyclo[4.4.0] deca-5-ene.
<27> The method for producing a carbamate compound according to any one of <13> to <26>,
in which the primary amine compound is an aliphatic or aromatic primary polyamine compound.
<28> The method for producing a carbamate compound according to any one of <13> to <27>,
in which the hydroxy compound is an aromatic hydroxy compound.
<29> A method for producing an isocyanate compound, including:
a thermal decomposition step of performing thermal decomposition on a carbamate compound obtained by the method for producing a carbamate compound according to any one of <13> to <28>.
<30> A method for recovering an amine compound represented by General Formula (IIa) from a liquid-phase component containing a compound having a higher boiling point than a boiling point of an isocyanate compound which is a by-product in a method for producing an isocyanate compound represented by General Formula (VIIa), the recovery method including:

a step (a) of reacting the liquid-phase component, an aromatic hydroxy compound, an active hydrogen-containing compound, and a catalyst in a reactor to obtain a reaction solution containing the amine compound represented by General Formula (IIa).

$$R^{71a}\left(-NCO\right)_{n71a} \qquad (VIIa)$$

(in General Formula (VIIa), $R^{71a}$ is an n71 a-valent organic group, and n71a is an integer of 2 or more and 8 or less)

$$R^{21a}\left(-NH_2\right)_{n21a} \qquad (IIa)$$

(in General Formula (IIa), $R^{21a}$ is an n21a-valent organic group, and a relational expression of $R^{21a} = R^{71a}$ is satisfied, and n21a is an integer of 2 or more and 8 or less, and a relational expression of n21a = n71a is satisfied)

<31> The recovery method according to <30>,

in which the step (a) includes the following step (a1) and step (a2),
a step (a1) of mixing the liquid-phase component with the aromatic hydroxy compound, and
a step (a2) of reacting the mixture obtained in the step (a1), the active hydrogen-containing compound, and the catalyst in the reactor to obtain the reaction solution containing the amine compound represented by General Formula (IIa).

<32> The recovery method according to <31>,

in which the step (a2) includes the following step (a2-1) and step (a2-2),
a step (a2-1) of mixing the mixture obtained in the step (a1), an amine compound as the active hydrogen-containing compound, and the catalyst, and
a step (a2-2) of reacting the mixture obtained in the step (a2-1) with water as the active hydrogen-containing compound in the reactor to obtain the reaction solution containing the amine compound represented by General Formula (IIa).

<33> The recovery method according to any one of <30> to <32>,
in which the active hydrogen-containing compound is water.
<34> The recovery method according to any one of <30> to <32>,
in which the active hydrogen-containing compound is water and a primary amine compound.
<35> The recovery method according to <34>,

in which the primary amine compound is the amine compound represented by General Formula (IIa).

<36> The recovery method according to any one of <30> to <35>,

in which, in the method for producing the isocyanate compound represented by General Formula (VIIa), a carbonic acid derivative, a hydroxy compound, and the amine compound represented by General Formula (IIa) are used as raw materials.

<37> The recovery method according to any one of <30> to <36>,

in which the liquid-phase component is a liquid-phase component which is obtained by subjecting a reaction solution containing a carbamate compound produced from a carbonic acid derivative, a hydroxy compound, and the amine compound represented by General Formula (IIa) to a thermal decomposition reaction, supplying a composition containing the produced isocyanate compound represented by General Formula (VIIa) to a distillation device, extracting the liquid-phase component from the distillation device when separating the isocyanate compound represented by General Formula (VIIa) as a gas-phase component.

<38> The recovery method according to any one of <30> to <37>,

in which the liquid-phase component includes a compound having one or more functional groups selected from the group consisting of a group represented by Formula (IX-1), a group represented by Formula (IX-2), a group represented by Formula (IX-3), a group represented by Formula (IX-4), a group represented by Formula (IX-5), a group represented by Formula (IX-6), a group represented by Formula (IX-7), a group represented by Formula (IX-8), a group represented by Formula (IX-9), a group represented by Formula (IX-10), a group represented by Formula (IX-11), and a group represented by Formula (IX-12).

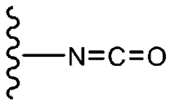

(IX-1)

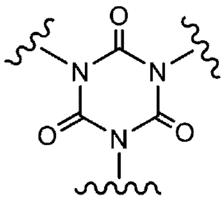

(IX-2)

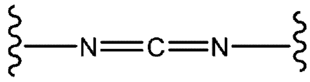

(IX-3)

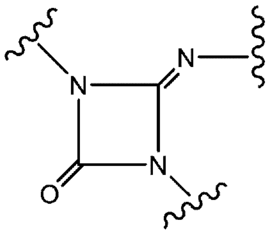

(IX-4)

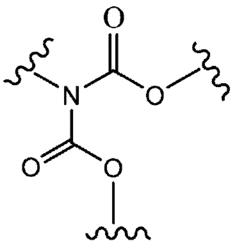

(IX-5)

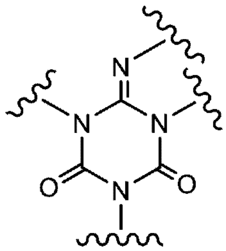

(IX-6)

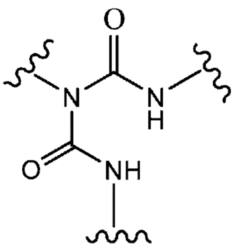

(IX-7)

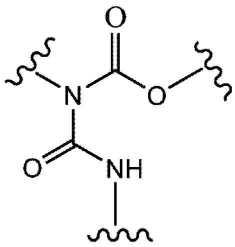

(IX-8)

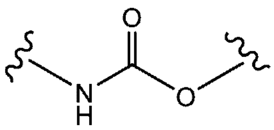

(IX-9)

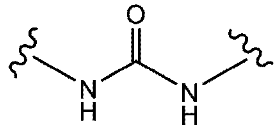

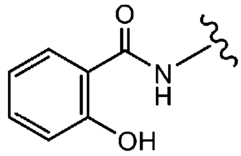

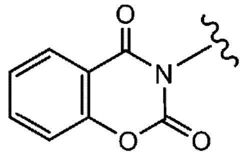

(IX-10) (IX-11) (IX-12)

(in Formulae (IX-1) to (IX-12), a wavy line represents a bonding site)

<39> The recovery method according to <38>,
in which the liquid-phase component includes a compound having two or more functional groups selected from the group consisting of the group represented by Formula (IX-1), the group represented by Formula (IX-2), the group represented by Formula (IX-3), the group represented by Formula (IX-4), the group represented by Formula (IX-5), the group represented by Formula (IX-6), the group represented by Formula (IX-7), the group represented by Formula (IX-8), the group represented by Formula (IX-9), the group represented by Formula (IX-10), the group represented by Formula (IX-11), and the group represented by Formula (IX-12).
<40> The recovery method according to any one of <30> to <39>,
in which the catalyst is one or more compounds selected from the group consisting of a hydroxide or an oxide of an alkali metal, a hydroxide or an oxide of an alkaline earth metal, a tertiary amine compound, a metal oxide of Group 12, a metal oxide of Group 13, and a metal oxide of Group 14.
<41> The recovery method according to <40>,
in which the catalyst is one or more compounds selected from the group consisting of a hydroxide or an oxide of an alkali metal, and a hydroxide or an oxide of an alkaline earth metal.
<42> The recovery method according to <41>,
in which the catalyst is a hydroxide or an oxide of an alkali metal.
<43> The recovery method according to any one of <30> to <42>, further including:

the following step (b); and
the following step (c),
a step (b) of separating the amine compound represented by General Formula (IIa) from the reaction solution containing the amine compound represented by General Formula (IIa), and
a step (c) of purifying the amine compound represented by General Formula (IIa).

<44> The recovery method according to <43>, further including:

the following step (d),
a step (d) of re-using the amine compound represented by General Formula (IIa), which has been purified in the step (c), as a raw material in the method for producing the isocyanate compound represented by General Formula (VIIa).

<45> The recovery method according to any one of <30> to <44>, further including:

the following step (e); and
the following step (f),
a step (e) of separating a hydroxy compound from the reaction solution containing the amine compound represented by General Formula (IIa), and
a step (f) of purifying the hydroxy compound.

<46> The recovery method according to <45>, further including:

the following step (g),
a step (g) of re-using the hydroxy compound purified in the step (f) as a raw material in the method for producing the isocyanate compound represented by General Formula (VIIa).

<47> The recovery method according to <45>, further including:

the following step (h),
a step (h) of re-using, in the step (a), one or more residual liquids selected from the group consisting of a residual liquid after separating the amine compound represented by General Formula (IIa) in the step (b), a residual liquid after purifying the amine compound represented by General Formula (IIa) in the step (c), a residual liquid after separating the hydroxy compound in the step (e), and a residual liquid after purifying the hydroxy compound in the step (f).

<48> An isocyanate composition containing:

an isocyanate compound;
a carbonyl compound represented by General Formula (I); and
a modified product of an isocyanate compound, in which a part of an isocyanate group of the isocyanate compound is converted into one or more functional groups selected from the group consisting of an isocyanurate group, a carbodiimide group, a uretonimine group, and an allophanate group,
in which the isocyanate compound is a different compound from the carbonyl compound, and
a value of {3 × (a molar amount of the isocyanurate group) + 2 × (a molar amount of the carbodiimide group) + 3 × (a molar amount of the uretonimine group) + 2 × (a molar amount of the allophanate group)} ÷ (a molar amount of the carbonyl compound) is 0.001 or more and 8.0 or less.

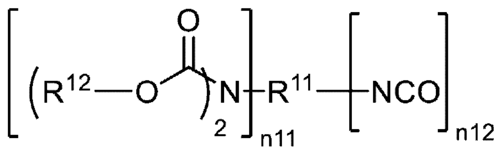
(I)

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less)

<49> The isocyanate composition according to <48>,

in which $R^{11}$ is a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom, and
$R^{12}$ is a monovalent aromatic hydrocarbon group having 6 or more and 20 or less carbon atoms, which may have an oxygen atom.

<50> The isocyanate composition according to <48> or <49>,

in which the isocyanate compound is a compound represented by General Formula (VIIb).

$$R^{71b}\!-\!\left(NCO\right)_{n71b} \qquad (VIIb)$$

(in General Formula (VIIb), $R^{71b}$ is an n71b-valent organic group, and a relational expression of $R^{71b} = R^{11}$ is satisfied, and n71b is an integer of 2 or more and 8 or less, and a relational expression of n71b = n11 + n12 is satisfied)

<51> The isocyanate composition according to any one of <48> to <50>,

in which the isocyanurate group is a group represented by General Formula (X-1), the carbodiimide group is a group represented by General Formula (X-2), the uretonimine group is a group represented by General Formula (X-3), and the allophanate group is a group represented by General Formula (X-4).

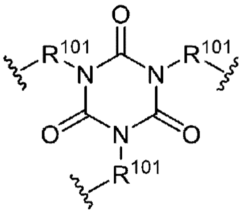 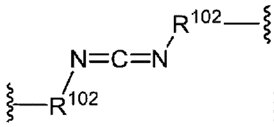 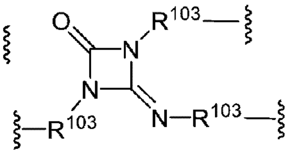 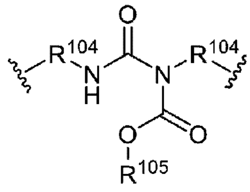

(X-1) (X-2) (X-3) (X-4)

(in General Formulae (X-1) to (X-4), $R^{101}$, $R^{102}$, $R^{103}$, and $R^{104}$ are each independently a di- or higher and octa- or lower valent organic group, and relational expressions of $R^{101} = R^{71b}$, $R^{102} = R^{71b}$, $R^{103} = R^{71b}$, and $R^{104} = R^{71b}$ are satisfied, $R^{105}$ is a monovalent organic group, and a relational expression of $R^{105} = R^{12}$ is satisfied, and a wavy line represents a bonding site)

<52> The isocyanate composition according to any one of <48> to <51>,

in which a content of the isocyanate compound is 1% by mass or more and 99% by mass or less with respect to a total mass of the isocyanate composition, and
a total content of the carbonyl compound and the modified product of the isocyanate compound is 1 % by mass or more and 99% by mass or less with respect to the total mass of the isocyanate composition.

<53> The isocyanate composition according to any one of <48> to <52>, further containing:

one or more compounds selected from the group consisting of a carbamate group-containing compound represented by General Formula (XI) and a carbonic acid ester represented by General Formula (III-1a), each of which is contained in an amount of 2.0 ppm by mass or more and 99% by mass or less with respect to a total mass of the isocyanate composition.

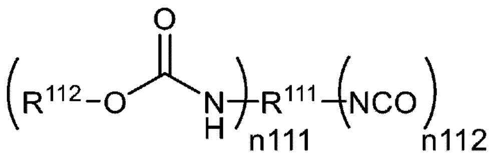 ( XI )

(in General Formula (XI), $R^{111}$ is an (n111 + n112)-valent organic group, and a relational expression of $R^{111} = R^{11}$ is satisfied, $R^{112}$ is a monovalent organic group, and a relational expression of $R^{112} = R^{12}$ is satisfied, n111 is an integer of 1 or more and 8 or less, n112 is an integer of 0 or more and 7 or less, a sum of n111 and n112 is an integer of 2 or more and 8 or less, and a relational expression of n111 + n112 = n11 + n12 is satisfied)

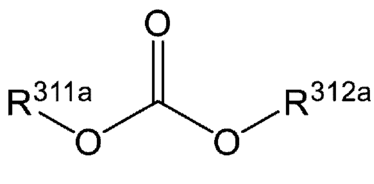 ( III-1a )

(in General Formula (III-1a), $R^{311a}$ and $R^{312a}$ are each independently a monovalent organic group, and a relational expression of $R^{311a} = R^{312a} = R^{12}$ is satisfied)

<54> The isocyanate composition according to <53>, further containing:
one or more compounds selected from the group consisting of the carbamate group-containing compound represented by General Formula (XI) and the carbonic acid ester represented by General Formula (III-1a), each of which is contained in an amount of 2.0 ppm by mass or more and $1.0 \times 10^4$ ppm by mass or less with respect to the total mass of the isocyanate composition.
<55> A method for producing an isocyanate compound, including:
distilling and purifying the isocyanate composition according to any one of <48> to <54> to continuously recover the isocyanate compound as a gas-phase component.

[Advantageous Effects of Invention]

According to the above-described production method of the aspect, the yield is favorable, stable continuous operation can be performed, and an amount of by-products incorporated can be suppressed.
In addition, according to the above-described method for producing a carbamate compound of the aspect, it is possible to provide a method for producing a carbamate compound, which does not use a metal catalyst, is inexpensive, and has a favorable yield.
In addition, according to the above-described recovery method of the aspect, it is possible to efficiently regenerate a useful component including an amine compound from a liquid-phase component remaining after production of an isocyanate compound.
In addition, according to the above-described isocyanate composition of the aspect, it is possible to provide an isocyanate composition which can prevent fixation of by-products to a device and improve the yield of an isocyanate compound in a case of producing an isocyanate compound without using phosgene. The above-described method for producing an isocyanate compound of the aspect is a method using the above-described isocyanate composition, in which fixation of by-products to a device can be prevented and the yield of the isocyanate compound can be improved.

[Brief Description of Drawings]

FIG. 1 is a schematic configuration diagram showing an apparatus used in a step (1) of Example 1-1.
FIG. 2 is a schematic configuration diagram showing an apparatus used in a step (1a) of Example 1-1.
FIG. 3 is a schematic configuration diagram showing an apparatus used in a step (2) in Example 1-1.
FIG. 4 is a schematic configuration diagram showing an apparatus used in step (3) of Example 1-1.
FIG. 5 is a schematic configuration diagram showing an apparatus used in a step (4) of Example 1-1.
FIG. 6 is a schematic configuration diagram showing an apparatus used in a step (6) in Example 1-1.
FIG. 7 is a schematic configuration diagram showing an apparatus used in a step (5) of Example 1-1.
FIG. 8 is a schematic configuration diagram showing an apparatus used in a step (5) of Example 1-3.
FIG. 9 is a schematic configuration diagram showing an apparatus used in a step (2) in Examples 1-7.
FIG. 10 is a schematic configuration diagram showing an apparatus used in a step (2) of Example 1-8.
FIG. 11 is a schematic configuration diagram showing an apparatus used in a step (1) in Example 1-9.
FIG. 12 is a schematic configuration diagram showing an apparatus used in a step (2) in Example 1-9.
FIG. 13 is a schematic configuration diagram showing an apparatus used in a step (1) in Examples 1-16.
FIG. 14 is a schematic configuration diagram showing an apparatus used in a step (1b) in Examples 1-16.
FIG. 15 is a schematic configuration diagram showing an apparatus used in a step (2) in Examples 1-16.
FIG. 16 is a schematic configuration diagram showing an apparatus used in a step (1a) of Comparative Example 1-6.
FIG. 17 is a schematic configuration diagram showing an apparatus used in a step (1b) of Comparative Example 1-6.
FIG. 18 is a schematic configuration diagram showing an apparatus used in a step (2) of Comparative Example 1-6.
FIG. 19 is a schematic configuration diagram of a production apparatus of a carbamate compound in Examples.
FIG. 20 is a production apparatus of an isocyanate compound used in Examples.
FIG. 21 is a recovery device for an amine compound used in Examples.
FIG. 22 is a separation device for an amine compound used in Examples.
FIG. 23 is a production apparatus of a carbamate compound used in Examples.
FIG. 24 is an apparatus used in a preliminary concentrating step of a reaction solution containing a carbamate compound in Examples.
FIG. 25 is a production apparatus of an isocyanate compound used in Examples.
FIG. 26 is a recovery device for an amine compound used in Examples.
FIG. 27 is a production apparatus of a carbamate compound used in Examples.
FIG. 28 is a recovery device for an amine compound used in Examples.
FIG. 29 is an apparatus used in a transesterification reaction step of a carbamate compound in Examples.
FIG. 30 is a production apparatus of an isocyanate compound used in Examples.
FIG. 31 is a recovery device for an amine compound used in Examples.
FIG. 32 is an apparatus used in a thermal decomposition step of a carbamate compound in Examples.
FIG. 33 is an apparatus used in a purification step (low-boiling separation step) of an isocyanate compound in Examples.
FIG. 34 is an apparatus used in a purification step (high-boiling separation step) of an isocyanate compound in Examples.
FIG. 35 is a recovery device for an amine compound used in Examples.

FIG. 36 is an apparatus used in a thermal decomposition step of a carbamate compound used in Examples.
FIG. 37 is a schematic configuration diagram showing a production apparatus of a carbamate compound used in Examples.
FIG. 38 is a schematic configuration diagram showing a thermal decomposition reaction device used in Examples.
FIG. 39 is a schematic configuration diagram showing a low-boiling separation device used in Examples.
FIG. 40 is a schematic configuration diagram showing a high-boiling separation device used in Examples.

[Description of Embodiments]

Hereinafter, the best mode for carrying out the present invention (hereinafter, referred to as "present embodiment") will be described in detail. The present invention is not limited to the following aspects, and can be variously modified and implemented within the scope of the gist thereof.

In the present specification, in a case where the IUPAC rule and the Nomenclature rule defined by IUPAC shown later are referred to (except for a case in which IUPAC recommendation of another year is specially cited), it means that the "Nomenclature of Organic Chemistry and Biochemistry" (Revised 2nd Edition published by Nankodo Co., Ltd. in Japan in 1992) is referred to, which is based on Recommendations 1979, and includes all the rules of organic chemistry and biochemistry published as a separate volume of "Chemical Nomenclature" in 1980, and the subsequent revisions and recommendations are added thereto. A term "organic" generally refers to a group of compounds to be named according to the nomenclature disclosed in the nomenclature. The target may be a target described in the recommendation issued in 1993. However, the "organic" compound targeted in the above Nomenclature also includes an organometallic compound and a metal complex. In the present embodiment, unless particularly described, terms such as "organic group" and "substituent" mean a group formed of atoms not including a metal atom and a metalloid. Furthermore, in the present embodiment, preferably, an "organic compound", an "organic group", or a "substituent", which is composed of atoms selected from H (hydrogen atom), C (carbon atom), N (nitrogen atom), O (oxygen atom), S (sulfur atom), Cl (chlorine atom), Br (bromine atom), and I (iodine atom) is used.

In the following description, terms "aliphatic" and "aromatic" are frequently used. According to the above-mentioned IUPAC rules, organic compounds are classified into aliphatic compounds and aromatic compounds. The aliphatic compound is defined based on the IUPAC recommendation of 1995. In the recommendation, the aliphatic compound is defined as "Acyclic or cyclic, saturated or unsaturated carbon compounds, excluding aromatic compounds".

In addition, the "aliphatic compound" used in the description of the present embodiment includes both saturated and unsaturated compounds, and both chain compounds and ring compounds, and refers to an "organic compound", an "organic group", or a "substituent", which is composed of an atom selected from the group consisting of the above-described H (hydrogen atom), C (carbon atom), N (nitrogen atom), O (oxygen atom), S (sulfur atom), Si (silicon atom), Cl (chlorine atom), Br (bromine atom), and I (iodine atom), and a halogen atom.

In a case where an aromatic group such as an aralkyl group is bonded to an aliphatic group, the group may be represented as "an aliphatic group substituted with an aromatic group" or "a group consisting of an aliphatic group to which an aromatic group is bonded". This is based on the reactivity in the present embodiment, and this is because the properties related to the reaction of a group such as an aralkyl group are extremely similar to the aliphatic reactivity rather than the aromaticity.

In addition, a non-aromatic reactive group including an aralkyl group, an alkyl group, and the like may be expressed as "an aliphatic group which may be substituted with an aromatic group", "an aliphatic group to which an aromatic group may be bonded", or the like.

In the explanation of the general formula of the compound used in the present specification, the definition according to the Nomenclature rule determined by IUPAC described above is used, but the common name may be used for the specific base name and the name of the exemplary compound. In addition, in the present specification, in a case where the number of atoms, the number of substituents, and the number of groups are described, all of these represent integers.

"Active hydrogen" in the present specification refers to a hydrogen atom bonded to an oxygen atom, a sulfur atom, a nitrogen atom, a silicon atom, or the like, and a hydrogen atom of a terminal methine group. Although the hydrogen of the hydroxy group is also the active hydrogen, the hydroxy group is also included in a composition including a product obtained by the production method of the present embodiment or a reaction solution including a reaction raw material, and is not a group that has a bad influence. Therefore, unless otherwise specified, the hydroxy group is excluded from the group including an active hydrogen. The "active hydrogen" is, for example, hydrogen included in an atomic group such as a -OH group, a -C(=O)OH group, a - C(=O)H group, a -SH group, a $-SO_3H$ group, a $-SO_2H$ group, a -SOH group, a $-NH_2$ group, a -NH- group, a -SiH group, and a -C=CH group.

As a compound having a hydroxy group (-OH group), an alcohol and an aromatic hydroxy compound are exemplary examples.

The "alcohol" in the present specification is a compound in which a hydroxy group is bonded to a saturated

carbon atom (Compounds in which a hydroxy group, -OH, is attached to a saturated carbon atom: $R_3COH$) as defined by IUPAC (Rule C-201), and does not include an aromatic hydroxy compound in which a hydroxy group is bonded to an aromatic ring.

The "aromatic hydroxy compound" in the present specification is phenols "Compounds having one or more hydroxy groups attached to a benzene or other arene ring in which one or more hydroxy groups are bonded to a benzene ring or another arene ring", as described in the definition (Rule C-202) of IUPAC.

<<Method for producing isocyanate compound>>

The method for producing an isocyanate compound according to the present embodiment (hereinafter, may be simply referred to as "production method according to the present embodiment") includes the following steps (1) to (4):

a step (1) of reacting a primary amine compound with a carbonic acid derivative to obtain an N-substituted carbamate compound while extracting a by-product compound having a boiling point lower than a boiling point of the N-substituted carbamate compound (hereinafter, also referred to as "low-boiling compound");
a step (2) of performing thermal decomposition of the N-substituted carbamate compound on a reaction solution containing the N-substituted carbamate compound, which is obtained in the step (1), in the presence of an aprotic solvent to obtain an isocyanate compound while extracting a by-product hydroxy compound;
a step (3) of separating the isocyanate compound and the aprotic solvent from a reaction solution containing the isocyanate compound, which is obtained in the step (2); and
a step (4) of removing a component having a boiling point higher than a boiling point of the isocyanate compound (hereinafter, may be simply referred to as "high-boiling-point component") from a fraction containing the isocyanate compound obtained in the step (3) to purify the isocyanate compound.

In the production method according to the present embodiment, the generation reaction of the N-substituted carbamate compound is carried out while extracting the low-boiling compound by-produced in the step (1), and the generation reaction of the isocyanate compound is carried out while extracting the hydroxy compound by-produced in the step (2). Therefore, in the reactions of the step (1) and the step (2), in which a reaction equilibrium constant K in the related art is biased to the raw material side to approximately $10^{-3}$ or more and $10^{-1}$ or less, the reaction equilibrium can be shifted to the production side, and the yield of the isocyanate compound can be improved. In addition, in the above-described step (4), the high-boiling-point component in the fraction containing the isocyanate compound functions as a solvent, so that the amount of the high-boiling-point component incorporated can be suppressed, and the isocyanate compound can be isolated and purified with high purity.

Hereinafter, each step of the production method according to the present embodiment will be described in detail. In addition, details of raw materials used in each step and obtained products will be described later.

<Step (1): production step of N-substituted carbamate compound>

In the step (I), the primary amine compound and the carbonic acid derivative are reacted with each other to obtain an N-substituted carbamate compound while extracting a by-product low-boiling compound.

The step (1) can also be referred to as a production step of the N-substituted carbamate compound. In the step (1), a terminal amino group of the primary amine compound is converted by the reaction of the primary amine compound with the carbonic acid derivative, and the N-substituted carbamate compound is obtained.

Here, in a case where urea is used as the carbonic acid derivative, after a reaction intermediate in which the terminal amino group of the primary amine compound is a ureido group is generated in the presence of the hydroxy compound, the terminal ureido group further reacts with the hydroxy compound, and a compound in which the terminal ureido group is substituted with a terminal structure in which a residue obtained by removing a hydroxyl group from the hydroxy compound is bonded through a carbamate group, that is, the N-substituted carbamate compound is obtained. In this case, the by-product low-boiling compound is ammonia.

In addition, in a case where a urea derivative (a compound represented by General Formula (IV) described later) is used as the carbonic acid derivative, the by-product low-boiling compound is one or more compounds selected from the group consisting of an alkylamine corresponding to an alkyl group in which an amino group of ammonia or urea is substituted.

In the step (I), hereinafter, the case where urea is used as the carbonic acid derivative will be described.

The method in which the production reaction of the N-substituted carbamate compound through the above-described ureido intermediate and the extraction of by-product ammonia is not particularly limited as long as it can be performed at the same time; but the step (1) is preferably performed using a distillation column, a tubular evaporator, a thin film evaporator, or a falling film-type evaporator, more preferably performed using a distillation column, a thin film

evaporator, or a falling film-type evaporator, still more preferably performed a distillation column or a falling film-type evaporator, and particularly preferably performed using a distillation column. In a case where a distillation column is used, the distillation is preferably performed by a method called a reactive distillation method, because a chemical change or reaction is involved in the step. The reactive distillation method is a method in which the above-described reaction is carried out in a reactor using a mixture of the hydroxy compound, the primary amine compound, and one or more compounds selected from the group consisting of urea and urea derivatives, and by-product ammonia is extracted from the reactor by distillation. Since a boiling point of the ammonia is lower than that of the N-substituted carbamate compound which is the main product, the fraction containing the N-substituted carbamate compound can be efficiently obtained as a liquid-phase component by extracting the ammonia which is the by-product as a gas-phase component.

In addition, the step (1) is preferably performed by a continuous method. The continuous method is a method in which the above-described mixture is continuously supplied to a reactor to perform a production reaction of the N-substituted carbamate compound through a ureido intermediate, and ammonia as a by-product is continuously extracted from the reactor. A supply rate of the above-described mixture to the reactor and an extraction rate of the ammonia can be appropriately adjusted according to a production rate of the N-substituted carbamate compound and the ammonia, which are products.

A use amount of the primary amine compound and the one or more compounds selected from the group consisting of urea and urea derivatives can be an amount at which a molar ratio of an amino group of the primary amine compound to a urea group of the compound is 1:3 to 3:1, and is preferably an amount at which the molar ratio is 1:2 to 2:1, more preferably an amount at which the molar ratio is 1:1.5 to 1.5:1, and still more preferably an amount at which the molar ratio is 1:1. In a case where the amount of the above-described two compounds is within the above-described range, the ureidation reaction can be performed more efficiently.

A use amount of the hydroxy compound is preferably an amount at which a molar ratio of a hydroxyl group of the hydroxy compound to the amino group of the primary amine compound is 1/1 or more and 300/1 or less, more preferably an amount at which the molar ratio is 1/1 or more and 250/1 or less, and still more preferably an amount at which the molar ratio is 1/1 or more and 200/1 or less. In a case where the molar ratio of the hydroxyl group of the hydroxyl compound to the amino group of the primary amine compound is equal to or more than the above-described lower limit value, a carbamate group can be more efficiently produced. On the other hand, in a case of being equal to or less than the above-described upper limit value, the amount of the hydroxy compound used can be further reduced while maintaining a production efficiency of the carbamate group.

In addition, in order to quickly recover the low-boiling-point decomposition product, a reactor carrier agent can be introduced, and the gas-phase component containing the carrier agent can be carried out in a thermal decomposition reactor. The term "carrier agent" as used herein refers to a substance that is substantially inert and is in a gaseous state under thermal decomposition reaction conditions.

Specific examples of such a carrier agent include an inert gas and a hydrocarbon gas. Examples of the inert gas include nitrogen, argon, helium, carbon dioxide, methane, ethane, and propane. Among these, an inert gas such as nitrogen is preferable as the carrier agent.

As a material having the same effect, organic solvents having a low boiling point may be used. Examples of the low-boiling-point organic solvent include halogenated hydrocarbons, lower hydrocarbons, and ethers. Examples of the hydrocarbon halides include dichloromethane, chloroform, and carbon tetrachloride. Examples of the lower hydrocarbons include pentane, hexane, heptane, and benzene. Examples of the ethers include tetrahydrofuran and dioxane.

These carrier agents may be used alone, or two or more thereof may be mixed and used. In addition, it is preferable to use these carrier agents after being pre-heated.

A reaction temperature varies depending on the kind of the primary amine compound used and a reaction pressure, but it is preferably 40°C or higher and 380°C or lower, more preferably 50°C or higher and 320°C or lower, still more preferably 60°C or higher and 300°C or lower, particularly preferably 80°C or higher and 300°C or lower, and most preferably 100°C or higher and 280°C or lower.

The reaction pressure varies depending on the kind of the compounds to be used and the reaction temperature, but may be any of decompression, normal pressure, or pressurization. The pressure is preferably in a range of 20 Pa or more and $2 \times 10^6$ Pa or less, more preferably in a range of 30 Pa or more and $1.5 \times 10^6$ Pa or less, still more preferably in a range of 50 Pa or more and $1 \times 10^6$ Pa or less, and particularly preferably in a range of 50 Pa or more and $0.5 \times 10^6$ Pa or less.

A reaction time (in the case of the continuous method, a residence time) is not particularly limited, but is preferably 0.001 hours or more and 100 hours or less, more preferably 0.005 hours or more and 50 hours or less, and still more preferably 0.01 hours or more and 10 hours or less.

In addition, in a case where a carbonic acid ester is used as the carbonic acid derivative, the carbonic acid ester is activated by directly reacting with the terminal amino group of the primary amine compound. The carbonic acid ester is a compound in which one or two atoms of two hydrogens of carbonic acid $CO(OH)_2$ are substituted with an alkyl group or an aryl group, and is a compound represented by General Formula (III) described later. In this case, a compound

in which a carbamate group is substituted with the alkyl group or the aryl group derived from the carbonic acid ester, that is, the N-substituted carbamate compound is obtained. In addition, the by-product low-boiling compound is a hydroxy compound formed of the alkyl group or the aryl group derived from the carbonic acid ester and a hydroxy group.

Reaction conditions between the carbonic acid ester and the primary amine compound vary depending on the compounds to be reacted, but a molar amount of the carbonic acid ester is preferably an amount of 1 time or more and more preferably an amount of 1.01 times or more and 1,000 times or less with respect to the molar amount of the terminal amino group of the primary amine compound, in terms of a stoichiometric ratio. In order to increase the reaction rate and complete the reaction at an early stage, the molar amount of the carbonic acid ester is preferably an excessive amount with respect to the molar amount of the terminal amino group of the primary amine, and in consideration of a size of a carbamating reactor, the amount is more preferably 1.01 times or more and 50 times or less and particularly preferably 1.2 times or more and 10 times or less.

The reaction between the carbonic acid ester and the primary amine compound is preferably carried out in a liquid phase using an appropriate solvent. The solvent can be appropriately selected depending on the compounds to be used, and for example, oxygen atom-containing compounds such as aliphatic hydrocarbons, aromatic hydrocarbons, aromatic hydroxy compounds, alcohols, and ethers; sulfur atom compounds such as thiols and sulfides; halogenated aliphatic hydrocarbons; halogenated aromatic hydrocarbons; or water can be used, and these compounds can be appropriately used in combination.

The primary amine compound is preferably present in a liquid state in the carbamating reactor. At this time, it is preferable that the primary amine compound is supplied in a state of being dissolved in a solvent, and as the solvent for dissolving the primary amine compound, the above-described solvent is preferably used. It is also preferable that the primary amine compound is supplied as a mixture with the hydroxy compound, the water, or the carbonic acid ester.

The carbamylation reaction in the step (1) is carried out by supplying the carbonic acid ester and the primary amine compound to the carbamating reactor. The reaction temperature is preferably 0°C or higher and 150°C or lower. In order to increase the reaction rate, a high temperature is preferable, but from the viewpoint of suppressing an undesirable side reaction, 10°C or higher and 100°C or lower is more preferable. In order to keep the reaction temperature constant, a known cooling device or heating device may be installed in the above-described carbamating reactor.

In addition, the reaction pressure varies depending on the kinds of the compounds to be used and the reaction temperature, but may be any of a reduced pressure, a normal pressure, and a pressurized pressure, and is usually in a range of 20 Pa or more and $1 \times 10^6$ Pa or less.

The reaction time (in a case of the continuous method, the residence time) is not particularly limited, and is usually preferably 0.001 hours or more and 50 hours or less, more preferably 0.01 hours or more and 20 hours or less, and still more preferably 0.1 hours or more and 10 hours or less. In addition, the reaction can be terminated by, for example, collecting the reaction solution and confirming that a desired amount of the carbamic acid ester is produced by liquid chromatography.

In the present embodiment, a catalyst may be or may not be used in the reaction between the carbonic acid ester and the primary amine compound, in addition to these compounds. In a case where the catalyst is not used, it is possible to prevent thermal denaturation of the carbamic acid ester due to the influence of a metal component derived from the catalyst.

In a case where the catalyst is used, the reaction can be completed in a short time, and the reaction temperature can be lowered.

In a case where the catalyst is used, for example, an organic metal compound or an inorganic metal compound, such as tin, lead, copper, and titanium; an alkaline metal or alkaline earth metal alcoholate, which is a basic catalyst such as a methylate, ethylate, or butylate (each isomer) of lithium, sodium, potassium, calcium, or barium; or the like can be used.

In the reaction for producing the N-substituted carbamate compound, a reactor which meets the conditions is appropriately selected. The reactor may be a plug flow reactor or a tank-type reactor. Specifically, a conventionally known reactor such as a stirring tank, a pressurized stirring tank, a reduced pressure stirring tank, a tower-type reactor, a distillation column, a filled column, and a thin film distillation column can be appropriately combined and used. In order to efficiently recover the gas-phase component, a known distillation device is preferably used. For example, various known methods are used, such as a method using a reactor including any one of a distillation column, a multi-stage distillation column, a multi-tube type reactor, a tank-type reactor, a continuous multi-stage distillation column, a tray column, a filled column, a thin film evaporator, a reactor having a support inside, a forced circulation reactor, a falling film evaporator, a falling drop evaporator, and a falling film-type evaporator, and a method in which these reactors are combined. From the viewpoint of rapidly removing the by-product low-boiling compound from the reaction system, a structure having a large gas-liquid contact area, which can rapidly move the by-product low-boiling compound to the gas phase, is preferable. Among these, it is particularly preferable that the step (1) is performed using one or more reactors selected from the group consisting of a tank-type reactor, a distillation column, a tubular evaporator, a thin film evaporator, and a falling film-type evaporator.

In a case where a filled column is used, as a solid filling material included in the filled column, a filling material generally used in a distillation column or an absorption column can be appropriately used. Specific examples of a preferred solid filling material include a Raschig ring, a Cascade Mini Ring, a Ressing ring, a spiral ring, a ball ring, an Intalox saddle, a Stedman packing, a MacMahon packing, a Dixon packing, a Helix packing, a coil packing, and a heat pipe packing. In a case where the distillation column is a tray column, various tray columns having sieve trays, cascade trays, turbo grid trays, ripple trays, dual trays, or the like can be appropriately used.

A material of the solid filling material is not particularly limited as long as the material is inert to the decomposition product, and the solid filling material may be made of a magnetic material, a metal material, or the like. Among these, a material having high thermal conductivity is preferable as the material of the solid filling material. As the material, in addition to SUS304, SUS316, and SUS316L, silicon oxide (compositional formula: SiO2), aluminum oxide (compositional formula: Al2O3), or fluorocarbon (-CHF- or a compound having a repeating unit of CF2) is generally preferable. In addition, glass, ceramic, and fluororesin, which are made of these components, are also preferable. In a case of glass or ceramic, a content of silicon oxide or aluminum oxide is not particularly limited, and various materials can be selected in which the content of silicon oxide may be 60% by mass or more, or the content of aluminum oxide may be 60% by mass or more.

The ceramic or the ceramics referred to herein is a sintered body obtained by sintering an inorganic substance. Here, regardless of metal or non-metal, the inorganic substance is a general term for semiconductors such as silicon, molded bodies of inorganic compounds such as carbides, nitrides, borides, and titanium oxides, powders, films, and the like. Examples of the fluororesin include polytetrafluoroethylene, polychlorotrifluoroethylene, polyvinylidene fluoride, polyvinyl fluoride, a perfluoroalkoxy fluororesin, a tetrafluoroethylene/hexafluoropropylene copolymer, an ethylene/tetrafluoroethylene copolymer, and an ethylene/chlorotrifluoroethylene copolymer. Other metal atoms may be contained in the preferred filling material shown here as long as it does not depart from the gist of the present embodiment.

The type of the condenser provided in the reactor is not particularly limited, and a known condenser can be used. For example, it is possible to appropriately combine and use a condenser known in the related art, such as a multi-tube cylindrical condenser, a double-tube condenser, a single-tube condenser, and an air-cooled condenser. The condenser may be provided inside the reactor, may be provided outside the reactor and connected to the reactor with a pipe, or may be adopted in various forms in consideration of the type of the reactor or the condenser, the method of handling the condensate, and the like. Materials of the reactor and the condenser are not particularly limited, and known materials can be used as long as they do not adversely affect the above-described N-substituted carbamate compound or the isocyanate as a product. For example, a glass, stainless steel, carbon steel, Hastelloy material, a material on which a glass lining is applied, or a material on which a Teflon (registered trademark) coating is applied can also be used. Among these, SUS304, SUS316, SUS316L, or the like can be preferably used because they are inexpensive. As necessary, known process equipment such as a flowmeter, a thermometer, and a measuring instrument, a reboiler, a pump, and a condenser may be added, heating may be performed by a known method such as steam or a heater, and cooling may also be performed by a known method such as natural cooling, cooling water, or brine. A step may be added as necessary.

In the step (1), the reaction (ureido reaction) which produces a reaction intermediate in which the terminal amino group of the primary amine compound becomes a ureido group by the reaction of the primary amine compound with the one or more compounds selected from the group consisting of urea and a urea derivative and the reaction which produces the N-substituted carbamate compound by the reaction of the reaction intermediate having the ureido group and the hydroxy compound may be carried out in the same reactor or may be carried out in separate reactors.

For the ureidation reaction, a conventionally known reactor such as a stirring tank, a pressure stirring tank, a decompression stirring tank, a tower-type reactor, a continuous stirring type reactor, a distillation column, a filled column, a plug flow reactor, or a thin film distillation device can be appropriately combined and used; and a stirring tank, a pressure stirring tank, a decompression stirring tank, a tower-type reactor, a continuous stirring type reactor, or a plug flow reactor is preferable, and from the viewpoint of production efficiency, it is more preferable to perform the reaction by a continuous method using a continuous stirring type reactor or a plug flow reactor.

In addition, in the step (1), other steps can be further added as necessary. Examples of the other steps include a step of performing a transesterification reaction of N-substituted carbamate using the N-substituted carbamate obtained in the above-described reaction and a hydroxy compound different from the hydroxy compound used in the above-described reaction, thereby producing a different N-substituted carbamate; a step of separating a part or all of the hydroxy compounds from the reaction solution obtained in the above-described reaction; a step of recovering the ammonia produced in the above-described reaction; and a step of decomposing and converting by-products into valuable materials. In addition, in the step (1), it is also one of preferable aspects to control the reaction conditions by appropriately refluxing as necessary.

<Step (2): production step of isocyanate compound (thermal decomposition step)>

In the step (2), the N-substituted carbamate compound is thermally decomposed using the reaction solution containing the N-substituted carbamate compound obtained in the step (1), in the presence of an aprotic solvent, and the isocyanate compound is obtained while extracting a by-product hydroxy compound.

The step (2) can also be referred to a production step of an isocyanate compound or a thermal decomposition step. In the step (2), the isocyanate compound is obtained by the thermal decomposition reaction of the N-substituted carbamate compound. In addition, the by-product hydroxy compound is a compound having the same structure as the hydroxy compound used in the step (1) or the hydroxy compound derived from the carbonic acid ester used in the step (1) (a hydroxy compound having a structure in which a hydroxy group is bonded to $R^{311}$ or $R^{312}$ in a compound represented by General Formula (III-1) described later).

In the step (2), the method in which the thermal decomposition reaction of the above-described N-substituted carbamate compound and the extraction of by-product hydroxy compound is not particularly limited as long as it can be performed at the same time; but the step (2) is preferably performed using a stirring tank, a pressure stirring tank, a decompression stirring tank, a tower-type reactor, a continuous stirring type reactor, a distillation column, a tubular evaporator, a thin film evaporator, or a falling film-type evaporator, more preferably performed using a distillation column, a thin film evaporator, or a falling film-type evaporator, still more preferably performed a distillation column or a falling film-type evaporator, and particularly preferably performed using a distillation column. In a case where a distillation column is used, the distillation is preferably performed by a method called a reactive distillation method, because a chemical change or the like is involved in the step. The step (2) using the reactive distillation method is a method in which the by-product hydroxy compound is extracted from the above-described reactor by distillation while performing the thermal decomposition reaction of the N-substituted carbamate compound in the reactor. In the above-described step (1), it is preferable to use a hydroxy compound having a lower standard boiling point than that of the isocyanate compound which is the main product, and accordingly, the hydroxy compound which is the by-product can be extracted as a gas-phase component, and the fraction containing the isocyanate compound can be more efficiently obtained as the liquid-phase component. The "standard boiling point" referred to herein indicates a boiling point at 1 atm.

In addition, it is also preferable to use a hydroxy compound having a higher standard boiling point than that of the isocyanate compound which is the main product. As a result, the hydroxy compound can be extracted as the liquid-phase component, and the fraction containing the isocyanate compound as the gas-phase component can be obtained.

A difference in standard boiling point between the isocyanate compound and the hydroxy compound is preferably 300°C or lower, more preferably 250°C or lower, and still more preferably 200°C or lower. On the other hand, the lower limit value of the difference in standard boiling point between the isocyanate compound and the hydroxy compound is usually 10°C or higher, preferably 25°C or higher, more preferably 50°C or higher, and still more preferably 100°C or higher.

In a case where the difference in standard boiling point between the isocyanate compound and the hydroxy compound is within the above-described range, the hydroxy compound after the thermal decomposition can be more efficiently extracted into the gas phase, a reverse reaction between the isocyanate compound and the hydroxy compound to be produced can be further reduced, and the yield of the isocyanate compound can be further improved.

In addition, the step (2) is preferably performed by a continuous method. The continuous method is a method in which the reaction solution containing the N-substituted carbamate compound is continuously supplied to a reactor to perform the thermal decomposition reaction of the N-substituted carbamate compound, and the hydroxy compound as a by-product is continuously extracted from the reactor. A supply rate of the above-described reaction solution to the reactor and an extraction rate of the hydroxy compound can be appropriately adjusted according to a production rate of the isocyanate compound and the hydroxy compound, which are products.

From the viewpoint of further improving the yield of the isocyanate compound, it is preferable that the standard boiling point of the aprotic solvent is lower than the standard boiling point of the isocyanate compound and higher than the hydroxy compound.

A difference in standard boiling point between the isocyanate compound and the aprotic solvent is preferably 100°C or lower, more preferably 90°C or lower, and still more preferably 80°C or lower. On the other hand, the lower limit value of the difference in standard boiling point between the isocyanate compound and the aprotic solvent is usually 10°C or higher, preferably 15°C or higher, more preferably 20°C or higher, still more preferably 25°C or higher, and particularly preferably 30°C or higher.

In a case where the difference in standard boiling point between the isocyanate compound and the aprotic solvent is equal to or less than the above-described upper limit value, the accompanying of the aprotic solvent can be further suppressed when extracting the hydroxy compound after the thermal decomposition into the gas phase, and the concentration of the isocyanate in the liquid phase can be prevented from being increased, whereby the reverse reaction between the isocyanate and the hydroxy compound to be produced, the denaturation reaction with the carbamate as the raw material, and the reaction of isocyanates can be further reduced. On the other hand, in a case of being equal to or more than the above-described lower limit value, in the step (3) described later, the aprotic solvent and the isocyanate

compound can be more efficiently separated from each other, and the yield of the isocyanate compound can be further improved.

In addition, the standard boiling point of the isocyanate compound is usually 150°C or higher, preferably 250°C or higher, more preferably 255°C or higher, still more preferably 300°C or higher, and particularly preferably 350°C or higher.

On the other hand, the upper limit of the standard boiling point of the isocyanate compound is not particularly limited, but is preferably 450°C or lower, more preferably 430°C or lower, and still more preferably 420°C or lower.

In a case where the standard boiling point of the isocyanate compound is equal to or more than the above-described lower limit value, the isocyanate compound tends to be more easily separated from the hydroxy compound which is a by-product in the step (2). On the other hand, in a case where the standard boiling point of the isocyanate compound is equal to or less than the above-described upper limit value, the isocyanate compound tends to be more easily separated from the aprotic solvent or the high-boiling-point component in the step (3) and step (4) described later.

A use amount of the aprotic solvent is not particularly limited, and is usually in a range of 0.1 % by mass or more and 100,000% by mass or less, preferably 1 % by mass or more and 10,000% by mass or less, more preferably 5% by mass or more and 5,000% by mass or less, still more preferably 10% by mass or more and 4,000% by mass or less, even more preferably 20% by mass or more and 3,000% by mass or less, particularly preferably 20% by mass or more and 2,500% by mass or less, and most preferably 30% by mass or more and 2,000% by mass or less with respect to the total mass of the reaction solution containing the N-substituted carbamate compound as the raw material.

In a case where the use amount of the aprotic solvent is equal to or more than the above-described lower limit value, the concentration of the produced isocyanate compound is further lowered, and thus it is possible to further suppress generation of nurate due to the reaction between the isocyanate compounds during the thermal decomposition and the generation of allophanate due to the reaction between the isocyanate compound and the N-substituted carbamate compound as the raw material, and it is possible to further improve the thermal decomposition yield of the isocyanate compound and to further suppress the precipitation of the polymer. On the other hand, in a case where the use amount of the aprotic solvent is equal to or less than the above-described upper limit value, the production efficiency of the isocyanate compound in the same apparatus can be further improved. In addition, the reaction may be carried out using a single aprotic solvent or a plurality of kinds of aprotic solvents in combination, and the aprotic solvent can be optionally selected according to the reaction conditions.

A thermal decomposition temperature of the N-substituted carbamate compound varies depending on the kind of the N-substituted carbamate compound used, but is preferably 140°C or higher and 380°C or lower, more preferably 160°C or higher and 320°C or lower, still more preferably 180°C or higher and 300°C or lower, particularly preferably 200°C or higher and 300°C or lower, and most preferably 220°C or higher and 280°C or lower. In order to keep the reaction temperature constant, a known cooling device and a heating device may be installed in the above-described thermal decomposition reactor.

In a case where the thermal decomposition temperature is equal to or more than the above-described lower limit value, the thermal decomposition reaction of the N-substituted carbamate compound proceeds further, and as the temperature is, the thermal decomposition rate is faster, and the thermal decomposition of the N-substituted carbamate compound is completed in a short time. On the other hand, in a case of being equal to or less than the above-described upper limit value, the modification reaction from isocyanate to nurate or the like can be further suppressed. Furthermore, since the amount of the polymer of isocyanate generated by the nuration can be further reduced, adhesion to the reaction device and clogging of the pipe can be further suppressed, and operability can be further improved.

In addition, the reaction pressure varies depending on the kind of the compounds to be used and the reaction temperature, but may be any of decompression, normal pressure, or pressurization. The pressure can be a pressure at which a saturated vapor pressure of the aprotic solvent to be used is reached, and is preferably in a range of 20 Pa or more and $10 \times 10^6$ Pa or less.

A reaction time (in the case of the continuous method, a residence time) is not particularly limited, but is preferably 0.001 hours or more and 100 hours or less, more preferably 0.005 hours or more and 50 hours or less, and still more preferably 0.01 hours or more and 10 hours.

In the thermal decomposition reaction, a catalyst is not necessarily required, but the catalyst may be used to lower the reaction temperature or to complete the reaction at an early stage. The catalyst is used in an amount of preferably 0.01 % by mass or more and 30% by mass or less, and more preferably 0.5% by mass or more and 20% by mass or less with respect to the mass of the N-substituted carbamate.

As the catalyst, for example, a Lewis acid, a transition metal compound which generates a Lewis acid, an organic tin compound, a copper metal, zinc, or various compounds of an iron metal can be used. Specific examples of the catalyst include Lewis acids represented by $AlX_3$, $TiX_3$, $TiX_4$, $VOX_3$, $VX_5$, $ZnX_2$, $FeX_3$, and $SnX_4$ (here, X is an oxygen atom, a nitrogen atom, a halogen atom, an acetoxy group, an alkoxy group, or an aryloxy group, and the number of valences satisfies the oxidation number of the metal) and transition metal compounds that generate Lewis acids; organic tin compounds represented by $(CH_3)_3SnOCOCH_3$, $(C_2H_5)SnOCOC_6H_5$, $Bu_3SnOCOCH_3$, $Ph_3SnOCOCH_3$,

$Bu_2Sn(OCOCH_3)_2$, $Bu_2Sn(OCOC_{11}H_{23})_2$, $Ph_3SnOCH_3$, $(C_2H_5)_3SnOPh$, $Bu_2Sn(OCH_3)_2$, $Bu_2Sn(OC_2H_5)_2$, $Bu_2Sn(OPh)_2$, $Ph_2Sn(CH_3)_2$, $(C_2H_5)_3SnOH$, PhSnOH, $Bu_2SnO$, $(C_8H_{17})_2SnO$, $Bu_2SnCl_2$, and BuSnO(OH); copper group metal compounds such as CuCl, $CuCl_2$, CuBr, $CuBr_2$, CuI, $CuI_2$, $Cu(OAc)_2$, $Cu(acac)_2$, olefinic acid copper, $Bu_2Cu$, $(CH3O)_2Cu$, AgNO3, AgBr, silver picrate, and AgC6H6ClO4; zinc compounds such as $Zn(acac)_2$; iron group metal compounds such as $Fe(C_{10}H_8)(CO)_5$, $Fe(CO)_5$, $Fe(C_4H_6)(CO)_3$, $Co(mesitylene)_2(PEt_2Ph_2)$, $C_OC_5F_5(CO)_7$, and ferrocene (here, Bu represents a butyl group, Ph represents a phenyl group, and acac represents an acetylacetonate ligand); and amines such as 1,4-diazabicyclo[2,2,2]octane, triethylenediamine, and triethylamine. Among these, organic metal catalysts such as dibutyltin dilaurate, lead octylate, and stannooctate are exemplary examples. These compounds may be used alone or as a mixture of two or more kinds thereof. In addition, it is also preferable to hold the above-described metal component on a carrier which is substantially inert and does not impair the reaction. As the carrier, various materials such as activated carbon, silicon, aluminum, zirconium, titanium, oxides thereof, and composite oxides thereof are used.

As a shape of the catalyst, various shapes such as powder, columnar, ringshaped, and spherical are preferably used, and a columnar shape, a ring shape, or a spherical shape is particularly preferable.

However, in a case where a catalyst is used in the above-described step (1), a catalyst residue or the like may be supplied to the step (2). In the thermal decomposition reaction of the step (2), such a catalyst residue or the like may be present.

In the reaction for producing the N-substituted carbamate compound, a reactor which meets the conditions is appropriately selected. As the reactor, the reactors described in the step (1) are exemplary examples.

In addition, a step of recovering the decomposition gas and distilling and separating the decomposition gas by utilizing a difference in boiling point may be added according to the purpose. For example, PCT International Publication No. WO2013/111353 (Reference Document 1) discloses a method of recovering isocyanate and a hydroxy compound produced by a thermal decomposition reaction as gas-phase components, and distilling and separating the components in a multi-stage distillation column. In a case where the multi-stage distillation column is a tray column, various tray columns having sieve trays, cascade trays, turbo grid trays, ripple trays, dual trays, or the like can be appropriately used. In addition, in the step (2), it is also one of preferable aspects to control the reaction conditions by appropriately refluxing as necessary.

In a case where the multi-stage distillation column is a filled column, as a solid filling material included in the filled column, a filling material generally used in a distillation column or an absorption column can be appropriately used. Specific examples of a preferred solid filling material include a Raschig ring, a Cascade Mini Ring, a Ressing ring, a spiral ring, a ball ring, an Intalox saddle, a Stedman packing, a MacMahon packing, a Dixon packing, a Helix packing, a coil packing, a heat pipe packing, Mellapak, Sulzer packing, Goodroll packing, and Flexipack.

An interior material such as a filling material is preferably inert to the decomposition product, and as the material, in addition to SUS304, SUS316, and SUS316L, silicon oxide (compositional formula: $SiO_2$), aluminum oxide (compositional formula: $Al_2O_3$), or fluorocarbon (-CHF- or a compound having a repeating unit of $CF_2$) is generally preferable. In addition, glass, ceramic, and fluororesin, which are made of these components, are also preferable. In a case of glass or ceramic, a content of silicon oxide or aluminum oxide is not particularly limited, and various materials can be selected in which the content of silicon oxide may be 60% by mass or more, or the content of aluminum oxide may be 60% by mass or more.

The ceramic referred to herein is a sintered body obtained by sintering an inorganic substance. Here, regardless of metal or non-metal, the inorganic substance is a general term for semiconductors such as silicon, molded bodies of inorganic compounds such as carbides, nitrides, borides, and titanium oxides, powders, films, and the like. Examples of the fluororesin include polytetrafluoroethylene, polychlorotrifluoroethylene, polyvinylidene fluoride, polyvinyl fluoride, a perfluoroalkoxy fluororesin, a tetrafluoroethylene/hexafluoropropylene copolymer, an ethylene/tetrafluoroethylene copolymer, and an ethylene/chlorotrifluoroethylene copolymer. Other metal atoms may be contained in the preferred filling material shown here as long as it does not depart from the gist of the present embodiment.

<Step (3): concentration step of isocyanate compound>

In the step (3), the isocyanate compound and the aprotic solvent are separated from the reaction solution containing the isocyanate compound obtained in the step (2). The amount of the reaction solution can be reduced by separating the excess aprotic solvent, and the productivity can be improved and the equipment cost can be reduced by reducing the equipment of each step.

The step (3) can also be referred to as a concentration step of the isocyanate compound. The method for separating the aprotic solvent is not particularly limited, but known distillation separation is preferable from the viewpoint of productivity and ease of process design.

From the viewpoint of suppressing side reactions, it is preferable that the distillation separation in the step (3) is carried out under as low temperature conditions as possible and for a short time to separate the aprotic solvent from

the reaction solution containing the isocyanate compound. Therefore, it is preferable that the step (3) is also performed by a continuous method in the same manner as the thermal decomposition reaction in the step (2) described above.

The separation method by the continuous method is a method in which the reaction solution containing the isocyanate compound is continuously supplied to a reactor, and the aprotic solvent is continuously extracted from the reactor. In addition, in the above-described step (2), it is preferable to use an aprotic solvent having a boiling point lower than that of the isocyanate compound, and accordingly, the aprotic solvent can be extracted as a gas-phase component, and the fraction containing the isocyanate compound as a liquid-phase component can be more efficiently separated.

Alternatively, it is also preferable to use an aprotic solvent having a boiling point higher than that of the isocyanate compound, and accordingly, the aprotic solvent can be extracted as a liquid-phase component, and the fraction containing the isocyanate compound as a gas-phase component can be more efficiently separated.

The pressure in the step (3) varies depending on the kind of the compounds to be used and the reaction temperature, but may be any of decompression, normal pressure, or pressurization as long as the isocyanate compound and the aprotic solvent can be separated. The pressure can be a pressure at which a saturated vapor pressure of the aprotic solvent to be used is reached, and is preferably in a range of 20 Pa or more and $1 \times 10^6$ Pa or less.

The operation time (in the case of the continuous method, the retention time) in the step (3) is not particularly limited as long as the isocyanate compound and the aprotic solvent can be separated, but it is preferably 5 seconds or longer and 100 hours or shorter, more preferably 10 seconds or longer and 50 hours or shorter, still more preferably 20 seconds or longer and 10 hours or shorter, even more preferably 20 seconds or longer and 1 hour or shorter, even still more preferably 20 seconds or longer and 50 minutes or shorter, particularly preferably 20 seconds or longer and 45 minutes or shorter, more particularly preferably 20 seconds or longer and 40 minutes or shorter, still more particularly preferably 30 seconds or longer and 30 minutes or shorter, and most preferably 30 seconds or longer and 25 minutes or shorter. In a case where the operation time is equal to or less than the above-described upper limit value, the operation time can be further shortened, and the side reactions of the isocyanate compound can be further suppressed. In addition, in a case where the operation time is equal to or more than the above-described lower limit value, the aprotic solvent can be more sufficiently extracted into the gas phase.

The temperature in the step (3) is not particularly limited as long as the isocyanate compound and the aprotic solvent are stable and the isocyanate compound and the aprotic solvent can be separated, but it is preferable that the aprotic solvent is separated under as low temperature conditions as possible and for a short time because uretdione is produced by reacting the isocyanate compound at 100°C or higher and 130°C or lower.

From the viewpoint of suppressing the modification of the isocyanate compound, the temperature is preferably 300°C or lower, more preferably in a range of 20°C or higher and 290°C or lower, and still more preferably in a range of 30°C or higher and 280°C or lower.

The form of the separation is not particularly limited, but it is preferable to use a known distillation device in order to efficiently recover the gas-phase component. As such a distillation device, various known methods are used, such as a method using a reactor including any one of a distillation column, a multi-stage distillation column, a multi-tube type reactor, a continuous multi-stage distillation column, a filled column, a thin film evaporator, a reactor having a support inside, a forced circulation reactor, a falling film evaporator, and a falling drop evaporator, and a method in which these reactors are combined. From the viewpoint of rapidly removing the aprotic solvent from the reaction system, a method using a distillation column, a multi-stage distillation column, a continuous multi-stage distillation column, a filled column, a tubular reactor, a thin film evaporator, a falling film evaporator, or a falling drop evaporator is preferable. In addition, since the aprotic solvent can be rapidly moved to the gas phase, a separation type having a structure with a large gas-liquid contact area is preferable. In addition, in a case where the multi-stage distillation column is a filled column, as a solid filling material included in the filled column, a filling material generally used in a distillation column or an absorption column, described in the step (2) above, can be appropriately used. Specific examples of a preferred solid filling material include a Raschig ring, a Cascade Mini Ring, a Mellapak, a Sulzer packing, and a Goodroll packing.

A material of the process equipment and the line may be any known material as long as it does not adversely affect the isocyanate or the like, but SUS304, SUS316, SUS316L, or the like can be preferably used because of the low cost.

In addition, in the step (3), the above-described distillation separation may be performed once, or may be performed a plurality of times. By performing the distillation separation a plurality of times, the aprotic solvent can be removed, and a fraction containing a more concentrated isocyanate compound can be obtained. From the viewpoint of balance between cost and production efficiency, the number of distillations can be 1 or more and 5 or less, preferably 1 or more and 4 or less, more preferably 2 or more and 3 or less, and still more preferably 2. In addition, in a case where the distillation is adopted in the step (3), it is also one of preferable aspects to control the conditions by appropriately refluxing as necessary.

<Step (4): isolation step of isocyanate compound (purification step)>

In the step (4), a component having a boiling point higher than that of the isocyanate compound (high-boiling-point component) is removed from the fraction containing the isocyanate compound obtained in the step (3) to purify the isocyanate compound.

The step (4) can also be referred to as an isolation step of the isocyanate compound or a purification step. The method for purifying the isocyanate compound is not particularly limited, but known distillation separation is preferable from the viewpoint of productivity and ease of process design.

From the viewpoint of suppressing side reactions, it is preferable that the distillation separation in the step (4) is carried out under as low temperature conditions as possible and for a short time to purify the isocyanate compound by removing the high-boiling-point component from the fraction containing the isocyanate compound. Therefore, it is preferable that the step (4) is also performed by a continuous method in the same manner as the thermal decomposition reaction in the step (2) described above.

The separation method by the continuous method is a method in which the fraction containing the isocyanate compound is continuously supplied to a reactor, and the isocyanate compound is continuously extracted from the reactor.

In addition, the fraction containing the isocyanate compound contains the high-boiling-point component having a boiling point higher than that of the isocyanate compound, and it is preferable to contain a carbonyl compound represented by General Formula (I) (hereinafter, may be referred to as "carbonyl compound (I)") in an amount of 1 ppm by mass or more and 50% by mass or less with respect to the mass of the fraction containing the isocyanate compound.

By purifying the isocyanate compound in the step (4) in the presence of the carbonyl compound (I) as the high-boiling-point component with the above-described specific amount, the carbonyl compound (I) acts as a solvent having a boiling point higher than that of the isocyanate compound (hereinafter, may be referred to as "high-boiling-point solvent"), and the isocyanate compound can be extracted from the reactor as a gas-phase component, and more efficiently isolated and purified. On the other hand, the high-boiling-point solvent remains at a bottom portion of the reactor as a liquid-phase component.

The content of the carbonyl compound (I) is preferably 1 ppm by mass or more and 50% by mass or less, more preferably 10 ppm by mass or more and 30% by mass or less, still more preferably 0.01% by mass or more and 15% by mass or less, and particularly preferably 1% by mass or more and 10% by mass or less with respect to the mass of the fraction containing the isocyanate compound. In a case where the content of the carbonyl compound (I) is equal to or more than the above-described lower limit value, the yield of the isocyanate compound can be further improved. On the other hand, in a case of being equal to or less than the above-described upper limit value, the amount of the reaction solution is further reduced, and the productivity can be improved and the equipment cost can be further reduced by reducing the size of the equipment in each step.

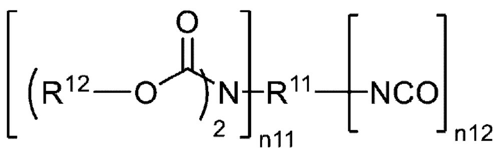

(I)

(in General Formula (1), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less)

The pressure in the step (4) varies depending on the kind of the compounds to be used and the reaction temperature, but may be any of decompression, normal pressure, or pressurization as long as the isocyanate compound and the high-boiling-point component can be separated. The pressure can be a pressure at which a saturated vapor pressure of the isocyanate compound is reached, and is preferably in a range of 20 Pa or more and $1 \times 10^6$ Pa or less.

The operation time (in the case of the continuous method, the retention time) in the step (4) is not particularly limited as long as the isocyanate compound and the high-boiling-point component can be separated, but it is preferably 5 seconds or longer and 100 hours or shorter, more preferably 10 seconds or longer and 50 hours or shorter, still more preferably 20 seconds or longer and 10 hours or shorter, even more preferably 20 seconds or longer and 1 hour or shorter, even still more preferably 20 seconds or longer and 30 minutes or shorter, particularly preferably 20 seconds or longer and 25 minutes or shorter, more particularly preferably 20 seconds or longer and 20 minutes or shorter, still more particularly preferably 30 seconds or longer and 15 minutes or shorter, and most preferably 30 seconds or longer and 10 minutes or shorter. In a case where the operation time is equal to or less than the above-described upper limit value, the operation time can be further shortened, and the side reactions of the isocyanate compound can be further suppressed. In addition, in a case where the operation time is equal to or more than is the above-described lower limit value, the isocyanate compound can be more sufficiently extracted into the gas phase.

The temperature in the step (4) is not particularly limited as long as the isocyanate compound and the high-boiling-point component are stable and the isocyanate compound and the high-boiling-point component can be separated, but it is preferable that the aprotic solvent is separated under as low temperature conditions as possible and for a short time because uretdione is produced by reacting the isocyanate compound at 100°C or higher and 130°C or lower.
From the viewpoint of suppressing the modification of the isocyanate compound, the temperature is preferably 250°C or lower, more preferably in a range of 20°C or higher and 240°C or lower, and still more preferably in a range of 30°C or higher and 230°C or lower.
The form of the isolation device is not particularly limited, but it is preferable to use a known distillation device in order to efficiently recover the gas-phase component. As such a distillation device, various known methods are used, such as a method using a reactor including any one of a distillation column, a multi-stage distillation column, a multi-tube type reactor, a continuous multi-stage distillation column, a filled column, a thin film evaporator, a reactor having a support inside, a forced circulation reactor, a falling film evaporator, and a falling drop evaporator, and a method in which these reactors are combined. From the viewpoint of rapidly removing the isocyanate compound from the reaction system, a method using a distillation column, a multi-stage distillation column, a tubular reactor, a continuous multi-stage distillation column, a filled column, a thin film evaporator, or a falling film evaporator is preferable; and a method using a distillation column, a multi-stage distillation column, a tubular thin film evaporator, a thin film evaporator, a tubular falling film evaporator, or the like is more preferable. In addition, since the isocyanate compound can be rapidly moved to the gas phase, a reactor having a structure with a large gas-liquid contact area is preferable.
A material of the isolation device and the line may be any known material as long as it does not adversely affect the isocyanate or the like, but SUS304, SUS316, SUS316L, or the like can be preferably used because of the low cost.
In addition, the order of the separation of the components in the step (3) and in the step (4) can be changed. That is, a step (3') of first separating the component having a boiling point higher than that of the isocyanate compound from the reaction solution containing the isocyanate compound obtained in the step (2) to recover a component containing the isocyanate compound and a step (4') of separating the isocyanate compound and the aprotic solvent from the component containing the isocyanate compound obtained in the step (3') to purify the isocyanate compound can be performed in this order. The step (3') is the same operation as the step (4), and the step (4') is the same operation as the step (3). In addition, in the step (4), it is also one of preferable aspects to control the conditions by appropriately refluxing as necessary.

<Step (5): reproduction step of primary amine compound and hydroxy compound>

It is preferable that he production method according to the present embodiment further includes the following step (5), in addition to the above-described steps (1) to (4):
a step (5) of hydrolyzing the fraction containing the aprotic solvent separated in the step (3) or the component having a boiling point higher than the boiling point of the isocyanate compound removed in the step (4) (high-boiling-point component) in the presence of an alkali and water to obtain the primary amine compound and the hydroxy compound.
In the fraction containing the aprotic solvent separated in the step (3), there is a case in which the high-boiling-point component is contained in a case where the aprotic solvent has a higher boiling point than the isocyanate compound.
The high-boiling-point component includes the above-described carbonyl compound (I), which is presumed to be produced by a reaction between the isocyanate compound and the aprotic solvent, or includes a polymer having one or more bonds selected from the group consisting of a biuret bond, an allophanate bond, an isocyanurate bond, a urea bond, a carbodiimide bond, a uretonimine bond, and an iminotrimer bond. The high-boiling-point component including these is hydrolyzed under alkaline conditions to obtain the primary amine compound and the hydroxy compound used in the step (1). Therefore, the step (5) can also be referred to as a regeneration step of the primary amine compound and the hydroxy compound.
The above-described hydroxy compound may be pre-mixed with the high-boiling-point component before the hydrolysis reaction. The hydroxy compound tends to have high solubility in the high-boiling-point component. Therefore, by dissolving the high-boiling-point component in the hydroxy compound in advance, the reaction system can be made uniform, and the reaction can be made to proceed rapidly.
In addition, by supplying and mixing the hydroxy compound in advance with the high-boiling-point component, it is possible to further suppress the modification of various compounds included in the high-boiling-point component and to maintain the liquid phase state.
In addition, in a case where the obtained primary amine compound and hydroxy compound are separated after the hydrolysis reaction, in general, in a state in which the primary amine compound and water are mixed, both may be in a state of being mixed, and there is a concern that phase separation may be difficult. However, in a case where the hydroxy compound is added in advance to the high-boiling-point component, the primary amine compound and water are easily separated from each other.
In addition, in a case where the primary amine compound and hydroxy compound are purified by distillation

separation after the hydrolysis reaction, there is a concern that the alkali used may precipitate in the distillation column and the distillation may not be significantly carried out. However, by adding the hydroxy compound in advance to the high-boiling-point component, the state of being dissolved in the alkali can be maintained, and the distillation separation can be continuously performed.

From the viewpoint of dissolving the high-boiling-point component and maintaining the liquid phase state, in a case where the high-boiling-point component collected as the liquid-phase component in the step (4) includes the hydroxy compound, the liquid-phase component may be used as it is in the hydrolysis reaction in the step (5). Alternatively, a pipe for supplying the hydroxy compound may be provided to a pipe for recovering the high-boiling-point component continuously extracted as a liquid-phase component from the reactor in the step (4), and the hydroxy compound may be added.

A content of the hydroxy compound is preferably 20% by mass or more and 300% by mass or less, and more preferably 50% by mass or more and 150% by mass or less with respect to the total mass of the fraction containing the aprotic solvent separated in the step (3) and the component having a boiling point higher than that of the isocyanate compound removed in the step (4). In a case where the content of the hydroxy compound is within the above-described range, the reaction system in the step (5) can be made uniform, and the reaction can be made to proceed rapidly.

In a case where the high-boiling-point component is transferred from the reactor in the step (4) to the reactor in the step (5), it is preferable to transfer the high-boiling-point component while holding the high-boiling-point component in a liquid state. Therefore, the high-boiling-point component is preferably supplied to the reactor in a state of being held at 50°C or higher and 300°C or lower, and more preferably at 100°C or higher and 260°C or lower in the step (5).

The use amount (molar amount) of water is preferably 1.0 times or more and 200 times or less, more preferably 5 times or more and 150 times or less, and still more preferably 5 times or more and 100 times or less in terms of a stoichiometric ratio with respect to the total molar amount of the isocyanate group, the biuret bond, the allophanate bond, the isocyanurate bond, the carbamate bond, and the urea bond, contained in the high-boiling-point component.

By setting the use amount of water to be equal to or less than the above-described upper limit value, it is possible to further suppress an undesirable reaction caused by an acidic atmosphere due to an increase in ion accumulation caused by a large amount of high-temperature and high-pressure water, for example, production of an imine compound which is a by-product obtained by a deammoniation reaction of the primary amine compound. In addition, energy load in the subsequent dewater step can be further reduced, and it is possible to suppress consumption energy in the production and to reduce the size of the device. On the other hand, in a case where the use amount of water is equal to or more than the above-described lower limit value, the decomposition reaction of the high-boiling-point component proceeds more favorably.

In addition, since hydrolyzability varies depending on the formulation of the bonds included in the high-boiling-point component, the use amount of water varies depending on the formulation of the decomposition target, but efficient decomposition can be carried out as long as the use amount is approximately within the above-described range.

Furthermore, in a case where the reaction can be efficiently carried out by using an alkali or the like, the amount of water can be further reduced. In a case where the decomposition efficiency is reduced, a crosslinking bond represented by a urea bond, which can be produced as an intermediate of the hydrolysis reaction, is increased, and a high-molecular-weight substance, that is, a gel may be generated in the system. The gel becomes a scale, adheres to the inside of the device, and significantly reduces the reaction efficiency, and thus it is required to clean the device, which is a factor that reduces the economic efficiency. However, by setting the use amount of water to be within the above-described range, the decomposition proceeds more favorably, and the generation of scale in the device can be further suppressed, and thus the primary amine compound can be efficiently recovered.

The alkali is not particularly limited, and examples thereof include hydroxides and oxides of alkali metals and alkaline earth metals, such as sodium hydroxide, potassium hydroxide, magnesium hydroxide, magnesium oxide, calcium oxide, and barium oxide; heterocyclic compounds such as pyridine, methylpyridine (including isomers), dipropylethylamine, N-methylmorpholine, N-ethylmorpholine, triethylamine, and triethylenediamine; and tertiary amines. These alkalis can be used alone or in combination of two or more kinds thereof.

Among these, as the alkali, hydroxides and oxides of alkali metals and alkaline earth metals are preferable, and hydroxides of alkali metals, such as sodium hydroxide and potassium hydroxide, are more preferable.

In addition, a shape of the alkali may be uniform or non-uniform with respect to the reaction solution. It is preferable to use a uniform alkali, and accordingly, the decomposition reaction in the reactor can be rapidly progressed, the generation of byproducts can be suppressed, and the yield can be further improved.

The use amount (molar amount) of the alkali can be 0.001 times or more and 10 times or less, preferably 0.01 times or more and 1 time or less, and more preferably 0.03 times or more and 0.5 times or less, in terms of a stoichiometric ratio, with respect to the molar amount of the isocyanate group, the biuret bond, the allophanate bond, the isocyanurate bond, the carbamate bond, the urea bond, the carbodiimide bond, the uretonimine bond, the iminotrimer bond, the free displaceable terminal, a cyclized product of the free displaceable terminal, and the oxycarbonylcarbamate bond, included in the high-boiling-point component.

In a case where the alkali is not used, the high-boiling-point component is not decomposed or decomposed slowly, and thus the yield of the primary amine compound is decreased. On the other hand, decomposability of the bonds included in the high-boiling-point component varies depending on the bonding mode, and the decomposition of each bonding mode can be promoted by appropriately selecting the type of alkali, the reaction form, and the like.

For example, the free displaceable terminal, the cyclized product of the free displaceable terminal, the isocyanurate bond, and the iminotrimer bond are generally less likely to undergo a decomposition reaction by water and less likely to generate the primary amine compound than the biuret bond, the allophanate bond, the carbamate bond, the oxycarbonyl carbamate bond, the urea bond, the carbodiimide bond, and the uretonimine bond. In particular, it is known that the isocyanurate bond and the iminotrimer bond have poor hydrolyzability, and that the progress of decomposition is slow by a known method. These bonds are cross-linking bonds, and in a case where the high-molecular-weight substance remains in the reaction solution, when the primary amine compound or the hydroxy compound is separated from the reaction solution, the device is clogged by precipitation or the amount of the primary amine compound which can be recovered is reduced.

On the other hand, in a case where the high-boiling-point component, the hydroxy compound, the water, and the alkali are used, the decomposition of the various bonds described above is promoted, and the high-molecular-weight substance is not left in the reaction solution. Therefore, when the primary amine compound or the hydroxy compound is separated from the reaction solution, the clogging in the device due to the precipitation or the like can be avoided, and thus the primary amine compound can be efficiently recovered.

The reaction temperature of the hydrolysis in the step (5) can be appropriately set according to the formulation of the high-boiling-point component to be decomposed, but in consideration of the energy load in the hydrolysis reaction, it is preferably 100°C or higher and 300°C or lower, more preferably 160°C or higher and 300°C or lower, and still more preferably 200°C or higher and 280°C or lower. In a temperature range where the reaction temperature is equal to or higher than the above-described upper limit value, the decomposition efficiency is almost saturated, and the environment of high-temperature and high-pressure water is a reaction field in which the ion accumulation increases and the acidity is improved. Therefore, an undesirable reaction may occur, and examples thereof include the production of an imine compound which is a by-product obtained by a deammoniation reaction of the primary amine compound. On the other hand, in the temperature range of equal to or lower than the above-described lower limit value, the decomposition efficiency is reduced, and thus a crosslinking bond represented by a urea bond, which can be produced as an intermediate of the hydrolysis reaction, is increased, and a high-molecular-weight substance, that is, a gel may be generated in the system. The gel becomes a scale, adheres to the inside of the device, and significantly reduces the reaction efficiency, and thus it is required to clean the device, which is a factor that reduces the economic efficiency.

However, in a case where the reaction temperature is within the above-described range, the decomposition of the crosslinking molecules represented by the urea bond proceeds more favorably, and the generation of scale in the device can be further suppressed. Therefore, the primary amine compound can be more efficiently recovered while maintaining continuous operability.

The reaction pressure can be 0.01 kPa or more and 10 MPa (absolute pressure) or less, and the reaction can be carried out under reduced pressure, under normal pressure, or under pressure. The reaction time (in the case of the continuous reaction, the retention time) can be set to 0.01 hours or longer and 100 hours or shorter, and the reaction can be terminated when the desired amount of the primary amine compound, which is the target compound, is produced by appropriately sampling the reaction solution and measuring the amount of the primary amine compound produced.

In the hydrolysis reaction in the step (5), there is a case in which a low-boiling-point component having a boiling point of 30°C or lower is generated, and the pressure of the reaction system is increased by the low-boiling-point component, or the reaction is delayed by the presence of the low-boiling-point component. Therefore, it is preferable to perform the hydrolysis reaction while extracting at least a part of the low-boiling-point component as a gas-phase component outside the reaction system. In this case, it is preferable to prevent water from being extracted to the outside of the reaction system together with the low-boiling-point component, for example, by providing a condenser in the middle of a line for extracting the low-boiling-point component from the reactor in which the hydrolysis reaction is performed. In addition, in a case where carbon dioxide is generated by the reaction with the water, for example, in a case where the isocyanate group, the biuret bond, the allophanate bond, the isocyanurate bond, the carbamate bond, the urea bond, the carbodiimide bond, the uretonimine bond, the iminotrimer bond, the free displaceable terminal, the cyclized product of the free displaceable terminal, or the oxycarbonyl carbamate bond is used, it is preferable to extract the generated carbon dioxide outside the reaction system.

The generated carbon dioxide forms a carbonate with the primary amine compound, which is generated by the hydrolysis reaction, and thus the hydrolysis reaction may be delayed. The ease of formation of the carbonate depends on nucleophilicity of the primary amine compound, and the order of carbonate formation is generally aliphatic amines ≥ substituted cyclic aliphatic polyamines > aromatic amines. Therefore, it is preferable to adjust the amount of carbon dioxide generated by the structure of the primary amine compound extracted from the reaction system as necessary.

The reactor for performing the hydrolysis reaction in the step (5) is not particularly limited, and a known reactor

can be used. For example, a reactor known in the related art can be appropriately combined and used according to the reaction method or conditions, such as a stirring tank, a pressurized stirring tank, a depressurized stirring tank, a tower-type reactor, a distillation column, a filled column, a thin film evaporator, a paddle dryer equipped with a forced transportation device, an extruder equipped with a degassing function, a vertical thin film evaporator equipped with a forced transportation device, and a tubular reactor. In addition, the reaction may be a batch type or a continuous flow type, and a reaction device may be selected according to each reaction type. A material of the reactor is not particularly limited, and a known material can be used. For example, a glass, stainless steel, carbon steel, Hastelloy material, a material on which a glass lining is applied, a material on which a Teflon (registered trademark) coating is applied, or the like can be used. SUS304, SUS316, SUS316L, and the like are inexpensive and can be preferably used. As necessary, known process equipment such as measuring instruments, for example, a flowmeter and a thermometer, a mechanism for holding pressure, a reboiler, a pump, and a condenser may be added. The heating may be performed by a known method such as steam or a heater, and the cooling may be performed by a known method such as natural cooling, cooling water, or brine.

The primary amine compound can be recovered from the reaction solution obtained by the hydrolysis reaction through a known separation method such as distillation separation, liquid-liquid phase separation, solid-liquid separation, and membrane separation. The recovered primary amine compound is preferably re-used as a raw material in the above-described step (1). In addition, it is also preferable to recover the hydroxy compound produced by the reaction between the high-boiling-point component and water and re-use the hydroxy compound as a raw material in the step (1).

The method for recovering the primary amine compound and the hydroxy compound preferably includes the following steps (5a) to (5d); and these steps can be continuously performed, or the steps (5a) to (5d) can be performed simultaneously. In addition, for example, operations of the steps (5c) and (5d) can be performed before operations of the steps (5a) and (5b), and some steps can be replaced and performed.

[Step (5a): separation of primary amine compound]

In the step (5a), the primary amine compound is separated from the reaction solution after the hydrolysis reaction. As a method for separating the primary amine compound, a known method can be used, and examples thereof include distillation separation, liquid-liquid phase separation, solid-liquid separation, and membrane separation. In the step (5a), the primary amine compound can also be continuously separated from the liquid-phase component (reaction solution) continuously discharged in the above-described step (5).

[Step (5b): purification of primary amine compound]

In the step (5b), the primary amine compound separated in the above-described step (5a) is purified. As a method for purifying the primary amine compound, a known method can be used, and examples thereof include distillation separation, liquid-liquid phase separation, solid-liquid separation, and membrane separation. In the step (5b), the continuously separated primary amine compound in the step (5a) can also be continuously purified.

[Step (5c): separation of hydroxy compound]

In the step (5c), the hydroxy compound which is a raw material in the above-described step (1) is separated from the reaction solution after the hydrolysis reaction. As a method for separating the hydroxy compound, a known method can be used, and examples thereof include distillation separation, liquid-liquid phase separation, solid-liquid separation, and membrane separation. In the step (5c), the hydroxy compound can also be continuously separated from the gas-phase component continuously discharged in the above-described step (5).

[Step (5d): purification of hydroxy compound]

In the step (5d), the hydroxy compound separated in the above-described step (5c) is purified. As a method for purifying the hydroxy compound, a known method can be used, and examples thereof include distillation separation, liquid-liquid phase separation, solid-liquid separation, and membrane separation. In the step (5d), the hydroxy compound continuously separated in the above-described step (5c) can also be continuously purified.

The primary amine compound and the hydroxy compound recovered by the above-described recovery method can be re-used in the above-described step (1).

That is, it is preferable that the production method according to the present embodiment further includes the following step (7):

a step (7) of re-using, in the step (1), the primary amine compound and the hydroxy compound recovered in the step (5).

[Step (7): re-using step of primary amine compound and hydroxy compound]

In the step (7), the primary amine compound and the hydroxy compound recovered in the step (5) are re-used in the step (1).

In this case, there is a concern that the production of the N-substituted carbamate compound in the step (1), the quality of the obtained N-substituted carbamate compound, and the quality of the isocyanate compound obtained in the subsequent step may be affected. Therefore, in the above-described step (5) (specifically, the above-described step (5b) and the above-described step (5d)), it is preferable to distill and recover the primary amine compound and the hydroxy compound such that the content of the metal component is 1,000 ppm by mass or less and the content of the halogen atom is 1,000 ppm by mass or less with respect to the total mass of the primary amine compound.

In addition, the hydroxy compound can also be re-used for mixing with the high-boiling-point component in advance in the above-described step (5).

<Other steps>

The production method according to the present embodiment can further include other steps, in addition to the above-described steps (1) to (5).

[Step (1a): concentration step of N-substituted carbamate compound]

In a step (1a), the N-substituted carbamate compound obtained in the step (1) is concentrated. That is, the step (1a) can also be referred to as a concentration step of the N-substituted carbamate compound.

The method for concentrating the N-substituted carbamate compound is not particularly limited, but from the viewpoint of productivity and ease of process design, a known distillation separation is preferable.

From the viewpoint of suppressing side reactions, it is preferable that the distillation separation in the step (1a) is carried out under as low temperature conditions as possible and for a short time to concentrate the N-substituted carbamate compound by removing unreacted raw material components such as the hydroxy compound from the reaction solution containing the N-substituted carbamate compound. Therefore, it is preferable that the step (1a) is also performed by a continuous method in the same manner as the reaction in the step (1) described above.

The concentration method by the continuous method is a method in which the reaction solution containing the N-substituted carbamate compound is continuously supplied to a reactor, and the concentrated N-substituted carbamate compound is continuously extracted from the reactor. A supply rate of the above-described reaction solution to the reactor and an extraction rate of the N-substituted carbamate compound can be appropriately adjusted according to a degree of concentration of the N-substituted carbamate compound.

In addition, in the step (1a), the aprotic solvent used in the subsequent step (1) may be pre-mixed and then concentrated as described above. As the aprotic solvent used in this case, it is preferable to use a solvent having a higher boiling point than the hydroxy compound. As a result, liquid drying due to concentration can be further prevented.

[Step (6): solvent recovery step (solvent re-using step)]

In the step (6), the hydroxy compound extracted in the step (2) is re-used by circulating the hydroxy compound to the step (1), and the aprotic solvent separated in the step (3) is re-used by circulating the aprotic solvent to the step (2). That is, the step (6) can be referred to as a solvent recovery step or a solvent re-using step.

The hydroxy compound extracted in the step (2) and the aprotic solvent separated in the step (3) may be separately separated and purified, and each of the hydroxy compound and the aprotic solvent may be recovered. Alternatively, the components extracted in these steps may be collected together and mixed, and separated and purified to recover the hydroxy compound and the aprotic solvent.

As a method for separating and purifying the hydroxy compound and the aprotic solvent, a known method can be used, and examples thereof include distillation separation, liquid-liquid phase separation, solid-liquid separation, and membrane separation. As the distillation separation method, for example, a method including any of a multi-stage distillation column, a tray column, a filled column, a thin film evaporator, a falling film evaporator, a falling drop evaporator, or a falling film-type evaporator is used. In the step (6), each of the hydroxy compound and the aprotic solvent can also be continuously separated from the gas-phase component continuously discharged in the above-described step (2) and the gas-phase component continuously discharged in (3). In addition, it is also one of preferable aspects to control the conditions by appropriately refluxing as necessary.

In addition, in a case where distillation separation is adopted for the separation and purification after the components extracted in each step are collected and mixed, it is preferable to recover the hydroxy compound from the top of the column and recover the aprotic solvent as a side-cut fraction from the middle of the column due to the difference

in boiling point.

In addition, the recovered hydroxy compound can be re-used for mixing with the high-boiling-point component in advance in the step (5), and can be re-used as a raw material of the step (1).

<Raw materials and products>

Hereinafter, the raw materials and products (including intermediate products) used in the method for producing an isocyanate compound according to the present embodiment will be described in detail.

[Primary amine compound]

As the primary amine compound, for example, a compound represented by General Formula (II) (hereinafter, may be referred to as "primary amine compound (II)") or the like is an exemplary example.

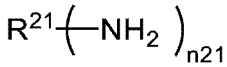 (II)

(in General Formula (II), $R^{21}$ is an n21-valent organic group, and n21 is an integer of 2 or more)

($R^{21}$)

$R^{21}$ is an n21-valent organic group, and is preferably an organic group having 3 or more and 85 or less carbon atoms and more preferably an organic group having 3 or more and 30 or less carbon atoms.

The organic group as $R^{21}$ is an aliphatic hydrocarbon group, an aromatic hydrocarbon group, or a group obtained by bonding an aliphatic hydrocarbon group and an aromatic hydrocarbon group. Specific examples of $R^{21}$ include a cyclic hydrocarbon group, an acyclic hydrocarbon group, a group in which an acyclic hydrocarbon group and one or more cyclic groups are bonded, and a group in which these groups are covalently bonded to a specific non-metal atom. Examples of the above-described cyclic group include a cyclic hydrocarbon group, a heterocyclic group, a heterocyclic spiro group, and a hetero-crosslinked ring group. Examples of the above-described cyclic hydrocarbon group include a monocyclic hydrocarbon group, a condensed polycyclic hydrocarbon group, a crosslinked cyclic hydrocarbon group, a spiro hydrocarbon group, a ring-assembled hydrocarbon group, and a cyclic hydrocarbon group having a side chain. Examples of the above-described non-metal atom include carbon, oxygen, nitrogen, sulfur, and silicon.

The phrase "covalently bonded to a specific non-metal atom" means that, for example, the above-described group is in a state of being covalently bonded to a group represented by Formulae (II)-1a to (II)-1p.

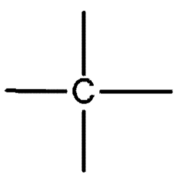
(II)-1a

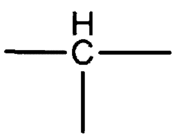
(II)-1b

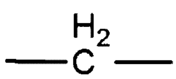
(II)-1c

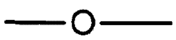
(II)-1d

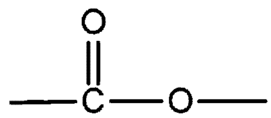
(II)-1e

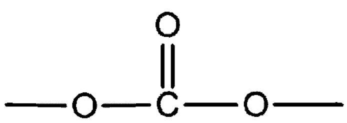
(II)-1f

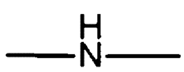
(II)-1g

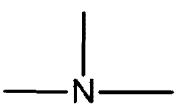
(II)-1h

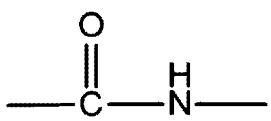
(II)-1j

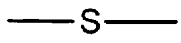 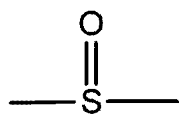 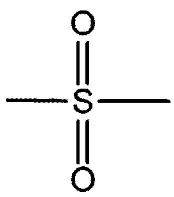 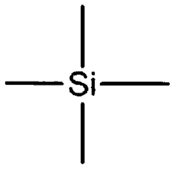

(II)-1k (II)-1m (II)-1n (II)-1p

Among these, as the organic group in $R^{21}$, a cyclic hydrocarbon group, an acyclic hydrocarbon group, a group in which an acyclic hydrocarbon group and one or more cyclic groups are bonded, or a group in which these groups are covalently bonded to the group represented by Formula (II)-1a, (11)-1b, (II)-1c, (II)-1e, or (II)-1j is preferable.

In a case where $R^{21}$ is an aliphatic hydrocarbon group, specific examples of the primary amine compound (II) include aliphatic diamines, aliphatic triamines, and substituted cyclic aliphatic polyamines.

Examples of the aliphatic diamines include ethylenediamine, diaminopropane (each isomer), diaminobutane (each isomer), diaminopentane (each isomer), diaminohexane (each isomer), and diaminodecane (each isomer).

Examples of the aliphatic triamines include triaminohexane (each isomer), triaminononane (each isomer), triaminodecane (each isomer), triaminoundecane (each isomer), and 4-aminomethyl-1,8-octanediannine.

Examples of the substituted cyclic aliphatic polyamines include diaminocyclobutane (each isomer), diaminocyclohexane (each isomer), 3-aminomethyl-3,5,5-trimethylcyclohexylamine (at least one isomer of a cis form and a trans form), and methylenebis(cyclohexylamine) (each isomer).

In a case where $R^{21}$ is an aromatic group, specific examples of the primary amine compound (II) include aromatic diamines and aromatic triamines.

Examples of the aromatic diamines include diaminobenzene (each isomer), diaminotoluene (each isomer), methylenedianiline (each isomer), diaminomethylidene (each isomer), diaminobiphenyl (each isomer), diaminodibenzyl (each isomer), bis (aminophenyl) propane (each isomer), bis (aminophenyl) ether (each isomer), bis (aminophenoxyethane) (each isomer), diaminoxylene (each isomer), diaminosol (each isomer), diaminophenol (each isomer), diaminonaphthalene (each isomer), diaminomethylbenzene (each isomer), diaminomethylpyridine (each isomer), diaminomethylnaphthalene (each isomer), diaminodiphenylmethane (each isomer), and tetramethylxylylenediamine (each isomer).

Examples of the aromatic triamines include triaminobenzene (each isomer), triaminomethylbenzene (each isomer), tris(aminopropane-yl)benzene (each isomer), tris(aminopropane-yl)-methylbenzene (each isomer), tris(aminomethyl)-methylbenzene (each isomer), and ((aminophenylene)bis(methylene))bis(aminobenzene) (each isomer).

In a case where the aliphatic hydrocarbon group or the aromatic group as $R^{21}$ has 1 or more to 4 ester groups, as the primary amine compound (II), specifically, for example, an amine compound having an ester group obtained by reacting a carboxy group of amino acid with a hydroxy compound is preferable. Specific examples of the amine compound having such an ester group include acrylic acid-2-aminoethyl ester, 2-methyl-acrylic acid-2-aminoethyl ester, acrylic acid-2-aminopropyl ester, 2-methyl-acrylic acid-2-aminopropyl ester, acrylic acid-3-aminopropyl ester, 2-methyl-acrylic acid-3-aminopropyl ester, acrylic acid-4-aminobutyl ester, 2-methyl-acrylic acid-4-aminobutyl ester, acrylic acid-5-aminopentyl ester, 2-methyl-acrylic acid-5-aminopentyl ester, acrylic acid-6-aminohexyl ester, 2-methyl-acrylic acid-6-aminohexyl ester, acrylic acid-8-aminooctyl ester, 2-methyl-acrylic acid-8-aminooctyl ester, acrylic acid-10-aminodecyl ester, 2-methyl-acrylic acid-10-aminodecyl ester, acrylic acid-11-aminoundecyl ester, 2-methyl-acrylic acid-11-aminoundecyl ester, acrylic acid-12-aminododecyl ester, 2-methyl-acrylic acid-12-aminododecyl ester, lysine methylesterdiamine, lysine ethylesterdiamine, 2-aminoethyl-2,5-diaminopentanoate, 2-aminoethyl-2,6-diaminohexanoate, bis(2-aminoethyl)-2-aminobutanedioate, bis(2-aminoethyl)-2-aminopentanedioate, and tris(2-aminoethyl)hexane-1,3,6-tricarboxylate.

In a case where the aliphatic hydrocarbon group or the aromatic group as $R^{21}$ has 1 or more and 4 or less nitrogen atoms, as the primary amine compound (II), specifically, for example, an amine compound having 1 or more and 4 or less secondary or tertiary amines other than the terminal of the aliphatic hydrocarbon group or the aromatic group. Specific examples of the amine compound having 1 or more and 4 or less secondary or tertiary amines other than the terminal of the aliphatic hydrocarbon group or the aromatic group include 2-(dimethylamino)ethylamine, 2-(diethylamino)ethylamine, 2-(diisopropylamino)ethylamine, 2-(cyclohexylamino)ethylamine, 3-(cyclohexylamino)propylamine, 3-(diethylamino)propylamine, 3-(dimethylamino)propylamine, diethylenetriamine, diisopropyltriamine, bis-(3-aminopropyl)methyleneamine, 3-(2-aminoethylamino)propylamine, N,N'-bis(3-aminopropyl)ethylenediamine, 1-(3-aminopropyl)imidazole, trisaminoethylamine, and trisaminopropylamine.

(n21)

n21 represents the number of amino groups, is an integer of 2 or more, preferably an integer of 2 or more and 10 or less, more preferably an integer of 3 or more, and still more preferably 3.

Among these, the primary amine compound (II) is preferably diaminohexane (each isomer), 4-aminomethyl-1,8-octanediamine, triaminohexane (each isomer), triaminoundecane (each isomer), 3-aminomethyl-3,5,5-trimethylcyclohexylamine, methylenebis(cyclohexylamine) (each isomer), diaminotolylene (each isomer), methylenedianiline (each isomer), triaminobenzene (each isomer), triaminomethylbenzene (each isomer), or tris(aminopropyl)-methylbenzene (each isomer).

As the primary amine compound (II), an amino acid derivative is also preferably used.

The amino acid derivative referred to herein means a compound synthesized using an amino acid as a raw material. As will be described later, the amino acid may be a natural amino acid or a synthetic amino acid. In addition, the derivative may be a derivative alone or in a form of an inorganic acid salt.

As a preferred amino acid derivative, for example, a compound represented by General Formula (II-1) or (II-2) (hereinafter, each of which may be abbreviated as "amino acid derivative (II-1)" or the like).

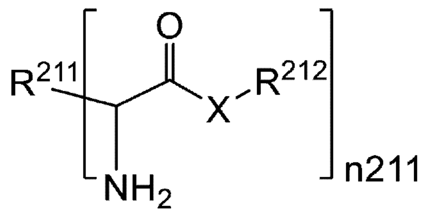

( II-1 )

In General Formula (II-1), $R^{211}$ is an n211-valent organic group, and is the same as $R^{21}$ described above. Among these, $R^{211}$ is preferably a structure obtained by removing a -NHCOOH group from an amino acid; more preferably an aliphatic hydrocarbon group having 1 or more carbon atoms or an aromatic group having 6 or more carbon atoms, each of which may include a primary amino group, a sulfur atom, an oxygen atom, or a halogen atom; and still more preferably a divalent or trivalent aliphatic hydrocarbon group having 1 or more and 15 or less carbon atoms or a divalent or trivalent aromatic group having 6 or more and 15 or less carbon atoms.

In General Formula (II-1), $X^{211}$ is an oxygen atom or a secondary amino group (-NH-). Among these, $X^{211}$ is preferably an oxygen atom.

In General Formula (II-1), $R^{212}$ is a monovalent aliphatic hydrocarbon group having 1 or more and 15 or less carbon atoms, an aromatic group having 6 or more and 15 or less carbon atoms, or a hydrogen atom. Among them, $R^{212}$ is preferably an alkyl group having 1 or more and 6 or less carbon atoms, and more preferably an alkyl group having 1 or more and 3 or less carbon atoms.

n211 is an integer of 2 or more, and preferably 2 or 3.

In an $\alpha$-amino acid, the bonding mode of the amino group or the carboxy group to the $\alpha$-carbon can be stereospecifically two types, and each of them is distinguished as a D-type or L-type optical isomer. The amino acid derivative may be a D-type, an L-type, or a mixture or a racemate thereof. Many amino acids which can be industrially and inexpensively obtained are amino acids produced by fermentation, and most of them are L-type amino acids, which can be preferably used. In the present specification, although the three-dimensional arrangement is not shown, either the D type or the L type is shown.

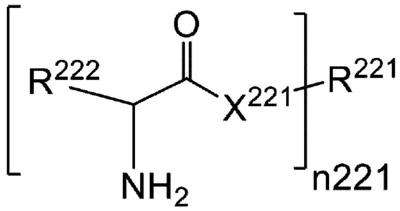

( II-2 )

In General Formula (II-2), $R^{221}$ is an n221-valent organic group or a hydrogen atom, and in a case of the n221-valent organic group, $R^{221}$ is the same as $R^{21}$ described above. Among the above, $R^{221}$ is preferably a monovalent to tetravalent aliphatic hydrocarbon group having 1 or more and 15 or less carbon atoms, or a monovalent to tetravalent aromatic group having 6 or more and 15 or less carbon atoms.

In General Formula (II-2), $X^{221}$ is an oxygen atom or a secondary amino group (-NH-), and an oxygen atom is preferable.

In General Formula (II-2), n221 is an integer of 1 or more and 4 or less.

In General Formula (II-2), $R^{222}$ is a monovalent aliphatic hydrocarbon group, a monovalent aromatic group, or a hydrogen atom. Among these, $R^{222}$ is preferably a monovalent aliphatic hydrocarbon group having 1 or more carbon atoms, a monovalent aromatic group having 6 or more carbon atoms, each of which may include one or more selected from the group consisting of a primary amino group, a sulfur atom, an oxygen atom, and a halogen atom, or a hydrogen atom.

In addition, a monovalent aliphatic hydrocarbon group having 1 or more and 15 or less carbon atoms or a monovalent aromatic group having 6 or more and 15 or less carbon atoms, each of which may include one or more selected from the group consisting of a primary amino group, a sulfide bond, an ether bond, and a disulfide bond; a group having 7 or more and 15 or less carbon atoms, in which an aliphatic hydrocarbon group and an aromatic group are bonded, each of which may include one or more selected from the group consisting of a primary amino group, a sulfide bond, an ether bond, and a disulfide bond; a group represented by General Formula (II-2a) or (II-2b), or a hydrogen atom is more preferable.

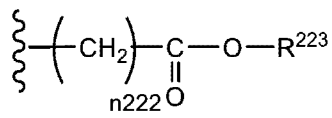

( II-2a )

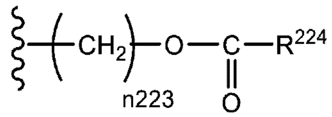

( II-2b )

In General Formula (II-2a), $R^{221}$ is a group represented by General Formula (II-2c), (11-2d), or (II-2e), or a hydrocarbon group having 1 or more and 10 or less carbon atoms.

In General Formula (II-2a), n222 is an integer of 0 or more and 5 or less.

In General Formula (II-2a), a wavy line represents a bonding site.

In General Formula (II-2b), $R^{224}$ is an aliphatic hydrocarbon group having 1 or more and 15 or less carbon atoms or an aromatic group having 6 or more and 15 or less carbon atoms.

In General Formula (II-2b), n223 is an integer of 0 or more and 5 or less.

In General Formulae (II-2b), a wavy line represents a bonding site.

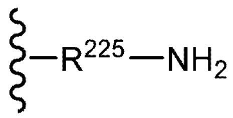

( II-2c )

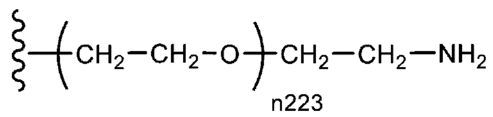

( II-2d )

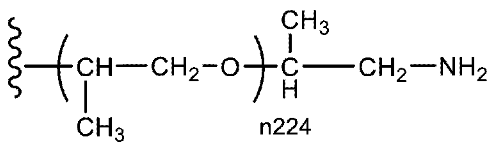

( II-2e )

In General Formula (II-2c), $R^{225}$ is a divalent aliphatic hydrocarbon group having 1 or more and 10 or less carbon atoms, and a linear or branched alkylene group having 1 or more and 6 or less carbon atoms is preferable.

In General Formula (II-2d), n223 is an integer of 1 or more and 9 or less.

In General Formula (II-2e), n224 is an integer of 0 or more and 9 or less.

In General Formulae (II-2c) to (II-2e), wavy lines represent a bonding site.

As the amino acid derivative, an amino acid ester is preferable. Therefore, preferred examples of amino acid derivatives (II-1) and (II-2) are as follows.

Specific examples of the preferred amino acid derivative (II-1) include compounds represented by General Formulae (II-1-1) to (II-1-3).

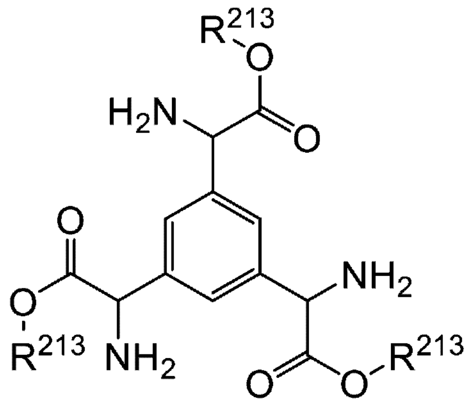

( II-1-1 )

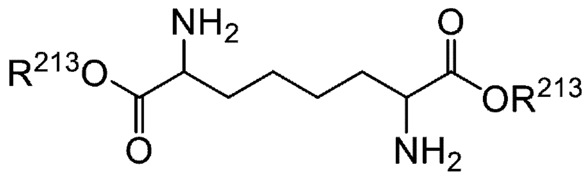

( II-1-2 )

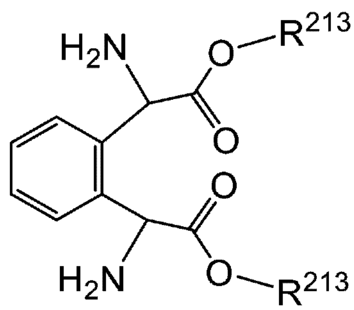

( II-1-3 )

In General Formulae (II-1-1) to (II-1-3), $R^{213}$ is the same as $R^{212}$ described above. Among them, $R^{213}$ is preferably an alkyl group having 1 or more and 6 or less carbon atoms, and more preferably an alkyl group having 1 or more and 3 or less carbon atoms.

Examples of the preferred amino acid derivative (II-2) include compounds represented by General Formulae (II-2-1) to (II-2-4).

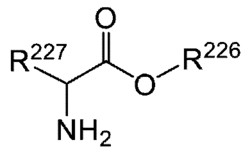

( II-2-1 )

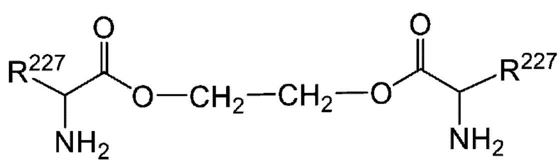

( II-2-2 )

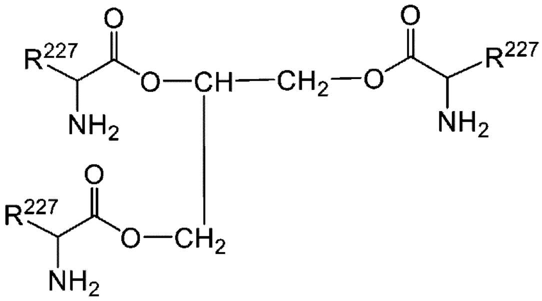

( II-2-3 )

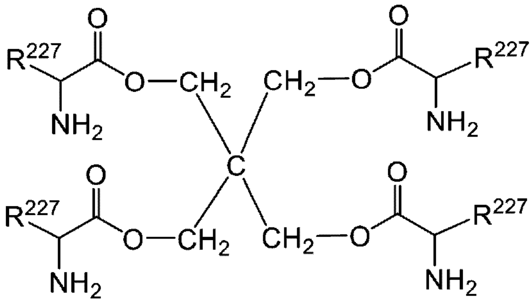

( II-2-4 )

In General Formulae (II-2-1) to (II-2-4), $R^{226}$ is a monovalent aliphatic hydrocarbon group having 1 or more and 15 or less carbon atoms, a monovalent aromatic group having 6 or more and 15 or less carbon atoms, or a hydrogen atom.

In General Formulae (II-2-1) to (II-2-4), $R^{227}$ is the same as $R^{222}$.

As the amino acid derivative, a compound represented by General Formula (II-3) (hereinafter, may be referred to as "amino acid derivative (11-3)") is also preferably used.

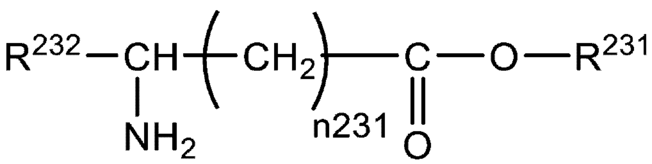

( II-3 )

In General Formula (II-3), n231 is 1 or 2.

In General Formula (II-3), $R^{231}$ is an aliphatic hydrocarbon group having 1 or more and 15 or less carbon atoms or an aromatic group having 6 or more and 15 or less carbon atoms, each of which may include an ether bond or a sulfide bond, a group having 7 or more and 15 or less carbon atoms, in which an aliphatic hydrocarbon group and an aromatic group are bonded, each of which may include an ether bond or a sulfide bond, or a group represented by General Formula (II-2c), (II-2d), or (II-2e).

In General Formula (II-3), $R^{232}$ is a monovalent aliphatic hydrocarbon group having 1 or more and 15 or less carbon atoms or a monovalent aromatic group having 6 or more and 15 or less carbon atoms, each of which may include one or more selected from the group consisting of a primary amino group, a sulfide bond, an ether bond, and a disulfide bond; a group having 7 or more and 15 or less carbon atoms, in which an aliphatic hydrocarbon group and an aromatic group are bonded, each of which may include one or more selected from the group consisting of a primary amino group, a sulfide bond, an ether bond, and a disulfide bond; a group represented by General Formula (II-2a) or (II-2b), or a hydrogen atom.

Examples of the preferred amino acid derivative (11-3) include compounds represented by General Formulae (II-3-1) to (II-3-39).

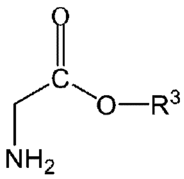

( II-3-1 )

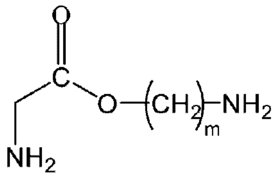

( II-3-2 )

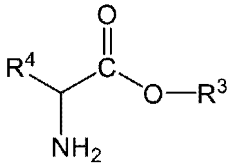

( II-3-3 )

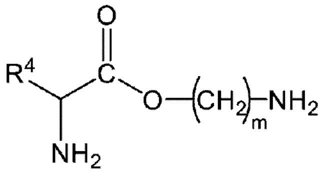

( II-3-4 )

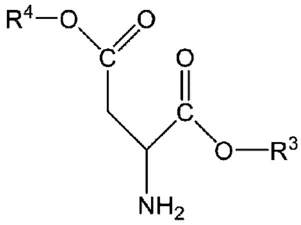

( II-3-5 )

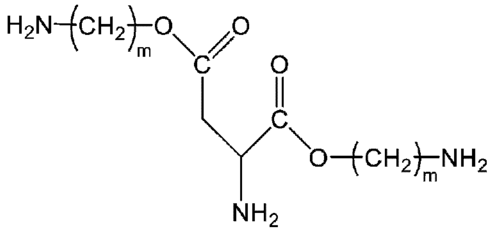

( II-3-6 )

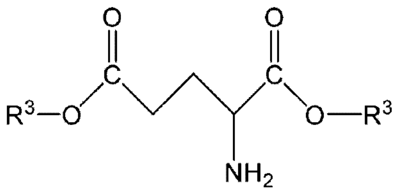

( II-3-7)

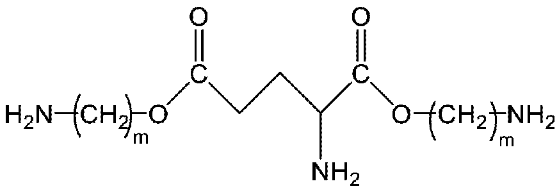

( II-3-8 )

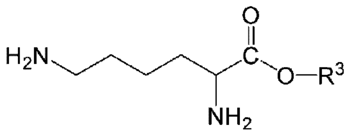

(II-3-9)

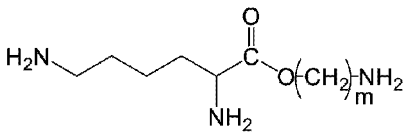

(II-3-10)

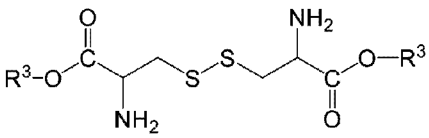

(II-3-11)

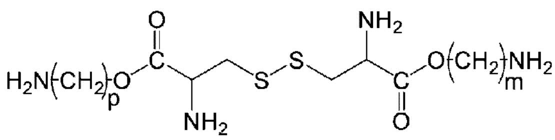

(II-3-12)

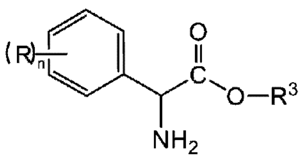

( II-3-13 )

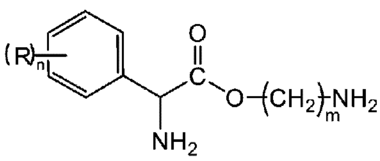

( II-3-14 )

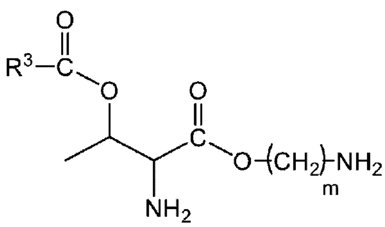

( II-3-15 )

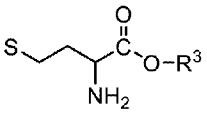
( II-3-16 )

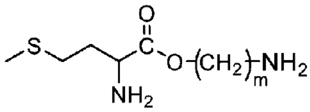
( II-3-17 )

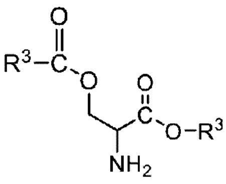
( II-3-18 )

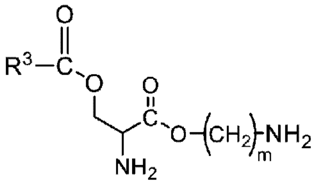
( II-3-19 )

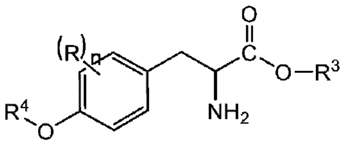
( II-3-20 )

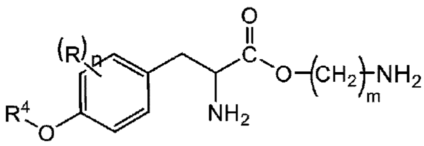
( II-3-21 )

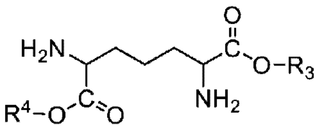
( II-3-22 )

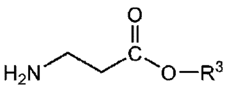
( II-3-23 )

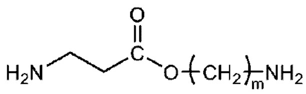
( II-3-24 )

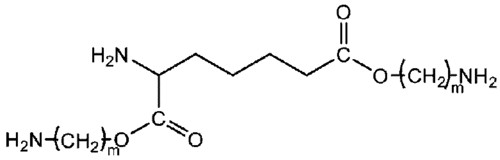
( II-3-25 )

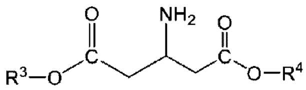
( II-3-26 )

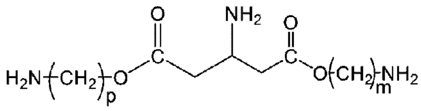
( II-3-27 )

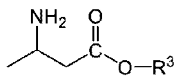
( II-3-28 )

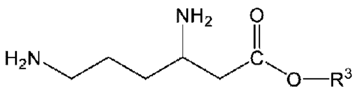
( II-3-29 )

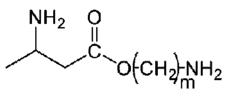
( II-3-30 )

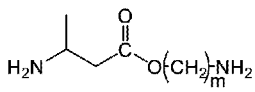
( II-3-31 )

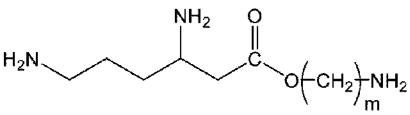
( II-3-32 )

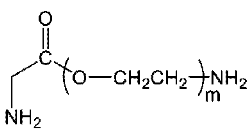
( II-3-33 )

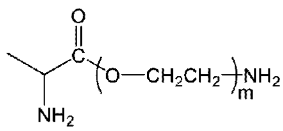
( II-3-34 )

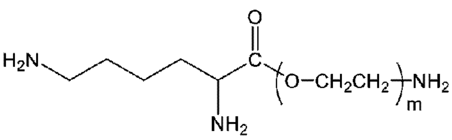
( II-3-35 )

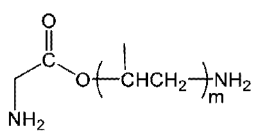
( II-3-36 )

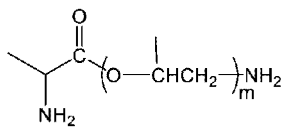
( II-3-37 )

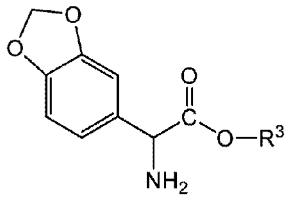

( II-3-38 )

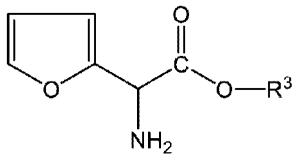

( II-3-39 )

In General Formulae (II-3-1) to (II-3-39), $R^3$ and $R^4$ are each independently an aliphatic hydrocarbon group having 1 or more and 10 or less carbon atoms or a hydrogen atom. Among these, $R^3$ is preferably an alkyl group having 1 or more and 4 or less carbon atoms. $R^4$ is preferably an alkyl group having 1 or more and 6 or less carbon atoms.

In General Formulae (II-3-1) to (II-3-39), R is a halogen atom, an alkyl group having 1 or more and 6 or less carbon atoms, or an alkoxy group having 1 or more and 6 or less carbon atoms. Among these, R is preferably a halogen atom, an alkyl group having 1 or more and 4 or less carbon atoms, or an alkoxy group having 1 or more and 4 or less carbon atoms.

In General Formulae (II-3-1) to (II-3-39), n is an integer of 0 or more and 2 or less. Among these, n is preferably 1 or 2.

In General Formulae (II-3-1) to (11-3-39), m and p are each independently an integer of 1 or more and 10 or less, preferably an integer of 1 or more and 6 or less, and more preferably an integer of 1 or more and 3 or less.

Among these, as the amino acid derivative (II-3), the compounds represented by General Formula (II-3-3), (II-3-4), (II-3-13), (II-3-14), (II-3-38), or (II-3-39) are preferable.

Among these, as the amino acid derivative (11-3), an amino acid ester derived from a lysine skeleton such as lysine methyl ester, lysine ethyl ester, and lysine β-aminoethyl ester; an amino acid ester derived from a glutamic acid skeleton such as glutamic acid methyl ester and glutamic acid di(β-aminoethyl) ester; an amino acid ester derived from a methionine skeleton such as methionine methyl ester; an amino acid ester derived from a glycine skeleton such as glycine methyl ester; an amino acid ester derived from a phenylalanine skeleton such as phenylalanine methyl ester; an amino acid ester derived from an aspartic acid skeleton such as aspartic acid methyl ester; an amino acid ester derived from an alanine skeleton such as alanine methyl ester; an amino acid ester derived from a leucine skeleton such as leucine methyl ester; an amino acid ester derived from an isoleucine skeleton such as isoleucine methyl ester; or an amino acid ester derived from a valine skeleton such as valine methyl ester is particularly preferable.

The above-described amino acid ester can be produced, for example, by reacting an amino acid with a compound having an alcoholic hydroxy group in the presence of an inorganic acid, or by reacting an amino acid inorganic acid salt with an amino alcohol inorganic acid salt in the presence of an inorganic acid.

The inorganic acid may be any inorganic acid such as hydrochloric acid, nitric acid, phosphoric acid, sulfuric acid, boric acid, and hydrofluoric acid; and is preferably sulfuric acid, phosphoric acid, or hydrochloric acid and more preferably hydrochloric acid.

The amino acid ester may be used in a form of an inorganic acid salt. The amino acid ester inorganic acid salt is formed by the above-described inorganic acid, and is preferably an amino acid ester sulfate, an amino acid ester phosphate, or an amino acid ester hydrochloride, and more preferably an amino acid ester hydrochloride.

The amino acid is preferably an aliphatic or aromatic amino acid having 2 or more and 18 or less carbon atoms, which has at least one amino group and at least one carboxy group, or a lactam having a 3-membered or more and a 12-membered or less ring.

The amino acid may be a natural amino acid or a synthetic amino acid.

Examples of the natural amino acid include alanine, arginine, asparagine, glutamine, glutamic acid, glycine, histidine, isoleucine, leucine, lysine, aspartic acid, methionine, phenylalanine, tryptophan, valine, and ornithine.

The synthetic amino acid can be produced by a known method, and can be produced by, for example, a Strecker synthesis using an aldehyde compound. Examples of the aldehyde include a compound represented by General Formula (A) (hereinafter, may be abbreviated as "compound (A)").

$$R^a\left(CHO\right)_a \qquad (A)$$

In General Formula (A), $R^a$ is a monovalent aliphatic hydrocarbon group having 1 or more carbon atoms or an aromatic group having 6 or more carbon atoms, each of which may include an oxygen atom or a halogen atom. Among these, $R^a$ is preferably a monovalent aliphatic hydrocarbon group having 1 or more and 12 or less carbon atoms or an

aromatic group having 6 or more carbon atoms.

In General Formula (A), a is an integer of 1 or more and 3 or less.

Examples of the preferred compound (A) include acetaldehyde, propionaldehyde, hexylaldehyde, octylaldehyde, caprylaldehyde, phenylacetaldehyde, benzaldehyde, dimethoxybenzaldehyde, chlorobenzaldehyde, fluorobenzaldehyde, heliotropin, cyclamen benzaldehyde, furfural, naphthaldehyde, and phthalaldehyde. In a case where these compounds have an isomer structure, the isomer is also included.

The amino acid inorganic acid salt is an inorganic acid salt of the above-described amino acid.

The amino acid particularly preferably used is an aliphatic monoaminomonocarboxylic acid, a diaminomonocarboxylic acid, a monoaminodicarboxylic acid, a diaminodicarboxylic acid, or the like. A lactam formed by cyclization of these amino acids is also preferably used.

Specific examples of the above-described compound include glycine, 3-aminopropionic acid, ω-aminocaproic acid, ω-aminolauric acid, alanine, isoleucine, 3-aminobutyric acid, 4-aminocyclohexanecarboxylic acid, phenylalanine, methionine, aminobenzoic acid, aspartic acid, glutamic acid, lysine, rancitonic acid, 1-amino-2,3,4-butanetricarboxylic acid, and lactam, pyrrolidone, caprolactam, laurolactam, or the like of the above-described amino acids.

The above-described compound containing the alcoholic hydroxy group is preferably an aminoalcohol inorganic acid salt.

The aminoalcohol inorganic acid salt can be produced by reacting the above-described amino acid inorganic acid salt with an alcohol.

The aminoalcohol inorganic acid salt is an inorganic acid salt of an aminoalcohol having 2 or more and 12 or less carbon atoms, which has one primary or secondary hydroxyl group and one primary amino group. The amino alcohol may include an element atom such as oxygen or sulfur in the alkylene chain thereof, or may include a substituent such as a nitro group, a halogen group, an alkyl group, or a phenyl group, which is inert to the esterification reaction.

Specific examples of the amino alcohol include ethanolamine, 1 -amino-2-propanol, 2-amino-1-propanol, 2-aminoisobutanol, 2-amino-1-butanol, 2-(2-aminoethoxy)-ethanol, and 2-aminocyclohexanol.

As the alcohol, a known alcohol can be used, and a monohydric alcohol having 1 or more and 10 or less carbon atoms is preferable.

Specific examples thereof include methanol, ethanol, propanol, butanol, pentanol, hexanol, octanol, decanol, cyclopentanol, and cyclohexanol. In a case where these compounds include an isomer, the isomer can also be used.

[Hydroxy compound]

Examples of the hydroxy compound include alcohols and aromatic hydroxy compounds.

Examples of the alcohols include methyl alcohol, propyl alcohol, butyl alcohol, amyl alcohol, hexyl alcohol, heptyl alcohol, octyl alcohol, nonyl alcohol, decyl alcohol, undecyl alcohol, lauryl alcohol, dodecyl alcohol, stearyl alcohol, eicosyl alcohol, allyl alcohol, crotyl alcohol, propargyl alcohol, cyclopentanol, cyclohexanol, benzyl alcohol, cinnamyl alcohol, ethylene glycol, diethylene glycol, polyethylene glycol, propylene glycol, polypropylene glycol, 1,3-butanediol, 1,4-butanediol, hydrogenated bisphenol A, neopentyl glycol, glycerin, trimethylolpropane, pentaerythritol, ethanolamine, propanolamine (1-amino-2-propanol), dimethanolamine, diethanolamine, dipropanolamine, and 1-amino-2-butanol.

Specific examples of the aromatic hydroxy compounds include a compound represented by General Formula (VI) (hereinafter, may be referred to as "aromatic hydroxy compound (VI)").

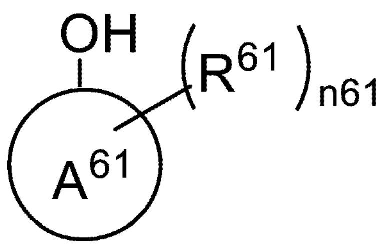

(VI)

(in General Formula (VI), a ring $A^{61}$ is an aromatic hydrocarbon ring having 6 or more and 20 or less carbon atoms, $R^{61}$ is a hydrogen atom, an alkyl group having 1 or more and 20 or less carbon atoms, an alkoxy group having 1 or more and 20 or less carbon atoms, an aryl group having 6 or more and 20 or less carbon atoms, an aryloxy group having 6 or more and 20 or less carbon atoms, an aralkyl group having 7 or more and 20 or less carbon atoms, an aralkyloxy group having 7 or more and 20 or less carbon atoms, or a hydroxy group, $R^{61}$ may be bonded to the ring $A^{61}$ to form a ring structure, and n61 is an integer of 1 or more and 10 or less)

[$R^{61}$]

Examples of the alkyl group having 1 or more and 20 or less carbon atoms, as $R^{61}$, include a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), a dodecyl group (each isomer), and an octadecyl group (each isomer).

Examples of the alkoxy group having 1 or more and 20 or less carbon atoms, as $R^{61}$, include a methoxy group, an ethoxy group, a propoxy group (each isomer), a butyloxy group (each isomer), a pentyloxy group (each isomer), a hexyloxy group (each isomer), a heptyloxy group (each isomer), an octyloxy group (each isomer), a nonyloxy group (each isomer), a decyloxy group (each isomer), a dodecyloxy group (each isomer), and an octadecyloxy group (each isomer).

Examples of the aryl group having 6 or more and 20 or less carbon atoms, as $R^{61}$, include a phenyl group and a naphthyl group.

Examples of the aryl group having an alkyl group as the substituent in $R^{61}$ include a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), a biphenyl group (each isomer), a dimethylphenyl group (each isomer), a diethylphenyl group (each isomer), a dipropylphenyl group (each isomer), a dibutylphenyl group (each isomer), a dipentylphenyl group (each isomer), a dihexylphenyl group (each isomer), a diheptylphenyl group (each isomer), a terphenyl group (each isomer), a trimethylphenyl group (each isomer), a triethylphenyl group (each isomer), a tripropylphenyl group (each isomer), and a tributylphenyl group (each isomer).

Examples of the aryloxy group having 6 or more and 20 or less carbon atoms, as $R^{61}$, include a phenoxy group, a methylphenoxy group (each isomer), an ethylphenoxy group (each isomer), a propylphenoxy group (each isomer), a butylphenoxy group (each isomer), a pentylphenoxy group (each isomer), a hexylphenoxy group (each isomer), a heptylphenoxy group (each isomer), an octylphenoxy group (each isomer), a nonylphenoxy group (each isomer), a decylphenoxy group (each isomer), a phenylphenoxy group (each isomer), a dimethylphenoxy group (each isomer), a diethylphenoxy group (each isomer), a dipropylphenoxy group (each isomer), a dibutylphenoxy group (each isomer), a dipentylphenoxy group (each isomer), a dihexylphenoxy group (each isomer), a diheptylphenoxy group (each isomer), a diphenylphenoxy group (each isomer), a trimethylphenoxy group (each isomer), a triethylphenoxy group (each isomer), a tripropylphenoxy group (each isomer), and a tributyl phenoxy group (each isomer).

Examples of the aralkyl group having 7 or more and 20 or less carbon atoms, as $R^{61}$, include a phenylmethyl group, a phenylethyl group (each isomer), a phenylpropyl group (each isomer), a phenylbutyl group (each isomer), a phenylpentyl group (each isomer), a phenylhexyl group (each isomer), a phenylheptyl group (each isomer), a phenyloctyl group (each isomer), and a phenylnonyl group (each isomer).

Examples of the aralkyloxy group having 7 or more and 20 or less carbon atoms, as $R^{61}$, include a phenylmethoxy group, a phenylethoxy group (each isomer), a phenylpropyloxy group (each isomer), a phenylbuthyloxy group (each isomer), a phenylpentyloxy group (each isomer), a phenylhexyloxy group (each isomer), a phenylheptyloxy group (each isomer), a phenyloctyloxy group (each isomer), and a phenylnonyloxy group (each isomer).

[$A^{61}$]

The ring $A^{61}$ is an aromatic hydrocarbon ring having 6 or more and 20 or less carbon atoms. The ring $A^{61}$ may be a monocyclic ring, a polycyclic ring, or a fused ring.

Specific examples of the ring $A^{61}$ include a benzene ring, a naphthalene ring, an anthracene ring, a phenanthrene ring, a naphthacene ring, a chrysene ring, a pyrene ring, a triphenylene ring, a pentylene ring, an azulene ring, a heptalene ring, an indacene ring, a biphenylene ring, an acenaphthylene ring, an acenanthrylene ring, and an acenaphthylene ring. Among these, the ring $A^{11}$ is preferably a benzene ring, a naphthalene ring, or an anthracene ring, and more preferably a benzene ring.

In addition, these rings may have a substituent other than $R^{61}$. Examples of the substituent other than $R^{61}$ include the same one as that described in $R^{61}$. $R^{61}$ and the substituent other than $R^{61}$ consist of different functional groups.

[n61]

n61 represents the number of substituents $R^{61}$ and is an integer of 1 or more and 10 or less.

In General Formula (VI), examples of the compound in which the ring $A^{61}$ is a benzene ring include a compound represented by General Formula (VI-1).

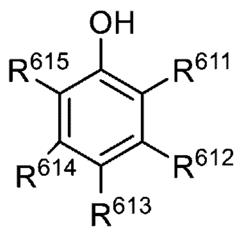

( VI-1 )

(in General Formula (VI-1), $R^{611}$ to $R^{615}$ are each independently the same as $R^{61}$)

Among these, it is preferable that at least one of $R^{611}$ to $R^{615}$ is a hydrogen atom, and it is more preferable that all of $R^{611}$ to $R^{615}$ are hydrogen atoms.

Examples of the preferred hydroxy compound include phenol, 2-ethylphenol, 2-propylphenol (each isomer), 2-butylphenol (each isomer), 2-pentylphenol (each isomer), 2-hexylphenol (each isomer), 2-heptylphenol (each isomer), 2-phenylphenol, 2,6-dimethylphenol, 2,4-diethylphenol, 2,6-diethylphenol, 2,4-dipropylphenol (each isomer), 2,6-dipropylphenol (each isomer), 2,4-dibutylphenol (each isomer), 2,4-dipentylphenol (each isomer), 2,4-dihexylphenol (each isomer), 2,4-diheptylphenol (each isomer), 2-methyl-6-ethylphenol, 2-methyl-6-propylphenol (each isomer), 2-methyl-6-butylphenol (each isomer), 2-methyl-6-pentylphenol (each isomer), 2-ethyl-6-propylphenol (each isomer), 2-ethyl-6-butylphenol (each isomer), 2-ethyl-6-pentylphenol (each isomer), 2-propyl-6-butylphenol (each isomer), 2-ethyl-4-methylphenol (each isomer), 2-ethyl-4-propylphenol (each isomer), 2-ethyl-4-butylphenol (each isomer), 2-ethyl-4-pentylphenol (each isomer), 2-ethyl-4-hexylphenol (each isomer), 2-ethyl-4-heptylphenol (each isomer), 2-ethyl-4-octylphenol (each isomer), 2-ethyl-4-phenylphenol (each isomer), 2-ethyl-4-cumylphenol (each isomer), 2-propyl-4-methylphenol (each isomer), 2-propyl-4-ethylphenol (each isomer), 2-propyl-4-butylphenol (each isomer), 2-propyl-4-pentylphenol (each isomer), 2-propyl-4-hexylphenol (each isomer), 2-propyl-4-heptylphenol (each isomer), 2-propyl-4-octylphenol (each isomer), 2-propyl-4-phenylphenol (each isomer), 2-propyl-4-cumylphenol (each isomer), 2-butyl-4-methylphenol (each isomer), 2-butyl-4-ethylphenol (each isomer), 2-butyl-4-propylphenol (each isomer), 2-butyl-4-pentylphenol (each isomer), 2-butyl-4-hexylphenol (each isomer), 2-butyl-4-heptylphenol (each isomer), 2-butyl-4-octylphenol (each isomer), 2-butyl-4-phenylphenol (each isomer), 2-butyl-4-cumylphenol (each isomer), 2-pentyl-4-methylphenol (each isomer), 2-pentyl-4-ethylphenol (each isomer), 2-pentyl-4-propylphenol (each isomer), 2-pentyl-4-butylphenol (each isomer), 2-pentyl-4-hexylphenol (each isomer), 2-pentyl-4-heptylphenol (each isomer), 2-pentyl-4-octylphenol (each isomer), 2-pentyl-4-phenylphenol (each isomer), 2-pentyl-4-cumylphenol (each isomer), 2-hexyl-4-methylphenol (each isomer), 2-hexyl-4-ethylphenol (each isomer), 2-hexyl-4-propylphenol (each isomer), 2-hexyl-4-butylphenol (each isomer), 2-hexyl-4-pentylphenol (each isomer), 2-hexyl-4-heptylphenol (each isomer), 2-hexyl-4-octylphenol (each isomer), 2-hexyl-4-phenylphenol (each isomer), 2-hexyl-4-cumylphenol (each isomer), 2-heptyl-4-methylphenol (each isomer), 2-heptyl-4-ethylphenol (each isomer), 2-heptyl-4-propylphenol (each isomer), 2-heptyl-4-butylphenol (each isomer), 2-heptyl-4-pentylphenol (each isomer), 2-heptyl-4-hexylphenol (each isomer), 2-heptyl-4-octylphenol (each isomer), 2-heptyl-4-phenylphenol (each isomer), 2-heptyl-4-cumylphenol (each isomer), 2,4,6-trimethylphenol, 2,6-dimethyl-4-ethylphenol, 2,6-dimethyl-4-propylphenol (each isomer), 2,6-dimethyl-4-butylphenol (each isomer), 2,6-dimethyl-4-pentylphenol (each isomer), 2,6-dimethyl-4-hexylphenol (each isomer), 2,6-dimethyl-4-phenylphenol, 2,6-dimethyl-4-cumylphenol, 2,4,6-triethylphenol, 2,6-diethyl-4-methylphenol, 2,6-diethyl-4-propylphenol (each isomer), 2,6-diethyl-4-butylphenol (each isomer), 2,6-diethyl-4-pentylphenol (each isomer), 2,6-diethyl-4-hexylphenol (each isomer), 2,6-diethyl-4-phenylphenol, 2,6-diethyl-4-cumylphenol, 2,4,6-tripropylphenol (each isomer), 2,6-dipropyl-4-ethylphenol (each isomer), 2,6-dipropyl-4-methylphenol (each isomer), 2,6-dipropyl-4-butylphenol (each isomer), 2,6-dipropyl-4-pentylphenol (each isomer), 2,6-dipropyl-4-hexylphenol (each isomer), 2,6-dipropyl-4-phenylphenol (each isomer), 2,6-dipropyl-4-cumylphenol (each isomer), 2,4-dimethyl-6-ethylphenol, 2-methyl-4,6-diethylphenol, 2-methyl-4-propyl-6-ethylphenol (each isomer), 2-methyl-4-butyl-6-ethylphenol (each isomer), 2-methyl-4-pentyl-6-ethylphenol (each isomer), 2-methyl-4-hexyl-6-ethylphenol (each isomer), 2-methyl-4-phenyl-6-ethylphenol (each isomer), 2-methyl-4-cumyl-6-ethylphenol (each isomer), 2,4-dimethyl-6-propylphenol (each isomer), 2-methyl-4,6-dipropylphenol (each isomer), 2-methyl-4-ethyl-6-propylphenol (each isomer), 2-methyl-4-butyl-6-propylphenol (each isomer), 2-methyl-4-pentyl-6-propylphenol (each isomer), 2-methyl-4-hexyl-6-propylphenol (each isomer), 2-methyl-4-phenyl-6-propylphenol (each isomer), 2-methyl-4-cumyl-6-propylphenol (each isomer), 2,4-dimethyl-6-butylphenol, 2-methyl-4,6-dibutylphenol, 2-methyl-4-propyl-6-butylphenol (each isomer), 2-methyl-4-ethyl-6-butylphenol (each isomer), 2-methyl-4-pentyl-6-butylphenol (each isomer), 2-methyl-4-hexyl-6-butylphenol (each isomer), 2-methyl-4-phenyl-6-butylphenol (each isomer), 2-methyl-4-cumyl-6-butylphenol (each isomer), 2,4-dimethyl-6-pentylphenol (each isomer), 2,4-diethyl-6-pentylphenol (each isomer), 2-ethyl-4,6-pentylphenol (each isomer), 2-ethyl-4-butyl-6-pentylphenol (each isomer), 2-ethyl-4-propyl-6-pentylphenol (each isomer), 2-ethyl-4-hexyl-6-pentylphenol (each isomer), 2-ethyl-4-heptyl-6-pentylphenol (each isomer) isomer), 2-ethyl-4-octyl-6-pentylphenol (each isomer), 2-ethyl-4-phenyl-6-pentylphenol (each isomer), 2-ethyl-4-cumyl-6- pentylphenol (each isomer), 2-ethyl-4-methyl-6-hexylphenol (each isomer), 2,4-diethyl-6-pentylphenol (each isomer), 2-ethyl-4,6-pentylphenol

(each isomer), 2-ethyl-4-butyl-6-pentylphenol (each isomer), 2-ethyl-4-propyl-6-pentylphenol (each isomer), 2-ethyl-4-hexyl-6-pentylphenol (each isomer), 2-ethyl-4-heptyl-6-pentylphenol (each isomer), 2-ethyl-4-octyl-6-pentylphenol (each isomer), 2-ethyl-4-phenyl-6-pentylphenol (each isomer), 2-ethyl-4-cumyl-6-pentylphenol (each isomer), 2-ethyl-4-methyl-6-hexylphenol (each isomer), 2,4-diethyl-6-hexylphenol (each isomer), 2-ethyl-4,6-hexylphenol (each isomer), 2-ethyl-4-propyl-6-hexylphenol (each isomer), 2-ethyl-4-pentyl-6-hexylphenol (each isomer), 2-ethyl-4-butyl-6-hexylphenol (each isomer), 2-ethyl-4-heptyl-6-hexylphenol (each isomer), 2-ethyl-4-octyl-6-hexylphenol (each isomer), 2-ethyl-4-phenyl-6-hexylphenol (each isomer), 2-ethyl-4-cumyl-6-hexylphenol (each isomer), 2-propyl-4-methyl-6-butylphenol (each isomer), 2,4-dipropyl-6-butylphenol (each isomer), 2-propyl-4,6-butylphenol (each isomer), 2-propyl-4-ethyl-6-butylphenol (each isomer), 2-propyl-4-pentyl-6-butylphenol (each isomer), 2-propyl-4-hexyl-6-butylphenol (each isomer), 2-propyl-4-heptyl-6-butylphenol (each isomer), 2-propyl-4-octyl-6-butylphenol (each isomer), 2-propyl-4-phenyl-6-butylphenol (each isomer), 2-propyl-4-cumyl-6-butylphenol (each isomer), 2,4-dicumylphenol, methoxyphenol (each isomer), and ethoxyphenol (each isomer). Among these, phenol, methoxyphenol (each isomer), or ethoxyphenol (each isomer) is preferable.

The hydroxy compound is preferably a hydroxy compound having a standard boiling point lower than a standard boiling point of the isocyanate compound produced from the N-substituted carbamate compound described above.

[Carbonic acid derivative]

As the carbonic acid derivative, urea, urea derivatives, and carbonic acid esters are exemplary examples. The details of the carbonic acid ester will be described later.

Examples of the urea derivative include a compound represented by General Formula (IV) (hereinafter, may be referred to as "urea derivative (IV)") obtained by the following reaction formula.

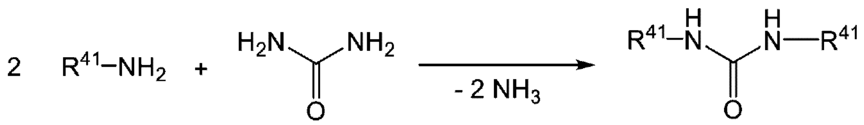


(IV)

In General Formula (IV), $R^{41}$ is a hydrogen atom or a monovalent organic group.

(R41)

As the monovalent organic group as $R^{41}$, the same one as $R^{52}$ described later is an exemplary example. Among these, $R^{41}$ is preferably an aliphatic hydrocarbon group having 1 or more and 20 or less carbon atoms, and more preferably an alkyl group having 1 or more and 10 or less carbon atoms.

In the above reaction formula, for the sake of simplicity of description, a case in which one kind of primary amine having one primary amino group in one molecule (that is, a monofunctional primary amine) is used and urea is used as the carbonic acid derivative is shown. However, those skilled in the art can easily understand that the same reaction occurs even in a case in which a primary amine having two or more primary amino groups in one molecule (that is, a di- or higher functional primary amine) is used as the primary amine or a case in which a compound other than urea, for example, N-alkyl urea or N,N'-dialkyl urea, in which each amino group of urea is substituted with an alkyl group, is used as the urea derivative.

In a case where the N-alkyl urea is used in the step (1) as the urea derivative (IV) obtained by the above reaction formula, the by-product low-boiling compound is ammonia and an alkylamine corresponding to the alkyl group; and in a case where the N,N'-dialkyl urea is used in the step (1), the by-product low-boiling compound is an alkylamine corresponding to the alkyl group.

[N-substituted carbamate compound]

As the N-substituted carbamate compound, for example, a compound represented by General Formula (V) (hereinafter, may be referred to as "carbamate compound (V)") is an exemplary example.

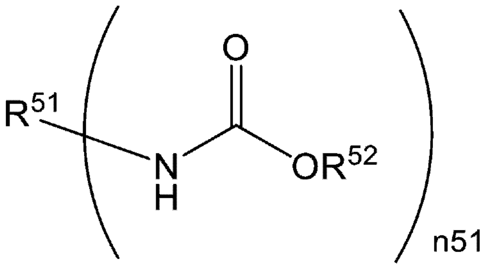

(V)

(in General Formula (V), $R^{51}$ is a di- or higher valent organic group, $R^{52}$ is a monovalent organic group, and n51 is an integer of 2 or more)

($R^{51}$)

In General Formula (V), $R^{51}$ is a di- or higher valent organic group, and is preferably a di- or higher and deca- or lower valent organic group. Among these, $R^{51}$ is preferably a di- or higher and tetra- or lower valent aliphatic hydrocarbon group having 1 or more and 20 or less carbon atoms, which may have 1 or more and 4 or less ester groups or a nitrogen atom, or a divalent or trivalent aromatic group having 6 or more and 20 or less carbon atoms, which may have 1 or more and 4 or less ester groups or a nitrogen atom.

As the aliphatic hydrocarbon group as $R^{51}$, an alkylene group or an alkanetriyl group, a cycloalkyl group, a cycloalkylene group or a cycloalkanetriyl group, or a group constituting the alkyl group, the alkylene group or the alkanetriyl group, and the cycloalkyl group, the cycloalkylene group or the cycloalkanetriyl group is preferable; and a linear or branched alkylene group or alkanetriyl group, a cycloalkylene group or a cycloalkanetriyl group, or a group constituting the alkylene group or the alkanetriyl group, and the cycloalkyl group, the cycloalkylene group or the cycloalkanetriyl group is more preferable.

Examples of the linear or branched alkylene group include a methylene group, an ethylene group, a propylene group, a trimethylene group, a pentylene group, an n-hexylene group, and a decamethylene group.

Examples of the cycloalkylene group include a cyclobutylene group and a cyclohexylene group.

Examples of the linear or branched alkanetriyl group include a hexanetriyl group, a nonanetriyl group, and a decanetriyl group.

Examples of the cycloalkanetriyl group include a cyclopropanetriyl group, a cyclobutanetriyl group, a cyclopentatriyl group, and a cyclohexatriyl group.

The aromatic hydrocarbon group as $R^{51}$ is preferably a group having a substituted or unsubstituted aromatic ring having 6 or more and 13 or less carbon atoms. Examples of the substituent include an alkyl group, an aryl group, and an aralkyl group. The aromatic ring may be an aromatic hydrocarbon ring or a heteroaromatic ring, and specific examples thereof include a benzene ring, a naphthalene ring, and a pyridine ring.

In a case where the aliphatic hydrocarbon group or the aromatic group as $R^{51}$ has 1 or more and 4 or less ester groups, as $R^{51}$, specifically, for example, a group obtained by removing a terminal primary amino group of an amine compound having an ester group obtained by reacting a carboxy group of amino acid with a hydroxy compound is preferable.

Specific examples of the amine compound having the ester group as used herein include acrylic acid-2-aminoethyl ester, 2-methyl-acrylic acid-2-aminoethyl ester, acrylic acid-2-aminopropyl ester, 2-methyl-acrylic acid-2-aminopropyl ester, acrylic acid-3-aminopropyl ester, 2-methyl-acrylic acid-3-aminopropyl ester, acrylic acid-4-aminobutyl ester, 2-methyl-acrylic acid-4-aminobutyl ester, acrylic acid-5-aminopentyl ester, 2-methyl-acrylic acid-5-aminopentyl ester, acrylic acid-6-aminohexyl ester, 2-methyl-acrylic acid-6-aminohexyl ester, acrylic acid-8-aminooctyl ester, 2-methyl-acrylic acid-8-aminooctyl ester, acrylic acid-10-aminodecyl ester, 2-methyl-acrylic acid-10-aminodecyl ester, acrylic acid-11-aminoundecyl ester, 2-methyl-acrylic acid-11-aminoundecyl ester, acrylic acid-12-aminododecyl ester, 2-methyl-acrylic acid-12-aminododecyl ester, lysine methylesterdiamine, lysine ethylesterdiamine, 2-aminoethyl-2,5-diaminopentanoate, 2-aminoethyl-2,6-diaminohexanoate, bis(2-aminoethyl)-2-aminobutanedioate, bis(2-aminoethyl)-2-aminopentanedioate, and tris(2-aminoethyl)hexane-1,3,6-tricarboxylate.

Examples of the amino acid used for producing the amine compound having an ester group include lysine, alanine, arginine, asparagine, glutamine, glycine, aspartic acid, glutamic acid, ornithine, histidine, isoleucine, leucine, methionine, phenylalanine, tryptophan, and valine. Among these, the amino acid is preferably lysine, arginine, glycine, aspartic acid, glutamic acid, or ornithine; and more preferably lysine, arginine, glycine, aspartic acid, or glutamic acid.

Examples of the hydroxy compound used for producing the amine compound having an ester group include alcohols and aromatic hydroxy compounds.

Examples of the alcohols include methyl alcohol, propyl alcohol, butyl alcohol, amyl alcohol, hexyl alcohol,

heptyl alcohol, octyl alcohol, nonyl alcohol, decyl alcohol, undecyl alcohol, lauryl alcohol, dodecyl alcohol, stearyl alcohol, eicosyl alcohol, allyl alcohol, crotyl alcohol, propargyl alcohol, cyclopentanol, cyclohexanol, benzyl alcohol, cinnamyl alcohol, ethylene glycol, diethylene glycol, polyethylene glycol, propylene glycol, polypropylene glycol, 1,3-butanediol, 1,4-butanediol, hydrogenated bisphenol A, neopentyl glycol, glycerin, trimethylolpropane, pentaerythritol, ethanolamine, propanolamine (1-amino-2-propanol), dimethanolamine, diethanolamine, dipropanolamine, and 1-amino-2-butanol.

Examples of the aromatic hydroxy compound include monophenols such as phenol (carbolic acid), 2-methoxyphenol, cresol, xylenol, carvacrol, thymol, and naphthol; and polyphenols such as catechol, resorcin, hydroquinone, bisphenol A, bisphenol F, pyrogallol, and phloroglucin.

In a case where the aliphatic hydrocarbon group or the aromatic group as $R^{51}$ has 1 or more and 4 or less nitrogen atoms, as $R^{51}$, an organic group obtained by removing a terminal amino group of an amine compound having 1 or more and 4 or less secondary or tertiary amines other than the terminal of the aliphatic hydrocarbon group or the aromatic group. Specific examples of the amine compound having 1 or more and 4 or less secondary or tertiary amines other than the terminal of the aliphatic hydrocarbon group or the aromatic group include 2-(dimethylamino)ethylamine, 2-(diethylamino)ethylamine, 2-(diisopropylamino)ethylamine, 2-(cyclohexylamino)ethylamine, 3-(cyclohexylamino)propylamine, 3-(diethylamino)propylamine, 3-(dimethylamino)propylamine, diethylenetriamine, diisopropyltriamine, bis-(3-aminopropyl)methyleneamine, 3-(2-aminoethylamino)propylamine, N,N'-bis(3-aminopropyl)ethylenediamine, 1-(3-aminopropyl)imidazole, trisaminoethylamine, and trisaminopropylamine. Among these, the amine compound is preferably an amine compound having one or more and two or less tertiary amines other than terminals and having an aliphatic hydrocarbon group or an aromatic group; and more preferably an amine compound having one tertiary amine other than terminal and having an aliphatic hydrocarbon group or an aromatic group.

($R^{52}$)

In General Formula (V), $R^{52}$ is a monovalent organic group. Among these, $R^{52}$ is preferably an aliphatic hydrocarbon group having 1 or more and 20 or less carbon atoms or an aromatic hydrocarbon group having 6 or more and 20 or less carbon atoms, which may include an oxygen atom.

Examples of the aliphatic hydrocarbon group as $R^{52}$ include alkyl groups such as a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), an undecyl group (each isomer), a dodecyl group (each isomer), a tridecyl group (each isomer), a tetradecyl group (each isomer), a pentadecyl group (each isomer), a hexadecyl group (each isomer), a heptadecyl group (each isomer), an octadecyl group (each isomer), a nonadecyl (each isomer), and an eicosyl group (each isomer); and cycloalkyl groups such as a cyclopentyl group, a cyclohexyl group, a cycloheptyl group, a cyclooctyl group, a cyclononyl group, and a cyclodecyl group.

Examples of the aliphatic hydrocarbon group which may include an oxygen atom, as $R^{52}$, include alkoxyalkyl groups such as a methoxymethyl group, a methoxyethyl group (each isomer), a methoxypropyl group (each isomer), a methoxybutyl group (each isomer), a methoxypentyl group (each isomer), a methoxyhexyl group (each isomer), a methoxyheptyl group (each isomer), a methoxyoctyl group (each isomer), a methoxynonyl group (each isomer), a methoxydecyl group (each isomer), a methoxyundecyl group (each isomer), a methoxydodecyl group (each isomer), a methoxytridecyl group (each isomer), a methoxytetradecyl group (each isomer), a methoxypentadecyl group (each isomer), a methoxyhexadecyl group (each isomer), a methoxyheptadecyl group (each isomer), a methoxyoctadecyl group (each isomer), a methoxynonadecyl (each isomer), an ethoxymethyl group, an ethoxyethyl group (each isomer), an ethoxypropyl group (each isomer), an ethoxybutyl group (each isomer), an ethoxypentyl group (each isomer), an ethoxyhexyl group (each isomer), an ethoxyheptyl group (each isomer), an ethoxyoctyl group (each isomer), an ethoxynonyl group (each isomer), an ethoxydecyl group (each isomer), an ethoxyundecyl group (each isomer), an ethoxydodecyl group (each isomer), an ethoxytridecyl group (each isomer), an ethoxytetradecyl group (each isomer), an ethoxypentadecyl group (each isomer), an ethoxyhexadecyl group (each isomer), an ethoxyheptadecyl group (each isomer), an ethoxyoctadecyl group (each isomer), a propyloxymethyl group (each isomer), a propyloxyethyl group (each isomer), a propyloxypropyl group (each isomer), a propyloxybutyl group (each isomer), a propyloxypentyl group (each isomer), a propyl oxyhexyl group (each isomer), a propyloxyheptyl group (each isomer), a propyloxyoctyl group (each isomer), a propyloxy nonyl group (each isomer), a propyloxydecyl group (each isomer), a propyloxyundecyl group (each isomer), a propyloxydodecyl group (each isomer), a propyloxytridecyl group (each isomer), a propyloxytetradecyl group (each isomer), a propyloxypentadecyl group (each isomer), a propyloxyhexadecyl group (each isomer), a propyloxyheptadecyl group (each isomer), a butyloxymethyl group (each isomer), a butyloxyethyl group (each isomer), a butyloxypropyl group (each isomer), a butyloxybutyl group (each isomer), a butyloxypentyl group (each isomer), a butyloxyhexyl group (each isomer), a butyloxyheptyl group (each isomer), a butyloxyoctyl group (each isomer), a butyloxy nonyl group (each isomer), a butyloxydecyl group (each isomer), a butyloxyundecyl group (each isomer), a butyloxydodecyl group (each isomer), a butyloxytridecyl group (each isomer), a butyloxytetradecyl group (each isomer), a butyloxypentadecyl group (each isomer), a butyloxyhexadecyl group (each isomer), a pentyloxymethyl group (each isomer), a pentyloxyethyl group (each isomer), a pentyloxypropyl group (each isomer), a pentyloxybutyl group (each isomer), a pentyloxypentyl group (each isomer), a pentyloxyhexyl group (each isomer), a pentyloxyheptyl group (each isomer), a pentyloxyoctyl group (each isomer), a pentyloxy nonyl group (each isomer), a pentyloxydecyl group (each isomer), a pentyloxyundecyl group (each isomer), a pentyloxydodecyl group (each isomer), a pentyloxytridecyl group (each isomer), a pentyloxytetradecyl group (each isomer), a pentyloxypentadecyl group (each isomer), a hexyloxymethyl group (each isomer), a hexyloxyethyl group (each isomer), a hexyloxypropyl group (each isomer), a hexyloxybutyl group (each isomer), a hexyloxypentyl group (each isomer), a hexyloxyhexyl group (each isomer), a hexyloxyheptyl group (each isomer), a hexyloxyoctyl group (each isomer), a hexyloxyonyl group (each isomer), a hexyloxydecyl group (each isomer), a hexyloxyundecyl group (each isomer), a hexyloxydodecyl group (each isomer), a hexyloxytridecyl group (each isomer), a hexyloxytetradecyl group (each isomer), a heptyloxymethyl group (each isomer), a heptyloxyethyl group (each isomer), a heptyloxypropyl group (each isomer), a heptyloxybutyl group (each isomer), a heptyloxypentyl group (each isomer), a heptyloxyhexyl group (each isomer), a heptyloxyheptyl group (each isomer), a heptyloxyoctyl group (each isomer), a heptyloxyonyl group (each isomer), a heptyloxydecyl group (each isomer), a heptyloxyundecyl group (each isomer), a heptyloxydodecyl group (each isomer), a heptyloxytridecyl group (each isomer), an octyloxymethyl group (each isomer), an octyloxyethyl group (each isomer), an octyloxypropyl group (each isomer), an octyloxybutyl group (each isomer), an octyloxypentyl group (each isomer), an octyloxyhexyl group (each isomer), an octyloxyheptyl group (each isomer), an octyloxyoctyl group (each isomer), an octyloxyonyl group (each isomer), an octyloxydecyl group (each isomer), an octyloxyundecyl group (each isomer), a heptyloxyundecyl group (each isomer), a heptyloxydodecyl group (each isomer), a heptyloxytridecyl group (each isomer), an octyloxymethyl group (each isomer), an octyloxyethyl group (each isomer), an octyloxypropyl group (each isomer), an octyloxybutyl group (each isomer), an octyloxypentyl group (each isomer), an octyloxyhexyl group (each isomer), an octyloxyheptyl group (each isomer), an octyloxyoctyl group (each isomer), an octyloxyonyl group (each isomer), an octyloxydecyl group (each isomer), an octyloxyundecyl group (each isomer), an octyloxidecyl group (each isomer), a nonyloxymethyl group (each isomer), a nonyloxyethyl group (each isomer), a nonyloxypropyl group (each isomer), a nonyloxybutyl group (each isomer), a nonyloxypentyl group (each isomer), a nonyloxyhexyl group (each isomer), a nonyloxyheptyl group (each isomer), a nonyloxyoctyl group (each isomer), a nonyloxyonyl group (each isomer), a nonyloxydecyl group (each isomer), a nonyloxyundecyl group (each isomer), a decyloxymethyl group (each isomer), a decyloxyethyl group (each isomer), a decyloxypropyl group (each isomer), a decyloxybutyl group (each isomer), a decyloxypentyl group (each isomer), a decyloxyhexyl group (each isomer), a decyloxyheptyl group (each isomer), a decyloxyoctyl group (each isomer), a decyloxyonyl group (each isomer), a decyloxydecyl group (each isomer), a decyloxyundecyl group (each isomer), a undecyloxyethyl group (each isomer), a undecyloxypropyl group (each isomer), a undecyloxybutyl group (each isomer), a undecyloxypentyl group (each isomer), a undecyloxyhexyl group (each isomer), a undecyloxyheptyl group (each isomer), a undecyloxyoctyl group (each isomer), a undecyloxyonyl group (each isomer), a dodecyloxymethyl group (each isomer), a dodecyloxyethyl group (each isomer), a dodecyloxypropyl group (each isomer), a dodecyloxybutyl group (each isomer), a dodecyloxypentyl group (each isomer), a dodecyloxyhexyl group (each isomer), a dodecyloxyheptyl group (each isomer), a dodecyldodecyloxyoctyl group (each isomer), a tridecyloxymethyl group (each isomer), a tridecyloxyethyl group (each isomer), a tridecyloxypropyl group (each isomer), a tridecyloxybutyl group (each isomer), a tridecyloxypentyl group (each isomer), a tridecyloxyhexyl group (each isomer), a tridecyloxyheptyl group (each isomer), a tetradecyloxymethyl group (each isomer), a tetradecyloxyethyl group (each isomer), a tetradecyloxypropyl group (each isomer), a tetradecyloxybutyl group (each isomer), a tetradecyloxypentyl group (each isomer), a tetradecyloxyhexyl group (each isomer), a pentadecyloxymethyl group, a pentadecyloxyethyl group (each isomer), a pentadecyloxypropyl group (each isomer), a pentadecyloxybutyl group (each isomer), a pentadecyloxypentyl group (each isomer), a hexadecyloxymethyl group (each isomer), a hexadecyloxyethyl group (each isomer), a hexadecyloxypropyl group (each isomer), a hexadecyloxybutyl group (each isomer), a heptadecyloxymethyl group (each isomer), a heptadecyloxyethyl group (each isomer), a heptadecyloxypropyl group (each isomer), an octadecyloxymethyl group (each isomer), and an octadecyloxyethyl group (each isomer).

Examples of the aromatic hydrocarbon group as $R^{52}$ include aryl groups such as a phenyl group, a naphthyl group, an anthryl group, a pyrenyl group, and a phenanthryl group; and aryl groups having an alkyl group as a substituent, such as a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl

group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), and a dibutylhexylphenyl group (each isomer).

Examples of the aromatic hydrocarbon group which may include an oxygen atom, as $R^{52}$, include an alkoxyaryl group such as a methoxyphenyl group (each isomer) and an ethoxyphenyl group (each isomer).

Among these, as $R^{52}$, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a methyltetradecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a phenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer)a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a

triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

(n51)

In General Formula (V), n51 represents the number of carbamate groups, is an integer of 2 or more, preferably an integer of 2 or more and 10 or less, more preferably an integer of 3 or more, and still more preferably 3.
Examples of the preferred N-substituted carbamate compound (V) include compounds represented by Formulae (V-1) to (V-5).

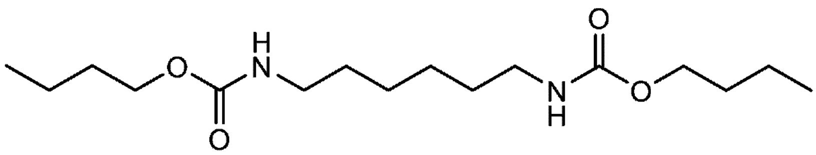
( V-1 )

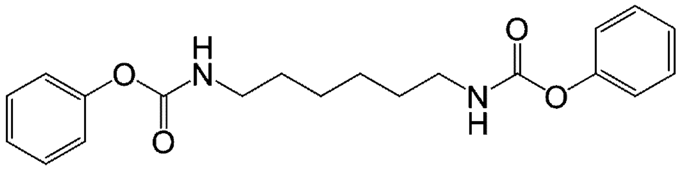
( V-2 )

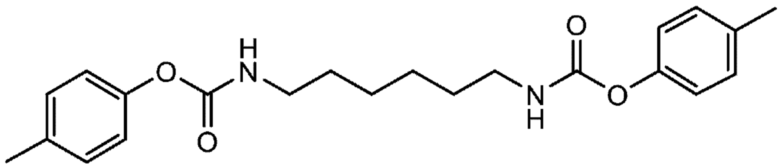
( V-3 )

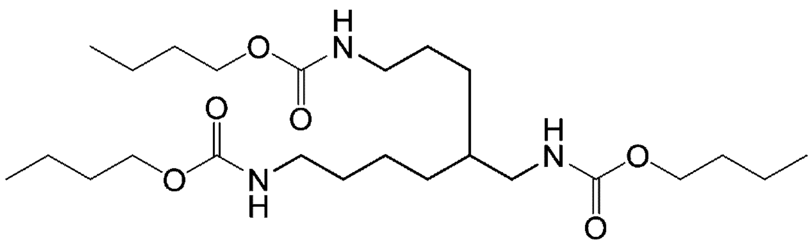
( V -4 )

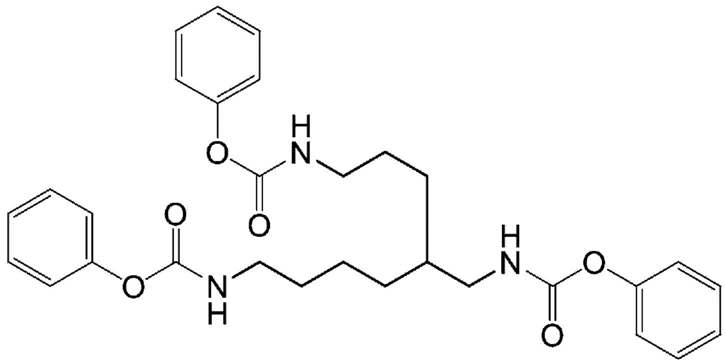

( V -5 )

[Aprotic solvent]

From the viewpoint of not reacting with the isocyanate, the aprotic solvent is preferably a carbonic acid derivative, an ether, a hydrocarbon, a ketone, or a mixture thereof, more preferably a carbonic acid derivative, a hydrocarbon, a ketone, or a mixture thereof, and still more preferably a carbonic acid derivative.

(Carbonic acid derivative)

In the present specification, the carbonic acid derivative refers to a compound having a carbonyl group.
As the carbonic acid derivative, a compound represented by General Formula (III) (hereinafter, may be referred to as "carbonic acid derivative (III)") is preferable.

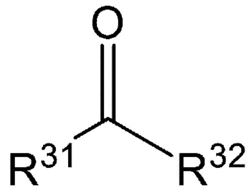

( III )

(in General Formula (III), $R^{31}$ and $R^{32}$ are each independently a substituted or unsubstituted alkoxy group having 1 or more and 20 or less carbon atoms or an aryloxy group having 6 or more and 20 or less carbon atoms)

($R^{31}$ and $R^{32}$)

$R^{31}$ and $R^{32}$ may be the same or different from each other, but are preferably the same.
Examples of the alkoxy group having 1 or more and 20 or less carbon atoms, as $R^{31}$ and $R^{32}$, include a methoxy group, an ethoxy group, a propyloxy group (each isomer), a butoxy group (each isomer), and a hexyloxy group (each isomer).
Examples of the aryloxy group having 6 or more and 20 or less carbon atoms, as $R^{31}$ and $R^{32}$, include a phenoxy group and a naphthyloxy group.
Examples of the substituent of the alkoxy group, the aryloxy group, and the aralkyloxy group include an alkyl group and an alkoxy group.
Among these, $R^{31}$ and $R^{32}$ are each independently preferably a substituted or unsubstituted aryloxy group having 6 or more and 20 or less carbon atoms.
Examples of the preferred carbonic acid derivative (III) include a carbonic acid ester.
The term "carbonic acid ester" refers to a compound in which one or two of two hydrogen atoms of carbonic acid $CO(OH)_2$ are substituted with an alkyl group or an aryl group. The carbonic acid ester is a compound represented by General Formula (III-1) (hereinafter, maybe referred to as "carbonic acid ester (III-1)").

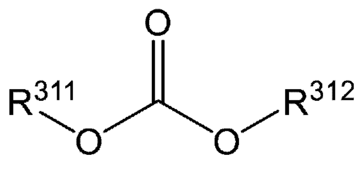

( III -1)

(in General Formula (III-1), $R^{311}$ and $R^{312}$ are each independently a substituted or unsubstituted alkyl group having 1 or more and 20 or less carbon atoms or an aryl group having 6 or more and 20 or less carbon atoms)

($R^{311}$ and $R^{312}$)

$R^{311}$ and $R^{312}$ may be the same or different from each other, but are preferably the same.

Examples of the alkyl group having 1 or more and 20 or less carbon atoms, as $R^{311}$ and $R^{312}$, include alkyl groups such as a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), an undecyl group (each isomer), a dodecyl group (each isomer), a tridecyl group (each isomer), a tetradecyl group (each isomer), a pentadecyl group (each isomer), a hexadecyl group (each isomer), a heptadecyl group (each isomer), an octadecyl group (each isomer), a nonadecyl (each isomer), and an eicosyl group (each isomer); and cycloalkyl groups such as a cyclopentyl group, a cyclohexyl group, a cycloheptyl group, a cyclooctyl group, a cyclononyl group, and a cyclodecyl group.

Examples of the aryl group having 6 or more and 20 or less carbon atoms, as $R^{311}$ and $R^{312}$, include a phenyl group, a naphthyl group, an anthryl group, a pyrenyl group, and a phenanthryl group.

Examples of the substituent of the alkyl group include an alkoxy group.

Examples of the substituent of the aryl group include an alkyl group and an alkoxy group.

Examples of the alkyl group having an alkoxy group as a substituent, that is, examples of the alkoxyalkyl group include a methoxyethyl group (each isomer), a methoxypropyl group (each isomer), a methoxybutyl group (each isomer), a methoxypentyl group (each isomer), a methoxyhexyl group (each isomer), a methoxyheptyl group (each isomer), a methoxyoctyl group (each isomer), a methoxynonyl group (each isomer), a methoxydecyl group (each isomer), a methoxyundecyl group (each isomer), a methoxydodecyl group (each isomer), a methoxytridecyl group (each isomer), a methoxytetradecyl group (each isomer), a methoxypentadecyl group (each isomer), a methoxyhexadecyl group (each isomer), a methoxyheptadecyl group (each isomer), a methoxyoctadecyl group (each isomer), a methoxynonadecyl (each isomer), an ethoxymethyl group, an ethoxyethyl group (each isomer), an ethoxypropyl group (each isomer), an ethoxybutyl group (each isomer), an ethoxypentyl group (each isomer), an ethoxyhexyl group (each isomer), an ethoxyheptyl group (each isomer), an ethoxyoctyl group (each isomer), an ethoxynonyl group (each isomer), an ethoxydecyl group (each isomer), an ethoxyundecyl group (each isomer), an ethoxydodecyl group (each isomer), an ethoxytridecyl group (each isomer), an ethoxytetradecyl group (each isomer), an ethoxypentadecyl group (each isomer), an ethoxyhexadecyl group (each isomer), an ethoxyheptadecyl group (each isomer), an ethoxyoctadecyl group (each isomer), a propyloxymethyl group (each isomer), a propyloxyethyl group (each isomer), a propyloxypropyl group (each isomer), a propoxybutyl group (each isomer), a propoxybutyl group (each isomer), a propoxyhexyl group (each isomer), a propoxyheptyl group (each isomer), a propoxyoctyl group (each isomer), a propoxynonyl group (each isomer), a propoxydecyl group (each isomer), a propoxyundecyl group (each isomer), a propoxydodecyl group (each isomer), a propoxytridecyl group (each isomer), a propoxytetradecyl group (each isomer), a propoxypentadecyl group (each isomer), a propoxyhexadecyl group (each isomer), a propoxyheptadecyl group (each isomer), a butyloxymethyl group (each isomer), a butyloxyethyl group (each isomer), a butyloxypropyl group (each isomer), a butoxybutyl group (each isomer), a butoxybutyl group (each isomer), a butoxyhexyl group (each isomer), a butoxyheptyl group (each isomer), a butoxyoctyl group (each isomer), a butoxynonyl group (each isomer), a butoxydecyl group (each isomer), a butoxyundecyl group (each isomer), a group (each isomer), a butoxypentyl group (each isomer), a butoxyhexyl group (each isomer), a butoxyheptyl group (each isomer), a butoxyoctyl group (each isomer), a butoxynonyl group (each isomer), a butoxydecyl group (each isomer), a butoxyundecyl group (each isomer), a butyloxidodecyl group (each isomer), a butyloxotridecyl group (each isomer), a butyloxotetradecyl group (each isomer), a butyloxopentadecyl group (each isomer), a butyloxhexadecyl group (each isomer), a pentyloxymethyl group (each isomer), a pentyloxyethyl group (each isomer), a pentyloxopropyl group (each isomer), a pentyloxybutyl group (each isomer), a pentyloxypentyl group (each isomer), a pentyloxyhexyl group (each isomer), a pentyloxyheptyl group (each isomer), a pentyloxyoctyl group (each isomer), a pentyloxynonyl group (each isomer), a pentyloxodecyl group (each isomer), a pentyloxundecyl group (each isomer), a pentyloxydodecyl group (each isomer), a pentyloxotridecyl group (each isomer), a pentyloxotetradecyl group (each isomer), a pentyloxopentadecyl group (each isomer), a hexyloxymethyl group (each isomer), a hexyloxyethyl group (each isomer), a hexyloxopropyl group (each isomer), a hexyloxybutyl group (each isomer), a hexyloxy pentyl group (each isomer), a hexyloxy hexyl group (each isomer), a hexyloxy heptyl group (each isomer), a hexyloxy octyl group (each isomer), a hexyloxy nonyl group (each isomer), a hexyloxy decyl group (each isomer), a hexyloxy undecyl group (each isomer), a hexyloxy dodecyl group (each isomer), a hexyloxy tridecyl group (each isomer), a hexyloxy tetradecyl group (each isomer), a heptyloxy methyl group (each isomer), a heptyloxy ethyl group (each isomer), a heptyloxy propyl group (each isomer), a heptyloxy butyl group (each isomer), a heptyloxy pentyl group (each isomer), a heptyloxy hexyl group (each isomer), a heptyloxy heptyl group (each isomer), a heptyloxy octyl group (each isomer), a heptyloxy nonyl group (each isomer), a heptyloxy decyl group (each isomer), a heptyloxy undecyl group (each isomer), a heptyloxy dodecyl group (each isomer), an

octyloxy methyl group (each isomer), an octyloxy ethyl group (each isomer), an octyloxy propyl group (each isomer), an octyloxy butyl group (each isomer), an octyloxy pentyl group (each isomer), an octyloxy hexyl group (each isomer), an octyloxy heptyl group (each isomer), an octyloxy octyl group (each isomer), an octyloxy nonyl group (each isomer), an octyloxy decyl group (each isomer), an octyloxy undecyl group (each isomer), an octyloxy dodecyl group (each isomer), an oxypentyl group (each isomer), an octyloxy hexyl group (each isomer), an octyloxy heptyl group (each isomer), an octyloxy octyl group (each isomer), an octyloxy nonyl group (each isomer), an octyloxy decyl group (each isomer), an octyloxy undecyl group (each isomer), an octyloxy dodecyl group (each isomer), a nonyloxymethyl group (each isomer), a nonyloxyethyl group (each isomer), a nonyloxypropyl group (each isomer), a nonyloxybutyl group (each isomer), a nonyloxypentyl group (each isomer), a nonyloxyhexyl group (each isomer), a nonyloxyheptyl group (each isomer), a nonyloxyoctyl group (each isomer), a nonyloxynonyl group (each isomer), a nonyloxydecyl group (each isomer), a nonyloxyundecyl group (each isomer), a decyloxymethyl group (each isomer), a decyloxyethyl group (each isomer), a decyloxypropyl group (each isomer), a decyloxybutyl group (each isomer), a decyloxypentyl group (each isomer), a decyloxyhexyl group (each isomer), a decyloxyheptyl group (each isomer), a decyloxyoctyl group (each isomer), a decyloxynonyl group (each isomer), a decyloxydecyl group (each isomer), an undecyloxymethyl group (each isomer), an undecyloxyethyl group (each isomer), an undecyloxypropyl group (each isomer), an undecyloxybutyl group (each isomer), an undecyloxypentyl group (each isomer), an undecyloxyhexyl group (each isomer), an undecyloxyheptyl group (each isomer), an undecyloxyoctyl group (each isomer), an undecyloxy nonyl group (each isomer), a dodecyloxymethyl group (each isomer), a dodecyloxyethyl group (each isomer), a dodecyloxypropyl group (each isomer), a dodecyloxybutyl group (each isomer), a dodecyloxypentyl group (each isomer), a dodecyloxyhexyl group (each isomer), a dodecyloxyheptyl group (each isomer), a dodecyloxyoctyl group (each isomer), a tridecyloxymethyl group (each isomer), a tridecyloxyethyl group (each isomer), a tridecyloxypropyl group (each isomer), a tridecyloxybutyl group (each isomer), a tridecyloxypentyl group (each isomer), a tridecyloxyhexyl group (each isomer), a tridecyloxyheptyl group (each isomer), a tetradecyloxymethyl group (each isomer), a tetradecyloxyethyl group (each isomer), a tetradecyloxypropyl group (each isomer), a tetradecyloxybutyl group (each isomer), a tetradecyloxypentyl group (each isomer), a tetradecyloxyhexyl group (each isomer), a pentadecyloxymethyl group, a pentadecyloxyethyl group (each isomer), a pentadecyloxypropyl group (each isomer), a pentadecyloxybutyl group (each isomer), a pentadecyloxypentyl group (each isomer), a hexadecyloxymethyl group (each isomer), a hexadecyloxyethyl group (each isomer), a hexadecyloxypropyl group (each isomer), a hexadecyloxybutyl group (each isomer), a heptadecyloxymethyl group (each isomer), a heptadecyloxyethyl group (each isomer), a heptadecyloxypropyl group (each isomer), an octadecyloxymethyl group (each isomer), and an octadecyloxyethyl group (each isomer).

Examples of the aryl group having an alkyl group as a substituent include a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), and a dibutylhexylphenyl group (each isomer).

Examples of the aryl group having an alkoxy group as a substituent, that is, examples of the alkoxyaryl group include a methoxyphenyl group (each isomer) and an ethoxyphenyl group (each isomer).

Among the above, $R^{311}$ and $R^{312}$ are preferably a substituted or unsubstituted aryl group.

In addition, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is more preferable.

In addition, a phenyl group, a methoxyphenyl group (each isomer), or an ethoxyphenyl group (each isomer) is still more preferable.

Examples of the preferred carbonic acid ester (III-1) include diphenyl carbonate, bis(2-methoxyphenyl) carbonate, and bis(2-ethoxyphenyl) carbonate.

[Isocyanate compound]

As the isocyanate compound, for example, a compound represented by General Formula (VII) (hereinafter, may be referred to as "isocyanate compound (VII)") is an exemplary example.

$$R^{71}\left(-NCO\right)_{n71} \quad (VII)$$

(in General Formula (VII), $R^{71}$ and n71 are each the same as $R^{51}$ and n51 described above)

In a case where $R^{71}$ is an aliphatic hydrocarbon group, specific examples of the isocyanate (VII) include aliphatic diisocyanates, aliphatic triisocyanates, and substituted cyclic aliphatic polyisocyanates.

Examples of the aliphatic diisocyanates include diisocyanatoethane, diisocyanatopropane (each isomer), diisocyanatobutane (each isomer), diisocyanatopentane (each isomer), diisocyanatohexane (each isomer), diisocyanatodecane (each isomer), isophorone diisocyanate (each isomer), and dicyclohexylmethane diisocyanate (each isomer).

Examples of the aliphatic triisocyanates include triisocyanatohexane (each isomer), 4-isocyanatomethyl-1,8-octamethylenediisocyanate, triisocyanatononane (each isomer), and triisocyanatodecane (each isomer).

Examples of the substituted cyclic aliphatic polyisocyanates include diisocyanatocyclobutane (each isomer), diisocyanatocyclohexane (each isomer), 3-isocyanatomethyl-3,5,5-trimethylcyclohexylisocyanate (at least one isomer of a cis form and a trans form), and methylenebis(cyclohexylisocyanate) (each isomer).

In a case where $R^{71}$ is an aromatic group, specific examples of the isocyanate (VII) include aromatic diisocyanates and aromatic triisocyanates.

Examples of the aromatic diisocyanates include diisocyanatobenzene (each isomer), diisocyanatotoluene (each isomer), bis(isocyanatophenyl)methane (each isomer), diisocyanatomesitylene (each isomer), diisocyanatobiphenyl (each isomer), diisocyanatodibenzyl (each isomer), bis(isocyanatophenyl)propane (each isomer), bis(isocyanatophenyl)ether (each isomer), bis(isocyanatophenoxyethane) (each isomer), diisocyanatoxylene (each isomer), diisocyanatoanisole (each isomer), diisocyanatophenethol (each isomer), diisocyanatonaphthalene (each isomer), diisocyanatomethylbenzene (each isomer), diisocyanatomethylpyridine (each isomer), diisocyanatomethylnaphthalene (each isomer), diisocyanatodiphenylmethane (each isomer), and tetramethylxylylene diisocyanate (each isomer).

Examples of the aromatic triisocyanates include triisocyanatobenzene (each isomer), triisocyanato-methylbenzene (each isomer), tris(isocyanatopropan-yl)benzene (each isomer), tris(isocyanatopropan-yl)-methylbenzene (each isomer), tris(isocyanatomethyl)-methylbenzene (each isomer), and ((isocyanato-phenylene)bis(methylene))bis(isocyanatebenzene) (each isomer).

In a case where $R^{71}$ is an aromatic group, reactivity of the isocyanate group directly bonded to the aromatic group is increased by an electron-withdrawing effect of the aromatic group. Furthermore, the reactivity is further enhanced by mutual electron-withdrawing action as the number of isocyanate groups directly bonded to the same aromatic compound increases. From the viewpoint of reactivity, the isocyanate group with respect to $R^{71}$ is preferably 2 or more and more preferably 3 or more. On the other hand, in a case where the reactivity of the isocyanate is high, a reaction with water, between isocyanates, and with other impurities is caused, and the stability of the compound at room temperature and during heating is low. From the viewpoint of stability of the isocyanate compound, the number of isocyanate groups directly bonded to $R^{71}$ is preferably 4 or less, and more preferably 3 or less.

Specific examples of the isocyanate (VII) in a case where the aliphatic hydrocarbon group or the aromatic group as $R^{71}$ has 1 or more and 4 or less ester groups or a nitrogen atom include 2-isocyanatoethyl acrylate, 2-isocyanatoethyl 2-methylacrylate, 2-isocyanatopropyl acrylate, 2-isocyanatopropyl 2-methylacrylate, 3-isocyanatopropyl acrylate, 3-isocyanatopropyl 2-methylacrylate, 4-isocyanatobutyl acrylate, 4-isocyanatobutyl 2-methylacrylate, 5-isocyanatopentyl acrylate, 5-isocyanatopentyl 2-methylacrylate,acrylic acid-6-isocyanato-hexyl ester, 2-methyl-acrylic acid-6-isocyanato-hexyl ester, acrylic acid-8-isocyanato-octyl ester, 2-methyl-acrylic acid-8-isocyanato-octyl ester, acrylic acid-10-isocyanato-decyl ester, 2-methyl-acrylic acid-10-isocyanato-decyl ester, acrylic acid-11-isocyanato-undecyl ester, 2-methyl-acrylic acid-11-isocyanato-undecyl ester, acrylic acid-12-isocyanato-dodecyl ester, 2-methyl-acrylic acid-12-isocyanato-dodecyl ester, lysine methyl ester diisocyanate, lysine ethyl ester diisocyanate, 2-isocyanatoethyl-2,5-diisocyanatopentanoate, 2-isocyanatoethyl-2,6-diisocyanatohexanoate, bis(2-isocyanatoethyl)-2-isocyanatobutanedioate, bis(2-isocyanatoethyl)-2-isocyanatopentanedioate, tris(2-isocyanatoethyl) hexane-1,3,6-tricarboxylate, trisisocyanatethylamine, and trisisocyanatopropylamine.

The isocyanate compound obtained by the production method according to the present embodiment can be suitably used as a raw material for producing a polyurethane foam, a coating material, an adhesive, or the like. According to the production method according to the present embodiment, the isocyanate compound can be produced with a high yield without using the highly toxic phosgene.

[Carbonyl compound (I)]

The carbonyl compound (I) is a by-product by-produced in a case where the N-substituted carbamate compound of the step (2) is thermally decomposed to produce an isocyanate compound. It is considered that the carbonyl compound

(I) is generated by a reaction in which the compound having an isocyanate terminal or an N-substituted carbamate terminal is reacted with the carbonic acid ester by intermolecular interactions under reaction conditions such as a temperature drop under thermal decomposition conditions and a reaction in which each terminal group is inserted into the carbonic acid ester skeleton proceeds. In the production method according to the present embodiment, the carbonyl compound (I) acts as a good solvent for a modified product of the isocyanate compound, and the carbonyl compound (I) acts as a terminal sealing agent to suppress an increase in molecular weight of the modified product of the isocyanate compound, whereby it is possible to suppress the generation of a solid matter and an increase in liquid viscosity in the generation system of the isocyanate compound due to the thermal decomposition reaction of the carbamate compound. In addition, the carbonyl compound (I) has a higher boiling point than the isocyanate compound and has fewer cross-linking points than the modified product of the isocyanate compound. Therefore, the carbonyl compound (I) can also act as a solvent during the purification of the isocyanate compound. Alternatively, the carbonyl compound (I) is bonded to a functional group such as a carbodiimide group, and the increase in molecular weight due to the bonding of the modified products of the isocyanate to each other is prevented. These actions make it possible to improve operability in the purification of the isocyanate compound and to recover the isocyanate compound at a high yield.

As the carbonyl compound (I), for example, a compound represented by General Formula (I) is an exemplary example.

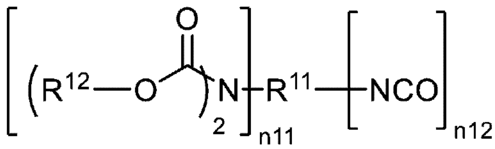

(I)

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less)

($R^{11}$)

$R^{11}$ is an (n11 + n12)-valent organic group, that is, a di- or higher and octa- or lower valent organic group. Among these, $R^{11}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

Specific examples of $R^{11}$ include the same groups as those described as the organic group in $R^{51}$ above.

Among these, $R^{11}$ is preferably a group represented by any one of Formulae (Ia-1) to (Ia-28), and more preferably a group represented by Formula (Ia-1), (Ia-2), (Ia-3), (Ia-14), (Ia-18), or (Ia-19). In each formula, a wavy line represents a bonding site.

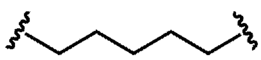

(Ia-1)

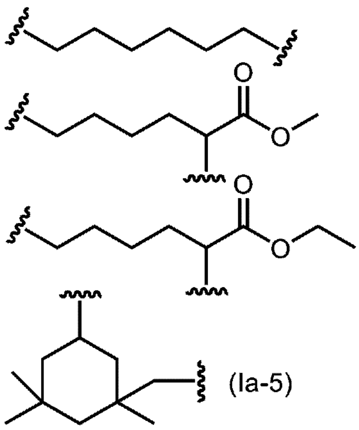

(Ia-2)

(Ia-3)

(Ia-4)

(Ia-5)

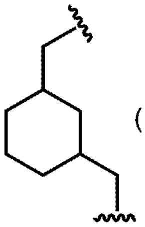

(Ia-6)

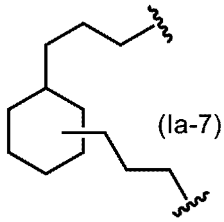

(Ia-7)

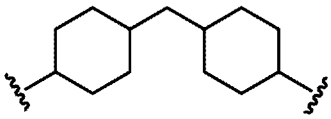

(Ia-8)

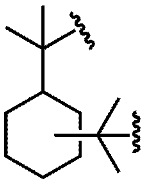

(Ia-9)

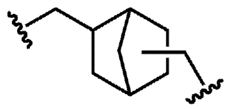

(Ia-10)

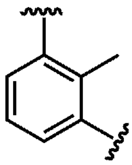

(Ia-11)

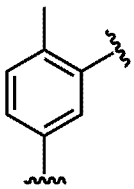

(Ia-12)

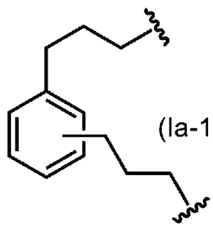

(Ia-13)

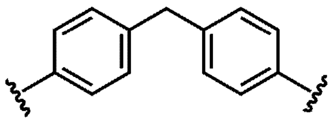

(Ia-14)

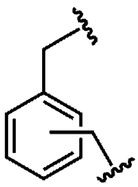

(Ia-15)

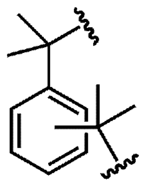

(Ia-16)

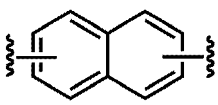

(Ia-17)

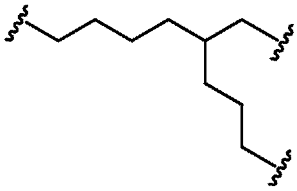
(Ia-18)

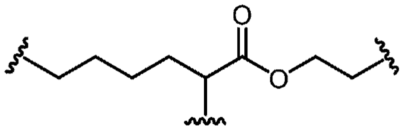
(Ia-19)

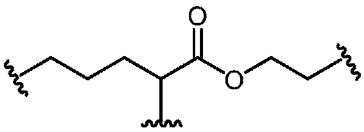
(Ia-20)

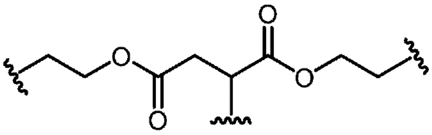
(Ia-21)

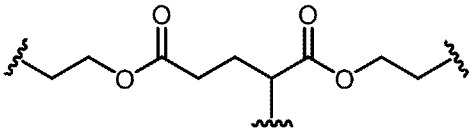
(Ia-22)

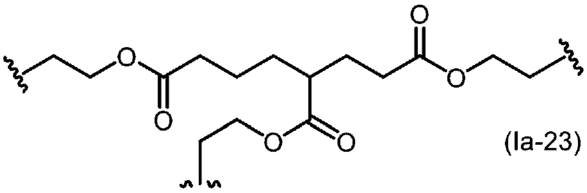
(Ia-23)

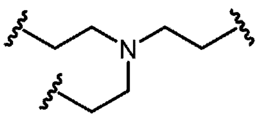
(Ia-24)

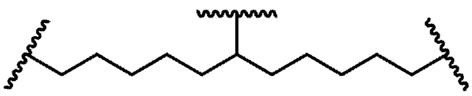
(Ia-25)

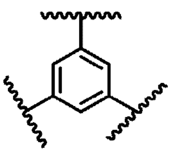
(Ia-26)

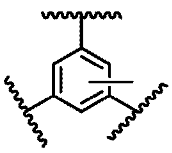
(Ia-27)

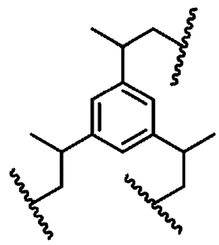
(Ia-28)

($R^{12}$)

$R^{12}$ is a monovalent organic group, and is preferably an aliphatic hydrocarbon group having 1 or more and 20 or less carbon atoms or an aromatic hydrocarbon group having 6 or more and 20 or less carbon atoms, each of which may include an oxygen atom.

Specific examples of $R^{12}$ include the same groups as those described in $R^{52}$ above.

(n11 and n12)

n11 represents the number of carbamate groups, and is an integer of 1 or more and 8 or less.

n12 represents the number of isocyanate groups, and is an integer of 0 or more and 7 or less.

The sum of n11 and n12 is an integer of 2 or more and 8 or less, preferably an integer of 2 or more and 6 or less, more preferably an integer of 2 or more and 5 or less, and still more preferably an integer of 3 or more and 4 or less. In a case where the sum of n11 and n12 is equal to or more than the above-described lower limit value, the standard boiling point also increases as the molecular weight of the carbonyl compound increases, and the separation from the isocyanate becomes easier. On the other hand, in a case of being equal to or less than the above-described upper limit value, the modification reaction with the highly reactive isocyanate group of the isocyanate compound is further suppressed, and the fixation or the sticking to the device can be further reduced.

Examples of the preferred carbonyl compound (I) include compounds represented by Formulae (I-1) to (I-17b). In addition, a substituent R' in Formulae (1-1) to (I-17b) is preferably a phenyl group, a (o-, m-, or p-methyl)phenyl group, an ethyl group, a propyl group (each isomer), or a butyl group (each isomer).

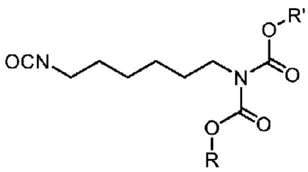
(I-1)

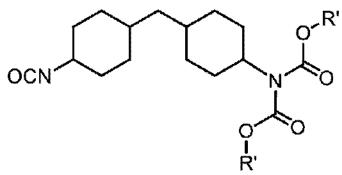
(I-2)

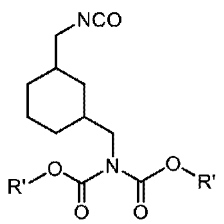
(I-3)

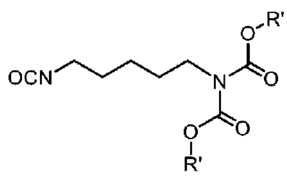
(I-4)

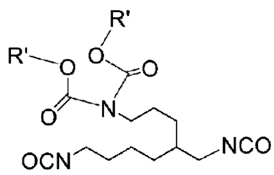
(I-5a)

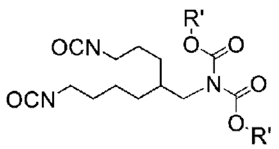
(I-5b)

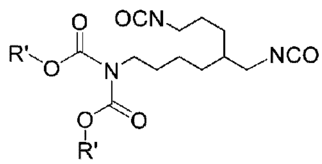
(I-5c)

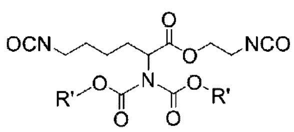
(I-6a)

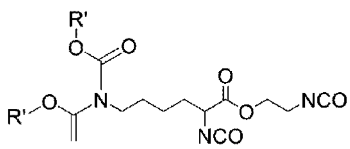
(I-6b)

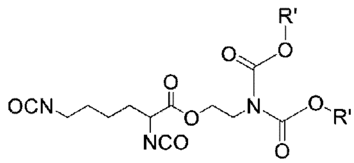
(I-6c)

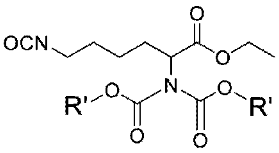

( I - 7a)

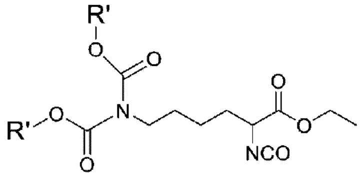

( I - 7b)

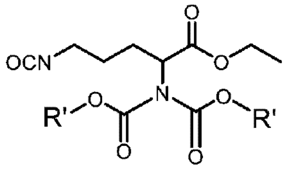

( I-17a)

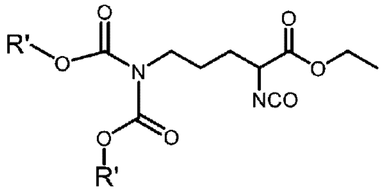

(I-17b)

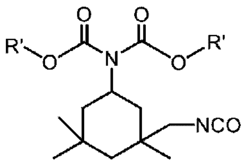

( I -8a )

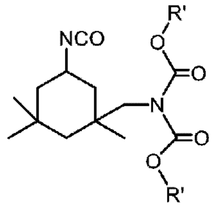

( I -8b )

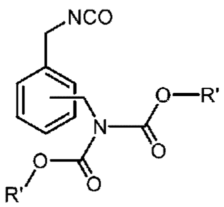

( I -9 )

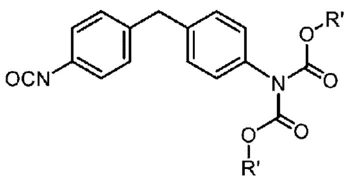

( I -10 )

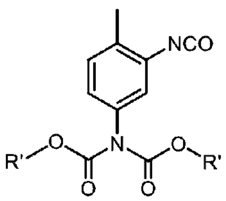

( I -11a )

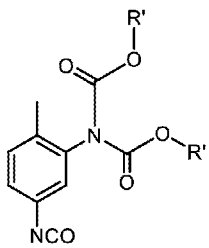

( I -11b )

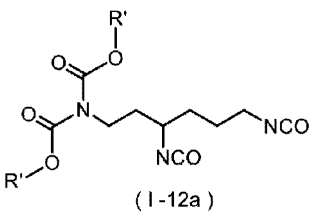

( I -12a )

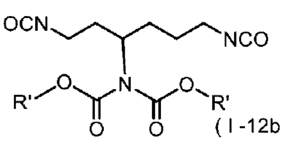

( I -12b )

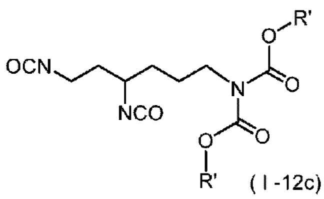

( I -12c)

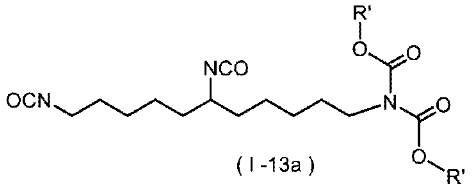

( I -13a )

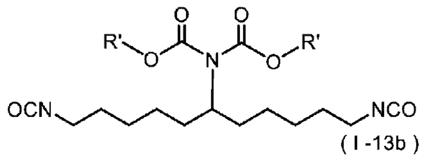

( I -13b )

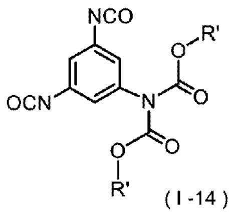

( I -14 )

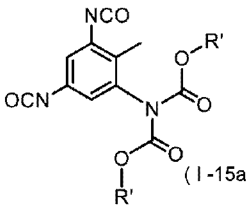

( I -15a )

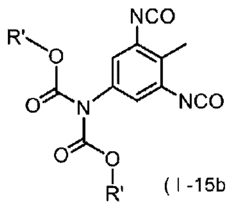

( I -15b )

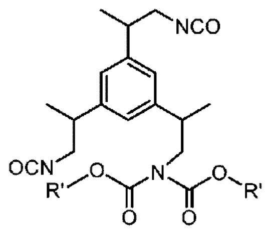

( I -16 )

«Method for producing carbamate compound»

The method for producing a carbamate compound according to the present embodiment includes a reaction step of reacting a primary amine compound, a carbonic acid derivative, and a hydroxy compound in the presence of, as a catalyst, an amine compound having no active hydrogen to obtain a carbamate compound.

In the production method according to the present embodiment, the reaction for producing the carbamate compound (specifically, the N-substituted carbamate) is an equilibrium reaction, but a time required for reaching the equilibrium can be shortened by using the amine compound having no active hydrogen as a catalyst. As a result, preferable effects such as a reduction in the size of the device and an improvement in the selectivity of the carbamate compound due to a reduction in side reactions are obtained.

In addition, since the amine compound having no active hydrogen is a non-metal catalyst, it can be easily separated from the reaction mixture by selecting an amine compound having an appropriate vapor pressure, and the modification at the time of obtaining the isocyanate compound by the thermal decomposition of the carbamate compound can be suppressed.

In the production method according to the present embodiment, any amine compound described later may be used as the catalyst as long as amine compound does not have active hydrogen, but in a case where the vapor pressure of the catalyst is higher than the vapor pressure of the hydroxy compound under the reaction conditions of the reaction step, the catalyst can be easily separated and recovered from the reaction solution containing the hydroxy compound and the N-substituted carbamate. On the other hand, in a case where the vapor pressure of the catalyst is lower than the vapor pressure of the hydroxy compound under the reaction conditions of the reaction step, the catalyst concentration in the liquid phase is kept high, which is effective for promoting the reaction.

Each step of the method for producing a carbamate compound according to the present embodiment will be described in detail below.

<Reaction step: carbamylation reaction step>

In the reaction step, a primary amine compound, a carbonic acid derivative, and a hydroxy compound are reacted with each other in the presence of, as a catalyst, an amine compound having no active hydrogen to obtain a carbamate compound.

The reaction step preferably includes the following step (X1) and step (X2):

a carbamate synthesis step (step (X1)) of generating an N-substituted carbamate by a carbamylation reaction of a primary amine compound, a carbonic acid derivative, and a hydroxy compound in the presence of an amine compound having no active hydrogen as a catalyst, and recovering a gas-phase component which contains a compound having a carbonyl group derived from the carbonic acid derivative and the hydroxy compound, and may contain one or more compounds selected from the group consisting of an active hydrogen compound derived from the carbonic acid derivative and the amine compound having no active hydrogen; and

a condensation step (step (X2)) of condensing the gas-phase component with a condenser.

Hereinafter, each of these steps will be described.

[Step (X1): carbamate synthesis step]

The step (X1) is a carbamate synthesis step of generating a carbamate compound by a carbamylation reaction of, as raw materials, a primary amine compound, a carbonic acid derivative, and a hydroxy compound in the presence of an amine compound having no active hydrogen as a catalyst, and recovering a gas-phase component which contains the hydroxy compound and a compound having a carbonyl group derived from the carbonic acid derivative, and may contain one or more compounds selected from the group consisting of an active hydrogen compound derived from the carbonic acid derivative and the amine compound having no active hydrogen.
The step (X1) can be largely divided into two methods, that is, a method (method (1A)) for producing an N-substituted carbamate by "simultaneously" reacting the primary amine compound, the carbonic acid derivative, and the hydroxy compound in the presence of at least one amine compound having no active hydrogen as a catalyst; and a method (method (1B)) including a step (b1) of reacting the primary amine compound and the carbonic acid derivative in the presence or absence of the amine compound having no active hydrogen as a catalyst to produce a carbonyl compound having a group derived from the primary amine compound, and a step (b2) of reacting the carbonyl compound and the hydroxy compound in the presence of the amine compound having no active hydrogen to produce an N-substituted carbamate.
The "carbonyl compound having a group derived from the primary amine compound" is a compound which is generated by a reaction between the primary amine compound and the carbonic acid derivative, and is represented by General Formula (XII).

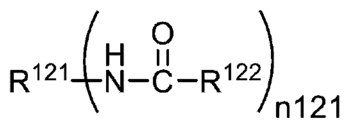
( XII )

(in the general formula, $R^{121}$ is a group derived from the primary amine compound, that is, the same as $R^{21b}$ in General Formula (IIb) described later, $R^{122}$ is a group derived from the carbonic acid derivative, that is, the same as $R^{321}$, $R^{322}$, $R^{323}$, or $R^{324}$ in General Formula (III-2) described later, $R^{331}$ in General Formula (III-3) described later, or $R^{311b}$ or $R^{312b}$ in General Formula (III-1b) described later, and n121 is the same as n21b in General Formula (IIb) described later)
In the production method according to the present embodiment, the method (1A) and the method (1B) may be performed in combination.

(Method (1A))

The term "simultaneously" in the method (1A) means that, in the method (1B), the step of producing the N-substituted carbamate is divided into two steps, whereas, in the method (1A), the step is not divided; and does not necessarily mean that the primary amine compound, the carbonic acid derivative, and the hydroxy compound react exactly at the same time.
Reaction conditions for producing the N-substituted carbamate by reacting the primary amine compound, the carbonic acid derivative, and the hydroxy compound in the presence of the catalyst vary depending on the compounds to be reacted, but the amount of the hydroxy compound used is usually an amount in a range of 1 time or more and 500 times or less in terms of a stoichiometric ratio with a molar ratio of a hydroxyl group of the hydroxy compound, with respect to the molar amount of an amino group of the primary amine compound used. In a case where the amount of the hydroxy compound used is small, a carbonyl compound or the like, which is complicatedly substituted, is easily generated. Therefore, it is preferable to use a large excess amount of the hydroxy compound, but in consideration of the size of the reactor, the amount of the hydroxy compound used is preferably in a range of 1 time or more and 200 times or less, more preferably in a range of 1.5 times or more and 100 times or less, and still more preferably in a range of 2 times or more and 60 times or less.
The amount of the carbonic acid derivative used (molar amount) is usually in a range of 1 time or more and 100 times or less in terms of a stoichiometric ratio with respect to the molar amount of the amino group of the primary amine compound. In a case where the amount of the carbonic acid derivative used is small, a carbonyl compound or the like, which is complicatedly substituted, is easily generated. Therefore, it is preferable to use an excessive amount of the carbonic acid derivative, but in a case where an excessively excessive amount of the carbonic acid derivative is used, the complicatedly substituted carbonyl compound is likely to be generated, or an unreacted carbonic acid derivative may remain. Therefore, the amount is preferably 1.0 time or more and 10 times or less, more preferably 1.0 times or more and 5 times or less, and still more preferably 1.05 times or more and 2 times or less.

The amount of the amine compound having no active hydrogen used as the catalyst (molar amount) may be appropriately adjusted within a range in which the reaction proceeds well, but for example, the amount can be 0.0001 times or more and 100 times or less, and is preferably 0.0001 times or more and 30 times or less, with respect to the molar amount of the amino group of the primary amine compound.

The amine compound having no active hydrogen used as the catalyst has a large effect of promoting the carbamylation reaction as a pKa of a conjugate acid is large, but in a case of being used in a large amount, the denaturation reaction may be promoted and the carbamate yield may be reduced. In a case where a strong base having a pKa of 12 or more is used as the conjugate acid, the preferred use amount (molar amount) is 0.1 times or less, more preferably 0.05 times or less, and still more preferably 0.01 times or less with respect to the molar amount of the amino group of the primary amine compound.

On the other hand, it is necessary to use a large amount of a base in which a pKa of a conjugate acid is 7 or more and 12 or less in order to obtain the same acceleration effect as in the case of using the above-described strong base. Since the effect of accelerating the modification reaction is smaller than that of the strong base, the above-described base may be used in a large amount, and the preferred range of the amount of the base used is 0.5 times or more and 30 times or less, more preferably 0.5 times or more and 10 times or less, and still more preferably 1 time or more and 3 times or less, with respect to the molar amount of the amino group of the primary amine compound. In addition, in a case where a weak base having a pKa of a conjugate acid of less than 7 is used, the preferred range of the amount of the weak base used is 0.5 times or more and 30 times or less, preferably 1 time or more and 10 times or less, with respect to the molar amount of the amino group of the primary amine compound.

The reaction temperature is preferably in a range of 100°C or higher and 350°C or lower, more preferably in a range of 120°C or higher and 320°C or lower, and still more preferably in a range of 140°C or higher and 300°C or lower, depending on the reactivity of the primary amine compound, the carbonic acid derivative, and the hydroxy compound used. In a case where the reaction temperature is equal to or higher than the above-described lower limit value, the reaction is further accelerated, and it is possible to more effectively suppress an increase in the number of carbonyl compounds which are complicatedly substituted. On the other hand, in a case where the reaction temperature is equal to or lower than the above-described upper limit value, it is possible to more effectively suppress the decomposition of the carbonic acid derivative and the accompanying side reaction, and it is possible to more effectively suppress the decomposition reaction, modification reaction, and the like of the N-substituted carbamate which is a dehydrogenation-modified or generated product of the hydroxy compound.

The reaction pressure varies depending on the composition of the reaction system, the reaction temperature, the reaction device, and the like, and the reaction pressure can be reduced, carried out at normal pressure, or increased. However, it is preferable that the reaction pressure is usually in a range of 0.01 kPa or more and 10 MPa or less (absolute pressure).

In the step (X1), the reaction for producing the N-substituted carbamate is mainly carried out in a liquid phase in many cases. Therefore, it is preferable that the hydroxy compound and the catalyst are present as liquid-phase components under the reaction conditions. On the other hand, as will be described later, the hydroxy compound and the compound having a carbonyl group derived from the carbonic acid derivative are introduced into the condenser as gas-phase components and are condensed in the condenser. Therefore, it is preferable that the hydroxy compound is present as a gas-phase component under the reaction conditions. Accordingly, the reaction conditions are set such that the catalyst is mainly present as the liquid-phase component, and the hydroxy compound is partially present as the liquid-phase component and partially present as the gas-phase component. In a case where a hydroxy composition constituting a plurality of hydroxy compounds is used, the reaction conditions are set such that at least one hydroxy compound is present as the liquid-phase component. Since such reaction conditions (reaction temperature and pressure) are closely related to the properties of the hydroxy compound and the catalyst to be used, particularly, the correlation between the temperature and the vapor pressure, the properties of the hydroxy compound and the catalyst to be used (the correlation between the temperature and the vapor pressure) are measured or investigated in advance and used as an index for determining the reaction conditions. Incidentally, it is a common sense for those skilled in the art that the correlation between the temperature and the vapor pressure of the substance is also greatly affected by the purity of the substance, the coexisting compound, and the amount thereof, and it is also clear that, in a case of setting the reaction conditions, not only the properties of the above-described hydroxy compound and the catalyst (the correlation between the temperature and the vapor pressure) but also the coexisting compound and the amount thereof should be taken into account.

The reaction of producing the N-substituted carbamate from the primary amine compound, the carbonic acid derivative, and the hydroxy compound is an equilibrium reaction, but in a case where one or more selected from the group consisting of a urea compound and N-unsubstituted carbamate are used as the carbonic acid derivative, the reaction is greatly biased toward the original system. Therefore, in order to increase the yield of the N-substituted carbamate, it is necessary to carry out the reaction while removing the by-product active hydrogen compound from the system as much as possible. The active hydrogen compound is removed such that the concentration of the active

hydrogen compound in the reaction solution is preferably 1,000 ppm by mass or less, more preferably 300 ppm by mass or less, still more preferably 100 ppm by mass or less, and particularly preferably 10 ppm by mass or less. Examples of the method include a reactive distillation method, a method using an inert gas, a membrane separation method, and an adsorption separation method. For example, the reactive distillation method is a method of separating an active hydrogen compound sequentially generated under a reaction condition into a gaseous phase by distillation. In order to increase the distillation efficiency of the active hydrogen compound, the distillation can also be carried out under boiling of the solvent or the hydroxy compound. In addition, the method using an inert gas is a method of separating an active hydrogen compound sequentially generated under a reaction from a reaction system by accompanying the active hydrogen compound in a gaseous state with an inert gas. As the inert gas, for example, nitrogen, helium, argon, carbon dioxide, methane, ethane, propane, or the like is used alone or in combination, and a method of introducing the inert gas into the reaction system is preferable. Examples of the adsorbent used in the adsorption separation method include adsorbents that can be used under the temperature conditions under which the reaction is carried out, such as silica, alumina, various zeolites, and diatomaceous earth. The method of removing these active hydrogen compounds to the outside of the system may be performed alone or in combination of a plurality of methods.

Examples of the by-product active hydrogen compound include a raw material primary amine compound generated from urea or a substituted urea, an amine compound other than the amine compound having no active hydrogen as the catalyst, and a hydroxy compound other than a solvent generated from the carbonic acid ester and the amine compound.

The reaction time (in the case of a continuous reaction, the residence time) varies depending on the formulation of the reaction system, the reaction temperature, the method of removing the active hydrogen compound, the reaction device, the reaction pressure, and the like, but is usually 0.01 hours or more and 100 hours or less. The reaction time can also be determined by the amount of the generated N-substituted carbamate, which is the target compound. For example, the reaction may be stopped after the reaction solution is sampled, the content of the N-substituted carbamate in the reaction solution is quantified, and it is confirmed that the reaction solution is produced with a yield of 10% by mass or more with respect to the mass of the raw material primary amine compound used, or the reaction may be stopped after it is confirmed that the yield is 90% by mass or more.

In the carbamylation reaction, it is not always necessary to use a reaction solvent, but a suitable solvent is used for the purpose of facilitating the reaction operation, for example, alkanes such as pentane (each isomer), hexane (each isomer), heptane (each isomer), octane (each isomer), nonane (each isomer), and decane (each isomer); aromatic hydrocarbons and alkyl-substituted aromatic hydrocarbons such as benzene, toluene, xylene (each isomer), ethylbenzene, diisopropylbenzene (each isomer), dibutylbenzene (each isomer), mesitylene, paracymene, tetralin, and naphthalene; nitrile compounds such as acetonitrile and benzonitrile; aromatic compounds substituted with a halogen or nitro group such as chlorobenzene, dichlorobenzene (each isomer), bromobenzene, dibromobenzene (each isomer), chloronaphthalene, bromonaphthalene, nitrobenzene, and nitronaphthalene; polycyclic hydrocarbon compounds such as diphenyl, substituted diphenyl, diphenylmethane, terphenyl, anthracene, and dibenzyltoluene (each isomer); alicyclic hydrocarbons such as cyclohexane, cyclopentane, cyclooctane, and ethylcyclohexane; ketones such as methyl ethyl ketone and acetophenone; esters such as dibutyl phthalate, dihexyl phthalate, dioctyl phthalate, and benzyl butyl phthalate; ethers and thioethers such as tetrahydrofuran, 1,4-dioxane, 1,2-dimethoxyethane, diphenyl ether, and diphenyl sulfide; ketone compounds such as acetone and methyl ethyl ketone; ester compounds such as ethyl acetate and ethyl benzoate; sulfoxides such as dimethyl sulfoxide and diphenyl sulfoxide; and the like are suitably used as the reaction solvent.

Needless to say, the hydroxy compound used in an excessive amount in the reaction is also suitably used as the reaction solvent.

The carbamylation reaction is carried out in a system having a gas phase which contains the hydroxy compound and the compound having a carbonyl group derived from the carbonic acid derivative, and may contain one or more compounds selected from the group consisting of an active hydrogen compound derived from the carbonic acid derivative and the amine compound having no active hydrogen, and having a liquid phase for performing the carbamylation reaction. Depending on the reaction conditions, the carbamylation reaction may also be carried out in the gas phase, but most of the carbamylation reaction is carried out in the liquid phase. In this case, a liquid-phase capacity content in the reactor in which the carbamylation reaction is carried out is preferably 50% by volume or less with respect to the capacity of the gas phase. In a case where the carbamylation reaction is continuously carried out for a long period of time, there is a case in which a by-product in a polymer form is generated due to a change in operating conditions (temperature, pressure, and the like). However, in a case where the liquid-phase capacity content in the reactor is large, it is possible to avoid the adhesion and accumulation of such a by-product in a polymer form in the reactor. However, in a case where one or more compounds selected from the group consisting of urea compounds and N-unsubstituted carbamates are used as the carbonic acid derivative, when the liquid-phase capacity content is too large, the removal efficiency of the by-product active hydrogen compound is deteriorated, and the yield of the N-substituted carbamate is lowered. Therefore, the liquid-phase capacity content is preferably 50% by volume or less, more preferably 30% by volume or less, and still more

preferably 20% by volume or less with respect to the capacity of the gas phase. The liquid-phase capacity content referred to herein represents a liquid capacity ratio with respect to a capacity of a gas phase in a case of a tank-type reactor, a stage below a feed stage (excluding a bottom portion of a column and a reboiler portion) in a case of a column-type reactor, and a thin-film evaporator in a case of a thin-film evaporator.

The reactor used when carrying out the carbamylation reaction is not particularly limited as long as it is a reactor equipped with a condenser, and a known reactor can be used. However, one or more reactors selected from the group consisting of a tank type and a tower type equipped with a condenser are preferably used. A material of the reactor is not particularly limited, and a known material can be used. For example, a glass, stainless steel, carbon steel, Hastelloy material, a material on which a glass lining is applied, or a material on which a Teflon (registered trademark) coating is applied can also be used. Among these, SUS304, SUS316, SUS316L, and the like are inexpensive and can be preferably used. As necessary, known process equipment such as measuring instruments, for example, a flowmeter and a thermometer, a reboiler, a pump, and a condenser may be added. The heating may be performed by a known method such as steam or a heater, and the cooling may be performed by a known method such as natural cooling, cooling water, or brine.

In the carbamylation reaction, it is possible to add a step as described below as necessary. Examples of the step which can be added include a step of removing the generated active hydrogen compound, a step of purifying the primary amine compound, a step of dissolving the carbonic acid derivative in the hydroxy compound, a step of dissolving the hydroxy compound, a step of separating or purifying the hydroxy compound, a step of purifying N-substituted carbamate from the generated reaction solution, a step of incinerating or disposing of by-products, and the like, and a step or a device within the range that can be assumed by those skilled in the art may be added.

(Method (1B))

Hereinafter, the method (1B) will be described.

1. Step (b1)

The amount of the hydroxy compound used (molar amount) and the amount of the amine compound having no active hydrogen used as a catalyst (molar amount) can be the same as the amounts used in the above-described method (1A).

The amount of the carbonic acid derivative used (molar amount) is usually in a range of 1 time or more and 100 times or less in terms of a stoichiometric ratio with respect to the molar amount of the amino group of the primary amine compound. In a case where the amount of the carbonic acid derivative used is small, a carbonyl compound or the like, which is complicatedly substituted, is easily generated. Therefore, it is preferable to use an excessive amount of the carbonic acid derivative. However, in a case where an excessively excessive amount of the carbonic acid derivative is used, the complicatedly substituted carbonyl compound is likely to be generated, or an unreacted carbonic acid derivative may remain. Therefore, the amount is preferably 1.0 time or more and 10 times or less and more preferably 1.1 times or more and 5 times or less.

The reaction temperature in the step (b1) can be usually in a range of 30°C or higher and 250°C or lower. In order to increase the reaction rate, a high temperature is preferable. On the other hand, in a case where a reaction (for example, a decomposition reaction of the carbonic acid derivative, a complex side reaction caused by the decomposition reaction, or the like) that is not preferred at the high temperature occurs, a complexly substituted carbonyl compound may be produced. Therefore, the temperature is preferably in a range of 50°C or higher and 200°C or lower and more preferably in a range of 70°C or higher and 180°C or lower. In order to keep the reaction temperature constant, a known cooling device or heating device may be installed in the reactor in which the step (b1) is performed.

The reaction pressure varies depending on the kinds of the compounds to be used, the formulation of the reaction system, the reaction temperature, the reaction device, and the like, it is usually in a range of 0.01 kPa or more and 10 MPa or less (absolute pressure), preferably in a range of 0.1 kPa or more and 5 MPa or less (absolute pressure).

The reaction time (in a case of the continuous method, the residence time) is not particularly limited, and is usually 0.001 hours or more and 100 hours or less, preferably 0.01 hours or more and 80 hours or less, and more preferably 0.1 hours or more and 50 hours or less. In addition, the reaction solution can be collected, and for example, the reaction can be terminated by confirming that a desired amount of the carbonyl compound having a group derived from the primary amine compound is generated by liquid chromatography. The step (b1) is a step of producing the carbonyl compound, but in the step (b1), in a case where a large amount of an amino group derived from the unreacted primary amine compound is present, a compound having a ureylene group or the like is produced in the step (b2) performed after the step (b1), and the amount of N-substituted carbamate produced is reduced, and the compound adheres to and is solidified in a reactor in many cases. Therefore, in the step (b1), it is preferable to reduce the amount of the amino group derived from the primary amine compound as much as possible. Specifically, the reaction is preferably continued until the ratio of the number of moles of the amino group derived from the organic amine to the number of

moles of the carbonyl group constituting the carbonyl compound derived from the primary amine compound is preferably 0.25 or less, more preferably 0.1 or less, and still more preferably 0.05 or less.

The hydroxy compound used in the step (b1) may be exactly the same as the hydroxy compound used in the step (b2), may be partially the same, or may be different. Among these, it is preferable that the hydroxy compound used in the step (b1) is the same as or partially the same as the hydroxy compound used in the step (b2) because the operation becomes easy. As will be described later, it is more preferable that the reaction of the step (b1) is carried out in the presence of an aromatic hydroxy compound, or the aromatic hydroxy compound is added after the reaction of the step (b 1) is carried out in the presence of an alcohol or an aromatic hydroxy compound.

The reaction solvent shown here can be used in any amount, but in a case where an alcohol is used as the reaction solvent, the amount of the alcohol used (molar amount) can be used in a range of more than 1 time and less than 100 times in terms of a stoichiometric ratio with respect to the molar amount of the amino group of the primary amine compound. In order to improve the fluidity of the reaction solution and efficiently proceed the reaction, it is preferable to use an excess of alcohol with respect to the amino group of the primary amine compound. On the other hand, there is a disadvantage that the reactor becomes large when an excessive amount of alcohol is used. Therefore, the use amount (molar amount) can be used in a range of preferably more than 5 times and less than 50 times, and more preferably more than 8 times and less than 20 times in terms of a stoichiometric ratio, with respect to the molar amount of the amino group of the primary amine compound.

In addition, in a case where the aromatic hydroxy compound is used as the reaction solvent in the step (b1), the use amount (molar amount) thereof can be in a range of 1 time or more and less than 100 times in terms of a stoichiometric ratio, with respect to the molar amount of the amino group of the primary amine compound. In order to improve the fluidity of the reaction solution and efficiently proceed the reaction, it is preferable to use an excess of aromatic hydroxy compound with respect to the amino group of the primary amine compound. On the other hand, there is a disadvantage that the reactor becomes large when an excessive amount of aromatic hydroxy compound is used. Therefore, the use amount (molar amount) can be used in a range of preferably more than 2 times and less than 50 times, and more preferably more than 3 times and less than 20 times in terms of a stoichiometric ratio, with respect to the molar amount of the amino group of the primary amine compound.

Among the alcohol and aromatic hydroxy compound, the aromatic hydroxy compound is preferably used as the hydroxy compound in consideration of the solubility of the carbonyl compound having a group derived from the primary amine compound to be produced. For example, Japanese Unexamined Patent Application, First Publication No. H6-41045 (Reference Document 1) discloses that polyhexamethylene-urea generated by the reaction between urea and hexamethylenediamine is not easily dissolved in n-butanol. However, in this regard, the aromatic hydroxy compound has excellent solubility in various reaction products.

In a case where an aromatic hydroxy compound is used as the reaction solvent, the aromatic hydroxy compound may be used alone or may be used in a mixture with another solvent, and the amount of the aromatic hydroxy compound used is in the range of the above-described value. Even in a case where the step (b1) is carried out in the presence of the alcohol and then the aromatic hydroxy compound is added, the aromatic hydroxy compound is used within the above-described range. At that time, the amount of alcohol used in the reaction of the step (b1) is also the amount of alcohol according to the above-described stoichiometric ratio with respect to the molar amount of the amino group of the primary amine compound.

The reaction device used in carrying out the reaction of the step (b1) is not particularly limited, and a known reactor can be used. For example, a reactor known in the related art can be appropriately combined and used according to the reaction method and conditions, such as a stirring tank, a pressurized stirring tank, a reduced pressure stirring tank, a tower-type reactor, a distillation column, a filled column, a thin film evaporator, and a tubular reactor. In addition, the reaction may be a batch type or a continuous flow type, and a reaction device may be selected according to each reaction type. From the viewpoint of efficiently performing the reaction, the continuous flow type is preferable, and in a case where the raw material solution is caused to flow in a tube-shaped flow path (pipe) having a small inner diameter and the reaction is performed, the efficiency is often good. In that case, the thickness and length of the flow path are important, but can be appropriately determined by the production amount of the carbonyl compound having a group derived from the primary amine compound, the area of the heat conduction surface with respect to the volume inside the hollow, and the necessary retention time (reaction time). One flow path can also share the front portion thereof as a supply step and the rear portion thereof as a reaction step. In this case, a portion where the solution flowing through the flow path can reach the target temperature can be regarded as being used in the reaction step, and the other portion can be regarded as being used in the supply step.

A material of the reactor is not particularly limited, and a known material can be used. For example, a glass, stainless steel, carbon steel, Hastelloy material, a material on which a glass lining is applied, or a material on which a Teflon (registered trademark) coating is applied can also be used. Among these, SUS304, SUS316, SUS316L, and the like are inexpensive and can be preferably used. As necessary, known process equipment such as measuring instruments, for example, a flowmeter and a thermometer, a mechanism for holding pressure, a reboiler, a pump, and a

condenser may be added. The heating may be performed by a known method such as steam or a heater, and the cooling may be performed by a known method such as natural cooling, cooling water, or brine.

In the step (b1), it is possible to add a step as described below as necessary. Examples of the step that can be added include a step of removing a by-product active hydrogen compound, a step of purifying a primary amine compound, a step of dissolving a carbonic acid derivative in a hydroxy compound, a step of dissolving a hydroxy compound, a step of separating or purifying a hydroxy compound, a step of purifying a carbonyl compound having a group derived from a primary amine compound from the generated reaction solution, and a step of incinerating or disposing of a by-product, and the like, and a step or a device within the range that can be assumed by those skilled in the art may be added.

From the viewpoint of selectively producing a carbonyl compound having a group derived from a primary amine compound in the step (b1), it is preferable that the active hydrogen compound derived from the carbonic acid derivative is dissolved to some extent in the reaction solution. On the other hand, in the step (b2) described later, it is preferable that the active hydrogen compound is removed. Therefore, in a case where the reaction solution in the step (b1) is used as a raw material in the step (b2), it is also a preferable method to separate the active hydrogen compound from the reaction solution in advance. The separation method is not particularly limited, but for example, the vapor phase part and the liquid phase part can be rapidly separated by sending the reaction solution to a reduced pressure container, and the liquid phase part containing the carbonyl compound having a group derived from the primary amine compound can be recovered and used.

In a case where the reaction solvent is used in the step (b1), the reaction solvent may be removed from the reaction solution of the step (b1) before the step (b2) is performed, or the step (b2) may be performed as it is without removing the reaction solvent. In particular, it is preferable that the hydroxy compound used as the reaction solvent in the step (b1) is used as it is as a part of the hydroxy compound in the step (b2).

2. Step (b2)

The step (b2) is a step of reacting the carbonyl compound having a group derived from the primary amine compound, obtained in the step (b1), with a hydroxy compound to produce an N-substituted carbamate.

In a case where the hydroxy compound is used as the reaction solvent in the step (b1) and the hydroxy compound is the same as a hydroxy compound in the step (b2), the step (b2) can be performed as it is using the reaction solution obtained in the step (b1).

In a case where the reaction solvent in the step (b1) is different from the hydroxy composition in the step (b2), the step (b2) may be performed by adding a hydroxy compound to the reaction solution obtained in the step (b1). In addition, one or a plurality of kinds of hydroxy compounds may be added to the reaction solution obtained in the step (b 1), and the step (b2) may be performed after a part or the whole of the reaction solvent in the step (b1) is separated. After removing a part or all of the reaction solvent in the step (b1), the step (b2) may be performed after adding a new hydroxy compound. The hydroxy compound to be added here includes one or more hydroxy compounds selected from the group consisting of an alcohol represented by General Formula (Via) described later and an aromatic hydroxy compound represented by General Formula (VIb) described later. The method for separating the reaction solvent used in the step (b1) is not particularly limited, and a known method such as distillation separation, membrane separation, or extraction separation can be used, but distillation separation is preferable.

The hydroxy compound used in the step (b2) is preferably an aromatic hydroxy compound represented by General Formula (VIb) described later.

The catalyst may be added to the reaction solution of the step (b1) before the step (b2) is performed without using the catalyst in the step (b1), and the step (b2) may be performed.

The reaction conditions for producing an N-substituted carbamate by the reaction between the carbonyl compound having a group derived from the primary amine compound and the hydroxy compound in the step (b2) vary depending on the compounds to be reacted, but the amount of the hydroxy compound used (molar amount) is in a range of 1 time or more and 500 times or less in terms of a stoichiometric ratio of the number of moles of the hydroxy compound, with respect to the number of moles of the carbonyl group of the carbonyl compound to be used. In a case where the amount of the hydroxy compound is small, a carbonyl compound that is complexly substituted or a high-molecular-weight compound having a carbonyl bond in a molecule is likely to be generated. Therefore, it is preferable to use a large excess of the hydroxy compound. Among these, in consideration of the size of the reactor, the amount (molar amount) of the hydroxy compound used is preferably in a range of 1 time or more and 200 times or less, more preferably in a range of 1.5 times or more and 100 times or less, and still more preferably in a range of 2 times or more and 60 times or less in terms of a stoichiometric ratio of the number of moles of the hydroxy compound, with respect to the number of moles of the carbonyl group of the carbonyl compound to be used.

Depending on the compounds to be used, the reaction temperature is in a range of 40°C or higher and 380°C or lower, more preferably in a range of 50°C or higher and 320°C or lower, still more preferably in a range of 60°C or

higher and 300°C or lower, particularly preferably in a range of 80°C or higher and 300°C or lower, and most preferably in a range of 100°C or higher and 280°C or lower. In a case where the reaction temperature is equal to or higher than the above-described lower limit value, the reaction is further accelerated, and it is possible to more effectively suppress an increase in the number of carbonyl compounds which are complicatedly substituted. On the other hand, in a case where the reaction temperature is equal to or lower than the above-described upper limit value, it is possible to more effectively suppress the decomposition of the carbonic acid derivative and the accompanying side reaction, and it is possible to more effectively suppress the decomposition reaction, modification reaction, and the like of the N-substituted carbamate which is a dehydrogenation-modified or generated product of the hydroxy compound.

The reaction pressure varies depending on the formulation of the reaction system, the reaction temperature, the method of removing the active hydrogen compound derived from the carbonic acid derivative, the reaction device, and the like, but in consideration of industrial feasibility, it is usually in a range of 0.01 Pa or more and 10 MPa or less (absolute pressure), and preferably in a range of 0.1 Pa or more and 5 MPa or less (absolute pressure) and more preferably in a range of 0.1 Pa or more and 1.5 MPa or less (absolute pressure).

In the step (b2), the reaction for producing the N-substituted carbamate is mainly carried out in a liquid phase in many cases. Therefore, it is preferable that the hydroxy compound and the catalyst are present as liquid-phase components under the reaction conditions. On the other hand, as will be described later, in the step (X2), the hydroxy compound and the compound having a carbonyl group derived from the carbonic acid derivative are introduced into the condenser as gas-phase components and are condensed in the condenser. Therefore, it is preferable that the hydroxy compound is present as a gas-phase component under the reaction conditions. Accordingly, the reaction conditions are set such that the catalyst is mainly present as the liquid-phase component, and the hydroxy compound is partially present as the liquid-phase component and partially present as the gas-phase component. In a case where a plurality of hydroxy compounds are used, the reaction conditions are set such that at least one hydroxy compound is present as the liquid-phase component. Since such reaction conditions (reaction temperature and pressure) are closely related to the properties of the hydroxy compound and the catalyst to be used, particularly, the correlation between the temperature and the vapor pressure, the properties of the hydroxy compound and the catalyst to be used (the correlation between the temperature and the vapor pressure) are measured or investigated in advance and used as an index for determining the reaction conditions. Incidentally, it is a common sense for those skilled in the art that the correlation between the temperature and the vapor pressure of the substance is also greatly affected by the purity of the substance, the coexisting compound, and the amount thereof, and it is also clear that, in a case of setting the reaction conditions, not only the properties of the above-described hydroxy compound and the catalyst (the correlation between the temperature and the vapor pressure) but also the coexisting compound and the amount thereof should be taken into account.

As described above, the reaction of producing the N-substituted carbamate is an equilibrium reaction, but in a case where one or more selected from the group consisting of a urea compound and N-unsubstituted carbamate are used as the carbonic acid derivative, the reaction is greatly biased toward the original system. Therefore, in order to increase the yield of the N-substituted carbamate, it is necessary to carry out the reaction while removing the by-product active hydrogen compound from the system as much as possible. The active hydrogen compound is removed such that the concentration of the active hydrogen compound in the reaction solution is preferably 1,000 ppm by mass or less, more preferably 300 ppm by mass or less, still more preferably 100 ppm by mass or less, and particularly preferably 10 ppm by mass or less. Examples of the method include a reactive distillation method, a method using an inert gas, a membrane separation method, and an adsorption separation method. For example, the reactive distillation method is a method of separating an active hydrogen compound sequentially generated under a reaction condition into a gaseous phase by distillation. In order to increase the distillation efficiency of the active hydrogen compound, the distillation can also be carried out under boiling of the solvent or the hydroxy compound. In addition, the method using an inert gas is a method of separating an active hydrogen compound sequentially generated under a reaction from a reaction system by accompanying the active hydrogen compound in a gaseous state with an inert gas. As the inert gas, for example, nitrogen, helium, argon, carbon dioxide, methane, ethane, propane, or the like is used alone or in combination, and a method of introducing the inert gas into the reaction system is preferable. Examples of the adsorbent used in the adsorption separation method include adsorbents that can be used under the temperature conditions under which the reaction is carried out, such as silica, alumina, various zeolites, and diatomaceous earth. The method of removing these active hydrogen compounds to the outside of the system may be performed alone or in combination of a plurality of methods.

The reaction time (in the case of a continuous reaction, the residence time) varies depending on the formulation of the reaction system, the reaction temperature, the method of removing the active hydrogen compound, the reaction device, the reaction pressure, and the like, but is usually 0.01 hours or more and 100 hours or less. The reaction time can also be determined by the amount of the generated N-substituted carbamate, which is the target compound. For example, the reaction may be stopped after the reaction solution is sampled, the content of the N-substituted carbamate in the reaction solution is quantified, and it is confirmed that the reaction solution is produced with a yield of 10% by mass or more with respect to the mass of the carbonyl compound having a group derived from the primary amine

compound, or the reaction may be stopped after it is confirmed that the yield is 90% by mass or more. The yield of the N-substituted carbamate is preferably 50% by mass or more, more preferably 80% by mass or more, and still more preferably 90% by mass or more with respect to the mass of the carbonyl compound having a group derived from the primary amine compound.

In the reaction, it is not always necessary to use a reaction solvent, but a suitable solvent may be used for the purpose of facilitating the reaction operation or the like. Examples of the reaction solvent include the same one as that described in the method (1A). Needless to say, the hydroxy compound used in an excessive amount in the reaction is also suitably used as the reaction solvent.

The reaction of the step (b2) is carried out in a system having a gas phase containing the hydroxy compound, the compound having a carbonyl group derived from the carbonic acid derivative, and the active hydrogen compound produced as a by-product in the reaction, and a liquid phase in which the reaction of the step (b2) is carried out. Most of the reactions in the step (b2) are carried out in the liquid phase, but there is a case in which the reaction also occurs in the gas phase depending on the reaction conditions. In this case, a liquid-phase capacity content in the reactor in which the reaction of the step (b2) is carried out is preferably 50% by volume or less with respect to the capacity of the gas phase. In a case where the reaction is continuously carried out for a long period of time, there is a case in which a by-product in a polymer form is generated due to a change in operating conditions (temperature, pressure, and the like). However, in a case where the liquid-phase capacity content in the reactor is large, it is possible to avoid the adhesion and accumulation of such a by-product in a polymer form in the reactor. However, when the liquid-phase capacity content is too large, the removal efficiency of the by-product active hydrogen compound is deteriorated, and the yield of the N-substituted carbamate is lowered. Therefore, the liquid-phase capacity content is preferably 50% by volume or less, more preferably 30% by volume or less, and still more preferably 20% by volume or less with respect to the capacity of the gas phase. The liquid-phase capacity content referred to herein represents a liquid capacity ratio with respect to a capacity of a gas phase in a case of a tank-type reactor, a stage below a feed stage (excluding a bottom portion of a column and a reboiler portion) in a case of a column-type reactor, and a thin-film evaporator in a case of a thin-film evaporator.

The reaction device used when carrying out the reaction of the step (b2) is not particularly limited as long as it is a reactor equipped with a condenser, and a known reactor can be used. However, one or more reactors selected from the group consisting of a tank type and a tower type equipped with a condenser are preferably used.

As described above, the reaction of the step (b2) is carried out in a system having a gas phase containing the hydroxy compound, the compound having a carbonyl group derived from the carbonic acid derivative, and the active hydrogen compound produced as a by-product in the reaction, and a liquid phase in which the reaction of the step (b2) is carried out. In addition, it is preferable that, in the system with the liquid phase in which most of the reactions in the step (b2) is performed, the liquid-phase capacity content in the reactor in which the reaction of step (b2) is carried out is 50% by volume or less with respect to the volume of the gas phase, and those that match the conditions are selected.

As the reactor, specifically, a conventionally known reactor such as a stirring tank, a pressurized stirring tank, a reduced pressure stirring tank, a tower-type reactor, a distillation column, a filled column, and a thin film distillation column can be appropriately combined and used. The type of the condenser provided in the reactor is not particularly limited, and a known condenser can be used. For example, it is possible to appropriately combine and use a condenser known in the related art, such as a multi-tube cylindrical condenser, a double-tube condenser, a single-tube condenser, and an air-cooled condenser. The condenser may be provided inside the reactor, may be provided outside the reactor and connected to the reactor with a pipe, or may be adopted in various forms in consideration of the type of the reactor or the condenser, the method of handling the condensate, and the like.

A material of the reactor and the condenser is not particularly limited, and a known material can be used. For example, a glass, stainless steel, carbon steel, Hastelloy material, a material on which a glass lining is applied, or a material on which a Teflon (registered trademark) coating is applied can also be used. Among these, SUS304, SUS316, SUS316L, and the like are inexpensive and can be preferably used. As necessary, known process equipment such as measuring instruments, for example, a flowmeter and a thermometer, a reboiler, a pump, and a condenser may be added. The heating may be performed by a known method such as steam or a heater, and the cooling may be performed by a known method such as natural cooling, cooling water, or brine.

In the step (b2), it is possible to add a step as described below as necessary. Examples of the step that can be added include a step of removing a product active hydrogen compound, a step of purifying a primary amine compound, a step of dissolving a carbonic acid derivative in a hydroxy compound, a step of separating or purifying a hydroxy compound, a step of purifying a carbonyl compound having a group derived from a primary amine compound from the generated reaction solution, and a step of incinerating or disposing of a by-product, and the like, and a step or a device within the range that can be assumed by those skilled in the art may be added.

In a case where the alcohol is used as the hydroxy compound, the N-substituted carbamate obtained by the above-described reaction is an N-substituted carbamate represented by General Formula (Va) described later. In addition, in a case where the aromatic hydroxy compound is used as the hydroxy compound, the N-substituted carbamate obtained

by the above-described reaction is an N-substituted carbamate represented by General Formula (Vb) described later.

In the production method according to the present embodiment, the following step (c1) can be further performed in the step (X1). That is, the step (X1) may further include the following step (c1):

a step (step (c1)) of reacting a compound having one or more functional groups selected from the group consisting of a ureylene group and a biuret group with a carbonic acid derivative in the presence of at least one amine compound having no active hydrogen as a catalyst.

PCT International Publication No. WO2014/157636 (Reference Document 2) discloses a method for obtaining an N-substituted carbamate by reacting a compound having one or more functional groups selected from the group consisting of a compound having a ureylene group (a group represented by Formula (XIII-1)) and a compound having a biuret group (a group represented by Formula (XIII-2)), generated in the production of an N-substituted carbamate, with a carbonic acid derivative. The catalyst suitable for the above-described step (X1) is also preferably used for a reaction for obtaining the N-substituted carbamate from the compound having one or more functional groups selected from the group consisting of a compound having a ureylene group and a compound having a biuret group, and the carbonic acid derivative.

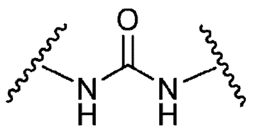
( XIII-1 )

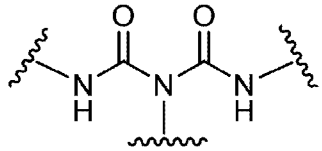
( XIII-2 )

(in Formulae (XIII-1) and (XIII-2), a wavy line represents a bonding site, and represents a bonding site with a group derived from the primary amine compound)

The step (c1) is preferably carried out in the presence of a hydroxy compound. The hydroxy compound may be an alcohol represented by General Formula (VIa) described later, or may be an aromatic hydroxy compound represented by General Formula (VIb) described later.

The reaction between the compound having a ureylene group represented by Formula (XIII-1) and the carbonic acid derivative is preferably carried out at 100°C or higher and 350°C or lower. At a low temperature, the reaction efficiency is poor, and at a too high temperature, a denaturation reaction occurs. Therefore, the reaction is preferably carried out at 120°C or higher and 330°C or lower and more preferably at 140°C or higher and 300°C or lower.

The amount of the carbonic acid derivative used (molar amount) varies depending on the kind of the carbonic acid derivative and the reaction conditions, but is usually such that the ratio of the number of moles of the carbonic acid derivative to the number of moles of the ureylene group of the compound having a ureylene group is 10 or less. In order to increase the reaction rate and improve the efficiency of the reaction, it is preferable that the amount of the carbonic acid derivative is large. However, in a case where the carbonic acid derivative is excessively used, a side reaction such as N-alkylation may occur. Therefore, the ratio of the number of moles of the carbonic acid derivative to the number of moles of the ureylene group of the compound having a ureylene group is preferably 5 or less and more preferably 3 or less.

The use amount of the amine compound having no active hydrogen used as the catalyst (molar amount) is not particularly limited, but for example, the use amount can be 0.0001 times or more and 100 times or less, preferably 20 times or less with respect to the molar amount of the ureylene group of the compound having a ureylene group. In a case where the amine compound having no active hydrogen is amidines or guanidines which are strong bases, the use amount is preferably 1 time or less, more preferably 0.2 times or less, and still more preferably 0.1 times or less with respect to the molar amount of the ureylene group.

The reaction between the compound having a ureylene group and the carbonic acid derivative is preferably carried out in the presence of a solvent. The solvent is not particularly limited as long as it is a stable compound at the reaction temperature and dissolves the compound having a ureylene group and the carbonic acid derivative; and the same solvent as that described in the step (X1) described above, an alcohol represented by General Formula (VIa) described later, a hydroxy compound such as an aromatic hydroxy compound represented by General Formula (VIb) described later can be used. In particular, an aromatic hydroxy compound is preferably used.

The step (c1) may be carried out using a compound having a ureylene group (which is by-produced in the step (X1)) contained in the reaction solution obtained in the step (X1) after the N-substituted carbamate is produced from the primary amine compound, the carbonic acid derivative, and the hydroxy compound in the step (X1), or may be carried out at the same time as the production of the N-substituted carbamate by the reaction of the primary amine compound, the carbonic acid derivative, and the hydroxy compound, and the N-substituted carbamate may be produced by the reaction of the compound having a ureylene group with the carbonic acid derivative. In such a case, the solvent used in the step (X1) or the hydroxy compound used in excess can also be used as the solvent.

The reaction may be carried out under any of the conditions of pressurization, normal pressure, and depressurization. In addition, the reaction is preferably carried out in an inert gas atmosphere such as nitrogen, argon, helium,

or neon.

As the reaction device, a conventionally known reactor such as a stirring tank, a pressurized stirring tank, a reduced pressure stirring tank, a tower-type reactor, a distillation column, a filled column, and a thin film distillation column can be appropriately combined and used. In order to keep the reaction temperature constant, a known cooling device and a heating device may be installed. In addition, a material thereof is not particularly limited, and a known material can be used. For example, a glass, stainless steel, carbon steel, Hastelloy material, a material on which a glass lining is applied, or a material on which a Teflon (registered trademark) coating is applied can also be used.

<Step (X2): condensation step of gas-phase component>

The step (X2) is a condensation step of condensing the gas-phase component which contains one or more selected from the group consisting of the carbonic acid derivative and the compound having a carbonyl group derived from the carbonic acid derivative and the hydroxy compound, and may contain one compound selected from the active hydrogen compound derived from the carbonic acid derivative and the amine compound having no active hydrogen as the catalyst, which are recovered in the step (X1), using the condenser provided in the reactor for performing the carbamate synthesis step (step (X1)).

The type of the condenser is not particularly limited, and a known condenser can be used. For example, it is possible to appropriately combine and use a condenser known in the related art, such as a multi-tube cylindrical condenser, a double-tube condenser, a single-tube condenser, and an air-cooled condenser. The condenser may be provided inside the reactor, may be provided outside the reactor and connected to the reactor with a pipe, or may be adopted in various forms in consideration of the type of the reactor or the condenser, the method of handling the condensate, and the like.

A material of the reactor and the condenser is not particularly limited, and a known material can be used. For example, a glass, stainless steel, carbon steel, Hastelloy material, a material on which a glass lining is applied, or a material on which a Teflon (registered trademark) coating is applied can also be used. Among these, SUS304, SUS316, SUS316L, and the like are inexpensive and can be preferably used. As necessary, known process equipment such as measuring instruments, for example, a flowmeter and a thermometer, a reboiler, a pump, and a condenser may be added. The heating may be performed by a known method such as steam or a heater, and the cooling may be performed by a known method such as natural cooling, cooling water, or brine.

In the step (X2), a step or a device within a range that can be assumed by those skilled in the art may be added as necessary.

The production method according to the present embodiment is a method for producing an N-substituted carbamate by subjecting a primary amine compound, a carbonic acid derivative, and a hydroxy compound to a carbamylation reaction using a reactor equipped with a condenser in the presence of an amine compound having no active hydrogen as a catalyst. In the carbamylation reaction, the gas-phase component which contains one or more selected from the group consisting of the carbonic acid derivative and the compound having a carbonyl group derived from the carbonic acid derivative and the hydroxy compound, and may contain one compound selected from the active hydrogen compound derived from the carbonic acid derivative and the amine compound having no active hydrogen as the catalyst is generated.

In the step (X2), the gas-phase component is introduced into the condenser provided in the reactor for performing the carbamylation step (step (1)), and a part or all of the hydroxy compound, a part or all of the one or more selected from the group consisting of the carbonic acid derivative and the compound having a carbonyl group derived from the carbonic acid derivative, and a part or all of the amine compound having no active hydrogen as the catalyst are condensed. At this time, the molar amount of the hydroxy compound to be condensed is preferably 1 times or more, more preferably 2 times or more, and still more preferably 3 times or more in terms of a stoichiometric ratio, with respect to the molar amount of the compound having a carbonyl group derived from the carbonic acid derivative to be condensed.

In the step (X2), the "compound having a carbonyl group derived from the carbonic acid derivative" mentioned as the component condensed in the condenser is a compound having a carbonyl group derived from the carbonic acid derivative used in the reaction of the primary amine compound with the carbonic acid derivative and the hydroxy compound. The compound having a carbonyl group derived from the carbonic acid derivative includes the carbonic acid derivative itself used as a raw material (one or more selected from the group consisting of an unreacted product and an excess component in a case where the carbonic acid derivative is used in excess with respect to the primary amine compound), a compound obtained by reacting the carbonic acid derivative with the hydroxy compound, and a compound obtained from the reacted carbonic acid derivative. It is difficult to identify all of the compounds into the compound having a carbonyl group derived from the carbonic acid derivative, but as specific compounds, carbonic acid derivatives used as raw materials, urea compounds such as isocyanic acid, biuret, and isocyanurate, which are by-products, N-unsubstituted carbamates in which the carbamate group is a group derived from a hydroxy compound, and carbonic acid esters in which the ester group is a group derived from a hydroxy compound are exemplary examples. The compound

having a carbonyl group derived from urea can be quantified by a method of detecting the carbonyl group contained in the compound by a method such as infrared spectroscopy, near-infrared spectroscopy, Raman spectroscopy, or ultraviolet spectroscopy. Alternatively, the amount can also be determined by a method of specifically analyzing the compound being produced by gas chromatography, liquid chromatography, NMR, or the like. These compounds having a carbonyl group derived from a carbonic acid derivative often have a high melting point and tend to be easily precipitated. Among the above-described compounds having a carbonyl group derived from the carbonic acid derivatives, urea is particularly noteworthy because the amount of urea produced (the amount detected) is large and the melting point is 135°C.

In the condensation operation, the molar amount of the hydroxy compound to be condensed is set to be equal to or more than the above-described lower limit value with respect to the molar amount of the compound having a carbonyl group derived from the carbonic acid derivative to be condensed, whereby it is possible to obtain a uniform liquid mixture in the condenser. Therefore, not only is the handling of the mixture facilitated, but also the occurrence of problems such as the adhesion and accumulation of the solid component in the condenser can be avoided. In order to set the molar amount of the hydroxy compound to be condensed within the above range, the condenser is preferably held at a temperature that is 90°C or higher lower than the standard boiling point of the hydroxy compound and is a temperature at which the hydroxy compound is not solidified.

The mixture of the hydroxy compound and the compound having a carbonyl group derived from a carbonic acid derivative, which is condensed by the above-mentioned condenser, may be reused for the reaction of the primary amine compound, the carbonic acid derivative, and the hydroxy compound by being circulated inside the reactor, or may be reused for the reaction of the primary amine compound, the carbonic acid derivative, and the hydroxy compound by recovering one or more selected from the group consisting of the hydroxy compound and the compound having a carbonyl group derived from a carbonic acid derivative from the mixture, or may be reused for the step of producing an N-unsubstituted carbamate (referring to the above-mentioned step (c1)).

At the time of reusing the condensate component, the amount of the active hydrogen compound derived from a carbonic acid derivative, which is contained in the mixture of the hydroxy compound and the compound having a carbonyl group derived from a carbonic acid derivative is preferably 5,000 ppm by mass or less, more preferably 3,000 ppm by mass or less, and still more preferably 2,000 ppm by mass or less with respect to the mass of the mixture.

Even in a case where the active hydrogen compound in an amount exceeding the upper limit value is contained, the active hydrogen compound can be reused in the reaction of the primary amine compound with the carbonic acid derivative and the hydroxy compound. However, as described above, the reaction of the primary amine compound with the carbonic acid derivative and the hydroxy compound is an equilibrium reaction, and in order to efficiently proceed with the reaction, it is necessary to remove the active hydrogen compound, which is a by-product, from the system. Therefore, by setting the amount of the active hydrogen compound in the mixture to be equal to or less than the above-described upper limit value, the amount of the active hydrogen compound extracted in the reaction can be further reduced, the introduction of the active hydrogen compound beyond the amount of the active hydrogen compound (which depends on the capacity of the urethane production reactor, reaction conditions, and the like) that can be extracted per unit time can be more effectively prevented, the concentration of the active hydrogen compound in the reaction solution can be reduced to a preferable range (the above-described range), and the yield of the N-substituted carbamate can be further improved. In addition, the amount of the active hydrogen compound in the mixture is preferably small, but by setting the amount to the above-described upper limit value or less, the amount of the active hydrogen compound can be minimized without much effort.

As described above, various compounds may be recovered as the compound having a carbonyl group derived from a carbonic acid derivative, but a mixture of a hydroxy compound and a compound having a carbonyl group derived from a carbonic acid derivative does not hinder the reuse of the condensate even if these compounds are included.

Next, the raw materials and the products used in the method for producing a carbamate compound according to the present embodiment will be described in detail below.

<Amine compound having no active hydrogen>

As the amine compound having no active hydrogen, an amine compound having one or more functional groups selected from the group consisting of a tertiary amino group, a nitrogen-containing aromatic group, an amidine group, and a guanidine group is preferably used.

From the viewpoint of the effect of accelerating the carbamylation reaction, the amine compound having no active hydrogen as the catalyst is preferably an amine compound in which the pKa of the conjugate acid is 6 or more and 17 or less; more preferably an amine compound having one or more functional groups selected from the group consisting of a tertiary amino group, an amidine group, and a guanidine group, in which the pKa of the conjugate acid is 10 or more and 15 or less; and still more preferably an amine compound having one or more functional groups selected from the group consisting of an amidine group and a guanidine group, in which the pKa of the conjugate acid is 12 or more and 15 or less.

On the other hand, an amine compound having too high a basicity tends to promote the modification reaction of carbamates, and the yield of carbamates may be reduced due to the modification. Therefore, from the viewpoint of suppressing the modification, the amine compound having no active hydrogen used as the catalyst is preferably an amine compound having a pKa of 1 or more and 15 or less; more preferably an amine compound having a pKa of 3 or more and 12 or less and having one or more functional groups selected from the group consisting of a tertiary amino group and a nitrogen-containing aromatic group; and still more preferably an amine compound having a nitrogen-containing aromatic group, in which the pKa of the conjugate acid is 4 or more and 7 or less.

[Compound having tertiary amino group]

The "tertiary amino group" in the present specification is a group represented by Formula (XIV). In Formula (XIV), a wavy line represents a bonding site, and represents a bonding site with the monovalent organic group.

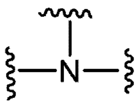

( XIV )

The total number of carbon atoms constituting the compound having a tertiary amino group is preferably 3 or more and 85 or less, and more preferably 3 or more and 30 or less. In addition, the number of tertiary amino groups included in the compound having a tertiary amino group is preferably 1 or more and 6 or less, and more preferably 1 or more and 3 or less.

As the preferred amine compound having a tertiary amino group, aromatic organic monotertiary amines such as N,N-dimethylaniline, N,N-diethylaniline, N-methyl-N-ethylaniline, and N,N-dimethylaminopyridine; aromatic organic polytertiary amines such as N,N,N',N'-tetramethylphenylenediamine (each isomer) and methylenebis(N,N-dimethylaniline) (each isomer); aliphatic monotertiary amines such as triethylamine, ethyldiisopropylamine, N-methylmorpholine, N-methylpiperidine, and quinuclidine; and aliphatic polytertiary amines such as N,N'-dimethylpiperazine, triethylenediamine, N,N,N' ,N'-tetramethylethylenediamine, N,N,N',N'-tetramethylhexanediamine, N,N,N',N'-tetramethylxylylenediamine, pentamethyldiethylenetriamine, bis(2-morpholinoethyl)ether, hexahydro-1,3,5-tris(3-dimethylaminopropyl)-1,3,5-triazine, and hexamethylenetetramine are exemplary examples.

[Compound having nitrogen-containing aromatic group]

The term "nitrogen-containing aromatic group" in the present specification refers to a heteroaromatic group which is a group in which at least one nitrogen atom is included in atoms constituting a heteroaromatic ring. The compound having a heteroaromatic group is composed of non-metal atoms (carbon, oxygen, nitrogen, sulfur, and silicon), and the total number of atoms is preferably 5 or more and 85 or less, and more preferably 6 or more and 30 or less. The non-metal atom is preferably selected from carbon, oxygen, and nitrogen, and more preferably a carbon atom. The total number of carbon atoms constituting the compound having a nitrogen-containing aromatic group is preferably 3 or more and 85 or less, and more preferably 3 or more and 30 or less.

In addition, the number of nitrogen-containing aromatic groups included in the compound having a nitrogen-containing aromatic group is preferably 1 or more and 5 or less, and more preferably 1 or more and 3 or less.

Preferred examples of the nitrogen-containing aromatic group include a pyridyl group, an imidazolyl group, a pyrazolyl group, a quinolyl group, an isoquinolyl group, an oxazolyl group, a thiazolyl group, a pyridazyl group, a pyrimidyl group, and a pyrazyl group, all of which may have a substituent.

Examples of the preferred amine compound having a nitrogen-containing aromatic group include pyridine, picoline (each isomer), lutidine (each isomer), collidine (each isomer), 1-methylimidazole, 1-methylpyrazole, quinoline, isoquinoline, methylquinoline (each isomer), oxazole, thiazole, pyridazine, pyrimidine, and pyrazine.

[Compound having amidine group]

As the compound having an amidine group, a compound represented by General Formula (VIII-1) is preferably used.

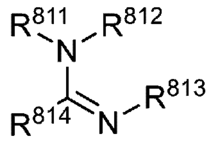

( VIII-1 )

(in General Formula (VIII-1), $R^{811}$, $R^{812}$, $R^{813}$, and $R^{814}$ are each independently a monovalent organic group, $R^{811}$ and $R^{812}$, $R^{812}$ and $R^{813}$, $R^{813}$ and $R^{814}$, or $R^{814}$ and $R^{811}$ may be each independently bonded to each other to form a ring structure, and a total number of carbon atoms in $R^{811}$, $R^{812}$, $R^{813}$, and $R^{814}$ is 5 or more and 85 or less)

Examples of the monovalent organic group as $R^{811}$, $R^{812}$, $R^{813}$, and $R^{814}$ include an aliphatic group, an aromatic group, a group formed by bonding an aliphatic group and an aromatic group, and a group in which the group is covalently bonded to a specific non-metal atom (carbon, oxygen, nitrogen, sulfur, or silicon).

$R^{811}$ and $R^{812}$, $R^{812}$ and $R^{813}$, $R^{813}$ and $R^{814}$, or $R^{814}$ and $R^{811}$ may be each independently bonded to each other to form a ring structure with a nitrogen atom.

The total number of carbon atoms constituting the compound having an amidine group is 5 or more and 85 or less, preferably 5 or more and 30 or less.

In addition, the compound having an amidine group preferably has one or more ring structures.

The number of amidine groups included in the compound having an amidine group is preferably 1 or more and 3 or less, and more preferably 1 or more and 2 or less.

Examples of the compound having a preferred amidine group include 1,2-dimethyl-1,4,5,6-tetrahydropyrimidine, 1,8-diazabicyclo-[5.4.0]undeca-7-ene (DBU), and 1,5-diazabicyclo-[4.3.0]nona-5-ene (DBN).

[Compound having guanidine group]

As the compound having a guanidine group, a compound represented by General Formula (VIII-2) is preferably used.

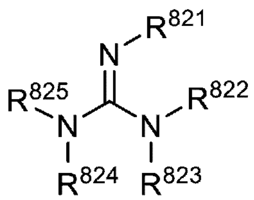

( VIII-2 )

(in General Formula (VIII-2), $R^{821}$, $R^{822}$, $R^{823}$, $R^{824}$, and $R^{825}$ are each independently a monovalent organic group, $R^{821}$ and $R^{822}$, $R^{822}$ and $R^{823}$, $R^{823}$ and $R^{824}$, $R^{824}$ and $R^{825}$, or $R^{825}$ and $R^{821}$ may be each independently bonded to each other to form a ring structure, and a total number of carbon atoms in $R^{821}$, $R^{822}$, $R^{823}$, $R^{824}$, and $R^{825}$ is 5 or more and 85 or less)

Examples of the monovalent organic group as $R^{821}$, $R^{822}$, $R^{823}$, $R^{824}$, and $R^{825}$ include an aliphatic group, an aromatic group, a group formed by bonding an aliphatic group and an aromatic group, and a group in which the group is covalently bonded to a specific non-metal atom (carbon, oxygen, nitrogen, sulfur, or silicon).

$R^{821}$ and $R^{822}$, $R^{822}$ and $R^{823}$, $R^{823}$ and $R^{824}$, $R^{824}$ and $R^{825}$, or $R^{825}$ and $R^{821}$ may be each independently bonded to each other to form a ring structure with a nitrogen atom.

The total number of carbon atoms constituting the compound having a guanidine group is 5 or more and 85 or less, preferably 5 or more and 30 or less.

In addition, the number of guanidine groups included in the compound having a guanidine group is preferably 1 or more and 3 or less, and more preferably 1 or more and 2 or less.

Examples of the preferred compound having a guanidine group include pentamethylguanidine and 7-methyl-1,5,7-triazabicyclo[4.4.0]deca-5-ene.

<Primary amine compound>

The "primary amine compound" in the present specification refers to a "primary amine" (monoprimary amine and polyprimary amine) defined by the IUPAC Nomenclature Rule C-8. Such a primary amine compound is a compound represented by General Formula (IIb) (hereinafter, may be referred to as "primary amine compound (II)").

$$R^{21b}\left(NH_2\right)_{n21b} \quad (IIb)$$

(in General Formula (IIb), $R^{21b}$ is an n21b-valent organic group, and n21b is an integer of 1 or more and 10 or less)

The organic group as $R^{21b}$ represents an aliphatic group, an aromatic group, or a group obtained by bonding an aliphatic group and an aromatic group, and represents an alicyclic hydrocarbon group, a cyclic hydrocarbon group (for example, a monocyclic hydrocarbon group, a fused polycyclic hydrocarbon group, a crosslinked cyclic hydrocarbon group, a spiro hydrocarbon group, a ring-assembled hydrocarbon group, a cyclic hydrocarbon group having a side chain, a heterocyclic group, a heterocyclic spiro group, a hetero crosslinking ring group, and a complex ring group), a group obtained by bonding an alicyclic hydrocarbon group and a cyclic hydrocarbon group, or a group in which these groups are covalently bonded to a specific non-metal atom (carbon, oxygen, nitrogen, sulfur, or silicon). In addition, the above-described covalent bond with the specific non-metal atom is, for example, a state in which the above-described group and one or more groups selected from the group consisting of groups represented by Formulae (IIb-a1) to (IIb-a10) are covalently bonded.

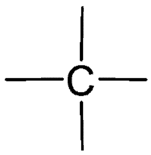 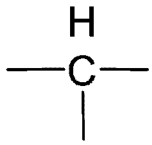 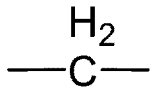

( IIb-a1 ) ( IIb-a2 ) ( IIb-a3 )

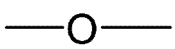 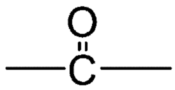 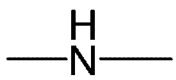 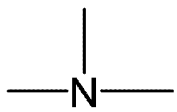

( IIb-a4 ) ( IIb-a5 ) ( IIb-a6 ) ( IIb-a7 )

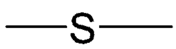 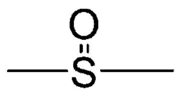 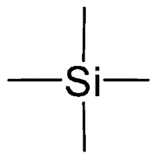

( IIb-a8 ) ( IIb-a9 ) ( IIb-a10 )

In consideration that side reactions are less likely to occur, $R^{21b}$ is an aliphatic group, an aromatic group, or a group obtained by bonding an aliphatic group and an aromatic group; and is preferably a group having 1 or more and 85 or less and selected from an alicyclic hydrocarbon group, a cyclic hydrocarbon group (a monocyclic hydrocarbon group, a fused polycyclic hydrocarbon group, a crosslinked cyclic hydrocarbon group, a spiro hydrocarbon group, a ring-assembled hydrocarbon group, or a cyclic hydrocarbon group having a side chain), or a group in which an acyclic hydrocarbon group and a cyclic hydrocarbon group are bonded (substituted with each other), and in consideration of fluidity and the like, more preferably a group having 1 or more and 70 or less carbon atoms and selected from these groups, and still more preferably a group having 1 or more and 13 or less carbon atoms and selected from these groups.

The preferred aliphatic group as $R^{21b}$ is a linear hydrocarbon group, a cyclic hydrocarbon group, or a group obtained by bonding a chain-like hydrocarbon group and a cyclic hydrocarbon group (for example, referring to a cyclic hydrocarbon group substituted with a chain-like hydrocarbon group, a chain-like hydrocarbon group substituted with a cyclic hydrocarbon group, or the like), having 5 or more and 70 or less carbon atoms.

Specific examples of the preferred primary amine compound include: 1) an aromatic primary monoamine compound that $R^{21b}$ is an aromatic group having 6 or more and 85 or less carbon atoms, which may be substituted with one or more groups selected from the group consisting of an aliphatic group and an aromatic group, and n21b is 1; 2) an aromatic primary polyamine compound that $R^{21b}$ is a group having 6 or more and 85 or less carbon atoms and containing one or more aromatic ring which may be substituted with one or more groups selected from the group consisting of an aliphatic group and an aromatic group, n21b is 2 or more, and the aromatic group in $R^{21b}$ is substituted with an

$NH_2$ group; and 3) an aliphatic primary (mono or poly)amine compound that $R^{21b}$ is an aliphatic group having 1 or more and 85 or less, which may be substituted with an aromatic group.

In the above, an amine compound in which an atom (preferably, a carbon atom) to which an $NH_2$ group is bonded is included in an aromatic ring is denoted as the aromatic amine compound, and an amine compound in which the atom (preferably, the carbon atom) to which the $NH_2$ group is bonded is bonded to an atom (mainly carbon) which is not in the aromatic ring is denoted as the aliphatic amine compound.

Among these, an aromatic primary polyamine compound or an aliphatic primary polyamine compound is preferable as the primary amine compound.

Specific examples of the preferred primary amine compounds are shown below.

[Aromatic primary monoamine compound]

The preferred aromatic primary monoamine compound is a compound represented by General Formula (IIb-1).

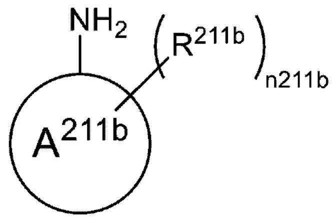

( IIb-1 )

(in General Formula (IIb-1), a ring $A^{211b}$ is an aromatic hydrocarbon ring having 6 or more and 20 or less carbon atoms, $R^{211b}$ is a hydrogen atom, or an alkyl group, a cycloalkyl group, an aryl group, or a group obtained by covalently bonding these groups to a specific non-metal atom (carbon, oxygen, nitrogen, sulfur, or silicon, each of which may be substituted with an aromatic group, $R^{211b}$ may be bonded to the ring $A^{211b}$ to form a ring structure, and n211b is an integer of 1 or more and 10 or less)

(A 211b)

The ring $A^{211b}$ is an aromatic hydrocarbon ring having 6 or more and 20 or less carbon atoms. The ring $A^{211b}$ may be a monocyclic ring, a polycyclic ring, or a fused ring.

Specific examples of the ring $A^{211b}$ include a benzene ring, a naphthalene ring, an anthracene ring, a phenanthrene ring, a naphthacene ring, a chrysene ring, a pyrene ring, a triphenylene ring, a pentylene ring, an azulene ring, a heptalene ring, an indacene ring, a biphenylene ring, an acenaphthylene ring, an acenanthrylene ring, and an acenaphthylene ring. Among these, the ring $A^{11}$ is preferably a benzene ring, a naphthalene ring, or an anthracene ring, and more preferably a benzene ring.

($R^{211b}$)

In a case where n211b is 2 or more, a plurality of $R^{211b}$'s may be the same or different from each other.

The total number of carbon atoms in the plurality of $R^{211b}$'s is preferably 1 or more and 64 or less, and more preferably 0 or more and 7 or less in consideration of fluidity and the like.

The total number of carbon atoms constituting the aromatic primary monoamine compound represented by General Formula (IIb-1) is 6 or more and 13 or less.

(n211b)

n211b represents the number of $R^{211b}$'s, is an integer of 1 or more and 10 or less, preferably an integer of 0 or more and 4 or less, more preferably an integer of 0 or more and 3 or less, and still more preferably an integer of 0 or more and 2 or less.

One or more selected from the group consisting of an ortho-position and a para-position of the $NH_2$ group of the aromatic primary monoamine compound represented by General Formula (IIb-1) are unsubstituted, that is, a hydrogen atom.

As a preferred aromatic primary monoamine compound, $R^{211b}$ is a hydrogen atom, a methyl group, or an ethyl group.

More specific examples of the preferred aromatic primary monoamine compound include aniline, aminotoluene (each isomer), dimethylaniline (each isomer), diethylaniline (each isomer), dipropylaniline (each isomer), aminonaphthalene (each isomer), aminomethylnaphthalene (each isomer), dimethylnaphthylamine (each isomer), and trimethylnaphthylamine (each isomer).

(Aromatic primary polyamine compound)

The aromatic primary polyamine compound is an aromatic primary polyamine compound that $R^{21b}$ in the primary amine compound represented by General Formula (IIb) described above is a group having 6 or more and 85 or less carbon atoms and containing one or more aromatic rings which may be substituted with one or more groups selected from the group consisting of an aliphatic group and an aromatic group, n21b is 2 or more, and the aromatic group in $R^{21b}$ is substituted with an $NH_2$ group.

In consideration of fluidity and the like, an aromatic primary polyamine compound that $R^{21b}$ is a group having 6 or more and 13 or less, which may be substituted with an alkyl group, an aryl group, or an aralkyl group, n21b is 2 or more and 8 or less carbon atoms, and the aromatic group in $R^{21b}$ is substituted with an $NH_2$ group is preferable.

Specific examples of the preferred aromatic primary polyamine compound include diaminobenzene (each isomer), diaminotoluene (each isomer), methylenedianiline (each isomer), diaminomethylidene (each isomer), diaminobiphenyl (each isomer), diaminodibenzyl (each isomer), bis (aminophenyl) propane (each isomer), bis (aminophenyl) ether (each isomer), bis (aminophenoxyethane) (each isomer), diaminoxylene (each isomer), diaminosol (each isomer), diaminophenol (each isomer), diaminonaphthalene (each isomer), diamino-methylbenzene (each isomer), diamino-methylpyridine (each isomer), diamino-methylnaphthalene (each isomer), and a polymethylene polyphenylamine represented by General Formula (11b-1-1).

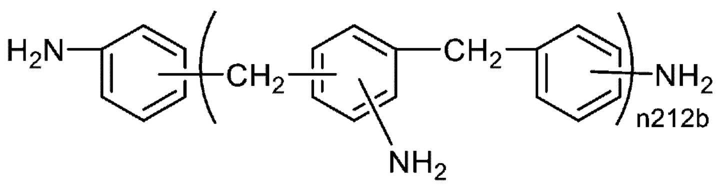

( IIb-1-1 )

(in the formula, n212b is an integer of 0 or more and 6 or less)

(Aliphatic primary (mono or poly)amine compound)

The aliphatic primary (mono or poly)amine compound is an aliphatic primary (mono or poly)amine compound that $R^{21b}$ in the primary amine compound represented by General Formula (IIb) described above is an aliphatic group having 1 or more and 85 or less carbon atoms, which may be substituted with an aromatic group.

A preferred aliphatic primary amine compound is an aliphatic primary amine compound that $R^{21b}$ is an acyclic aliphatic hydrocarbon group, a cyclic aliphatic (that is, an alicyclic) hydrocarbon group, a group obtained by bonding an acyclic aliphatic hydrocarbon group and a cyclic aliphatic (an alicyclic) hydrocarbon group, or a group obtained by covalently bonding these groups to a specific non-metal atom (carbon, oxygen, nitrogen, sulfur, or silicon), each of which has 1 or more and 70 or less carbon atoms, and n21b is 2 or more.

A more preferred aliphatic primary amine compound is an aliphatic primary polyamine compound that $R^{21b}$ is an acyclic aliphatic hydrocarbon group, a cyclic aliphatic (an alicyclic) hydrocarbon group, a group obtained by bonding an acyclic aliphatic hydrocarbon group and a cyclic aliphatic (an alicyclic) hydrocarbon group, or a group obtained by covalently bonding these groups to a specific non-metal atom (carbon, oxygen, nitrogen, sulfur, or silicon), each of which has 1 or more and 70 or less carbon atoms, and n21b is 2 or more and 5 or less.

In consideration of fluidity and the like when industrially producing in large quantities, a still more preferred aliphatic primary amine compound is an aliphatic organic polyprimary amine that $R^{21b}$ is an acyclic aliphatic hydrocarbon group, a cyclic aliphatic (an alicyclic) hydrocarbon group, a group obtained by bonding an acyclic aliphatic hydrocarbon group and a cyclic aliphatic (an alicyclic) hydrocarbon group, or a group obtained by covalently bonding these groups to a specific non-metal atom (carbon, oxygen, or nitrogen), each of which consists of carbon atoms and hydrogen atoms and has 5 or more and 13 or less carbon atoms, and n21b is 2 or more and 4 or less.

The most preferred aliphatic primary amine compound is an aliphatic primary amine compound that $R^{21b}$ is a group consisting of carbon atoms and hydrogen atoms, in which an acyclic aliphatic hydrocarbon group having 5 or more and 13 or less is covalently bonded to a specific non-metal atom (carbon, oxygen, or nitrogen), and n21b is 2 or 3.

Specific examples of the preferred aliphatic primary amine compound include:

aliphatic primary diamines such as ethylenediamine, diaminopropane (each isomer), diaminopentane (each isomer), diaminohexane (each isomer), diaminodecane (each isomer), xylylenediamine (each isomer), diaminocyclobutane

(each isomer), diaminocyclohexane (each isomer), bis(diaminomethyl)cyclohexane, 3-aminomethyl-3,5,5-trimethylcyclohexylamine (cis and/or trans form), and methylenebis(cyclohexylamine) (each isomer);
aliphatic primary triamines such as triaminohexane (each isomer), triaminononane (each isomer), and triaminodecane (each isomer); and
amino acid derivatives such as lysine methyl ester and lysine aminoethyl ester.

<Carbonic acid derivative>

The carbonic acid derivative in the present specification is a component used as a raw material for producing the carbamate compound together with the primary amine compound and the hydroxy compound. As the carbonic acid derivative, specifically, a urea compound, N-unsubstituted carbamate, and a carbonic acid ester are exemplary examples.

[Urea compound]

The urea compound in the present specification is a compound having at least one urea bond in the molecule. A preferred urea compound is a compound having one urea bond, and specific examples thereof include a compound represented by General Formula (III-2).

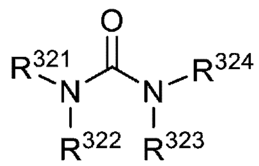

( III-2 )

(in General Formula (III-2), $R^{321}$, $R^{322}$, $R^{323}$, and $R^{324}$ are each independently an organic group having 1 or more and 20 or less carbon atoms, or a hydrogen atom, the total number of carbon atoms in $R^{321}$ and $R^{322}$ is an integer of 0 or more and 20 or less, and the total number of carbon atoms in $R^{323}$ and $R^{324}$ is an integer of 0 or more and 20 or less)

($R^{321}$, $R^{322}$, $R^{323}$, and $R^{324}$)

Examples of the organic group having 1 or more and 20 or less carbon atoms, as $R^{321}$, $R^{322}$, $R^{323}$, and $R^{324}$, include alkyl groups such as a methyl group, an ethyl group, a propyl group (each isomer), a butyl group (each isomer), a pentyl group (each isomer), a hexyl group (each isomer), a heptyl group (each isomer), and an octyl group (each isomer); aryl groups having 6 or more and 20 or less carbon atoms, such as a phenyl group, a methylphenyl group (each isomer), a biphenyl group (each isomer), a dimethylphenyl group (each isomer), a diethylphenyl group (each isomer), a terphenyl group (each isomer), a trimethylphenyl group (each isomer), a triethylphenyl group (each isomer), a tripropylphenyl group (each isomer), and a tributylphenyl group (each isomer); and aralkyl groups having 7 or more and 20 or less carbon atoms, such as a phenylmethyl group and a phenylethyl group (each isomer).

Specific examples of the urea compound include urea, methylurea, and ethylurea. Among these, urea in which, in General Formula (III-2), $R^{321}$, $R^{322}$, $R^{323}$, and $R^{324}$ are all hydrogen atoms is preferable.

The urea may include biuret, triuret, and cyanuric acid. However, generally, biuret, triuret, and cyanuric acid have low solubility in a solvent. Since it is preferable that the raw material or the reaction solution is a uniform solution during transferring, it is preferable that the contents thereof are as small as possible. In addition, the melting point of the urea is 135°C and the urea is solid at room temperature, but the shape thereof is not particularly limited, and for example, the urea can be used in a powder form or a granular form.

[N-unsubstituted carbamate]

As the N-unsubstituted carbamate, a compound represented by General Formula (III-3) is preferably used.

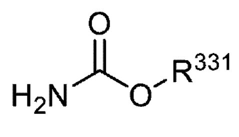

( III-3 )

(in General Formula (III-3), $R^{331}$ is an organic group having 1 or more and 20 or less carbon atoms)

($R^{331}$)

As the organic group having 1 or more and 20 or less carbon atoms, as $R^{331}$, the same organic groups described in $R^{321}$, $R^{322}$, $R^{323}$, and $R^{324}$ above are exemplary examples.
Specific examples of the N-unsubstituted carbamate include methyl carbamate, ethyl carbamate, propyl carbamate, butyl carbamate, and phenyl carbamate.

[Carbonic acid ester]

The term "carbonic acid ester" in the present specification refers to a compound in which one or two of two hydrogen atoms of carbonic acid $CO(OH)_2$ are substituted with an aliphatic group or an aromatic group.
As the carbonic acid ester, a compound represented by General Formula (III-1b) is preferably used.

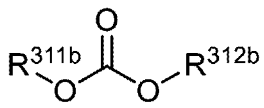 ( III-1b )

(in General Formula (III-1b), $R^{311b}$ and $R^{312b}$ are each independently an organic group having 1 or more and 20 or less carbon atoms)

($R^{311b}$ and $R^{312b}$)

As the organic group having 1 or more and 20 or less carbon atoms, as $R^{311b}$ and $R^{312b}$, the same organic groups described in $R^{321}$, $R^{322}$, $R^{323}$, and $R^{324}$ above are exemplary examples.
Specific examples of the carbonic acid ester include dimethyl carbonate, diethyl carbonate, and diphenyl carbonate.

<Hydroxy compound>

The hydroxy compound refers to a compound having a hydroxy group (-OH group). As the hydroxy compound, one or more compounds selected from the group consisting of alcohols and aromatic hydroxy compounds, which are compounds in which a hydroxy group is bonded to a carbon atom, are preferably used.

[Alcohol]

The "alcohol" in the present specification is a compound in which a hydroxy group is bonded to a saturated carbon atom (Compounds in which a hydroxy group, -OH, is attached to a saturated carbon atom: $R_3COH$) as defined by IUPAC (Rule C-201), and is a hydroxy compound represented by General Formula (VIa).

$$R^{61a}\!-\!\left(OH\right)_{n61a} \qquad (\text{VIa})$$

(in General Formula (Via), $R^{61a}$ is an n61a-valent aliphatic group, where $R^{61a}$ is a group having no active hydrogen other than a hydroxy group, and n61a is an integer of 1 or more and 5 or less)

($R^{61a}$)

In the aliphatic group as $R^{61a}$, an aliphatic group having 1 or more and 50 or less carbon atoms or an aromatic group having 7 or more and 50 or less carbon atoms may be bonded as a substituent.
The aliphatic group as $R^{61a}$ is an aliphatic hydrocarbon group in which an atom constituting the aliphatic group, other than a hydrogen atom, is a specific non-metal atom (carbon, oxygen, nitrogen, sulfur, silicon, or a halogen atom). Examples of the preferred aliphatic group include a chain-like aliphatic hydrocarbon group, a cyclic aliphatic (alicyclic) hydrocarbon group, or a group obtained by bonding a chain-like aliphatic hydrocarbon group and a cyclic aliphatic (alicyclic) hydrocarbon group (for example, referring to a cyclic hydrocarbon group substituted with a chain-like hydrocarbon group, a chain-like hydrocarbon group substituted with a cyclic hydrocarbon group, or the like).
In addition, the aliphatic group to which the aromatic group is bonded is an aliphatic group in which a linear or branched alkyl group or a cycloalkyl group is bonded to an aromatic group, or a group in which an aromatic group having

6 or more and 49 or less carbon atoms is bonded to an alkyl group having 1 or more and 44 or less carbon atoms. As described above, the aromatic group is preferably an aromatic group in which atoms other than hydrogen atoms constituting the aromatic group are specific non-metal atoms (carbon, oxygen, nitrogen, sulfur, silicon, and halogen atoms), and examples thereof include a monocyclic aromatic group, a fused polycyclic aromatic group, a bridged ring aromatic group, a ring assembly aromatic group, and a heterocyclic aromatic group. More preferably, the aromatic group is a substituted or unsubstituted phenyl group, a substituted or unsubstituted naphthyl group, or a substituted or unsubstituted anthryl group.

Specific examples of such $R^{61a}$ include a substituted or unsubstituted alkyl group and a cycloalkyl group, such as a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), a dodecyl group (each isomer), an octadecyl group (each isomer), a cyclopentyl group, a cyclohexyl group, a methylcyclopentyl group (each isomer), a methylcyclohexyl group (each isomer), a dimethylcyclohexyl group (each isomer), and a diethylcyclohexyl group (each isomer); and an aralkyl group such as a phenylmethyl group and a phenylethyl group (each isomer).

Among these alcohols, in consideration of industrial use, the alcohol is generally low in viscosity, and thus an alcohol having one or two alcoholic hydroxy groups (a hydroxy group directly added to a carbon atom constituting a hydroxy compound, other than a carbon atom in an aromatic ring) is preferable, and a monoalcohol having one alcoholic hydroxy group is more preferable.

Specific examples of the alcohol include substituted or unsubstituted alkyl alcohols and cycloalkyl alcohols, such as methanol, ethanol, propanol (each isomer), butanol (each isomer), pentanol (each isomer), hexanol (each isomer), heptanol (each isomer), octanol (each isomer), nonanol (each isomer), decanol (each isomer), dodecanol (each isomer), octadecanol (each isomer), cyclopentanol, cyclohexanol, cycloheptanol, cyclooctanol, methylcyclopentanol (each isomer), ethylcyclopentanol (each isomer), methylcyclohexanol (each isomer), ethylcyclohexanol (each isomer), propylcyclohexanol (each isomer), butylcyclohexanol (each isomer), pentylcyclohexanol (each isomer), hexylcyclohexanol (each isomer), dimethylcyclohexanol (each isomer), diethylcyclohexanol (each isomer), and dibutylcyclohexanol (each isomer); and alkyl alcohols substituted with an aryl group, such as phenylmethanol, phenylethanol (each isomer), phenylpropanol (each isomer), phenylbutanol (each isomer), phenylpentanol (each isomer), phenylhexanol (each isomer), phenylheptanol (each isomer), phenyloctanol (each isomer), and phenylnonanol (each isomer).

Among these, from the viewpoint of easy availability, solubility of a raw material or a product, and the like, an alkyl alcohol having 1 or more and 20 or less carbon atoms is preferably used.

[Aromatic hydroxy compound]

The "aromatic hydroxy compound" in the present specification is phenols "Compounds having one or more hydroxy groups attached to a benzene or other arene ring in which one or more hydroxy groups are bonded to a benzene ring or another arene ring", as described in the definition (Rule C-202) of IUPAC, and is a compound represented by General Formula (VIb).

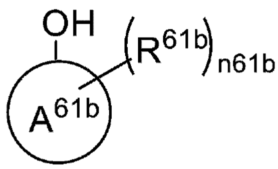

( VIb )

(in General Formula (VIb), a ring $A^{61b}$ is an aromatic hydrocarbon ring having 6 or more and 20 or less carbon atoms, $R^{61b}$ is a hydrogen atom or a monovalent organic group, and n61b is an integer of 1 or more and 6 or less)

($R^{61b}$)

The monovalent organic group as $R^{61b}$ is a halogen atom, an aliphatic group, an aromatic group, or a group in which these groups are bonded. More specifically, the monovalent organic group as $R^{61b}$ is an aliphatic group, an aromatic group, or a group obtained by bonding an aliphatic group and an aromatic group, and represents an alicyclic hydrocarbon group, a cyclic hydrocarbon group (for example, a monocyclic hydrocarbon group, a fused polycyclic hydrocarbon group, a crosslinked cyclic hydrocarbon group, a spiro hydrocarbon group, a ring-assembled hydrocarbon group, a cyclic hydrocarbon group having a side chain, a heterocyclic group, a heterocyclic spiro group, a hetero crosslinking ring group, and a complex ring group), a group obtained by bonding an alicyclic hydrocarbon group and a cyclic hydrocarbon group, or a group in which these groups are covalently bonded to a specific non-metal atom (carbon,

oxygen, nitrogen, sulfur, or silicon).

(n61b)

n61b represents the number of aromatic hydroxy groups bonded to the ring $A^{61b}$, and since the aromatic hydroxy group is generally low in viscosity, 1 or 2 is preferable and 1 is more preferable. That is, an aromatic di or monohydroxy compound is preferable, and an aromatic monohydroxy compound is more preferable.

(Ring $A^{61b}$)

The ring $A^{61b}$ is an aromatic hydrocarbon ring having 6 or more and 20 or less carbon atoms. The ring $A^{61b}$ may be a monocyclic ring, a polycyclic ring, or a fused ring.

Specific examples of the ring $A^{61b}$ include a benzene ring, a naphthalene ring, and an anthracene ring. In consideration of industrial use, the ring $A^{61b}$ is preferably a benzene ring which is easily available.

Examples of the more preferred aromatic hydroxy compound include an aromatic monohydroxy compound represented by General Formula (VIb-1).

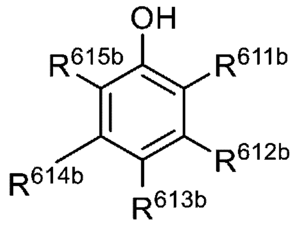

( VIb-1 )

(in General Formula (VIb-1), $R^{611b}$, $R^{612b}$, $R^{613b}$, $R^{614b}$, and $R^{615b}$ are each independently the same as $R^{61b}$ described above)

($R^{611b}$, $R^{612b}$, $R^{613b}$, $R^{614h}$, and $R^{615b}$)

Among these, as $R^{611b}$, $R^{612b}$, $R^{613b}$, $R^{614b}$, and $R^{615b}$, an alkyl group, a cycloalkyl group, an aryl group, an aralkyl group, a group in which these groups may include an ether group (substituted or unsubstituted alkyl ether, aryl ether, or aralkyl ether), a group in which two or more of these groups are bonded, a group in which these groups are covalently bonded to a specific non-metal atom (carbon, oxygen, nitrogen, sulfur, or silicon), a halogen atom, or a hydrogen atom is preferable.

The total number of carbon atoms in $R^{611b}$, $R^{612b}$, $R^{613b}$, $R^{614b}$, and $R^{615b}$ is an integer of 0 or more and 44 or less.

More preferred $R^{611b}$, $R^{612b}$, $R^{613b}$, $R^{614b}$, and $R^{615b}$ are each independently one or more groups selected from the group consisting of groups represented by the following (i) to (v):

(i) a hydrogen atom;
(ii) a halogen atom;
(iii) a group having 1 or more and 44 or less carbon atoms and having a carbon atom at an $\alpha$-position, in which three groups bonded to the carbon atom at the $\alpha$-position are each independently an alkyl group having 1 or more and 43 or less carbon atoms, a cycloalkyl group having 4 or more and 43 or less carbon atoms, an alkoxy group having 1 or more and 43 or less carbon atoms, a polyoxyalkylene alkyl ether group having 2 or more and 43 or less carbon atoms and having no OH group at a terminal, an aryl group having 6 or more and 43 or less carbon atoms, an aralkyl group having 7 or more and 43 or less carbon atoms, an aralkyloxy group having 7 or more and 43 or less carbon atoms, and a group in which two or more of these groups are bonded to each other;
(iv) an aryl group having 1 or more and 44 or less carbon atoms and substituted with a substituent, in which the substituent is an aryl group which may be substituted with the following substituent in a range of an integer of 1 or more and 5 or less, the substituent being a hydrogen atom, an alkyl group having 1 or more and 38 or less carbon atoms, a cycloalkyl group having 4 or more and 38 or less carbon atoms, an alkoxy group having 1 or more and 38 or less carbon atoms, a polyoxyalkylene alkyl ether group having 2 or more and 38 or less carbon atoms and having no OH group at a terminal, an aryl group having 6 or more and 38 or less carbon atoms, an aralkyl group having 7 or more and 38 or less carbon atoms, an aralkyloxy group having 7 or more and 38 or less carbon atoms, and a group in which two or more of these groups are bonded to each other; and
(v) a group having 1 or more and 44 or less carbon atoms and having an oxygen atom at an $\alpha$-position, in which a

group bonded to the oxygen atom at the α-position is an alkyl group having 1 or more and 44 or less carbon atoms, a cycloalkyl group having 4 or more and 44 or less carbon atoms, an alkoxy group having 1 or more and 44 or less carbon atoms, a polyoxyalkylene alkyl ether group having 2 or more and 44 or less carbon atoms and having no OH group at a terminal, an aryl group having 6 or more and 44 or less carbon atoms, an aralkyl group having 7 or more and 44 or less carbon atoms, an aralkyloxy group having 7 or more and 44 or less carbon atoms, and a group in which two or more of these groups are bonded to each other.

The term "atom at the α-position" used herein refers to an atom which is adjacent to a carbon atom on an aromatic hydrocarbon ring to which $R^{611b}$, $R^{612b}$, $R^{613b}$, $R^{614b}$, and $R^{615b}$ are bonded, among atoms constituting $R^{611b}$ $R^{612b}$, $R^{613b}$, $R^{614b}$, and $R^{615b}$.

In addition, the term "aralkyloxy group" in the present specification represents a group in which an oxygen atom is bonded to the aralkyl group defined above.

Specific examples of such $R^{611b}$, $R^{612b}$, $R^{613b}$, $R^{614b}$, and $R^{615b}$ include substituted or unsubstituted alkyl groups and cycloalkyl groups, such as a methyl group, an ethyl group, a propyl group (each isomer), a butyl group (each isomer), a cyclopentyl group, a cyclohexyl group, a methylcyclopentyl group (each isomer), an ethylcyclopentyl group (each isomer), a methylcyclohexyl group (each isomer), a dimethylcyclohexyl group (each isomer), and a diethylcyclohexyl group (each isomer); substituted or unsubstituted alkoxy groups and cycloalkoxy groups, such as a methoxy group, an ethoxy group, a propoxy group (each isomer), a butyloxy group (each isomer), a cyclopentyloxy group, a cyclohexyloxy group, a methylcyclopentyloxy group (each isomer), an ethylcyclopentyloxy group (each isomer), a methylcyclohexyloxy group (each isomer), a dimethylcyclohexyloxy group (each isomer), and a diethylcyclohexyloxy group (each isomer); substituted or unsubstituted aryl groups such as a phenyl group, a methylphenyl group (each isomer), an ethylphenyl group (each isomer), a biphenyl group (each isomer), a dimethylphenyl group (each isomer), a diethylphenyl group (each isomer), a terphenyl group (each isomer), a trimethylphenyl group (each isomer), and a triethylphenyl group (each isomer); substituted or unsubstituted aryloxy groups such as a phenoxy group, a methylphenoxy group (each isomer), an ethylphenoxy group (each isomer), a phenylphenoxy group (each isomer), a dimethylphenoxy group (each isomer), a diethylphenoxy group (each isomer), a diphenylphenoxy group (each isomer), a trimethylphenoxy group (each isomer), and a triethylphenoxy group (each isomer); aralkyl groups such as a phenylmethyl group, a phenylethyl group (each isomer), and a phenylpropyl group (each isomer); and aralkyloxy groups such as a phenylmethoxy group and a phenylethoxy group (each isomer).

Examples of the preferred aromatic hydroxy compound include the following compounds:
phenol, chlorophenol (each isomer), bromophenol (each isomer), dichlorophenol (each isomer), dibromophenol (each isomer), trichlorophenol (each isomer), tribromophenol (each isomer), phenol, methylphenol (each isomer), ethylphenol (each isomer), propylphenol (each isomer), butylphenol (each isomer), pentylphenol (each isomer), hexylphenol (each isomer), heptylphenol (each isomer), octylphenol (each isomer), nonylphenol (each isomer), cumylphenol (each isomer), dimethylphenol (each isomer), diethylphenol (each isomer), isopropylmethylphenol (each isomer), trimethylphenol (each isomer), triethylphenol (each isomer), dibutylmethylphenol (each isomer), (methoxymethyl)phenol (each isomer), (ethoxymethyl)phenol (each isomer), ((cyclopentyloxymethyl)phenol (each isomer), (cyclohexyloxymethyl)phenol (each isomer), (methylcyclopentyloxymethyl)phenol (each isomer),(methylcyclohexyloxymethyl)phenol (each isomer), (dimethylcyclohexyloxymethyl)phenol (each isomer), (phenoxy)methyl phenol, (methylphenoxy)methyl phenol (each isomer), (phenylphenoxy)methyl phenol (each isomer), (dimethylphenoxy)methyl phenol (each isomer), (diethylphenoxy)methyl phenol (each isomer), (diphenylphenoxy)methyl phenol (each isomer), (trimethylphenoxy)methyl phenol (each isomer), (phenylmethoxymethyl)phenol, (phenylethoxymethyl)phenol (each isomer), (phenylpropoxymethyl)phenol (each isomer), di(methoxymethyl)phenol, di(ethoxymethyl)phenol, di(propoxymethyl)phenol (each isomer), di(butoxymethyl)phenol (each isomer), di(cyclopentyloxymethyl)phenol, di(cyclohexyloxymethyl)phenol, di(methylcyclopentyloxymethyl)phenol (each isomer), di(methylcyclohexyloxymethyl)phenol (each isomer), bis(dimethylcyclohexyloxymethyl)phenol (each isomer), bis(diethylcyclohexyloxymethyl)phenol (each isomer), di(methylphenoxy)methyl phenol (each isomer), di(ethylphenoxy)methyl phenol (each isomer), nor (each isomer), bis(diethylcyclohexyloxymethyl)phenol (each isomer), di(methylphenoxy)methyl phenol (each isomer), di(ethylphenoxy)methyl phenol (each isomer), di(propylphenoxy methyl)phenol (each isomer), bis(dimethylphenoxy methyl)phenol (each isomer), bis(diethylphenoxy methyl)phenol (each isomer), bis(dipropylphenoxy methyl)phenol (each isomer), bis(dibutylphenoxy methyl)phenol (each isomer), bis(diphenylphenoxy methyl)phenol (each isomer), di(trimethylphenoxy methyl)phenol (each isomer), di(triethylphenoxy methyl)phenol (each isomer), di(phenylmethoxymethyl)phenol, di(phenylethoxymethyl)phenol (each isomer), di(phenylpropyloxymethyl)phenol (each isomer), tri(methoxymethyl)phenol, tri(ethoxymethyl)phenol, tri(cyclohexyloxymethyl)phenol, tri(methylcyclopentyloxymethyl)phenol (each isomer), tri(ethylcyclopentyloxymethyl)phenol (each isomer), tri(methylcyclohexyloxymethyl)phenol (each isomer), bis(dimethylcyclohexyloxymethyl)phenol (each isomer), bis(diethylcyclohexyloxymethyl)phenol (each isomer), bis(dibutylcyclohexyloxymethyl)phenol (each isomer), tri(phenoxy)methylphenol, tri(methylphenoxy)methylphenol (each isomer), tri(ethylphenoxy)methylphenol (each isomer), tri(phenylphenoxy)methylphenol (each isomer), bis(dimethylphenoxy)methylphenol (each isomer), bis(diethylphenoxy)methylphenol (each isomer), bis(diphenylphenoxy)methylphenol (each isomer), tri(trimethylphenoxy)methylphenol (each isomer), tri(triethylphenoxy)methylphenol (each isomer), tri(phenylmethoxymethyl)phenol, (phenylmethyl)phenol (each isomer), ((methylphenyl)methyl)phenol (each isomer), ((ethylphenyl)methyl)phenol (each isomer), ((biphenyl)methyl)phenol (each isomer), ((dimethylphenyl)methyl)phenol (each isomer), ((terphenyl)methyl)phenol (each isomer), ((trimethylphenyl)methyl)phenol (each isomer), ((triethylphenyl)methyl)phenol (each isomer), di(phenylmethyl)phenol (each isomer), di((methylphenyl)methyl)phenol (each isomer), di((ethylphenyl)methyl)phenol (each isomer), di((biphenyl)methyl)phenol (each isomer), di((dimethylphenyl)methyl)phenol (each isomer), di((diethylphenyl)methyl)phenol (each isomer), di((terphenyl)methyl)phenol (each isomer), di((trimethylphenyl)methyl)phenol (each isomer), di((triethylphenyl)methyl)phenol (each isomer), tri(phenylmethyl)phenol (each isomer), tri((methylphenyl)methyl)phenol (each isomer), tri(ethylphenyl)methyl)phenol (each isomer), tri((biphenyl)methyl)phenol (each isomer), tri((dimethylphenyl)methyl)phenol (each isomer), tri((diethylphenyl)methyl)phenol (each isomer), tri((terphenyl)methyl)phenol (each isomer), tri((trimethylphenyl)methyl)phenol (each isomer), tri((triethylphenyl)methyl)phenol (each isomer), tri(phenylmethyl)phenol (each isomer), tri((methylphenyl)methyl)phenol (each isomer), tri((ethylphenyl)methyl)phenol (each isomer), tri((biphenyl)methyl)phenol (each isomer), tri((dimethylphenyl)methyl)phenol (each isomer), tri((diethylphenyl)methyl)phenol (each isomer), tri((terphenyl)methyl)phenol (each isomer), tri((trimethylphenyl)methyl)phenol (each isomer), tri((triethylphenyl)methyl)phenol (each isomer), phenylethylphenol (each isomer), phenyl-n-propylphenol (each isomer), phenyl-n-butylphenol (each isomer), phenyl-n-pentylphenol (each isomer), phenyl-n-hexylphenol (each isomer), phenyl-n-heptylphenol (each isomer), phenyl-n-octylphenol (each isomer), phenyl-n-nonylphenol (each isomer), (methylamino)phenol, (ethylamino)phenol, (propylamino)phenol (each isomer), (butylamino)phenol (each isomer),(pentylamino)phenol (each isomer), (hexylamino)phenol (each isomer), di(methylamino)phenol, di(ethylamino)phenol, di(propylamino)phenol (each isomer), di(butylamino)phenol (each isomer), tri(methylamino)phenol, tri(ethylamino)phenol, methoxyphenol (each isomer), ethoxyphenol (each isomer), propyloxyphenol (each isomer), butyloxyphenol (each isomer), pentyloxyphenol (each isomer), hexyloxyphenol (each isomer), heptyloxyphenol (each isomer), octyloxyphenol (each isomer), nonyloxyphenol (each isomer), decyloxyphenol (each isomer), dodecyloxyphenol (each isomer), octadecyloxyphenol (each isomer), cyclopentyloxyphenol (each isomer), cyclohexyloxyphenol (each isomer), (methylcyclopentyloxy)phenol (each isomer), (ethylcyclopentyloxy)phenol (each isomer), (methylcyclohexyloxy)phenol (each isomer), (ethylcyclohexyloxy)phenol (each isomer), (propylcyclohexyloxy)phenol (each isomer), (dimethylcyclohexyloxy)phenol (each isomer), (diethylcyclohexyloxy)phenol (each isomer), phenoxyphenol, (methylphenoxy)phenol (each isomer), (dimethylphenoxy)phenol (each isomer), (trimethylphenoxy)phenol (each isomer), (phenylmethoxy)phenol, and lu (each isomer), (propylcyclohexyloxy)phenol (each isomer), (dimethylcyclohexyloxy)phenol (each isomer), (diethylcyclohexyloxy)phenol (each isomer), phenoxyphenol, (methylphenoxy)phenol (each isomer), (dimethylphenoxy)phenol (each isomer), (trimethylphenoxy)phenol (each isomer), (phenylmethoxy)phenol,(phenylethyloxy)phenol (each isomer), dimethoxyphenol (each isomer), diethoxyphenol (each isomer), dicyclohexyloxyphenol (each isomer), di(methylcyclopentyloxy)phenol (each isomer), bis(dimethylcyclohexyloxy)phenol (each isomer), di(methylphenoxy)phenol (each isomer), bis(dimethylphenoxy)phenol (each isomer), di(trimethylphenoxy)phenol (each isomer), di(phenylmethoxy)phenol, trimethoxyphenol (each isomer), triethoxyphenol (each isomer), tripropyloxyphenol (each isomer), tributyloxyphenol (each isomer), tricyclopentyloxyphenol (each isomer),tricyclohexyloxyphenol (each isomer), triphenyloxyphenol, tri(methylphenoxy)phenol (each isomer), tri(ethylphenoxy)phenol (each isomer), tri(propylphenoxy)phenol (each isomer), tri(dimethylphenoxy)phenol (each isomer), tri(diethylphenoxy)phenol (each isomer), tri(trimethylphenoxy)phenol (each isomer), tri(triethylphenoxy)phenol (each isomer), tri(phenylmethoxy)phenol, tri(phenylethoxy)phenol (each isomer), phenylphenol (each isomer), hydroxyphenylphenol (each isomer), hydroxyphenoxyphenol (each isomer), hydroxyphenyl-propylphenol (each isomer), and naphthol (each isomer).

Among these, as the aromatic hydroxy compound, in order to performing easy transfer, an aromatic hydroxy compound in which the total number of carbon atoms in $R^{611b}$, $R^{612b}$, $R^{613b}$, $R^{614b}$, and $R^{615b}$ is 0 or more and 13 or less is preferable.

In addition, an aromatic hydroxy compound in which the total number of carbon atoms in $R^{611b}$, $R^{612b}$, $R^{613b}$, $R^{614b}$, and $R^{615b}$ is 0 or more and 9 or less, and $R^{611b}$, $R^{612b}$, $R^{613b}$, $R^{614b}$, and $R^{615b}$ are each independently a hydrogen atom, a linear or branched alkyl group, a cycloalkyl group, a substituted or unsubstituted aryl group, a linear or branched alkoxy group, a substituted or unsubstituted aryloxy group, or a substituted or unsubstituted aralkyl group is more preferable.

<Carbamate compound>

Examples of the carbamate compound obtained by the production method according to the present embodiment include an N-substituted carbamate.

[N-substituted carbamate compound (Va)]

In the reaction of the primary amine compound, the urea, and the hydroxy compound, the N-substituted carbamate obtained in a case where the alcohol is used as the hydroxy compound is a compound represented by General Formula (Va) (hereinafter, may be referred to as "N-substituted carbamate compound (Va)").

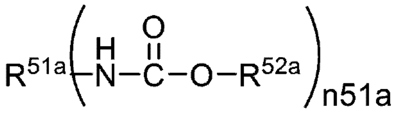
( Va )

(in General Formula (Va), $R^{51a}$ is an n51a-valent organic group and is a group derived from the primary amine compound, that is, $R^{51a}$ is the same as $R^{21b}$ described above, $R^{52a}$ is a group derived from an alcohol, that is, $R^{52a}$ is the same as $R^{61a}$ described above, and n51a is the same as n21b described above.)

Preferred examples of the N-substituted carbamate compound (Va) include 1) an N-aromatic monocarbamate compound in which $R^{51a}$ is a group having 6 or more and 85 or less carbon atoms, which contains one or more aromatic rings, and n51a is 1; 2) an N-aromatic polycarbamate compound in which $R^{51a}$ is a group having 6 or more and 85 or less carbon atoms, which contains one or more aromatic rings, and n51a is 2 or more; and 3) an N-aliphatic polycarbamate compound in which $R^{51a}$ is an aliphatic group having 1 or more and 85 or less carbon atoms, which may be substituted with an aromatic group, and n51a is 2 or more.

Specific examples of the N-substituted carbamate compound (Va) are shown below.

(N-aromatic monocarbamate compound)

The preferred N-aromatic monocarbamate compound is an N-aromatic monocarbamate compound represented by General Formula (Va-1).

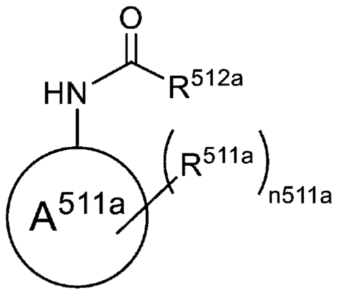
( Va-1 )

(in General Formula (Va-1), $R^{511a}$ is the same as $R^{211b}$ described above, n511a is the same as n211b described above, $R^{512a}$ is the same as $R^{61a}$ described above, and a ring $A^{511a}$ is the same as $A^{211b}$ described above)

(N-aromatic polycarbamate compound)

The N-aromatic polycarbamate compound is an N-aromatic polycarbamate compound in which $R^{51a}$ in General Formula (Va) is a group having 6 or more and 85 or less carbon atoms, which contains one or more aromatic rings, and n51a is 2 or more. Among these, in consideration of fluidity and the like, an N-aromatic polycarbamate compound in which $R^{51a}$ in General Formula (Va) is a group having 6 or more and 13 or less carbon atoms and containing one or more aromatic rings, which may be substituted with an aliphatic group or an aromatic group, and n51a is 2 or more is preferable.

Specific examples of the N-aromatic polycarbamate compound include polymethylene polyphenyl polycarbamate represented by General Formula (Va-2).

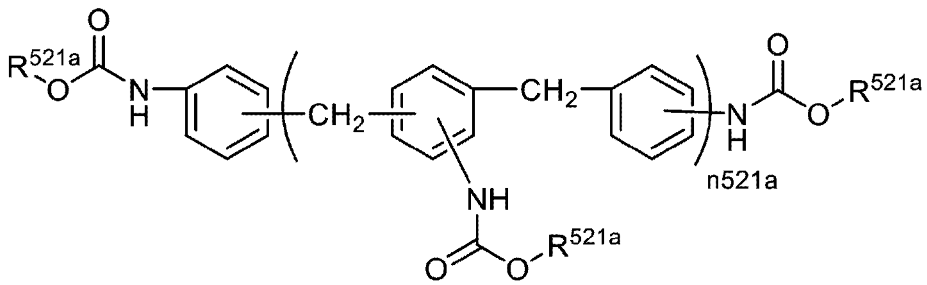

( Va-2 )

(in General Formula (Va-2), $R^{121a}$ is the same as $R^{61a}$ described above, and n521a is the same as n212b described above and is an integer of 0 or more and 6 or less)

(N-aliphatic polycarbamate compound)

The N-aliphatic polycarbamate compound is an N-aliphatic polycarbamate compound in which $R^{51a}$ in General Formula (Va) is an aliphatic group having 1 or more and 85 or less carbon atoms, which may be substituted with an aromatic group, and n51a is 2 or more.

As the N-aliphatic polycarbamate compound, an N-aromatic polycarbamate compound in which the aliphatic group as $R^{51a}$ is a chain-like aliphatic hydrocarbon group, a cyclic aliphatic hydrocarbon group, a group obtained by bonding a chain-like aliphatic hydrocarbon group and a cyclic aliphatic hydrocarbon group, or a group obtained by covalently bonding these groups to a specific non-metal atom (carbon, oxygen, nitrogen, sulfur, or silicon), and n51a is 2 or more is preferable.

In addition, an N-aliphatic polycarbamate compound in which the aliphatic group as $R^{51a}$ is an acyclic aliphatic hydrocarbon group, a cyclic aliphatic hydrocarbon group, a group obtained by bonding an acyclic aliphatic hydrocarbon group and a cyclic aliphatic hydrocarbon group, or a group obtained by covalently bonding these groups to a specific non-metal atom (carbon, oxygen, nitrogen, sulfur, or silicon), each of which has 1 or more and 70 or less carbon atoms, and n51a is 2 or more and 5 or less is more preferable.

In consideration of fluidity and the like when industrially producing in large quantities, an N-aliphatic polycarbamic acid ester in which $R^{51a}$ is an acyclic aliphatic hydrocarbon group, a cyclic aliphatic hydrocarbon group, a group obtained by bonding an acyclic aliphatic hydrocarbon group and a cyclic aliphatic hydrocarbon group, or a group obtained by covalently bonding these groups to a specific non-metal atom (carbon, oxygen, nitrogen, sulfur, or silicon), each of which consists of carbon atoms and hydrogen atoms and has 6 or more and 13 or less carbon atoms, and n51a is 2 or more and 4 or less is still more preferable.

The N-aliphatic polycarbamate compound is determined depending on the type of the primary amine compound and the alcohol used, and thus it is not possible to list all of N-aliphatic polycarbamate compounds. However, examples thereof include pentanediyl-di(methyl carbamic acid ester) (each isomer), N,N'-pentanediyl-di(ethyl carbamic acid ester) (each isomer), N,N'-pentanediyl-di(propyl carbamic acid ester) (each isomer), N,N'-pentanediyl-di(butyl carbamic acid ester) (each isomer), N,N'-pentanediyl-di(pentyl carbamic acid ester) (each isomer), N,N'-pentanediyl-di(hexyl carbamic acid ester) (each isomer), N,N'-pentanediyl-di(heptyl carbamic acid ester) (each isomer), N,N'-pentanediyl-di(octyl carbamic acid ester) (each isomer), N,N'-pentanediyl-di(nonyl carbamic acid ester) (each isomer), N,N'-pentanediyl-di(decyl carbamic acid ester) (each isomer), N,N'-pentanediyl-di(dodecyl carbamic acid ester) (each isomer), N,N'-pentanediyl-di(octadecyl carbamic acid ester) (each isomer), N,N'-hexanediyl-di(methyl carbamic acid ester) (each isomer), N,N'-hexanediyl-di(ethyl carbamic acid ester) (each isomer), N,N'-hexanediyl-di(propyl carbamic acid ester) (each isomer), N,N'-hexanediyl-di(butyl carbamic acid ester) (each isomer), N,N'-hexanediyl-di(pentyl carbamic acid ester) (each isomer), N,N'-hexanediyl-di(hexyl carbamic acid ester) (each isomer), N,N'-hexanediyl-di(heptyl carbamic acid ester) (each isomer), N,N'-hexanediyl-di(octyl carbamic acid ester) (each isomer), N,N'-hexamethylene-di(carbamic acid nonyl ester) (each isomer), N,N'-hexamethylene-di(carbamic acid decyl ester) (each isomer), N,N'-hexamethylene-di(carbamic acid dodecyl ester) (each isomer), N,N'-hexamethylene-di(carbamic acid octadecyl ester) (each isomer), N,N',N"-nonyl-tri(carbamic acid methyl ester) (each isomer), N,N',N"-nonyl-tri(carbamic acid ethyl ester) (each isomer), N,N',N"-nonyl-tri(carbamic acid propyl ester) (each isomer), N,N',N"-nonyl-tri(carbamic acid butyl ester) (each isomer), N,N',N"-nonyl-tri(carbamic acid pentyl ester) (each isomer), N,N',N"-nonyl-tri(carbamic acid hexyl ester) (each isomer), N,N',N"-nonyl-tri(carbamic acid heptyl ester) (each isomer), N,N',N"-nonyl-tri(carbamic acid octyl ester) (each isomer), N,N',N"-nonyl-tri(carbamic acid nonyl ester) (each isomer), N,N',N"-nonyl-tri(carbamic acid decyl ester) (each isomer), N,N',N"-nonyl-tri(carbamic acid dodecyl ester) (each isomer), N,N',N"-nonyl-tri(carbamic acid octadecyl ester) (each isomer), N,N'-methylenediphenylene-di(carbamic acid methyl ester) (each isomer), N,N'-methylenediphenylene-di(carbamic acid ethyl ester) (each isomer), N,N'-methylenediphenylene-di(carbamic acid propyl ester) (each isomer), N,N'-methylenediphenylene-di(carbamic acid butyl ester) (each isomer), N,N'-methylene diphenylene-di(pentyl ester of carbamic acid) (each isomer),

N,N'-methylene diphenylene-di(hexyl ester of carbamic acid) (each isomer), N,N'-methylene diphenylene-di(heptyl ester of carbamic acid) (each isomer), N,N'-methylene diphenylene-di(octyl ester of carbamic acid) (each isomer), N,N'-methylene diphenylene-di(nonyl ester of carbamic acid) (each isomer), N,N'-methylene diphenylene-di(decyl ester of carbamic acid) (each isomer), N,N'-methylene diphenylene-di(dodecyl ester of carbamic acid) (each isomer), N,N'-methylene diphenylene-di(octadecyl ester of carbamic acid) (each isomer), 3-(methoxycarbonylamino-methyl)-3,5,5-trimethylcyclohexylcarbamic acid methyl ester (each isomer), 3-(ethoxycarbonylamino-methyl)-3,5,5-trimethylcyclohexylcarbamic acid ethyl ester (each isomer), 3-(propyloxycarbonylamino-methyl)-3,5,5-trimethylcyclohexylcarbamic acid propyl ester (each isomer), 3-(buthyloxycarbonylamino-methyl)-3,5,5-trimethylcyclohexylcarbamic acid butyl ester (each isomer), 3-(pentylcarbamoylmethyl)-3,5,5-trimethylcyclohexylcarbamate pentyl ester (each isomer), 3-(hexylcarbamoylmethyl)-3,5,5-trimethylcyclohexylcarbamate hexyl ester (each isomer), 3-(heptylcarbamoylmethyl)-3,5,5-trimethylcyclohexylcarbamate heptyl ester (each isomer), 3-(octylcarbamoylmethyl)-3,5,5-trimethylcyclohexylcarbamate octyl ester (each isomer), 3-(nonylcarbamoylmethyl)-3,5,5-trimethylcyclohexylcarbamate nonyl ester (each isomer), 3-(decylcarbamoylmethyl)-3,5,5-trimethylcyclohexylcarbamate decyl ester (each isomer), 3-(dodecylcarbamoylmethyl)-3,5,5-trimethylcyclohexylcarbamate dodecyl ester (each isomer), 3-(octadecylcarbamoylmethyl)-3,5,5-trimethylcyclohexylcarbamate octadecyl ester (each isomer), toluene-di(methylcarbamate) (each isomer), toluene-di(ethylcarbamate) (each isomer), toluene-di(propylcarbamate) (each isomer), toluene-di(butylcarbamate) (each isomer), toluene-di(pentylcarbamate) (each isomer), toluene-di(carbamate hexyl ester) (each isomer), toluene-di(carbamate heptyl ester) (each isomer), toluene-di(carbamate octyl ester) (each isomer), toluene-di(carbamate nonyl ester) (each isomer), toluene-di(carbamate decyl ester) (each isomer), toluene-di(carbamate dodecyl ester) (each isomer), toluene-di(carbamate octadecyl ester) (each isomer), N,N'-methylene dicyclohexyl-di(methyl carbamate) (each isomer), N,N'-methylene dicyclohexyl-di(ethyl carbamate) (each isomer), N,N'-methylene dicyclohexyl-di(propyl carbamate) (each isomer), N,N'-methylene dicyclohexyl-di(butyl carbamate) (each isomer), N,N'-methylene dicyclohexyl-di(pentyl carbamate) (each isomer), N,N'-methylene dicyclohexyl-di(hexyl carbamate) (each isomer), N,N'-methylene dicyclohexyl-di(heptyl carbamate) (each isomer), N,N'-methylene dicyclohexyl-di(octyl carbamate) (each isomer), N,N'-methylene dicyclohexyl-di(nonyl carbamate) (each isomer), N,N'-methylene dicyclohexyl-di(decyl carbamate) (each isomer), N,N'-methylene dicyclohexyl-di(decyl carbamic acid ester) (each isomer), N,N'-methylene dicyclohexyl-di(octadecyl carbamic acid ester) (each isomer), N,N'-bis(methoxycarbonyl) lysine methyl ester, N,N'-bis(ethoxycarbonyl) lysine ethyl ester, N,N'-bis(propyloxycarbonyl) lysine propyl ester, N,N',N"-tris(methoxycarbonyl) lysine (2-aminoethyl) ester, N-phenylcarbamic acid methyl ester (each isomer), N-phenylcarbamic acid ethyl ester (each isomer), N-phenylcarbamic acid propyl ester (each isomer), N-phenylcarbamic acid butyl ester (each isomer), N-phenylcarbamic acid pentyl ester (each isomer), N-phenylcarbamic acid hexyl ester (each isomer), N-phenylcarbamic acid heptyl ester (each isomer), N-phenylcarbamic acid octyl ester (each isomer), N-phenylcarbamic acid nonyl ester (each isomer), N-phenylcarbamic acid decyl ester (each isomer), N-phenylcarbamic acid dodecyl ester (each isomer), N-phenylcarbamic acid octadecyl ester (each isomer), N-dimethylphenylcarbamic acid methyl ester (each isomer), N-dimethylphenylcarbamic acid ethyl ester (each isomer), N-dimethylphenylcarbamic acid propyl ester (each isomer), N-dimethylphenylcarbamic acid butyl ester (each isomer), N-dimethylphenylcarbamic acid pentyl ester (each isomer), N-dimethylphenylcarbamic acid hexyl ester (each isomer), N-dimethylphenylcarbamic acid heptyl ester (each isomer), N-dimethylphenylcarbamic acid octyl ester (each isomer), N-dimethylphenylcarbamic acid nonyl ester (each isomer), N-dimethylphenylcarbamic acid decyl ester (each isomer), N-dimethylphenylcarbamic acid dodecyl ester (each isomer), and N-dimethylphenylcarbamic acid octadecyl ester (each isomer).

[N-substituted carbamate compound (Vb)]

In the reaction of the primary amine compound, the urea, and the hydroxy compound, the N-substituted carbamate obtained in a case where the aromatic hydroxy compound is used as the hydroxy compound is a compound represented by General Formula (Vb) (hereinafter, may be referred to as "N-substituted carbamate compound (Vb)").

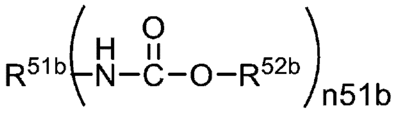

( Vb )

(in General Formula (Vb), $R^{51b}$ is an n51b-valent organic group and is a group derived from the primary amine compound, that is, $R^{51b}$ is the same as $R^{21b}$ described above, $R^{52b}$ is a group derived from the aromatic hydroxy compound, that is, $R^{52b}$ is the same as $R^{61b}$ described above, and n51b is the same as n21b described above)

Preferred examples of the N-substituted carbamate compound (Vb) include:

1) an N-aromatic monocarbamate compound in which $R^{51b}$ is an aromatic group having 6 or more and 85 or less carbon atoms, which may be substituted with an aliphatic group or an aromatic group, and n51b is 1;

2) an N-aromatic polycarbamate compound in which $R^{51b}$ is a group having 6 or more and 85 or less carbon atoms and containing one or more aromatic rings, which may be substituted with an aliphatic group or an aromatic group, and n51b is 2 or more; and

3) an N-aliphatic polycarbamate compound in which $R^{51b}$ is an aliphatic group having 1 or more and 85 or less carbon atoms, which may be substituted with an aromatic group, and n51b is 2 or more.

Specific examples of the N-substituted carbamate compound (Vb) are shown below.

(N-aromatic monocarbamate compound)

The preferred N-aromatic monocarbamate compound is an N-aromatic monocarbamate compound represented by General Formula (Vb).

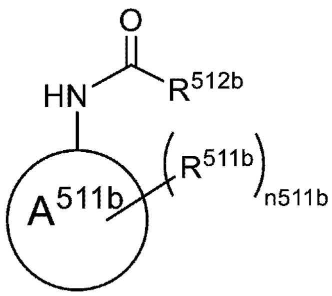

( Vb-1 )

(in General Formula (Vb-1), $R^{511b}$ is the same as $R^{211b}$ described above, n511b is the same as n211b described above, $R^{512b}$ is the same as $R^{61b}$ described above, and a ring $A^{511b}$ is the same as $A^{61b}$ described above)

(N-aromatic polycarbamate compound)

The N-aromatic polycarbamate compound is an N-aromatic polycarbamate compound in which $R^{51b}$ in General Formula (Vb) is a group having 6 or more and 85 or less carbon atoms and containing one or more aromatic rings, which may be substituted with an aliphatic group or an aromatic group, and n51b is 2 or more.

Among these, in consideration of fluidity and the like, an N-aromatic polycarbamate compound in which the $R^{51b}$ group in General Formula (Vb) is a group having 6 or more and 13 or less carbon atoms and substituted with an alkyl group, an aryl group, or an aralkyl group, and n51b is 2 or more and 8 or less is preferable.

In addition, preferred examples of the N-aromatic polycarbamate compound include polymethylene polyphenyl polycarbamate represented by General Formula (Vb-2).

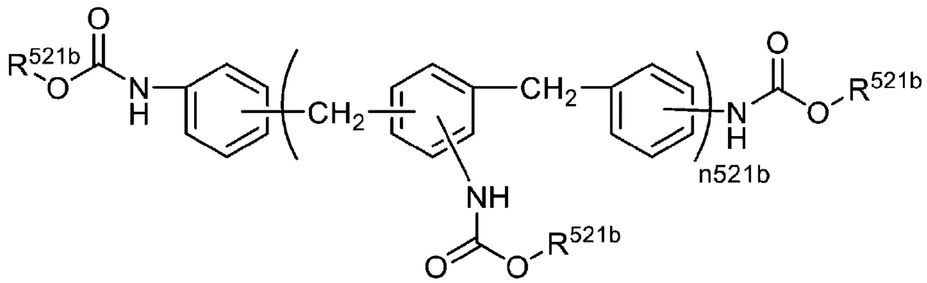

( Vb-2 )

(in General Formula (Vb-2), $R^{521b}$ is the same as $R^{61b}$ described above, and n521b is the same as 212b described above and is an integer of 0 or more and 6 or less)

(N-aliphatic polycarbamate compound)

The N-aliphatic polycarbamate compound is an N-aromatic polycarbamate compound in which $R^{51b}$ in General Formula (Vb) is a group having 6 or more and 85 or less carbon atoms and containing one or more aromatic rings, which may be substituted with an aliphatic group or an aromatic group, and n51b is 2 or more.

Among these, the N-aliphatic polycarbamate compound is preferably an N-aliphatic polycarbamate compound in which the aliphatic group as $R^{51b}$ is an acyclic aliphatic hydrocarbon group, a cyclic aliphatic hydrocarbon group, a

group obtained by bonding an acyclic aliphatic hydrocarbon group and a cyclic aliphatic hydrocarbon group, or a group obtained by covalently bonding these groups to a specific non-metal atom (carbon, oxygen, nitrogen, sulfur, or silicon), each of which has 1 or more and 70 or less carbon atoms, and n51b is 2 or more.

In addition, an N-aliphatic polycarbamate compound in which $R^{51b}$ is an acyclic aliphatic hydrocarbon group, a cyclic aliphatic hydrocarbon group, a group obtained by bonding an acyclic aliphatic hydrocarbon group and a cyclic aliphatic hydrocarbon group, or a group obtained by covalently bonding these groups to a specific non-metal atom (carbon, oxygen, nitrogen, sulfur, or silicon), each of which has 1 or more and 70 or less carbon atoms, and n51b is 2 or more and 5 or less is more preferable.

In consideration of fluidity and the like when industrially producing in large quantities, an N-aliphatic polycarbamate compound in which $R^{51b}$ is an acyclic aliphatic hydrocarbon group, a cyclic aliphatic hydrocarbon group, a group obtained by bonding an acyclic aliphatic hydrocarbon group and a cyclic aliphatic hydrocarbon group, or a group obtained by covalently bonding these groups to a specific non-metal atom (carbon, oxygen, nitrogen, sulfur, or silicon), each of which consists of carbon atoms and hydrogen atoms and has 5 or more and 13 or less carbon atoms, and n51b is 2 or more and 4 or less is still more preferable.

The N-aliphatic polycarbamate compound is determined depending on the type of the primary amine compound and the aromatic hydroxy compound used, and thus it is not possible to list all of N-aliphatic polycarbamate compounds. However, examples thereof include N,N'-pentanediyl-di(phenyl carbamic acid ester), N,N'-pentanediyl-di(methylphenyl carbamic acid ester), N,N'-pentanediyl-di(ethylphenyl carbamic acid ester), N,N'-pentanediyl-di(propylphenyl carbamic acid ester), N,N'-pentanediyl-di(butylphenyl carbamic acid ester), N,N'-pentanediyl-di(pentylphenyl carbamic acid ester), N,N'-pentanediyl-di(hexylphenyl carbamic acid ester), N,N'-pentanediyl-di(dimethylphenyl carbamic acid ester), N,N'-pentanediyl-di(trimethylphenyl carbamic acid ester), N,N'-pentanediyl-di(cumyl carbamic acid ester), N,N'-hexanediyl-di(phenyl carbamic acid ester), N,N'-hexamethylene-di(carbamate(methylphenyl)ester) (each isomer), N,N'-hexamethylene-di(carbamate(ethylphenyl)ester) (each isomer), N,N'-hexamethylene-di(carbamate(propylphenyl)ester) (each isomer), N,N'-hexamethylene-di(carbamate(butylphenyl)ester) (each isomer), N,N'-hexamethylene-di(carbamate(pentylphenyl)ester) (each isomer), N,N'-hexamethylene-di(carbamate(hexylphenyl)ester) (each isomer), N,N'-hexamethylene-di(carbamate(dimethylphenyl)ester) (each isomer), N,N'-hexamethylene-di(carbamate(trimethylphenylphenyl)ester) (each isomer), N,N'-hexamethylene-di(carbamate kumyl ester) (each isomer), N,N',N''-nonyl-tri(carbamate phenyl ester) (each isomer), N,N',N''-nonyl-tri(carbamate methylphenyl ester) (each isomer), N,N',N''-nonyl-tri(carbamate ethylphenyl ester) (each isomer), N,N',N''-nonyl-tri(carbamate propylphenyl ester) (each isomer), N,N',N''-nonyl-tri(carbamate butylphenyl ester) (each isomer), N,N',N''-nonyl-tri(carbamate pentylphenyl ester) (each isomer), N,N',N''-nonyl-tri(carbamic acid hexylphenyl ester) (each isomer), N,N',N''-nonyl-tri(carbamic acid dimethylphenyl ester) (each isomer), N,N',N''-nonyl-tri(carbamic acid trimethylphenyl ester) (each isomer), N,N',N''-nonyl-tri(carbamic acid cymyl ester) (each isomer), N,N'-methylene diphenylene-di(phenyl ester) (each isomer), N,N'-methylene diphenylene-di(methylphenyl ester) (each isomer), N,N'-methylene diphenylene-di(ethylphenyl ester) (each isomer), N,N'-methylene diphenylene-di(propylphenyl ester) (each isomer), N,N'-methylene diphenylene-di(butylphenyl ester) (each isomer), N,N'-methylene diphenylene-di(pentylphenyl ester) (each isomer), N,N'-methylene diphenylene-di(hexylphenyl ester) (each isomer), N,N'-methylene diphenylene-di(dimethylphenyl ester) (each isomer), N,N'-methylene diphenylene-di(carbamic acid (trimethylphenyl) ester) (each isomer), N,N'-methylene diphenylene-di(carbamic acid cumyl ester) (each isomer), 3-(phenoxycarbonylamino-methyl)-3,5,5-trimethylcyclohexylcarbamate phenyl ester, 3-((methylphenoxy)carbonylamino-methyl)-3,5,5-trimethylcyclohexylcarbamate (methylphenyl) ester (each isomer), 3-((ethylphenoxy)carbonylamino-methyl)-3,5,5-trimethylcyclohexylcarbamate (ethylphenyl) ester (each isomer), 3-((propylphenoxy)carbonylamino-methyl)-3,5,5-trimethylcyclohexylcarbamate (propylphenyl) ester (each isomer), 3-((butylphenoxy)carbonylamino-methyl)-3,5,5-trimethylcyclohexylcarbamic acid (butylphenyl) ester (each isomer), 3-((pentylphenoxy)carbonylamino-methyl)-3,5,5-trimethylcyclohexylcarbamic acid (pentylphenyl) ester (each isomer), 3-((hexylphenoxy)carbonylamino-methyl)-3,5,5-trimethylcyclohexylcarbamic acid (hexylphenyl) ester (each isomer), 3-((dimethylphenoxy)carbonylamino-methyl)-3,5,5-trimethylcyclohexylcarbamic acid (dimethylphenyl) ester (each isomer), 3-((trimethylphenoxy)carbonylamino-methyl)-3,5,5-trimethylcyclohexylcarbamate (trimethylphenyl) ester (each isomer), 3-((kumyloxy)carbonylamino-methyl)-3,5,5-trimethylcyclohexylcarbamate kumyl ester (each isomer), toluene-di(phenylcarbamate) (each isomer), toluene-di(methylphenylcarbamate) (each isomer), toluene-di(ethylphenylcarbamate) (each isomer), toluene-di(propylphenylcarbamate) (each isomer), toluene-di(butylphenylcarbamate) (each isomer), toluene-di(pentylphenylcarbamate) (each isomer), toluene-di(hexylphenylcarbamate) (each isomer), toluene-di(dimethylphenylcarbamate) (each isomer), toluene-di(trimethylphenylcarbamate) (each isomer), toluene-di(kumylcarbamate) (each isomer), N,N'-methylene dicyclohexyl-di(phenylcarbamate) (each isomer), N,N'-methylene dicyclohexyl-di(carbamate (methylphenyl) ester) (each isomer), N,N'-methylene dicyclohexyl-di(carbamate (ethylphenyl) ester) (each isomer), N,N'-methylene dicyclohexyl-di(carbamate (propylphenyl) ester) (each isomer), N,N'-methylene dicyclohexyl-di(carbamate (butylphenyl) ester) (each isomer), N,N'-methylene dicyclohexyl-di(carbamate (pentylphenyl) ester) (each isomer), N,N'-methylene dicyclohexyl-di(carbamate (hexylphenyl) ester) (each isomer), N,N'-methylene dicyclohexyl-di(carbamate (dimethylphenyl) ester) (each isomer), N,N'-methylene dicyclohexyl-di(carbamate (trimethylphenyl) ester) (each isomer), N,N'-methylene dicyclohexyl-di(carbamate kumyl ester) (each isomer), [amino acid] N,N'-bis(phenoxy-carbonyl) lysine phenyl ester, N,N',N"-tris(phenoxy-carbonyl) lysine (2-aminoethyl) ester, N-phenylcarbamate phenyl ester, N-phenylcarbamate (methylphenyl) ester (each isomer), N-phenylcarbamate (ethylphenyl) ester (each isomer), N-phenylcarbamate (propylphenyl) ester (each isomer), N-phenylcarbamate (butylphenyl) ester (each isomer), N-phenylcarbamic acid (pentylphenyl) ester (each isomer), N-phenylcarbamic acid (hexylphenyl) ester (each isomer), N-phenylcarbamic acid (heptylphenyl) ester (each isomer), N-phenylcarbamic acid (octylphenyl) ester (each isomer), N-phenylcarbamic acid (nonylphenyl) ester (each isomer), N-phenylcarbamic acid (decylphenyl) ester (each isomer), N-phenylcarbamic acid (dodecylphenyl) ester (each isomer), N-phenylcarbamic acid (octadecylphenyl) ester (each isomer), N-phenylcarbamic acid (dimethylphenyl) ester (each isomer), N-phenylcarbamic acid (diethylphenyl) ester (each isomer), N-phenylcarbamic acid (dipropylphenyl) ester (each isomer), N-phenylcarbamic acid (dibutylphenyl) ester (each isomer), N-phenylcarbamic acid (dipentylphenyl) ester (each isomer), N-phenylcarbamic acid (dihexylphenyl) ester (each isomer), N-phenylcarbamic acid (dihexylphenyl) ester (each isomer), N-phenylcarbamic acid (dioctylphenyl) ester (each isomer), N-phenylcarbamic acid (dinonylphenyl) ester (each isomer), dioctylphenyl) ester (each isomer), N-phenylcarbamic acid (dinonylphenyl) ester (each isomer), N-phenylcarbamic acid (didecylphenyl) ester (each isomer), N-phenylcarbamic acid (didodecylphenyl) ester (each isomer), N-phenylcarbamic acid phenyl ester, N-phenylcarbamic acid (methylphenyl) ester (each isomer), N-phenylcarbamic acid (ethylphenyl) ester (each isomer), N-phenylcarbamic acid (propylphenyl) ester (each isomer), N-phenylcarbamic acid (butylphenyl) ester (each isomer), N-phenylcarbamic acid (pentylphenyl) ester (each isomer), N-phenylcarbamic acid (hexylphenyl) ester (each isomer), N-phenylcarbamic acid (heptylphenyl) ester (each isomer), N-phenylcarbamic acid (octylphenyl) ester (each isomer), N-phenylcarbamic acid (nonylphenyl) ester (each isomer), N-phenylcarbamic acid (decylphenyl) ester (each isomer), N-phenylcarbamic acid (dodecylphenyl) ester (each isomer), N-phenylcarbamic acid (octadecylphenyl) ester (each isomer), N-phenylcarbamic acid (dimethylphenyl) ester (each isomer), N-phenylcarbamic acid (diethylphenyl) ester (each isomer), N-phenylcarbamic acid (dipropylphenyl) ester (each isomer), N-phenylcarbamic acid (dibutylphenyl) ester (each isomer), N-phenylcarbamic acid (dipentylphenyl) ester (each isomer), N-phenylcarbamic acid (dihexylphenyl) ester (each isomer), N-phenylcarbamic acid (diheptylphenyl) ester (each isomer), N-phenylcarbamic acid (dioctylphenyl) ester (each isomer), N-phenylcarbamic acid (dinonylphenyl) ester (each isomer), N-phenylcarbamic acid (didecylphenyl) ester (each isomer), N-phenylcarbamic acid (didodecylphenyl) ester (each isomer), N-phenylcarbamic acid (dioctadecylphenyl) ester (each isomer), N-dimethylphenylcarbamic acid phenyl ester (each isomer), N-dimethylphenylcarbamic acid (methylphenyl) ester (each isomer), N-dimethylphenylcarbamic acid (ethylphenyl) ester (each isomer), N-dimethylphenylcarbamic acid (propylphenyl) ester (each isomer), N-dimethylphenylcarbamic acid (butylphenyl) ester (each isomer), N-dimethylphenylcarbamic acid (pentylphenyl) ester (each isomer), N-dimethylphenylcarbamic acid (hexylphenyl) ester (each isomer), N-dimethylphenylcarbamic acid (heptylphenyl) ester (each isomer), N-dimethylphenylcarbamic acid (octylphenyl) ester (each isomer), N-dimethylphenylcarbamic acid (nonylphenyl) ester (each isomer), N-dimethylphenylcarbamic acid (decylphenyl) ester (each isomer), N-dimethylphenylcarbamic acid (dodecylphenyl) ester (each isomer), N-dimethylphenylcarbamic acid (octadecylphenyl) ester (each isomer), N-dimethylphenylcarbamic acid (dimethylphenyl) ester (each isomer), N-dimethylphenylcarbamic acid (diethylphenyl) ester (each isomer), N-dimethylphenylcarbamic acid (dipropylphenyl) ester (each isomer), N-dimethylphenylcarbamic acid (dibutylphenyl) ester (each isomer), N-dimethylphenylcarbamic acid (dipentylphenyl) ester (each isomer), N-dimethylphenylcarbamic acid (dihexylphenyl) ester (each isomer), N-dimethylphenylcarbamic acid (diheptylphenyl) ester (each isomer), N-dimethylphenylcarbamic acid (dioctylphenyl) ester (each isomer), N-dimethylphenylcarbamic acid (dinonylphenyl) ester (each isomer), N-dimethylphenylcarbamic acid (didecylphenyl) ester (each isomer), N-dimethylphenylcarbamic acid (didodecylphenyl) ester (each isomer), and N-dimethylphenylcarbamic acid (dioctadecylphenyl) ester (each isomer).

<<Method for producing isocyanate compound>>

The method for producing an isocyanate compound according to the present embodiment includes a thermal decomposition step of thermally decomposing the carbamate compound obtained by the above-described production method.

Hereinafter, the step in the method for producing an isocyanate compound according to the present embodiment will be described in detail.

<Thermal decomposition step>

In the thermal decomposition step, the carbamate compound obtained by the above-described method for producing a carbamate compound is thermally decomposed.

The reaction temperature at which the carbamate compound is thermally decomposed is usually in a range of 100°C or higher and 300°C or lower, and a high temperature is preferable in order to increase the reaction rate. On the other hand, at a high temperature, a side reaction may be caused by one or more compounds selected from the group consisting of the carbamate compound and the isocyanate compound, which is a product. Therefore, the temperature

is preferably in a range of 150°C or higher and 280°C or lower. In order to keep the reaction temperature constant, a known cooling device and a heating device may be installed in the above-described reactor.

In addition, the reaction pressure varies depending on the kinds of the compounds to be used and the reaction temperature, but may be any of a reduced pressure, a normal pressure, and a pressurized pressure, and is usually in a range of 20 Pa or more and $1 \times 10^6$ Pa or less.

The reaction time (in a case of the continuous method, the residence time) is not particularly limited, and is usually 0.001 hours or more and 100 hours or less, preferably 0.005 hours or more and 50 hours or less, and more preferably 0.01 hours or more and 10 hours or less.

In the thermal decomposition reaction, it is not always necessary to use a reaction solvent other than the hydroxy compound, but a suitable solvent can be used for the purpose of facilitating the reaction operation. Examples of the reaction solvent include the same one as that described in the method (1A).

The carbamate compound may cause a side reaction in a case where the carbamate compound is held at a high temperature for a long period of time. Therefore, it is preferable that the time during which the carbamate compound and the isocyanate compound are held at a high temperature is as short as possible. Therefore, the thermal decomposition reaction is preferably carried out by a continuous method. The continuous method is a method in which a mixture containing a carbamate compound is continuously supplied to a reactor to be subjected to a thermal decomposition reaction, and the isocyanate compound and the hydroxy compound to be produced are continuously extracted from the thermal decomposition reactor. In the continuous method, the low-boiling-point component generated by the thermal decomposition reaction of the carbamate compound is preferably recovered from the upper part of the thermal decomposition reactor as a gas-phase component, and the remainder is recovered from the bottom portion of the thermal decomposition reactor as a liquid-phase component. All the compounds present in the thermal decomposition reactor can be recovered as gas-phase components, but by allowing the liquid-phase components to be present in the thermal decomposition reactor, there is an effect of dissolving a polymer-like compound generated by a side reaction caused by one or more compounds selected from the group consisting of a carbamate compound and an isocyanate compound and preventing the polymer-like compound from adhering and accumulating in the thermal decomposition reactor. The isocyanate compound and the hydroxy compound are generated by the pyrolytic reaction of the carbamate compound, but at least one of these compounds is recovered as a gas-phase component. The compound to be recovered as a gas-phase component depends on the conditions of the thermal decomposition reaction and the like.

The form of the thermal decomposition reactor is not particularly limited, but it is preferable to use a known distillation device in order to efficiently recover the gas-phase component. For example, various known methods are used, such as a method using a reactor including any one of a distillation column, a multi-stage distillation column, a multi-tube type reactor, a continuous multi-stage distillation column, a filled column, a thin film evaporator, a reactor having a support inside, a forced circulation reactor, a falling film evaporator, and a falling drop evaporator, and a method in which these reactors are combined. From the viewpoint of rapidly removing the low-boiling-point component from the reaction system, the method is preferably a method using a reactor such as a tubular reactor, and more preferably a tubular thin film evaporator, a tubular falling film evaporator, or the like, and a structure having a large gas-liquid contact area in which the low-boiling point component to be produced is rapidly moved to the gas phase is preferable.

The material of the thermal decomposition reactor and the line may be any known material as long as it does not have an adverse effect on the carbamate compound, the hydroxy compound and the isocyanate compound which are products, and the like. Among these, SUS304, SUS316, SUS316L, and the like are inexpensive and can be preferably used.

The recovered isocyanate compound may contain a hydroxy compound or the like depending on the reaction conditions, the conditions for recovering the isocyanate compound, the reaction apparatus, and the like. In such a case, the operation such as distillation can be further performed to obtain an isocyanate compound having a desired purity.

The isocyanate compound may be purified after obtaining the isocyanate compound by the thermal decomposition of the carbamate compound. It is preferable to separate or remove the catalyst used in the synthesis of the carbamate compound between the thermal decomposition reaction and the purification. In a state where the isocyanate compound and the above-described catalyst coexist, there are cases where an undesirable phenomenon such as denaturation or discoloration of the isocyanate compound occurs. The isocyanate-modified product causes the pipe to be clogged and significantly makes continuous operation difficult.

As a method for removing the catalyst, a known method can be used, and a method such as membrane separation, distillation separation, crystallization, chemical adsorption, or physical adsorption can be preferably used. The catalyst formed of an amine compound having no active hydrogen, which is used in the method for producing a carbamate compound of the present embodiment, is more easily removed than an inorganic base and is effective in suppressing the denaturation of the isocyanate compound in the thermal decomposition step of the carbamate compound.

In a case where the low-boiling-point component generated by the thermal decomposition reaction of the carbamate compound is mainly a hydroxy compound, when a catalyst having a higher vapor pressure than the hydroxy compound is used in the production of the carbamate compound, the catalyst can be separated from the isocyanate

compound together with the hydroxy compound without providing a step for removing the catalyst, which is economical.

On the other hand, in a case where the low-boiling-point component generated by the thermal decomposition reaction of the carbamate compound is mainly an isocyanate compound, in a case where a catalyst having a lower vapor pressure than that of the hydroxy compound is used in the production of the carbamate compound, the generated isocyanate compound is rapidly separated from the catalyst, which is effective for suppressing the modification of the isocyanate.

The "low-boiling-point component generated by the thermal decomposition reaction of the carbamate compound" referred to herein corresponds to one or more compounds selected from the group consisting of a hydroxy compound and an isocyanate compound generated by the thermal decomposition reaction of the carbamate compound, but particularly refers to a compound that can be present as a gas under the conditions in which the thermal decomposition reaction is carried out.

In addition, in the method for producing an isocyanate compound according to the present embodiment, other steps other than the thermal decomposition step can be added as necessary. Examples of other steps include a step of separating and preliminarily concentrating a part or all of the hydroxy compound from the reaction solution containing the carbamate compound obtained by the above-described production method (also referred to as "carbamate concentration step") before the thermal decomposition step; and a step of purifying the isocyanate compound.

Hereinafter, the products in the method for producing an isocyanate compound according to the present embodiment will be described in detail.

<Isocyanate compound>

As the isocyanate compound obtained by the method for producing an isocyanate compound according to the present embodiment, a compound represented by General Formula (Vile) (hereinafter, may be referred to as "isocyanate compound (VIIc)") is preferably used.

$$R^{71c}\left(-N=C=O\right)_{n71c} \qquad (VIIc)$$

(in General Formula (VIIc), $R^{71c}$ is an n71c-valent organic group, and n71c is an integer of 1 or more and 12 or less)

[$R^{71c}$]

$R^{71c}$ is an n71c-valent organic group. That is, $R^{71c}$ is a monovalent to dodecavalent organic group. Among these, $R^{71c}$ is preferably an organic group consisting of a carbon atom, an oxygen atom, and a hydrogen atom, and more preferably an organic group having no active hydrogen.

As the aliphatic hydrocarbon group as $R^{71c}$, an alkylene group or an alkanetriyl group, a cycloalkyl group, a cycloalkylene group or a cycloalkanetriyl group, or a group constituting the alkyl group, the alkylene group or the alkanetriyl group, and the cycloalkyl group, the cycloalkylene group or the cycloalkanetriyl group is preferable; and a linear or branched alkylene group or alkanetriyl group, a cycloalkylene group or a cycloalkanetriyl group, or a group constituting the alkylene group or the alkanetriyl group, and the cycloalkyl group, the cycloalkylene group or the cycloalkanetriyl group is more preferable.

Examples of the linear or branched alkylene group include a methylene group, an ethylene group, a propylene group, a trimethylene group, a pentylene group, an n-hexylene group, and a decamethylene group.

Examples of the cycloalkylene group include a cyclobutylene group and a cyclohexylene group.

Examples of the linear or branched alkanetriyl group include a hexanetriyl group, a nonanetriyl group, and a decanetriyl group.

Examples of the cycloalkanetriyl group include a cyclopropanetriyl group, a cyclobutanetriyl group, a cyclopentatriyl group, and a cyclohexatriyl group.

The aromatic hydrocarbon group as $R^{71c}$ is preferably a group having a substituted or unsubstituted aromatic ring having 6 or more and 13 or less carbon atoms. Examples of the substituent include an alkyl group, an aryl group, and an aralkyl group. The aromatic ring may be an aromatic hydrocarbon ring or a heteroaromatic ring, and specific examples thereof include a benzene ring, a naphthalene ring, and a pyridine ring.

Examples of the preferred isocyanate compound (VIIc) include a monofunctional isocyanate compound, a bifunctional isocyanate compound, and a polyfunctional isocyanate compound.

Examples of the monofunctional isocyanate compound include isocyanate compounds having a saturated hydrocarbon group, such as methyl isocyanate, ethyl isocyanate, propyl isocyanate (each isomer), butyl isocyanate (each isomer), pentyl isocyanate (each isomer), hexyl isocyanate (each isomer), octyl isocyanate (each isomer), nonyl

isocyanate (each isomer), decyl isocyanate (each isomer), undecyl isocyanate (each isomer), dodecyl isocyanate (each isomer), tridecyl isocyanate (each isomer), tetradecyl isocyanate (each isomer), pentadecyl isocyanate (each isomer), hexadecyl isocyanate (each isomer), heptadecyl isocyanate (each isomer), octadecyl isocyanate (each isomer), nonadecyl isocyanate (each isomer), and icosyl isocyanate (each isomer); isocyanate compounds having an unsaturated bond, such as 2-isocyanatomethyl acrylate, 2-isocyanatoethyl acrylate, 2-isocyanatopropyl acrylate (each isomer), 2-isocyanatobutyl acrylate (each isomer), 2-isocyanatomethyl methacrylate, 2-isocyanatoethyl methacrylate, 2-isocyanatopropyl methacrylate (each isomer), 2-isocyanatobutyl methacrylate (each isomer), phenyl isocyanate, and benzyl isocyanate; isocyanate compounds having an ether group, such as (S)-2-isocyanato-3-tarshaributoxypropionic acid methyl, (S)-2-isocyanato-3-tarshaributoxypropionic acid ethyl, (S)-2-isocyanato-3-tarshaributoxypropionic acid propyl (each isomer), (S)-2-isocyanato-3-tarshaributoxypropionic acid butyl (each isomer), (S)-2-isocyanato-3-tarshaributoxypropionic acid pentyl (each isomer), (S)-2-isocyanato-3-tarshaributoxypropionic acid hexyl (each isomer), and (S)-2-isocyanato-3-tarshaributoxypropionic acid dodecyl (each isomer); isocyanate compounds having a halogen group, such as fluorophenyl isocyanate (each isomer), chlorophenyl isocyanate (each isomer), bromophenyl isocyanate (each isomer), and iodophenyl isocyanate (each isomer); and isocyanate compounds having a carbonyl group, such as glycine methyl ester isocyanate, glycine ethyl ester isocyanate, glycine propyl ester isocyanate (each isomer), glycine butyl ester isocyanate (each isomer), glycine pentyl ester isocyanate (each isomer), glycine hexyl ester isocyanate (each isomer), glycine decyl ester isocyanate (each isomer), leucine methyl ester isocyanate (methyl 2-isocyanato-4-methylpentanoate), leucine ethyl ester isocyanate (ethyl 2-isocyanato-4-methylpentanoate), leucine propyl ester isocyanate (each isomer), leucine butyl ester isocyanate (each isomer), leucine pentyl ester isocyanate (each isomer), leucine hexyl ester isocyanate (each isomer), leucine decyl ester isocyanate (each isomer), and ethyl isocyanatoacetate.

Examples of the bifunctional isocyanate compound include isocyanate compounds having a saturated hydrocarbon group, such as dimethylene diisocyanate, trimethylene diisocyanate (each isomer), tetramethylene diisocyanate (each isomer), pentamethylene diisocyanate (each isomer), hexamethylene diisocyanate (each isomer), heptamethylene diisocyanate (each isomer), octamethylene diisocyanate (each isomer), diisocyanatocyclohexane (each isomer), bisisocyanatomethylcyclohexane (each isomer), diisocyanic acid isophorone (each isomer), dicyclohexylmethane diisocyanate (each isomer), diisocyanatodimethylpropane (each isomer), diisocyanatodimethylpentane (each isomer), diisocyanatodimethylhexane (each isomer), diisocyanatodimethylheptane (each isomer), diisocyanatodimethyloctane (each isomer), diisocyanatodimethylnonane (each isomer), diisocyanatodimethyldecane (each isomer), diisocyanatomethylethylpropane (each isomer), diisocyanatomethylethylbutane (each isomer), diisocyanatomethylethylpentane (each isomer), diisocyanatomethylethylhexane (each isomer), diisocyanatomethylethylheptane (each isomer), diisocyanatomethylethyloctane (each isomer), diisocyanatodiethylpropane (each isomer), diisocyanatodiethylpentane (each isomer), diisocyanatodiethylhexane (each isomer), diisocyanatodiethylheptane (each isomer), diisocyanatodiethyloctane (each isomer), and diisocyanatodiethylnonane (each isomer); isocyanate compounds having an unsaturated hydrocarbon group, such as diphenylmethane diisocyanate (each isomer), tolylene diisocyanate (each isomer), naphthalene diisocyanate (each isomer), xylylene diisocyanate (each isomer), tetramethylxylylene diisocyanate (each isomer), methylenebis(diisoamylphenylene) diisocyanate (each isomer), oxybis(phenylene isocyanate) (each isomer), carbonylbis(phenylene) diisocyanate (each isomer), butene diisocyanate (each isomer), and butynylene diisocyanate; and isocyanate compounds having an ester group, such as lysine methyl ester isocyanate, lysine ethyl ester isocyanate, lysine propyl ester isocyanate (each isomer), lysine butyl ester isocyanate (each isomer), lysine pentyl ester isocyanate (each isomer), and lysine hexyl ester isocyanate (each isomer).

Examples of the polyfunctional isocyanate include isocyanate compounds having a saturated hydrocarbon group, such as propylene triisocyanate, butane triisocyanate (each isomer), pentane triisocyanate (each isomer), hexane triisocyanate (each isomer), heptane triisocyanate (each isomer), octane triisocyanate (each isomer), nonane triisocyanate (each isomer), decane triisocyanate (each isomer), undecane triisocyanate (each isomer), dodecane triisocyanate (each isomer), tridecane triisocyanate (each isomer), tetradecane triisocyanate (each isomer), pentadecane triisocyanate (each isomer), hexadecane triisocyanate (each isomer), heptadecane triisocyanate (each isomer), octadecane triisocyanate (each isomer),nonadecane triisocyanate (each isomer), and eicosane triisocyanate (each isomer); isocyanate compounds having an unsaturated hydrocarbon group, such as polymeric MDI (each isomer); isocyanate compounds having an ester group, such as 2-isocyanatoethyl 2,6-diisocyanatohexanoate; and isocyanate compounds obtained by crosslinking a bi- or higher functional isocyanate compound having an isocyanurate group, a bi- or higher functional isocyanate compound having a biuret group, a bi- or higher functional isocyanate compound having an allophanate group, and a bi- or higher functional isocyanate compound having two or more isocyanate groups with a compound having two or more active hydrogens.

<<Method for recovering amine compound>>

The recovery method according to the present embodiment is a method for recovering an amine compound represented by General Formula (IIa) (hereinafter, may be referred to as "amine compound (IIa)") from a liquid-phase

component containing a compound (hereinafter, may be referred to as "high-boiling-point compound") having a higher boiling point than a boiling point of an isocyanate compound which is a by-product in a method for producing an isocyanate compound represented by General Formula (VIIa) (hereinafter, may be referred to as "isocyanate compound (VIIa).

$$R^{71a}\left(-NCO\right)_{n71a} \qquad (VIIa)$$

(in General Formula (VIIa), $R^{71a}$ is an n71a-valent organic group, and n71a is an integer of 2 or more and 8 or less)

$$R^{21a}\left(-NH_2\right)_{n21a} \qquad (IIa)$$

(in General Formula (IIa), $R^{21a}$ is an n21a-valent organic group, and a relational expression of $R^{21a} = R^{71a}$ is satisfied, and n21 a is an integer of 2 or more and 8 or less, and a relational expression of n21a = n71a is satisfied)

The recovery method according to the present embodiment includes the following step (a):
a step (a) of reacting the liquid-phase component, an aromatic hydroxy compound, an active hydrogen-containing compound, and a catalyst in a reactor to obtain a reaction solution containing the amine compound represented by General Formula (IIa).

In the recovery method according to the present embodiment, the liquid-phase component containing the high-boiling-point compound, the aromatic hydroxy compound, the active hydrogen-containing compound, and the catalyst are mixed and reacted to regenerate the amine compound (IIa).

The above-described liquid-phase component, aromatic hydroxy compound, active hydrogen-containing compound, and catalyst may be mixed in advance and supplied to a reactor for carrying out a decomposition reaction, or may be separately supplied. In addition, within a range that does not impair the essence of the present embodiment, the reaction solution can be preheated before being supplied to the reactor in which the decomposition reaction is performed.

In addition, in the recovery method according to the present embodiment, from the viewpoint of transfer to the reactor in which a reaction is carried out between the liquid-phase component and the aromatic hydroxy compound and the active hydrogen-containing compound and the catalyst, it is preferable that, in the high-boiling-point compound in the liquid-phase component, a relational expression that [X/Y $\times$ {(n71a) - 1)}] is 2 or less is satisfied with a sum (X) of a biuret group, an allophanate group, an isocyanurate group, a urea group, a carbodiimide group, a uretonimine group, and an iminotrimer group, a sum (Y) of an isocyanate group, a carbamate group, a Fries rearrangement body, a cyclized product of the Fries rearrangement body, and an oxycarbonyl carbamate group, and n71a in General Formula (Vila).

Contents of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group can be calculated from the content of the $-CH_2-$ group adjacent to the N atom of the functional group quantitatively by simple NMR measurement, for example, by infrared spectroscopy (IR measurement) and nuclear magnetic resonance spectroscopy (NMR measurement), and the value of [X/Y $\times$ {(n71a) - 1)}] is calculated.

By having the above-described configuration in the recovery method according to the present embodiment, it is possible to efficiently regenerate a useful component including an amine compound from a liquid-phase component remaining after production of an isocyanate compound.

Next, each step of the recovery method according to the present embodiment will be described in detail.

<Step (a)>

The step (a) is a step of reacting the liquid-phase component, an aromatic hydroxy compound, an active hydrogen-containing compound, and a catalyst in a reactor to obtain a reaction solution containing the amine compound (IIa).

The step (a) preferably includes the following step (a1) and step (a2):

a step (a1) of mixing the liquid-phase component with the aromatic hydroxy compound, and
a step (a2) of reacting the mixture obtained in the step (a1), the active hydrogen-containing compound, and the catalyst in the reactor to obtain the reaction solution containing the amine compound represented by General Formula (IIa).

[Step (a1)]

The step (a1) is a step of mixing the liquid-phase component containing the high-boiling-point compound produced as a by-product in the method for producing the isocyanate compound (VIIa) with the aromatic hydroxy compound.

The aromatic hydroxy compound used in the step (a1) is preferably an aromatic hydroxy compound used in the method for producing the isocyanate compound (VIIa). The aromatic hydroxy compound may be used alone, or two or more kinds thereof may be used in combination.

The aromatic hydroxy compound is often highly soluble in the high-boiling-point compound containing the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, the oxycarbonyl carbamate group, and the like described, and thus has an effect of making the reaction system uniform and promoting the reaction to proceed rapidly.

In a case where the liquid-phase component containing the high-boiling-point compound extracted continuously from the thermal decomposition reactor is extracted continuously from the thermal decomposition reactor from the viewpoint of dissolving the high-boiling-point compound and maintaining the liquid phase state, the liquid-phase component may be used as it is in the reaction, and the aromatic hydroxy compound may be added by providing a pipe for supplying the aromatic hydroxy compound to a pipe for recovering the liquid-phase component containing the high-boiling-point compound extracted continuously from the separation device in the step (x2) described later.

The amount of the aromatic hydroxy compound used is preferably 10% by mass or more and 500% by mass or less, more preferably 20% by mass or more and 300% by mass or less, and still more preferably 50% by mass or more and 300% by mass or less with respect to the total mass of the liquid-phase component. In a case where the liquid-phase component is transferred from the thermal decomposition reactor and the separation device in the step (x2) described later to the reactor in which the reaction of the step (a1) in the recovery method according to the present embodiment is performed, it is preferable to transfer the liquid-phase component while maintaining the liquid-phase component in a liquid state. Therefore, the liquid-phase component is supplied to the reactor for the step (a1) at preferably 50°C or higher and 300°C or lower and more preferably 100°C or higher and 260°C or lower. By supplying and mixing the aromatic hydroxy compound with the liquid-phase component, the liquid-phase component is prevented from being modified with the isocyanate compound (VIIa), the biuret group, the urea group, the carbodiimide group, the urethane imine group, or the like contained in the liquid-phase component, and the liquid phase state is maintained.

In addition, in a case where water is used for the active hydrogen-containing compound in the step (a2) described later, in a case where the oil layer and the water layer are separated from the reaction solution obtained by the reaction between the high-boiling-point compound and the active hydrogen-containing compound by the liquid-liquid phase separation of the oil-in-water emulsion in the step (b) and the step (e) described later, the amine compound (IIa) and the isocyanate compound (VIIa) are separated as the oil layer and the hydroxy compound used for the production of the amine compound (IIa) and the isocyanate compound (VIIa) and the water layer, in general, the amine compound (IIa) and the water are in a state of being mixed with each other, and thus, the phase separation cannot be performed. However, by adding the aromatic hydroxy compound to the liquid-phase component, it is possible to achieve an effect of performing the phase separation between the amine compound (IIa) and the hydroxy compound and the active hydrogen compound.

In addition, in the step (c) and step (f) described later, when the amine compound (IIa) separated in the step (b) and the hydroxy compound separated in the step (e) are purified by distillation, the catalyst used in the step (a2) which will be described later or the method (B) for producing an isocyanate compound (VIIa) which will be described later may be precipitated in the distillation column and distillation may not be able to be significantly carried out. However, by adding the aromatic hydroxy compound to the liquid-phase component, it is possible to maintain a state in which the catalyst used in the step (a2) described later or the method (B) for producing an isocyanate compound (VIIa) which will be described later is dissolved and to continuously carry out distillation separation, and the like.

[Step (a2)]

The step (a2) is a step of reacting the composition mixed in the step (a1), the active hydrogen-containing compound, and the catalyst in a reactor.

(Active hydrogen-containing compound)

The active hydrogen-containing compound is preferably at least one selected from the group consisting of water, urea, alcohol (where an aromatic hydroxy compound is not included), an alkylthiol, and an amine compound (preferably, a primary amine compound); and more preferably at least one selected from the group consisting of water,

a primary amine compound, and a hydroxy compound. These compounds may be used alone or in combination of two or more kinds thereof. Among these, water, a combination of water and a primary amine compound, a combination of water and alcohol, or a combination of urea and alcohol is still more preferable. In addition, water or a combination of water and a primary amine compound is particularly preferable. Furthermore, water or a combination of water and an aliphatic primary amine compound is most preferable.

In addition, it is also preferable to supply the amine compound (IIa) to the step (a2) from the viewpoint that the decomposition reaction in the reactor is rapidly progressed and the yield is improved. That is, the amine compound (IIa) which is the target substance can also be used as the active hydrogen-containing compound.

As the alcohol used in the reaction, it is preferable to use the alcohol used for producing a carbamate compound in the production of the isocyanate compound (VIIa).

In a case where water is used as the active hydrogen-containing compound, the amount of water used (molar amount) is preferably 5 times or more and 200 times or less, more preferably 5 times or more and 150 times or less, and still more preferably 5 times or more and 100 times or less in terms of a stoichiometric ratio, with respect to the total molar amount of nitrogen atoms included in each functional group of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group, which are contained in the liquid-phase component. By setting the amount of water used (molar amount) to be equal to or less than the above-described lower limit value, it is possible to further suppress an undesirable reaction caused by an acidic atmosphere due to an increase in ion accumulation caused by a large amount of high-temperature and high-pressure water, for example, production of hexamethyleneimine which is a by-product obtained by a deammoniation reaction of hexamethylenediamine. Energy load in the subsequent dewater step can be further reduced, and it is possible to suppress consumption energy in the production and to reduce the size of the device. On the other hand, in a case where the amount of water used (molar amount) is equal to or more than the above-described lower limit value, the decomposition reaction of the high-boiling-point compound proceeds more favorably.

Since hydrolyzability varies depending on the formulation of the functional groups contained in the liquid-phase component containing the high-boiling-point compound, the required amount of water varies depending on the formulation of the decomposition target, but efficient decomposition can be carried out as long as the use amount is approximately within the above-described range.

In a case where the reaction can be efficiently carried out by applying a catalyst or the like, the amount of water can be further reduced. Therefore, in a case where the catalyst is used, the amount of water used (molar amount) is preferably 5 times or more and 50 times or less in terms of a stoichiometric ratio, with respect to the total molar amount of nitrogen atoms included in each functional group of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group, which are contained in the liquid-phase component. In a case where the decomposition efficiency is reduced, a crosslinking bond represented by a urea group, which can be produced as an intermediate of the hydrolysis reaction, is increased, and a high-molecular-weight substance, that is, a gel may be generated in the system. The gel becomes a scale, adheres to the inside of the device, and significantly reduces the reaction efficiency, and thus it is required to clean the device, which is a factor that reduces the economic efficiency. Within the above-described range, the decomposition proceeds more favorably, and the generation of scale in the device can be further suppressed, and thus the amine compound (IIa) can be efficiently recovered.

In a case where the urea, the alcohol, and the primary amine compound are used as the active hydrogen-containing compound, the amount of these active hydrogen-containing compounds used (molar amount) is usually 0.1 times or more and 100 times or less in terms of a stoichiometric ratio, with respect to the total molar amount of nitrogen atoms included in each functional group of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group, which are contained in the liquid-phase component.

(Catalyst)

The catalyst is not particularly limited, and examples thereof include hydroxides and oxides of alkali metals and alkaline earth metals, such as sodium hydroxide, potassium hydroxide, magnesium hydroxide, magnesium oxide, calcium oxide, and barium oxide; heterocyclic compounds or tertiary amines, such as pyridine, methylpyridine (including isomers), dipropylethylamine, N-methylmorpholine, N-ethylmorpholine, triethylamine, and triethylenediamine; and metal oxides of Groups 3 to 14, such as aluminum oxide, zinc oxide, titanium oxide, zirconium oxide, cerium oxide, iron oxide, tin oxide, vanadium oxide, molybdenum oxide, manganese oxide, cobalt oxide, and nickel oxide. Among these, the

catalyst is preferably one or more compounds selected from the group consisting of a hydroxide or an oxide of an alkali metal, a hydroxide or an oxide of an alkaline earth metal, a tertiary amine compound, a metal oxide of Group 12, a metal oxide of Group 13, and a metal oxide of Group 14. In addition, one or more compounds selected from the group consisting of a hydroxide or an oxide of an alkali metal, and a hydroxide or an oxide of an alkaline earth metal are more preferable; a hydroxide or an oxide of an alkali metal is still more preferable; and a hydroxide or an alkali metal, such as sodium hydroxide and potassium hydroxide, is particularly preferable. These catalysts can be used alone or in combination of two or more kinds thereof.

In addition, a shape of the catalyst may be uniform or non-uniform with respect to the reaction solution. Among these, it is preferable to use a homogeneous catalyst because it has an effect of rapidly promoting the decomposition reaction in the reactor, contributing to the suppression of by-product generation, and improving the yield.

The amount of the catalyst used (molar amount) is usually 0.001 times or more and 10 times or less, preferably 0.005 times or more and 5.00 times or less and more preferably 0.02 times or more and 3.00 times or less in terms of a stoichiometric ratio, with respect to the total molar amount of nitrogen atoms included in each functional group of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group, which are contained in the liquid-phase component.

In a case where the catalyst is not used, the high-boiling-point compound is not decomposed or decomposed slowly, and thus the yield of the amine compound (IIa) is decreased. On the other hand, the decomposability of the functional group contained in the liquid-phase component is different for each bonding mode, and the decomposition of each bonding mode can be promoted by appropriately selecting the combination of the catalyst and the active hydrogen compound, the type of the catalyst, the form of the reaction, and the like.

For example, the Fries rearrangement body, the cyclized product of the Fries rearrangement body, the isocyanurate group, and the iminotrimer group described above are generally less likely to undergo a decomposition reaction by water and less likely to generate the amine compound (IIa) than the biuret group, the allophanate group, the carbamate group, the oxycarbonyl carbamate group, the urea group, the carbodiimide group, and the uretonimine group. In particular, it is known that the isocyanurate g and the iminotrimer group have poor hydrolyzability, and that the progress of decomposition is slow by a known method. These functional groups are cross-linking bonds, and in a case where the high-molecular-weight substance remains in the reaction solution, when the amine compound (IIa) or the hydroxy compound is separated from the reaction solution, the device is clogged by precipitation. Alternatively, the amount of the amine compound (IIa) which can be recovered is decreased.

On the other hand, in a case where the liquid-phase component, the aromatic hydroxy compound, the active hydrogen-containing compound, and the catalyst, which are by-products in the method for producing the isocyanate compound (VIIa), are used, the decomposition of the functional group is promoted, and the high-molecular-weight substance is not left in the reaction solution. Therefore, when the amine compound (IIa) or the hydroxy compound is separated from the reaction solution, the clogging in the device due to the precipitation or the like can be avoided, and thus the amine compound (IIa) can be efficiently recovered.

The reaction temperature of the regeneration reaction can be set according to the compounds to be used, but is preferably 100°C or higher and 300°C or lower, more preferably 160°C or higher and 270°C or lower, and still more preferably 190°C or higher and 240°C or lower, in consideration of the energy load in the present step. Even in a case where the temperature is raised above the above-described range, the decomposition efficiency is almost saturated. Rather, the environment of high-temperature and high-pressure water is a reaction field in which the ion product increases and the acidity is improved, and thus an undesirable reaction may occur. Examples of the undesirable reaction include the production of a by-product, hexamethylene imine, which is internally cyclized by the deammoniation reaction of hexamethylenediamine. In a case where the decomposition efficiency is reduced, a crosslinking bond represented by a urea group, which can be produced as an intermediate of the hydrolysis reaction, is increased, and a high-molecular-weight substance, that is, a gel may be generated in the system. The gel becomes a scale, adheres to the inside of the device, and significantly reduces the reaction efficiency, and thus it is required to clean the device, which is a factor that reduces the economic efficiency. Within the above-described temperature range, the decomposition of the crosslinking molecules represented by the urea group proceeds more favorably, and the generation of scale in the device can be further suppressed. Therefore, the amine compound (IIa) can be more efficiently recovered while maintaining continuous operability.

The reaction pressure is usually in a range of 0.01 kPa or more and 10 MPa (absolute pressure) or less, and it can be carried out under reduced pressure, under normal pressure, or under pressure.

The reaction time (in a case of the continuous reaction, the residence time) is usually 0.01 hours or more and 100 hours or less, preferably 0.1 hours or more and 10 hours or less, more preferably 0.5 hours or more and 3 hours or less, and still more preferably 1 hours or more and 3 hours or less. The reaction can also be terminated when the desired amount of the amine compound (IIa), which is the target compound, is produced by appropriately sampling the

reaction solution and measuring the amount of the primary amine compound produced.

In the reaction, there is a case in which a low-boiling-point component having a boiling point of 30°C or lower is generated, and the pressure of the reaction system is increased by the low-boiling-point component, or the reaction is delayed by the presence of the low-boiling-point component. Therefore, it is preferable to perform the reaction while extracting at least a part of the low-boiling-point component as a gas-phase component outside the reaction system. In this case, it is preferable to prevent the active hydrogen-containing compound from being extracted to the outside of the reaction system together with the low-boiling-point component, for example, by providing a condenser in the middle of a line for extracting the low-boiling-point component from the reactor in which the reaction is performed. In particular, when water is used as the active hydrogen-containing compound, in a case where carbon dioxide is generated by the reaction with water, for example, in a case where the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, or the oxycarbonyl carbamate group is used, it is preferable to extract the generated carbon dioxide outside the reaction system.

The generated carbon dioxide forms a carbonate with the amine compound (IIa), which is generated by the reaction, and thus the reaction may be delayed. The ease of formation of the carbonate depends on nucleophilicity of the amine compound (IIa), and the order of carbonate formation is generally aliphatic amines ≥ substituted cyclic aliphatic polyamines > aromatic amines. Therefore, it is preferable to adjust the amount of carbon dioxide generated by the structure of the amine compound (IIa) extracted from the reaction system as necessary.

The reactor used for the reaction is not particularly limited, and a known reactor can be used. For example, a reactor known in the related art can be appropriately combined and used according to the reaction method or conditions, such as a stirring tank, a pressurized stirring tank, a depressurized stirring tank, a tower-type reactor, a distillation column, a filled column, a thin film evaporator, a paddle dryer equipped with a forced transportation device, an extruder equipped with a degassing function, a vertical thin film evaporator equipped with a forced transportation device, and a tubular reactor.

In addition, the reaction may be a batch type or a continuous flow type, and a reaction device may be selected according to each reaction type.

A material of the reactor is not particularly limited, and a known material can be used. For example, a glass, stainless steel, carbon steel, Hastelloy material, a material on which a glass lining is applied, a material on which a Teflon (registered trademark) coating is applied, or the like can be used. SUS304, SUS316, SUS316L, and the like are inexpensive and can be preferably used. As necessary, known process equipment such as measuring instruments, for example, a flowmeter and a thermometer, a mechanism for holding pressure, a reboiler, a pump, and a condenser may be added. The heating may be performed by a known method such as steam or a heater, and the cooling may be performed by a known method such as natural cooling, cooling water, or brine.

The step (a2) preferably includes the following step (a2-1) and step (a2-2):

a step (a2-1) of mixing the mixture obtained in the step (a1), an amine compound as the active hydrogen-containing compound, and the catalyst, and

a step (a2-2) of reacting the mixture obtained in the step (a2-1) with water as the active hydrogen-containing compound in the reactor to obtain the reaction solution containing the amine compound (II).

(Step (a2-1))

The step (a2-1) is a step of mixing the mixture obtained in the step (a1), an amine compound as the active hydrogen-containing compound, and the catalyst.

The temperature in the reactor in the step (a2-1) is preferably 100°C or higher and 350°C or lower, more preferably 150°C or higher and 300°C or lower, and still more preferably 180°C or higher and 280°C or lower.

The mixing time (in a case of continuous mixing, the retention time) in the step (a2-1) is usually 0.01 hours or more and 10 hours or less. The mixing time is preferably 0.1 hours or more and 5 hours or less, more preferably 0.2 hours or more and 2.5 hours or less, and still more preferably 0.3 hours or more and 2 hours or less. The mixing can also be terminated when the desired amount of the urea group, which is the target compound, is produced by appropriately sampling the reaction solution and measuring the amount of the primary amine compound produced.

The amine compound used in the step (a2-1) is a primary amine compound or a secondary amine compound; and a primary amine compound is preferable and the amine compound (IIa) is more preferable. That is, the amine compound (IIa) which is the target substance can be used as the active hydrogen-containing compound.

The amount of amine used in the step (a2-1) (molar amount) is preferably in a range of 0.5 times or more and 200 times or less, more preferably in a range of 1 time or more and 50 times or less, and still more preferably in a range of 2 times or more and 20 times or less in terms of a stoichiometric ratio, with respect to the total molar amount of nitrogen atoms included in each functional group of the isocyanate group, the biuret group, the allophanate group, the isocyanurate group, the carbamate group, the urea group, the carbodiimide group, the uretonimine group, the iminotrimer group, the

Fries rearrangement body, the cyclized product of the Fries rearrangement body, and the oxycarbonyl carbamate group, which are contained in the liquid-phase component. The amine compound reacts with the biuret group, the allophanate group, the carbamate group, and the uretonimine group contained in the liquid-phase component to form a corresponding urea group, and with the carbodiimide group to form a corresponding urea group and an amidine group, and these reactions proceed quantitatively at the time of mixing. Since the urea group and the amidine group are crosslinkable bonds, in a case where a high-molecular-weight substance is formed, the high-molecular-weight substance is precipitated as a gel. On the other hand, in a case where the amount of the amine compound is within the above-described range, the decomposition of the crosslinking molecules proceeds more smoothly, and the generation of scale in the device can be further suppressed. Therefore, the amine compound (IIa) can be more efficiently recovered while maintaining continuous operability.

Examples of the catalyst used in the step (a2-1) include the same catalysts as those described in the step (a2). Among these, hydroxides, oxides, and phenoxydes of alkali metals, or hydroxides, oxides, and phenoxydes of alkaline earth metals are preferable. From the viewpoint of solubility in the reaction system, sodium phenoxide or potassium phenoxide is more preferable.

In a case where the mixture obtained in the step (a1) and the amine as the active hydrogen-containing compound are mixed, the urea group corresponding to the isocyanurate group contained in the liquid-phase component is formed. The main reaction proceeds quantitatively. With respect to the iminotrimer group, corresponding urea groups and amidine groups are formed. In a case of mixing the mixture obtained in the step (a1), the amine as the active hydrogen-containing compound, and the catalyst, by using a catalyst that is uniform in the reaction system, the isocyanurate group and the iminotrimer group can be significantly decomposed. As described above, the isocyanurate group, which is a crosslinking bond, and the iminotrimer group form the high-molecular-weight substance and cause scale in the pipe. Therefore, the catalyst which accelerates the decomposition is used, and the effect of suppressing the scale in the pipe is exhibited.

The molar amount of the catalyst used in the step (a2-1) is usually 0.001 times or more and 10 times or less, preferably 0.005 times or more and 5.00 times or less and more preferably 0.02 times or more and 3.00 times or less in terms of a stoichiometric ratio, with respect to the total molar amount of nitrogen atoms included in each functional group of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group, which are contained in the liquid-phase component.

The liquid-phase component, the amine compound, and the catalyst may be mixed in advance and supplied to the reactor in which the step (a2-2) is performed, or may be separately supplied and mixed in the reactor. In addition, within a range that does not impair the essence of the present embodiment, the reaction solution can be preheated before being supplied to the reactor in which the step (a2-2) is performed.

(Step (a2-2))

The step (a2-2) is a step of reacting the mixture obtained in the step (a2-1) with water as the active hydrogen-containing compound in the reactor to obtain the reaction solution containing the amine compound represented by General Formula (II).

The amount of water used in the step (a2-2) (molar amount) is the same as the amount of water used in the step (a2) (molar amount).

The temperature in the reactor in the step (a2-2) is preferably 100°C or higher and 350°C or lower, more preferably 150°C or higher and 300°C or lower, and still more preferably 180°C or higher and 240°C or lower.

The pressure in the reactor is preferably 0.01 kPa or more and 15 MPa (absolute pressure) or less, and it can be carried out under reduced pressure, under normal pressure, or under pressure.

In consideration of the above-described preferred temperature range, the compound generated in the decomposition reaction, and the like, the pressurization is more preferably 0.101 MPa or more and 15 MPa or less (absolute pressure), still more preferably 0.5 MPa or more and 7 MPa or less (absolute pressure), and particularly preferably 0.5 MPa or more and 3 MPa or less (absolute pressure).

The reaction time (in a case of the continuous reaction, the residence time) is 0.01 hours or more and 100 hours or less, preferably 0.1 hours or more and 10 hours or less, more preferably 0.2 hours or more and 3 hours or less, and still more preferably 0.3 hours or more and 2 hours or less. In addition, a time when the desired amount of the amine compound (IIa), which is the target compound, is produced by appropriately sampling the reaction solution and measuring the amount of the primary amine compound produced can be set as the reaction time.

The amine compound (IIa) can be recovered from the reaction solution obtained by the reaction through distillation separation, liquid-liquid phase separation, solid-liquid separation, membrane separation, or the like. The collected amine compound (IIa) is preferably re-used as the amine compound (IIa) in the step (a2-1). In addition, in a case where an aromatic hydroxy compound is generated by the reaction between the high-boiling-point compound and the active

hydrogen-containing compound, the aromatic hydroxy compound can be recovered and re-used as the aromatic hydroxy compound in the step (a2-1) or the step (a1).

It is preferable that the recovery method according to the present embodiment further includes the following step (b), step (c), step (e), and step (f). These steps (b), (c), (e), and (f) can be performed continuously, or can be performed simultaneously. Alternatively, the operations of the step (e) and the step (f) can be performed before the step (b) and the step (c). In this case, in the step (e), a reaction solution containing the amine compound (IIa) obtained in the step (a2) is used as a raw material, and, on the other hand, in the step (b), a residual liquid after the hydroxy compound is separated in the step (e) is used as a raw material. Alternatively, the steps may be performed by replacing some of the steps.

A step (b) of separating the above-described amine compound (IIa) from the reaction solution containing the above-described amine compound (IIa);

a step (c) of purifying the amine compound represented by General Formula (IIa);
a step (e) of separating the hydroxy compound from the reaction solution containing the amine compound (IIa); and
a step (f) of purifying the hydroxy compound.

<Step (b)>

The step (b) is a step of separating the amine compound (IIa) from the liquid obtained in the step (a2) or the step (a2-2) described above, or the step (e) or the step (f) described later. As a method for separating the amine compound (IIa) from the reaction solution obtained in the step (a2) or the step (a2-2), a known method can be used, and examples thereof include distillation separation, liquid-liquid phase separation, solid-liquid separation, and membrane separation.

In the step (b), the amine compound (IIa) can also be continuously separated from the liquid-phase component (reaction solution) continuously discharged in the step (a2) or the step (a2-2) described above.

<Step (c)>

The step (c) is a step of purifying the amine compound (IIa) separated in the step (b). As a method for purifying the amine compound (IIa), a known method can be used, and examples thereof include distillation separation, liquid-liquid phase separation, solid-liquid separation, and membrane separation.

In the step (c), the continuously separated amine compound (IIa) in the step (b) can also be continuously purified.

<Step (e)>

The step (e) is a step of separating the hydroxy compound used in the production of the isocyanate compound (VIIa) from the liquid obtained in the step (a2), the step (a2-2), the step (b), or the step (c).

For example, in a case where the step (a2) or the step (a2-2) is carried out using a compound including a carbamate group (-NH-C(=O)-OR), such as a carbamate compound described later, a hydroxy compound represented by R-OH is generated by the reaction with the active hydrogen-containing compound. That is, the hydroxy compound recovered here is mainly a hydroxy compound used as a raw material for producing the isocyanate compound. Therefore, in a case where the hydroxy compound used as a raw material in the production of the isocyanate compound and the aromatic hydroxy compound used in the step (a) are the same, the hydroxy compound used as a raw material in the production of the isocyanate compound and the aromatic hydroxy compound used in the step (a) are separated together. On the other hand, in a case where the hydroxy compound used as a raw material in the production of the isocyanate compound and the aromatic hydroxy compound used in the step (a) are different from each other, the hydroxy compound used as a raw material in the production of the isocyanate compound is mainly separated, depending on the separation method. In this case, in the step (e), it is preferable to separate the hydroxy compound from the liquid obtained in the step (a2), the step (a2-2), the step (b), or the step (c) and recover the hydroxy compound. As a method for the separation in the step (e), a known method can be used, and examples thereof include distillation separation, liquid-liquid phase separation, solid-liquid separation, and membrane separation.

<Step (f)>

The step (f) is a step of purifying the hydroxy compound separated in the step (e).

After the step (e), it is preferable to further perform the step (f) to purify the hydroxy compound. As a method for purifying the hydroxy compound, a known method can be used, and examples thereof include distillation separation, liquid-liquid phase separation, solid-liquid separation, and membrane separation.

In the step (f), the hydroxy compound continuously separated in the step (e) can also be continuously purified.

It is preferable that the recovery method according the present embodiment further includes the following step (d) and step (g):

a step (d) of re-using the amine compound (II) purified in the step (c) as a raw material in the method for producing the isocyanate compound (Vila); and
a step (g) of re-using the hydroxy compound purified in the step (f) as a raw material in the method for producing the isocyanate compound (VIIa).

<Step (d)>

The step (d) is a step of re-using the amine compound (IIa) purified in the step (c) as a raw material of the isocyanate compound (VIIa).
In this case, since the method for producing the isocyanate compound (VIIa) and the quality of the isocyanate compound (VIIa) produced by the method are often affected, in the step (c), it is preferable to distill and recover the amine compound (IIa) such that the content of the metal component is 1,000 ppm by mass or less and the content of the halogen atom is 1,000 ppm by mass or less with respect to the total mass of the amine compound (IIa).

<Step (g)>

The step (g) is a step of re-using the hydroxy compound purified in the step (f) as a raw material in the method for producing the isocyanate compound (VIIa).
In this case, since the method for producing the isocyanate compound (VIIa) and the quality of the isocyanate compound (VIIa) produced by the method are often affected, in the step (g), it is preferable to distill and recover the hydroxy compound such that the content of the metal component is 1,000 ppm by mass or less and the content of the halogen atom is 1,000 ppm by mass or less with respect to the total mass of the hydroxy compound.
It is preferable that the recovery method according to the present embodiment further includes the following step (h):
a step (h) of re-using, in the step (a), one or more residual liquids selected from the group consisting of a residual liquid after separating the amine compound (IIa) in the step (b), a residual liquid after purifying the amine compound (IIa) in the step (c), a residual liquid after separating the hydroxy compound in the step (e), and a residual liquid after purifying the hydroxy compound in the step (f).

<Step (h)>

The step (h) is a step of re-using, in the step (a), one or more residual liquids selected from the group consisting of a residual liquid after separating the amine compound (IIa) in the step (b), a residual liquid after purifying the amine compound represented by General Formula (IIa) in the step (c), a residual liquid after separating the hydroxy compound in the step (e), and a residual liquid after purifying the hydroxy compound in the step (f).
Since these residual liquids contain the active hydrogen-containing compound, the catalyst, and the like, in a case where one or more of the residual liquids are re-used, the amine compound (IIa) can be recovered more efficiently in the recovery method according to the present embodiment.
Next, various raw materials and products used in the recovery method according to the present embodiment will be described in detail below.

<High-boiling-point compound>

The high-boiling-point compound contained in the liquid-phase component is presumed to be produced as a by-product in a case of producing the isocyanate compound (VIIa) using the amine compound (IIa) as a raw material, and to be produced by a reaction of one or more compounds selected from the group consisting of the amine compound (IIa), a carbamate compound described later, which is a reaction intermediate, and the isocyanate compound (VIIa).
The high-boiling-point compound may include a group represented by Formula (IX-1) (isocyanate group), a group represented by Formula (IX-2) (isocyanurate group), a group represented by Formula (IX-3) (carbodiimide group), a group represented by Formula (IX-4) (uretonimine group), a group represented by Formula (IX-5) (oxycarbonylcarbamate group), a group represented by Formula (IX-6) (iminotrimer group), a group represented by Formula (IX-7) (biuret group), a group represented by Formula (IX-8) (allophanate group), a group represented by Formula (IX-9) (carbamate group), a group represented by Formula (IX-10) (urea group), a group represented by Formula (IX-11) (functional group generated by a Fries rearrangement reaction; hereinafter, may be referred to as "Fries rearrangement body" or "Fries rearrangement group"), or a group represented by Formula (IX-12) (cyclized product of the Fries rearrangement body).

The high-boiling-point compound that is by-produced in the step of producing the isocyanate compound (VIIa) through the carbamate compound described later may include the group represented by Formula (IX-11) (Fries rearrangement body), the group represented by Formula (IX-12) (a cyclized product of a free displaceable group), the group represented by Formula (IX-5) (oxycarbonylcarbamate group), or the like. In particular, the high-boiling-point compound that is a by-product in the step of producing the isocyanate compound (VIIa) by thermal decomposition using the carbamate compound and the carbonic acid ester described later may include the group represented by Formula (IX-5) (oxycarbonylcarbamate group).

In addition, the high-boiling-point compound that is a by-product in the step of producing the isocyanate compound (VIIa) through the carbamate compound synthesized from urea or N-unsubstituted carbamic acid, which will be described later, may include the group represented by Formula (IX-2) (isocyanurate group), the group represented by Formula (IX-3) (carbodiimide group), or the like. Among these, in the group represented by Formula (IX-2) (isocyanurate group), that is produced as a by-product in the step of producing the isocyanate compound (VIIa) through the carbamate compound synthesized from urea or N-unsubstituted carbamic acid, each of three nitrogen atoms in Formula (IX-2) may be bonded to $R^{71a}$ in General Formula (VIIa) described later or a hydrogen atom, that is, each of three bonds represented by wavy lines in Formula (IX-2) may be bonded to $R^{71a}$ in General Formula (VIIa) described later or a hydrogen atom.

Among these, the high-boiling-point compound is preferably a compound having one or more functional groups selected from the group consisting of a group represented by Formula (IX-1) (isocyanate group), a group represented by Formula (IX-2) (isocyanurate group), a group represented by Formula (IX-3) (carbodiimide group), a group represented by Formula (IX-4) (uretonimine group), a group represented by Formula (IX-5) (oxycarbonylcarbamate group), a group represented by Formula (IX-6) (iminotrimer group), a group represented by Formula (IX-7) (biuret group), a group represented by Formula (IX-8) (allophanate group), a group represented by Formula (IX-9) (carbamate group), a group represented by Formula (IX-10) (urea group), a group represented by Formula (IX-11) (Fries rearrangement body), and a group represented by Formula (IX-12) (cyclized product of the Fries rearrangement body); and more preferably a compound having two or more functional group thereof.

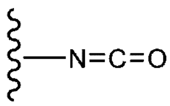

(IX-1)

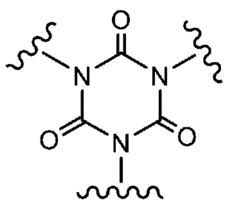

(IX-2)

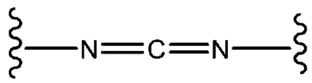

(IX-3)

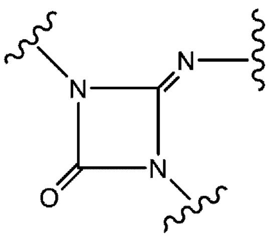

(IX-4)

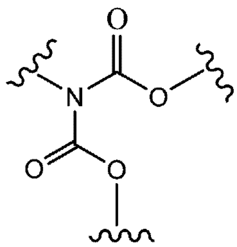

(IX-5)

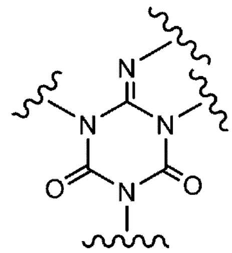

(IX-6)

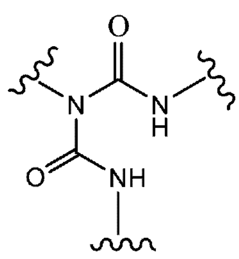

(IX-7)

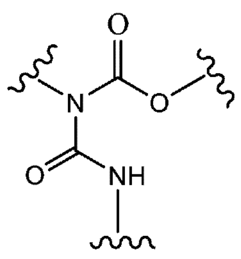

(IX-8)

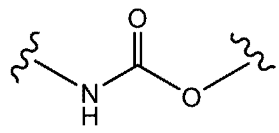

(IX-9)

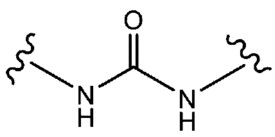

(IX-10)

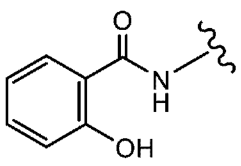

(IX-11)

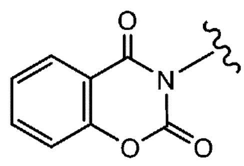

(IX-12)

(in Formulae (IX-1) to (IX-12), a wavy line represents a bonding site)

In the liquid-phase component used in the recovery method according to the present embodiment, the total content of nitrogen atoms included in each functional group of the group represented by Formula (IX-1) (isocyanate group), the group represented by Formula (IX-2) (isocyanurate group), the group represented by Formula (IX-3) (carbodiimide group), the group represented by Formula (IX-4) (uretonimine group), the group represented by Formula (IX-5) (oxycarbonylcarbamate group), the group represented by Formula (IX-6) (iminotrimer group), the group represented by Formula (IX-7) (biuret group), the group represented by Formula (IX-8) (allophanate group), the group represented by Formula (IX-9) (carbamate group), the group represented by Formula (IX-10) (urea group), the group represented by Formula (IX-11) (Fries rearrangement body), and the group represented by Formula (IX-12) (cyclized product of the Fries rearrangement body) is preferably 0.10 mol or more and 15.00 mol or less, more preferably 0.50 mol or more and 12.00 mol or less, and still more preferably 1.00 mol or more and 12.00 mol or less with respect to 1 kg of the liquid-phase component.

In the liquid-phase component, a crosslinking bond represented by a urea group, which can be produced as an intermediate of the hydrolysis reaction, is increased, and a high-molecular-weight substance, that is, a gel may be generated in the system. The gel becomes a scale, adheres to the inside of the device, and significantly reduces the reaction efficiency, and thus it is required to clean the device, which is a factor that reduces the economic efficiency. On the other hand, in a case where the content of the above-described functional group is within the above-described range, the decomposition proceeds more favorably, and the generation of scale in the device can be further suppressed, and thus the amine compound (IIa) can be efficiently recovered.

The content of these functional groups can be calculated from the content of the $-CH_2-$ group adjacent to the N atom of the functional group quantitatively by simple NMR measurement, for example, by infrared spectroscopy (IR measurement) and nuclear magnetic resonance spectroscopy (NMR measurement), estimating the total mass of the functional group from this value, and dividing the total mass by the mass of the sample (liquid-phase component) used for the measurement.

The high-boiling-point compound is a compound having a boiling point higher than that of the isocyanate compound (VIIa). Specifically, the compound is a compound that does not become a gas-phase component under the same conditions as a compound extracted as a gas-phase component from the thermal decomposition reactor in the method for producing the isocyanate compound (VIIa) through a carbamate compound, or a compound that does not become a gas-phase component in the distillation purification step of the isocyanate compound (VIIa). The boiling point of the high-boiling-point compound is not particularly limited, but is, for example, 300°C or higher at the operating pressure of a thermal decomposition reactor or a distillation device.

<Isocyanate compound (VIIa)>

The isocyanate compound (VIIa) is a compound represented by General Formula (VIIa).

$$R^{71a}\left(-NCO\right)_{n71a} \qquad (VIIa)$$

(in General Formula (VIIa), $R^{71a}$ is an n71a-valent organic group, and n71a is an integer of 2 or more and 8 or less)

[$R^{71a}$]

$R^{71a}$ is an n71a-valent organic group. That is, $R^{71a}$ is a di- or higher and octa- or lower valent organic group. Among these, $R^{71a}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

As the aliphatic hydrocarbon group as $R^{71a}$, an alkylene group or an alkanetriyl group, a cycloalkyl group, a cycloalkylene group or a cycloalkanetriyl group, or a group constituting the alkyl group, the alkylene group or the alkanetriyl group, and the cycloalkyl group, the cycloalkylene group or the cycloalkanetriyl group is preferable; and a linear or branched alkylene group or alkanetriyl group, a cycloalkylene group or a cycloalkanetriyl group, or a group constituting the alkylene group or the alkanetriyl group, and the cycloalkyl group, the cycloalkylene group or the cycloalkanetriyl group is more preferable.

Examples of the linear or branched alkylene group include a methylene group, an ethylene group, a propylene group, a trimethylene group, a pentylene group, an n-hexylene group, and a decamethylene group.

Examples of the cycloalkylene group include a cyclobutylene group and a cyclohexylene group.

Examples of the linear or branched alkanetriyl group include a hexanetriyl group, a nonanetriyl group, and a decanetriyl group.

Examples of the cycloalkanetriyl group include a cyclopropanetriyl group, a cyclobutanetriyl group, a cyclopentatriyl group, and a cyclohexatriyl group.

The aromatic hydrocarbon group as $R^{71a}$ is preferably a group having a substituted or unsubstituted aromatic ring having 6 or more and 13 or less carbon atoms. Examples of the substituent include an alkyl group, an aryl group, and an aralkyl group. The aromatic ring may be an aromatic hydrocarbon ring or a heteroaromatic ring, and specific examples thereof include a benzene ring, a naphthalene ring, and a pyridine ring.

Among these, $R^{71a}$ is preferably a group represented by any one of Formulae (Ia-1) to (Ia-24), and more preferably a group represented by Formula (Ia-1), (Ia-2), (Ia-3), (Ia-14), (Ia-18), or (Ia-19). In each formula, a wavy line represents a bonding site.

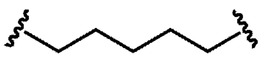
(Ia-1)

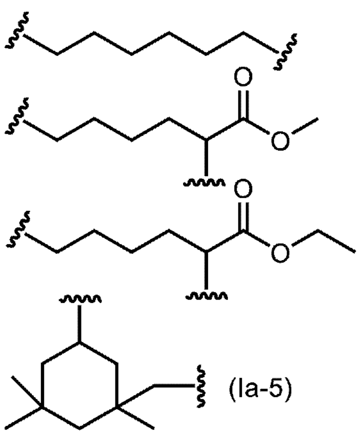
(Ia-2)

(Ia-3)

(Ia-4)

(Ia-5)

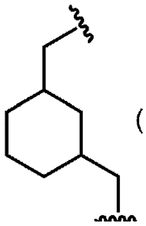
(Ia-6)

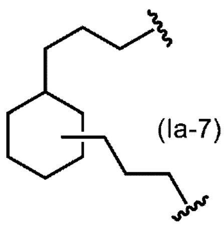
(Ia-7)

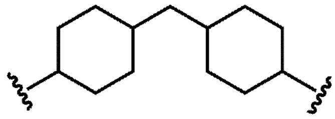 (Ia-8)

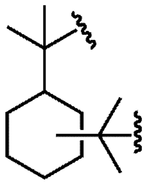 (Ia-9)

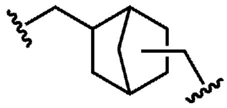 (Ia-10)

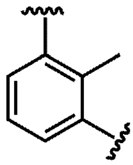 (Ia-11)

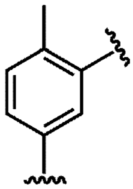 (Ia-12)

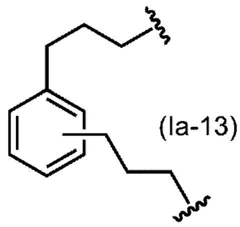 (Ia-13)

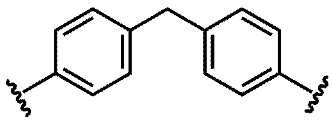 (Ia-14)

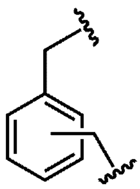 (Ia-15)

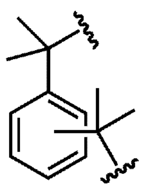 (Ia-16)

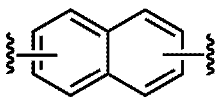 (Ia-17)

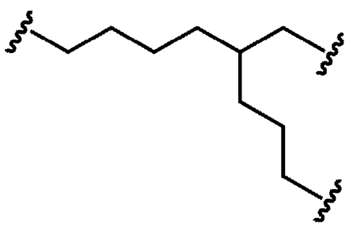 (Ia-18)

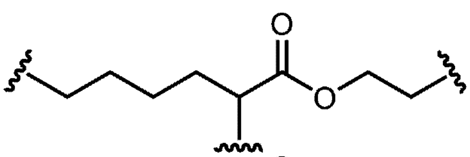 (Ia-19)

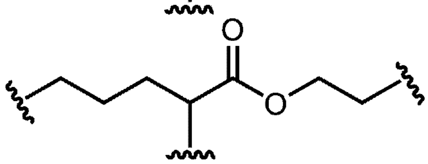 (Ia-20)

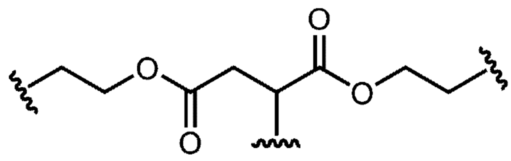

(Ia-21)

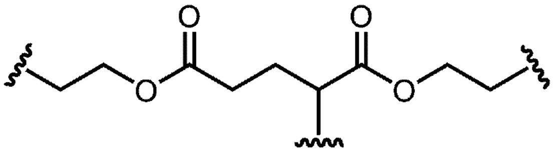

(Ia-22)

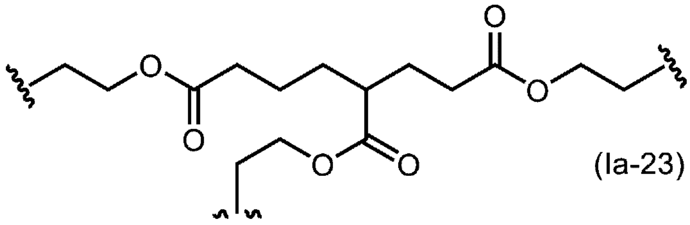

(Ia-23)

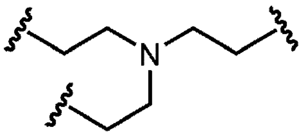

(Ia-24)

[n71a]

n71a represents the number of isocyanate groups, and is an integer of 2 or more and 8 or less, preferably an integer of 2 or more and 4 or less and more preferably an integer of 2 or more and 3 or less.

In a case where $R^{71a}$ is an aliphatic hydrocarbon group, specific examples of the isocyanate compound (VIIa) include aliphatic diisocyanates, aliphatic triisocyanates, and substituted cyclic aliphatic polyisocyanates.

Examples of the aliphatic diisocyanates include diisocyanatoethane, diisocyanatopropane (each isomer), diisocyanatobutane (each isomer), diisocyanatopentane (each isomer), diisocyanatohexane (each isomer), and diisocyanatodecane (each isomer).

Examples of the aliphatic triisocyanates include triisocyanatohexane (each isomer), 4-isocyanatomethyl-1,8-octamethylenediisocyanate, triisocyanatononane (each isomer), and triisocyanatodecane (each isomer).

Examples of the substituted cyclic aliphatic polyisocyanates include diisocyanatocyclobutane (each isomer), diisocyanatocyclohexane (each isomer), 3-isocyanatomethyl-3,5,5-trimethylcyclohexylisocyanate (also referred to as isophorone diisocyanate) (each isomer), 1,3-bis(isocyanatomethyl)cyclohexane (at least one isomer of a cis form and a trans form), and methylenebis(cyclohexylisocyanate) (also referred to as dicyclohexylmethane diisocyanate) (each isomer).

In a case where $R^{71a}$ is an aromatic group, specific examples of the isocyanate compound (VIIa) include aromatic diisocyanates and aromatic triisocyanates.

Examples of the aromatic diisocyanates include diisocyanatobenzene (each isomer), diisocyanatotoluene (each isomer), bis(isocyanatophenyl)methane (each isomer), diisocyanatomesitylene (each isomer), diisocyanatobiphenyl (each isomer), diisocyanatodibenzyl (each isomer), bis(isocyanatophenyl)propane (each isomer), bis(isocyanatophenyl)ether (each isomer), bis(isocyanatophenoxyethane) (each isomer), diisocyanatoxylene (each isomer), diisocyanatoanisole (each isomer), diisocyanatophenethol (each isomer), diisocyanatonaphthalene (each isomer), diisocyanatomethylbenzene (each isomer), diisocyanatomethylpyridine (each isomer), diisocyanatomethylnaphthalene (each isomer), diisocyanatodiphenylmethane (each isomer), and tetramethylxylylene diisocyanate (each isomer).

Examples of the aromatic triisocyanates include triisocyanatobenzene (each isomer), triisocyanato-methylbenzene (each isomer), tris(isocyanatopropan-yl)benzene (each isomer), tris(isocyanatopropan-yl)-methylbenzene (each isomer), tris(isocyanatomethyl)-methylbenzene (each isomer), ((isocyanato-phenylene)bis(methylene))bis(isocyanatebenzene) (each isomer), and triphenylmethane triisocyanate.

Specific examples of the isocyanate compound (VIIa) in a case where the aliphatic hydrocarbon group or the aromatic group as $R^{71a}$ has 1 or more and 4 or less ester groups include 2-isocyanatoethyl acrylate, 2-isocyanatoethyl

2-methylacrylate, 2-isocyanatopropyl acrylate, 2-isocyanatopropyl 2-methylacrylate, 3-isocyanatopropyl acrylate, 3-isocyanatopropyl 2-methylacrylate, 4-isocyanatobutyl acrylate, 4-isocyanatobutyl 2-methylacrylate, 5-isocyanatopentyl acrylate, 5-isocyanatopentyl 2-methylacrylate,acrylic acid-6-isocyanato-hexyl ester, 2-methyl-acrylic acid-6-isocyanato-hexyl ester, acrylic acid-8-isocyanato-octyl ester, 2-methyl-acrylic acid-8-isocyanato-octyl ester, acrylic acid-10-isocyanato-decyl ester, 2-methyl-acrylic acid-10-isocyanato-decyl ester, acrylic acid-11-isocyanato-undecyl ester, 2-methyl-acrylic acid-11-isocyanato-undecyl ester, acrylic acid-12-isocyanato-dodecyl ester, 2-methyl-acrylic acid-12-isocyanato-dodecyl ester, lysine methyl ester diisocyanate, lysine ethyl ester diisocyanate, 2-isocyanatoethyl-2,5-diisocyanatopentanoate, 2-isocyanatoethyl-2,6-diisocyanatohexanoate, bis(2-isocyanatoethyl)-2-isocyanatobutanedioate, bis(2-isocyanatoethyl)-2-isocyanatopentanedioate, and tris(2-isocyanatoethyl)hexane-1,3,6-tricarboxylate.

Examples of the preferred isocyanate compound (VIIa) include pentamethylene diisocyanate, hexamethylene diisocyanate, 4-isocyanatomethyl-1,8-octamethylene diisocyanate, lysine ethyl ester diisocyanate, methylene bis(cyclohexyl isocyanate) (HMDI), 3-isocyanatomethyl-3,5,5-trimethylcyclohexyl isocyanate (isophorone diisocyanate), diisocyanatotoluene (each isomer), diisocyanatodiphenylmethane (MDI), triisocyanatobenzene (each isomer), and triphenylmethane triisocyanate.

[Method for producing isocyanate compound (Vila)]

The isocyanate compound (VIIa) can be produced using the amine compound (IIa) as a raw material, and a known method can be used as the production method. Examples thereof include the following method (X) and the following method (Y).

Method (X): method of producing a carbamate compound using the amine compound (IIa), a carbonic acid derivative, and a hydroxy compound (step (x1)), and of producing the isocyanate compound (VIIa) by subjecting the carbamate compound to a thermal decomposition reaction (step (x2))

Method (Y): method of reacting the amine compound (IIa) with phosgene to produce the isocyanate compound (VIIa) while dehydrochlorinating

(Method (X))

In the method (X), other steps other than the step (x1) and the step (x2) can be added as necessary. Examples of the other steps include a step of performing an ester exchange reaction of a carbamate using the carbamate compound obtained in the step (x1), the hydroxy compound used in the step (x1), and a hydroxy compound of a different kind to produce a carbamate of a different kind (step (x1-a)); a step of separating and preliminarily concentrating a part or all of the hydroxy compound from the reaction solution of the step (x1) (step (x1-b)); a step of purifying the isocyanate compound (VIIa) (step (x3)); and a step of recovering ammonia, a hydroxy compound, and the like produced as by-products from the carbonic acid derivative in the step (x1).

In addition, in the above-described step (x3),

a step of distilling and separating a gas-phase component containing a compound having a lower boiling point than that of the isocyanate compound (VIIa) (hereinafter, may be referred to as "low-boiling-point compound") to separate a liquid-phase component containing the isocyanate compound (VIIa) (hereinafter, may be referred to as "low-boiling separation") (step (x3-1)); and

a step of distilling and separating a gas-phase component containing the isocyanate compound (VIIa) to separate the high-boiling-point compound from the isocyanate compound (VIIa) (hereinafter, may be referred to as "high-boiling separation") (step (x3-2))

can be carried out separately, or the order of carrying out the step (x3-1) and the step (x3-2) can be interchanged.

(1) Step (x1)

The step (x1) is a step of reacting the amine compound (IIa) with a carbonic acid derivative and a hydroxy compound to produce a carbamate compound, and separately collecting one or more compounds selected from the group consisting of urea and isocyanic acid, a hydroxy compound, and a gas-phase component containing ammonia from the produced carbamate compound.

The step (x1) can be performed in two methods, which are the following method (x1-1) and the following method (x1-2). In the production method according to the present embodiment, the method (x1-1) and the method (x1-2) may be combined.

Method (x1-1): method of producing the carbamate compound by "simultaneously" reacting the amine compound (IIa), the urea, and the hydroxy compound

Method (x1-2): step (x1-2-1) of reacting the amine compound (IIa) with the urea to produce a compound having a ureido group; and a step (x1-2-2) of reacting the compound having a ureido group obtained in the step (x1-2-1) with the hydroxy compound to produce the carbamate compound

(1-1) Method (x1-1)

In the method (x1-1), the amount of the hydroxy compound used (molar amount) is usually 1 time or more and 500 times or less, preferably 5 times or more and 200 times or less, more preferably 10 times or more and 150 times or less, and still more preferably 10 times or more and 100 times or less in terms of a stoichiometric ratio, with respect to the molar amount of the amino group of the amine compound (IIa) used.

The amount of the urea used (molar amount) is usually 1 time or more and 100 times or less, preferably 1 time or more and 10 times or less, more preferably 1 time or more and 3 times or less, and still more preferably 1 time or more and 1.5 times or less in terms of a stoichiometric ratio, with respect to the molar amount of the amino group of the amine compound (IIa) used.

The reaction temperature is usually 100°C or higher and 350°C or lower, preferably 150°C or higher and 300°C or lower, more preferably 180°C or higher and 280°C or lower, and still more preferably 200°C or higher and 260°C or lower.

The reaction pressure is usually 0.01 kPa or more and 10 MPa (absolute pressure), preferably 1 kPa or more and 3 MPa or less, more preferably 10 kPa or more and 1 MPa or less, and still more preferably 101 kPa or more and 0.5 MPa or less.

In order to increase the yield of the carbamate compound, it is necessary to carry out the reaction while removing the by-produced ammonia from the system as much as possible. Examples of a method for removing the ammonia to the outside of the system include a reactive distillation method, a method using an inert gas, a method using membrane separation, and a method using adsorption separation. In the reaction, a solvent or a catalyst can be used as necessary.

The reaction time (in a case of the continuous reaction, the residence time) is usually 0.01 hours or more and 100 hours or less, preferably 0.1 hours or more and 10 hours or less, more preferably 0.2 hours or more and 2 hours or less, and still more preferably 0.3 hours or more and 1 hour or less. The reaction time can also be determiined by the amount of the generated carbamate compound, which is the target compound of the method (x1-1).

(1-2) Method (x1-2)

Next, the step (x1-2-1) and the step (x1-2-2) in the method (x1-2) will be described.

In the step (x1-2-1), the amine compound (IIa) and urea are reacted with each other to produce a compound having a ureido group.

In the step (x1-2-1), the number of moles of urea is in a range of 1 to 100 times with respect to the number of amino groups of the compound represented by General Formula (III). Among these, it is preferably 1 time to 10 times, more preferably 1 time to 3 times, and still more preferably 1 time to 1.5 times.

The reaction temperature is usually 30°C or higher and 250°C or lower, preferably 100°C or higher and 200°C or lower, more preferably 120°C or higher and 200°C or lower, and still more preferably 130°C or higher and 180°C or lower.

The reaction pressure is usually 0.01 kPa or more and 10 MPa (absolute pressure), preferably 1 kPa or more and 3 MPa or less, more preferably 10 kPa or more and 1 MPa or less, and still more preferably 50 kPa or more and 0.5 MPa or less.

The reaction time (in a case of the continuous method, the residence time) is 0.01 hours or more and 100 hours or less, preferably 0.1 hours or more and 10 hours or less, more preferably 0.2 hours or more and 2 hours or less, and still more preferably 0.3 hours or more and 1 hour or less. The reaction can also be terminated after it is confirmed that the desired amount of the compound having a ureido group is produced.

In the step (x1-2-1), a catalyst or a solvent can be used as necessary. Preferably, the aromatic hydroxy compound used in the step (a) is used as the solvent.

In the step (x1-2-2), the compound having a ureido group obtained in the step (x1-2-1) and the hydroxy compound are reacted with each other to produce the carbamate compound.

In a case where the aromatic hydroxy compound is used as the reaction solvent in the step (x1-2-1), the step (x1-2-2) can be performed as it is using the reaction solution obtained in the step (x1-2-1).

The amount of the hydroxy compound used (molar amount) is usually 1 time or more and 500 times or less, preferably 5 times or more and 200 times or less, more preferably 10 times or more and 150 times or less, and still more preferably 10 times or more and 100 times or less in terms of a stoichiometric ratio, with respect to the molar amount of the ureido group of the compound having a ureido group used.

The reaction temperature is usually 100°C or higher and 350°C or lower, preferably 150°C or higher and 300°C or lower, more preferably 180°C or higher and 280°C or lower, and still more preferably 200°C or higher and 260°C or lower.

The reaction pressure is usually 0.01 kPa or more and 10 MPa (absolute pressure), preferably 1 kPa or more

and 3 MPa or less, more preferably 10 kPa or more and 1 MPa or less, and still more preferably 101 kPa or more and 0.5 MPa or less.

In order to increase the yield of the carbamate compound, it is necessary to carry out the reaction while removing the by-produced ammonia from the system as much as possible. Examples of a method for removing the ammonia to the outside of the system include a reactive distillation method, a method using an inert gas, a method using membrane separation, and a method using adsorption separation. In the reaction, a solvent or a catalyst can be used as necessary.

The reaction time (in a case of the continuous reaction, the residence time) is usually 0.01 hours or more and 100 hours or less, preferably 0.1 hours or more and 10 hours or less, more preferably 0.2 hours or more and 2 hours or less, and still more preferably 0.3 hours or more and 1 hour or less. The reaction time can also be determined by the amount of the generated carbamate compound, which is the target compound of the step (x1-2-2).

In the step (x1-2-2), a solvent or a catalyst can be used as necessary.

The catalyst is not particularly limited, and examples thereof include hydroxides and oxides of alkali metals and alkaline earth metals, such as sodium hydroxide, potassium hydroxide, magnesium hydroxide, magnesium oxide, calcium oxide, and barium oxide; heterocyclic compounds or tertiary amines, such as pyridine, methylpyridine (including isomers), dipropylethylamine, N-methylmorpholine, N-ethylmorpholine, triethylamine, and triethylenediamine; and metal oxides of Groups 3 to 14, such as aluminum oxide, zinc oxide, titanium oxide, zirconium oxide, cerium oxide, iron oxide, tin oxide, vanadium oxide, molybdenum oxide, manganese oxide, cobalt oxide, and nickel oxide.

In addition, a shape of the catalyst may be uniform or non-uniform with respect to the reaction solution.

The amount of the catalyst used is usually set to an amount in which the molar amount of the catalyst is 0.001 times or more and 10 times or less in terms of a stoichiometric ratio, with respect to the molar amount of the ureido group of the compound having a ureido group used. These catalysts can be used alone or in combination of two or more kinds thereof.

(2) Step (x2)

The carbamate compound produced in the step (x1) is a compound represented by General Formula (Vc) (hereinafter, may be referred to as "carbamate compound (Vc)").

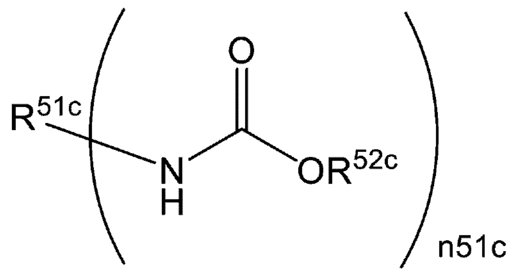

(Vc)

(in General Formula (Vc), $R^{51c}$ is an n51c- or higher valent organic group, and a relational expression of $R^{51c} = R^{71a}$ is satisfied, $R^{52c}$ is a monovalent organic group, and is a group derived from the hydroxy compound, where the "group derived from the hydroxy compound" is a residue obtained by removing a hydroxy group from the hydroxy compound, and n51c is an integer of 2 or more and 8 or less, and a relational expression of n51c = n71a is satisfied)

The step (x2) is a step of subjecting the carbamate compound (Vc) produced in the step (x1) to a thermal decomposition reaction to produce a component containing the isocyanate compound (VIIa).

The step (x2) can be performed in two methods, which are the following method (x2-1) and the following method (x2-2).

Method (x2-1): method of producing a component containing the isocyanate compound (VIIa) by subjecting the carbamate compound (Vc) to a thermal decomposition reaction to separate a gas-phase component containing the isocyanate compound (VIIa) and the high-boiling-point compound

Method (x2-2): method of producing the isocyanate compound (VIIa) by subjecting the carbamate compound (Vc) to a thermal decomposition reaction to recover a component containing the isocyanate compound (VIIa) and the high-boiling-point compound as liquid-phase components together and separating a gas-phase component containing the isocyanate compound (VIIa) and the high-boiling-point compound from the recovered liquid

The method (x2-1) and the method (x2-2) can be carried out using an appropriate solvent.

The reaction temperature at which the carbamate compound is thermally decomposed is usually in a range of 100°C or higher and 300°C or lower. The reaction pressure is usually in a range of 20 Pa or more and $1 \times 10^6$ Pa or less.

A catalyst is not necessarily required, but the catalyst may be used to lower the reaction temperature or to complete the reaction at an early stage. As the catalyst, it is possible to use an organic tin compound, a copper metal,

zinc, a compound of an iron metal, or the like, in an amount of 0.01% by mass or more and 30% by mass or less with respect to the mass of the carbamate compound.

The reaction time (in the case of the continuous method, the retention time) is preferably as short as possible within a range in which the progress of the desired reaction is not hindered.

In the method (x2-1), the hydroxy compound and the like contained in the reaction solution obtained in the step (x1) can be used as a solvent. In addition, in the thermal decomposition of the carbamate compound (Vc) in the method (x2-1), specifically, a method is adopted in which a mixture containing the carbamate compound (Vc) is continuously supplied to a reactor (which may be referred to as a thermal decomposition reactor) to carry out a thermal decomposition reaction, a part of the generated isocyanate compound (VIIa) and hydroxy compound is continuously extracted from the thermal decomposition reactor as a gas-phase component, and the remaining liquid-phase component is continuously extracted from the thermal decomposition reactor.

The form of the thermal decomposition reactor used in the method (x2-1) is not particularly limited, but it is preferable to use a known distillation device in order to efficiently recover the gas-phase component. As the form of the thermal decomposition reactor using a distillation device, various known methods are used, such as a method using a reactor including any one of a distillation column, a multi-stage distillation column, a multi-tube type reactor, a continuous multi-stage distillation column, a filled column, a thin film evaporator, a reactor having a support inside, a forced circulation reactor, a falling film evaporator, and a falling drop evaporator, and a method in which these reactors are combined. As the thermal decomposition reactor using the distillation device used in the present embodiment, a structure having a large gas-liquid contact area in which the low-boiling-point component to be produced (in the method (x2-1), the isocyanate compound (VIIa) and the hydroxy compound) is rapidly moved to the gas phase is preferable, a method using a tubular reactor is more preferable, and a method using a tubular thin film evaporator or a tubular falling film evaporator is still more preferable. As described above, from the viewpoint of recovering the gas-phase component containing the compound (VIIa) from the thermal decomposition reaction device, it is more preferable that the thermal decomposition reaction device includes the above-described tubular reactor and a separation tank for separating the gas-phase component and the liquid-phase component.

In the thermal decomposition of the carbamate compound (Vc) in the method (x2-2), specifically, a method is adopted in which a mixture containing the carbamate compound (Vc) is continuously supplied to a reactor (which may be referred to as a thermal decomposition reactor) to carry out a thermal decomposition reaction, the hydroxy compound is continuously extracted from the thermal decomposition reactor as a gas-phase component, and the remaining liquid-phase component containing the isocyanate compound (VIIa) and the high-boiling-point compound is continuously extracted from the thermal decomposition reactor.

In order to increase the yield of the carbamate compound (Vc), it is necessary to carry out the reaction while removing the by-produced hydroxy compound from the system as much as possible. Examples of a method for removing the hydroxy compound to the outside of the system include a reactive distillation method, a method using an inert gas, a method using membrane separation, and a method using adsorption separation. In the reaction, a solvent or a catalyst can be used as necessary.

The form of the thermal decomposition reactor used in the method (x2-2) is not particularly limited, but it is preferable to use a known distillation device in order to efficiently recover the gas-phase component. As the form of the thermal decomposition reactor using a distillation device, various known methods are used, such as a method using a reactor including any one of a distillation column, a multi-stage distillation column, a multi-tube type reactor, a continuous multi-stage distillation column, a filled column, a thin film evaporator, a reactor having a support inside, a forced circulation reactor, a falling film evaporator, and a falling drop evaporator, and a method in which these reactors are combined. As the thermal decomposition reactor using a distillation device used in the present embodiment, a structure having a large gas-liquid contact area, which can rapidly move the low-boiling-point component to be produced (in the method (x2-2), the hydroxy compound) to the gas phase and efficiently separate the carbamate compound (Vc), is preferable, a method using a tubular thin film evaporator or a tubular falling film evaporator is more preferable, and a method using a distillation column or a multi-stage distillation column is still more preferable.

As a method for separating the isocyanate compound (VIIa) from the mixture containing the isocyanate compound (VIIa) and the high-boiling-point compound, a method is adopted in which the mixture is continuously supplied to a separation device, the isocyanate compound (VIIa) is continuously extracted from the separation device as a gas-phase component, and the residual liquid containing the high-boiling-point compound is continuously extracted from the separation device as a liquid-phase component. The type of the separation device is not particularly limited, but for example, a known various methods such as a method using a separation device including any one of a distillation column, a multi-stage distillation column, a multi-tubular reactor, a continuous multi-stage distillation column, a filled column, and a thin film evaporator, and a method in which these are combined are used.

As described above, in the method (X), the liquid-phase component containing the high-boiling-point compound, which is continuously extracted from the thermal decomposition reactor, or the liquid-phase component including the high-boiling-point compound, which is continuously extracted in the distillation step of the isocyanate compound (VIIa),

is used as the liquid-phase component containing the high-boiling-point compound, which is a raw material of the recovery method according to the present embodiment.

The "high boiling point" of the high-boiling-point compound referred to herein does not refer to an absolute boiling point, but refers to a compound that is not a gas-phase component under the same conditions as a compound extracted as a gas-phase component from a thermal cracking reactor or a compound extracted as a gas-phase component in a distillation step of the isocyanate compound (VIIa).

As described above, the high-boiling-point compound often contains a biuret group, an allophanate group, an isocyanurate group, a urea group, a carbodiimide group, a uretonimine group, an iminotrimer group, and the like. In addition, in the method (X), by using the aromatic hydroxy compound in the step (x1), the high-boiling-point compound may include a Fries rearrangement body in which a carbamate group is free-rearranged, and a cyclized product of the Fries rearrangement body, which include an oxycarbonyl carbamate group. In a case where urea or N-unsubstituted carbamic acid is used in the step (x1), the high-boiling-point compound may include an isocyanurate group, a carbodiimide group, or the like. Among the above, in the high-boiling-point compound having an isocyanurate group which is by-produced by using urea or N-unsubstituted carbamic acid, each of the three nitrogen atoms of the isocyanurate group is bonded to $R^{71a}$ in General Formula (VIIa) to be described later or a hydrogen atom, that is, the bonding destination indicated by the three wavy lines in Formula (IX-2) may be $R^{71a}$ in General Formula (VIIa) to be described later or a hydrogen atom. The liquid-phase component continuously extracted from the thermal decomposition reactor may contain the carbamate compound (Vc) or the hydroxy compound used in the step (x1). The liquid-phase component continuously extracted from the separation device in the method (x2-2) may contain the isocyanate compound (VIIa), a carbodiimide group, a uretonimine group, an isocyanurate group, and an iminotrimer group. In particular, a high-boiling-point compound which is a by-product in a step of producing the isocyanate compound (VIIa) by thermal decomposition using a carbonic acid ester as a solvent may include an oxycarbonylcarbamate group.

In the recovery method according to the present embodiment, from the viewpoint of transfer to a reactor for carrying out a reaction between the liquid-phase component, the aromatic hydroxy compound, the active hydrogen-containing compound, and the catalyst, the liquid-phase component is preferably a liquid-phase component having a viscosity of 100 mPa-s or less at 150°C. The viscosity of the liquid-phase component can be measured by a known viscometer, for example, a capillary viscometer, a falling ball viscometer, or a rotary viscometer. Specifically, for example, the liquid-phase component can be heated to a predetermined temperature in a nitrogen atmosphere and measured using a B-type viscometer. In a case where the liquid-phase component contains a volatile component and it is difficult to measure the viscosity at 150°C, the viscosity may be measured at a low temperature, and the logarithm of the viscosity and the reciprocal of the measurement temperature (absolute temperature) may be plotted to calculate the viscosity at 150°C.

(Method (Y))

The method (Y) is a method including a step (y1) of reacting the amine compound (IIa) with phosgene to produce the isocyanate compound (VIIa) while dehydrochlorinating. The reaction between the amine compound (IIa) and phosgene is carried out in a solution or in a gas phase to produce a carbamic acid chloride, and the carbamic acid chloride is thermally decomposed to separate a by-product hydrogen chloride, thereby producing the isocyanate compound (VIIa). The formation of the carbamic acid chloride and the thermal decomposition of the carbamic acid chloride may be performed in separate steps or may be performed simultaneously in the same step.

The phosgene is used as a mixture with a solvent. The solvent to be used is not particularly limited as long as it is inert to phosgene, N-substituted carbamic acid chloride, and isocyanate, which is a decomposition product of the N-substituted carbamic acid chloride. Specific examples thereof include ester-based solvents such as amyl formate, amyl formate, acetic acid-n-butyl, acetic acid-n-amyl, methyl isobutyl acetate, methoxybutyl acetate, 2-ethylbutyl acetate, 2-ethylhexyl acetate, cyclohexyl acetate, methyl cyclohexyl acetate, benzyl acetate, ethyl propionate, propionic acid-n-butyl, isoamyl propionate, butyl stearate, methyl salicylate, dimethyl phthalate, and methyl benzoate; aromatic solvents such as benzene, toluene, xylene, chlorobenzene, dichlorobenzene, trichlorobenzene, nitrobenzene, naphthalene, chloronaphthalene, and dichloronaphthalene; and mixtures thereof.

The liquefied phosgene can be used for the reaction as it is or after being regasified.

An oxygen concentration in the raw material other than the phosgene used in the present embodiment is preferably 0.1% by mass or less.

The reaction temperature is preferably 10°C or higher and 300°C or lower, more preferably 30°C or higher and 250°C or lower, and still more preferably 50°C or higher and 200°C or lower.

The reaction pressure may be under reduced pressure, under atmospheric pressure, or under pressure.

As the reaction solvent, the same solvent as the solvent for dissolving the phosgene described above can be used. Among these, it is preferable to use the same kind of solvent as the solvent for dissolving the phosgene described above. In consideration of the solubility and ease of handling of the raw material compound or the product, as the reaction

solvent, chlorobenzene, orthodichlorobenzene, or isoamyl acetate is preferably used.

The reaction device is not particularly limited, and a known reactor can be used. For example, a conventionally known reactor such as a stirring tank, a pressurized stirring tank, a reduced pressure stirring tank, a tower-type reactor, a distillation column, a filled column, and a thin film distillation column can be appropriately combined and used.

In the method (Y), the liquid-phase component after the isocyanate compound (VIIa) is recovered is used as the liquid-phase component containing the high-boiling-point compound, which is a raw material of the recovery method according to the present embodiment. The liquid-phase component obtained by the method (Y) may contain a solvent used in the reaction.

Next, various raw materials used in the method for producing the isocyanate compound (VIIa) will be described in detail. As the amine compound used in the method for producing the isocyanate compound (VIIa), those described as the "amine compound (IIa)" in the amine compound recovered by the method for recovering an amine compound according to the present embodiment described later are preferably used. In addition, as the hydroxy compound used in the method for producing the isocyanate compound (VIIa), those described as the "hydroxy compound" in the hydroxy compound recovered by the method for recovering the isocyanate compound of the present embodiment described later are preferably used.

(Carbamate compound)

The carbamate compound (Vc) is a compound represented by General Formula (Vc).

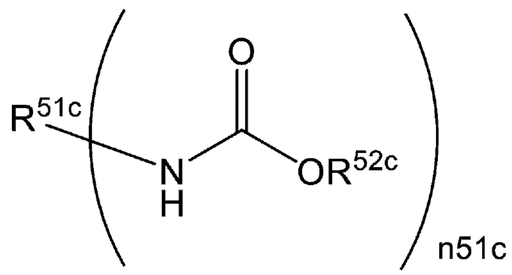

(Vc)

(in General Formula (Vc), $R^{51c}$ is an n51c- or higher valent organic group, and a relational expression of $R^{51c} = R^{71a}$ is satisfied, $R^{52c}$ is a monovalent organic group, and is a group derived from the hydroxy compound, where the "group derived from the hydroxy compound" is a residue obtained by removing a hydroxy group from the hydroxy compound, and n51c is an integer of 2 or more and 8 or less, and a relational expression of n51c = n7 la is satisfied)

($R^{51c}$)

$R^{51c}$ is an n51c-valent organic group, and a relational expression of $R^{51c} = R^{71a}$ is satisfied. That is, $R^{51c}$ is the same as $R^{71a}$ described above.

Among these, $R^{51c}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

In addition, as $R^{51c}$, specifically, a group represented by any one of Formulae (Ia-1) to (Ia-24) is preferable, and a group represented by Formula (Ia-1), (Ia-2), (Ia-3), (Ia-14), (Ia-18), or (Ia-19) is more preferable.

($R^{52c}$)

$R^{52c}$ is a monovalent organic group, and is a residue obtained by removing a hydroxyl group from the hydroxy compound.

Among the above, $R^{52c}$ is preferably an aromatic hydrocarbon group having 6 or more and 20 or less carbon atoms, which may include an oxygen atom.

In addition, as $R^{52c}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

(n51c)

n51c represents the number of carbamate groups, and a relational expression of n51c = n71a is satisfied. That is, n51c is the same as n71a described above. n51c is an integer of 2 or more and 8 or less, preferably an integer of 2 or more and 4 or less and more preferably an integer of 2 or more and 3 or less.

Examples of the preferred carbamate compound (Vc) include compounds represented by Formulae (Vc-1) to (Vc-4).

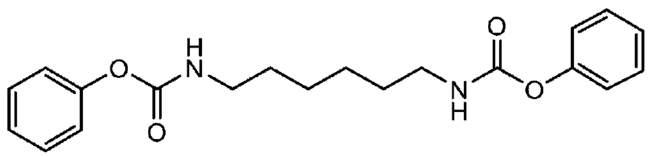

(Vc－1)

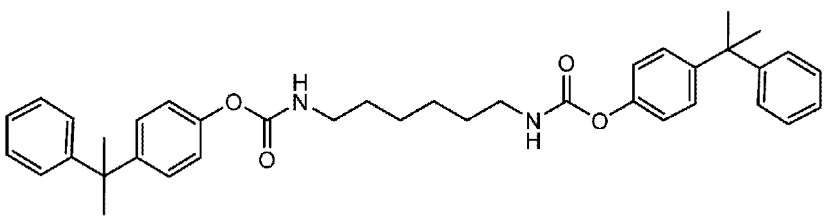

(Vc－2)

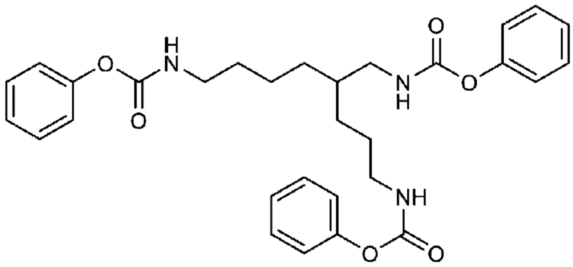

(V c − 3)

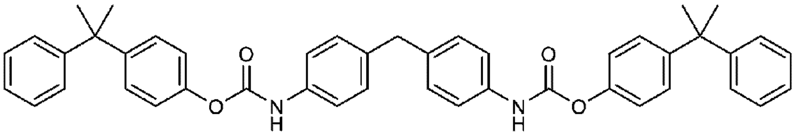

(V c − 4)

(Carbonic acid derivative)

As the carbonic acid derivative, urea, N-unsubstituted carbamic acid ester, and a carbonic acid ester are exemplary examples.

As the N-unsubstituted carbamic acid ester, ethyl N-unsubstituted carbamate, butyl N-unsubstituted carbamate, hexyl N-unsubstituted carbamate, octyl N-unsubstituted carbamate, phenyl N-unsubstituted carbamate, or the like is preferably used. The term "N-unsubstituted" refers to $H_2N$-COOR (R represents a hydrocarbon group), and is used for clarifying a difference with a structure R'-NH-COOR (R and R' each independently represent a hydrocarbon group) in which one hydrogen atom bonded to the nitrogen atom is substituted with a hydrocarbon group.

As the carbonic acid ester, dimethyl carbonate, diethyl carbonate, dibutyl carbonate, dihexyl carbonate, dioctyl carbonate, diphenyl carbonate, di(methylphenyl) carbonate, or the like is preferably used.

Among these carbonic acid derivatives, urea is preferable.

<Hydroxy compound>

In the recovery method according to the present embodiment, the hydroxy compound recovered is preferably a compound represented by General Formula (VIc) (hereinafter, may be referred to as "hydroxy compound (Vic)"). As described above, the hydroxy compound can also be said to be a raw material for producing the isocyanate compound (VIIa). In addition, in the recovery method according to the present embodiment, as the aromatic hydroxy compound used in the step (a1), a hydroxy compound having an aromatic group is preferably used among the hydroxy compounds described later.

$R^{61c}$-OH (VIc)

(in General Formula (VIc), $R^{61c}$ is a monovalent organic group)

[$R^{61c}$]

$R^{61c}$ is a monovalent organic group, and is preferably an aliphatic hydrocarbon group having 1 or more and 20 or less carbon atoms or an aromatic hydrocarbon group having 6 or more and 20 or less carbon atoms, each of which may include an oxygen atom.

Examples of the aliphatic hydrocarbon group as $R^{61c}$ include alkyl groups such as a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), an undecyl group (each isomer), a dodecyl group (each isomer), a tridecyl group (each isomer), a tetradecyl group (each isomer), a pentadecyl group (each isomer), a hexadecyl group (each isomer), a heptadecyl group (each isomer), an octadecyl group (each isomer), a nonadecyl (each isomer), and an eicosyl group (each isomer); and cycloalkyl groups such as a cyclopentyl group, a cyclohexyl group, a cycloheptyl group, a cyclooctyl group, a cyclononyl group, and a cyclodecyl group.

Examples of the aliphatic hydrocarbon group which may include an oxygen atom, as $R^{61c}$, include alkoxyalkyl groups such as a methoxyethyl group (each isomer), a methoxypropyl group (each isomer), a methoxybutyl group (each isomer), a methoxypentyl group (each isomer), a methoxyhexyl group (each isomer), a methoxyheptyl group (each

isomer), a methoxyoctyl group (each isomer), a methoxynonyl group (each isomer), a methoxydecyl group (each isomer), a methoxyundecyl group (each isomer), a methoxydodecyl group (each isomer), a methoxytridecyl group (each isomer), a methoxytetradecyl group (each isomer), a methoxypentadecyl group (each isomer), a methoxyhexadecyl group (each isomer), a methoxyheptadecyl group (each isomer), a methoxyoctadecyl group (each isomer), a methoxynonadecyl (each isomer), an ethoxymethyl group, an ethoxyethyl group (each isomer), an ethoxypropyl group (each isomer), an ethoxybutyl group (each isomer), an ethoxypentyl group (each isomer), an ethoxyhexyl group (each isomer), an ethoxyheptyl group (each isomer), an ethoxyoctyl group (each isomer), an ethoxynonyl group (each isomer), an ethoxydecyl group (each isomer), an ethoxyundecyl group (each isomer), an ethoxydodecyl group (each isomer), an ethoxytridecyl group (each isomer), an ethoxytetradecyl group (each isomer), an ethoxypentadecyl group (each isomer), an ethoxyhexadecyl group (each isomer), an ethoxyheptadecyl group (each isomer), an ethoxyoctadecyl group (each isomer), a propyloxymethyl group (each isomer), a propyloxyethyl group (each isomer), a propyloxypropyl group (each isomer), a propoxybutyl group (each isomer), a propoxybutyl group (each isomer), a propoxyhexyl group (each isomer), a propoxyheptyl group (each isomer), a propoxyoctyl group (each isomer), a propoxynonyl group (each isomer), a propoxydecyl group (each isomer), a propoxyundecyl group (each isomer), a propoxydodecyl group (each isomer), a propoxytridecyl group (each isomer), a propoxytetradecyl group (each isomer), a propoxypentadecyl group (each isomer), a propoxyhexadecyl group (each isomer), a propoxyheptadecyl group (each isomer), a butyloxymethyl group (each isomer), a butyloxyethyl group (each isomer), a butyloxypropyl group (each isomer), a butoxybutyl group (each isomer), a butoxybutyl group (each isomer), a butoxyhexyl group (each isomer), a butoxyheptyl group (each isomer), a butoxyoctyl group (each isomer), a butoxynonyl group (each isomer), a butoxydecyl group (each isomer), a butoxyundecyl group (each isomer), a group (each isomer), a butoxypentyl group (each isomer), a butoxyhexyl group (each isomer), a butoxyheptyl group (each isomer), a butoxyoctyl group (each isomer), a butoxynonyl group (each isomer), a butoxydecyl group (each isomer), a butoxyundecyl group (each isomer), a butyloxidodecyl group (each isomer), a butyloxotridecyl group (each isomer), a butyloxotetradecyl group (each isomer), a butyloxopentadecyl group (each isomer), a butyloxhexadecyl group (each isomer), a pentyloxymethyl group (each isomer), a pentyloxyethyl group (each isomer), a pentyloxopropyl group (each isomer), a pentyloxybutyl group (each isomer), a pentyloxypentyl group (each isomer), a pentyloxyhexyl group (each isomer), a pentyloxyheptyl group (each isomer), a pentyloxyoctyl group (each isomer), a pentyloxynonyl group (each isomer), a pentyloxodecyl group (each isomer), a pentyloxundecyl group (each isomer), a pentyloxydodecyl group (each isomer), a pentyloxotridecyl group (each isomer), a pentyloxotetradecyl group (each isomer), a pentyloxopentadecyl group (each isomer), a hexyloxymethyl group (each isomer), a hexyloxyethyl group (each isomer), a hexyloxopropyl group (each isomer), a hexyloxybutyl group (each isomer), a hexyloxy pentyl group (each isomer), a hexyloxy hexyl group (each isomer), a hexyloxy heptyl group (each isomer), a hexyloxy octyl group (each isomer), a hexyloxy nonyl group (each isomer), a hexyloxy decyl group (each isomer), a hexyloxy undecyl group (each isomer), a hexyloxy dodecyl group (each isomer), a hexyloxy tridecyl group (each isomer), a hexyloxy tetradecyl group (each isomer), a heptyloxy methyl group (each isomer), a heptyloxy ethyl group (each isomer), a heptyloxy propyl group (each isomer), a heptyloxy butyl group (each isomer), a heptyloxy pentyl group (each isomer), a heptyloxy hexyl group (each isomer), a heptyloxy heptyl group (each isomer), a heptyloxy octyl group (each isomer), a heptyloxy nonyl group (each isomer), a heptyloxy decyl group (each isomer), a heptyloxy undecyl group (each isomer), a heptyloxy dodecyl group (each isomer), an octyloxy methyl group (each isomer), an octyloxy ethyl group (each isomer), an octyloxy propyl group (each isomer), an octyloxy butyl group (each isomer), an octyloxy pentyl group (each isomer), an octyloxy hexyl group (each isomer), an octyloxy heptyl group (each isomer), an octyloxy octyl group (each isomer), an octyloxy nonyl group (each isomer), an octyloxy decyl group (each isomer), an octyloxy undecyl group (each isomer), an octyloxy dodecyl group (each isomer), an oxypentyl group (each isomer), an octyloxy hexyl group (each isomer), an octyloxy heptyl group (each isomer), an octyloxy octyl group (each isomer), an octyloxy nonyl group (each isomer), an octyloxydecyl group (each isomer), an octyloxyundecyl group (each isomer), an octyloxydodecyl group (each isomer), a nonyloxymethyl group (each isomer), a nonyloxyethyl group (each isomer), a nonyloxypropyl group (each isomer), a nonyloxybutyl group (each isomer), a nonyloxypentyl group (each isomer), a nonyloxyhexyl group (each isomer), a nonyloxyheptyl group (each isomer), a nonyloxyoctyl group (each isomer), a nonyloxyonyl group (each isomer), a nonyloxydecyl group (each isomer), a nonyloxyundecyl group (each isomer), a decyloxymethyl group (each isomer), a decyloxyethyl group (each isomer), a decyloxypropyl group (each isomer), a decyloxybutyl group (each isomer), a decyloxypentyl group (each isomer), a decyloxyhexyl group (each isomer), a decyloxyheptyl group (each isomer), a decyloxyoctyl group (each isomer), a decyloxyonyl group (each isomer), a decyloxydecyl group (each isomer), an undecyloxymethyl group (each isomer), an undecyloxyethyl group (each isomer), an undecyloxypropyl group (each isomer), an undecyloxybutyl group (each isomer), an undecyloxypentyl group (each isomer), an undecyloxyhexyl group (each isomer), an undecyloxyheptyl group (each isomer), an undecyloxyoctyl group (each isomer), an undecyloxyonyl group (each isomer), a dodecyloxymethyl group (each isomer), a dodecyloxyethyl group (each isomer), a ruthenium group (each isomer), an undecyloxyhexyl group (each isomer), an undecyloxyheptyl group (each isomer), an undecyloxyoctyl group (each isomer), an undecyloxyonyl group (each isomer), a dodecyloxyhexyl group (each isomer), a dodecyloxyheptyl group (each isomer), a dodecyloxyoctyl group (each isomer), a dodecyloxyonyl group (each isomer), a dodecylooxymethyl group (each isomer), a dodecyloxyethyl group (each isomer), a dodecyloxypropyl group (each isomer), a dodecyloxybutyl group (each isomer), a dodecyloxypentyl group (each isomer), a dodecyloxyhexyl group (each isomer), a dodecyloxyheptyl group (each isomer), a dodecyloxyoctyl group (each isomer), a tridecyloxymethyl group (each isomer), a tridecyloxyethyl group (each isomer), a tridecyloxypropyl group (each isomer), a tridecyloxybutyl group (each isomer), a tridecyloxypentyl group (each isomer), a tridecyloxyhexyl group (each isomer), a tridecyloxyheptyl group (each isomer), a tetradecyloxymethyl group (each isomer), a tetradecyloxyethyl group (each isomer), a tetradecyloxypropyl group (each isomer), a tetradecyloxybutyl group (each isomer), a tetradecyloxypentyl group (each isomer), a tetradecyloxyhexyl group (each isomer), a pentadecyloxymethyl group, a pentadecyloxyethyl group (each isomer), a pentadecyloxypropyl group (each isomer), a pentadecyloxybutyl group (each isomer), a pentadecyloxypentyl group (each isomer), a hexadecyloxymethyl group (each isomer), a hexadecyloxyethyl group (each isomer), a hexadecyloxypropyl group (each isomer), a hexadecyloxybutyl group (each isomer), a heptadecyloxymethyl group (each isomer), a heptadecyloxyethyl group (each isomer), a heptadecyloxypropyl group (each isomer), an octadecyloxymethyl group (each isomer), and an octadecyloxyethyl group (each isomer).

Examples of the aromatic hydrocarbon group as $R^{61c}$ include aryl groups such as a phenyl group, a naphthyl group, an anthryl group, a pyrenyl group, and a phenanthryl group; and aryl groups having an alkyl group as a substituent, such as a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), and a dibutylhexylphenyl group (each isomer).

Examples of the aromatic hydrocarbon group which may include an oxygen atom, as $R^{61c}$, include an alkoxyaryl group such as a methoxyphenyl group (each isomer) and an ethoxyphenyl group (each isomer).

Among these, as $R^{61c}$, an aromatic group is preferable; and a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each

isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a methyltetradecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is more preferable. In addition, a phenyl group, a methoxyphenyl group (each isomer), or an ethoxyphenyl group (each isomer) is still more preferable.

Examples of the preferred hydroxy compound (VIc) include ethanol, 1-butanol, and an aromatic hydroxy compound represented by General Formula (VIc-1) (hereinafter, may be referred to as "aromatic hydroxy compound (VIc-1)").

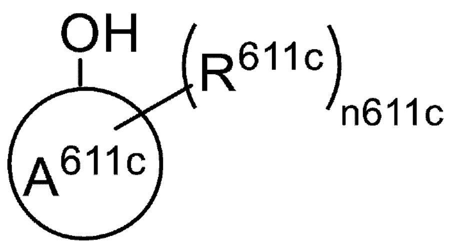

(VIc -1)

(in General Formula (VIc-1), a ring $A^{611c}$ is an aromatic hydrocarbon ring having 6 or more and 20 or less carbon atoms, $R^{611c}$ is a hydrogen atom, an alkyl group having 1 or more and 20 or less carbon atoms, an alkoxy group having 1 or more and 20 or less carbon atoms, an aryl group having 6 or more and 20 or less carbon atoms, an aryloxy group having 6 or more and 20 or less carbon atoms, an aralkyl group having 7 or more and 20 or less carbon atoms, an aralkyloxy group having 7 or more and 20 or less carbon atoms, or a hydroxy group, $R^{611c}$ may be bonded to the ring $A^{611c}$ to form a ring structure, and n611c is an integer of 1 or more and 10 or less)

($R^{611c}$)

Examples of the alkyl group having 1 or more and 20 or less carbon atoms, as $R^{611c}$, include a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), a dodecyl group (each isomer), and an octadecyl group (each isomer).

Examples of the alkoxy group having 1 or more and 20 or less carbon atoms, as $R^{611c}$, include a methoxy group, an ethoxy group, a propoxy group (each isomer), a butyloxy group (each isomer), a pentyloxy group (each isomer), a hexyloxy group (each isomer), a heptyloxy group (each isomer), an octyloxy group (each isomer), a nonyloxy group (each isomer), a decyloxy group (each isomer), a dodecyloxy group (each isomer), and an octadecyloxy group (each isomer).

Examples of the aryl group having 6 or more and 20 or less carbon atoms, as $R^{611c}$, include a phenyl group and a naphthyl group.

Examples of the aryl group having an alkyl group as the substituent in $R^{611c}$ include a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), a biphenyl group (each isomer), a dimethylphenyl group (each isomer), a diethylphenyl group (each isomer), a dipropylphenyl group (each isomer), a dibutylphenyl group (each isomer), a dipentylphenyl group (each isomer), a dihexylphenyl group (each isomer), a diheptylphenyl group (each isomer), a terphenyl group (each isomer), a trimethylphenyl group (each isomer), a triethylphenyl group (each isomer), a tripropylphenyl group (each isomer), and a tributylphenyl group (each isomer).

Examples of the aryloxy group having 6 or more and 20 or less carbon atoms, as $R^{611c}$, include a phenoxy group, a methylphenoxy group (each isomer), an ethylphenoxy group (each isomer), a propylphenoxy group (each isomer), a butylphenoxy group (each isomer), a pentylphenoxy group (each isomer), a hexylphenoxy group (each isomer), a heptylphenoxy group (each isomer), an octylphenoxy group (each isomer), a nonylphenoxy group (each isomer), a decylphenoxy group (each isomer), a phenylphenoxy group (each isomer), a dimethylphenoxy group (each isomer), a diethylphenoxy group (each isomer), a dipropylphenoxy group (each isomer), a dibutylphenoxy group (each isomer), a dipentylphenoxy group (each isomer), a dihexylphenoxy group (each isomer), a diheptylphenoxy group (each isomer), a diphenylphenoxy group (each isomer), a trimethylphenoxy group (each isomer), a triethylphenoxy group (each isomer), a tripropylphenoxy group (each isomer), and a tributylphenoxy group (each isomer).

Examples of the aralkyl group having 7 or more and 20 or less carbon atoms, as $R^{611c}$, include a phenylmethyl group, a phenylethyl group (each isomer), a phenylpropyl group (each isomer), a phenylbutyl group (each isomer), a phenylpentyl group (each isomer), a phenylhexyl group (each isomer), a phenylheptyl group (each isomer), a phenyloctyl group (each isomer), and a phenylnonyl group (each isomer).

Examples of the aralkyloxy group having 7 or more and 20 or less carbon atoms, as $R^{611c}$, include a phenylmethoxy group, a phenylethoxy group (each isomer), a phenylpropyloxy group (each isomer), a phenylbuthyloxy group (each isomer), a phenylpentyloxy group (each isomer), a phenylhexyloxy group (each isomer), a phenylheptyloxy group (each isomer), a phenyloctyloxy group (each isomer), and a phenylnonyloxy group (each isomer).

($A^{611c}$)

The ring $A^{611c}$ is an aromatic hydrocarbon ring having 6 or more and 20 or less carbon atoms. The ring $A^{611c}$ may be a monocyclic ring, a polycyclic ring, or a fused ring.

Specific examples of the ring $A^{611c}$ include a benzene ring, a naphthalene ring, an anthracene ring, a phenanthrene ring, a naphthacene ring, a chrysene ring, a pyrene ring, a triphenylene ring, a pentylene ring, an azulene ring, a heptalene ring, an indacene ring, a biphenylene ring, an acenaphthylene ring, an acenanthrylene ring, and an acenaphthylene ring. Among these, the ring $A^{611c}$ is preferably a benzene ring, a naphthalene ring, or an anthracene ring, and more preferably a benzene ring.

In addition, these rings may have a substituent other than $R^{611c}$. Examples of the substituent other than $R^{611c}$ include the same one as that described in $R^{611c}$. $R^{611c}$ and the substituent other than $R^{611c}$ consist of different functional groups.

(n611c)

n611 c represents the number of substituents $R^{611c}$ and is an integer of 1 or more and 10 or less.

In General Formula (VIc-1), examples of the compound in which the ring $A^{611c}$ is a benzene ring include a compound represented by General Formula (VIc-1-1) (hereinafter, may be referred to as "aromatic hydroxy compound (VIc-1-1)").

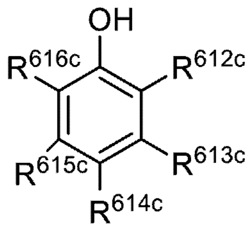

( VIc-1-1 )

(in General Formula (VIc-1-1), $R^{612c}$, $R^{613c}$, $R^{614c}$, $R^{615c}$, and $R^{616c}$ are each independently the same as $R^{611c}$ described above)

Among these, it is preferable that at least one of $R^{612c}$, $R^{613c}$, $R^{614c}$, $R^{615c}$, or $R^{616c}$ is a hydrogen atom, and it is more preferable that all of $R^{612c}$, $R^{613c}$, $R^{614c}$, $R^{615c}$, and $R^{616c}$ are hydrogen atoms.

Examples of the preferred aromatic hydroxy compound (VIc-1-1) include phenol, 2-ethylphenol, 2-propylphenol (each isomer), 2-butylphenol (each isomer), 2-pentylphenol (each isomer), 2-hexylphenol (each isomer), 2-heptylphenol (each isomer), 2-phenylphenol, 2,6-dimethylphenol, 2,4-diethylphenol, 2,6-diethylphenol, 2,4-dipropylphenol (each isomer), 2,6-dipropylphenol (each isomer), 2,4-dibutylphenol (each isomer), 2,4-dipentylphenol (each isomer), 2,4-dihexylphenol (each isomer), 2,4-diheptylphenol (each isomer), 2-methyl-6-ethylphenol, 2-methyl-6-propylphenol (each isomer), 2-methyl-6-butylphenol (each isomer), 2-methyl-6-pentylphenol (each isomer), 2-ethyl-6-propylphenol (each isomer), 2-ethyl-6-butylphenol (each isomer), 2-ethyl-6-pentylphenol (each isomer), 2-propyl-6-butylphenol (each isomer), 2-ethyl-4-methylphenol (each isomer), 2-ethyl-4-propylphenol (each isomer), 2-ethyl-4-butylphenol (each isomer), 2-ethyl-4-pentylphenol (each isomer), 2-ethyl-4-hexylphenol (each isomer), 2-ethyl-4-heptylphenol (each isomer), 2-ethyl-4-octylphenol (each isomer), 2-ethyl-4-phenylphenol (each isomer), 2-ethyl-4-cumylphenol (each isomer), 2-propyl-4-methylphenol (each isomer), 2-propyl-4-ethylphenol (each isomer), 2-propyl-4-butylphenol (each isomer), 2-propyl-4-pentylphenol (each isomer), 2-propyl-4-hexylphenol (each isomer), 2-propyl-4-heptylphenol (each isomer), 2-propyl-4-octylphenol (each isomer), 2-propyl-4-phenylphenol (each isomer), 2-propyl-4-cumylphenol (each isomer), 2-butyl-4-methylphenol (each isomer), 2-butyl-4-ethylphenol (each isomer), 2-butyl-4-propylphenol (each isomer), 2-butyl-4-pentylphenol (each isomer), 2-butyl-4-hexylphenol (each isomer), 2-butyl-4-heptylphenol (each isomer), 2-butyl-4-octylphenol (each isomer), 2-butyl-4-phenylphenol (each isomer), 2-butyl-4-cumylphenol (each isomer), 2-pentyl-4-methylphenol (each isomer), 2-pentyl-4-ethylphenol (each isomer), 2-pentyl-4-propylphenol (each isomer), 2-pentyl-4-butylphenol (each isomer), 2-pentyl-4-hexylphenol (each isomer), 2-pentyl-4-heptylphenol (each isomer), 2-pentyl-4-octylphenol (each isomer), 2-pentyl-4-phenylphenol (each isomer), 2-pentyl-4-cumylphenol (each isomer), 2-hexyl-4-methylphenol (each isomer), 2-hexyl-4-ethylphenol (each isomer), 2-hexyl-4-propylphenol (each isomer), 2-hexyl-4-butylphenol (each isomer), 2-hexyl-4-pentylphenol (each isomer), 2-hexyl-4-heptylphenol (each isomer), 2-hexyl-4-octylphenol (each isomer), 2-hexyl-4-phenylphenol (each isomer), 2-hexyl-4-cumylphenol (each isomer), 2-heptyl-4-methylphenol (each isomer), 2-heptyl-4-ethylphenol (each isomer), 2-heptyl-4-propylphenol (each isomer), 2-heptyl-4-butylphenol (each isomer), 2-heptyl-4-pentylphenol (each isomer), 2-heptyl-4-hexylphenol (each isomer), 2-heptyl-4-octylphenol (each isomer), 2-heptyl-4-phenylphenol (each isomer), 2-heptyl-4-cumylphenol (each isomer), 2,4,6-trimethylphenol, 2,6-dimethyl-4-ethylphenol, 2,6-dimethyl-4-propylphenol (each isomer), 2,6-dimethyl-4-butylphenol (each isomer), 2,6-dimethyl-4-pentylphenol (each isomer), 2,6-dimethyl-4-hexylphenol (each isomer), 2,6-dimethyl-4-phenylphenol, 2,6-dimethyl-4-cumylphenol, 2,4,6-triethylphenol, 2,6-diethyl-4-methylphenol, 2,6-diethyl-4-propylphenol (each isomer), 2,6-diethyl-4-butylphenol (each isomer), 2,6-diethyl-4-pentylphenol (each isomer), 2,6-diethyl-4-hexylphenol (each isomer), 2,6-diethyl-4-phenylphenol, 2,6-diethyl-4-cumylphenol, 2,4,6-tripropylphenol (each isomer), 2,6-dipropyl-4-ethylphenol (each isomer), 2,6-dipropyl-4-methylphenol (each isomer), 2,6-dipropyl-4-butylphenol (each isomer), 2,6-dipropyl-4-pentylphenol (each isomer), 2,6-dipropyl-4-hexylphenol (each isomer), 2,6-dipropyl-4-phenylphenol (each isomer), 2,6-dipropyl-4-cumylphenol (each isomer), 2,4-dimethyl-6-ethylphenol, 2-methyl-4,6-diethylphenol, 2-methyl-4-propyl-6-ethylphenol (each isomer), 2-methyl-4-butyl-6-ethylphenol (each isomer), 2-methyl-4-pentyl-6-ethylphenol (each isomer), 2-methyl-4-hexyl-6-ethylphenol (each isomer), 2-methyl-4-phenyl-6-ethylphenol (each isomer), 2-methyl-4-cumyl-6-ethylphenol (each isomer), 2,4-dimethyl-6-propylphenol (each isomer), 2-methyl-4,6-dipropylphenol (each isomer), 2-methyl-4-ethyl-6-propylphenol (each isomer), 2-methyl-4-butyl-6-propylphenol (each isomer), 2-methyl-4-pentyl-6-propylphenol (each isomer), 2-methyl-4-hexyl-6-propylphenol (each isomer), 2-methyl-4-phenyl-6-propylphenol (each isomer), 2-methyl-4-cumyl-6-propylphenol (each isomer), 2,4-dimethyl-6-butylphenol, 2-methyl-4,6-dibutylphenol, 2-methyl-4-propyl-6-butylphenol (each isomer), 2-methyl-4-ethyl-6-butylphenol (each isomer), 2-methyl-4-pentyl-6-butylphenol (each isomer), 2-methyl-4-hexyl-6-butylphenol (each isomer), 2-methyl-4-phenyl-6-butylphenol (each isomer), 2-methyl-4-cumyl-6-butylphenol (each isomer), 2,4-dimethyl-6-pentylphenol, 2-methyl-4,6-dipentylphenol, 2-methyl-4-propyl-6-pentylphenol (each isomer), 2-methyl-4-butyl-6-pentylphenol (each isomer), 2-methyl-4-ethyl-6-pentylphenol (each isomer), 2-methyl-4-hexyl-6-pentylphenol (each isomer), 2-methyl-4-phenyl-6-pentylphenol (each isomer), 2-methyl-4-cumyl-6-pentylphenol (each isomer), 2,4-dimethyl-6-hexylphenol, 2-methyl-4,6-dihexylphenol, 2-methyl-4-propyl-6-hexylphenol (each isomer), 2-methyl-4-butyl-6-hexylphenol (each isomer), 2-methyl-4-pentyl-6-hexylphenol (each isomer), 2-methyl-4-ethyl-6-hexylphenol (each isomer), 2-methyl-4-phenyl-6-hexylphenol (each isomer), 2-methyl-4-cumyl-6-hexylphenol (each isomer), 2-ethyl-4-methyl-6-propylphenol (each isomer), 2,4-diethyl-6-propylphenol (each isomer), 2-ethyl-4,6-propylphenol (each isomer), 2-ethyl-4-butyl-6-propylphenol (each isomer), 2-ethyl-4-pentyl-6-propylphenol (each isomer), 2-ethyl-4-hexyl-6-propylphenol (each isomer), 2-ethyl-4-heptyl-6-propylphenol (each isomer), 2-ethyl-4-octyl-6-propylphenol (each isomer), 2-ethyl-4-phenyl-6-propylphenol (each isomer), 2-ethyl-4-cumyl-6-propylphenol (each isomer), 2-ethyl-4-methyl-6-butylphenol (each isomer), 2,4-diethyl-6-butylphenol (each isomer), 2-ethyl-4,6-butylphenol (each isomer), 2-ethyl-4-propyl-6-butylphenol (each isomer), 2-ethyl-4-pentyl-6-butylphenol (each isomer), 2-ethyl-4-hexyl-6-butylphenol (each isomer), 2-ethyl-4-heptyl-6-butylphenol (each isomer), 2-ethyl-4-octyl-6-butylphenol (each isomer), 2-ethyl-4-phenyl-6-butylphenol (each isomer), 2-ethyl-4-cumyl-6-butylphenol (each isomer), 2-ethyl-4-methyl-6-pentylphenol (each isomer), 2,4-diethyl-6-pentylphenol (each isomer), 2-ethyl-4,6-pentylphenol (each isomer), 2-ethyl-4-butyl-6-pentylphenol (each isomer), 2-ethyl-4-propyl-6-pentylphenol (each isomer), 2-ethyl-4-hexyl-6-pentylphenol (each isomer), 2-ethyl-4-heptyl-6-pentylphenol (each isomer), 2-ethyl-4-octyl-6-pentylphenol (each isomer), 2-ethyl-4-phenyl-6-pentylphenol (each isomer), 2-ethyl-4-cumyl-6-pentylphenol (each isomer), 2-ethyl-4-methyl-6-hexylphenol (each isomer), 2,4-diethyl-6-hexylphenol (each isomer), 2-ethyl-4,6-hexylphenol (each isomer), 2-ethyl-4-propyl-6-hexylphenol (each isomer), 2-ethyl-4-pentyl-6-hexylphenol (each isomer), 2-ethyl-4-butyl-6-hexylphenol (each isomer), 2-ethyl-4-heptyl-6-hexylphenol (each isomer), 2-ethyl-4-octyl-6-hexylphenol (each isomer), 2-ethyl-4-phenyl-6-hexylphenol (each isomer), 2-ethyl-4-cumyl-6-hexylphenol (each isomer), 2-propyl-4-methyl-6-butylphenol (each isomer), 2,4-dipropyl-6-butylphenol (each isomer), 2-propyl-4,6-butylphenol (each isomer), 2-propyl-4-ethyl-6-butylphenol (each isomer), 2-propyl-4-pentyl-6-butylphenol (each isomer), 2-propyl-4-hexyl-6-butylphenol (each isomer), 2-propyl-4-heptyl-6-butylphenol (each isomer), 2-propyl-4-octyl-6-butylphenol (each isomer), 2-propyl-4-phenyl-6-butylphenol (each isomer), 2-propyl-4-cumyl-6-butylphenol (each isomer), 2,4-dicumylphenol, methoxyphenol (each isomer), and ethoxyphenol (each isomer). Among these, phenol, methoxyphenol (each isomer), or ethoxyphenol (each isomer) is preferable.

<Amine compound (IIa)>

The amine compound (IIa) recovered by the recovery method according to the present embodiment is a compound represented by General Formula (IIa). As described above, the amine compound (IIa) can also be said to be a raw material for producing the isocyanate compound (VIIa).

$$R^{21a}\left(-NH_2\right)_{n21a} \qquad (IIa)$$

(in General Formula (IIa), $R^{21a}$ is an n21a-valent organic group, and a relational expression of $R^{21a} = R^{71a}$ is satisfied, and n21a is an integer of 2 or more and 8 or less, and a relational expression of n21a = n71a is satisfied)

[$R^{21a}$]

$R^{21a}$ is an n21a-valent organic group, and a relational expression of $R^{21a} = R^{71a}$ is satisfied. That is, $R^{21a}$ is the same as $R^{71a}$ described above.

Among these, $R^{21a}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

In addition, as $R^{21a}$, specifically, a group represented by any one of Formulae (Ia-1) to (Ia-24) is preferable, and a group represented by Formula (Ia-1), (Ia-2), (Ia-3), (Ia-14), (Ia-18), or (Ia-19) is more preferable.

[n21a]

n21a represents the number of amino groups and is an integer of 2 or more and 8 or less, and a relational expression of n21a = n71a is satisfied. That is, n21a is the same as n71a described above. n21a is an integer of 2 or more and 8 or less, preferably an integer of 2 or more and 6 or less, more preferably an integer of 2 or more and 5 or less, and still more preferably an integer of 3 or more and 4 or less.

In a case where $R^{21a}$ is an aliphatic hydrocarbon group, specific examples of the amine compound (IIa) include aliphatic diamines, aliphatic triamines, and substituted cyclic aliphatic polyamines.

Examples of the aliphatic diamines include ethylenediamine, diaminopropane (each isomer), diaminobutane (each isomer), diaminopentane (each isomer), diaminohexane (each isomer), and diaminodecane (each isomer).

Examples of the aliphatic triamines include triaminohexane (each isomer), triaminononane (each isomer),

triaminodecane (each isomer), and 4-aminomethyl-1,8-octanediamine.

Examples of the substituted cyclic aliphatic polyamines include diaminocyclobutane (each isomer), diaminocyclohexane (each isomer), 3-aminomethyl-3,5,5-trimethylcyclohexylamine (a cis form and/or a trans form), methylenebis(cyclohexylamine) (each isomer), and triaminocyclohexane (each isomer).

In a case where $R^{21a}$ is an aromatic group, specific examples of the amine compound (IIa) include aromatic diamines and aromatic triamines.

Examples of the aromatic diamines include diaminobenzene (each isomer), diaminotoluene (each isomer), methylenedianiline (each isomer), diaminomethylidene (each isomer), diaminobiphenyl (each isomer), diaminodibenzyl (each isomer), bis (aminophenyl) propane (each isomer), bis (aminophenyl) ether (each isomer), bis (aminophenoxyethane) (each isomer), diaminoxylene (each isomer), diaminosol (each isomer), diaminophenol (each isomer), diaminonaphthalene (each isomer), diamino-methylbenzene (each isomer), diamino-methylpyridine (each isomer), diamino-methylnaphthalene (each isomer), and diamino-diphenylmethane (each isomer).

Examples of the aromatic triamines include triaminobenzene (each isomer) and methanetriyltrianiline (each isomer).

Specific examples of the amine compound (IIa) in a case where the aliphatic hydrocarbon group or the aromatic group as $R^{21a}$ has 1 or more and 4 or less ester groups include 2-aminoethyl acrylate, 2-aminoethyl 2-methylacrylate, 2-aminopropyl acrylate, 2-aminopropyl 2-methylacrylate, 3-aminopropyl acrylate, 3-aminopropyl 2-methylacrylate, 4-aminobutyl acrylate, 4-aminobutyl 2-methylacrylate, 5-aminopentyl acrylate, 5-aminopentyl 2-methylacrylate, 6-aminohexyl acrylate, 2-methyl-6-aminohexyl acrylate,acrylic acid-8-aminooctyl ester, 2-methyl-acrylic acid-8-aminooctyl ester, acrylic acid-10-aminodecyl ester, 2-methyl-acrylic acid-10-aminodecyl ester, acrylic acid-11-aminonondecyl ester, 2-methyl-acrylic acid-11-aminonondecyl ester, acrylic acid-12-aminododecyl ester, 2-methyl-acrylic acid-12-aminododecyl ester, lysine methylesterdiamine, lysine ethylesterdiamine, 2-aminoethyl-2,5-diaminopentanoate, 2-aminoethyl-2,6-diaminohexanoate, bis(2-aminoethyl)-2-aminobutanedioate, bis(2-aminoethyl)-2-aminopentanedioate, and tris(2-aminoethyl) hexane-1,3,6-tricarboxylate.

Examples of the preferred amine compound (IIa) include diaminopentane (each isomer), diaminohexane (each isomer), 4-aminomethyl-1,8-octanediamine, lysine ethyl ester diamine, 3-aminomethyl-3,5,5-trimethylcyclohexylamine (cis and/or trans form), methylenebis(cyclohexylamine), triaminocyclohexane (each isomer), diaminotoluene (each isomer), methylenedianiline (each isomer), triaminobenzene (each isomer), and methanetriyltrianiline (each isomer).

<<Isocyanate composition>>

The isocyanate composition according to the present embodiment contains an isocyanate compound; a carbonyl compound represented by General Formula (I) (hereinafter, may be referred to as "carbonyl compound (I)"); and
a modified product of an isocyanate compound, in which a part of an isocyanate group of the isocyanate compound is converted into one or more functional groups selected from the group consisting of an isocyanurate group, a carbodiimide group, a uretonimine group, and an allophanate group.

The isocyanate compound is a different compound from the carbonyl compound.

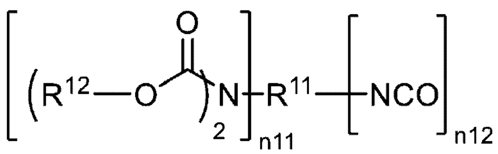 (I)

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less)

The carbonyl compound (I) and the modified product of the isocyanate compound are by-products by-produced in a case where a carbamate compound described later is thermally decomposed to produce an isocyanate compound.

In the isocyanate composition according to the present embodiment, the carbonyl compound (I) acts as a good solvent for a modified product of the isocyanate compound, and the carbonyl compound (I) acts as a terminal sealing agent to suppress an increase in molecular weight of the modified product of the isocyanate compound, whereby it is possible to suppress the generation of a solid matter and an increase in liquid viscosity in the generation system of the isocyanate compound due to the thermal decomposition reaction of the carbamate compound.

In addition, the carbonyl compound (I) has a higher boiling point than the isocyanate compound and has fewer cross-linking points than the modified product of the isocyanate compound. Therefore, the carbonyl compound (I) can also act as a solvent during the purification of the isocyanate compound. Alternatively, the carbonyl compound (I) is bonded to a functional group such as a carbodiimide group, and the increase in molecular weight due to the bonding of

the modified products of the isocyanate to each other is prevented. These actions make it possible to improve operability in the purification of the isocyanate compound and to recover the isocyanate compound at a high yield.

In the isocyanate composition according to the present embodiment, a value of {3 × (Molar amount of isocyanurate group) + 2 × (Molar amount of carbodiimide group) + 3 × (Molar amount of uretonimine group) + 2 × (Molar amount of allophanate group)} ÷ (Molar amount of carbonyl compound) is 0.001 or more and 8.0 or less, preferably 0.01 or more and 7.8 or less, more preferably 0.1 or more and 7.5 or less, still more preferably 1.0 or more and 7.0 or less, and particularly preferably 2.0 or more and 6.5 or less. In a case where the above-described value is equal to or less than the above-described upper limit value, in particular, in the thermal decomposition step and the purification step described later, in a case of producing an isocyanate compound without using phosgene, it is possible to prevent the by-product from being fixed to the device and to improve the yield of the isocyanate compound. On the other hand, in a case of being equal to or more than the above-described lower limit value, the amount of the carbonyl compound used can be suppressed, and the raw material cost can be suppressed.

The above-described value can be calculated by substituting the measured values into the above expression after measuring the molar amount of each of the isocyanurate group, the carbodiimide group, the uretonimine group, the allophanate group, and the carbonyl compound by a combination of $^1$H-NMR, liquid chromatography, and gas chromatography. As detailed conditions of the above-described analysis, conditions described in Examples can be adopted.

In the isocyanate composition according to the present embodiment, a content of the isocyanate compound is preferably 1% by mass or more and 99% by mass or less, more preferably 5% by mass or more and 99% by mass or less, still more preferably 10% by mass or more and 99% by mass or less, even more preferably 30% by mass or more and 99% by mass or less, even still more preferably 50% by mass or more and 99% by mass or less, and particularly preferably 70% by mass or more and 99% by mass or less with respect to the total mass of the isocyanate composition.

In the isocyanate composition according to the present embodiment, the total content of the carbonyl compound (I) and the modified product of the isocyanate compound is preferably 1% by mass or more and 99% by mass or less, more preferably 1% by mass or more and 95% by mass or less, still more preferably 1% by mass or more and 90% by mass or less, even more preferably 1% by mass or more and 70% by mass or less, even still more preferably 1% by mass or more and 50% by mass or less, and particularly preferably 1 % by mass or more and 30% by mass or less with respect to the total mass of the isocyanate composition.

In the isocyanate composition according to the present embodiment, a content of the carbonyl compound (I) is preferably 1% by mass or more and 99% by mass or less, more preferably 3% by mass or more and 95% by mass or less, still more preferably 5% by mass or more and 90% by mass or less, even more preferably 7% by mass or more and 80% by mass or less, even still more preferably 10% by mass or more and 70% by mass or less, and particularly preferably 10% by mass or more and 60% by mass or less with respect to the total mass of the isocyanate composition.

The content of each of the compounds in the isocyanate composition according to the present embodiment can be calculated by, for example, $^1$H-NMR liquid chromatography and gas chromatography analysis in combination. As detailed conditions of the above-described analysis, conditions described in Examples can be adopted.

By having the above-described configuration of the isocyanate composition according to the present embodiment, it is possible to prevent fixation of by-products to a device and improve the yield of an isocyanate compound in a case of producing an isocyanate compound without using phosgene.

Each component contained in the isocyanate composition according to the present embodiment will be described in detail below.

<Carbonyl compound (I)>

The carbonyl compound (I) is a compound represented by General Formula (I).

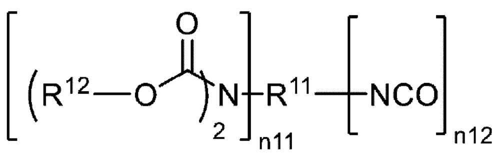

(I)

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less)

[$R^{11}$]

$R^{11}$ is an (n11 + n12)-valent organic group, that is, a di- or higher and octa- or lower valent organic group. Among these, $R^{11}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

As the aliphatic hydrocarbon group as $R^{11}$, an alkylene group or an alkanetriyl group, a cycloalkyl group, a cycloalkylene group or a cycloalkanetriyl group, or a group constituting the alkyl group, the alkylene group or the alkanetriyl group, and the cycloalkyl group, the cycloalkylene group or the cycloalkanetriyl group is preferable; and a linear or branched alkylene group or alkanetriyl group, a cycloalkylene group or a cycloalkanetriyl group, or a group constituting the alkylene group or the alkanetriyl group, and the cycloalkyl group, the cycloalkylene group or the cycloalkanetriyl group is more preferable.

Examples of the linear or branched alkylene group include a methylene group, an ethylene group, a propylene group, a trimethylene group, a pentylene group, an n-hexylene group, and a decamethylene group.

Examples of the cycloalkylene group include a cyclobutylene group and a cyclohexylene group.

Examples of the linear or branched alkanetriyl group include a hexanetriyl group, a nonanetriyl group, and a decanetriyl group.

Examples of the cycloalkanetriyl group include a cyclopropanetriyl group, a cyclobutanetriyl group, a cyclopentatriyl group, and a cyclohexatriyl group.

The aromatic hydrocarbon group as $R^{11}$ is preferably a group having a substituted or unsubstituted aromatic ring having 6 or more and 13 or less carbon atoms. Examples of the substituent include an alkyl group, an aryl group, and an aralkyl group. The aromatic ring may be an aromatic hydrocarbon ring or a heteroaromatic ring, and specific examples thereof include a benzene ring, a naphthalene ring, and a pyridine ring.

In a case where the aliphatic hydrocarbon group or the aromatic group as $R^{11}$ has 1 or more and 4 or less ester groups, as $R^{11}$, specifically, for example, a group obtained by removing a terminal primary amino group of an amine compound having an ester group obtained by reacting a carboxy group of amino acid with a hydroxy compound is preferable.

Specific examples of the amine compound having an ester group herein include 2-aminoethyl acrylate, 2-aminoethyl 2-methylacrylate, 2-aminopropyl acrylate, 2-aminopropyl 2-methylacrylate, 3-aminopropyl acrylate, 3-aminopropyl 2-methylacrylate, 4-aminobutyl acrylate, 4-aminobutyl 2-methylacrylate, 5-aminopentyl acrylate, 5-aminopentyl 2-methylacrylate, 6-aminohexyl acrylate,2-methyl-6-aminohexyl acrylate, acrylic acid-8-aminooctyl ester, 2-methyl-acrylic acid-8-aminooctyl ester, acrylic acid-10-aminodecyl ester, 2-methyl-acrylic acid-10-aminodecyl ester, acrylic acid-11-aminonondecyl ester, 2-methyl-acrylic acid-11-aminonondecyl ester, acrylic acid-12-aminododecyl ester, 2-methyl-acrylic acid-12-aminododecyl ester, lysine methylesterdiamine, lysine ethylesterdiamine, 2-aminoethyl-2,5-diaminopentanoate, 2-aminoethyl-2,6-diaminohexanoate, bis(2-aminoethyl)-2-aminobutanedioate, bis(2-aminoethyl)-2-aminopentanedioate, and tris(2-aminoethyl) hexane-1,3,6-tricarboxylate.

Examples of the amino acid used for producing the amine compound having an ester group include lysine, alanine, arginine, asparagine, glutamine, glycine, aspartic acid, glutamic acid, ornithine, histidine, isoleucine, leucine, methionine, phenylalanine, tryptophan, and valine. Among these, the amino acid is preferably lysine, arginine, glycine, aspartic acid, glutamic acid, or ornithine; and more preferably lysine, arginine, glycine, aspartic acid, or glutamic acid.

Examples of the hydroxy compound used for producing the amine compound having an ester group include alcohols and aromatic hydroxy compounds.

Examples of the alcohols include methyl alcohol, propyl alcohol, butyl alcohol, amyl alcohol, hexyl alcohol, heptyl alcohol, octyl alcohol, nonyl alcohol, decyl alcohol, undecyl alcohol, lauryl alcohol, dodecyl alcohol, stearyl alcohol, eicosyl alcohol, allyl alcohol, crotyl alcohol, propargyl alcohol, cyclopentanol, cyclohexanol, benzyl alcohol, cinnamyl alcohol, ethylene glycol, diethylene glycol, polyethylene glycol, propylene glycol, polypropylene glycol, 1,3-butanediol, 1,4-butanediol, hydrogenated bisphenol A, neopentyl glycol, glycerin, trimethylolpropane, pentaerythritol, ethanolamine, propanolamine (1-amino-2-propanol), dimethanolamine, diethanolamine, dipropanolamine, and 1-amino-2-butanol.

Examples of the aromatic hydroxy compound include monophenols such as phenol (carbolic acid), 2-methoxyphenol, cresol, xylenol, carvacrol, thymol, and naphthol; and polyphenols such as catechol, resorcin, hydroquinone, bisphenol A, bisphenol F, pyrogallol, and phloroglucin.

In a case where the aliphatic hydrocarbon group or the aromatic group as $R^{11}$ has 1 or more and 4 or less nitrogen atoms, as $R^{11}$, an organic group obtained by removing a terminal amino group of an amine compound having 1 or more and 4 or less secondary or tertiary amines other than the terminal of the aliphatic hydrocarbon group or the aromatic group. Specific examples of the amine compound having 1 or more and 4 or less secondary or tertiary amines other than the terminal of the aliphatic hydrocarbon group or the aromatic group include 2-(dimethylamino)ethylamine, 2-(diethylamino)ethylamine, 2-(diisopropylamino)ethylamine, 2-(cyclohexylamino)ethylamine, 3-(cyclohexylamino)propylamine, 3-(diethylamino)propylamine, 3-(dimethylamino)propylamine, diethylenetriamine, diisopropyltriamine, bis-(3-aminopropyl)methyleneamine, 3-(2-aminoethylamino)propylamine, N,N'-bis(3-aminopropyl)ethylenediamine, 1-(3-aminopropyl)imidazole, trisaminoethylamine, and trisaminopropylamine. Among these, the amine compound is preferably an amine compound having one or more and two or less tertiary amines other than terminals and having an aliphatic hydrocarbon group or an aromatic group; and more preferably an amine compound having one tertiary amine other than terminal and having an aliphatic hydrocarbon group or an aromatic group.

Among these, $R^{11}$ is preferably a group represented by any one of Formulae (Ia-1) to (Ia-24), and more preferably a group represented by Formula (Ia-1), (Ia-2), (Ia-3), (Ia-14), (Ia-18), or (Ia-19). In each formula, a wavy line represents a bonding site.

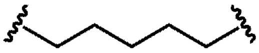
(Ia-1)

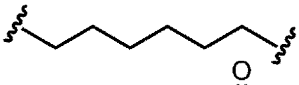
(Ia-2)

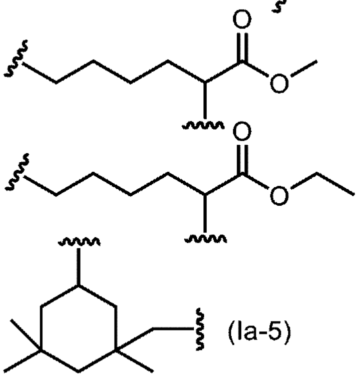
(Ia-3)

(Ia-4)

(Ia-5)

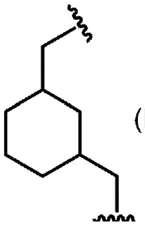
(Ia-6)

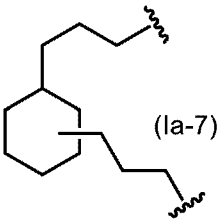
(Ia-7)

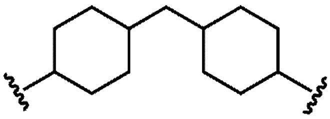
(Ia-8)

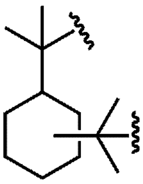
(Ia-9)

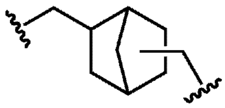
(Ia-10)

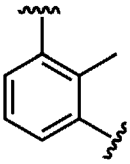
(Ia-11)

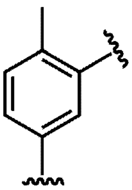
(Ia-12)

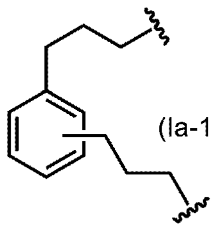
(Ia-13)

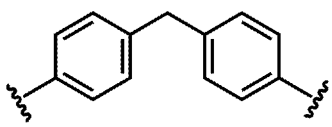
(Ia-14)

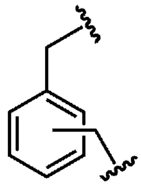
(Ia-15)

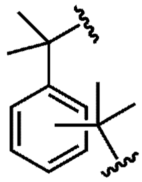
(Ia-16)

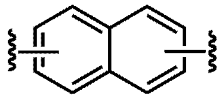
(Ia-17)

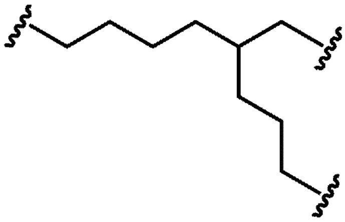
(Ia-18)

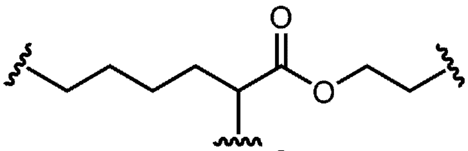
(Ia-19)

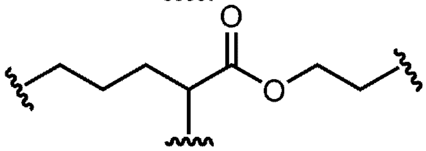
(Ia-20)

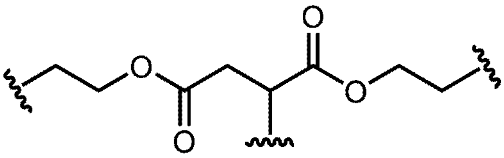
(Ia-21)

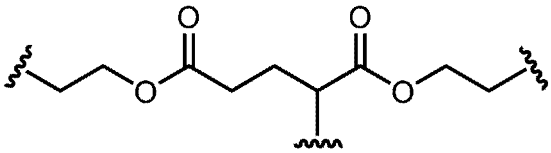
(Ia-22)

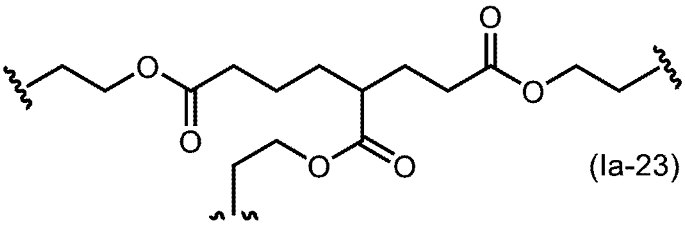
(Ia-23)

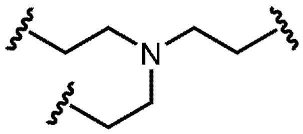

(Ia-24)

[$R^{12}$]

$R^{12}$ is a monovalent organic group, and is preferably an aliphatic hydrocarbon group having 1 or more and 20 or less carbon atoms or an aromatic hydrocarbon group having 6 or more and 20 or less carbon atoms, each of which may include an oxygen atom.

Examples of the aliphatic hydrocarbon group as $R^{12}$ include alkyl groups such as a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), an undecyl group (each isomer), a dodecyl group (each isomer), a tridecyl group (each isomer), a tetradecyl group (each isomer), a pentadecyl group (each isomer), a hexadecyl group (each isomer), a heptadecyl group (each isomer), an octadecyl group (each isomer), a nonadecyl (each isomer), and an eicosyl group (each isomer); and cycloalkyl groups such as a cyclopentyl group, a cyclohexyl group, a cycloheptyl group, a cyclooctyl group, a cyclononyl group, and a cyclodecyl group.

Examples of the aliphatic hydrocarbon group which may include an oxygen atom, as $R^{12}$, include alkoxyalkyl groups such as a methoxymethyl group, a methoxyethyl group (each isomer), a methoxypropyl group (each isomer), a methoxybutyl group (each isomer), a methoxypentyl group (each isomer), a methoxyhexyl group (each isomer), a methoxyheptyl group (each isomer), a methoxyoctyl group (each isomer), a methoxynonyl group (each isomer), a methoxydecyl group (each isomer), a methoxyundecyl group (each isomer), a methoxydodecyl group (each isomer), a methoxytridecyl group (each isomer), a methoxytetradecyl group (each isomer), a methoxypentadecyl group (each isomer), a methoxyhexadecyl group (each isomer), a methoxyheptadecyl group (each isomer), a methoxyoctadecyl group (each isomer), a methoxynonadecyl (each isomer), an ethoxymethyl group, an ethoxyethyl group (each isomer), an ethoxypropyl group (each isomer), an ethoxybutyl group (each isomer), an ethoxypentyl group (each isomer), an ethoxyhexyl group (each isomer), an ethoxyheptyl group (each isomer), an ethoxyoctyl group (each isomer), an ethoxynonyl group (each isomer), an ethoxydecyl group (each isomer), an ethoxyundecyl group (each isomer), an ethoxydodecyl group (each isomer), an ethoxytridecyl group (each isomer), an ethoxytetradecyl group (each isomer), an ethoxypentadecyl group (each isomer), an ethoxyhexadecyl group (each isomer), an ethoxyheptadecyl group (each isomer), an ethoxyoctadecyl group (each isomer), a propyloxymethyl group (each isomer), a propyloxyethyl group (each isomer), a propyloxypropyl group (each isomer), a propoxybutyl group (each isomer), a propoxybutyl group (each isomer), a propoxyhexyl group (each isomer), a propoxyheptyl group (each isomer), a propoxyoctyl group (each isomer), a propoxynonyl group (each isomer), a propoxydecyl group (each isomer), a propoxyundecyl group (each isomer), a propoxydodecyl group (each isomer), a propoxytridecyl group (each isomer), a propoxytetradecyl group (each isomer), a propoxypentadecyl group (each isomer), a propoxyhexadecyl group (each isomer), a propoxyheptadecyl group (each isomer), a butyloxymethyl group (each isomer), a butyloxyethyl group (each isomer), a butyloxypropyl group (each isomer), a butoxybutyl group (each isomer), a butoxybutyl group (each isomer), a butoxyhexyl group (each isomer), a butoxyheptyl group (each isomer), a butoxyoctyl group (each isomer), a butoxynonyl group (each isomer), a butoxydecyl group (each isomer), a butoxyundecyl group (each isomer), a group (each isomer), a butoxypentyl group (each isomer), a butoxyhexyl group (each isomer), a butoxyheptyl group (each isomer), a butoxyoctyl group (each isomer), a butoxynonyl group (each isomer), a butoxydecyl group (each isomer), a butoxyundecyl group (each isomer), a butyloxydodecyl group (each isomer), a butyloxotridecyl group (each isomer), a butyloxotetradecyl group (each isomer), a butyloxopentadecyl group (each isomer), a butyloxhexadecyl group (each isomer), a pentyloxymethyl group (each isomer), a pentyloxyethyl group (each isomer), a pentyloxopropyl group (each isomer), a pentyloxybutyl group (each isomer), a pentyloxypentyl group (each isomer), a pentyloxyhexyl group (each isomer), a pentyloxyheptyl group (each isomer), a pentyloxyoctyl group (each isomer), a pentyloxynonyl group (each isomer), a pentyloxodecyl group (each isomer), a pentyloxundecyl group (each isomer), a pentyloxydodecyl group (each isomer), a pentyloxotridecyl group (each isomer), a pentyloxotetradecyl group (each isomer), a pentyloxopentadecyl group (each isomer), a hexyloxymethyl group (each isomer), a hexyloxyethyl group (each isomer), a hexyloxopropyl group (each isomer), a hexyloxybutyl group (each isomer), a hexyloxybutyl group (each isomer), a hexyloxy pentyl group (each isomer), a hexyloxy hexyl group (each isomer), a hexyloxy heptyl group (each isomer), a hexyloxy octyl group (each isomer), a hexyloxy nonyl group (each isomer), a hexyloxy decyl group (each isomer), a hexyloxy undecyl group (each isomer), a hexyloxy dodecyl group (each isomer), a hexyloxy tridecyl group (each isomer), a hexyloxy tetradecyl group (each isomer), a heptyloxy methyl group (each isomer), a heptyloxy ethyl group (each isomer), a heptyloxy propyl group (each isomer), a heptyloxy butyl group (each isomer), a heptyloxy pentyl group (each isomer), a heptyloxy hexyl group (each isomer), a heptyloxy heptyl group (each isomer), a heptyloxy

octyl group (each isomer), a heptyloxy nonyl group (each isomer), a heptyloxy decyl group (each isomer), a heptyloxy undecyl group (each isomer), a heptyloxy dodecyl group (each isomer), an octyloxy methyl group (each isomer), an octyloxy ethyl group (each isomer), an octyloxy propyl group (each isomer), an octyloxy butyl group (each isomer), an octyloxy pentyl group (each isomer), an octyloxy hexyl group (each isomer), an octyloxy heptyl group (each isomer), an octyloxy octyl group (each isomer), an octyloxy nonyl group (each isomer), an octyloxy decyl group (each isomer), an octyloxy undecyl group (each isomer), an octyloxy dodecyl group (each isomer), an oxypentyl group (each isomer), an octyloxy hexyl group (each isomer), an octyloxy heptyl group (each isomer), an octyloxy octyl group (each isomer), an octyloxy nonyl group (each isomer), an octyloxy decyl group (each isomer), an octyloxy undecyl group (each isomer), an octyloxy dodecyl group (each isomer), a nonyloxymethyl group (each isomer), a nonyloxyethyl group (each isomer), a nonyloxypropyl group (each isomer), a nonyloxybutyl group (each isomer), a nonyloxypentyl group (each isomer), a nonyloxyhexyl group (each isomer), a nonyloxyheptyl group (each isomer), a nonyloxyoctyl group (each isomer), a nonyloxynonyl group (each isomer), a nonyloxydecyl group (each isomer), a nonyloxyundecyl group (each isomer), a decyloxymethyl group (each isomer), a decyloxyethyl group (each isomer), a decyloxypropyl group (each isomer), a decyloxybutyl group (each isomer), a decyloxypentyl group (each isomer), a decyloxyhexyl group (each isomer), a decyloxyheptyl group (each isomer), a decyloxyoctyl group (each isomer), a decyloxynonyl group (each isomer), a decyloxydecyl group (each isomer), an undecyloxymethyl group (each isomer), an undecyloxyethyl group (each isomer), an undecyloxypropyl group (each isomer), an undecyloxybutyl group (each isomer), an undecyloxypentyl groups (each isomer), an undecyloxyhexyl group (each isomer), an undecyloxyheptyl group (each isomer), an undecyloxyoctyl group (each isomer), an undecyloxynonyl group (each isomer), a dodecyloxymethyl group (each isomer), a dodecyloxyethyl group (each isomer), a dodecyloxypropyl group (each isomer), a dodecyloxybutyl group (each isomer), a dodecyloxypentyl group (each isomer), a dodecyloxyhexyl group (each isomer), a dodecyloxyheptyl group (each isomer), a dodecyldecyloxyoctyl group (each isomer), a tridecyloxymethyl group (each isomer), a tridecyloxyethyl group (each isomer), a tridecyloxypropyl group (each isomer), a tridecyloxybutyl group (each isomer), a tridecyloxypentyl group (each isomer), a tridecyloxyhexyl group (each isomer), a tridecyloxyheptyl group (each isomer), a tetradecyloxymethyl group (each isomer), a tetradecyloxyethyl group (each isomer), a tetradecyloxypropyl group (each isomer), a tetradecyloxybutyl group (each isomer), a tetradecyloxypentyl group (each isomer), a tetradecyloxyhexyl group (each isomer), a pentadecyloxymethyl group, a pentadecyloxyethyl group (each isomer), a pentadecyloxypropyl group (each isomer), a pentadecyloxybutyl group (each isomer), a pentadecyloxypentyl group (each isomer), a hexadecyloxymethyl group (each isomer), a hexadecyloxyethyl group (each isomer), a hexadecyloxypropyl group (each isomer), a hexadecyloxybutyl group (each isomer), a heptadecyloxymethyl group (each isomer), a heptadecyloxyethyl group (each isomer), a heptadecyloxypropyl group (each isomer), an octadecyloxymethyl group (each isomer), and an octadecyloxyethyl groups (each isomer).

Examples of the aromatic hydrocarbon group as $R^{12}$ include aryl groups such as a phenyl group, a naphthyl group, an anthryl group, a pyrenyl group, a phenanthryl group; and aryl groups having an alkyl group as a substituent, such as a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each

isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), and a dibutylhexylphenyl group (each isomer).

Examples of the aromatic hydrocarbon group which may include an oxygen atom, as $R^{12}$, include an alkoxyaryl group such as a methoxyphenyl group (each isomer) and an ethoxyphenyl group (each isomer).

Among these, as $R^{12}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

[n11 and n12]

n11 is an integer of 1 or more and 8 or less.

n12 represents the number of isocyanate groups, and is an integer of 0 or more and 7 or less.

The sum of n11 and n12 (n11 + n12) is an integer of 2 or more and 8 or less, preferably an integer of 2 or more and 6 or less, more preferably an integer of 2 or more and 5 or less, and still more preferably an integer of 3 or more

and 4 or less. In general, as the value of (n11 + n 12) increases, the boiling point increases in association with an increase in the molecular weight of the carbonyl compound (I), and the separation from the isocyanate becomes easier. On the other hand, in the production of the carbonyl compound (I), in a case where a highly reactive isocyanate group is heated, the modification reaction is invoked, which causes fixation and sticking to the device. Therefore, from the viewpoint of the production efficiency of the carbonyl compound (I), (n11 + n12) is preferably 6 or less, more preferably 5 or less, and still more preferably 4 or less.

Examples of the preferred carbonyl compound (I) include compounds represented by Formulae (I-1a) to (1-6) (hereinafter, may be referred to as "carbonyl compound (I-1a)" and the like). The carbonyl compounds (I-1a) to (I-1c), the carbonyl compounds (I-3a) to (I-3c), and the carbonyl compounds (I-4a) and (I-4b) may be used as a mixture, or may be used as individual compounds.

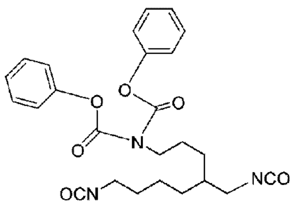

( I - 1a)

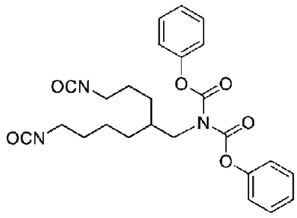

( I - 1b)

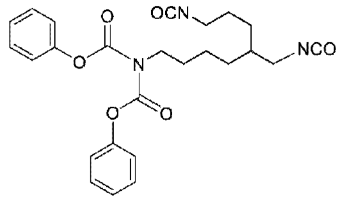

( I - 1c)

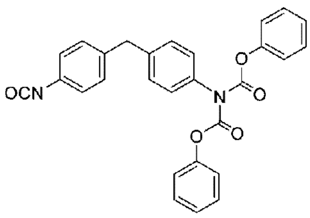

( I -2 )

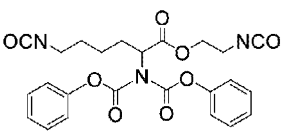

( I - 3a)

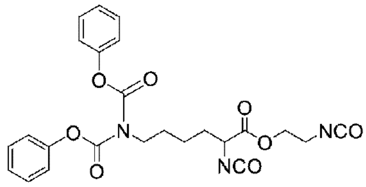

( I - 3b)

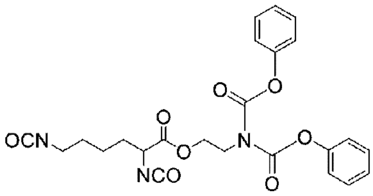

( I - 3c)

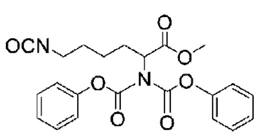

( I - 4a)

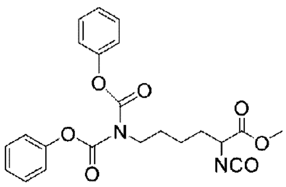

( I - 4b)

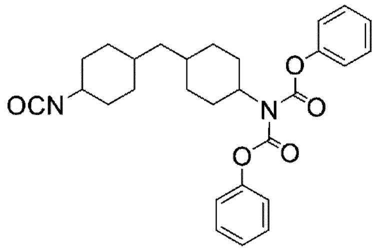

(I - 5)

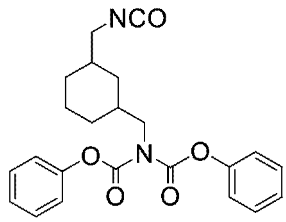

(I - 6)

<Isocyanate compound>

As the isocyanate compound, for example, a compound represented by General Formula (VIIb) (hereinafter, may be referred to as "isocyanate compound (VIIb)") is preferably used.

$$R^{71b}\left(NCO\right)_{n71b} \qquad (VIIb)$$

(in General Formula (VIIb), $R^{71b}$ is an n71b-valent organic group, and a relational expression of $R^{71b} = R^{11}$ is satisfied, and n71b is an integer of 2 or more and 8 or less, and a relational expression of n71b = n11 + n12 is satisfied)

[$R^{71b}$]

$R^{71b}$ is an n71b-valent organic group, and a relational expression of $R^{71b} = R^{11}$ is satisfied. That is, $R^{71b}$ is the same as $R^{11}$ described above.

In a case where $R^{71b}$ is an aliphatic hydrocarbon group, specific examples of the isocyanate compound (VIIb) include aliphatic diisocyanates, aliphatic triisocyanates, and substituted cyclic aliphatic polyisocyanates.

Examples of the aliphatic diisocyanates include diisocyanatoethane, diisocyanatopropane (each isomer), diisocyanatobutane (each isomer), diisocyanatopentane (each isomer), diisocyanatohexane (each isomer), and diisocyanatodecane (each isomer).

Examples of the aliphatic triisocyanates include triisocyanatohexane (each isomer), 4-isocyanatomethyl-1,8-octamethylenediisocyanate, triisocyanatononane (each isomer), and triisocyanatodecane (each isomer).

Examples of the substituted cyclic aliphatic polyisocyanates include diisocyanatocyclobutane (each isomer), diisocyanatocyclohexane (each isomer), 3-isocyanatomethyl-3,5,5-trimethylcyclohexylisocyanate (also referred to as isophorone diisocyanate) (each isomer), 1,3-bis(isocyanatomethyl)cyclohexane (at least one isomer of a cis form and a trans form), and methylenebis(cyclohexylisocyanate) (also referred to as dicyclohexylmethane diisocyanate) (each isomer).

In a case where $R^{71b}$ is an aromatic group, specific examples of the isocyanate compound (VIIb) include aromatic diisocyanates and aromatic triisocyanates.

Examples of the aromatic diisocyanates include diisocyanatobenzene (each isomer), diisocyanatotoluene (each isomer), bis(isocyanatophenyl)methane (each isomer), diisocyanatomesitylene (each isomer), diisocyanatobiphenyl (each isomer), diisocyanatodibenzyl (each isomer), bis(isocyanatophenyl)propane (each isomer), bis(isocyanatophenyl)ether (each isomer), bis(isocyanatophenoxyethane) (each isomer), diisocyanatoxylene (each isomer), diisocyanatoanisole (each isomer), diisocyanatophenethol (each isomer), diisocyanatonaphthalene (each isomer), diisocyanatomethylbenzene (each isomer), diisocyanatomethylpyridine (each isomer), diisocyanatomethylnaphthalene (each isomer), diisocyanatodiphenylmethane (each isomer), and tetramethylxylylene diisocyanate (each isomer).

Examples of the aromatic triisocyanates include triisocyanatobenzene (each isomer), triisocyanato-methylbenzene (each isomer), tris(isocyanatopropan-yl)benzene (each isomer), tris(isocyanatopropan-yl)-methylbenzene (each isomer), tris(isocyanatomethyl)-methylbenzene (each isomer), and ((isocyanato-phenylene)bis(methylene))bis(isocyanatebenzene) (each isomer).

In a case where $R^{71b}$ is an aromatic group, reactivity of the isocyanate group directly bonded to the aromatic group is increased by an electron-withdrawing effect of the aromatic group. Furthermore, the reactivity is further enhanced by mutual electron-withdrawing action as the number of isocyanate groups directly bonded to the same aromatic compound increases. From the viewpoint of reactivity, the isocyanate group with respect to $R^{71b}$ is preferably 2 or more and more preferably 3 or more. On the other hand, in a case where the reactivity of the isocyanate is high, a reaction with water, between isocyanates, and with other impurities is caused, and the stability of the compound at room temperature and during heating is low. From the viewpoint of stability of the isocyanate compound, the number of isocyanate groups directly bonded to $R^{71b}$ is preferably 4 or less, and more preferably 3 or less.

Specific examples of the isocyanate compound (VIIb) in a case where the aliphatic hydrocarbon group or the aromatic group as $R^{71b}$ has 1 or more and 4 or less ester groups or a nitrogen atom include 2-isocyanatoethyl acrylate, 2-isocyanatoethyl 2-methylacrylate, 2-isocyanatopropyl acrylate, 2-isocyanatopropyl 2-methylacrylate, 3-isocyanatopropyl acrylate, 3-isocyanatopropyl 2-methylacrylate, 4-isocyanatobutyl acrylate, 4-isocyanatobutyl 2-methylacrylate, 5-isocyanatopentyl acrylate, 5-isocyanatopentyl 2-methylacrylate,acrylic acid-6-isocyanato-hexyl ester, 2-methyl-acrylic acid-6-isocyanato-hexyl ester, acrylic acid-8-isocyanato-octyl ester, 2-methyl-acrylic acid-8-isocyanato-octyl ester, acrylic acid-10-isocyanato-decyl ester, 2-methyl-acrylic acid-10-isocyanato-decyl ester, acrylic acid-11-isocyanato-undecyl ester, 2-methyl-acrylic acid-11-isocyanato-undecyl ester, acrylic acid-12-isocyanato-dodecyl ester, 2-methyl-acrylic acid-12-isocyanato-dodecyl ester, lysine methyl ester diisocyanate, lysine ethyl ester diisocyanate, 2-isocyanatoethyl-2,5-diisocyanatopentanoate, 2-isocyanatoethyl-2,6-diisocyanatohexanoate (lysine triisocyanate), bis(2-isocyanatoethyl)-2-isocyanatobutanedioate, bis(2-isocyanatoethyl)-2-isocyanatopentanedioate, tris(2-isocyanatoethyl) hexane-1,3,6-tricarboxylate, trisisocyanatethylamine, and trisisocyanatopropylamine.

In a case where the isocyanate compound has an electron-withdrawing group such as an ester group and an N atom, the reactivity of the isocyanate group is improved. The smaller the number of carbon atoms between the isocyanate group and the ester group or the nitrogen atom, the more the reactivity of the isocyanate group is improved. Therefore, the number of carbon atoms is preferably 1 or more and 20 or less, more preferably 1 or more and 8 or less, and still more preferably 1 or more and 3 or less. On the other hand, the carbon-carbon or carbon-nitrogen bond between the isocyanate group, which is an electron-withdrawing group, and the ester group or the nitrogen atom is likely to be dissociated, and the thermal stability of the compound may be impaired. Therefore, from the viewpoint of the stability of the compound in which the isocyanate group and the ester group or the nitrogen atom coexist, the larger the number of carbon atoms between the isocyanate group and the ester group or the nitrogen atom, the more the stability of the compound is improved. Therefore, the number of carbon atoms is preferably 1 or more, more preferably 2 or more, and still more preferably 3 or more.

The isocyanate compound (Vllb) is often used as a raw material for a coating material, and thus a property (weather resistance) of not being colored when exposed to light such as sunlight is required. In a case where $R^{71b}$ has an aromatic group, the aromatic group may absorb light energy when exposed to sunlight, thereby causing coloring. From this, from the viewpoint of weather resistance, $R^{71b}$ is preferably a group having no aromatic group and more preferably a di- or higher and tetra- or lower valent aliphatic hydrocarbon group having 1 or more and 20 or less carbon atoms, which may have 1 or more and 4 or less ester groups or a nitrogen atom.

In addition, as $R^{71b}$, specifically, a group represented by any one of Formulae (Ia-1) to (Ia-24) is preferable, and a group represented by Formula (Ia-1), (Ia-2), (Ia-3), (Ia-14), (Ia-1 8), or (Ia-19) is more preferable.

(n71b)

n71b represents the number of isocyanate groups, and a relational expression of n71b = n11 + n12 is satisfied. n71b is an integer of 2 or more and 8 or less, preferably an integer of 2 or more and 6 or less, more preferably an integer of 2 or more and 5 or less, and still more preferably an integer of 3 or more and 4 or less. In general, a polymer can be obtained by reacting an isocyanate having an n71b or more valance with a dihydroxy compound or a diamine compound having an active hydrogen group. As the value of n71b increases, the number of crosslinking points (isocyanate groups) per isocyanate molecule increases. Therefore, the crosslinking density at the time of polymerization increases, and it is possible to shorten the curing time and improve the hardness of the polymer. The expression "the crosslinking density increases" used herein means that the average molecular chain length between the crosslinking points decreases. In a case where the number of isocyanate groups in the isocyanate molecule is 3 or more (n71b is 3 or more), since the linear polymer and the polymer can be further bonded to each other and the molecular weight of the polymer is likely to increase, it is possible to expect a dramatic reduction in the curing time and a dramatic improvement in the physical properties of the polymer such as the hardness. On the other hand, in the production of isocyanate, when a highly reactive isocyanate compound is heated, a modification reaction is invoked, which causes the isocyanate compound to be fixed to the device or to be stuck. Therefore, the valence n71b of the isocyanate group in the isocyanate compound is preferably 6 or less, more preferably 5 or less, and still more preferably 4 or less.

Examples of the preferred isocyanate compound (VIIb) include 4-isocyanatomethyl-1,8-octamethylenediisocyanate (TTI), 2-isocyanatoethyl-2,6-diisocyanatohexanoate (LTI), lysine methyl ester diisocyanate (LDI), methylene bis(cyclohexyl isocyanate) (HMDI), 1,3-bis(isocyanatomethyl)cyclohexane (HXDI), and diisocyanatodiphenylmethane (MDI).

<Modified product of isocyanate compound>

The modified product is a compound derived from the above-described isocyanate compound, and is a compound in which a part of an isocyanate group of the isocyanate compound is converted into one or more functional groups selected from the group consisting of an isocyanurate group, a carbodiimide group, a uretonimine group, and an allophanate group.

The isocyanurate group is a structure in which an isocyanate group is trimerized, and is a group represented by General Formula (X-1).

The carbodiimide group is a structure in which an isocyanate group is dimerized, and is a group represented by General Formula (X-2).

The uretonimine group is a structure in which an isocyanate group is added to a carbodiimide group, and is a group represented by General Formula (X-3).

The allophanate group is a structure in which an isocyanate group is added to a carbamate group, and is a group represented by General Formula (X-4).

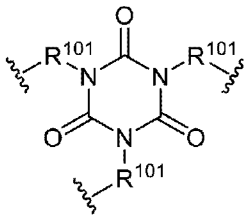
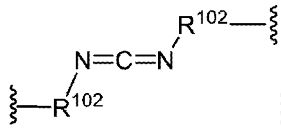
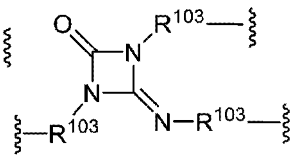
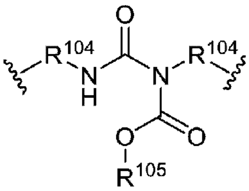

(X-1) (X-2) (X-3) (X-4)

(in General Formulae (X-1) to (X-4), $R^{101}$, $R^{102}$, $R^{103}$, and $R^{104}$ are each independently a di- or higher and octa- or lower valent organic group, and relational expressions of $R^{101} = R^{71b}$, $R^{102} = R^{71b}$, $R^{103} = R^{71b}$, and $R^{104} = R^{71b}$ are satisfied, $R^{105}$ is a monovalent organic group, and a relational expression of $R^{105} = R^{12}$ is satisfied, and a wavy line represents a bonding site)

[$R^{101}$, $R^{102}$, $R^{103}$, and $R^{104}$]

$R^{101}$, $R^{102}$, $R^{103}$, and $R^{104}$ are each independently a di- or higher and octa- or lower valent organic group, and relational expressions of $R^{101} = R^{71b}$, $R^{102} = R^{71b}$, $R^{103} = R^{71b}$, and $R^{104} = R^{71b}$ are satisfied. That is, $R^{101}$, $R^{102}$, $R^{103}$, and $R^{104}$ are the same as $R^{71b}$ described above.

Among these, $R^{101}$, $R^{102}$, $R^{103}$, and $R^{104}$ are each independently preferably a group having no aromatic group and more preferably a di- or higher and tetra- or lower valent aliphatic hydrocarbon group having 1 or more and 20 or less carbon atoms, which may have 1 or more and 4 or less ester groups or a nitrogen atom.

In addition, as each of $R^{101}$, $R^{102}$, $R^{103}$, and $R^{104}$, specifically, a group represented by any one of Formulae (Ia-1) to (Ia-24) is preferable, and a group represented by Formula (Ia-1), (Ia-2), (Ia-3), (Ia-14), (Ia-18), or (Ia-19) is more preferable.

[$R^{105}$]

$R^{105}$ is a monovalent organic group, and a relational expression of $R^{105} = R^{12}$ is satisfied. That is, $R^{105}$ is the same as $R^{12}$ described above.

Among these, $R^{105}$ is preferably an aliphatic hydrocarbon group having 1 or more and 20 or less carbon atoms or an aromatic hydrocarbon group having 6 or more and 20 or less carbon atoms, which may include an oxygen atom.

In addition, as $R^{105}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each

isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a phenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

It is preferable that the isocyanate composition according to the present embodiment further contains one or more compounds selected from the group consisting of a carbamate group-containing compound represented by General Formula (XI) and having a content of 2.0 ppm by mass or more and $1.0 \times 10^4$ ppm by mass or less with respect to the total mass of the isocyanate composition (hereinafter, may be referred to as "carbamate group-containing compound (XI)"), and a carbonic acid ester represented by General Formula (III-1a) and having a content of 2.0 ppm by mass or more and $1 .0 \times 10^4$ ppm by mass or less with respect to the total mass of the isocyanate composition (hereinafter, may be referred to as "carbonic acid ester (III-1a)").

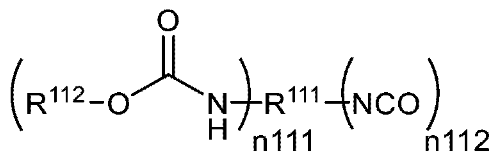

(XI)

(in General Formula (XI), $R^{111}$ is an (n111 + n112)-valent organic group, and a relational expression of $R^{111} = R^{11}$ is satisfied, $R^{112}$ is a monovalent organic group, and a relational expression of $R^{112} = R^{12}$ is satisfied, n111 is an integer of 1 or more and 8 or less, n112 is an integer of 0 or more and 7 or less, a sum of n111 and n112 is an integer of 2 or more and 8 or less, and a relational expression of n111 + n112 = n11 + n12 is satisfied)

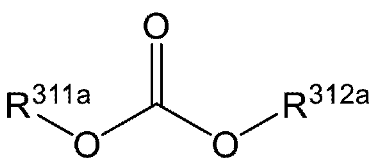

(III-1a)

(in General Formula (111-1a), $R^{311a}$ and $R^{312a}$ are each independently a monovalent organic group, and a relational expression of $R^{311a} = R^{312a} = R^{12}$ is satisfied)

The carbamate group-containing compound (XI) and the carbonic acid ester (III-1a) also exhibit the same effects as those of the above-described carbonyl compound (I). In order to suppress the modification reaction of the isocyanate compound, it is preferable that the content of these compounds in the isocyanate composition is large, but it is preferable that the content is not too large in consideration of the suppression of coloring of the composition. Therefore, the lower limit value of the content of each of the carbamate group-containing compound (XI) and the carbonic acid ester (III-1a) is preferably 2.0 ppm by mass, more preferably 3.0 ppm by mass, still more preferably 5.0 ppm by mass, and particularly preferably 10 ppm by mass, with respect to the total mass of the isocyanate composition. On the other hand, the upper limit value of the content of each of the carbamate group-containing compound (XI) and the carbonic acid ester (III-1a) is preferably 99% by mass, more preferably 70% by mass, still more preferably 10% by mass, even more preferably 1% by mass ($1.0 \times 10^4$ ppm by mass), particularly preferably $3.0 \times 10^3$ ppm by mass, and most preferably $1.0 \times 10^3$ ppm by mass with respect to the total mass of the isocyanate composition.

That is, the content of each of the carbamate group-containing compound (XI) and the carbonic acid ester (III-1a) is preferably 2.0 ppm by mass or more and 99% by mass or less, more preferably 2.0 ppm by mass or more and 70% by mass or less, still more preferably 2.0 ppm by mass or more and 10% by mass or less, even more preferably 2.0 ppm by mass or more and $1.0 \times 10^4$ ppm by mass or less, even still more preferably 3.0 ppm by mass or more and $3.0 \times 10^3$ ppm by mass or less, particularly preferably 5.0 ppm by mass or more and $1.0 \times 10^3$ ppm by mass or less, and most preferably 10 ppm by mass or more and $1.0 \times 10^3$ ppm by mass or less with respect to the total mass of the isocyanate composition.

In a case where the content of each of the carbamate group-containing compound (XI) and the carbonic acid ester (III-1a) is equal to or more than the above-described lower limit value, the modification reaction of the isocyanate compound can be further suppressed. On the other hand, in a case of being equal to or less than the above-described upper limit value, the coloring of the composition caused by the unsaturated bond can be further suppressed.

<Carbamate group-containing compound (XI)>

The carbamate group-containing compound is a compound represented by General Formula (XI).

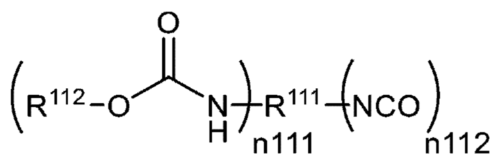

(XI)

(in General Formula (XI), $R^{111}$ is an (n111 + n112)-valent organic group, and a relational expression of $R^{111} = R^{11}$ is satisfied, $R^{112}$ is a monovalent organic group, and a relational expression of $R^{112} = R^{12}$ is satisfied, n 111 is an integer of 1 or more and 8 or less, n112 is an integer of 0 or more and 7 or less, a sum of n111 and n112 is an integer of 2 or more and 8 or less, and a relational expression of n111 + n112 = n11 + n12 is satisfied)

[$R^{111}$]

$R^{111}$ is an (n111 + n112)-valent organic group, and a relational expression of $R^{111} = R^{11}$ is satisfied. That is, $R^{111}$ is the same as $R^{11}$ described above.

Among these, $R^{111}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

In addition, as $R^{111}$, specifically, a group represented by any one of Formulae (Ia-1) to (Ia-24) is preferable, and a group represented by Formula (Ia-1), (Ia-2), (Ia-3), (Ia-14), (Ia-18), or (Ia-19) is more preferable.

[$R^{112}$]

$R^{112}$ is a monovalent organic group, and a relational expression of $R^{112} = R^{12}$ is satisfied. That is, $R^{112}$ is the same as $R^{12}$ described above.

Among these, $R^{112}$ is preferably an aliphatic hydrocarbon group having 1 or more and 20 or less carbon atoms or an aromatic hydrocarbon group having 6 or more and 20 or less carbon atoms, which may include an oxygen atom.

In addition, as $R^{112}$, specifically, a phenyl group, a naphthyl group, an anthryl group, a pyrenyl group, a phenanthryl group, a methylphenyl group (each isomer), an ethylphenyl group (each isomer), a propylphenyl group (each

isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

[n111 and n112]

n111 is an integer of 1 or more and 8 or less.
n112 is an integer of 0 or more and 7 or less.
The sum of n111 and n112 is an integer of 2 or more and 8 or less, and a relational expression of n111 + n112 = n11 + n12 is satisfied.
Examples of the preferred carbamate compound (XI) include compounds represented by Formulae (XI-1a) to (XI-6).

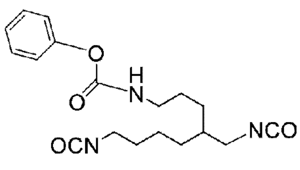
( XI - 1a)

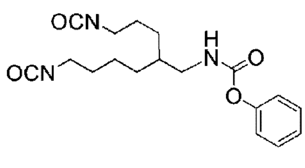
( XI - 1b)

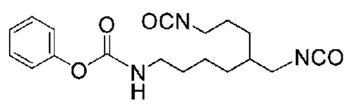
( XI - 1c)

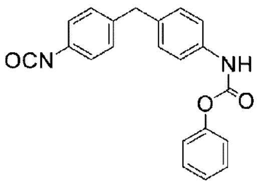

( XI -2 )

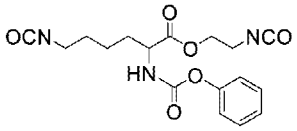

( XI - 3a)

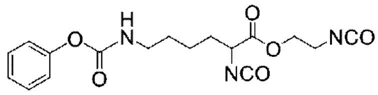

( XI - 3b)

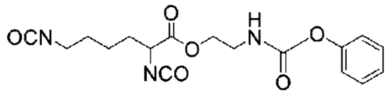

( XI - 3c)

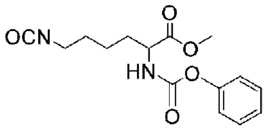

( XI - 4a)

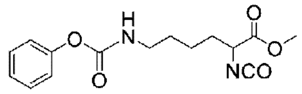

( XI - 4b)

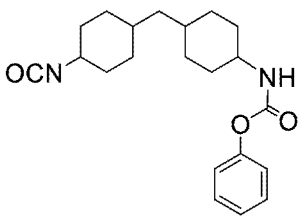

( XI - 5)

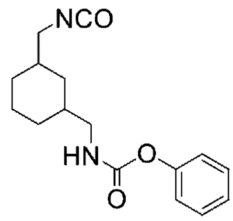

( XI - 6)

<Carbonic acid ester (III-1a)>

The carbonic acid ester is a compound represented by General Formula (III-1a).

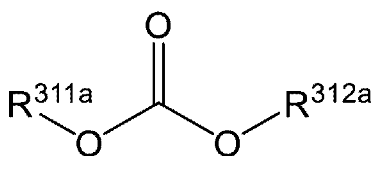

( III-1a )

(in General Formula (111-1a), $R^{311a}$ and $R^{312a}$ are each independently a monovalent organic group, and a relational expression of $R^{311a} = R^{312a} = R^{12}$ is satisfied)

[$R^{311a}$ and $R^{312a}$]

$R^{311a}$ and $R^{312a}$ are the same as each other, and a relational expression of $R^{311a} = R^{312a} = R^{12}$ is satisfied.
That is, examples of $R^{311a}$ and $R^{312a}$ include the same groups as those described in $R^{12}$ above.
Among the above, $R^{311a}$ and $R^{312a}$ are preferably a substituted or unsubstituted aryl group.
In addition, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each

isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methylnonylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is more preferable.

In addition, a phenyl group, a methoxyphenyl group (each isomer), or an ethoxyphenyl group (each isomer) is still more preferable.

Examples of the preferred carbonic acid ester (III-1a) include diphenyl carbonate, bis(2-methoxyphenyl) carbonate, and bis(2-ethoxyphenyl) carbonate.

<Method for producing isocyanate composition>

The isocyanate composition of the present embodiment is obtained by performing a thermal decomposition reaction of a carbamate compound represented by General Formula (Vd) (hereinafter, may be referred to as a "carbamate compound (Vd)") in the presence of the above-described carbonic acid ester (III-1a) as a solvent (thermal decomposition step), or performing a distillation of a component having a boiling point lower than that of the isocyanate compound (hereinafter, may be referred to as a "low-boiling component") after performing the thermal decomposition reaction of the carbamate compound (Vd) in the presence of the above-described carbonic acid ester (III-1a) as a solvent (thermal decomposition step) (low-boiling separation step).

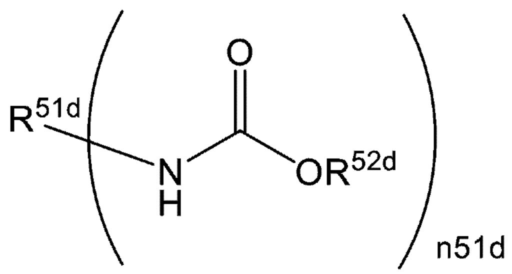

(Vd)

(in General Formula (Vd), $R^{51d}$ is an n51d- or higher valent organic group, and a relational expression of $R^{51d} = R^{71h}$ is satisfied, $R^{52d}$ is a monovalent organic group, and a relational expression of $R^{52d} = R^{12}$ is satisfied, and n51b is an integer of 2 or more and 8 or less, and a relational expression of n51b = n71b is satisfied)

[Thermal decomposition step]

In the thermal decomposition step, the thermal decomposition reaction of the carbamate compound (Vd) is carried out in the presence of the carbonic acid ester (III-1a) as a solvent to obtain an isocyanate composition containing an isocyanate compound, a carbonyl compound (I), and a modified product of the isocyanate compound.

As described above, the modified product of the carbonyl compound (I) and the isocyanate compound is produced as a by-product of the thermal decomposition reaction of the carbamate compound (Vd). The carbonyl compound (I) is produced by a reaction of one or more compounds selected from the group consisting of an isocyanate compound and a carbamate compound with one or more compounds selected from the group consisting of a carbonic acid ester and a hydroxy compound. In the modified product of the isocyanate compound, the isocyanurate group, the carbodiimide group, the uretonimine group, and the allophanate group are generated by the reaction between the isocyanate compounds, the reaction between the carbamate compounds (Vd), or the reaction between the isocyanate compound and the carbamate compound (Vd). Therefore, in the thermal decomposition step, the use amount of the carbonic acid ester (III-1a) is increased, or the thermal decomposition reaction is carried out under reflux conditions to prevent the evaporation of the excess carbonic acid ester (111-1a), whereby the production ratio of the carbonyl compound (I) can be relatively increased.

It is presumed that the carbonyl compound (I) is generated by a reaction mechanism represented by Formula (F), (G), or (H).

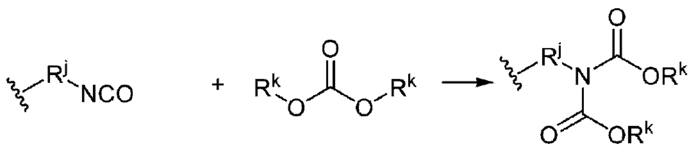
(F)

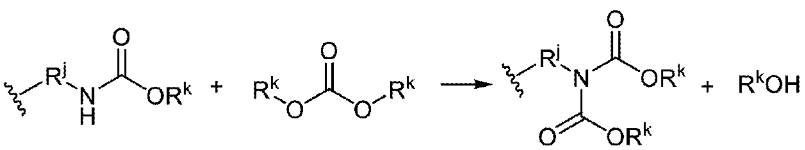
(G)

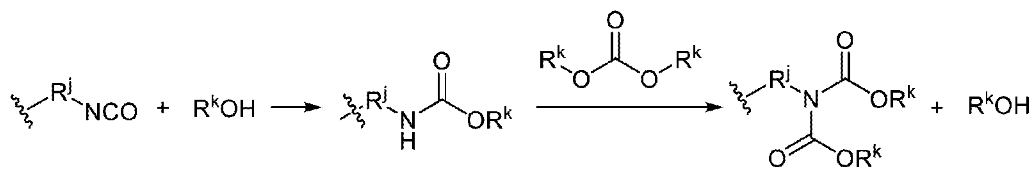
(H)

(in Formulae (F) to (H), $R^j$ is a di- or higher valent organic group, and $R^k$ is a monovalent organic group)

In the thermal decomposition step, a side reaction represented by Formulae (B) to (E) described above occurs, and the isocyanurate group, the carbodiimide group, and the allophanate group generated act as crosslinking points, whereby a high-molecular-weight component, that is, a modified product of the isocyanate compound is generated, and the purification of the solid matter or an increase in the liquid viscosity may occur. However, the carbonyl compound (I), which is a by-product as well as the modified product of the isocyanate compound, is present in the thermal decomposition reaction system. Therefore, the carbonyl compound (I) acts as a good solvent for the modified product of the isocyanate compound, and the carbonyl compound (I) acts as a terminal sealing agent. As a result, the effect of suppressing the generation of a solid material and an increase in the liquid viscosity in the thermal decomposition reaction is obtained by suppressing the polymerization of the modified product of the isocyanate compound.

The carbonic acid ester (III-1a) may be supplied to the reactor before the reaction starts, may be supplied during the reaction, or both may be supplied. Among these, it is preferable that the catalyst is supplied to the reactor before the reaction.

In the thermal decomposition step, the amount of the carbonic acid ester (III-1a) used as a solvent (molar amount) is preferably large from the viewpoint of suppressing side reactions, but in consideration of the size of the reactor and the like, the amount thereof is preferably 0.001 times or more and 100 times or less, more preferably 0.01

times or more and 80 times or less, and still more preferably 0.1 times or more and 50 times or less in terms of a stoichiometric ratio, with respect to the carbamate compound.

When the carbamate compound contains the carbonic acid ester (111-1a), in a case where the carbamate compound is produced by a method to be described below, the carbonic acid ester (111-1a) may be used as it is, or the carbonic acid ester (III-1a) may be newly added to the carbamate compound (Vd).

The reaction temperature is usually 100°C or higher and 400°C or lower, and a high temperature is preferable in order to increase the reaction rate. On the other hand, at a high temperature, side reactions as described above may be caused by one or more compounds selected from the group consisting of the carbamate compound and the isocyanate compound, which is a product, so that the reaction temperature is preferably 130°C or higher and 300°C or lower and more preferably 150°C or higher and 280°C or lower. In order to keep the reaction temperature constant, a known cooling device and a heating device may be installed in the reactor.

In addition, the reaction pressure varies depending on the kinds of the compounds to be used and the reaction temperature, but may be any of a reduced pressure, a normal pressure, and a pressurized pressure, and is usually in a range of 20 Pa or more and $1 \times 10^6$ Pa or less.

The reaction time (in a case of the continuous method, the retention time) is not particularly limited, and is usually 0.001 hours or more and 100 hours or less, preferably 0.01 hours or more and 50 hours or less and more preferably 0.1 hours or more and 10 hours or less.

A catalyst can be used, and the amount of the catalyst used is preferably 0.01 % by mass or more and 30% by mass or less, and more preferably 0.5% by mass or more and 20% by mass or less with respect to the mass of the carbamate compound.

Examples of the catalyst include organic metal catalysts such as dibutyltin dilaurate, lead octylate, and stannous octoate; and amines such as 1,4-diazabicyclo[2,2,2]octane, triethylenediamine, and triethylamine. Among these, organic metal catalysts such as dibutyltin dilaurate, lead octylate, and stannooctate are suitable. These compounds may be used alone or as a mixture of two or more kinds thereof.

As described above, the thermal decomposition reaction is a reaction in which a carbamate compound (Vd) is converted into a corresponding isocyanate compound (VIIb) and a hydroxy compound, but the thermal decomposition reaction is an equilibrium reaction. Therefore, in order to efficiently obtain the isocyanate compound (VIIb) in the thermal decomposition reaction, it is preferable to extract the hydroxy compound, which is a product in the thermal decomposition reaction, as a gas-phase component from the system of the thermal decomposition reaction by, for example, a distillation method.

[Low-boiling separation step]

After the thermal decomposition step, the low-boiling component may be distilled off, and the distillation method is not particularly limited as long as the low-boiling component can be separated as a gas-phase component. The term "low-boiling point component (low-boiling point component)" referred to herein is a component having a boiling point lower than that of the isocyanate compound, and is a compound that varies depending on the type of the compound that is used as a raw material for producing the isocyanate composition, but is mainly one or more compounds selected from the group consisting of a carbonic acid ester and a hydroxy compound used in the thermal decomposition step.

The pressure at the time of distilling off the low-boiling component varies depending on the type of the compound and the reaction temperature, but may be any of a reduced pressure, a normal pressure, and a pressurization within a range in which the separation of the carbonyl compound and the low-boiling component is possible, and it is preferably 20 Pa or more and $1 \times 10^6$ Pa or less, more preferably 20 Pa or more and $1 \times 10^4$ Pa or less, still more preferably 20 Pa or more and $1 \times 10^3$ Pa or less, and particularly preferably 20 Pa or more and $1 \times 10^2$ Pa or less.

The operation time (in the case of the continuous method, the retention time) when the low-boiling component is distilled off is not particularly limited as long as the carbonyl compound and the low-boiling component can be separated, and from the viewpoint of suppressing a side reaction with the carbonyl compound, the operation time is preferably 5 seconds or longer and 100 hours or shorter, more preferably 10 seconds or longer and 50 hours or shorter, and still more preferably 20 seconds or longer and 10 hours or shorter.

The temperature at the time of distilling off the low-boiling component is not particularly limited in a range in which the stability of the carbonyl compound and the separation of the carbonyl compound and the low-boiling component are possible, and from the viewpoint of suppressing the modification of the carbonyl compound, the temperature is preferably 20°C or higher and 300°C or lower, more preferably 30°C or higher and 280°C or lower, and still more preferably 40°C or higher and 250°C or lower.

Next, various raw materials used in the method for producing an isocyanate composition according to the present embodiment will be described in detail.

[Carbamate compound (Vd)]

The carbamate compound (Vd) is a compound represented by General Formula (Vd).

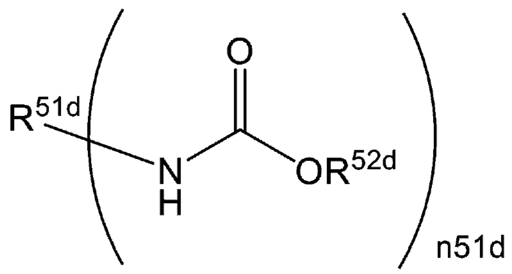

(Vd)

(in General Formula (Vd), $R^{51d}$ is an n51d- or higher valent organic group, and a relational expression of $R^{51d} = R^{71b}$ is satisfied, $R^{52d}$ is a monovalent organic group, and a relational expression of $R^{52d} = R^{12}$ is satisfied, and n51b is an integer of 2 or more and 8 or less, and a relational expression of n51b = n71b is satisfied)

($R^{51d}$)

$R^{51d}$ is an n51d-valent organic group, and a relational expression of $R^{51d} = R^{71b}$ is satisfied. That is, $R^{51d}$ is the same as $R^{71b}$ described above.

Among these, $R^{51d}$ is preferably a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom.

In addition, as $R^{31d}$, specifically, a group represented by any one of Formulae (Ia-1) to (Ia-24) is preferable, and a group represented by Formula (Ia-1), (Ia-2), (Ia-3), (Ta-14), (Ia-18), or (Ia-19) is more preferable.

($R^{52d}$)

$R^{52d}$ is a monovalent organic group, and a relational expression of $R^{52d} = R^{12}$ is satisfied. That is, $R^{52d}$ is the same as $R^{12}$ described above.

Among these, $R^{52d}$ is preferably an aliphatic hydrocarbon group having 1 or more and 20 or less carbon atoms or an aromatic hydrocarbon group having 6 or more and 20 or less carbon atoms, which may include an oxygen atom.

In addition, as $R^{52d}$, specifically, a phenyl group, a naphthyl group, an anthryl group, a pyrenyl group, and a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each

isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

(n51d)

n51d represents the number of carbamate groups, and a relational expression of n51d = n71b is satisfied. n51d is an integer of 2 or more and 8 or less, preferably an integer of 2 or more and 6 or less, more preferably an integer of 2 or more and 5 or less, and still more preferably an integer of 3 or more and 4 or less.

Examples of the preferred carbamate compound (Vd) include compounds represented by Formulae (Vd-1) to (Vd-6).

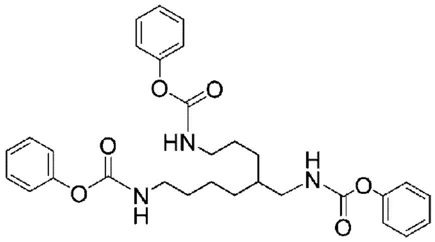

( Vd - 1)

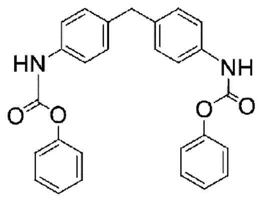

( Vd -2 )

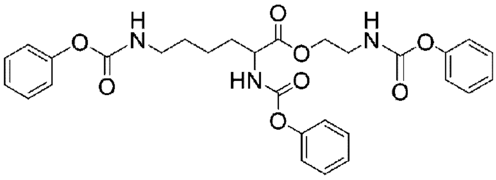

( Vd - 3)

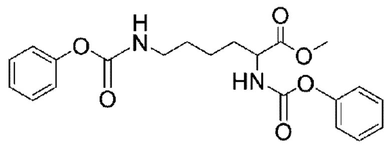

( Vd - 4)

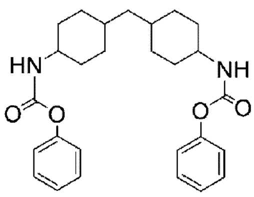

( Vd - 5)

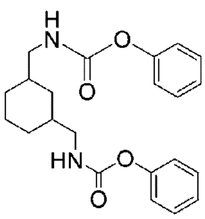

( Vd - 6)

[Method for producing carbamate compound (Vd)]

As the method for producing the carbamate compound (Vd), for example, a method for producing the carbamate compound from a carbonic acid derivative and an amine compound, or a method for producing the carbamate compound from a carbonic acid derivative, a hydroxy compound, and an amine compound is preferable.
In the synthesis reaction of the carbamate compound (Vd), a reactor that meets the appropriate conditions is selected. Specifically, a conventionally known reactor such as a stirring tank, a pressurized stirring tank, a reduced pressure stirring tank, a tower-type reactor, a distillation column, a filled column, and a thin film distillation column can be appropriately combined and used. For example, various known methods are used, such as a method using a reactor including any one of a distillation column, a multi-stage distillation column, a multi-tube type reactor, a continuous multi-stage distillation column, a filled column, a thin film evaporator, a reactor having a support inside, a forced circulation reactor, a falling film evaporator, and a falling drop evaporator, and a method in which these reactors are combined. From the viewpoint of rapidly mixing the amine compound, the carbonic acid derivative, and the hydroxy compound and then distilling the hydroxy compound after the reaction, a method using a stirring tank equipped with a distillation column or a multi-stage stirred tank is preferable.
The type of the condenser provided in the reactor is not particularly limited, and a known condenser can be used. For example, it is possible to appropriately combine and use a condenser known in the related art, such as a multi-tube cylindrical condenser, a double-tube condenser, a single-tube condenser, and an air-cooled condenser. The condenser may be provided inside the reactor, may be provided outside the reactor and connected to the reactor with a pipe, or may be adopted in various forms in consideration of the type of the reactor or the condenser, the method of handling the condensate, and the like.
The materials of the reactor and the condenser are not particularly limited, and known materials can be used as long as the materials do not adversely affect the carbonic acid derivative, the hydroxy compound, the amine compound, which are raw materials, and the carbamate compound (Vd), which is a product. For example, a glass, stainless steel, carbon steel, Hastelloy material, a material on which a glass lining is applied, or a material on which a Teflon (registered trademark) coating is applied can also be used. SUS304, SUS316, SUS316L, and the like are inexpensive and can be preferably used. As necessary, known process equipment such as measuring instruments, for example, a flowmeter and a thermometer, a reboiler, a pump, and a condenser may be added. The heating may be performed by a known method such as steam or a heater, and the cooling may be performed by a known method such as natural cooling, cooling water, or brine.
Next, various raw materials used in the method for producing the carbamate compound (Vd) will be described in detail.

(Carbonic acid derivative)

As the carbonic acid derivative, urea and carbonic acid esters are exemplary examples.
As the carbonic acid ester which is a raw material for producing the carbamate compound (Vd), the same carbonic acid ester as in the carbonic acid ester (IV) described above is preferably used. Among these, the carbonic acid derivative is preferably urea, diphenyl carbonate, or dibutyl carbonate, and more preferably urea or diphenyl carbonate.

(Amine compound)

As the amine compound, for example, a compound represented by General Formula (IIc) (hereinafter, may be referred to as "amine compound (IIc)") is preferably used.

$$R^{21c}\left(NH_2\right)_{n21c} \quad (IIc)$$

(in General Formula (IIc), $R^{21c}$ is an n21c-valent organic group, and a relational expression of $R^{21c} = R^{51d}$ is satisfied, and n21c is an integer of 2 or more and 8 or less, and a relational expression of n21c = n51d is satisfied)

(1) $R^{21c}$

$R^{21c}$ is an n21c-valent organic group, and a relational expression of $R^{21c} = R^{51d}$ is satisfied. That is, $R^{21c}$ is the same as $R^{51d}$ described above.
Among these, $R^{21c}$ is preferably a di- or higher and tetra- or lower valent aliphatic hydrocarbon group having

1 or more and 20 or less carbon atoms or a divalent or trivalent aromatic hydrocarbon group having 6 or more and 20 or less carbon atoms, each of which may have 1 or more and 4 or less ester groups or a nitrogen atom.
In addition, as $R^{21c}$, specifically, a group represented by any one of Formulae (Ia-1) to (Ia-24) is preferable, and a group represented by Formula (Ia-1), (Ia-2), (Ia-3), (Ia-14), (Ia-18), or (Ia-19) is more preferable.

(2) n21c

n21c represents the number of amino groups, and a relational expression of n21c = n51d is satisfied. n21c is an integer of 2 or more and 8 or less, preferably an integer of 2 or more and 6 or less, more preferably an integer of 2 or more and 5 or less, and still more preferably an integer of 3 or more and 4 or less.
Examples of the preferred amine compound (IIc) include 4-aminomethyl-1,8-octanediamine, 4,4'-diaminodiphenylmethane, lysine β-aminoethyl ester, lysine methyl ester, 4,4'-methylenebis(cyclohexylamine), and 1,3-di(aminomethyl)cyclohexane.

(Hydroxy compound)

As the hydroxy compound, a compound represented by General Formula (VId) (hereinafter, may be referred to as "hydroxy compound (VId)") is preferably used.

$$R^{61d}\text{—OH} \qquad (\text{VId})$$

(in General Formula (VId), $R^{61d}$ is a monovalent organic group, and a relational expression of $R^{61d} = R^{12}$ is satisfied)

(1) $R^{61d}$

$R^{61d}$ is a monovalent organic group, and a relational expression of $R^{61d} = R^{12}$ is satisfied. That is, $R^{61d}$ is the same as $R^{12}$ described above.
Among these, $R^{61d}$ is preferably an aliphatic hydrocarbon group having 1 or more and 20 or less carbon atoms or an aromatic hydrocarbon group having 6 or more and 20 or less carbon atoms, which may include an oxygen atom.
In addition, as $R^{61d}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a phenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyl decylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a methoxyphenyl group (each isomer), or an ethoxyphenyl group (each isomer) is preferable. In addition, a phenyl group, a methoxyphenyl group (each isomer), or an ethoxyphenyl group (each isomer) is more preferable.

Examples of the preferred hydroxy compound (VId) include an aromatic hydroxy compound represented by General Formula (VId-1) (hereinafter, may be referred to as "aromatic hydroxy compound (VId-1)").

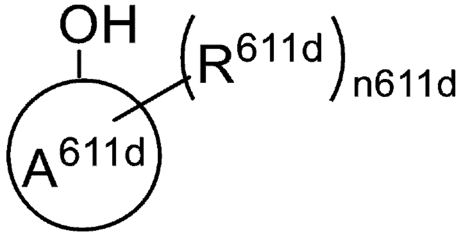

( VId -1 )

(in General Formula (VId-1), a ring $A^{611d}$ is an aromatic hydrocarbon ring having 6 or more and 20 or less carbon atoms, $R^{611d}$ is a hydrogen atom, an alkyl group having 1 or more and 20 or less carbon atoms, an alkoxy group having 1 or more and 20 or less carbon atoms, an aryl group having 6 or more and 20 or less carbon atoms, an aryloxy group having 6 or more and 20 or less carbon atoms, an aralkyl group having 7 or more and 20 or less carbon atoms, an aralkyloxy group having 7 or more and 20 or less carbon atoms, or a hydroxy group, $R^{611d}$ may be bonded to the ring $A^{611d}$ to form a ring structure, and n611d is an integer of 1 or more and 10 or less)

(2) $R^{611d}$

Examples of the alkyl group having 1 or more and 20 or less carbon atoms, as $R^{611d}$, include a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), a dodecyl group (each isomer), and an octadecyl group (each isomer).

Examples of the alkoxy group having 1 or more and 20 or less carbon atoms, as $R^{611d}$, include a methoxy group, an ethoxy group, a propoxy group (each isomer), a butyloxy group (each isomer), a pentyloxy group (each isomer), a hexyloxy group (each isomer), a heptyloxy group (each isomer), an octyloxy group (each isomer), a nonyloxy group (each isomer), a decyloxy group (each isomer), a dodecyloxy group (each isomer), and an octadecyloxy group (each isomer).

Examples of the aryl group having 6 or more and 20 or less carbon atoms, as $R^{611d}$, include a phenyl group and a naphthyl group.

Examples of the aryl group having an alkyl group as the substituent in $R^{611d}$ include a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), a biphenyl group (each isomer), a dimethylphenyl group (each isomer), a diethylphenyl group (each isomer), a dipropylphenyl group (each isomer), a dibutylphenyl group (each isomer), a dipentylphenyl group (each isomer), a dihexylphenyl group (each isomer), a diheptylphenyl group (each isomer), a terphenyl group (each isomer), a trimethylphenyl group (each isomer), a triethylphenyl group (each isomer), a tripropylphenyl group (each isomer), and a tributylphenyl group (each isomer).

Examples of the aryloxy group having 6 or more and 20 or less carbon atoms, as $R^{611d}$, include a phenoxy group, a methylphenoxy group (each isomer), an ethylphenoxy group (each isomer), a propylphenoxy group (each isomer), a butylphenoxy group (each isomer), a pentylphenoxy group (each isomer), a hexylphenoxy group (each isomer), a heptylphenoxy group (each isomer), an octylphenoxy group (each isomer), a nonylphenoxy group (each isomer), a

decylphenoxy group (each isomer), a phenylphenoxy group (each isomer), a dimethylphenoxy group (each isomer), a diethylphenoxy group (each isomer), a dipropylphenoxy group (each isomer), a dibutylphenoxy group (each isomer), a dipentylphenoxy group (each isomer), a dihexylphenoxy group (each isomer), a diheptylphenoxy group (each isomer), a diphenylphenoxy group (each isomer), a trimethylphenoxy group (each isomer), a triethylphenoxy group (each isomer), a tripropylphenoxy group (each isomer), and a tributylphenoxy group (each isomer).

Examples of the aralkyl group having 7 or more and 20 or less carbon atoms, as $R^{611d}$, include a phenylmethyl group, a phenylethyl group (each isomer), a phenylpropyl group (each isomer), a phenylbutyl group (each isomer), a phenylpentyl group (each isomer), a phenylhexyl group (each isomer), a phenylheptyl group (each isomer), a phenyloctyl group (each isomer), and a phenylnonyl group (each isomer).

Examples of the aralkyloxy group having 7 or more and 20 or less carbon atoms, as $R^{611d}$, include a phenylmethoxy group, a phenylethoxy group (each isomer), a phenylpropyloxy group (each isomer), a phenylbuthyloxy group (each isomer), a phenylpentyloxy group (each isomer), a phenylhexyloxy group (each isomer), a phenylheptyloxy group (each isomer), a phenyloctyloxy group (each isomer), and a phenylnonyloxy group (each isomer).

(3) $A^{611d}$

The ring $A^{611d}$ is an aromatic hydrocarbon ring having 6 or more and 20 or less carbon atoms. The ring $A^{611d}$ may be a monocyclic ring, a polycyclic ring, or a fused ring.

Specific examples of the ring $A^{611d}$ include a benzene ring, a naphthalene ring, an anthracene ring, a phenanthrene ring, a naphthacene ring, a chrysene ring, a pyrene ring, a triphenylene ring, a pentylene ring, an azulene ring, a heptalene ring, an indacene ring, a biphenylene ring, an acenaphthylene ring, an acenanthrylene ring, and an acenaphthylene ring. Among these, the ring $A^{511}$ is preferably a benzene ring, a naphthalene ring, or an anthracene ring, and more preferably a benzene ring.

In addition, these rings may have a substituent other than $R^{611d}$. Examples of the substituent other than $R^{611d}$ include the same one as that described in $R^{611d}$. $R^{611d}$ and the substituent other than $R^{611d}$ consist of different functional groups.

(4) n611d

n611d represents the number of substituents $R^{611d}$ and is an integer of 1 or more and 10 or less.

In General Formula (VId-1), examples of the compound in which the ring $A^{611d}$ is a benzene ring include a compound represented by General Formula (VId-1-1) (hereinafter, may be referred to as "hydroxy compound (VId-1-1)").

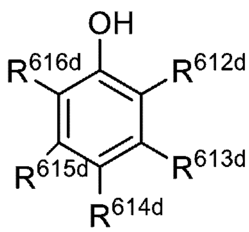

( VId-1-1 )

(in General Formula (VId-1-1), $R^{612d}$, $R^{613d}$, $R^{614d}$, $R^{615d}$, and $R^{616d}$ are each independently the same as $R^{611d}$ described above)

Among these, it is preferable that at least one of $R^{612d}$, $R^{613d}$, $R^{614d}$, $R^{615d}$, or $R^{616d}$ is a hydrogen atom, and it is more preferable that all of $R^{612d}$, $R^{613d}$, $R^{614d}$, $R^{615d}$, and $R^{616d}$ are hydrogen atoms.

Examples of the preferred hydroxy compound (VId-1-1) include phenol, 2-ethylphenol, 2-propylphenol (each isomer), 2-butylphenol (each isomer), 2-pentylphenol (each isomer), 2-hexylphenol (each isomer), 2-heptylphenol (each isomer), 2-phenylphenol, 2,6-dimethylphenol, 2,4-diethylphenol, 2,6-diethylphenol, 2,4-dipropylphenol (each isomer), 2,6-dipropylphenol (each isomer), 2,4-dibutylphenol (each isomer), 2,4-dipentylphenol (each isomer), 2,4-dihexylphenol (each isomer), 2,4-diheptylphenol (each isomer), 2-methyl-6-ethylphenol, 2-methyl-6-propylphenol (each isomer), 2-methyl-6-butylphenol (each isomer), 2-methyl-6-pentylphenol (each isomer), 2-ethyl-6-propylphenol (each isomer), 2-ethyl-6-butylphenol (each isomer), 2-ethyl-6-pentylphenol (each isomer), 2-propyl-6-butylphenol (each isomer), 2-ethyl-4-methylphenol (each isomer), 2-ethyl-4-propylphenol (each isomer), 2-ethyl-4-butylphenol (each isomer), 2-ethyl-4-pentylphenol (each isomer), 2-ethyl-4-hexylphenol (each isomer), 2-ethyl-4-heptylphenol (each isomer), 2-ethyl-4-octylphenol (each isomer), 2-ethyl-4-phenylphenol (each isomer), 2-ethyl-4-cumylphenol (each isomer), 2-propyl-4-methylphenol (each isomer), 2-propyl-4-ethylphenol (each isomer), 2-propyl-4-butylphenol (each isomer), 2-propyl-4-pentylphenol (each isomer), 2-propyl-4-hexylphenol (each isomer), 2-propyl-4-heptylphenol (each isomer), 2-propyl-4-octylphenol (each isomer), 2-propyl-4-phenylphenol (each isomer), 2-propyl-4-cumylphenol (each isomer), 2-butyl-4-methylphenol (each isomer), 2-butyl-4-ethylphenol (each isomer), 2-butyl-4-propylphenol (each isomer), 2-butyl-4-pentylphenol (each isomer), 2-butyl-4-hexylphenol (each isomer), 2-butyl-4-heptylphenol (each isomer), 2-butyl-4-octylphenol (each isomer), 2-butyl-4-phenylphenol (each isomer), 2-butyl-4-cumylphenol (each isomer), 2-pentyl-4-methylphenol (each isomer), 2-pentyl-4-ethylphenol (each isomer), 2-pentyl-4-propylphenol (each isomer), 2-pentyl-4-butylphenol (each isomer), 2-pentyl-4-hexylphenol (each isomer), 2-pentyl-4-heptylphenol (each isomer), 2-pentyl-4-octylphenol (each isomer), 2-pentyl-4-phenylphenol (each isomer), 2-pentyl-4-cumylphenol (each isomer), 2-hexyl-4-methylphenol (each isomer), 2-hexyl-4-ethylphenol (each isomer), 2-hexyl-4-propylphenol (each isomer), 2-hexyl-4-butylphenol (each isomer), 2-hexyl-4-pentylphenol (each isomer), 2-hexyl-4-heptylphenol (each isomer), 2-hexyl-4-octylphenol (each isomer), 2-hexyl-4-phenylphenol (each isomer), 2-hexyl-4-cumylphenol (each isomer), 2-heptyl-4-methylphenol (each isomer), 2-heptyl-4-ethylphenol (each isomer), 2-heptyl-4-propylphenol (each isomer), 2-heptyl-4-butylphenol (each isomer), 2-heptyl-4-pentylphenol (each isomer), 2-heptyl-4-hexylphenol (each isomer), 2-heptyl-4-octylphenol (each isomer), 2-heptyl-4-phenylphenol (each isomer), 2-heptyl-4-cumylphenol (each isomer), 2,4,6-trimethylphenol, 2,6-dimethyl-4-ethylphenol, 2,6-dimethyl-4-propylphenol (each isomer), 2,6-dimethyl-4-butylphenol (each isomer), 2,6-dimethyl-4-pentylphenol (each isomer), 2,6-dimethyl-4-hexylphenol (each isomer), 2,6-dimethyl-4-phenylphenol, 2,6-dimethyl-4-cumylphenol, 2,4,6-triethylphenol, 2,6-diethyl-4-methylphenol, 2,6-diethyl-4-propylphenol (each isomer), 2,6-diethyl-4-butylphenol (each isomer), 2,6-diethyl-4-pentylphenol (each isomer), 2,6-diethyl-4-hexylphenol (each isomer), 2,6-diethyl-4-phenylphenol, 2,6-diethyl-4-cumylphenol, 2,4,6-tripropylphenol (each isomer), 2,6-dipropyl-4-ethylphenol (each isomer), 2,6-dipropyl-4-methylphenol (each isomer), 2,6-dipropyl-4-butylphenol (each isomer), 2,6-dipropyl-4-pentylphenol (each isomer), 2,6-dipropyl-4-hexylphenol (each isomer), 2,6-dipropyl-4-phenylphenol (each isomer), 2,6-dipropyl-4-cumylphenol (each isomer), 2,4-dimethyl-6-ethylphenol, 2-methyl-4,6-diethylphenol, 2-methyl-4-propyl-6-ethylphenol (each isomer), 2-methyl-4-butyl-6-ethylphenol (each isomer), 2-methyl-4-pentyl-6-ethylphenol (each isomer), 2-methyl-4-hexyl-6-ethylphenol (each isomer), 2-methyl-4-phenyl-6-ethylphenol (each isomer), 2-methyl-4-cumyl-6-ethyl Phenol (each isomer), 2,4-dimethyl-6-propylphenol (each isomer), 2-methyl-4,6-dipropylphenol (each isomer), 2-methyl-4-ethyl-6-propylphenol (each isomer), 2-methyl-4-butyl-6-propylphenol (each isomer), 2-methyl-4-pentyl-6-propylphenol (each isomer), 2-methyl-4-hexyl-6-propylphenol (each isomer), 2-methyl-4-phenyl-6-propylphenol (each isomer), 2-methyl-4-cumyl-6-propylphenol (each isomer), 2,4-dimethyl-6-butylphenol, 2-methyl-4,6-dibutylphenol, 2-methyl-4-propyl-6-butylphenol (each isomer), 2-methyl-4-ethyl-6-butylphenol (each isomer), 2-methyl-4-pentyl-6-butylphenol (each isomer), 2-methyl-4-hexyl-6-butylphenol (each isomer), 2-methyl-4-phenyl-6-butylphenol (each isomer), 2-methyl-4-cumyl-6-butylphenol (each isomer), 2,4-dimethyl-6-pentylphenol, 2-methyl-4,6-dipentylphenol, 2-methyl-4-propyl-6-pentylphenol (each isomer), 2-methyl-4-butyl-6-pentylphenol (each isomer), 2-methyl-4-ethyl-6-pentylphenol (each isomer), 2-methyl-4-hexyl-6-pentylphenol (each isomer), 2-methyl-4-phenyl-6-pentylphenol (each isomer), 2-methyl-4-cumyl-6-pentylphenol (each isomer), 2,4-dimethyl-6-hexylphenol, 2-methyl-4,6-dihexylphenol, 2-methyl-4-propyl-6-hexylphenol (each isomer), 2-methyl-4-butyl-6-hexylphenol (each isomer), 2-methyl-4-pentyl-6-hexylphenol (each isomer), 2-methyl-4-ethyl-6-hexylphenol (each isomer), 2-methyl-4-phenyl-6-hexylphenol (each isomer), 2-methyl-4-cumyl-6-hexylphenol (each isomer), 2-ethyl-4-methyl-6-propylphenol (each isomer), 2,4-diethyl-6-propylphenol (each isomer), 2-ethyl-4,6-propylphenol (each isomer), 2-ethyl-4-butyl-6-propylphenol (each isomer), 2-ethyl-4-pentyl-6-propylphenol (each isomer), 2-ethyl-4-hexyl-6-propylphenol (each isomer), 2-ethyl-4-heptyl-6-propylphenol (each isomer), 2-ethyl-4-octyl-6-propylphenol (each isomer), 2-ethyl-4-phenyl-6-propylphenol (each isomer), 2-ethyl-4-cumyl-6-propylphenol (each isomer), 2-ethyl-4-methyl-6-butylphenol (each isomer), 2,4-diethyl-6-butylphenol (each isomer), 2-ethyl-4,6-butylphenol (each isomer), 2-ethyl-4-propyl-6-butylphenol (each isomer), 2-ethyl-4-pentyl-6-butylphenol (each isomer), 2-ethyl-4-hexyl-6-butylphenol (each isomer), 2-ethyl-4-heptyl-6-butylphenol (each isomer), 2-ethyl-4-octyl-6-butylphenol (each isomer), 2-ethyl-4-phenyl-6-butylphenol (each isomer), 2-ethyl-4-cumyl-6-butylphenol (each isomer), 2-ethyl-4-methyl-6-pentylphenol (each isomer), 2,4-diethyl-6-pentylphenol (each isomer), 2-ethyl-4,6-pentylphenol (each isomer), 2-ethyl-4-butyl-6-pentylphenol (each isomer), 2-ethyl-4-propyl-6-pentylphenol (each isomer), 2-ethyl-4-hexyl-6-pentylphenol (each isomer), 2-ethyl-4-heptyl-6-pentylphenol (each isomer) isomer), 2-ethyl-4-octyl-6-pentylphenol (each isomer), 2-ethyl-4-phenyl-6-pentylphenol (each isomer), 2-ethyl-4-cumyl-6-pentylphenol (each isomer), 2-ethyl-4-methyl-6-hexylphenol (each isomer), 2,4-diethyl-6-hexylphenol (each isomer), 2-ethyl-4,6-hexylphenol (each isomer), 2-ethyl-4-propyl-6-hexylphenol (each isomer), 2-ethyl-4-pentyl-6-hexylphenol (each isomer), 2-ethyl-4-butyl-6-hexylphenol (each isomer), 2-ethyl-4-heptyl-6-hexylphenol (each isomer), 2-ethyl-4-octyl-6-hexylphenol (each isomer), 2-ethyl-4-phenyl-6-hexylphenol (each isomer), 2-ethyl-4-cumyl-6- Hexylphenol (each isomer), 2-propyl-4-methyl-6-butylphenol (each isomer), 2,4-dipropyl-6-butylphenol (each isomer), 2-propyl-4,6-butylphenol (each isomer), 2-propyl-4-ethyl-6-butylphenol (each isomer), 2-propyl-4-pentyl-6-butylphenol (each isomer), 2-propyl-4-hexyl-6-butylphenol (each isomer), 2-propyl-4-heptyl-6-butylphenol (each isomer), 2-propyl-4-octyl-6-butylphenol (each isomer), 2-propyl-4-phenyl-6-butylphenol (each isomer), 2-propyl-4-cumyl-6-butylphenol (each isomer), 2,4-dicumylphenol, methoxyphenol (each isomer), and ethoxyphenol (each isomer). Among these, phenol, methoxyphenol (each isomer), or ethoxyphenol (each isomer) is preferable.

<<Method for producing isocyanate compound>>

The method for producing an isocyanate compound according to the present embodiment includes distilling and purifying the isocyanate composition described above and continuously recovering the isocyanate compound as a gas-phase component (hereinafter, may be referred to as "high-boiling separation step").

In the method for producing an isocyanate compound according to the present embodiment, by using the isocyanate composition described above, the fixation of byproducts to the device can be prevented and the yield of the isocyanate compound can be improved. That is, the method for producing an isocyanate compound according to the present embodiment can also be referred to as a method for recovering an isocyanate compound or a method for purifying an isocyanate compound.

The isocyanate composition can be obtained by using the method described in the section of "Method for producing isocyanate composition" above. In a case of the isocyanate composition that has not been subjected to the low-boiling separation step after the thermal decomposition step, it is preferable to use the isocyanate composition as a composition used in the method for producing an isocyanate compound of the present embodiment after the low-boiling separation step.

Hereinafter, the method for producing an isocyanate compound according to the present embodiment will be described in detail.

[High-boiling separation step]

In the high-boiling separation step, the isocyanate composition described above is distilled and purified, and the isocyanate compound is continuously recovered as a gas-phase component and separated from a component having a boiling point higher than that of the isocyanate compound (high-boiling component) (hereinafter, referred to as "high-boiling separation").

The distillation method is not particularly limited as long as the isocyanate can be separated as a gas-phase component. The "high-boiling component (high-boiling-point component)" referred to herein is a component having a boiling point higher than that of isocyanate, and the high-boiling-point component varies depending on the type of the compound which is used as a raw material for producing the isocyanate compound, but is mainly the carbonyl compound (I) or the carbamate compound (Vd) (a carbamate compound which is used as a raw material for the thermal decomposition step) which is produced in the thermal decomposition step, or a modified product of the isocyanate compound.

In the high-boiling separation step, the recovery rate of the isocyanate compound recovered in the gas phase may exceed 100% by mass due to the reaction in which the uretonimine group produced in the thermal decomposition step or the low-boiling separation step is regenerated into an isocyanate group and a carbodiimide group as shown in Formula (J).

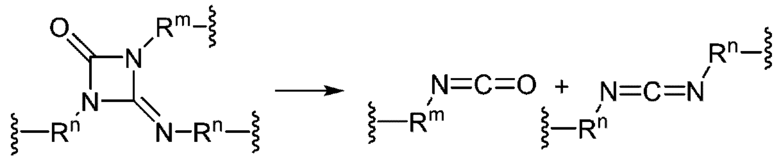
(J)

(in Formula (J), $R^m$ and $R^n$ are each independently a di- or higher valent organic group)

On the other hand, in the high-boiling separation step, the isocyanate compound is distilled off, and thus, there is a concern that the high-boiling component is concentrated and solidified, which makes it difficult to continue the operation, and it is difficult to recover the isocyanate compound at a high yield. This is because the modified product of the isocyanate compound has a high molecular weight, and the isocyanate group of the isocyanate polymer and the carbodiimide group generated by the above-described Formula (J) are bonded to form a uretonimine group (reverse reaction of Formula (J)) during high-boiling fraction separation, and thus the modified products of the isocyanate compound are bonded to each other to increase the molecular weight.

The carbonyl compound (1) has a higher boiling point than the isocyanate compound and has fewer cross-linking points than the modified product of the isocyanate compound. Therefore, the carbonyl compound (I) can act as a solvent in the high-boiling separation step. Alternatively, the carbonyl compound (I) is bonded to the carbodiimide group, and the modified products of the isocyanate compound are bonded to each other to prevent an increase in molecular weight. These actions make it possible to improve operability in the high-boiling separation step and to recover the isocyanate compound at a high yield.

The pressure at the time of separating the high-boiling component varies depending on the type of the compound and the reaction temperature, but may be any of a reduced pressure, a normal pressure, and a pressurization in a range

in which the isocyanate compound and the low-boiling-point component can be separated, and it is preferably 0.1 Pa or more and $1 \times 10^6$ Pa or less, more preferably 1 Pa or more and $1 \times 10^4$ Pa or less, and still more preferably 5 Pa or more and $1 \times 10^3$ Pa or less.

The operation time (in the case of the continuous method, the retention time) at the time of separating the high-boiling component is not particularly limited as long as the isocyanate compound and the low-boiling-point component can be separated, and from the viewpoint of suppressing a side reaction with the isocyanate compound, the operation time is preferably 5 seconds or longer and 100 hours or shorter, more preferably 10 seconds or longer and 50 hours or shorter, still more preferably 15 seconds or longer and 10 hours or shorter, particularly preferably 20 seconds or longer and 1 hour or shorter, and most preferably 25 seconds or longer and 10 minutes or shorter.

The temperature at the time of separating the high-boiling component is not particularly limited in a range in which the isocyanate compound is stable and the isocyanate compound and the low-boiling-point component can be separated, and from the viewpoint of suppressing the modification of the isocyanate compound, the temperature is preferably 20°C or higher and 250°C or lower, more preferably 30°C or higher and 230°C or lower, and still more preferably 40°C or higher and 200°C or lower.

[Apparatus and material]

The material of the reactor and the line in which the thermal decomposition step and the purification step are performed may be any known material as long as the material does not adversely affect the carbamate compound, the hydroxy compound and the isocyanate compound which are the products, and the carbonic acid ester which is the solvent, but SUS304, SUS316, SUS316L, and the like are inexpensive and can be preferably used.

The type of the reactor is not particularly limited, and a known reactor having a trough shape or a tower shape can be used. In the thermal decomposition reaction and the subsequent distillation of low-boiling components, a low boiling point mixture containing a hydroxy compound to be produced is preferably extracted from the reactor as a gaseous component, and a line for extracting a part or all of a liquid mixture from the reactor, which contains an unreacted carbamate compound or a compound that has not been extracted as a gaseous component, is preferably used. As such a reactor, various known methods are used, such as a method using a reactor including any one of a stirring tank, a multi-stage stirring tank, a distillation column, a multi-stage distillation column, a multi-tube type reactor, a continuous multi-stage distillation column, a filled column, a thin film evaporator, a reactor having a support inside, a forced circulation reactor, a falling film evaporator, a falling drop evaporator, a microflow phase reactor, and a bubble column, and a method in which these reactors are combined.

From the viewpoint of separating the hydroxy compound to be produced and the isocyanate compound, a method using a stirring tank equipped with a distillation column or a multi-stage stirring tank is preferable, and a structure having a large gas-liquid contact area in which the low-boiling-point component to be produced is promptly moved to the gas phase is preferable.

Among the purification steps of the isocyanate composition, in the low-boiling separation step, a device is preferably used which is provided with a line for extracting the low-boiling component as a gaseous component from the reactor and extracting a part or all of the liquid mixture containing the compound that has not been extracted as a liquid from the reactor. As such a reactor, various known methods are used, such as a method using a reactor including any one of a stirring tank, a multi-stage stirring tank, a distillation column, a multi-stage distillation column, a multi-tube type reactor, a continuous multi-stage distillation column, a filled column, a thin film evaporator, a reactor having a support inside, a forced circulation reactor, a falling film evaporator, a falling drop evaporator, a microflow phase reactor, and a bubble column, and a method in which these reactors are combined.

In addition, with the purification step of the isocyanate composition, in the high-boiling separation step, a device is preferably used which is provided with a line for extracting the isocyanate compound as a gaseous component from the reactor and extracting a part or all of the liquid mixture containing the compound that has not been extracted as a liquid from the reactor. As such a reactor, various known methods are used, such as a method using a reactor including any one of a stirring tank, a multi-stage stirring tank, a distillation column, a multi-stage distillation column, a multi-tube type reactor, a continuous multi-stage distillation column, a filled column, a thin film evaporator, a reactor having a support inside, a forced circulation reactor, a falling film evaporator, a falling drop evaporator, a microflow phase reactor, and a bubble column, and a method in which these reactors are combined.

[Examples]

Hereinafter, the present invention will be specifically described with reference to Examples, but the scope of the present invention is not limited to these Examples. Hereinafter, "%" means "% by mass", and "ppm" means "ppm by mass".

<<First test>>

<Analysis method>

[Liquid chromatography analysis method]

The analysis was performed under the following conditions.

(Measurement conditions)

Device: LC-10AT manufactured by Shimadzu Corporation
Column: Inertsil ODS
Particle diameter: 5 μm, inner diameter: 2.1 mm, length: 250 mm
Column temperature: 40°C
Developing solvent: water/acetonitrile = 90/10
Solvent flow rate: 1 mL/min
Detector: photodiode array detector

(3-1) Preparation of liquid chromatography analysis sample

1.0 g of the sample solution was weighed, and a solution obtained by adding 10 g of acetic acid to the sample solution and uniformly mixing was used as a liquid chromatography analysis sample.

(3-2) Quantitative analysis method

Each standard substance was analyzed, and a quantitative analysis of the analysis sample solution was performed based on a created calibration curve. The yield of the product in each step was calculated from the measured values.

[Gas chromatography analysis method]

The analysis was performed under the following conditions.

(Measurement conditions)

Device: GC-2010 manufactured by Shimadzu Corporation
Column: DB-1
Diameter: 0.25 mm, length: 30 m, film thickness: 1.0 μm
Column temperature: 60°C to 300°C
Injection port temperature: 300°C
Carrier gas: helium
Carrier gas flow rate: 40 mL/min
Detector: flame ionization detector (FID)

(2-1) Preparation of gas chromatography analysis sample

1.0 g of the sample solution was weighed, and a solution obtained by adding 10 g of acetonitrile and 0.1 g of anisole as an internal standard substance to the sample solution and uniformly mixing was used as a gas chromatography analysis sample.
For the filled column described in the present example, unless otherwise specified, a filled column filled with a ceramic Raschig ring was used, which satisfied the reaction results and the separation results of each step.

[Example 1-1]

1. Production of isocyanate compound

(1) Step (1): production step of carbamate compound

A step (1) was performed using a device shown in FIG. 1.

A mixture of 28.4 kg of n-butanol, 2.5 kg of urea, and 1.1 kg of 1,6-hexamethylenediamine were premixed in a tank A101, and the mixture was heated to 170°C in a tank A102. Thereafter, the mixture was supplied from a line A21 to a distillation column A201 having a column bottom temperature of 240°C and a column top pressure of 0.4 MPa at a rate of 2 kg/hour, and a carbamylation reaction solution was recovered in a storage tank A205 through a line A22. The gas-phase component generated in the distillation column A201 was introduced into a condenser A203 through a line A23, condensed as a mixture of n-butanol and ammonia, extracted into a tank A204, and a part of the mixed solution was refluxed from a line A24 at a rate of 1 kg/hour.

2. Step (1a): concentration step of N-substituted carbamate compound

Using a device shown in FIG. 2, carbon dioxide was supplied from a line A30 to the carbamylation reaction solution obtained in the step (1) and recovered in the storage tank A205 at a rate of 1 kg/hour, the mixture was premixed on the line A31, and the mixture was supplied to a thin film evaporator A301 with a temperature of 150°C and an internal pressure of 0.2 MPa at a rate of 1.5 kg/hour. The reaction solution in which the carbamate component was concentrated was recovered in a storage tank A303 at a rate of 0.2 kg/hour through a line A32. In a case where the recovered reaction solution was analyzed by liquid chromatography, an N-substituted carbamate compound represented by Formula (V-1) was produced with a yield of 95% by mass with respect to 1,6-hexamethylenediamine.

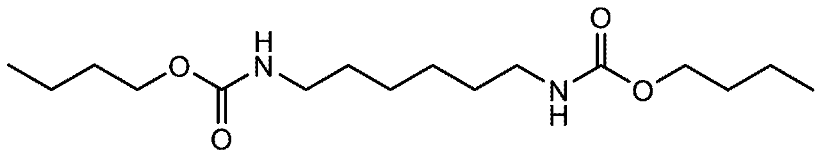

( V-1 )

3. Step (2): production step of isocyanate compound (thermal decomposition step)

A thermal decomposition reaction was performed using a device shown in FIG. 3. The reaction solution recovered in the tank A303 was supplied from a line B11 to a distillation column B101 in a total reflux state by supplying dibutyl carbonate from a line B15 under conditions of 260°C and 800 kPa. A part of the condensed liquid, which was obtained by passing the gas-phase component containing n-butanol produced in the thermal decomposition reaction through a line B13 to a condenser B103 and cooling the gas-phase component to 100°C with a condenser A403, was refluxed in the distillation column B101 through a line B14, and the remaining condensed liquid was recovered in a tank B104. The condensate liquid recovered in the tank B104 was re-used as a raw material of the step (1). On the other hand, the reaction solution containing the isocyanate compound was recovered in a storage tank B105 through a line B12. In a case where the recovered reaction solution was analyzed by liquid chromatography, 1,6-hexamethylenediisocyanate was produced with a yield of 89% by mass with respect to the N-substituted carbamate compound represented by Formula (V-1). In addition, in the fraction, a carbonyl compound (a high-boiling-point product) represented by Formula (I-1-1) was by-produced in an amount equivalent to 5% by mass with respect to the produced 1,6-hexamethylene diisocyanate.

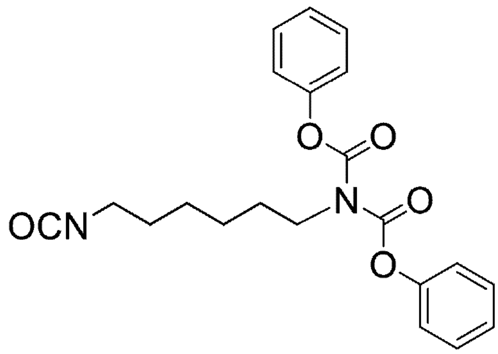

( I-1-1 )

4. Step (3): concentration step of isocyanate compound

The isocyanate compound was concentrated using the device shown in FIG. 4. The reaction solution recovered in the storage tank B105 was supplied to a distillation column C101 with a column top pressure of 20 kPa and a column top temperature of 130°C. The gas-phase component containing dibutyl carbonate was distilled and separated in the distillation column C101, and the liquid phase component containing the obtained 1,6-hexamethylene diisocyanate was recovered into the storage tank C105 through the line C12. The gas-phase component generated in the distillation column C101 was introduced into a condenser C103 through a line C13, condensed as a solution containing dibutyl carbonate, and extracted into a tank C104, and a part of the solution was refluxed in a line C14. The condensate liquid recovered in the tank C104 was re-used as a raw material of the step (2).

5. Step (4): isolation step of isocyanate compound (purification step)

An isocyanate compound was isolated (purified) using a device shown in FIG. 5. The fraction containing the 1.6-hexamethylene diisocyanate recovered in the storage tank C105 was evaporated in an evaporator D102 in which the pressure was set to 1 kPa and the temperature was set to 150°C from a line D11, and the gas-phase component was condensed in an evaporator (condensation part) D104 and extracted from a line D14. In a case where the reaction solution discharged through the line D14 was analyzed by gas chromatography, the purity of the recovered 1,6-hexamethylenediisocyanate was 99% by mass, and the content of the component represented by Formula (1-1-1) was 0.01% by mass. The high-boiling-point component was recovered from a bottom portion 0103 of the evaporator after extraction from a line D13. In a case where the liquid-phase component containing the carbonyl compound (high-boiling-point product) represented by Formula (1-1-1), which had been discharged through the line D13, was analyzed, it was found that the liquid-phase component had a formulation of 6.8% by mass of 1,6-hexamethylene diisocyanate, 25% by mass of the carbonyl compound represented by Formula (I-1-1), and 68% by mass of other high-boiling-point components, with respect to the total mass of the liquid-phase component.

6. Step (5): reproduction step of primary amine compound and hydroxy compound

A step (5) was performed using a device shown in FIG. 6.

5 kg of the liquid-phase component containing the recovered carbonyl compound (high-boiling-point product) represented by Formula (1-1-1) and 5 kg of n-butanol were supplied from a line F10 to a reactor F101 in a state of being held at 180°C. The liquid-phase component included a 1,6-hexamethylenediamine skeleton and an n-butyl skeleton, in addition to the carbonyl compound represented by Formula (1-1-1). Next, 10 kg of water and 0.6 kg of a 30% by mass sodium hydroxide aqueous solution were added thereto, and the mixture was heat-treated at 250°C for 3 hours. In a case where the reaction solution was analyzed by gas chromatography, 1,6-hexamethylenediamine was contained. Thereafter, the recovered reaction solution was distilled under reduced pressure with a vacuum distiller F102 to recover each of 1,6-hexamethylenediamine and n-butanol at a yield of 90% by mass (1,6-hexamethylenediamine skeleton and n-butyl skeleton components included in the supplied liquid-phase component). Both the 1,6-hexamethylenediamine and n-butanol recovered were re-used in the step (1) (production step of N-substituted carbamate compound).

7. Step (6): solvent recovery step (solvent re-using step)

A step (6) was performed using a device shown in FIG. 7.

Components distilled off as a gas phase from each of the step (1a) (concentration step of the N-substituted carbamate compound), the step (2) (production step of the isocyanate compound and thermal decomposition step), and

the step (3) (concentration step of isocyanate compound) were supplied to a distillation column E102 with a column top temperature of 95°C and a column top pressure of 40 kPa from a line E12 at a rate of 1.3 kg/hour, and distillation separation was performed. The n-butanol was extracted from the column top at a rate of 0.6 kg/hour, the dibutyl carbonate was extracted as a middle fraction (a side-cut fraction) at a rate of 0.54 kg/hour, and the high-boiling-point component was extracted from the column bottom at a rate of 0.16 kg/hour. The n-Butanol was re-used as a solvent used in the step (1) (production step of N-substituted carbamate compound), the dibutyl carbonate was re-used as a solvent used in the step (1a) (concentration step of N-substituted carbamate compound) and the step (2) (production step of isocyanate compound and thermal decomposition step), and the column bottom component was extracted as a high-boiling-point component.

[Example 1-2]

Reaction and purification were performed under the conditions described in Example 1-1, except that isopropanol was used instead of n-butanol in the step (1) of Example 1-1, and diisopropyl carbonate was used instead of dibutyl carbonate in the step (2) of Example 1-1. The operating conditions of the steps (1) to (3) were as described in the table below corresponding to the vapor pressure of isopropanol and dipropyl carbonate.

[Example 1-3]

In the step (5) of Example 1-1, the operation was performed as follows, instead of the method described in Example 1-1.

1. Step (5-1): regeneration step of carbonyl compound (I)

10 kg of water and 0.6 kg of a 30% by mass sodium hydroxide aqueous solution were added to 5 kg of the liquid-phase component containing the carbonyl compound (high-boiling-point product) represented by Formula (I-2) and 5 kg of n-butanol recovered in a state of being held at 180°C, and the mixture was supplied to a reactor E201 with a stirring blade having a volume such that the average retention time was 30 minutes, from a line E20 shown in FIG. 8, and the mixture was heated at 250°C. The reaction solution was continuously extracted out of the reaction tank and then supplied to a reactor E202, and a heat treatment was performed at the same temperature as in the reactor E201 for 3 hours. In a case where the reaction solution was analyzed by gas chromatography, 1,6-hexamethylenediamine was contained. Thereafter, the recovered reaction solution was distilled under reduced pressure with a vacuum distiller E203 to recover each of 1,6-hexamethylenediamine and n-butanol at a yield of 98% by mass. Both the 1,6-hexamethylenediamine and n-butanol recovered were re-used in the step (1) (production step of N-substituted carbamate compound).

[Example 1-4]

The operation was performed by the method described in Example 1-1, except that carbonic acid dibutyl used in the step (2) of Example 1-1 was changed to a mixed solvent of carbonic acid dibutyl:m-xylene = 9:1 (mass ratio). The operating conditions of the step (3) was as described in the table below corresponding to the vapor pressure of carbonic acid dibutyl and m-xylene.

[Example 1-5]

The operation was performed under the conditions described in Example 1-1 and in the table below, except that N-butylurea was used instead of the urea used in the step (1) of Example 1-1. The gas-phase component generated in the distillation column A201 was ammonia derived from N-butyl urea and n-butylamine.

[Example 1-6]

The operation was performed under the conditions described in Example 1-1 and in the table below, except that N,N-dibutylurea was used instead of the urea used in the step (1) of Example 1-1. The gas-phase component generated in the distillation column A201 was n-butylamine derived from N,N-dibutylurea.

[Example 1-7]

In the step (2) of Example 1-1, the operation was performed as follows, instead of the method described in

Example 1-1.

A thermal decomposition reaction was performed using a device shown in FIG. 9.

The N-substituted carbamate concentrate obtained in the step (1a) of Example 1-1 was mixed in advance with dibutyl carbonate, and the mixture was supplied to a tubular thermal decomposition reactor B201 held at 260°C and 0.8 MPa from a line B20 at 0.2 kg/hour and subjected to a thermal decomposition reaction. As a result, 1,6-hexamethylenediisocyanate was produced from the N-substituted carbamate compound represented by Formula (V-1) in a yield of 86% by mass. The component containing the isocyanate obtained by the thermal decomposition was recovered and supplied to a distillation column B202.

As a result of the distillation and separation in the step (3) and the step (4), 1,6-hexamethylene diisocyanate, which is a product, was obtained with a purity of 95% by mass.

[Example 1-8]

In the step (2) of Example 1-1, the operation was performed as follows, instead of the method described in Example 1-1.

A thermal decomposition step was performed using a device shown in FIG. 10. Thermal decomposition reactors B301 and B302 were both falling film-type evaporators, the internal pressure was set to 0.6 MPa and the jacket temperature was set to 250°C. The column top pressure of a separation column B303 was set to 0.5 kPa, hexadecane was set to be in a total reflux state, the reaction solution recovered from the storage tank through a line B30a was supplied at approximately 2.1 kg/hour, and the hexadecane was supplied a line B30b at approximately 1 kg/hour. The liquid-phase component of the thermal decomposition reactor B301 was continuously supplied to the thermal decomposition reactor B302 through a line B31b. The liquid-phase component recovered from the bottom portion of the thermal decomposition reactor B302 was recovered from a line B32b and supplied to a separation column B303. The gas-phase components generated in the thermal decomposition reactor were extracted from the thermal decomposition reactor through lines B31a and B32a.

A separation column B303 was filled with a ceramic Raschig ring, the heat quantity necessary for the distillation separation was supplied from a reboiler B305, and the column bottom component was recovered from a line B33. The component containing hexamethylene diisocyanate was recovered from the middle stage of the separation column B303 and supplied to a separation column B307 through a line B34. The heat quantity necessary for distillation separation was supplied from a reboiler B308 to perform the distillation separation of hexamethylene diisocyanate in the separation column B307, and the hexamethylene diisocyanate was recovered from a line B36 provided in the middle of the separation column B307. The gas-phase component recovered from the column top of the separation column B307 was condensed by a condenser B306 and then recovered from a line B35. The component containing hexadecane recovered from the column bottom of the separation column B307 was recovered in a storage tank B309 through a line B37 and recycled through the line B30b.

[Example 1-9]

In the step (1) and the step (2) of Example 1-1, the operations were performed as follows, instead of the methods described in Example 1-1.

1. Step (1): production step of N-substituted carbamate compound

A carbamylation step was carried out using a device shown in FIG. 11. 2.3 kg of urea and 28.4 kg of n-butanol were supplied from each of lines A40 and A41 to a stirring tank A401. The mixture was heated to 130°C to obtain a uniform solution, and 1.5 kg of hexamethylenediamine was supplied from a line A42 to the stirring tank A401 at approximately 0.3 kg/hour. After supplying the total amount of hexamethylenediamine and stirring the mixture for 2 hours, the reaction solution was analyzed. As a result, 1,6-hexamethylenediurea was produced from hexamethylenediamine in a yield of approximately 95% by mass. The 1,6-hexamethylenediurea was supplied to a multi-stage distillation column A403 through lines A43 and A45. In the middle, ammonia was separated by a gas-liquid separator A402 and extracted from a line A44.

The multi-stage distillation column A403 was a distillation column using a cascade mini-ring (registered trademark) as a filling material, and in advance, n-butanol was charged to the column bottom at a rate of approximately 3.0 kg/hour and a mixed liquid of n-butanol and urea (urea concentration: approximately 6.8% by weight) was charged to the storage tank A405 at a rate of approximately 3.0 kg/hour, and the inside of the column was set to a total reflux state with a column bottom temperature set to 250°C. Here, the reaction solution after the liquid-gas separation was injected from the line A45 at approximately 1.5 kg/hour, and n-butanol was supplied from the line A46 at the bottom portion of the column at approximately 2.5 kg/hour. The mixed solution of n-butanol and urea obtained in the condenser A404 was

supplied from the line A48. The surplus mixed solution was recovered in the storage tank A406. The reaction solution was extracted from the bottom portion of the multi-stage distillation column A403, and recovered in the storage tank A407 through the line A49, and n-butanol was distilled off to perform an operation of concentrating the N-substituted carbamate to 5 times.

2. Step (2): production step of isocyanate compound (thermal decomposition step)

A thermal decomposition step was performed using a device shown in FIG. 12. Thermal decomposition reactors B401 and B402 were both falling film-type evaporators, the internal pressure was set to 0.8 MPa and the jacket temperature was set to 260°C. The column top pressure of a separation column B403 was set to 0.5 kPa to make decane in a total reflux state, and a raw material in which the N-substituted carbamate and dibutyl carbonate were pre-mixed was supplied from a line B40 at approximately 2.1 kg/hour, and the decane was supplied from a line B41 at approximately 1 kg/hour. The liquid-phase component of the thermal decomposition reactor B401 was continuously supplied to the thermal decomposition reactor B402 through a line B42. The liquid-phase component at the bottom portion of the thermal decomposition reactor B402 was recovered from the line B43.

The liquid-phase component generated in the thermal decomposition reactor was extracted from the thermal decomposition reactor through lines B42 and B43 and supplied to a separation column B403. The separation column B403 was filled with a ceramic Raschig ring, and the heat quantity required for the distillation separation was supplied from a reboiler B405.

The component containing hexamethylene diisocyanate was recovered from the column bottom of the separation column B403 and supplied to a separation column B407 through a line B44. The hexamethylene diisocyanate was distilled and separated in the separation column B407. The heat quantity necessary for the distillation separation was supplied from a reboiler B408, and the hexamethylene diisocyanate was recovered from a line B46 provided in the middle of the separation column B407.

[Examples 1-10 and 1-11]

In Examples 1-10 and 1-11, the operations were performed under the conditions described in Examples 1-7 and 1-8, except that phenol was used instead of n-butanol used in the step (1) of Examples 1-7 and 1-8; diphenyl carbonate:m-xylene = 95: 5 (mass ratio) was used instead of dibutyl carbonate used in the step (2) of Examples 1-7 and 1-8; a fraction containing isocyanate, phenol, m-xylene, and diphenyl carbonate as gas components was recovered in the step (2); and a component including phenol and m-xylene as the column top component, and isocyanate, diphenyl carbonate, and other high-boiling-point components as the bottom column component was extracted in the step (3). For the step (3), the operation was performed under the conditions described in the table below corresponding to the vapor pressure at which the low-boiling-point component containing the non-hydroxy compound could be distilled off. In addition, as the carbonyl compound (high-boiling-point product) by-produced in step (2), a compound having a structure of a phenyl group was by-produced instead of the n-butyl group having a structure represented by Formula (I-1-1).

[Example 1-12]

The production of the N-substituted carbamate was carried out by the following step, instead of the step (1) of Example 1-1.

8.9 kg of diphenyl carbonate was dissolved in 17.8 kg of acetonitrile in a stirring tank, 4.2 kg of acetonitrile containing 1.05 kg of 1,6-hexamethylenediamine was mixed therewith, and the mixture was stirred at 50°C for 3 hours. A mixture of acetonitrile, which had been distilled in advance by a known method, was supplied from a line A21 to a distillation column A201 with a column bottom temperature of 110°C and a column top pressure of 5 kPa at a rate of 2 kg/hour, and the carbamylation reaction solution was recovered in a storage tank A205 through a line A22. The gas-phase component generated in the distillation column A201 was introduced into a condenser A203 through a line A23, condensed as a mixture of phenol and a trace amount of acetonitrile, extracted into a tank A204, and a part of the mixed solution was refluxed from a line A24 at a rate of 3 kg/hour.

The operation was performed by the method described in Example 1-1, except that carbonic acid dibutyl as the thermal decomposition solvent used in the step (2) of Example 1-1 was changed to a mixed solvent of carbonic acid dibutyl:m-xylene = 95:5 (mass ratio).

[Example 1-13]

The operation was performed under the conditions described in Example 1-12, except that a stirring mixing tank was used instead of the distillation column used in the step (1) of Example 1-12.

[Comparative Example 1-1]

In the step (1) of Example 1-1, the operation was performed under the conditions described in Example 1-1, except that the operating pressure was set to 2 MPaG, and as a result, substantially no low-boiling-point component was obtained at the column top in the step (1), and the yield of the N-substituted carbamate was 3% by mass.

[Comparative Example 1-2]

In the step (2) of Example 1-1, the operation was performed under the conditions described in Example 1-1, except that the operating pressure was set to 12 MPaG, and as a result, substantially no low-boiling-point component was obtained at the column top in the step (2), and the yield of 1,6-hexamethylene was 13% by mass.

[Example 1-14]

The operation was performed under the conditions described in Example 1-1, except that the retention time was changed to 90 minutes in the step (2) of Example 1-1, and the yield of 1,6-hexamethylene diisocyanate was 90% by mass.

[Example 1-15]

The operation was performed under the conditions described in Example 1-1, except that a thin film evaporator was used instead of the distillation column in the step (2) of Example 1-1, and the yield of 1,6-hexamethylene diisocyanate was 89% by mass.

[Example 1-16]

In the steps up to the step (2) of Example 1-1, the following operation was performed with reference to Example 1-4 of PCT International Publication No. WO2019/131855 (Reference Document 2).

1. Step (1): production step of N-substituted carbamate compound

Using a device shown in FIG. 13, 50 kg of phenol was supplied to a stirring tank A501 through a line A50, and 38 kg of diphenyl carbonate was supplied to obtain a uniform solution. 8.2 kg of 1,6-hexamethylenediamine was slowly added thereto in a state in which the stirring tank A501 was held at 50°C. After completion of the addition, stirring was continued for 3 hours, and the reaction solution was transferred to a storage tank A502 through a line A51. In a case where the reaction solution was analyzed by liquid chromatography, an N-substituted carbamate represented by Formula (V-2) was produced with a yield of 95% by mass with respect to 1,6-hexamethylenediamine.

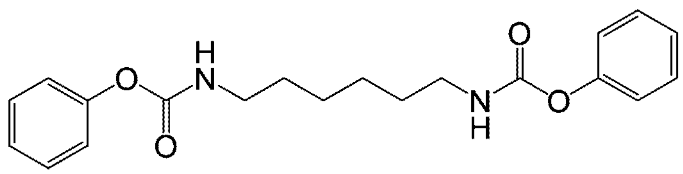

( V-2 )

2. Step (1b): transesterification step of N-substituted carbamate

Using a device shown in FIG. 14, p-cresol was supplied to a multi-stage distillation column A601 through a line A65, and the multi-stage distillation column A601 was heated by a reboiler A602 to be in a total reflux state. The reaction solution obtained in the above-described step (1) was supplied through a line A61, and a transesterification reaction of the compound represented by Formula (V-2) was carried out. The gas-phase component containing the phenol generated by the transesterification reaction was passed through a line A63 and supplied to a condenser A703. A part of the condensate obtained by cooling to 100°C in a condenser A603 was supplied to the multi-stage distillation column A601 through a line A64, and the remaining condensate was recovered in a storage tank A604. The obtained reaction solution was recovered in a storage tank A605 through a line A62.

In a case where the reaction solution was analyzed by liquid chromatography, the compound represented by Formula (V-3) was produced with a yield of 95% by mass with respect to Formula (V-2).

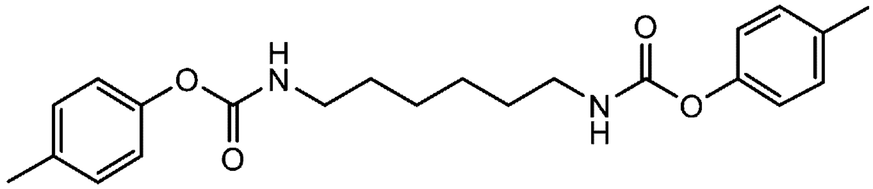

( V-3 )

3. Step (2): production step of isocyanate compound (thermal decomposition step)

A thermal decomposition was performed using a device shown in FIG. 15. The N-substituted carbamate liquid and diphenyl carbonate as raw materials were supplied to a thermal decomposition device B501 through a line B50 in a formulation of a mass ratio of 4:1. The thermal decomposition device B501 was a device for producing isocyanate by a thermal decomposition reaction of carbamate, and was composed of a falling film type tubular reactor and a separation tank for separating a liquid-phase component containing isocyanate and a gas-phase component. The internal pressure was set to 250 kPa, and the device was heated to 250°C by external heating.

The liquid-phase component generated in the thermal decomposition device B501 was supplied from a line B52 to the separation column B502 filled with a ceramic Raschig ring.

Diphenyl carbonate and 1,6-hexamethylene diisocyanate were separated. The heat quantity necessary for the distillation separation was supplied from a reboiler B505, and the column bottom component was recovered from a line B53 from the column bottom of the separation column B502.

The component containing 1,6-hexamethylene diisocyanate recovered from the column top of the separation column B502 was supplied through a condenser B504 and a line B55 to a purification column B503 filled with a ceramic Raschig ring, and the 1,6-hexamethylene diisocyanate was distilled and purified. The heat quantity required for the distillation purification was supplied from a reboiler B507.

The 1,6-hexamethylene diisocyanate was recovered from the column top of the purification column B503 through a condenser B506 and a line B56 at a rate of 2.4 kg/hour. The column bottom component of the purification column B503 was extracted from a line B54.

[Comparative Example 1-3]

The operation was performed under the conditions described in Example 1-1, except that the step (3) was not performed after obtaining the fraction containing 1,6-hexamethylenediisocyanate in the step (2) of Example 1-1. As a result, dibutyl carbonate, which was a low-boiling fraction, was accompanied in the fraction of 1,6-hexamethylenediisocyanate recovered in the step (4), and the purity of the product isocyanate was decreased. In addition, the high-boiling fraction with respect to the liquid rate supplied to the step (4) was small, liquid drying occurred, and the operation became difficult.

[Comparative Example 1-4]

The operation was performed under the conditions described in Example 1-1, except that the step (4) was not performed after obtaining the fraction containing 1,6-hexamethylene diisocyanate in the step (3) of Example 1-1. As a result, 9 wt% of the carbonyl compound represented by General Formula (I) and 8 wt% of high-boiling-point by-product were contained in the obtained isocyanate fraction.

[Comparative Example 1-5]

The operation was performed under the conditions described in Example 1-1, except that, in the step (2) of Example 1-1, the thermal decomposition solvent was changed from dibutyl carbonate to phenol and the operating pressure was set to 0.4 MPa. As a result, almost no low-boiling-point component was obtained at the column top in the step (2), and the yield of 1,6-hexamethylene diisocyanate was 5 wt%. In addition, the carbonyl compound (high-boiling-point product) represented by Formula (I-1-1) was not by-produced in the fraction containing the isocyanate. In a case where the isolation of 1,6-hexamethylene diisocyanate was attempted in the step (3) and the step (4), the isocyanate compound was modified and polymerized, and the operation became difficult, and the operation was stopped.

[Comparative Example 1-6]

The following operation was performed with reference to Examples 1-13 of PCT International Publication No.

WO2014/157636 (Reference Document 3).

1. Step (1): production step of N-substituted carbamate compound

1.6 kg of urea and 23 kg of phenol were supplied from each of lines A40 and A41 to a stirring tank A401 in FIG. 11. The mixture was heated to 130°C to obtain a uniform solution, and 1.4 kg of hexamethylenediamine was supplied from a line A42 to the stirring tank A401 at approximately 0.3 kg/hour. After supplying the total amount of hexamethylenediamine and stirring the mixture for 2 hours, the reaction solution was analyzed. As a result, 1,6-hexamethylenediurea was produced from hexamethylenediamine in a yield of approximately 95% by mass. The reaction solution containing the 1,6-hexamethylenediurea was supplied to a multi-stage distillation column A503 through lines A43 and A45. In the middle, ammonia was separated by a gas-liquid separator A402 and extracted from a line A44.

The multi-stage distillation column A403 was a distillation column filled with a cascade mini-ring (registered trademark) as a filling material. A phenol was charged into the column bottom in advance, and a mixed solution of urea and phenol (urea concentration: approximately 15% by weight) was charged into the storage tank A405, and the inside of the column was set to be in a total reflux state with a column bottom temperature set to 250°C. Here, the reaction solution after the liquid-gas separation was injected from the line A45 at approximately 1.5 kg/hour, and phenol was supplied from the line A46 at the bottom portion of the column at approximately 1.5 kg/hour. The mixed solution of phenol and urea obtained in the condenser A404 was supplied from the line A48. The surplus mixed solution was recovered in the storage tank A406. The reaction solution was extracted from the bottom portion of the multi-stage distillation column A403 at a rate of 2.3 kg/hour, and recovered in the storage tank A407 through the line A49.

2. Step (1a): concentration step of N-substituted carbamate compound

Next, a preliminary concentration was performed using a device shown in FIG. 16. A pre-concentrator A701 was a falling film-type evaporator, and the jacket temperature was set to 250°C and the internal pressure was set to 3 kPa. The reaction solution recovered in the storage tank A407 in the carbamating step was supplied to the pre-concentrator A701 through the line A71 at approximately 10 kg/hour. The generated gas-phase component was extracted from a line A73. The recovered product was phenol. On the other hand, the liquid-phase component of the pre-concentrator A701 was recovered through a line A72 into a storage tank A702 at approximately 2.5 kg/hour. 1,6-hexanediyldi(phenylcarbamate) was obtained from hexamethylenediamine in a yield of 96% by mass.

3. Step (1b): transesterification step of N-substituted carbamate

A transesterification was carried out using a device shown in FIG. 17. A continuous multi-stage distillation column A801 was a filled column filled with Raschig rings, and 4-(α,α-dimethylbenzyl)phenol was supplied to the bottom portion of the column in advance to set the inside of the column in a total reflux state of 4-(α,α-dimethylbenzyl)phenol. The heat quantity required was supplied from a reboiler A802. The column bottom temperature was set to 250°C. The liquid recovered in the preliminary concentrating step was supplied to a storage tank A702 from a line A81 at approximately 1 kg/hour, and 4-(α,α-dimethylbenzyl)phenol was supplied from a line A85 at approximately 4.2 kg/hour. The line A84 was closed at the same time as the supply of the reaction solution from the line A81 was started. The gas-phase component containing phenol as a main component was extracted from the column top, condensed through a line A83 in a condenser A803, and recovered in a storage tank A804. On the other hand, the reaction solution was extracted from the bottom portion of the column, and recovered at approximately 2.1 kg/hour in A storage tank A805 through a line A82. In a case where the liquid recovered in the storage tank A805 was analyzed, 1,6-hexanediyl-(carbamic acid (4-(α,α-dimethylbenzyl)phenyl)) was produced with a yield of 93% by mass with respect to 1,6-hexanediyl-(carbamic acid phenyl).

4. Step (2): production step of isocyanate compound (thermal decomposition step)

A thermal decomposition was carried out using a device shown in FIG. 18. Thermal decomposition reactors B601 and B602 were both falling film-type evaporators, the internal pressure was set to 1 kPa and the jacket temperature was set to 250°C. The column top pressure of a separation column B603 was set to 0.5 kPa, benzene toluene was in a total reflux state, and a 4-(α,α-dimethylbenzyl)phenol solution of N-substituted carbamate obtained in the step, not containing an aprotic solvent, was supplied from a line B60 at approximately 2.1 kg/hour. The liquid-phase component of the thermal decomposition reactor B601 was continuously supplied to the thermal decomposition reactor B602 through a line B61b. The liquid-phase component recovered from the bottom portion of the thermal decomposition reactor B602 was recovered from a line B63. The gas-phase component generated in the thermal decomposition reactor was extracted from the thermal decomposition reactor through lines B61a and B62 and supplied to a separation column B603. In a

case where the gas components contained in the lines B61a and B62 were sampled and analyzed, the corresponding 1,6-hexamethylenediisocyanate was obtained from the raw material of N-substituted carbamate with a yield of 88% by mass. The extracted liquid of B63 contained a high-boiling component, and since the carbonyl compound represented by Formula (I-1) was not by-produced, when the operation was performed under the high-concentration conditions, partial scaling occurred in the flow path of B63. In addition, since the amount of the low-boiling extractable component was large and the pressure loss was reduced by operating at a low pressure, it was necessary to increase pipe diameters of the lines B61a and B62.

5. Step (3): concentration step of isocyanate compound

The separation column B603 was filled with a ceramic Raschig ring, and the heat quantity required for the distillation separation was supplied from a reboiler B605.

6. Step (4): isolation step of isocyanate compound (purification step)

The component containing hexamethylene diisocyanate was recovered from the column top of the separation column B603, condensed in a condenser B604, and supplied to a separation column B607 through a line B64. The heat quantity necessary for distillation separation was supplied from a reboiler B608 to perform the distillation separation of hexamethylene diisocyanate in the separation column B607, and the 1,6-hexamethylene diisocyanate was recovered from a line B67 provided in the middle of the separation column B607.

The gas-phase component recovered from the top of the separation column B607 was condensed by a condenser B606 and recovered from the line B66, and the component containing the benzyltoluene and the dipropyl carbonate, recovered from the bottom of the separation column B607, was recycled to the separation column B603 through the line B68.

[Examples 1-17 to 1-20]

The production was performed by the methods described in Examples 1-10 to 1-12 and 1-14, except that the amount of hexamethylenediamine as the raw material used in Examples 1-10 to 1-12 and 1-14 was changed.

[Examples 1-21 and 1-22]

The production was performed by the methods described in Examples 1-12 and 1-14, except that the distillation column used in each step of Examples 1-12 and 1-14 was changed to a SUS304 sieve tray column.

[Examples 1-23 and 1-24]

The production was performed by the methods described in Examples 1-12 and 1-14, except that the filling material used in each step of Examples 1-12 and 1-14 was changed from Raschig rings to a Mellapak (manufactured by Sulzer Ltd.) made of SUS304.

[Examples 1-25 and 1-26]

The production was performed by the methods described in Examples 1-12 and 1-14, except that the thin film evaporator in the step (4) of Examples 1-12 and 1-14 was replaced with a filled column filled with a ceramic Raschig ring.

[Examples 1-27 and 1-28]

The production was performed by the methods described in Examples 1-12 and 1-14, except that a 30% by mass potassium hydroxide aqueous solution was used instead of the 30% by mass sodium hydroxide aqueous solution used in the step (5) of Examples 1-12 and 1-14.

[Example 1-29]

(Production of isocyanate compound)

1. Step (1): production step of N-substituted carbamate compound

Using the device shown in FIG. 1, a mixture of 3.1 kg of 4-aminomethyl-1,8-octanediamine, 3.7 kg of urea, and 88.2 kg of n-butanol were premixed in the tankA101, and the mixture was heated to 170°C in the tank A102. Thereafter, the mixture was supplied from the line A21 to the distillation column A201 having a column bottom temperature of 240°C and a column top pressure of 0.4 MPa at a rate of 1.0 kg/hour, and a carbamylation reaction solution was recovered in the storage tank A205 through the line A22. The gas-phase component generated in the distillation column A201 was introduced into the condenser A203 through the line A23, condensed as a mixture of n-butanol and ammonia, extracted into the tank A204, and a part of the mixed solution was refluxed from the line A24 at a rate of 1.3 kg/hour.

2. Step (1a): concentration step of N-substituted carbamate compound

Using a device shown in FIG. 2, carbon dioxide was supplied to the carbamylation reaction solution obtained in the step (1) and recovered in the storage tank A205 at a rate of 0.50 kg/hour, the mixture was pre-mixed on the line A31, and the mixture was supplied to a thin film evaporator A301 with a temperature of 150°C and an internal pressure of 30 kPa at a rate of 2.1 kg/hour. The reaction solution in which the carbamate component was concentrated was recovered in a storage tank A303 at a rate of 0.2 kg/hour through a line A32. In a case where the recovered reaction solution was analyzed by liquid chromatography, an N-substituted carbamate compound represented by Formula (V-1) was produced with a yield of 95% by mass with respect to 4-aminomethyl-1,8-octanediamine.

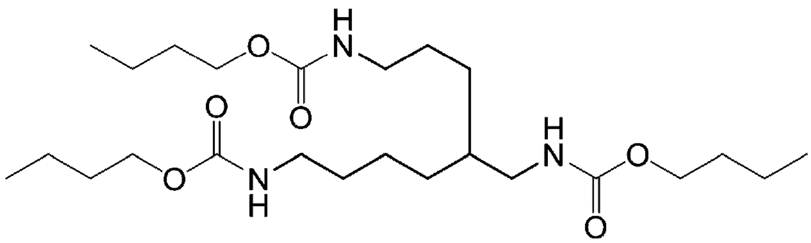

( V -4 )

3. Step (2): production step of isocyanate compound (thermal decomposition step)

A thermal decomposition reaction was performed using a device shown in FIG. 3. The reaction solution recovered in the tank A303 was supplied from a line B11 to a distillation column B101 in a total reflux state by supplying diphenyl carbonate from a line B15 under conditions of 250°C and 25 kPa. A part of the condensed liquid, which was obtained by passing the gas-phase component containing phenol produced in the thermal decomposition reaction through a line B13 to a condenser B103 and cooling the gas-phase component to 100°C with the condenser B103, was refluxed in the distillation column B101 through a line B14, and the remaining condensed liquid was recovered in a tank B104. The condensate liquid recovered in the tank B104 was re-used as a raw material of the step (1). On the other hand, the reaction solution containing the isocyanate compound was recovered in a storage tank B105 through a line B12. In a case where the recovered reaction solution was analyzed by liquid chromatography, the isocyanate compound represented by Formula (VII-1) (4-isocyanatomethyl-1,8-octamethylene diisocyanate) was produced with a yield of 92% by mass with respect to the N-substituted carbamate compound represented by Formula (V-4). In addition, in the fraction, a mixture (a high-boiling-point product) of carbonyl compounds represented by Formulae (I-5a-1) to (I-5c-1) was by-produced in an amount equivalent to 5% by mass with respect to the produced isocyanate compound represented by Formula (VII-1).

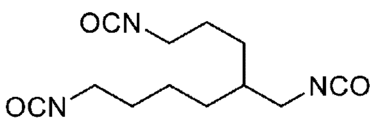

( VII - 1 )

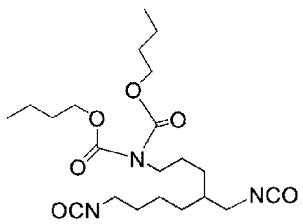

( I - 5a-1)

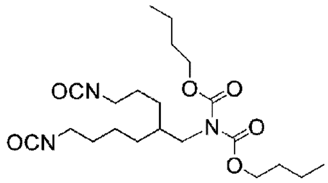

( I - 5b-1)

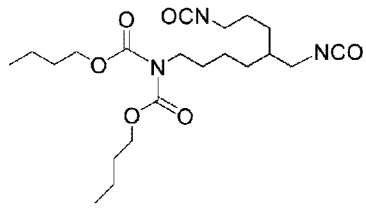

( I - 5c-1)

4. Step (3): concentration step of isocyanate compound

The isocyanate compound was concentrated using the device shown in FIG. 4. The reaction solution recovered in the storage tank B105 was supplied to a distillation column C101 with a column top pressure of 2 kPa and a column top temperature of 170°C. The gas-phase component containing diphenyl carbonate was distilled and separated in the distillation column C101, and the liquid phase component containing the obtained 4-isocyanatomethyl-1,8-octamethylene diisocyanate was recovered into the storage tank C105 through the line C12. The gas-phase component generated in the distillation column C101 was introduced into a condenser C103 through a line C13, condensed as a solution containing diphenyl carbonate, and extracted into a tank C104, and a part of the solution was refluxed in a line C14. The condensate liquid recovered in the tank C104 was re-used as a raw material of the step (2).

5. Step (4): isolation step of isocyanate compound (purification step)

An isocyanate compound was isolated (purified) using a device shown in FIG. 5. The fraction containing the 4-isocyanatomethyl-1,8-octamethylene diisocyanate recovered in the storage tank C105 was evaporated in an evaporator D102 in which the pressure was set to 100 Pa and the temperature was set to 200°C from a line D11, and the gas-phase component was condensed in an evaporator (condensation part) D104 and extracted from a line D14. In a case where the reaction solution discharged through the line D14 was analyzed by gas chromatography, the purity of the recovered 4-isocyanatomethyl-1,8-octamethylene diisocyanate was 99.1% by mass. The high-boiling-point component was recovered from a bottom portion D103 of the evaporator after extraction from a line D13. In a case where the liquid-phase component containing the carbonyl compounds (high-boiling-point products) represented by Formulae (I-5a-1) to (I-5c-1), which had been discharged through the line D13, was analyzed, it was found that the liquid-phase component had a formulation of 6.8% by mass of 4-isocyanatomethyl-1,8-octamethylene diisocyanate, 25.0% by mass of the carbonyl compound represented by Formula (1-1), and 68.2% by mass of other high-boiling-point components, with respect to the total mass of the liquid-phase component.

6. Step (5): reproduction step of primary amine compound and hydroxy compound

A step (5) was performed using a device shown in FIG. 6.

5 kg of the liquid-phase component containing the recovered carbonyl compounds (high-boiling-point products) represented by Formulae (I-5a-1) to (I-5c-1) and 5 kg of phenol were supplied from a line F10 to a reactor F101 in a state of being held at 180°C. The liquid-phase component included a 4-aminomethyl-1,8-octanediamine skeleton and an n-butyl skeleton, in addition to the carbonyl compounds represented by Formulae (I-5a-1) to (I-5c-1). Next, 10 kg of water and 0.6 kg of a 30% by mass sodium hydroxide aqueous solution were added thereto, and the mixture was heat-treated at 240°C for 3 hours. In a case where the reaction solution was analyzed by gas chromatography, 4-aminomethyl-1,8-octanediamine was contained. Thereafter, the recovered reaction solution was distilled under reduced pressure with a vacuum distiller F102 to recover each of 4-aminomethyl-1,8-octanediamine and n-butanol at a yield of 90% by mass. Both the 4-aminomethyl-1,8-octanediamine and n-butanol recovered were re-used in the step (1) (production step of N-substituted carbamate compound).

7. Step (6): solvent recovery step (solvent re-using step)

A step (6) was performed using a device shown in FIG. 7.

Components distilled off as a gas phase from each of the step (1a) (concentration step of the N-substituted carbamate compound), the step (2) (production step of the isocyanate compound and thermal decomposition step), and the step (3) (concentration step of isocyanate compound) were supplied to a distillation column E101 with a column top temperature of 95°C and a column top pressure of 40 kPa from a line E12 at a rate of 1.3 kg/hour, and distillation

separation was performed. The N-butanol was extracted from the column top at a rate of 0.6 kg/hour, the dibutyl carbonate was extracted as a middle fraction (a side-cut fraction) at a rate of 0.54 kg/hour, and the high-boiling-point component was extracted from the column bottom at a rate of 0.16 kg/hour. The n-Butanol was re-used as a solvent used in the step (1) (production step of N-substituted carbamate compound), the dibutyl carbonate was re-used as a solvent used in the step (1a) (concentration step of N-substituted carbamate compound) and the step (2) (production step of isocyanate compound and thermal decomposition step), and the column bottom component was extracted as a high-boiling-point component.

[Examples 1-30 to 1-267]

Various isocyanates were produced by changing the conditions of the steps (1) to (5) as described in the tables below.

In each table, abbreviations for compounds represent the following compounds. In addition, in the step (4), the content of the carbamate compound (I) is a percentage with respect to the mass of the isocyanate compound. In addition, in the step (5), the amount of the skeleton component derived from the primary amine skeleton and the hydroxy compound skeleton (used in the step (1)) included in the supplied liquid-phase component up to the step (4) was quantified, and the recovered amount of each skeleton component was defined as the amine recovery rate and the hydroxy compound recovery rate. In addition, for the step (6), the operating conditions corresponding to the vapor pressure of each component used in each of Examples and Comparative Examples were used.

(Primary amine compound)

HDA: diaminohexane
TAN: 4-aminomethyl-1,8-octanediamine
TAH: triaminohexane
TAU: triaminoundecane
IPDA: 3-aminomethyl-3,5,5-trimethylcyclohexylamine
HMDA: 4,4'-methylenebis(cyclohexylamine)
XDA: diaminoxylene
MDA: 4,4'-methylenedianiline
TAB: triaminobenzene
TAMB triaminomethylbenzene
TAPMB: tri(amino-propane-yl)-methylbenzene
LTA: 2,6-diaminohexanoic acid 2-aminoethyl
ODA: omithine ethyl ester diamine
LDA: lysine ethyl ester diamine

(Carbonic acid derivative)

DEC: diethyl carbonate
DPC: diphenyl carbonate

(Solvent in step (1))

n-BuOH: n-butanol
iPrOH: isopropanol
PhOH: phenol
MeCN: acetonitrile

(Hydroxy compound)

p-cresol: p-cresol
PCP: 4-($\alpha$,$\alpha$-dimethylbenzyl)phenol

(Low-boiling-point compound in step (1))

$NH_3$: ammonia
PhOH: phenol
EtOH: ethanol

(Solvent in step (2))

DBC: dibutyl carbonate
DiPrC: diisopropyl carbonate
m-Xy: m-xylene
DEC: diethyl carbonate
DPC: diphenyl carbonate
PCP: 4-($\alpha$,$\alpha$-dimethylbenzyl)phenol

(Reaction form in step (2))

FF: falling film-type evaporator
FF $\times$ 2: two falling film-type evaporators connected in series

(Low-boiling-point compound in step (2))

n-BuOH: n-butanol
PhOH: phenol
EtOH: ethanol

(Aprotic solvent extracted in step (3))

DBC: dibutyl carbonate
DiPrC: diisopropyl carbonate
m-Xy: m-xylene
DEC: diethyl carbonate
DPC: diphenyl carbonate

(Liquid-phase component extracted in step (4))

heavy: by-product having a boiling point higher than that of the isocyanate compound
DPC: diphenyl carbonate

(Organic solvent in step (5))

n-BuOH: n-butanol
iPrOH: isopropanol
PhOH: phenol

[Table 1-1]

| | Step (1) | | | | | | | | | | | | Step (1b) |
|---|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | | Hydroxy compound |
| | Type | Amount kg | Type | Type | Amount kg | °C | MPaG | min | | | % | | |
| Example 1-1 | HDA | 1.05 | Urea | n-BuOH | 28.4 | 220 | 1.3 | 30 | Distillation column | NH3 | 91 | | - |
| Example 1-2 | HDA | 1.05 | Urea | iPrOH | 28.4 | 220 | 0.45 | 30 | Distillation column | NH3 | 89 | | - |
| Example 1-3 | HDA | 1.05 | Urea | n-BuOH | 28.4 | 220 | 1.3 | 30 | Distillation column | NH3 | 91 | | - |
| Example 1-4 | HDA | 1.05 | Urea | n-BuOH | 28.4 | 220 | 1.3 | 30 | Distillation column | NH3 | 91 | | - |
| Example 1-5 | HDA | 1.05 | N-Butylurea | n-BuOH | 26.1 | 220 | 1.3 | 30 | Distillation column | N-Butylamine·NH3 | 90 | | - |
| Example 1-6 | HDA | 1.05 | N,N'-Butylurea | n-BuOH | 23.7 | 220 | 1.3 | 30 | Distillation column | N-Butylamine | 88 | | - |
| Example 1-7 | HDA | 1.05 | Urea | n-BuOH | 28.4 | 220 | 1.3 | 30 | Distillation column | NH3 | 91 | | - |
| Example 1-8 | HDA | 1.05 | Urea | n-BuOH | 28.4 | 220 | 1.3 | 30 | Distillation column | NH3 | 91 | | - |
| Example 1-9 | HDA | 1.05 | Urea | n-BuOH | 28.4 | 220 | 1.3 | 30 | Distillation column | NH3 | 91 | | - |
| Example 1-10 | HDA | 1.05 | Urea | PhOH | 28.4 | 240 | 0.3 | 30 | Distillation column | NH3 | 93 | | - |
| Example 1-11 | HDA | 1.05 | Urea | PhOH | 28.4 | 240 | 0.3 | 30 | Distillation column | NH3 | 93 | | - |
| Example 1-12 | HDA | 1.05 | DPC | MeCN | 22.0 | 50 | 0.01 | 40 | Distillation column | PhOH | 95 | | - |
| Example 1-13 | HDA | 1.05 | DPC | MeCN | 22.0 | 50 | 0.01 | 40 | Stirring tank | PhOH | 94 | | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-14 | HDA | 1.05 | Urea | PhOH | 28.4 | 240 | 0.3 | 30 | Distillation column | NH3 | 91 | - |
| Example 1-15 | HDA | 1.05 | Urea | n-BuOH | 28.4 | 220 | 1.3 | 30 | Distillation column | NH3 | 91 | - |
| Example 1-16 | HDA | 8.20 | DPC | PhOH | 50.0 | 50 | 0.01 | 180 | Stirring tank | PhOH | 91 | p-cresol |
| Example 1-17 | HDA | 0.70 | Urea | PhOH | 29.6 | 240 | 0.3 | 30 | Distillation column | NH3 | 94 | - |
| Example 1-18 | HDA | 0.70 | Urea | PhOH | 29.6 | 240 | 0.3 | 30 | Distillation column | NH3 | 95 | - |
| Example 1-19 | HDA | 0.70 | DPC | MeCN | 25.3 | 50 | 0.01 | 40 | Distillation column | PhOH | 96 | - |
| Example 1-20 | HDA | 0.70 | Urea | PhOH | 29.6 | 240 | 0.3 | 30 | Distillation column | NH3 | 92 | - |
| Example 1-21 | HDA | 1.05 | DPC | MeCN | 22.0 | 50 | 0.01 | 40 | Distillation column (sieve tray) | PhOH | 95 | - |
| Example 1-22 | HDA | 1.05 | Urea | PhOH | 28.4 | 240 | 0.3 | 30 | Distillation column (sieve tray) | NH3 | 91 | - |
| Example 1-23 | HDA | 0.70 | DPC | MeCN | 25.3 | 50 | 0.01 | 40 | Distillation column | PhOH | 95 | - |
| | | | | | | | | | (structured filling substance) | | | |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-24 | HDA | 0.70 | Urea | PhOH | 29.6 | 240 | 0.3 | 30 | Distillation column (structured filling substance) | NH3 | 91 | - |
| Example 1-25 | HDA | 0.70 | DPC | MeCN | 25.3 | 50 | 0.01 | 40 | Distillation column | PhOH | 95 | - |
| Example 1-26 | HDA | 0.70 | Urea | PhOH | 29.6 | 240 | 0.3 | 30 | Distillation column | NH3 | 91 | - |
| Example 1-27 | HDA | 1.05 | DPC | MeCN | 22.0 | 50 | 0.01 | 40 | Distillation column | PhOH | 95 | - |
| Example 1-28 | HDA | 1.05 | Urea | PhOH | 28.4 | 240 | 0.3 | 30 | Distillation column | NH3 | 91 | - |
| Comparative Example 1-1 | HDA | 1.05 | Urea | n-BuOH | 28.4 | 220 | 2 | 30 | Distillation column | NH3 | 3 | - |
| Comparative Example 1-2 | HDA | 1.05 | Urea | n-BuOH | 28.4 | 220 | 1.3 | 120 | Distillation column | NH3 | 91 | - |
| Comparative Example 1-3 | HDA | 1.05 | Urea | n-BuOH | 28.4 | 220 | 1.3 | 120 | Distillation column | NH3 | 91 | - |
| Comparative Example 1-4 | HDA | 1.05 | Urea | n-BuOH | 28.4 | 220 | 1.3 | 120 | Distillation column | NH3 | 91 | - |
| Comparative Example 1-5 | HDA | 1.05 | Urea | n-BuOH | 28.4 | 220 | 1.3 | 30 | Distillation column | NH3 | 91 | - |
| Comparative Example 1-6 | HDA | 1.05 | Urea | PhOH | 28.4 | 240 | 0.3 | 30 | Distillation column | NH3 | 93 | PhOH → PCP |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-29 | TAN | 1.04 | Urea | n-BuOH | 26.9 | 220 | 1.3 | 30 | Distillation column | NH3 | 92 | - |
| Example 1-30 | TAN | 1.04 | Urea | PhOH | 26.9 | 240 | 0.3 | 30 | Distillation column | NH3 | 95 | - |
| Example 1-31 | TAN | 1.04 | Urea | PhOH | 26.9 | 240 | 0.3 | 30 | Distillation column | NH3 | 95 | - |
| Example 1-32 | TAN | 1.04 | Urea | PhOH | 26.9 | 240 | 0.3 | 30 | Distillation column | NH3 | 95 | - |
| Example 1-33 | TAN | 1.04 | Urea | PhOH | 26.9 | 240 | 0.3 | 30 | Distillation column | NH3 | 95 | - |
| Example 1-34 | TAN | 1.04 | N-Butylurea | PhOH | 23.4 | 240 | 0.3 | 30 | Distillation column | N-Butylamine·NH3 | 90 | - |
| Example 1-35 | TAN | 1.04 | N,N'-Butylurea | PhOH | 19.9 | 240 | 0.3 | 30 | Distillation column | N-Butylamine | 89 | - |
| Example 1-36 | TAN | 1.04 | DPC | MeCN | 17.3 | 90 | 0.13 | 40 | Distillation column | PhOH | 95 | - |
| Example 1-37 | TAN | 1.04 | DPC | MeCN | 17.3 | 90 | 0.13 | 120 | Stirring tank | PhOH | 95 | - |
| Example 1-38 | TAN | 1.04 | DEC | MeCN | 23.3 | 90 | 0.13 | 80 | Stirring tank | EtOH | 93 | - |
| Example 1-39 | TAN | 0.69 | Urea | n-BuOH | 26.9 | 220 | 1.3 | 30 | Distillation column | NH3 | 92 | - |
| Example 1-40 | TAN | 0.69 | Urea | PhOH | 26.9 | 240 | 0.3 | 30 | Distillation column | NH3 | 95 | - |
| Example 1-41 | TAN | 0.69 | Urea | PhOH | 26.9 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-42 | TAN | 0.69 | DPC | MeCN | 26.9 | 50 | 0.01 | 30 | Distillation column | PhOH | 95 | - |
| Example 1-43 | TAN | 0.69 | Urea | PhOH | 26.9 | 240 | 0.3 | 30 | Distillation column | NH3 | 95 | - |
| Example 1-44 | TAN | 1.04 | DPC | MeCN | 23.4 | 50 | 0.01 | 30 | Distillation column (sieve tray) | PhOH | 95 | - |
| Example 1-45 | TAN | 1.04 | Urea | PhOH | 19.9 | 240 | 0.3 | 30 | Distillation column (sieve tray) | NH3 | 95 | - |
| Example 1-46 | TAN | 1.04 | DPC | MeCN | 17.3 | 50 | 0.01 | 30 | Distillation column (structured filling substance) | PhOH | 95 | - |
| Example 1-47 | TAN | 1.04 | Urea | PhOH | 17.3 | 240 | 0.3 | 30 | Distillation column (structured filling substance) | NH3 | 95 | - |
| Example 1-48 | TAN | 1.56 | DPC | MeCN | 60.9 | 50 | 0.01 | 30 | Distillation column | PhOH | 95 | - |
| Example 1-49 | TAN | 1.56 | Urea | PhOH | 67.3 | 240 | 0.3 | 30 | Distillation column | NH3 | 91 | - |
| Example 1-50 | TAN | 1.04 | DPC | MeCN | 26.9 | 50 | 0.01 | 30 | Distillation column | PhOH | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-51 | TAN | 1.04 | Urea | PhOH | 26.9 | 240 | 0.3 | 30 | Distillation column | NH3 | 95 | - |
| Example 1-52 | XDA | 1.23 | Urea | n-BuOH | 33.7 | 240 | 0.3 | 30 | Distillation column | NH3 | 92 | - |
| Example 1-53 | XDA | 1.23 | Urea | PhOH | 33.7 | 240 | 0.3 | 30 | Distillation column | NH3 | 94 | - |
| Example 1-54 | XDA | 1.23 | Urea | PhOH | 33.7 | 240 | 0.3 | 30 | Distillation column | NH3 | 94 | - |
| Example 1-55 | XDA | 1.23 | Urea | PhOH | 33.7 | 240 | 0.3 | 30 | Distillation column | NH3 | 94 | - |
| Example 1-56 | XDA | 1.23 | Urea | PhOH | 33.7 | 240 | 0.3 | 30 | Distillation column | NH3 | 94 | - |
| Example 1-57 | XDA | 1.23 | N-Butylurea | PhOH | 31.4 | 240 | 0.3 | 30 | Distillation column | N-Butylamine·NH3 | 92 | - |
| Example 1-58 | XDA | 1.23 | N,N'-Butvlurea | PhOH | 29.1 | 240 | 0.3 | 30 | Distillation column | N-Butylamine | 91 | - |
| Example 1-59 | XDA | 1.23 | DPC | MeCN | 27.3 | 90 | 0.13 | 40 | Distillation column | PhOH | 94 | - |
| Example 1-60 | XDA | 1.23 | DPC | MeCN | 31.3 | 90 | 0.13 | 120 | Stirring tank | PhOH | 94 | - |
| Example 1-61 | XDA | 1.23 | DEC | MeCN | 33.7 | 90 | 0.13 | 80 | Stirring tank | EtOH | 92 | - |
| Example 1-62 | XDA | 0.82 | Urea | n-BuOH | 33.7 | 220 | 1.3 | 30 | Distillation column | NH3 | 92 | - |
| Example 1-63 | XDA | 0.82 | Urea | PhOH | 33.7 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-64 | XDA | 0.82 | Urea | PhOH | 33.7 | 240 | 0.3 | 30 | Distillation column | NH3 | 95 | - |
| Example 1-65 | XDA | 0.82 | DPC | MeCN | 33.7 | 50 | 0.01 | 30 | Distillation column | PhOH | 95 | - |

[Table 1-2]

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-66 | XDA | 0.82 | Urea | PhOH | 33.7 | 240 | 0.3 | 30 | Distillation column | NH3 | 95 | - |
| Example 1-67 | XDA | 1.23 | DPC | MeCN | 33.7 | 50 | 0.01 | 30 | Distillation column (sieve tray) | PhOH | 95 | - |
| Example 1-68 | XDA | 1.23 | Urea | PhOH | 33.7 | 240 | 0.3 | 30 | Distillation column (sieve tray) | NH3 | 95 | - |
| Example 1-69 | XDA | 1.23 | DPC | MeCN | 33.7 | 50 | 0.01 | 30 | Distillation column (structured filling substance) | PhOH | 95 | - |
| Example 1-70 | XDA | 1.23 | Urea | PhOH | 33.7 | 240 | 0.3 | 30 | Distillation column (structured filling substance) | NH3 | 95 | - |
| Example 1-71 | XDA | 1.23 | DPC | MeCN | 33.7 | 50 | 0.01 | 30 | Distillation column | PhOH | 95 | - |
| Example 1-72 | XDA | 1.23 | Urea | PhOH | 33.7 | 240 | 0.3 | 30 | Distillation column | NH3 | 91 | - |
| Example 1-73 | XDA | 1.23 | DPC | MeCN | 33.7 | 50 | 0.01 | 30 | Distillation column | PhOH | 95 | - |
| Example 1-74 | XDA | 1.23 | Urea | PhOH | 33.7 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-75 | MDA | 1.79 | Urea | n-BuOH | 50.2 | 240 | 0.3 | 30 | Distillation column | NH3 | 91 | - |
| Example 1-76 | MDA | 1.79 | Urea | PhOH | 50.2 | 240 | 0.3 | 30 | Distillation column | NH3 | 94 | - |
| Example 1-77 | MDA | 1.79 | Urea | PhOH | 50.2 | 240 | 0.3 | 30 | Distillation column | NH3 | 94 | - |
| Example 1-78 | MDA | 1.79 | Urea | PhOH | 50.2 | 240 | 0.3 | 30 | Distillation column | NH3 | 94 | - |
| Example 1-79 | MDA | 1.79 | Urea | PhOH | 50.2 | 240 | 0.3 | 30 | Distillation column | NH3 | 94 | - |
| Example 1-80 | MDA | 1.79 | N-Butylurea | PhOH | 47.9 | 240 | 0.3 | 30 | Distillation column | N-Butylamine· NH3 | 92 | - |
| Example 1-81 | MDA | 1.79 | N,N'-Butylurea | PhOH | 45.5 | 240 | 0.3 | 30 | Distillation column | N-Butylamine | 91 | - |
| Example 1-82 | MDA | 1.79 | DPC | MeCN | 43.8 | 90 | 0.13 | 40 | Distillation column | PhOH | 95 | - |
| Example 1-83 | MDA | 1.79 | DPC | MeCN | 47.8 | 90 | 0.13 | 120 | Stirring tank | PhOH | 95 | - |
| Example 1-84 | MDA | 1.79 | DEC | MeCN | 50.2 | 90 | 0.13 | 80 | Stirring tank | EtOH | 92 | - |
| Example 1-85 | MDA | 1.19 | Urea | n-BuOH | 50.2 | 220 | 1.3 | 30 | Distillation column | NH3 | 92 | - |
| Example 1-86 | MDA | 1.19 | Urea | PhOH | 50.2 | 240 | 0.3 | 30 | Distillation column | NH3 | 95 | - |
| Example 1-87 | MDA | 1.19 | Urea | PhOH | 50.2 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-88 | MDA | 1.19 | DPC | MeCN | 50.2 | 50 | 0.01 | 30 | Distillation column | PhOH | 95 | - |
| Example 1-89 | MDA | 1.19 | Urea | PhOH | 50.2 | 240 | 0.3 | 30 | Distillation column | NH3 | 95 | - |
| Example 1-90 | MDA | 1.79 | DPC | MeCN | 50.2 | 50 | 0.01 | 30 | Distillation column (sieve tray) | PhOH | 95 | - |
| Example 1-91 | MDA | 1.79 | Urea | PhOH | 50.2 | 240 | 0.3 | 30 | Distillation column (sieve tray) | NH3 | 95 | - |
| Example 1-92 | MDA | 1.79 | DPC | MeCN | 50.2 | 50 | 0.01 | 30 | Distillation column (structured filling substance) | PhOH | 95 | - |
| Example 1-93 | MDA | 1.79 | Urea | PhOH | 50.2 | 240 | 0.3 | 30 | Distillation column (structured filling substance) | NH3 | 95 | - |
| Example 1-94 | MDA | 1.79 | DPC | MeCN | 50.2 | 50 | 0.01 | 30 | Distillation column | PhOH | 95 | - |
| Example 1-95 | MDA | 1.79 | Urea | PhOH | 50.2 | 240 | 0.3 | 30 | Distillation column | NH3 | 91 | - |
| Example 1-96 | MDA | 1.79 | DPC | MeCN | 50.2 | 50 | 0.01 | 30 | Distillation column | PhOH | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-97 | MDA | 1.79 | Urea | PhOH | 50.2 | 240 | 0.3 | 30 | Distillation column | NH3 | 95 | - |
| Example 1-98 | HMDA | 1.90 | Urea | n-BuOH | 53.4 | 240 | 0.3 | 30 | Distillation column | NH3 | 92 | - |
| Example 1-99 | HMDA | 1.90 | Urea | PhOH | 53.4 | 240 | 0.3 | 30 | Distillation column | NH3 | 94 | - |
| Example 1-100 | HMDA | 1.90 | Urea | PhOH | 53.4 | 240 | 0.3 | 30 | Distillation column | NH3 | 94 | - |
| Example 1-101 | HMDA | 1.90 | Urea | PhOH | 53.4 | 240 | 0.3 | 30 | Distillation column | NH3 | 94 | - |
| Example 1-102 | HMDA | 1.90 | Urea | PhOH | 53.4 | 240 | 0.3 | 30 | Distillation column | NH3 | 94 | - |
| Example 1-103 | HMDA | 1.90 | N-Butylurea | PhOH | 51.1 | 240 | 0.3 | 30 | Distillation column | N-Butylamine·NH3 | 92 | - |
| Example 1-104 | HMDA | 1.90 | N,N'-Butylurea | PhOH | 48.8 | 240 | 0.3 | 30 | Distillation column | N-Butylamine | 91 | - |
| Example 1-105 | HMDA | 1.90 | DPC | MeCN | 47.0 | 90 | 0.13 | 40 | Distillation column | PhOH | 95 | - |
| Example 1-106 | HMDA | 1.90 | DPC | MeCN | 51.0 | 90 | 0.13 | 120 | Stirring tank | PhOH | 94 | - |
| Example 1-107 | HMDA | 1.90 | DPC | MeCN | 53.4 | 90 | 0.13 | 120 | Stirring tank | PhOH | 92 | - |
| Example 1-108 | HMDA | 1.27 | Urea | n-BuOH | 53.4 | 220 | 1.3 | 30 | Distillation column | NH3 | 92 | - |
| Example 1-109 | HMDA | 1.27 | Urea | PhOH | 53.4 | 240 | 0.3 | 30 | Distillation column | NH3 | 95 | - |

(continued)

| | Step (1) | | | | | | | | | | | | Step (1b) |
|---|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-110 | HMDA | 1.27 | Urea | PhOH | 53.4 | 240 | 0.3 | 30 | Distillation column | NH3 | 95 | - |
| Example 1-111 | HMDA | 1.27 | DPC | MeCN | 53.4 | 50 | 0.01 | 30 | Distillation column | PhOH | 95 | - |
| Example 1-112 | HMDA | 1.27 | Urea | PhOH | 53.4 | 240 | 0.3 | 30 | Distillation column | NH3 | 95 | - |
| Example 1-113 | HMDA | 1.90 | DPC | MeCN | 53.4 | 50 | 0.01 | 30 | Distillation column (sieve tray) | PhOH | 95 | - |
| Example 1-114 | HMDA | 1.90 | Urea | PhOH | 53.4 | 240 | 0.3 | 30 | Distillation column (sieve tray) | NH3 | 95 | - |
| Example 1-115 | HMDA | 1.90 | DPC | MeCN | 53.4 | 50 | 0.01 | 30 | Distillation column (structured filling substance) | PhOH | 95 | - |
| Example 1-116 | HMDA | 1.90 | Urea | PhOH | 53.4 | 240 | 0.3 | 30 | Distillation column (structured filling substance) | NH3 | 95 | - |
| Example 1-117 | HMDA | 1.90 | DPC | MeCN | 53.4 | 50 | 0.01 | 30 | Distillation column | PhOH | 95 | - |
| Example 1-118 | HMDA | 1.90 | Urea | PhOH | 53.4 | 240 | 0.3 | 30 | Distillation column | NH3 | 91 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-119 | HMDA | 1.90 | DPC | MeCN | 53.4 | 50 | 0.01 | 30 | Distillation column | PhOH | 95 | - |
| Example 1-120 | HMDA | 1.90 | Urea | PhOH | 53.4 | 240 | 0.3 | 30 | Distillation column | NH3 | 95 | - |
| Example 1-121 | IPDA | 1.54 | Urea | n-BuOH | 42.7 | 240 | 0.3 | 30 | Distillation column | NH3 | 91 | - |
| Example 1-122 | IPDA | 1.54 | Urea | PhOH | 42.7 | 240 | 0.3 | 30 | Distillation column | NH3 | 93 | - |
| Example 1-123 | IPDA | 1.54 | Urea | PhOH | 42.7 | 240 | 0.3 | 30 | Distillation column | NH3 | 93 | - |
| Example 1-124 | IPDA | 1.54 | Urea | PhOH | 42.7 | 240 | 0.3 | 30 | Distillation column | NH3 | 93 | - |
| Example 1-125 | IPDA | 1.54 | Urea | PhOH | 42.7 | 240 | 0.3 | 30 | Distillation column | NH3 | 93 | - |
| Example 1-126 | IPDA | 1.54 | N-Butylurea | PhOH | 40.4 | 240 | 0.3 | 30 | Distillation column | N-Butylamine·NH3 | 92 | - |
| Example 1-127 | IPDA | 1.54 | N,N'-Butylurea | PhOH | 38.0 | 240 | 0.3 | 30 | Distillation column | N-Butylamine | 91 | - |
| Example 1-128 | IPDA | 1.54 | DPC | MeCN | 36.3 | 90 | 0.13 | 40 | Distillation column | PhOH | 95 | - |
| Example 1-129 | IPDA | 1.54 | DPC | MeCN | 40.3 | 90 | 0.13 | 120 | Stirring tank | PhOH | 94 | - |
| Example 1-130 | IPDA | 1.54 | DPC | MeCN | 42.7 | 90 | 0.13 | 120 | Stirring tank | PhOH | 92 | - |
| Example 1-131 | IPDA | 1.02 | Urea | n-BuOH | 42.7 | 220 | 1.3 | 30 | Distillation column | NH3 | 92 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-132 | IPDA | 1.02 | Urea | PhOH | 42.7 | 240 | 0.3 | 30 | Distillation column | NH3 | 95 | - |
| Example 1-133 | IPDA | 1.02 | Urea | PhOH | 42.7 | 240 | 0.3 | 30 | Distillation column | NH3 | 95 | - |
| Example 1-134 | IPDA | 1.02 | DPC | MeCN | 42.7 | 50 | 0.01 | 30 | Distillation column | PhOH | 95 | - |
| Example 1-135 | IPDA | 1.02 | Urea | PhOH | 42.7 | 240 | 0.3 | 30 | Distillation column | NH3 | 95 | - |
| Example 1-136 | IPDA | 1.54 | DPC | MeCN | 42.7 | 50 | 0.01 | 30 | Distillation column (sieve tray) | PhOH | 95 | - |

[Table 1-3]

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-137 | IPDA | 1.54 | Urea | PhOH | 42.7 | 240 | 0.3 | 30 | Distillation column (sieve tray) | NH3 | 95 | - |
| Example 1-138 | IPDA | 1.54 | DPC | MeCN | 42.7 | 50 | 0.01 | 30 | Distillation column (structured filling substance) | PhOH | 95 | - |
| Example 1-139 | IPDA | 1.54 | Urea | PhOH | 42.7 | 240 | 0.3 | 30 | Distillation column (structured filling substance) | NH3 | 95 | - |
| Example 1-140 | IPDA | 1.54 | DPC | MeCN | 42.7 | 50 | 0.01 | 30 | Distillation column | PhOH | 95 | - |
| Example 1-141 | IPDA | 1.54 | Urea | PhOH | 42.7 | 240 | 0.3 | 30 | Distillation column | NH3 | 91 | - |
| Example 1-142 | IPDA | 1.54 | DPC | MeCN | 42.7 | 50 | 0.01 | 30 | Distillation column | PhOH | 95 | - |
| Example 1-143 | IPDA | 1.54 | Urea | PhOH | 42.7 | 240 | 0.3 | 30 | Distillation column | NH3 | 95 | - |
| Example 1-144 | TAH | 0.79 | Urea | n-BuOH | 19.5 | 220 | 1.3 | 30 | Distillation column | NH3 | 92 | - |
| Example 1 -145 | TAH | 0.79 | Urea | PhOH | 19.5 | 240 | 0.3 | 30 | Distillation column | NH3 | 94 | - |
| Example 1-146 | TAH | 0.79 | Urea | PhOH | 19.5 | 240 | 0.3 | 30 | Distillation column | NH3 | 94 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-147 | TAH | 0.79 | Urea | PhOH | 19.5 | 240 | 0.3 | 30 | Distillation column | NH3 | 94 | - |
| Example 1 -148 | TAH | 0.79 | Urea | PhOH | 19.5 | 240 | 0.3 | 30 | Distillation column | NH3 | 94 | - |
| Example 1-149 | TAH | 0.79 | N-Butylurea | PhOH | 16.0 | 240 | 0.3 | 30 | Distillation column | N-Butylamine·NH3 | 92 | - |
| Example 1-150 | TAH | 0.79 | N,N'-Butylurea | PhOH | 12.5 | 240 | 0.3 | 30 | Distillation column | N-Butylamine | 92 | - |
| Example 1-151 | TAH | 0.79 | DPC | MeCN | 9.9 | 90 | 0.13 | 40 | Distillation column | PhOH | 95 | - |
| Example 1-152 | TAH | 0.79 | DPC | MeCN | 9.9 | 90 | 0.13 | 120 | Stirring tank | PhOH | 94 | - |
| Example 1-153 | TAH | 0.79 | DEC | MeCN | 15.9 | 90 | 0.13 | 80 | Stirring tank | EtOH | 92 | - |
| Example 1 -154 | TAH | 0.53 | Urea | n-BuOH | 19.5 | 220 | 1.3 | 30 | Distillation column | NH3 | 92 | - |
| Example 1-155 | TAH | 0.53 | Urea | PhOH | 19.5 | 240 | 0.3 | 30 | Distillation column | NH3 | 95 | - |
| Example 1-156 | TAH | 0.53 | Urea | PhOH | 19.5 | 240 | 0.3 | 30 | Distillation column | NH3 | 95 | - |
| Example 1-157 | TAH | 0.53 | DPC | MeCN | 19.5 | 50 | 0.01 | 30 | Distillation column | PhOH | 95 | - |
| Example 1-158 | TAH | 0.53 | Urea | PhOH | 19.5 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-159 | TAH | 0.79 | DPC | MeCN | 19.5 | 50 | 0.01 | 30 | Distillation column (sieve tray) | PhOH | 95 | - |
| Example 1-160 | TAH | 0.79 | Urea | PhOH | 19.5 | 240 | 0.3 | 30 | Distillation column (sieve tray) | NH3 | 95 | - |
| Example 1-161 | TAH | 0.79 | DPC | MeCN | 19.5 | 50 | 0.01 | 30 | Distillation column (structured filling substance) | PhOH | 95 | - |
| Example 1-162 | TAH | 0.79 | Urea | PhOH | 19.5 | 240 | 0.3 | 30 | Distillation column (structured filling substance) | NH3 | 95 | - |
| Example 1-163 | TAH | 0.79 | DPC | MeCN | 19.5 | 50 | 0.01 | 30 | Distillation column | PhOH | 95 | - |
| Example 1-164 | TAH | 0.79 | Urea | PhOH | 19.5 | 240 | 0.3 | 30 | Distillation column | NH3 | 91 | - |
| Example 1-165 | TAH | 0.79 | DPC | MeCN | 19.5 | 50 | 0.01 | 30 | Distillation column | PhOH | 95 | - |
| Example 1-166 | TAH | 0.79 | Urea | PhOH | 19.5 | 240 | 0.3 | 30 | Distillation column | NH3 | 95 | - |
| Example 1-167 | TAU | 1.21 | Urea | n-BuOH | 31.9 | 220 | 1.3 | 30 | Distillation column | NH3 | 92 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-168 | TAU | 1.21 | Urea | PhOH | 31.9 | 240 | 0.3 | 30 | Distillation column | NH3 | 94 | - |
| Example 1-169 | TAU | 1.21 | Urea | PhOH | 31.9 | 240 | 0.3 | 30 | Distillation column | NH3 | 94 | - |
| Example 1-170 | TAU | 1.21 | Urea | PhOH | 31.9 | 240 | 0.3 | 30 | Distillation column | NH3 | 94 | - |
| Example 1-171 | TAU | 1.21 | Urea | PhOH | 31.9 | 240 | 0.3 | 30 | Distillation column | NH3 | 94 | - |
| Example 1 -172 | TAU | 1.21 | N-Butylurea | PhOH | 28.4 | 240 | 0.3 | 30 | Distillation column | N-Butylamine·NH3 | 92 | - |
| Example 1-173 | TAU | 1.21 | N,N'-Butylurea | PhOH | 24.9 | 240 | 0.3 | 30 | Distillation column | N-Butylamine | 92 | - |
| Example 1-174 | TAU | 1.21 | DPC | MeCN | 22.3 | 90 | 0.13 | 40 | Distillation column | PhOH | 95 | - |
| Example 1-175 | TAU | 1.21 | DPC | MeCN | 22.3 | 90 | 0.13 | 120 | Stirring tank | PhOH | 94 | - |
| Example 1-176 | TAU | 1.21 | DEC | MeCN | 28.3 | 90 | 0.13 | 80 | Stirring tank | EtOH | 92 | - |
| Example 1-177 | TAU | 0.81 | Urea | n-BuOH | 31.9 | 220 | 1.3 | 30 | Distillation column | NH3 | 92 | - |
| Example 1-178 | TAU | 0.81 | Urea | PhOH | 31.9 | 240 | 0.3 | 30 | Distillation column | NH3 | 95 | - |
| Example 1-179 | TAU | 0.81 | Urea | PhOH | 31.9 | 240 | 0.3 | 30 | Distillation column | NH3 | 95 | - |
| Example 1-180 | TAU | 0.81 | DPC | MeCN | 31.9 | 50 | 0.01 | 30 | Distillation column | PhOH | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-181 | TAU | 0.81 | Urea | PhOH | 31.9 | 240 | 0.3 | 30 | Distillation column | NH3 | 95 | - |
| Example 1-182 | TAU | 1.21 | DPC | MeCN | 31.9 | 50 | 0.01 | 30 | Distillation column (sieve tray) | PhOH | 95 | - |
| Example 1-183 | TAU | 1.21 | Urea | PhOH | 31.9 | 240 | 0.3 | 30 | Distillation column (sieve tray) | NH3 | 95 | - |
| Example 1-184 | TAU | 1.21 | DPC | MeCN | 31.9 | 50 | 0.01 | 30 | Distillation column (structured filling substance) | PhOH | 95 | - |
| Example 1-185 | TAU | 1.21 | Urea | PhOH | 31.9 | 240 | 0.3 | 30 | Distillation column (structured filling substance) | NH3 | 95 | - |
| Example 1-186 | TAU | 1.21 | DPC | MeCN | 31.9 | 50 | 0.01 | 30 | Distillation column | PhOH | 95 | - |
| Example 1-187 | TAU | 1.21 | Urea | PhOH | 31.9 | 240 | 0.3 | 30 | Distillation column | NH3 | 91 | - |
| Example 1-188 | TAU | 1.21 | DPC | MeCN | 31.9 | 50 | 0.01 | 30 | Distillation column | PhOH | 95 | - |
| Example 1-189 | TAU | 1.21 | Urea | PhOH | 31.9 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-190 | TAB | 0.74 | Urea | n-BuOH | 18.1 | 220 | 1.3 | 30 | Distillation column | NH3 | 91 | - |
| Example 1-191 | TAB | 0.74 | Urea | PhOH | 18.1 | 240 | 0.3 | 30 | Distillation column | NH3 | 94 | - |
| Example 1-192 | TAB | 0.74 | Urea | PhOH | 18.1 | 240 | 0.3 | 30 | Distillation column | NH3 | 94 | - |
| Example 1-193 | TAB | 0.74 | Urea | PhOH | 18.1 | 240 | 0.3 | 30 | Distillation column | NH3 | 94 | - |
| Example 1-194 | TAB | 0.74 | Urea | PhOH | 18.1 | 240 | 0.3 | 30 | Distillation column | NH3 | 94 | - |
| Example 1-195 | TAB | 0.74 | N-Butylurea | PhOH | 14.6 | 240 | 0.3 | 30 | Distillation column | N-Butylamine·NH3 | 92 | - |
| Example 1-196 | TAB | 0.74 | N,N'-Butylurea | PhOH | 11.1 | 240 | 0.3 | 30 | Distillation column | N-Butylamine | 91 | - |
| Example 1-197 | TAB | 0.74 | DPC | MeCN | 8.5 | 90 | 0.13 | 40 | Distillation column | PhOH | 95 | - |
| Example 1-198 | TAB | 0.74 | DPC | MeCN | 8.5 | 90 | 0.13 | 120 | Stirring tank | PhOH | 94 | - |
| Example 1-199 | TAB | 0.74 | DEC | MeCN | 14.4 | 90 | 0.13 | 80 | Stirring tank | EtOH | 92 | - |
| Example 1-200 | TAB | 0.49 | Urea | n-BuOH | 18.1 | 220 | 1.3 | 30 | Distillation column | NH3 | 92 | - |
| Example 1-201 | TAB | 0.49 | Urea | PhOH | 18.1 | 240 | 0.3 | 30 | Distillation column | NH3 | 95 | - |
| Example 1-202 | TAB | 0.49 | Urea | PhOH | 18.1 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-203 | TAB | 0.49 | DPC | MeCN | 18.1 | 50 | 0.01 | 30 | Distillation column | PhOH | 95 | - |
| Example 1-204 | TAB | 0.49 | Urea | PhOH | 18.1 | 240 | 0.3 | 30 | Distillation column | NH3 | 95 | - |
| Example 1-205 | TAB | 0.74 | DPC | MeCN | 18.1 | 50 | 0.01 | 30 | Distillation column (sieve tray) | PhOH | 95 | - |
| Example 1-206 | TAB | 0.74 | Urea | PhOH | 18.1 | 240 | 0.3 | 30 | Distillation column (sieve tray) | NH3 | 95 | - |
| Example 1-207 | TAB | 0.74 | DPC | MeCN | 18.1 | 50 | 0.01 | 30 | Distillation column (structured filling substance) | PhOH | 95 | - |

[Table 1-4]

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-208 | TAB | 0.74 | Urea | PhOH | 18.1 | 240 | 0.3 | 30 | Distillation column (structured filling substance) | NH3 | 95 | - |
| Example 1-209 | TAB | 0.74 | DPC | MeCN | 18.1 | 50 | 0.01 | 30 | Distillation column | PhOH | 95 | - |
| Example 1-210 | TAB | 0.74 | Urea | PhOH | 18.1 | 240 | 0.3 | 30 | Distillation column | NH3 | 91 | - |
| Example 1-211 | TAB | 0.74 | DPC | MeCN | 18.1 | 50 | 0.01 | 30 | Distillation column | PhOH | 95 | - |
| Example 1-212 | TAB | 0.74 | Urea | PhOH | 18.1 | 240 | 0.3 | 30 | Distillation column | NH3 | 95 | - |
| Example 1-213 | TAMB | 0.83 | Urea | n-BuOH | 20.5 | 220 | 1.3 | 30 | Distillation column | NH3 | 91 | - |
| Example 1-214 | TAMB | 0.83 | Urea | PhOH | 20.5 | 240 | 0.3 | 30 | Distillation column | NH3 | 94 | - |
| Example 1-215 | TAMB | 0.83 | Urea | PhOH | 20.5 | 240 | 0.3 | 30 | Distillation column | NH3 | 94 | - |
| Example 1-216 | TAMB | 0.83 | Urea | PhOH | 20.5 | 240 | 0.3 | 30 | Distillation column | NH3 | 94 | - |
| Example 1-217 | TAMB | 0.83 | Urea | PhOH | 20.5 | 240 | 0.3 | 30 | Distillation column | NH3 | 94 | - |
| Example 1-218 | TAMB | 0.83 | N-Butylurea | PhOH | 17.1 | 240 | 0.3 | 30 | Distillation column | N-Butylamine·NH3 | 92 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-219 | TAMB | 0.83 | N,N'-Butylurea | PhOH | 13.6 | 240 | 0.3 | 30 | Distillation column | N-Butylamine | 91 | - |
| Example 1-220 | TAMB | 0.83 | DPC | MeCN | 10.9 | 90 | 0.13 | 40 | Distillation column | PhOH | 95 | - |
| Example 1-221 | TAMB | 0.83 | DPC | MeCN | 10.9 | 90 | 0.13 | 120 | Stirring tank | PhOH | 94 | - |
| Example 1-222 | TAMB | 0.83 | DEC | MeCN | 16.9 | 90 | 0.13 | 80 | Stirring tank | EtOH | 92 | - |
| Example 1-223 | TAMB | 0.55 | Urea | n-BuOH | 20.5 | 220 | 1.3 | 30 | Distillation column | NH3 | 92 | - |
| Example 1-224 | TAMB | 0.55 | Urea | PhOH | 20.5 | 240 | 0.3 | 30 | Distillation column | NH3 | 95 | - |
| Example 1-225 | TAMB | 0.55 | Urea | PhOH | 20.5 | 240 | 0.3 | 30 | Distillation column | NH3 | 95 | - |
| Example 1-226 | TAMB | 0.55 | DPC | MeCN | 20.5 | 50 | 0.01 | 30 | Distillation column | PhOH | 95 | - |
| Example 1-227 | TAMB | 0.55 | Urea | PhOH | 20.5 | 240 | 0.3 | 30 | Distillation column | NH3 | 95 | - |
| Example 1-228 | TAMB | 0.83 | DPC | MeCN | 20.5 | 50 | 0.01 | 30 | Distillation column(sieve tray) | PhOH | 95 | - |
| Example 1-229 | TAMB | 0.83 | Urea | PhOH | 20.5 | 240 | 0.3 | 30 | Distillation column(sieve tray) | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-230 | TAMB | 0.83 | DPC | MeCN | 20.5 | 50 | 0.01 | 30 | Distillation column (structured filling substance) | PhOH | 95 | - |
| Example 1-231 | TAMB | 0.83 | Urea | PhOH | 20.5 | 240 | 0.3 | 30 | Distillation column (structured filling substance) | NH3 | 95 | - |
| Example 1-232 | TAMB | 0.83 | DPC | MeCN | 20.5 | 50 | 0.01 | 30 | Distillation column | PhOH | 95 | - |
| Example 1-233 | TAMB | 0.83 | Urea | PhOH | 20.5 | 240 | 0.3 | 30 | Distillation column | NH3 | 91 | - |
| Example 1-234 | TAMB | 0.83 | DPC | MeCN | 20.5 | 50 | 0.01 | 30 | Distillation column | PhOH | 95 | - |
| Example 1-235 | TAMB | 0.83 | Urea | PhOH | 20.5 | 240 | 0.3 | 30 | Distillation column | NH3 | 95 | - |
| Example 1-236 | TAPMB | 1.50 | Urea | n-BuOH | 40.4 | 220 | 1.3 | 30 | Distillation column | NH3 | 92 | - |
| Example 1-237 | TAPMB | 1.50 | Urea | PhOH | 40.4 | 240 | 0.3 | 30 | Distillation column | NH3 | 94 | - |
| Example 1-238 | TAPMB | 1.50 | Urea | PhOH | 40.4 | 240 | 0.3 | 30 | Distillation column | NH3 | 95 | - |
| Example 1-239 | TAPMB | 1.50 | Urea | PhOH | 40.4 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-240 | TAPMB | 1.50 | Urea | PhOH | 40.4 | 240 | 0.3 | 30 | Distillation column | NH3 | 95 | - |
| Example 1-241 | TAPMB | 1.50 | N-Butylurea | PhOH | 36.9 | 240 | 0.3 | 30 | Distillation column | N-Butylamine· NH3 | 93 | - |
| Example 1-242 | TAPMB | 1.50 | N,N'-Butylurea | PhOH | 33.4 | 240 | 0.3 | 30 | Distillation column | N-Butylamine | 91 | - |
| Example 1-243 | TAPMB | 1.50 | DPC | MeCN | 30.8 | 90 | 0.13 | 40 | Distillation column | PhOH | 95 | - |
| Example 1-244 | TAPMB | 1.50 | DPC | MeCN | 30.8 | 90 | 0.13 | 120 | Stirring tank | PhOH | 94 | - |
| Example 1-245 | TAPMB | 1.50 | DEC | MeCN | 36.8 | 90 | 0.13 | 80 | Stirring tank | EtOH | 92 | - |
| Example 1-246 | TAPMB | 1.00 | Urea | n-BuOH | 40.4 | 220 | 1.3 | 30 | Distillation column | NH3 | 92 | - |
| Example 1-247 | TAPMB | 1.00 | Urea | PhOH | 40.4 | 240 | 0.3 | 30 | Distillation column | NH3 | 95 | - |
| Example 1-248 | TAPMB | 1.00 | Urea | PhOH | 40.4 | 240 | 0.3 | 30 | Distillation column | NH3 | 95 | - |
| Example 1-249 | TAPMB | 1.00 | DPC | MeCN | 40.4 | 50 | 0.01 | 30 | Distillation column | PhOH | 95 | - |
| Example 1-250 | TAPMB | 1.00 | Urea | PhOH | 40.4 | 240 | 0.3 | 30 | Distillation column | NH3 | 95 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-251 | TAPMB | 1.50 | DPC | MeCN | 40.4 | 50 | 0.01 | 30 | Distillation column (sieve tray) | PhOH | 95 | - |
| Example 1-252 | TAPMB | 1.50 | Urea | PhOH | 40.4 | 240 | 0.3 | 30 | Distillation column (sieve tray) | NH3 | 95 | - |
| Example 1-253 | TAPMB | 1.50 | DPC | MeCN | 40.4 | 50 | 0.01 | 30 | Distillation column (structured filling substance) | PhOH | 95 | - |
| Example 1-254 | TAPMB | 1.50 | Urea | PhOH | 40.4 | 240 | 0.3 | 30 | Distillation column (structured filling substance) | NH3 | 95 | - |
| Example 1-255 | TAPMB | 1.50 | DPC | MeCN | 40.4 | 50 | 0.01 | 30 | Distillation column | PhOH | 95 | - |
| Example 1-256 | TAPMB | 1.50 | Urea | PhOH | 40.4 | 240 | 0.3 | 30 | Distillation column | NH3 | 91 | - |
| Example 1-257 | TAPMB | 1.50 | DPC | MeCN | 40.4 | 50 | 0.01 | 30 | Distillation column | PhOH | 95 | - |
| Example 1-258 | TAPMB | 1.50 | Urea | PhOH | 40.4 | 240 | 0.3 | 30 | Distillation column | NH3 | 95 | - |
| Example 1-259 | LTA | 0.88 | DPC | MeCN | 12.6 | 90 | 0.13 | 40 | Distillation column | PhOH | 94 | - |

(continued)

| | Step (1) | | | | | | | | | | | Step (1b) |
|---|---|---|---|---|---|---|---|---|---|---|---|---|
| | Primary amine compound | Carbonic acid derivative | | Solvent | | Temperature | Pressure | Average retention time | Reaction form | By-product low-boiling-point compound | Yield | Hydroxy compound |
| | Type | Amount kg | Type | Type | Amount kg | °C | MPaG | min | | | % | |
| Example 1-260 | LTA | 0.88 | DPC | MeCN | 12.6 | 90 | 0.13 | 120 | Stirring tank | PhOH | 94 | - |
| Example 1-261 | LTA | 0.88 | DEC | MeCN | 18.5 | 90 | 0.13 | 120 | Stirring tank | EtOH | 92 | - |
| Example 1-262 | ODA | 1.45 | DPC | MeCN | 33.7 | 90 | 0.13 | 40 | Distillation column | PhOH | 94 | - |
| Example 1-263 | ODA | 1.45 | DPC | MeCN | 33.7 | 90 | 0.13 | 120 | Stirring tank | PhOH | 94 | - |
| Example 1-264 | ODA | 1.45 | DEC | MeCN | 37.7 | 90 | 0.13 | 120 | Stirring tank | EtOH | 93 | - |
| Example 1-265 | LDA | 1.57 | DPC | MeCN | 37.4 | 90 | 0.13 | 40 | Distillation column | PhOH | 94 | - |
| Example 1-266 | LDA | 1.57 | DPC | MeCN | 37.4 | 90 | 0.13 | 120 | Stirring tank | PhOH | 94 | - |
| Example 1-267 | LDA | 1.57 | DEC | MeCN | 41.4 | 90 | 0.13 | 120 | Stirring tank | EtOH | 92 | - |

[Table 1-5]

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-1 | DBC | 260 | 800 | 15 | Distillation column | n-BuOH | 89 | 130 | 20 | 25 | DBC |
| Example 1-2 | DiPrC | 260 | 800 | 15 | Distillation column | n-BuOH | 89 | 100 | 20 | 25 | DiPrC |
| Example 1-3 | DBC | 260 | 800 | 15 | Distillation column | n-BuOH | 88 | 130 | 20 | 25 | DBC |
| Example 1-4 | DBC·m - Xy | 260 | 800 | 15 | Distillation column | n-BuOH | 90 | 130 | 20 | 25 | DBC·m - Xy |
| Example 1-5 | DBC | 260 | 800 | 15 | Distillation column | n-BuOH | 89 | 130 | 20 | 25 | DBC |
| Example 1-6 | DBC | 260 | 800 | 15 | Distillation column | n-BuOH | 89 | 130 | 20 | 25 | DBC |
| Example 1-7 | DBC | 260 | 800 | 10 | FF | n-BuOH | 86 | 130 | 20 | 25 | DBC |
| Example 1-8 | DBC | 260 | 800 | 15 | FF × 2 | n-BuOH | 92 | 130 | 20 | 25 | DBC |
| Example 1-9 | DBC | 260 | 800 | 15 | FF × 2 | n-BuOH | 94 | 130 | 20 | 25 | DBC |
| Example 1-10 | DPC·m - Xy | 245 | 20 | 10 | FF | PhOH | 87 | 250 | 20 | 25 | DPC·m - Xy |
| Example 1-11 | DPC·m - Xy | 245 | 20 | 15 | FF × 2 | PhOH | 95 | 250 | 20 | 25 | DPC·m - Xy |
| Example 1-12 | DBC·m - Xy | 245 | 20 | 15 | Distillation column | PhOH | 90 | 250 | 20 | 25 | DBC·m - Xy |
| Example 1-13 | DBC·m - Xy | 245 | 20 | 15 | Distillation column | PhOH | 90 | 250 | 20 | 25 | DBC·m - Xy |
| Example 1-14 | DBC | 245 | 600 | 90 | Distillation column | PhOH | 92 | 130 | 20 | 25 | DBC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-15 | DBC | 260 | 800 | 10 | Thin film | n-BuOH | 89 | 130 | 20 | 25 | DBC |
| Example 1-16 | DPC | 250 | 1 | 15 | FF | PhOH | 91 | 250 | 20 | 25 | DPC |
| Example 1-17 | DPC·m - Xy | 245 | 20 | 10 | FF | PhOH | 87 | 250 | 20 | 25 | DPC·m - Xy |
| Example 1-18 | DPC·m - Xy | 245 | 20 | 15 | FF × 2 | PhOH | 95 | 250 | 20 | 25 | DPC·m - Xy |
| Example 1-19 | DBC·m - Xy | 245 | 20 | 15 | Distillation column | PhOH | 90 | 250 | 20 | 25 | DBC·m - Xy |
| Example 1-20 | DBC | 245 | 600 | 90 | Distillation column | PhOH | 92 | 130 | 20 | 25 | DBC |
| Example 1-21 | DBC·m - Xy | 245 | 20 | 15 | Distillation column (sieve tray) | PhOH | 89 | 250 | 20 | 25 | DBC·m - Xy |
| Example 1-22 | DBC | 245 | 600 | 90 | Distillation column (sieve tray) | PhOH | 88 | 130 | 20 | 25 | DBC |
| Example 1-23 | DBC·m - Xy | 245 | 20 | 15 | Distillation column | PhOH | 89 | 250 | 20 | 25 | DBC·m - Xy |
| | | | | | (structured filling substance) | | | | | | |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-24 | DBC | 245 | 600 | 90 | Distillation column (structured filling substance) | PhOH | 88 | 130 | 20 | 25 | DBC |
| Example 1-25 | DBC·m - Xy | 245 | 20 | 15 | Distillation column | PhOH | 90 | 250 | 20 | 25 | DBC·m - Xy |
| Example 1-26 | DBC | 245 | 600 | 90 | Distillation column | PhOH | 92 | 130 | 20 | 25 | DBC |
| Example 1-27 | DBC·m - Xy | 245 | 20 | 15 | Distillation column | PhOH | 90 | 250 | 20 | 25 | DBC·m - Xy |
| Example 1-28 | DBC | 245 | 600 | 90 | Distillation column | PhOH | 92 | 130 | 20 | 25 | DBC |
| Comparative Example 1-1 | - | - | - | - | - | - | - | - | - | - | - |
| Comparative Example 1-2 | DBC | 260 | 1200 | 15 | Distillation column | n-BuOH | 13 | - | - | - | - |
| Comparative Example 1-3 | DBC | 260 | 800 | 15 | Distillation column | n-BuOH | 88 | - | - | - | - |
| Comparative Example 1-4 | DBC | 260 | 800 | 15 | Distillation column | n-BuOH | 88 | 130 | 20 | 25 | DBC |
| Comparative Example 1-5 | PhOH | 245 | 400 | 15 | Distillation column | PhOH | 5 | - | - | - | - |
| Comparative Example 1-6 | PCP | 250 | 1 | 15 | FF $\times$ 2 | HDI·PCP·BT | 86 | 250 | 20 | 25 | (BT) |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-29 | DBC | 260 | 800 | 15 | Distillation column | n-BuOH | 86 | 130 | 20 | 25 | DBC |
| Example 1-30 | DPC | 245 | 20 | 15 | Distillation column | PhOH | 96 | 250 | 20 | 25 | DPC |
| Example 1-31 | DPC | 245 | 20 | 10 | FF | PhOH | 86 | 130 | 20 | 25 | DPC |
| Example 1-32 | DPC | 245 | 20 | 15 | FF × 2 | PhOH | 94 | 130 | 20 | 25 | DPC |
| Example 1-33 | DPC | 245 | 20 | 10 | Thin film | PhOH | 92 | 130 | 20 | 25 | DPC |
| Example 1-34 | DPC | 245 | 20 | 15 | Distillation column | PhOH | 96 | 245 | 20 | 25 | DPC |
| Example 1-35 | DPC | 245 | 20 | 15 | Distillation column | PhOH | 96 | 245 | 20 | 25 | DPC |
| Example 1-36 | DPC | 245 | 20 | 15 | Distillation column | PhOH | 96 | 245 | 20 | 25 | DPC |
| Example 1-37 | DPC | 245 | 20 | 15 | Distillation column | PhOH | 96 | 245 | 20 | 25 | DPC |
| Example 1-38 | DEC | 260 | 1000 | 15 | Distillation column | EtOH | 84 | 245 | 20 | 25 | DEC |
| Example 1-39 | DBC | 260 | 800 | 15 | Distillation column | n-BuOH | 86 | 130 | 20 | 25 | DBC |
| Example 1-40 | DPC | 245 | 20 | 15 | Distillation column | PhOH | 96 | 250 | 20 | 25 | DPC |
| Example 1-41 | DPC | 245 | 20 | 10 | FF | PhOH | 86 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-42 | DPC | 245 | 20 | 15 | FF × 2 | PhOH | 94 | 130 | 20 | 25 | DPC |
| Example 1-43 | DPC | 245 | 20 | 10 | Thin film | PhOH | 92 | 130 | 20 | 25 | DPC |
| Example 1-44 | DPC | 245 | 20 | 15 | Distillation column (sieve tray) | PhOH | 96 | 250 | 20 | 25 | DPC |
| Example 1-45 | DPC | 245 | 20 | 15 | Distillation column (sieve tray) | PhOH | 92 | 130 | 20 | 25 | DPC |
| Example 1-46 | DPC | 245 | 20 | 15 | Distillation column (structured filling substance) | PhOH | 96 | 250 | 20 | 25 | DPC |
| Example 1-47 | DPC | 245 | 20 | 15 | Distillation column (structured filling substance) | PhOH | 92 | 130 | 20 | 25 | DPC |
| Example 1-48 | DPC | 245 | 20 | 15 | Distillation column | PhOH | 90 | 250 | 20 | 25 | DPC |
| Example 1-49 | DPC | 245 | 600 | 15 | Distillation column | PhOH | 92 | 130 | 20 | 25 | DPC |
| Example 1-50 | DPC | 245 | 20 | 15 | Distillation column | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-51 | DPC | 245 | 20 | 15 | Distillation column | PhOH | 92 | 130 | 20 | 25 | DPC |
| Example 1-52 | DBC | 260 | 800 | 15 | Distillation column | n-BuOH | 89 | 245 | 20 | 25 | DBC |
| Example 1-53 | DPC | 245 | 20 | 15 | Distillation column | PhOH | 92 | 250 | 20 | 25 | DPC |
| Example 1-54 | DPC | 245 | 20 | 10 | FF | PhOH | 90 | 130 | 20 | 25 | DPC |
| Example 1-55 | DPC | 245 | 20 | 15 | FF × 2 | PhOH | 97 | 130 | 20 | 25 | DPC |
| Example 1-56 | DPC | 245 | 20 | 10 | Thin film | PhOH | 92 | 130 | 20 | 25 | DPC |
| Example 1-57 | DPC | 245 | 20 | 15 | Distillation column | PhOH | 89 | 245 | 20 | 25 | DPC |
| Example 1-58 | DPC | 245 | 20 | 15 | Distillation column | PhOH | 89 | 245 | 20 | 25 | DPC |
| Example 1-59 | DPC | 245 | 20 | 15 | Distillation column | PhOH | 89 | 245 | 20 | 25 | DPC |
| Example 1-60 | DPC | 245 | 20 | 15 | Distillation column | PhOH | 89 | 245 | 20 | 25 | DPC |
| Example 1-61 | DEC | 260 | 1000 | 15 | Distillation column | EtOH | 87 | 245 | 20 | 25 | DEC |
| Example 1-62 | DBC | 260 | 800 | 15 | Distillation column | n-BuOH | 86 | 130 | 20 | 25 | DBC |
| Example 1-63 | DPC | 245 | 20 | 15 | Distillation column | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-64 | DPC | 245 | 20 | 10 | FF | PhOH | 86 | 130 | 20 | 25 | DPC |
| Example 1-65 | DPC | 245 | 20 | 15 | FF × 2 | PhOH | 94 | 130 | 20 | 25 | DPC |

[Table 1-6]

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-66 | DPC | 245 | 20 | 10 | Thin film | PhOH | 92 | 130 | 20 | 25 | DPC |
| Example 1-67 | DPC | 245 | 20 | 15 | Distillation column (sieve tray) | PhOH | 96 | 250 | 20 | 25 | DPC |
| Example 1-68 | DPC | 245 | 20 | 15 | Distillation column (sieve tray) | PhOH | 92 | 130 | 20 | 25 | DPC |
| Example 1-69 | DPC | 245 | 20 | 15 | Distillation column (structured filling substance) | PhOH | 96 | 250 | 20 | 25 | DPC |
| Example 1-70 | DPC | 245 | 20 | 15 | Distillation column (structured filling substance) | PhOH | 92 | 130 | 20 | 25 | DPC |
| Example 1-71 | DPC | 245 | 20 | 15 | Distillation column | PhOH | 90 | 250 | 20 | 25 | DPC |
| Example 1-72 | DPC | 245 | 600 | 15 | Distillation column | PhOH | 92 | 130 | 20 | 25 | DPC |
| Example 1-73 | DPC | 245 | 20 | 15 | Distillation column | PhOH | 96 | 250 | 20 | 25 | DPC |
| Example 1-74 | DPC | 245 | 20 | 15 | Distillation column | PhOH | 92 | 130 | 20 | 25 | DPC |
| Example 1-75 | DBC | 260 | 800 | 15 | Distillation column | n-BuOH | 88 | 245 | 20 | 25 | DBC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-76 | DPC | 245 | 20 | 15 | Distillation column | PhOH | 90 | 250 | 20 | 25 | DPC |
| Example 1-77 | DPC | 245 | 20 | 10 | FF | PhOH | 88 | 130 | 20 | 25 | DPC |
| Example 1-78 | DPC | 245 | 20 | 15 | FF × 2 | PhOH | 95 | 130 | 20 | 25 | DPC |
| Example 1-79 | DPC | 245 | 20 | 10 | Thin film | PhOH | 89 | 130 | 20 | 25 | DPC |
| Example 1-80 | DPC | 245 | 20 | 15 | Distillation column | PhOH | 88 | 245 | 20 | 25 | DPC |
| Example 1-81 | DPC | 245 | 20 | 15 | Distillation column | PhOH | 88 | 245 | 20 | 25 | DPC |
| Example 1-82 | DPC | 245 | 20 | 15 | Distillation column | PhOH | 88 | 245 | 20 | 25 | DPC |
| Example 1-83 | DPC | 245 | 20 | 15 | Distillation column | PhOH | 88 | 245 | 20 | 25 | DPC |
| Example 1-84 | DEC | 260 | 1000 | 15 | Distillation column | EtOH | 86 | 245 | 20 | 25 | DEC |
| Example 1-85 | DBC | 260 | 800 | 15 | Distillation column | n-BuOH | 86 | 130 | 20 | 25 | DBC |
| Example 1-86 | DPC | 245 | 20 | 15 | Distillation column | PhOH | 96 | 250 | 20 | 25 | DPC |
| Example 1-87 | DPC | 245 | 20 | 10 | FF | PhOH | 86 | 130 | 20 | 25 | DPC |
| Example 1-88 | DPC | 245 | 20 | 15 | FF × 2 | PhOH | 94 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-89 | DPC | 245 | 20 | 10 | Thin film | PhOH | 92 | 130 | 20 | 25 | DPC |
| Example 1-90 | DPC | 245 | 20 | 15 | Distillation column (sieve tray) | PhOH | 96 | 250 | 20 | 25 | DPC |
| Example 1-91 | DPC | 245 | 20 | 15 | Distillation column (sieve tray) | PhOH | 92 | 130 | 20 | 25 | DPC |
| Example 1-92 | DPC | 245 | 20 | 15 | Distillation column (structured filling substance) | PhOH | 96 | 250 | 20 | 25 | DPC |
| Example 1-93 | DPC | 245 | 20 | 15 | Distillation column (structured filling substance) | PhOH | 92 | 130 | 20 | 25 | DPC |
| Example 1-94 | DPC | 245 | 20 | 15 | Distillation column | PhOH | 90 | 250 | 20 | 25 | DPC |
| Example 1-95 | DPC | 245 | 600 | 15 | Distillation column | PhOH | 92 | 130 | 20 | 25 | DPC |
| Example 1-96 | DPC | 245 | 20 | 15 | Distillation column | PhOH | 96 | 250 | 20 | 25 | DPC |
| Example 1-97 | DPC | 245 | 20 | 15 | Distillation column | PhOH | 92 | 130 | 20 | 25 | DPC |
| Example 1-98 | DBC | 260 | 800 | 15 | Distillation column | n-BuOH | 87 | 245 | 20 | 25 | DBC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-99 | DPC | 245 | 20 | 15 | Distillation column | PhOH | 93 | 250 | 20 | 25 | DPC |
| Example 1-100 | DPC | 245 | 20 | 10 | FF | PhOH | 87 | 130 | 20 | 25 | DPC |
| Example 1-101 | DPC | 245 | 20 | 15 | FF × 2 | PhOH | 93 | 130 | 20 | 25 | DPC |
| Example 1-102 | DPC | 245 | 20 | 10 | Thin film | PhOH | 90 | 130 | 20 | 25 | DPC |
| Example 1-103 | DPC | 245 | 20 | 15 | Distillation column | PhOH | 93 | 245 | 20 | 25 | DPC |
| Example 1-104 | DPC | 245 | 20 | 15 | Distillation column | PhOH | 93 | 245 | 20 | 25 | DPC |
| Example 1-105 | DPC | 245 | 20 | 15 | Distillation column | PhOH | 93 | 245 | 20 | 25 | DPC |
| Example 1-106 | DPC | 245 | 20 | 15 | Distillation column | PhOH | 93 | 245 | 20 | 25 | DPC |
| Example 1-107 | DEC | 260 | 1000 | 15 | Distillation column | EtOH | 87 | 245 | 20 | 25 | DEC |
| Example 1-108 | DBC | 260 | 800 | 15 | Distillation column | n-BuOH | 86 | 130 | 20 | 25 | DBC |
| Example 1-109 | DPC | 245 | 20 | 15 | Distillation column | PhOH | 96 | 250 | 20 | 25 | DPC |
| Example 1-110 | DPC | 245 | 20 | 10 | FF | PhOH | 86 | 130 | 20 | 25 | DPC |
| Example 1-111 | DPC | 245 | 20 | 15 | FF × 2 | PhOH | 94 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-112 | DPC | 245 | 20 | 10 | Thin film | PhOH | 92 | 130 | 20 | 25 | DPC |
| Example 1-113 | DPC | 245 | 20 | 15 | Distillation column (sieve tray) | PhOH | 96 | 250 | 20 | 25 | DPC |
| Example 1-114 | DPC | 245 | 20 | 15 | Distillation column (sieve tray) | PhOH | 92 | 130 | 20 | 25 | DPC |
| Example 1-115 | DPC | 245 | 20 | 15 | Distillation column (structured filling substance) | PhOH | 96 | 250 | 20 | 25 | DPC |
| Example 1-116 | DPC | 245 | 20 | 15 | Distillation column (structured filling substance) | PhOH | 92 | 130 | 20 | 25 | DPC |
| Example 1-117 | DPC | 245 | 20 | 15 | Distillation column | PhOH | 90 | 250 | 20 | 25 | DPC |
| Example 1-118 | DPC | 245 | 600 | 15 | Distillation column | PhOH | 92 | 130 | 20 | 25 | DPC |
| Example 1-119 | DPC | 245 | 20 | 15 | Distillation column | PhOH | 96 | 250 | 20 | 25 | DPC |
| Example 1-120 | DPC | 245 | 20 | 15 | Distillation column | PhOH | 92 | 130 | 20 | 25 | DPC |
| Example 1-121 | DBC | 260 | 800 | 15 | Distillation column | n-BuOH | 87 | 170 | 20 | 25 | DBC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-122 | DPC | 245 | 20 | 15 | Distillation column | PhOH | 94 | 170 | 20 | 25 | DPC |
| Example 1-123 | DPC | 245 | 20 | 10 | FF | PhOH | 87 | 170 | 20 | 25 | DPC |
| Example 1-124 | DPC | 245 | 20 | 15 | FF × 2 | PhOH | 93 | 170 | 20 | 25 | DPC |
| Example 1-125 | DPC | 245 | 20 | 10 | Thin film | PhOH | 90 | 170 | 20 | 25 | DPC |
| Example 1-126 | DPC | 245 | 20 | 15 | Distillation column | PhOH | 94 | 170 | 20 | 25 | DPC |
| Example 1-127 | DPC | 245 | 20 | 15 | Distillation column | PhOH | 94 | 170 | 20 | 25 | DPC |
| Example 1-128 | DPC | 245 | 20 | 15 | Distillation column | PhOH | 94 | 170 | 20 | 25 | DPC |
| Example 1-129 | DPC | 245 | 20 | 15 | Distillation column | PhOH | 94 | 170 | 20 | 25 | DPC |
| Example 1-130 | DEC | 260 | 1000 | 15 | Distillation column | EtOH | 87 | 170 | 20 | 25 | DEC |
| Example 1-131 | DBC | 260 | 800 | 15 | Distillation column | n-BuOH | 86 | 130 | 20 | 25 | DBC |
| Example 1-132 | DPC | 245 | 20 | 15 | Distillation column | PhOH | 96 | 250 | 20 | 25 | DPC |
| Example 1-133 | DPC | 245 | 20 | 10 | FF | PhOH | 86 | 130 | 20 | 25 | DPC |
| Example 1-134 | DPC | 245 | 20 | 15 | FF × 2 | PhOH | 94 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-135 | DPC | 245 | 20 | 10 | Thin film | PhOH | 92 | 130 | 20 | 25 | DPC |
| Example 1-136 | DPC | 245 | 20 | 15 | Distillation column (sieve tray) | PhOH | 96 | 250 | 20 | 25 | DPC |

[Table 1-7]

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-137 | DPC | 245 | 20 | 15 | Distillation column (sieve tray) | PhOH | 92 | 130 | 20 | 25 | DPC |
| Example 1-138 | DPC | 245 | 20 | 15 | Distillation column (structured filling substance) | PhOH | 96 | 250 | 20 | 25 | DPC |
| Example 1-139 | DPC | 245 | 20 | 15 | Distillation column (structured filling substance) | PhOH | 92 | 130 | 20 | 25 | DPC |
| Example 1-140 | DPC | 245 | 20 | 15 | Distillation column | PhOH | 90 | 250 | 20 | 25 | DPC |
| Example 1-141 | DPC | 245 | 600 | 15 | Distillation column | PhOH | 92 | 130 | 20 | 25 | DPC |
| Example 1-142 | DPC | 245 | 20 | 15 | Distillation column | PhOH | 96 | 250 | 20 | 25 | DPC |
| Example 1-143 | DPC | 245 | 20 | 15 | Distillation column | PhOH | 92 | 130 | 20 | 25 | DPC |
| Example 1-144 | DBC | 260 | 800 | 15 | Distillation column | n-BuOH | 88 | 130 | 20 | 25 | DBC |
| Example 1-145 | DPC | 245 | 20 | 15 | Distillation column | PhOH | 95 | 250 | 20 | 25 | DPC |
| Example 1-146 | DPC | 245 | 20 | 10 | FF | PhOH | 85 | 130 | 20 | 25 | DPC |
| Example 1-147 | DPC | 245 | 20 | 15 | FF × 2 | PhOH | 84 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-148 | DPC | 245 | 20 | 10 | Thin film | PhOH | 91 | 130 | 20 | 25 | DPC |
| Example 1-149 | DPC | 245 | 20 | 15 | Distillation column | PhOH | 95 | 245 | 20 | 25 | DPC |
| Example 1-150 | DPC | 245 | 20 | 15 | Distillation column | PhOH | 95 | 245 | 20 | 25 | DPC |
| Example 1-151 | DPC | 245 | 20 | 15 | Distillation column | PhOH | 95 | 245 | 20 | 25 | DPC |
| Example 1-152 | DPC | 245 | 20 | 15 | Distillation column | PhOH | 95 | 245 | 20 | 25 | DPC |
| Example 1-153 | DEC | 260 | 1000 | 15 | Distillation column | EtOH | 87 | 245 | 20 | 25 | DEC |
| Example 1-154 | DBC | 260 | 800 | 15 | Distillation column | n-BuOH | 86 | 130 | 20 | 25 | DBC |
| Example 1-155 | DPC | 245 | 20 | 15 | Distillation column | PhOH | 96 | 250 | 20 | 25 | DPC |
| Example 1-156 | DPC | 245 | 20 | 10 | FF | PhOH | 86 | 130 | 20 | 25 | DPC |
| Example 1-157 | DPC | 245 | 20 | 15 | FF × 2 | PhOH | 94 | 130 | 20 | 25 | DPC |
| Example 1-158 | DPC | 245 | 20 | 10 | Thin film | PhOH | 92 | 130 | 20 | 25 | DPC |
| Example 1-159 | DPC | 245 | 20 | 15 | Distillation column (sieve tray) | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-160 | DPC | 245 | 20 | 15 | Distillation column (sieve tray) | PhOH | 92 | 130 | 20 | 25 | DPC |
| Example 1-161 | DPC | 245 | 20 | 15 | Distillation column (structured filling substance) | PhOH | 96 | 250 | 20 | 25 | DPC |
| Example 1-162 | DPC | 245 | 20 | 15 | Distillation column (structured filling substance) | PhOH | 92 | 130 | 20 | 25 | DPC |
| Example 1-163 | DPC | 245 | 20 | 15 | Distillation column | PhOH | 90 | 250 | 20 | 25 | DPC |
| Example 1-164 | DPC | 245 | 600 | 15 | Distillation column | PhOH | 92 | 130 | 20 | 25 | DPC |
| Example 1-165 | DPC | 245 | 20 | 15 | Distillation column | PhOH | 96 | 250 | 20 | 25 | DPC |
| Example 1-166 | DPC | 245 | 20 | 15 | Distillation column | PhOH | 92 | 130 | 20 | 25 | DPC |
| Example 1-167 | DBC | 260 | 800 | 15 | Distillation column | n-BuOH | 88 | 130 | 20 | 25 | DBC |
| Example 1-168 | DPC | 245 | 20 | 15 | Distillation column | PhOH | 95 | 250 | 20 | 25 | DPC |
| Example 1-169 | DPC | 245 | 20 | 10 | FF | PhOH | 85 | 130 | 20 | 25 | DPC |
| Example 1-170 | DPC | 245 | 20 | 15 | FF × 2 | PhOH | 93 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-171 | DPC | 245 | 20 | 10 | Thin film | PhOH | 92 | 130 | 20 | 25 | DPC |
| Example 1-172 | DPC | 245 | 20 | 15 | Distillation column | PhOH | 95 | 245 | 20 | 25 | DPC |
| Example 1-173 | DPC | 245 | 20 | 15 | Distillation column | PhOH | 95 | 245 | 20 | 25 | DPC |
| Example 1-174 | DPC | 245 | 20 | 15 | Distillation column | PhOH | 95 | 245 | 20 | 25 | DPC |
| Example 1-175 | DPC | 245 | 20 | 15 | Distillation column | PhOH | 95 | 245 | 20 | 25 | DPC |
| Example 1-176 | DEC | 260 | 1000 | 15 | Distillation column | EtOH | 87 | 245 | 20 | 25 | DEC |
| Example 1-177 | DBC | 260 | 800 | 15 | Distillation column | n-BuOH | 86 | 130 | 20 | 25 | DBC |
| Example 1-178 | DPC | 245 | 20 | 15 | Distillation column | PhOH | 96 | 250 | 20 | 25 | DPC |
| Example 1-179 | DPC | 245 | 20 | 10 | FF | PhOH | 86 | 130 | 20 | 25 | DPC |
| Example 1-180 | DPC | 245 | 20 | 15 | FF × 2 | PhOH | 94 | 130 | 20 | 25 | DPC |
| Example 1-181 | DPC | 245 | 20 | 10 | Thin film | PhOH | 92 | 130 | 20 | 25 | DPC |
| Example 1-182 | DPC | 245 | 20 | 15 | Distillation column (sieve tray) | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-183 | DPC | 245 | 20 | 15 | Distillation column (sieve tray) | PhOH | 92 | 130 | 20 | 25 | DPC |
| Example 1-184 | DPC | 245 | 20 | 15 | Distillation column (structured filling substance) | PhOH | 96 | 250 | 20 | 25 | DPC |
| Example 1-185 | DPC | 245 | 20 | 15 | Distillation column (structured filling substance) | PhOH | 92 | 130 | 20 | 25 | DPC |
| Example 1-186 | DPC | 245 | 20 | 15 | Distillation column | PhOH | 90 | 250 | 20 | 25 | DPC |
| Example 1-187 | DPC | 245 | 600 | 15 | Distillation column | PhOH | 92 | 130 | 20 | 25 | DPC |
| Example 1-188 | DPC | 245 | 20 | 15 | Distillation column | PhOH | 96 | 250 | 20 | 25 | DPC |
| Example 1-189 | DPC | 245 | 20 | 15 | Distillation column | PhOH | 92 | 130 | 20 | 25 | DPC |
| Example 1-190 | DBC | 260 | 800 | 15 | Distillation column | n-BuOH | 89 | 130 | 20 | 25 | DBC |
| Example 1-191 | DPC | 245 | 20 | 15 | Distillation column | PhOH | 95 | 250 | 20 | 25 | DPC |
| Example 1-192 | DPC | 245 | 20 | 10 | FF | PhOH | 85 | 130 | 20 | 25 | DPC |
| Example 1-193 | DPC | 245 | 20 | 15 | FF × 2 | PhOH | 92 | 130 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-194 | DPC | 245 | 20 | 10 | Thin film | PhOH | 89 | 130 | 20 | 25 | DPC |
| Example 1-195 | DPC | 245 | 20 | 15 | Distillation column | PhOH | 95 | 245 | 20 | 25 | DPC |
| Example 1-196 | DPC | 245 | 20 | 15 | Distillation column | PhOH | 95 | 245 | 20 | 25 | DPC |
| Example 1-197 | DPC | 245 | 20 | 15 | Distillation column | PhOH | 95 | 245 | 20 | 25 | DPC |
| Example 1-198 | DPC | 245 | 20 | 15 | Distillation column | PhOH | 95 | 245 | 20 | 25 | DPC |
| Example 1-199 | DEC | 260 | 1000 | 15 | Distillation column | EtOH | 87 | 245 | 20 | 25 | DEC |
| Example 1-200 | DBC | 260 | 800 | 15 | Distillation column | n-BuOH | 86 | 130 | 20 | 25 | DBC |
| Example 1-201 | DPC | 245 | 20 | 15 | Distillation column | PhOH | 96 | 250 | 20 | 25 | DPC |
| Example 1-202 | DPC | 245 | 20 | 10 | FF | PhOH | 86 | 130 | 20 | 25 | DPC |
| Example 1-203 | DPC | 245 | 20 | 15 | FF × 2 | PhOH | 94 | 130 | 20 | 25 | DPC |
| Example 1-204 | DPC | 245 | 20 | 10 | Thin film | PhOH | 92 | 130 | 20 | 25 | DPC |
| Example 1-205 | DPC | 245 | 20 | 15 | Distillation column (sieve trav) | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-206 | DPC | 245 | 20 | 15 | Distillation column (sieve trav) | PhOH | 92 | 130 | 20 | 25 | DPC |
| Example 1-207 | DPC | 245 | 20 | 15 | Distillation column (structured filling substance) | PhOH | 96 | 250 | 20 | 25 | DPC |

[Table 1-8]

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-208 | DPC | 245 | 20 | 15 | Distillation column (structured filling substance) | PhOH | 92 | 130 | 20 | 25 | DPC |
| Example 1-209 | DPC | 245 | 20 | 15 | Distillation column | PhOH | 90 | 250 | 20 | 25 | DPC |
| Example 1-210 | DPC | 245 | 600 | 15 | Distillation column | PhOH | 92 | 130 | 20 | 25 | DPC |
| Example 1-211 | DPC | 245 | 20 | 15 | Distillation column | PhOH | 96 | 250 | 20 | 25 | DPC |
| Example 1-212 | DPC | 245 | 20 | 15 | Distillation column | PhOH | 92 | 130 | 20 | 25 | DPC |
| Example 1-213 | DBC | 260 | 800 | 15 | Distillation column | n-BuOH | 88 | 130 | 20 | 25 | DBC |
| Example 1-214 | DPC | 245 | 20 | 15 | Distillation column | PhOH | 95 | 250 | 20 | 25 | DPC |
| Example 1-215 | DPC | 245 | 20 | 10 | FF | PhOH | 86 | 130 | 20 | 25 | DPC |
| Example 1-216 | DPC | 245 | 20 | 15 | FF × 2 | PhOH | 93 | 130 | 20 | 25 | DPC |
| Example 1-217 | DPC | 245 | 20 | 10 | Thin film | PhOH | 91 | 130 | 20 | 25 | DPC |
| Example 1-218 | DPC | 245 | 20 | 15 | Distillation column | PhOH | 95 | 245 | 20 | 25 | DPC |
| Example 1-219 | DPC | 245 | 20 | 15 | Distillation column | PhOH | 95 | 245 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-220 | DPC | 245 | 20 | 15 | Distillation column | PhOH | 95 | 245 | 20 | 25 | DPC |
| Example 1-221 | DPC | 245 | 20 | 15 | Distillation column | PhOH | 95 | 245 | 20 | 25 | DPC |
| Example 1-222 | DEC | 260 | 1000 | 15 | Distillation column | EtOH | 87 | 245 | 20 | 25 | DEC |
| Example 1-223 | DBC | 260 | 800 | 15 | Distillation column | n-BuOH | 86 | 130 | 20 | 25 | DBC |
| Example 1-224 | DPC | 245 | 20 | 15 | Distillation column | PhOH | 96 | 250 | 20 | 25 | DPC |
| Example 1-225 | DPC | 245 | 20 | 10 | FF | PhOH | 86 | 130 | 20 | 25 | DPC |
| Example 1-226 | DPC | 245 | 20 | 15 | FF × 2 | PhOH | 94 | 130 | 20 | 25 | DPC |
| Example 1-227 | DPC | 245 | 20 | 10 | Thin film | PhOH | 92 | 130 | 20 | 25 | DPC |
| Example 1-228 | DPC | 245 | 20 | 15 | Distillation column (sieve tray) | PhOH | 96 | 250 | 20 | 25 | DPC |
| Example 1-229 | DPC | 245 | 20 | 15 | Distillation column (sieve tray) | PhOH | 92 | 130 | 20 | 25 | DPC |
| Example 1-230 | DPC | 245 | 20 | 15 | Distillation column (structured filling substance) | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-231 | DPC | 245 | 20 | 15 | Distillation column (structured filling substance) | PhOH | 92 | 130 | 20 | 25 | DPC |
| Example 1-232 | DPC | 245 | 20 | 15 | Distillation column | PhOH | 90 | 250 | 20 | 25 | DPC |
| Example 1-233 | DPC | 245 | 600 | 15 | Distillation column | PhOH | 92 | 130 | 20 | 25 | DPC |
| Example 1-234 | DPC | 245 | 20 | 15 | Distillation column | PhOH | 96 | 250 | 20 | 25 | DPC |
| Example 1-235 | DPC | 245 | 20 | 15 | Distillation column | PhOH | 92 | 130 | 20 | 25 | DPC |
| Example 1-236 | DBC | 260 | 800 | 15 | Distillation column | n-BuOH | 87 | 130 | 20 | 25 | DBC |
| Example 1-237 | DPC | 245 | 20 | 15 | Distillation column | PhOH | 96 | 250 | 20 | 25 | DPC |
| Example 1-238 | DPC | 245 | 20 | 10 | FF | PhOH | 85 | 130 | 20 | 25 | DPC |
| Example 1-239 | DPC | 245 | 20 | 15 | FF × 2 | PhOH | 94 | 130 | 20 | 25 | DPC |
| Example 1-240 | DPC | 245 | 20 | 10 | Thin film | PhOH | 92 | 130 | 20 | 25 | DPC |
| Example 1-241 | DPC | 245 | 20 | 15 | Distillation column | PhOH | 96 | 245 | 20 | 25 | DPC |
| Example 1-242 | DPC | 245 | 20 | 15 | Distillation column | PhOH | 96 | 245 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-243 | DPC | 245 | 20 | 15 | Distillation column | PhOH | 96 | 245 | 20 | 25 | DPC |
| Example 1-244 | DPC | 245 | 20 | 15 | Distillation column | PhOH | 96 | 245 | 20 | 25 | DPC |
| Example 1-245 | DEC | 260 | 1000 | 15 | Distillation column | EtOH | 96 | 245 | 20 | 25 | DEC |
| Example 1-246 | DBC | 260 | 800 | 15 | Distillation column | n-BuOH | 86 | 130 | 20 | 25 | DBC |
| Example 1-247 | DPC | 245 | 20 | 15 | Distillation column | PhOH | 96 | 250 | 20 | 25 | DPC |
| Example 1-248 | DPC | 245 | 20 | 10 | FF | PhOH | 86 | 130 | 20 | 25 | DPC |
| Example 1-249 | DPC | 245 | 20 | 15 | FF × 2 | PhOH | 94 | 130 | 20 | 25 | DPC |
| Example 1-250 | DPC | 245 | 20 | 10 | Thin film | PhOH | 92 | 130 | 20 | 25 | DPC |
| Example 1-251 | DPC | 245 | 20 | 15 | Distillation column (sieve tray) | PhOH | 96 | 250 | 20 | 25 | DPC |
| Example 1-252 | DPC | 245 | 20 | 15 | Distillation column (sieve tray) | PhOH | 92 | 130 | 20 | 25 | DPC |
| Example 1-253 | DPC | 245 | 20 | 15 | Distillation column (structured filling substance) | PhOH | 96 | 250 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-254 | DPC | 245 | 20 | 15 | Distillation column (structured filling substance) | PhOH | 92 | 130 | 20 | 25 | DPC |
| Example 1-255 | DPC | 245 | 20 | 15 | Distillation column | PhOH | 90 | 250 | 20 | 25 | DPC |
| Example 1-256 | DPC | 245 | 600 | 15 | Distillation column | PhOH | 92 | 130 | 20 | 25 | DPC |
| Example 1-257 | DPC | 245 | 20 | 15 | Distillation column | PhOH | 96 | 250 | 20 | 25 | DPC |
| Example 1-258 | DPC | 245 | 20 | 15 | Distillation column | PhOH | 92 | 130 | 20 | 25 | DPC |
| Example 1-259 | DPC | 245 | 20 | 15 | Distillation column | PhOH | 96 | 245 | 20 | 25 | DPC |
| Example 1-260 | DPC | 245 | 20 | 15 | Distillation column | PhOH | 96 | 245 | 20 | 25 | DPC |
| Example 1-261 | DEC | 260 | 1000 | 15 | Distillation column | EtOH | 96 | 245 | 20 | 25 | DEC |
| Example 1-262 | DPC | 245 | 20 | 15 | Distillation column | PhOH | 96 | 245 | 20 | 25 | DPC |
| Example 1-263 | DPC | 245 | 20 | 15 | Distillation column | PhOH | 96 | 245 | 20 | 25 | DPC |
| Example 1-264 | DEC | 260 | 1000 | 15 | Distillation column | EtOH | 96 | 245 | 20 | 25 | DEC |
| Example 1-265 | DPC | 245 | 20 | 15 | Distillation column | PhOH | 96 | 245 | 20 | 25 | DPC |

(continued)

| | Step (2) | | | | | | | Step (3) | | | |
|---|---|---|---|---|---|---|---|---|---|---|---|
| | Solvent | Temperature | Pressure | Average retention time | Reaction form | By-product low-boiling-point compound | Yield | Temperature | Pressure | Average retention time | Extracted aprotic solvent |
| | | °C | kPa | min | | | % | °C | kPa | min | |
| Example 1-266 | DPC | 245 | 20 | 15 | Distillation column | PhOH | 96 | 245 | 20 | 25 | DPC |
| Example 1-267 | DEC | 260 | 1000 | 15 | Distillation column | EtOH | 96 | 245 | 20 | 25 | DEC |

[Table 1-9]

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-1 | 5 | 150 | 1 | 3 | Thin film | Formula (I) ·heavy | 0.01 |
| Example 1-2 | 5 | 150 | 1 | 3 | Thin film | Formula (I) ·heavy | 0.01 |
| Example 1-3 | 5 | 150 | 1 | 3 | Thin film | Formula (I) ·heavy | 0.01 |
| Example 1-4 | 5 | 150 | 1 | 3 | Thin film | Formula (I) ·heavy | 0.01 |
| Example 1-5 | 5 | 150 | 1 | 3 | Thin film | Formula (I) ·heavy | 0.01 |
| Example 1-6 | 5 | 150 | 1 | 3 | Thin film | Formula (I) ·heavy | 0.01 |
| Example 1-7 | 5 | 150 | 1 | 3 | Thin film | Formula (I) ·heavy | 0.01 |
| Example 1-8 | 5 | 150 | 1 | 3 | Thin film | Formula (I) ·heavy | 0.01 |
| Example 1-9 | 5 | 150 | 1 | 3 | Thin film | Formula (I)-heavy | 0.01 |
| Example 1-10 | 5 | 150 | 1 | 3 | Thin film | Formula (I) ·heavy·DPC | 0.01 |
| Example 1-11 | 5 | 150 | 1 | 3 | Thin film | Formula (I) ·heavy·DPC | 0.01 |
| Example 1-12 | 6 | 150 | 1 | 3 | Thin film | Formula (I)-heavy | 0.01 |
| Example 1-13 | 6 | 150 | 1 | 3 | Thin film | Formula (I) ·heavy | 0.01 |
| Example 1-14 | 5 | 150 | 1 | 3 | Thin film | Formula (I) ·heavy | 0.01 |
| Example 1-15 | 5 | 150 | 1 | 3 | Thin film | Formula (I)-heavy | 0.1 |
| Example 1-16 | 5 | 150 | 1 | 3 | Thin film | Formula (I) ·heavy·DPC | 0.01 |
| Example 1-17 | 5 | 150 | 1 | 3 | Thin film | Formula (I) ·heavy·DPC | 0.01 |
| Example 1-18 | 5 | 150 | 1 | 3 | Thin film | Formula (I) ·heavy·DPC | 0.01 |
| Example 1-19 | 6 | 150 | 1 | 3 | Thin film | Formula (I) ·heavy | 0.01 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-20 | 5 | 150 | 1 | 3 | Thin film | Formula (I)-heavy | 0.01 |
| Example 1-21 | 6 | 150 | 1 | 3 | Thin film | Formula (I) ·heavy | 0.01 |
| Example 1-22 | 5 | 150 | 1 | 3 | Thin film | Formula (I) ·heavy | 0.01 |
| Example 1-23 | 6 | 150 | 1 | 3 | Thin film | Formula (I)-heavy | 0.01 |
| Example 1-24 | 5 | 150 | 1 | 3 | Thin film | Formula (I) ·heavy | 0.01 |
| Example 1-25 | 6 | 150 | 1 | 3 | Distillation column (filled column) | Formula (I) ·heavy | 0.008 |
| Example 1-26 | 5 | 150 | 1 | 3 | Distillation column (filled column) | Formula (I) ·heavy | 0.008 |
| Example 1-27 | 6 | 150 | 1 | 3 | Thin film | Formula (I) ·heavy | 0.01 |
| Example 1-28 | 5 | 150 | 1 | 3 | Thin film | Formula (I) ·heavy | 0.01 |
| Comparative Example 1-1 | - | - | - | - | - | - | - |
| Comparative Example 1-2 | - | - | - | - | - | - | 0.01 |
| Comparative Example 1-3 | - | - | /Difficult to be operated | - | - | - | 0.1 |
| Comparative Example 1-4 | - | Product NCOpurity lowered | - | - | - | - | 9 |
| Comparative Example 1-5 | - | - | - | - | - | - | 0.01 |
| Comparative Example 1-6 | 5 | 150 | 1 | 3 | Thin film | - | 0.01 |
| Example 1-29 | 5 | 180 | 0.1 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-30 | 9 | 180 | 0.1 | 3 | Thin film | Formula (I) ·heavy | 0.03 |
| Example 1-31 | 5 | 180 | 0.1 | 3 | Thin film | Formula (I)-heavy | 0.03 |
| Example 1-32 | 5 | 180 | 0.1 | 3 | Thin film | Formula (I) ·heavy | 0.03 |
| Example 1-33 | 5 | 180 | 0.1 | 3 | Thin film | Formula (I) ·heavy | 0.03 |
| Example 1-34 | 9 | 180 | 0.1 | 3 | Thin film | Formula (I)-heavy | 0.03 |
| Example 1-35 | 9 | 180 | 0.1 | 3 | Thin film | Formula (I) ·heavy | 0.03 |
| Example 1-36 | 9 | 180 | 0.1 | 3 | Thin film | Formula (I) ·heavy | 0.03 |
| Example 1-37 | 9 | 180 | 0.1 | 3 | Thin film | Formula (I) ·heavy | 0.03 |
| Example 1-38 | 9 | 180 | 0.1 | 3 | Thin film | Formula (I) ·heavy | 0.03 |
| Example 1-39 | 5 | 180 | 0.1 | 3 | Thin film | Formula (I) ·heavy | 0.03 |
| Example 1-40 | 9 | 180 | 0.1 | 3 | Thin film | Formula (I) ·heavy | 0.03 |
| Example 1-41 | 5 | 180 | 0.1 | 3 | Thin film | Formula (I) ·heavy | 0.03 |
| Example 1-42 | 5 | 180 | 0.1 | 3 | Thin film | Formula (I) ·heavy | 0.03 |
| Example 1-43 | 5 | 180 | 0.1 | 3 | Thin film | Formula (I) ·heavy | 0.03 |
| Example 1-44 | 9 | 180 | 0.1 | 3 | Thin film | Formula (I) ·heavy | 0.03 |
| Example 1-45 | 5 | 180 | 0.1 | 3 | Thin film | Formula (I) ·heavy | 0.03 |
| Example 1-46 | 9 | 180 | 0.1 | 3 | Thin film | Formula (I) ·heavy | 0.03 |
| Example 1-47 | 5 | 180 | 0.1 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-48 | 9 | 150 | 0.1 | 8 | Distillation column (filled column) | Formula (I) ·heavy | 0.008 |
| Example 1-49 | 5 | 150 | 0.1 | 8 | Distillation column (filled column) | Formula (I) ·heavy | 0.008 |
| Example 1-50 | 9 | 180 | 0.1 | 3 | Thin film | Formula (I) ·heavy | 0.03 |
| Example 1-51 | 5 | 180 | 0.1 | 3 | Thin film | Formula (I) ·heavy | 0.03 |
| Example 1-52 | 9 | 130 | 0.1 | 3 | Thin film | Formula (I) ·heavy | 0.02 |
| Example 1-53 | 9 | 130 | 0.1 | 3 | Thin film | Formula (I) ·heavy | 0.02 |
| Example 1-54 | 5 | 130 | 0.1 | 3 | Thin film | Formula (I) ·heavy | 0.02 |
| Example 1-55 | 5 | 130 | 0.1 | 3 | Thin film | Formula (I) ·heavy | 0.02 |
| Example 1-56 | 5 | 130 | 0.1 | 3 | Thin film | Formula (I) ·heavy | 0.02 |
| Example 1-57 | 10 | 130 | 0.1 | 3 | Thin film | Formula (I) ·heavy | 0.02 |
| Example 1-58 | 10 | 130 | 0.1 | 3 | Thin film | Formula (I) ·heavy | 0.02 |
| Example 1-59 | 10 | 130 | 0.1 | 3 | Thin film | Formula (I) ·heavy | 0.02 |
| Example 1-60 | 10 | 130 | 0.1 | 3 | Thin film | Formula (I) ·heavy | 0.02 |
| Example 1-61 | 10 | 130 | 0.1 | 3 | Thin film | Formula (I) ·heavy | 0.02 |
| Example 1-62 | 9 | 130 | 0.1 | 3 | Thin film | Formula (I) ·heavy | 0.03 |
| Example 1-63 | 9 | 130 | 0.1 | 3 | Thin film | Formula (I) ·heavy | 0.03 |
| Example 1-64 | 5 | 130 | 0.1 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-65 | 5 | 130 | 0.1 | 3 | Thin film | Formula (I) ·heavy | 0.03 |

[Table 1-10]

| | Step (4) | | | | | | |
|---|---|---|---|---|---|---|---|
| | Content of compound (I) before peration of step (4) | Temperature | Pressure | Average retention time | Reaction form | Extracted liquid-phase component | Content of compound (I) in product |
| | (% by mass) | °C | kPa | min | | | (% by mass) |
| Example 1-66 | 5 | 130 | 0.1 | 3 | Thin film | Formula (I) ·heavy | 0.03 |
| Example 1-67 | 9 | 130 | 0.1 | 3 | Thin film | Formula (I) ·heavy | 0.03 |
| Example 1-68 | 9 | 130 | 0.1 | 3 | Thin film | Formula (I) -heavy | 0.03 |
| Example 1-69 | 9 | 130 | 0.1 | 3 | Thin film | Formula (I)-heavy | 0.03 |
| Example 1-70 | 9 | 130 | 0.1 | 3 | Thin film | Formula (I) ·heavy | 0.03 |
| Example 1-71 | 9 | 130 | 0.1 | 3 | Distillation column (filled column) | Formula (I) -heavy | 0.008 |
| Example 1-72 | 9 | 130 | 0.1 | 3 | Distillation column (filled column) | Formula (I) ·heavy | 0.008 |
| Example 1-73 | 9 | 130 | 0.1 | 3 | Thin film | Formula (I) -heavy | 0.03 |
| Example 1-74 | 9 | 130 | 0.1 | 3 | Thin film | Formula (I) ·heavy | 0.03 |
| Example 1-75 | 10 | 160 | 0.2 | 3 | Thin film | Formula (I) ·heavy | 0.02 |
| Example 1-76 | 10 | 160 | 0.2 | 3 | Thin film | Formula (I) -heavy | 0.02 |

(continued)

| | Step (4) | | | | | | |
|---|---|---|---|---|---|---|---|
| | Content of compound (I) before peration of step (4) | Temperature | Pressure | Average retention time | Reaction form | Extracted liquid-phase component | Content of compound (I) in product |
| | (% by mass) | °C | kPa | min | | | (% by mass) |
| Example 1-77 | 5 | 160 | 2 | 3 | Thin film | Formula (I) ·heavy | 0.02 |
| Example 1-78 | 5 | 160 | 2 | 3 | Thin film | Formula (I) ·heavy | 0.02 |
| Example 1-79 | 5 | 160 | 2 | 3 | Thin film | Formula (I) ·heavy | 0.02 |
| Example 1-80 | 10 | 160 | 0.2 | 3 | Thin film | Formula (I) ·heavy | 0.02 |
| Example 1-81 | 10 | 160 | 0.2 | 3 | Thin film | Formula (I) -heavy | 0.02 |
| Example 1-82 | 10 | 160 | 0.2 | 3 | Thin film | Formula (I) ·heavy | 0.02 |
| Example 1-83 | 10 | 160 | 0.2 | 3 | Thin film | Formula (I) ·heavy | 0.02 |
| Example 1-84 | 10 | 160 | 0.2 | 3 | Thin film | Formula (I) ·heavy | 0.02 |
| Example 1-85 | 10 | 160 | 0.2 | 3 | Thin film | Formula (I) ·heavy | 0.03 |
| Example 1-86 | 10 | 160 | 0.2 | 3 | Thin film | Formula (I) ·heavy | 0.03 |
| Example 1-87 | 5 | 160 | 2 | 3 | Thin film | Formula (I) ·heavy | 0.03 |
| Example 1-88 | 5 | 160 | 2 | 3 | Thin film | Formula (I) ·heavy | 0.03 |
| Example 1-89 | 5 | 160 | 2 | 3 | Thin film | Formula (I) ·heavy | 0.03 |
| Example 1-90 | 10 | 160 | 0.2 | 3 | Thin film | Formula (I) ·heavy | 0.03 |
| Example 1-91 | 10 | 160 | 0.2 | 3 | Thin film | Formula (I) ·heavy | 0.03 |
| Example 1-92 | 10 | 160 | 0.2 | 3 | Thin film | Formula (I) ·heavy | 0.03 |
| Example 1-93 | 10 | 160 | 0.2 | 3 | Thin film | Formula (I) ·heavy | 0.03 |
| Example 1-94 | 10 | 160 | 0.2 | 3 | Distillation column (filled column) | Formula (I) ·heavy | 0.008 |

(continued)

| | Step (4) | | | | | | |
|---|---|---|---|---|---|---|---|
| | Content of compound (I) before peration of step (4) | Temperature | Pressure | Average retention time | Reaction form | Extracted liquid-phase component | Content of compound (I) in product |
| | (% by mass) | °C | kPa | min | | | (% by mass) |
| Example 1-95 | 10 | 160 | 0.2 | 3 | Distillation column (filled column) | Formula (I) ·heavy | 0.008 |
| Example 1-96 | 10 | 160 | 0.2 | 3 | Thin film | Formula (I) ·heavy | 0.03 |
| Example 1-97 | 10 | 160 | 0.2 | 3 | Thin film | Formula (I) ·heavy | 0.03 |
| Example 1-98 | 10 | 170 | 0.2 | 3 | Thin film | Formula (I) ·heavy | 0.01 |
| Example 1-99 | 10 | 180 | 0.2 | 3 | Thin film | Formula (I) ·heavy | 0.01 |
| Example 1-100 | 5 | 150 | 2 | 3 | Thin film | Formula (I) ·heavy | 0.01 |
| Example 1-101 | 5 | 150 | 2 | 3 | Thin film | Formula (I) ·heavy | 0.01 |
| Example 1-102 | 5 | 150 | 2 | 3 | Thin film | Formula (I) ·heavy | 0.01 |
| Example 1-103 | 10 | 170 | 0.2 | 3 | Thin film | Formula (I) ·heavy | 0.01 |
| Example 1-104 | 10 | 170 | 0.2 | 3 | Thin film | Formula (I) ·heavy | 0.01 |
| Example 1-105 | 10 | 170 | 0.2 | 3 | Thin film | Formula (I) ·heavy | 0.01 |
| Example 1-106 | 10 | 170 | 0.2 | 3 | Thin film | Formula (I) ·heavy | 0.01 |
| Example 1-107 | 10 | 170 | 0.2 | 3 | Thin film | Formula (I) ·heavy | 0.01 |
| Example 1-108 | 10 | 170 | 0.2 | 3 | Thin film | Formula (I) ·heavy | 0.03 |
| Example 1-109 | 10 | 180 | 0.2 | 3 | Thin film | Formula (I) ·heavy | 0.03 |
| Example 1-110 | 5 | 150 | 2 | 3 | Thin film | Formula (I) ·heavy | 0.03 |
| Example 1-111 | 5 | 150 | 2 | 3 | Thin film | Formula (I) ·heavy | 0.03 |
| Example 1-112 | 5 | 150 | 2 | 3 | Thin film | Formula (I) ·heavy | 0.03 |

(continued)

| | Step (4) | | | | | | |
|---|---|---|---|---|---|---|---|
| | Content of compound (I) before peration of step (4) | Temperature | Pressure | Average retention time | Reaction form | Extracted liquid-phase component | Content of compound (I) in product |
| | (% by mass) | °C | kPa | min | | | (% by mass) |
| Example 1-113 | 10 | 180 | 0.2 | 3 | Thin film | Formula (I) ·heavy | 0.03 |
| Example 1-114 | 10 | 180 | 0.2 | 3 | Thin film | Formula (I) ·heavy | 0.03 |
| Example 1-115 | 10 | 180 | 0.2 | 3 | Thin film | Formula (I) ·heavy | 0.03 |
| Example 1-116 | 10 | 180 | 0.2 | 3 | Thin film | Formula (I) ·heavy | 0.03 |
| Example 1-117 | 10 | 180 | 0.2 | 3 | Distillation column (filled column) | Formula (I) ·heavy | 0.008 |
| Example 1-118 | 10 | 180 | 0.2 | 3 | Distillation column (filled column) | Formula (I) ·heavy | 0.008 |
| Example 1-119 | 10 | 180 | 0.2 | 3 | Thin film | Formula (I) ·heavy | 0.03 |
| Example 1-120 | 10 | 180 | 0.2 | 3 | Thin film | Formula (I) ·heavy | 0.03 |
| Example 1-121 | 10 | 170 | 0.2 | 3 | Thin film | Formula (I) ·heavy | 0.01 |
| Example 1-122 | 10 | 170 | 0.2 | 3 | Thin film | Formula (I) ·heavy | 0.01 |
| Example 1-123 | 5 | 170 | 0.2 | 3 | Thin film | Formula (I) ·heavy | 0.01 |
| Example 1-124 | 5 | 170 | 0.2 | 3 | Thin film | Formula (I) ·heavy | 0.01 |
| Example 1-125 | 5 | 170 | 0.2 | 3 | Thin film | Formula (I) ·heavy | 0.01 |
| Example 1-126 | 10 | 170 | 0.2 | 3 | Thin film | Formula (I) ·heavy | 0.01 |
| Example 1-127 | 10 | 170 | 0.2 | 3 | Thin film | Formula (I) ·heavy | 0.01 |
| Example 1-128 | 10 | 170 | 0.2 | 3 | Thin film | Formula (I) ·heavy | 0.01 |
| Example 1-129 | 10 | 170 | 0.2 | 3 | Thin film | Formula (I) ·heavy | 0.01 |
| Example 1-130 | 10 | 170 | 0.2 | 3 | Thin film | Formula (I) ·heavy | 0.01 |

(continued)

| | Step (4) | | | | | | |
|---|---|---|---|---|---|---|---|
| | Content of compound (I) beforeperation of step (4) | Temperature | Pressure | Average retention time | Reaction form | Extracted liquid-phase component | Content of compound (I) in product |
| | (% by mass) | °C | kPa | min | | | (% by mass) |
| Example 1-131 | 10 | 170 | 0.2 | 3 | Thin film | Formula (I) ·heavy | 0.03 |
| Example 1-132 | 10 | 170 | 0.2 | 3 | Thin film | Formula (I) ·heavy | 0.03 |
| Example 1-133 | 5 | 170 | 0.2 | 3 | Thin film | Formula (I) ·heavy | 0.03 |
| Example 1-134 | 5 | 170 | 0.2 | 3 | Thin film | Formula (I) ·heavy | 0.03 |
| Example 1-135 | 5 | 170 | 0.2 | 3 | Thin film | Formula (I) ·heavy | 0.03 |
| Example 1-136 | 10 | 170 | 0.2 | 3 | Thin film | Formula (I) ·heavy | 0.03 |

[Table 1-11]

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-137 | 10 | 170 | 0.2 | 3 | Thin film | Formula (I) ·heavy | 0.03 |
| Example 1-138 | 10 | 170 | 0.2 | 3 | Thin film | Formula (I) ·heavy | 0.03 |
| Example 1-139 | 10 | 170 | 0.2 | 3 | Thin film | Formula (I) ·heavy | 0.03 |
| Example 1-140 | 10 | 170 | 0.2 | 3 | Distillation column (filled column) | Formula (I) ·heavy | 0.008 |
| Example 1-141 | 10 | 170 | 0.2 | 3 | Distillation column (filled column) | Formula (I) ·heavy | 0.008 |
| Example 1-142 | 10 | 170 | 0.2 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-143 | 10 | 170 | 0.2 | 3 | Thin film | Formula (I) ·heavy | 0.03 |
| Example 1-144 | 5 | 165 | 0.1 | 3 | Thin film | Formula (I) ·heavy | 0.02 |
| Example 1-145 | 10 | 165 | 0.1 | 3 | Thin film | Formula (I) ·heavy | 0.02 |
| Example 1-146 | 5 | 165 | 0.1 | 3 | Thin film | Formula (I) ·heavy | 0.02 |
| Example 1-147 | 5 | 165 | 0.1 | 3 | Thin film | Formula (I) ·heavy | 0.02 |
| Example 1-148 | 5 | 165 | 0.1 | 3 | Thin film | Formula (I) ·heavy | 0.02 |
| Example 1-149 | 10 | 165 | 0.1 | 3 | Thin film | Formula (I) ·heavy | 0.02 |
| Example 1-150 | 10 | 165 | 0.1 | 3 | Thin film | Formula (I) ·heavy | 0.02 |
| Example 1-151 | 10 | 165 | 0.1 | 3 | Thin film | Formula (I) ·heavy | 0.02 |
| Example 1-152 | 10 | 165 | 0.1 | 3 | Thin film | Formula (I) ·heavy | 0.02 |
| Example 1-153 | 10 | 165 | 0.1 | 3 | Thin film | Formula (I) ·heavy | 0.02 |
| Example 1-154 | 5 | 165 | 0.1 | 3 | Thin film | Formula (I) ·heavy | 0.03 |
| Example 1-155 | 10 | 165 | 0.1 | 3 | Thin film | Formula (I) ·heavy | 0.03 |
| Example 1-156 | 5 | 165 | 0.1 | 3 | Thin film | Formula (I) ·heavy | 0.03 |
| Example 1-157 | 5 | 165 | 0.1 | 3 | Thin film | Formula (I) ·heavy | 0.03 |
| Example 1-158 | 5 | 165 | 0.1 | 3 | Thin film | Formula (I) ·heavy | 0.03 |
| Example 1-159 | 10 | 165 | 0.1 | 3 | Thin film | Formula (I) ·heavy | 0.03 |
| Example 1-160 | 10 | 165 | 0.1 | 3 | Thin film | Formula (I) ·heavy | 0.03 |
| Example 1-161 | 10 | 165 | 0.1 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-162 | 10 | 165 | 0.1 | 3 | Thin film | Formula (I) ·heavy | 0.03 |
| Example 1-163 | 10 | 165 | 0.1 | 3 | Distillation column (filled column) | Formula (I) ·heavy | 0.008 |
| Example 1-164 | 10 | 165 | 0.1 | 3 | Distillation column (filled column) | Formula (I) ·heavy | 0.008 |
| Example 1-165 | 10 | 165 | 0.1 | 3 | Thin film | Formula (I) ·heavy | 0.03 |
| Example 1-166 | 10 | 165 | 0.1 | 3 | Thin film | Formula (I) ·heavy | 0.03 |
| Example 1-167 | 5 | 180 | 0.05 | 3 | Thin film | Formula (I) ·heavy | 0.03 |
| Example 1-168 | 10 | 180 | 0.05 | 3 | Thin film | Formula (I) ·heavy | 0.03 |
| Example 1-169 | 5 | 180 | 0.05 | 3 | Thin film | Formula (I) ·heavy | 0.03 |
| Example 1-170 | 5 | 180 | 0.05 | 3 | Thin film | Formula (I) ·heavy | 0.03 |
| Example 1-171 | 5 | 180 | 0.05 | 3 | Thin film | Formula (I) ·heavy | 0.03 |
| Example 1-172 | 10 | 180 | 0.05 | 3 | Thin film | Formula (I) ·heavy | 0.03 |
| Example 1-173 | 10 | 180 | 0.05 | 3 | Thin film | Formula (I) ·heavy | 0.03 |
| Example 1-174 | 10 | 180 | 0.05 | 3 | Thin film | Formula (I) ·heavy | 0.03 |
| Example 1-175 | 10 | 180 | 0.05 | 3 | Thin film | Formula (I) ·heavy | 0.03 |
| Example 1-176 | 10 | 180 | 0.05 | 3 | Thin film | Formula (I) ·heavy | 0.03 |
| Example 1-177 | 5 | 180 | 0.05 | 3 | Thin film | Formula (I) ·heavy | 0.03 |
| Example 1-178 | 10 | 180 | 0.05 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-179 | 5 | 180 | 0.05 | 3 | Thin film | Formula (I) ·heavy | 0.03 |
| Example 1-180 | 5 | 180 | 0.05 | 3 | Thin film | Formula (I) ·heavy | 0.03 |
| Example 1-181 | 5 | 180 | 0.05 | 3 | Thin film | Formula (I) ·heavy | 0.03 |
| Example 1-182 | 10 | 180 | 0.05 | 3 | Thin film | Formula (I) ·heavy | 0.03 |
| Example 1-183 | 10 | 180 | 0.05 | 3 | Thin film | Formula (I) ·heavy | 0.03 |
| Example 1-184 | 10 | 180 | 0.05 | 3 | Thin film | Formula (I) ·heavy | 0.03 |
| Example 1-185 | 10 | 180 | 0.05 | 3 | Thin film | Formula (I) ·heavy | 0.03 |
| Example 1-186 | 10 | 180 | 0.05 | 3 | Distillation column (filled column) | Formula (I) ·heavy | 0.008 |
| Example 1-187 | 10 | 180 | 0.05 | 3 | Distillation column (filled column) | Formula (I) ·heavy | 0.008 |
| Example 1-188 | 10 | 180 | 0.05 | 3 | Thin film | Formula (I) ·heavy | 0.03 |
| Example 1-189 | 10 | 180 | 0.05 | 3 | Thin film | Formula (I) ·heavy | 0.03 |
| Example 1-190 | 5 | 170 | 0.1 | 3 | Thin film | Formula (I) ·heavy | 0.02 |
| Example 1-191 | 10 | 170 | 0.1 | 3 | Thin film | Formula (I) ·heavy | 0.02 |
| Example 1-192 | 5 | 170 | 0.1 | 3 | Thin film | Formula (I) ·heavy | 0.02 |
| Example 1-193 | 5 | 170 | 0.1 | 3 | Thin film | Formula (I) ·heavy | 0.02 |
| Example 1-194 | 5 | 170 | 0.1 | 3 | Thin film | Formula (I) ·heavy | 0.02 |
| Example 1-195 | 10 | 170 | 0.1 | 3 | Thin film | Formula (I) ·heavy | 0.02 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-196 | 10 | 170 | 0.1 | 3 | Thin film | Formula (I) ·heavy | 0.02 |
| Example 1-197 | 10 | 170 | 0.1 | 3 | Thin film | Formula (I) ·heavy | 0.02 |
| Example 1-198 | 10 | 170 | 0.1 | 3 | Thin film | Formula (I) ·heavy | 0.02 |
| Example 1-199 | 10 | 170 | 0.1 | 3 | Thin film | Formula (I) ·heavy | 0.02 |
| Example 1-200 | 5 | 170 | 0.1 | 3 | Thin film | Formula (I) ·heavy | 0.03 |
| Example 1-201 | 10 | 170 | 0.1 | 3 | Thin film | Formula (I) ·heavy | 0.03 |
| Example 1-202 | 5 | 170 | 0.1 | 3 | Thin film | Formula (I) ·heavy | 0.03 |
| Example 1-203 | 5 | 170 | 0.1 | 3 | Thin film | Formula (I) ·heavy | 0.03 |
| Example 1-204 | 5 | 170 | 0.1 | 3 | Thin film | Formula (I) ·heavy | 0.03 |
| Example 1-205 | 10 | 170 | 0.1 | 3 | Thin film | Formula (I) ·heavy | 0.03 |
| Example 1-206 | 10 | 170 | 0.1 | 3 | Thin film | Formula (I) ·heavy | 0.03 |
| Example 1-207 | 10 | 170 | 0.1 | 3 | Thin film | Formula (I) ·heavy | 0.03 |

[Table 1-12]

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-208 | 10 | 170 | 0.1 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-209 | 10 | 170 | 0.1 | 3 | Distillation column (filled column) | Formula (I) ·heavy | 0.008 |
| Example 1-210 | 10 | 170 | 0.1 | 3 | Distillation column (filled column) | Formula (I) ·heavy | 0.008 |
| Example 1-211 | 10 | 170 | 0.1 | 3 | Thin film | Formula (I) ·heavy | 0.03 |
| Example 1-212 | 10 | 170 | 0.1 | 3 | Thin film | Formula (I) ·heavy | 0.03 |
| Example 1-213 | 5 | 180 | 0.1 | 3 | Thin film | Formula (I) ·heavy | 0.04 |
| Example 1-214 | 10 | 180 | 0.1 | 3 | Thin film | Formula (I) ·heavy | 0.04 |
| Example 1-215 | 5 | 180 | 0.1 | 3 | Thin film | Formula (I)-heavy | 0.04 |
| Example 1-216 | 5 | 180 | 0.1 | 3 | Thin film | Formula (I) ·heavy | 0.04 |
| Example 1-217 | 5 | 180 | 0.1 | 3 | Thin film | Formula (I) ·heavy | 0.04 |
| Example 1-218 | 10 | 180 | 0.1 | 3 | Thin film | Formula (I) ·heavy | 0.04 |
| Example 1-219 | 10 | 180 | 0.1 | 3 | Thin film | Formula (I) ·heavy | 0.04 |
| Example 1-220 | 10 | 180 | 0.1 | 3 | Thin film | Formula (I) ·heavy | 0.04 |
| Example 1-221 | 10 | 180 | 0.1 | 3 | Thin film | Formula (I) ·heavy | 0.04 |
| Example 1-222 | 10 | 180 | 0.1 | 3 | Thin film | Formula (I) ·heavy | 0.04 |
| Example 1-223 | 5 | 180 | 0.1 | 3 | Thin film | Formula (I)-heavy | 0.03 |
| Example 1-224 | 10 | 180 | 0.1 | 3 | Thin film | Formula (I) ·heavy | 0.03 |
| Example 1-225 | 5 | 180 | 0.1 | 3 | Thin film | Formula (I) ·heavy | 0.03 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-226 | 5 | 180 | 0.1 | 3 | Thin film | Formula (I)-heavy | 0.03 |
| Example 1-227 | 5 | 180 | 0.1 | 3 | Thin film | Formula (I) ·heavy | 0.03 |
| Example 1-228 | 10 | 180 | 0.1 | 3 | Thin film | Formula (I) ·heavy | 0.03 |
| Example 1-229 | 10 | 180 | 0.1 | 3 | Thin film | Formula (I) ·heavy | 0.03 |
| Example 1-230 | 10 | 180 | 0.1 | 3 | Thin film | Formula (I) ·heavy | 0.03 |
| Example 1-231 | 10 | 180 | 0.1 | 3 | Thin film | Formula (I) ·heavy | 0.03 |
| Example 1-232 | 10 | 180 | 0.1 | 3 | Distillation column (filled column) | Formula (I) ·heavy | 0.008 |
| Example 1-233 | 10 | 180 | 0.1 | 3 | Distillation column (filled column) | Formula (I) ·heavy | 0.008 |
| Example 1-234 | 10 | 180 | 0.1 | 3 | Thin film | Formula (I) ·heavy | 0.03 |
| Example 1-235 | 10 | 180 | 0.1 | 3 | Thin film | Formula (I) ·heavy | 0.03 |
| Example 1-236 | 5 | 180 | 0.02 | 3 | Thin film | Formula (I) ·heavy | 0.07 |
| Example 1-237 | 10 | 180 | 0.02 | 3 | Thin film | Formula (I) ·heavy | 0.07 |
| Example 1-238 | 5 | 180 | 0.02 | 3 | Thin film | Formula (I) ·heavy | 0.07 |
| Example 1-239 | 5 | 180 | 0.02 | 3 | Thin film | Formula (I) ·heavy | 0.07 |
| Example 1-240 | 5 | 180 | 0.02 | 3 | Thin film | Formula (I) ·heavy | 0.07 |
| Example 1-241 | 10 | 180 | 0.02 | 3 | Thin film | Formula (I) ·heavy | 0.07 |
| Example 1-242 | 10 | 180 | 0.02 | 3 | Thin film | Formula (I) ·heavy | 0.07 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-243 | 10 | 180 | 0.02 | 3 | Thin film | Formula (I) ·heavy | 0.07 |
| Example 1-244 | 10 | 180 | 0.02 | 3 | Thin film | Formula (I) ·heavy | 0.07 |
| Example 1-245 | 10 | 180 | 0.02 | 3 | Thin film | Formula (I) ·heavy | 0.07 |
| Example 1-246 | 5 | 180 | 0.02 | 3 | Thin film | Formula (I) ·heavy | 0.03 |
| Example 1-247 | 10 | 180 | 0.02 | 3 | Thin film | Formula (I) ·heavy | 0.03 |
| Example 1-248 | 5 | 180 | 0.02 | 3 | Thin film | Formula (I) ·heavy | 0.03 |
| Example 1-249 | 5 | 180 | 0.02 | 3 | Thin film | Formula (I) ·heavy | 0.03 |
| Example 1-250 | 5 | 180 | 0.02 | 3 | Thin film | Formula (I) ·heavy | 0.03 |
| Example 1-251 | 10 | 180 | 0.02 | 3 | Thin film | Formula (I) ·heavy | 0.03 |
| Example 1-252 | 10 | 180 | 0.02 | 3 | Thin film | Formula (I) ·heavy | 0.03 |
| Example 1-253 | 10 | 180 | 0.02 | 3 | Thin film | Formula (I) ·heavy | 0.03 |
| Example 1-254 | 10 | 180 | 0.02 | 3 | Thin film | Formula (I) ·heavy | 0.03 |
| Example 1-255 | 10 | 180 | 0.02 | 3 | Distillation column (filled column) | Formula (I) ·heavy | 0.008 |
| Example 1-256 | 10 | 180 | 0.02 | 3 | Distillation column (filled column) | Formula (I) ·heavy | 0.008 |
| Example 1-257 | 10 | 180 | 0.02 | 3 | Thin film | Formula (I) ·heavy | 0.03 |
| Example 1-258 | 10 | 180 | 0.02 | 3 | Thin film | Formula (I) ·heavy | 0.03 |
| Example 1-259 | 10 | 160 | 0.01 | 3 | Thin film | Formula (I)-heavy | 0.08 |

(continued)

| | Step (4) | | | | | | |
|---|---|---|---|---|---|---|---|
| | Content of compound (I) before operation of step (4) | Temperature | Pressure | Average retention time | Reaction form | Extracted liquid-phase component | Content of compound (I) in product |
| | (% by mass) | °C | kPa | min | | | (% by mass) |
| Example 1-260 | 10 | 160 | 0.01 | 3 | Thin film | Formula (I) ·heavy | 0.08 |
| Example 1-261 | 10 | 160 | 0.01 | 3 | Thin film | Formula (I) ·heavy | 0.08 |
| Example 1-262 | 10 | 160 | 0.03 | 3 | Thin film | Formula (I)-heavy | 0.06 |
| Example 1-263 | 10 | 160 | 0.03 | 3 | Thin film | Formula (I) ·heavy | 0.06 |
| Example 1-264 | 10 | 160 | 0.03 | 3 | Thin film | Formula (I) ·heavy | 0.06 |
| Example 1-265 | 10 | 150 | 0.01 | 3 | Thin film | Formula (I) ·heavy | 0.02 |
| Example 1-266 | 10 | 180 | 0.01 | 3 | Thin film | Formula (I) ·heavy | 0.02 |
| Example 1-267 | 10 | 180 | 0.01 | 3 | Thin film | Formula (I) ·heavy | 0.02 |

[Table 1-13]

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-1 | 5.0 | n-BuOH | 5.0 | 5.0 | 30 wt%-aq. NaOH | 0.6 | Stirring tank | 90 | 90 |
| Example 1-2 | 5.0 | iPrOH | 5.0 | 5.0 | 30 wt%-aq. NaOH | 0.6 | Stirring tank | 88 | 88 |
| Example 1-3 | 5.0 | n-BuOH | 5.0 | 5.0 | 30 wt%-aq. NaOH | 0.6 | Stirring tank + PFR | 91 | 91 |
| Example 1-4 | 5.0 | n-BuOH | 5.0 | 5.0 | 30 wt%-aq. NaOH | 0.6 | Stirring tank | 90 | 90 |
| Example 1-5 | 5.0 | n-BuOH | 5.0 | 5.0 | 30 wt%-aq. NaOH | 0.6 | Stirring tank | 90 | 90 |
| Example 1-6 | 5.0 | n-BuOH | 5.0 | 5.0 | 30 wt%-aq. NaOH | 0.6 | Stirring tank | 90 | 90 |
| Example 1-7 | 5.0 | n-BuOH | 5.0 | 5.0 | 30 wt%-aq. NaOH | 0.6 | Stirring tank | 90 | 90 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-8 | 5.0 | n-BuOH | 5.0 | 5.0 | 30 wt%-aq. NaOH | 0.6 | Stirring tank | 90 | 90 |
| Example 1-9 | 5.0 | n-BuOH | 5.0 | 5.0 | 30 wt%-aq. NaOH | 0.6 | Stirring tank | 90 | 90 |
| Example 1-10 | 5.0 | PhOH | 5.0 | 5.0 | 30 wt%-aq. NaOH | 0.6 | Stirring tank | 93 | 93 |
| Example 1-11 | 5.0 | PhOH | 5.0 | 5.0 | 30 wt%-aq. NaOH | 0.6 | Stirring tank | 93 | 93 |
| Example 1-12 | 5.0 | n-BuOH | 5.0 | 5.0 | 30 wt%-aq. NaOH | 0.6 | Stirring tank | 93 | 93 |
| Example 1-13 | 5.0 | n-BuOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-14 | 5.0 | n-BuOH | 5.0 | 5.0 | 30 wt%-aq. NaOH | 0.6 | Stirring tank | 90 | 90 |
| Example 1-15 | 5.0 | n-BuOH | 5.0 | 5.0 | 30 wt%-aq. NaOH | 0.6 | Stirring tank | 90 | 90 |
| Example 1-16 | 5.0 | PhOH | 5.0 | 5.0 | 30 wt%-aq. NaOH | 0.6 | Stirring tank | 93 | 93 |
| Example 1-17 | 5.0 | PhOH | 5.0 | 5.0 | 30 wt%-aq. NaOH | 0.6 | Stirring tank | 90 | 90 |
| Example 1-18 | 5.0 | PhOH | 5.0 | 5.0 | 30 wt%-aq. NaOH | 0.6 | Stirring tank | 90 | 90 |
| Example 1-19 | 5.0 | n-BuOH | 5.0 | 5.0 | 30 wt%-aq. NaOH | 0.6 | Stirring tank | 90 | 90 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-20 | 5.0 | n-BuOH | 5.0 | 5.0 | 30 wt%-aq. NaOH | 0.6 | Stirring tank | 90 | 90 |
| Example 1-21 | 5.0 | n-BuOH | 5.0 | 5.0 | 30 wt%-aq. NaOH | 0.6 | Stirring tank | 90 | 90 |
| Example 1-22 | 5.0 | n-BuOH | 5.0 | 5.0 | 30 wt%-aq. NaOH | 0.6 | Stirring tank | 90 | 90 |
| Example 1-23 | 5.0 | n-BuOH | 5.0 | 5.0 | 30 wt%-aq. NaOH | 0.6 | Stirring tank | 90 | 90 |
| Example 1-24 | 5.0 | n-BuOH | 5.0 | 5.0 | 30 wt%-aq. NaOH | 0.6 | Stirring tank | 90 | 90 |
| Example 1-25 | 5.0 | n-BuOH | 5.0 | 5.0 | 30 wt%-aq. NaOH | 0.6 | Stirring tank | 90 | 90 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-26 | 5.0 | n-BuOH | 5.0 | 5.0 | 30 wt%-aq. NaOH | 0.6 | Stirring tank | 90 | 90 |
| Example 1-27 | 5.0 | n-BuOH | 5.0 | 5.0 | 30 wt%-aq. KOH | 0.6 | Stirring tank | 89 | 89 |
| Example 1-28 | 5.0 | n-BuOH | 5.0 | 5.0 | 30 wt%-aq. KOH | 0.6 | Stirring tank | 89 | 89 |
| Comparative Example 1-1 | - | - | - | - | - | - | - | - | - |
| Comparative Example 1-2 | - | - | - | - | - | - | - | - | - |
| Comparative Example 1-3 | - | - | - | - | - | - | - | - | - |
| Comparative Example 1-4 | - | - | - | - | - | - | - | - | - |
| Comparative Example 1-5 | - | - | - | - | - | - | - | - | - |
| Comparative Example 1-6 | 5.0 | PhOH | 5.0 | 5.0 | 30 wt%-aq. NaOH | 0.6 | Stirring tank + PFR | 95 | 95 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-29 | 5.0 | n-BuOH | 5.0 | 5.0 | 30 wt%-aq. NaOH | 0.6 | Stirring tank | 90 | 90 |
| Example 1-30 | 5.0 | PhOH | 5.0 | 5.0 | 30 wt%-aq. NaOH | 0.6 | Stirring tank | 93 | 93 |
| Example 1-31 | 5.0 | PhOH | 5.0 | 5.0 | 30 wt%-aq. NaOH | 0.6 | Stirring tank | 93 | 93 |
| Example 1-32 | 5.0 | PhOH | 5.0 | 5.0 | 30 wt%-aq. NaOH | 0.6 | Stirring tank | 93 | 93 |
| Example 1-33 | 5.0 | PhOH | 5.0 | 5.0 | 30 wt%-aq. NaOH | 0.6 | Stirring tank | 93 | 93 |
| Example 1-34 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-35 | 5.0 | PhOH | 5.0 | 5.0 | 30 wt%-aq. NaOH | 0.6 | Stirring tank | 93 | 93 |
| Example 1-36 | 5.0 | PhOH | 5.0 | 5.0 | 30 wt%-aq. NaOH | 0.6 | Stirring tank | 93 | 93 |
| Example 1-37 | 5.0 | PhOH | 5.0 | 5.0 | 30 wt%-aq. NaOH | 0.6 | Stirring tank | 93 | 93 |
| Example 1-38 | 5.0 | EtOH | 5.0 | 5.0 | 30 wt%-aq. NaOH | 0.6 | Stirring tank | 88 | 88 |
| Example 1-39 | 5.0 | n-BuOH | 5.0 | 5.0 | 30 wt%-aq. NaOH | 0.6 | Stirring tank | 90 | 90 |
| Example 1-40 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-41 | 5.0 | PhOH | 5.0 | 5.0 | 30 wt%-aq. NaOH | 0.6 | Stirring tank | 93 | 93 |
| Example 1-42 | 5.0 | PhOH | 5.0 | 5.0 | 30 wt%-aq. NaOH | 0.6 | Stirring tank | 93 | 93 |
| Example 1-43 | 5.0 | PhOH | 5.0 | 5.0 | 30 wt%-aq. NaOH | 0.6 | Stirring tank | 93 | 93 |
| Example 1-44 | 5.0 | PhOH | 5.0 | 5.0 | 30 wt%-aq. NaOH | 0.6 | Stirring tank | 93 | 93 |
| Example 1-45 | 5.0 | PhOH | 5.0 | 5.0 | 30 wt%-aq. NaOH | 0.6 | Stirring tank | 93 | 93 |
| Example 1-46 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-47 | 5.0 | PhOH | 5.0 | 5.0 | 30 wt%-aq. NaOH | 0.6 | Stirring tank | 93 | 93 |
| Example 1-48 | 5.0 | PhOH | 5.0 | 5.0 | 30 wt%-aq. NaOH | 0.6 | Stirring tank | 90 | 90 |
| Example 1-49 | 5.0 | PhOH | 5.0 | 5.0 | 30 wt%-aq. NaOH | 0.6 | Stirring tank | 90 | 90 |
| Example 1-50 | 5.0 | PhOH | 5.0 | 5.0 | 30 wt%-aq. KOH | 0.6 | Stirring tank | 93 | 93 |
| Example 1-51 | 5.0 | PhOH | 5.0 | 5.0 | 30 wt%-aq. KOH | 0.6 | Stirring tank | 93 | 93 |
| Example 1-52 | 5.0 | n-BuOH | 5.0 | 5.0 | 30 wt%-aq. NaOH | 0.6 | Stirring tank | 90 | 90 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-53 | 5.0 | PhOH | 5.0 | 5.0 | 30 wt%- aq. NaOH | 0.6 | Stirring tank | 93 | 93 |
| Example 1-54 | 5.0 | PhOH | 5.0 | 5.0 | 30 wt%- aq. NaOH | 0.6 | Stirring tank | 93 | 93 |
| Example 1-55 | 5.0 | PhOH | 5.0 | 5.0 | 30 wt%- aq. NaOH | 0.6 | Stirring tank | 93 | 93 |
| Example 1-56 | 5.0 | PhOH | 5.0 | 5.0 | 30 wt%- aq. NaOH | 0.6 | Stirring tank | 93 | 93 |
| Example 1-57 | 5.0 | PhOH | 5.0 | 5.0 | 30 wt%- aq. NaOH | 0.6 | Stirring tank | 93 | 93 |
| Example 1-58 | 5.0 | PhOH | 5.0 | 5.0 | 30 wt%- aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-59 | 5.0 | PhOH | 5.0 | 5.0 | 30 wt%-aq. NaOH | 0.6 | Stirring tank | 93 | 93 |
| Example 1-60 | 5.0 | PhOH | 5.0 | 5.0 | 30 wt%-aq. NaOH | 0.6 | Stirring tank | 93 | 93 |
| Example 1-61 | 5.0 | EtOH | 5.0 | 5.0 | 30 wt%-aq. NaOH | 0.6 | Stirring tank | 88 | 88 |
| Example 1-62 | 5.0 | n-BuOH | 5.0 | 5.0 | 30 wt%-aq. NaOH | 0.6 | Stirring tank | 90 | 90 |
| Example 1-63 | 5.0 | PhOH | 5.0 | 5.0 | 30 wt%-aq. NaOH | 0.6 | Stirring tank | 93 | 93 |
| Example 1-64 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine Recovery rate | Hydroxy compound recovery rate |
| | kg | Type | Blending amount kg | Blending amount kg | Type | Blending amount kg | | % | % |
| Example 1-65 | 5.0 | PhOH | 5.0 | 5.0 | 30 wt%-aq. NaOH | 0.6 | Stirring tank | 93 | 93 |

[Table 1-14]

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-66 | 5.0 | PhOH | 5.0 | 5.0 | 30 wt%-aq. NaOH | 0.6 | Stirring tank | 93 | 93 |
| Example 1-67 | 5.0 | PhOH | 5.0 | 5.0 | 30 wt%-aq. NaOH | 0.6 | Stirring tank | 93 | 93 |
| Example 1-68 | 5.0 | PhOH | 5.0 | 5.0 | 30 wt%-aq. NaOH | 0.6 | Stirring tank | 93 | 93 |
| Example 1-69 | 5.0 | PhOH | 5.0 | 5.0 | 30 wt%-aq. NaOH | 0.6 | Stirring tank | 93 | 93 |
| Example 1-70 | 5.0 | PhOH | 5.0 | 5.0 | 30 wt%-aq. NaOH | 0.6 | Stirring tank | 93 | 93 |
| Example 1-71 | 5.0 | PhOH | 5.0 | 5.0 | 30 wt%-aq. NaOH | 0.6 | Stirring tank | 90 | 90 |
| Example 1-72 | 5.0 | PhOH | 5.0 | 5.0 | 30 wt%-aq. NaOH | 0.6 | Stirring tank | 90 | 90 |
| Example 1-73 | 5.0 | PhOH | 5.0 | 5.0 | 30 wt%-aq. KOH | 0.6 | Stirring tank | 93 | 93 |
| Example 1-74 | 5.0 | PhOH | 5.0 | 5.0 | 30 wt%-aq. KOH | 0.6 | Stirring tank | 93 | 93 |
| Example 1-75 | 5.0 | n-BuOH | 5.0 | 5.0 | 30 wt%-aq. NaOH | 0.6 | Stirring tank | 90 | 90 |
| Example 1-76 | 5.0 | PhOH | 5.0 | 5.0 | 30 wt%-aq. NaOH | 0.6 | Stirring tank | 93 | 93 |
| Example 1-77 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-78 | 5.0 | PhOH | 5.0 | 5.0 | 30 wt%-aq. NaOH | 0.6 | Stirring tank | 93 | 93 |
| Example 1-79 | 5.0 | PhOH | 5.0 | 5.0 | 30 wt%-aq. NaOH | 0.6 | Stirring tank | 93 | 93 |
| Example 1-80 | 5.0 | PhOH | 5.0 | 5.0 | 30 wt%-aq. NaOH | 0.6 | Stirring tank | 93 | 93 |
| Example 1-81 | 5.0 | PhOH | 5.0 | 5.0 | 30 wt%-aq. NaOH | 0.6 | Stirring tank | 93 | 93 |
| Example 1-82 | 5.0 | PhOH | 5.0 | 5.0 | 30 wt%-aq. NaOH | 0.6 | Stirring tank | 93 | 93 |
| Example 1-83 | 5.0 | PhOH | 5.0 | 5.0 | 30 wt%-aq. NaOH | 0.6 | Stirring tank | 93 | 93 |
| Example 1-84 | 5.0 | EtOH | 5.0 | 5.0 | 30 wt%-aq. NaOH | 0.6 | Stirring tank | 88 | 88 |
| Example 1-85 | 5.0 | n-BuOH | 5.0 | 5.0 | 30 wt%-aq. NaOH | 0.6 | Stirring tank | 90 | 90 |
| Example 1-86 | 5.0 | PhOH | 5.0 | 5.0 | 30 wt%-aq. NaOH | 0.6 | Stirring tank | 93 | 93 |
| Example 1-87 | 5.0 | PhOH | 5.0 | 5.0 | 30 wt%-aq. NaOH | 0.6 | Stirring tank | 93 | 93 |
| Example 1-88 | 5.0 | PhOH | 5.0 | 5.0 | 30 wt%-aq. NaOH | 0.6 | Stirring tank | 93 | 93 |
| Example 1-89 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-90 | 5.0 | PhOH | 5.0 | 5.0 | 30 wt%-aq. NaOH | 0.6 | Stirring tank | 93 | 93 |
| Example 1-91 | 5.0 | PhOH | 5.0 | 5.0 | 30 wt%-aq. NaOH | 0.6 | Stirring tank | 93 | 93 |
| Example 1-92 | 5.0 | PhOH | 5.0 | 5.0 | 30 wt%-aq. NaOH | 0.6 | Stirring tank | 93 | 93 |
| Example 1-93 | 5.0 | PhOH | 5.0 | 5.0 | 30 wt%-aq. NaOH | 0.6 | Stirring tank | 93 | 93 |
| Example 1-94 | 5.0 | PhOH | 5.0 | 5.0 | 30 wt%-aq. NaOH | 0.6 | Stirring tank | 90 | 90 |
| Example 1-95 | 5.0 | PhOH | 5.0 | 5.0 | 30 wt%-aq. NaOH | 0.6 | Stirring tank | 90 | 90 |
| Example 1-96 | 5.0 | PhOH | 5.0 | 5.0 | 30 wt%-aq. KOH | 0.6 | Stirring tank | 93 | 93 |
| Example 1-97 | 5.0 | PhOH | 5.0 | 5.0 | 30 wt%-aq. KOH | 0.6 | Stirring tank | 93 | 93 |
| Example 1-98 | 5.0 | n-BuOH | 5.0 | 5.0 | 30 wt%-aq. NaOH | 0.6 | Stirring tank | 90 | 90 |
| Example 1-99 | 5.0 | PhOH | 5.0 | 5.0 | 30 wt%-aq. NaOH | 0.6 | Stirring tank | 93 | 93 |
| Example 1-100 | 5.0 | PhOH | 5.0 | 5.0 | 30 wt%-aq. NaOH | 0.6 | Stirring tank | 93 | 93 |
| Example 1-101 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-102 | 5.0 | PhOH | 5.0 | 5.0 | 30 wt%-aq. NaOH | 0.6 | Stirring tank | 93 | 93 |
| Example 1-103 | 5.0 | PhOH | 5.0 | 5.0 | 30 wt%-aq. NaOH | 0.6 | Stirring tank | 93 | 93 |
| Example 1-104 | 5.0 | PhOH | 5.0 | 5.0 | 30 wt%-aq. NaOH | 0.6 | Stirring tank | 93 | 93 |
| Example 1-105 | 5.0 | PhOH | 5.0 | 5.0 | 30 wt%-aq. NaOH | 0.6 | Stirring tank | 93 | 93 |
| Example 1-106 | 5.0 | PhOH | 5.0 | 5.0 | 30 wt%-aq. NaOH | 0.6 | Stirring tank | 93 | 93 |
| Example 1-107 | 5.0 | EtOH | 5.0 | 5.0 | 30 wt%-aq. NaOH | 0.6 | Stirring tank | 88 | 88 |
| Example 1-108 | 5.0 | n-BuOH | 5.0 | 5.0 | 30 wt%-aq. NaOH | 0.6 | Stirring tank | 90 | 90 |
| Example 1-109 | 5.0 | PhOH | 5.0 | 5.0 | 30 wt%-aq. NaOH | 0.6 | Stirring tank | 93 | 93 |
| Example 1-110 | 5.0 | PhOH | 5.0 | 5.0 | 30 wt%-aq. NaOH | 0.6 | Stirring tank | 93 | 93 |
| Example 1-111 | 5.0 | PhOH | 5.0 | 5.0 | 30 wt%-aq. NaOH | 0.6 | Stirring tank | 93 | 93 |
| Example 1-112 | 5.0 | PhOH | 5.0 | 5.0 | 30 wt%-aq. NaOH | 0.6 | Stirring tank | 93 | 93 |
| Example 1-113 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-114 | 5.0 | PhOH | 5.0 | 5.0 | 30 wt%-aq. NaOH | 0.6 | Stirring tank | 93 | 93 |
| Example 1-115 | 5.0 | PhOH | 5.0 | 5.0 | 30 wt%-aq. NaOH | 0.6 | Stirring tank | 93 | 93 |
| Example 1-116 | 5.0 | PhOH | 5.0 | 5.0 | 30 wt%-aq. NaOH | 0.6 | Stirring tank | 93 | 93 |
| Example 1-117 | 5.0 | PhOH | 5.0 | 5.0 | 30 wt%-aq. NaOH | 0.6 | Stirring tank | 90 | 90 |
| Example 1-118 | 5.0 | PhOH | 5.0 | 5.0 | 30 wt%-aq. NaOH | 0.6 | Stirring tank | 90 | 90 |
| Example 1-119 | 5.0 | PhOH | 5.0 | 5.0 | 30 wt%-aq. KOH | 0.6 | Stirring tank | 93 | 93 |
| Example 1-120 | 5.0 | PhOH | 5.0 | 5.0 | 30 wt%-aq. KOH | 0.6 | Stirring tank | 93 | 93 |
| Example 1-121 | 5.0 | n-BuOH | 5.0 | 5.0 | 30 wt%-aq. NaOH | 0.6 | Stirring tank | 90 | 90 |
| Example 1-122 | 5.0 | PhOH | 5.0 | 5.0 | 30 wt%-aq. NaOH | 0.6 | Stirring tank | 93 | 93 |
| Example 1-123 | 5.0 | PhOH | 5.0 | 5.0 | 30 wt%-aq. NaOH | 0.6 | Stirring tank | 93 | 93 |
| Example 1-124 | 5.0 | PhOH | 5.0 | 5.0 | 30 wt%-aq. NaOH | 0.6 | Stirring tank | 93 | 93 |
| Example 1-125 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-126 | 5.0 | PhOH | 5.0 | 5.0 | 30 wt%-aq. NaOH | 0.6 | Stirring tank | 93 | 93 |
| Example 1-127 | 5.0 | PhOH | 5.0 | 5.0 | 30 wt%-aq. NaOH | 0.6 | Stirring tank | 93 | 93 |
| Example 1-128 | 5.0 | PhOH | 5.0 | 5.0 | 30 wt%-aq. NaOH | 0.6 | Stirring tank | 93 | 93 |
| Example 1-129 | 5.0 | PhOH | 5.0 | 5.0 | 30 wt%-aq. NaOH | 0.6 | Stirring tank | 93 | 93 |
| Example 1-130 | 5.0 | EtOH | 5.0 | 5.0 | 30 wt%-aq. NaOH | 0.6 | Stirring tank | 88 | 88 |
| Example 1-131 | 5.0 | n-BuOH | 5.0 | 5.0 | 30 wt%-aq. NaOH | 0.6 | Stirring tank | 90 | 90 |
| Example 1-132 | 5.0 | PhOH | 5.0 | 5.0 | 30 wt%-aq. NaOH | 0.6 | Stirring tank | 93 | 93 |
| Example 1-133 | 5.0 | PhOH | 5.0 | 5.0 | 30 wt%-aq. NaOH | 0.6 | Stirring tank | 93 | 93 |
| Example 1-134 | 5.0 | PhOH | 5.0 | 5.0 | 30 wt%-aq. NaOH | 0.6 | Stirring tank | 93 | 93 |
| Example 1-135 | 5.0 | PhOH | 5.0 | 5.0 | 30 wt%-aq. NaOH | 0.6 | Stirring tank | 93 | 93 |
| Example 1-136 | 5.0 | PhOH | 5.0 | 5.0 | 30 wt%-aq. NaOH | 0.6 | Stirring tank | 93 | 93 |

[Table 1-15]

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-137 | 5.0 | PhOH | 5.0 | 5.0 | 30 wt%-aq. NaOH | 0.6 | Stirring tank | 93 | 93 |
| Example 1-138 | 5.0 | PhOH | 5.0 | 5.0 | 30 wt%-aq. NaOH | 0.6 | Stirring tank | 93 | 93 |
| Example 1-139 | 5.0 | PhOH | 5.0 | 5.0 | 30 wt%-aq. NaOH | 0.6 | Stirring tank | 93 | 93 |
| Example 1-140 | 5.0 | PhOH | 5.0 | 5.0 | 30 wt%-aq. NaOH | 0.6 | Stirring tank | 90 | 90 |
| Example 1-141 | 5.0 | PhOH | 5.0 | 5.0 | 30 wt%-aq. NaOH | 0.6 | Stirring tank | 90 | 90 |
| Example 1-142 | 5.0 | PhOH | 5.0 | 5.0 | 30 wt%-aq. KOH | 0.6 | Stirring tank | 93 | 93 |
| Example 1-143 | 5.0 | PhOH | 5.0 | 5.0 | 30 wt%-aq. KOH | 0.6 | Stirring tank | 93 | 93 |
| Example 1-144 | 5.0 | n-BuOH | 5.0 | 5.0 | 30 wt%-aq. NaOH | 0.6 | Stirring tank | 90 | 90 |
| Example 1-145 | 5.0 | PhOH | 5.0 | 5.0 | 30 wt%-aq. NaOH | 0.6 | Stirring tank | 93 | 93 |
| Example 1-146 | 5.0 | PhOH | 5.0 | 5.0 | 30 wt%-aq. NaOH | 0.6 | Stirring tank | 93 | 93 |
| Example 1-147 | 5.0 | PhOH | 5.0 | 5.0 | 30 wt%-aq. NaOH | 0.6 | Stirring tank | 93 | 93 |
| Example 1-148 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-149 | 5.0 | PhOH | 5.0 | 5.0 | 30 wt%-aq. NaOH | 0.6 | Stirring tank | 93 | 93 |
| Example 1-150 | 5.0 | PhOH | 5.0 | 5.0 | 30 wt%-aq. NaOH | 0.6 | Stirring tank | 93 | 93 |
| Example 1-151 | 5.0 | MeCN | 5.0 | 5.0 | 30 wt%-aq. NaOH | 0.6 | Stirring tank | 93 | 93 |
| Example 1-152 | 5.0 | MeCN | 5.0 | 5.0 | 30 wt%-aq. NaOH | 0.6 | Stirring tank | 93 | 93 |
| Example 1-153 | 5.0 | MeCN | 5.0 | 5.0 | 30 wt%-aq. NaOH | 0.6 | Stirring tank | 88 | 88 |
| Example 1-154 | 5.0 | n-BuOH | 5.0 | 5.0 | 30 wt%-aq. NaOH | 0.6 | Stirring tank | 90 | 90 |
| Example 1-155 | 5.0 | PhOH | 5.0 | 5.0 | 30 wt%-aq. NaOH | 0.6 | Stirring tank | 93 | 93 |
| Example 1-156 | 5.0 | PhOH | 5.0 | 5.0 | 30 wt%-aq. NaOH | 0.6 | Stirring tank | 93 | 93 |
| Example 1-157 | 5.0 | PhOH | 5.0 | 5.0 | 30 wt%-aq. NaOH | 0.6 | Stirring tank | 93 | 93 |
| Example 1-158 | 5.0 | PhOH | 5.0 | 5.0 | 30 wt%-aq. NaOH | 0.6 | Stirring tank | 93 | 93 |
| Example 1-159 | 5.0 | PhOH | 5.0 | 5.0 | 30 wt%-aq. NaOH | 0.6 | Stirring tank | 93 | 93 |
| Example 1-160 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-161 | 5.0 | PhOH | 5.0 | 5.0 | 30 wt%-aq. NaOH | 0.6 | Stirring tank | 93 | 93 |
| Example 1-162 | 5.0 | PhOH | 5.0 | 5.0 | 30 wt%-aq. NaOH | 0.6 | Stirring tank | 93 | 93 |
| Example 1-163 | 5.0 | PhOH | 5.0 | 5.0 | 30 wt%-aq. NaOH | 0.6 | Stirring tank | 90 | 90 |
| Example 1-164 | 5.0 | PhOH | 5.0 | 5.0 | 30 wt%-aq. NaOH | 0.6 | Stirring tank | 90 | 90 |
| Example 1-165 | 5.0 | PhOH | 5.0 | 5.0 | 30 wt%-aq. KOH | 0.6 | Stirring tank | 93 | 93 |
| Example 1-166 | 5.0 | PhOH | 5.0 | 5.0 | 30 wt%-aq. KOH | 0.6 | Stirring tank | 93 | 93 |
| Example 1-167 | 5.0 | n-BuOH | 5.0 | 5.0 | 30 wt%-aq. NaOH | 0.6 | Stirring tank | 90 | 90 |
| Example 1-168 | 5.0 | PhOH | 5.0 | 5.0 | 30 wt%-aq. NaOH | 0.6 | Stirring tank | 93 | 93 |
| Example 1-169 | 5.0 | PhOH | 5.0 | 5.0 | 30 wt%-aq. NaOH | 0.6 | Stirring tank | 93 | 93 |
| Example 1-170 | 5.0 | PhOH | 5.0 | 5.0 | 30 wt%-aq. NaOH | 0.6 | Stirring tank | 93 | 93 |
| Example 1-171 | 5.0 | PhOH | 5.0 | 5.0 | 30 wt%-aq. NaOH | 0.6 | Stirring tank | 93 | 93 |
| Example 1-172 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-173 | 5.0 | PhOH | 5.0 | 5.0 | 30 wt%-aq. NaOH | 0.6 | Stirring tank | 93 | 93 |
| Example 1-174 | 5.0 | PhOH | 5.0 | 5.0 | 30 wt%-aq. NaOH | 0.6 | Stirring tank | 93 | 93 |
| Example 1-175 | 5.0 | PhOH | 5.0 | 5.0 | 30 wt%-aq. NaOH | 0.6 | Stirring tank | 93 | 93 |
| Example 1-176 | 5.0 | EtOH | 5.0 | 5.0 | 30 wt%-aq. NaOH | 0.6 | Stirring tank | 88 | 88 |
| Example 1-177 | 5.0 | n-BuOH | 5.0 | 5.0 | 30 wt%-aq. NaOH | 0.6 | Stirring tank | 90 | 90 |
| Example 1-178 | 5.0 | PhOH | 5.0 | 5.0 | 30 wt%-aq. NaOH | 0.6 | Stirring tank | 93 | 93 |
| Example 1-179 | 5.0 | PhOH | 5.0 | 5.0 | 30 wt%-aq. NaOH | 0.6 | Stirring tank | 93 | 93 |
| Example 1-180 | 5.0 | PhOH | 5.0 | 5.0 | 30 wt%-aq. NaOH | 0.6 | Stirring tank | 93 | 93 |
| Example 1-181 | 5.0 | PhOH | 5.0 | 5.0 | 30 wt%-aq. NaOH | 0.6 | Stirring tank | 93 | 93 |
| Example 1-182 | 5.0 | PhOH | 5.0 | 5.0 | 30 wt%-aq. NaOH | 0.6 | Stirring tank | 93 | 93 |
| Example 1-183 | 5.0 | PhOH | 5.0 | 5.0 | 30 wt%-aq. NaOH | 0.6 | Stirring tank | 93 | 93 |
| Example 1-184 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-185 | 5.0 | PhOH | 5.0 | 5.0 | 30 wt%- aq. NaOH | 0.6 | Stirring tank | 93 | 93 |
| Example 1-186 | 5.0 | PhOH | 5.0 | 5.0 | 30 wt%- aq. NaOH | 0.6 | Stirring tank | 90 | 90 |
| Example 1-187 | 5.0 | PhOH | 5.0 | 5.0 | 30 wt%- aq. NaOH | 0.6 | Stirring tank | 90 | 90 |
| Example 1-188 | 5.0 | PhOH | 5.0 | 5.0 | 30 wt%- aq. KOH | 0.6 | Stirring tank | 93 | 93 |
| Example 1-189 | 5.0 | PhOH | 5.0 | 5.0 | 30 wt%- aq. KOH | 0.6 | Stirring tank | 93 | 93 |
| Example 1-190 | 5.0 | n-BuOH | 5.0 | 5.0 | 30 wt%- aq. NaOH | 0.6 | Stirring tank | 90 | 90 |
| Example 1-191 | 5.0 | PhOH | 5.0 | 5.0 | 30 wt%- aq. NaOH | 0.6 | Stirring tank | 93 | 93 |
| Example 1-192 | 5.0 | PhOH | 5.0 | 5.0 | 30 wt%- aq. NaOH | 0.6 | Stirring tank | 93 | 93 |
| Example 1-193 | 5.0 | PhOH | 5.0 | 5.0 | 30 wt%- aq. NaOH | 0.6 | Stirring tank | 93 | 93 |
| Example 1-194 | 5.0 | PhOH | 5.0 | 5.0 | 30 wt%- aq. NaOH | 0.6 | Stirring tank | 93 | 93 |
| Example 1-195 | 5.0 | PhOH | 5.0 | 5.0 | 30 wt%- aq. NaOH | 0.6 | Stirring tank | 93 | 93 |
| Example 1-196 | 5.0 | PhOH | 5.0 | 5.0 | 30 wt%- aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-197 | 5.0 | PhOH | 5.0 | 5.0 | 30 wt%-aq. NaOH | 0.6 | Stirring tank | 93 | 93 |
| Example 1-198 | 5.0 | PhOH | 5.0 | 5.0 | 30 wt%-aq. NaOH | 0.6 | Stirring tank | 93 | 93 |
| Example 1-199 | 5.0 | EtOH | 5.0 | 5.0 | 30 wt%-aq. NaOH | 0.6 | Stirring tank | 88 | 88 |
| Example 1-200 | 5.0 | n-BuOH | 5.0 | 5.0 | 30 wt%-aq. NaOH | 0.6 | Stirring tank | 90 | 90 |
| Example 1-201 | 5.0 | PhOH | 5.0 | 5.0 | 30 wt%-aq. NaOH | 0.6 | Stirring tank | 93 | 93 |
| Example 1-202 | 5.0 | PhOH | 5.0 | 5.0 | 30 wt%-aq. NaOH | 0.6 | Stirring tank | 93 | 93 |
| Example 1-203 | 5.0 | PhOH | 5.0 | 5.0 | 30 wt%-aq. NaOH | 0.6 | Stirring tank | 93 | 93 |
| Example 1-204 | 5.0 | PhOH | 5.0 | 5.0 | 30 wt%-aq. NaOH | 0.6 | Stirring tank | 93 | 93 |
| Example 1-205 | 5.0 | PhOH | 5.0 | 5.0 | 30 wt%-aq. NaOH | 0.6 | Stirring tank | 93 | 93 |
| Example 1-206 | 5.0 | PhOH | 5.0 | 5.0 | 30 wt%-aq. NaOH | 0.6 | Stirring tank | 93 | 93 |
| Example 1-207 | 5.0 | PhOH | 5.0 | 5.0 | 30 wt%-aq. NaOH | 0.6 | Stirring tank | 93 | 93 |

[Table 1-16]

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-208 | 5.0 | PhOH | 5.0 | 5.0 | 30 wt%-aq. NaOH | 0.6 | Stirring tank | 93 | 93 |
| Example 1-209 | 5.0 | PhOH | 5.0 | 5.0 | 30 wt%-aq. NaOH | 0.6 | Stirring tank | 90 | 90 |
| Example 1-210 | 5.0 | PhOH | 5.0 | 5.0 | 30 wt%-aq. NaOH | 0.6 | Stirring tank | 90 | 90 |
| Example 1-211 | 5.0 | PhOH | 5.0 | 5.0 | 30 wt%-aq. KOH | 0.6 | Stirring tank | 93 | 93 |
| Example 1-212 | 5.0 | PhOH | 5.0 | 5.0 | 30 wt%-aq. KOH | 0.6 | Stirring tank | 93 | 93 |
| Example 1-213 | 5.0 | n-BuOH | 5.0 | 5.0 | 30 wt%-aq. NaOH | 0.6 | Stirring tank | 90 | 90 |
| Example 1-214 | 5.0 | PhOH | 5.0 | 5.0 | 30 wt%-aq. NaOH | 0.6 | Stirring tank | 93 | 93 |
| Example 1-215 | 5.0 | PhOH | 5.0 | 5.0 | 30 wt%-aq. NaOH | 0.6 | Stirring tank | 93 | 93 |
| Example 1-216 | 5.0 | PhOH | 5.0 | 5.0 | 30 wt%-aq. NaOH | 0.6 | Stirring tank | 93 | 93 |
| Example 1-217 | 5.0 | PhOH | 5.0 | 5.0 | 30 wt%-aq. NaOH | 0.6 | Stirring tank | 93 | 93 |
| Example 1-218 | 5.0 | PhOH | 5.0 | 5.0 | 30 wt%-aq. NaOH | 0.6 | Stirring tank | 93 | 93 |
| Example 1-219 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-220 | 5.0 | PhOH | 5.0 | 5.0 | 30 wt%-aq. NaOH | 0.6 | Stirring tank | 93 | 93 |
| Example 1-221 | 5.0 | PhOH | 5.0 | 5.0 | 30 wt%-aq. NaOH | 0.6 | Stirring tank | 93 | 93 |
| Example 1-222 | 5.0 | EtOH | 5.0 | 5.0 | 30 wt%-aq. NaOH | 0.6 | Stirring tank | 88 | 88 |
| Example 1-223 | 5.0 | n-BuOH | 5.0 | 5.0 | 30 wt%-aq. NaOH | 0.6 | Stirring tank | 90 | 90 |
| Example 1-224 | 5.0 | PhOH | 5.0 | 5.0 | 30 wt%-aq. NaOH | 0.6 | Stirring tank | 93 | 93 |
| Example 1-225 | 5.0 | PhOH | 5.0 | 5.0 | 30 wt%-aq. NaOH | 0.6 | Stirring tank | 93 | 93 |
| Example 1-226 | 5.0 | PhOH | 5.0 | 5.0 | 30 wt%-aq. NaOH | 0.6 | Stirring tank | 93 | 93 |
| Example 1-227 | 5.0 | PhOH | 5.0 | 5.0 | 30 wt%-aq. NaOH | 0.6 | Stirring tank | 93 | 93 |
| Example 1-228 | 5.0 | PhOH | 5.0 | 5.0 | 30 wt%-aq. NaOH | 0.6 | Stirring tank | 93 | 93 |
| Example 1-229 | 5.0 | PhOH | 5.0 | 5.0 | 30 wt%-aq. NaOH | 0.6 | Stirring tank | 93 | 93 |
| Example 1-230 | 5.0 | PhOH | 5.0 | 5.0 | 30 wt%-aq. NaOH | 0.6 | Stirring tank | 93 | 93 |
| Example 1-231 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-232 | 5.0 | PhOH | 5.0 | 5.0 | 30 wt%-aq. NaOH | 0.6 | Stirring tank | 90 | 90 |
| Example 1-233 | 5.0 | PhOH | 5.0 | 5.0 | 30 wt%-aq. NaOH | 0.6 | Stirring tank | 90 | 90 |
| Example 1-234 | 5.0 | PhOH | 5.0 | 5.0 | 30 wt%-aq. KOH | 0.6 | Stirring tank | 93 | 93 |
| Example 1-235 | 5.0 | PhOH | 5.0 | 5.0 | 30 wt%-aq. KOH | 0.6 | Stirring tank | 93 | 93 |
| Example 1-236 | 5.0 | n-BuOH | 5.0 | 5.0 | 30 wt%-aq. NaOH | 0.6 | Stirring tank | 90 | 90 |
| Example 1-237 | 5.0 | PhOH | 5.0 | 5.0 | 30 wt%-aq. NaOH | 0.6 | Stirring tank | 93 | 93 |
| Example 1-238 | 5.0 | PhOH | 5.0 | 5.0 | 30 wt%-aq. NaOH | 0.6 | Stirring tank | 93 | 93 |
| Example 1-239 | 5.0 | PhOH | 5.0 | 5.0 | 30 wt%-aq. NaOH | 0.6 | Stirring tank | 93 | 93 |
| Example 1-240 | 5.0 | PhOH | 5.0 | 5.0 | 30 wt%-aq. NaOH | 0.6 | Stirring tank | 93 | 93 |
| Example 1-241 | 5.0 | PhOH | 5.0 | 5.0 | 30 wt%-aq. NaOH | 0.6 | Stirring tank | 93 | 93 |
| Example 1-242 | 5.0 | PhOH | 5.0 | 5.0 | 30 wt%-aq. NaOH | 0.6 | Stirring tank | 93 | 93 |
| Example 1-243 | 5.0 | PhOH | 5.0 | 5.0 | 30 wt%-aq. NaOH | 0.6 | Stirring tank | 93 | 93 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-244 | 5.0 | PhOH | 5.0 | 5.0 | 30 wt%-aq. NaOH | 0.6 | Stirring tank | 93 | 93 |
| Example 1-245 | 5.0 | EtOH | 5.0 | 5.0 | 30 wt%-aq. NaOH | 0.6 | Stirring tank | 88 | 88 |
| Example 1-246 | 5.0 | n-BuOH | 5.0 | 5.0 | 30 wt%-aq. NaOH | 0.6 | Stirring tank | 90 | 90 |
| Example 1-247 | 5.0 | PhOH | 5.0 | 5.0 | 30 wt%-aq. NaOH | 0.6 | Stirring tank | 93 | 93 |
| Example 1-248 | 5.0 | PhOH | 5.0 | 5.0 | 30 wt%-aq. NaOH | 0.6 | Stirring tank | 93 | 93 |
| Example 1-249 | 5.0 | PhOH | 5.0 | 5.0 | 30 wt%-aq. NaOH | 0.6 | Stirring tank | 93 | 93 |
| Example 1-250 | 5.0 | PhOH | 5.0 | 5.0 | 30 wt%-aq. NaOH | 0.6 | Stirring tank | 93 | 93 |
| Example 1-251 | 5.0 | PhOH | 5.0 | 5.0 | 30 wt%-aq. NaOH | 0.6 | Stirring tank | 93 | 93 |
| Example 1-252 | 5.0 | PhOH | 5.0 | 5.0 | 30 wt%-aq. NaOH | 0.6 | Stirring tank | 93 | 93 |
| Example 1-253 | 5.0 | PhOH | 5.0 | 5.0 | 30 wt%-aq. NaOH | 0.6 | Stirring tank | 93 | 93 |
| Example 1-254 | 5.0 | PhOH | 5.0 | 5.0 | 30 wt%-aq. NaOH | 0.6 | Stirring tank | 93 | 93 |
| Example 1-255 | 5.0 | PhOH | 5.0 | 5.0 | 30 wt%-aq. NaOH | 0.6 | Stirring tank | 90 | 90 |

(continued)

| | Step (5) | | | | | | | | |
|---|---|---|---|---|---|---|---|---|---|
| | Liquid-phase component | Organic solvent | | Water | Alkali | | Reaction form | Amine recovery rate | Hydroxy compound recovery rate |
| | kg | Type | Blending amount kg | Blending amount kg | Type | Blending amount kg | | % | % |
| Example 1-256 | 5.0 | PhOH | 5.0 | 5.0 | 30 wt%-aq. NaOH | 0.6 | Stirring tank | 90 | 90 |
| Example 1-257 | 5.0 | PhOH | 5.0 | 5.0 | 30 wt%-aq. KOH | 0.6 | Stirring tank | 93 | 93 |
| Example 1-258 | 5.0 | PhOH | 5.0 | 5.0 | 30 wt%-aq. KOH | 0.6 | Stirring tank | 93 | 93 |
| Example 1-259 | 5.0 | PhOH | 5.0 | 5.0 | 30 wt%-aq. NaOH | 0.6 | Stirring tank | 48 | 93 |
| Example 1-260 | 5.0 | PhOH | 5.0 | 5.0 | 30 wt%-aq. NaOH | 0.6 | Stirring tank | 48 | 93 |
| Example 1-261 | 5.0 | EtOH | 5.0 | 5.0 | 30 wt%-aq. NaOH | 0.6 | Stirring tank | 48 | 93 |
| Example 1-262 | 5.0 | PhOH | 5.0 | 5.0 | 30 wt%-aq. NaOH | 0.6 | Stirring tank | 48 | 93 |
| Example 1-263 | 5.0 | PhOH | 5.0 | 5.0 | 30 wt%-aq. NaOH | 0.6 | Stirring tank | 48 | 93 |
| Example 1-264 | 5.0 | EtOH | 5.0 | 5.0 | 30 wt%-aq. NaOH | 0.6 | Stirring tank | 48 | 93 |
| Example 1-265 | 5.0 | PhOH | 5.0 | 5.0 | 30 wt%-aq. NaOH | 0.6 | Stirring tank | 48 | 93 |
| Example 1-266 | 5.0 | PhOH | 5.0 | 5.0 | 30 wt%-aq. NaOH | 0.6 | Stirring tank | 48 | 93 |
| Example 1-267 | 5.0 | EtOH | 5.0 | 5.0 | 30 wt%-aq. NaOH | 0.6 | Stirring tank | 48 | 93 |

[Table 1-17]

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-1 | 95 | 40 | 20 | 1.3 | 0.6 | 0.54 | 0.16 |
| Example 1-2 | 95 | 165 | 20 | 1.3 | 0.6 | 0.54 | 0.16 |
| Example 1-3 | 95 | 40 | 20 | 1.3 | 0.6 | 0.54 | 0.16 |
| Example 1-4 | 95 | 40 | 20 | 1.3 | 0.6 | 0.54 | 0.16 |
| Example 1-5 | 95 | 40 | 20 | 1.3 | 0.6 | 0.54 | 0.16 |
| Example 1-6 | 95 | 40 | 20 | 1.3 | 0.6 | 0.54 | 0.16 |
| Example 1-7 | 95 | 40 | 20 | 1.3 | 0.6 | 0.54 | 0.16 |
| Example 1-8 | 95 | 40 | 20 | 1.3 | 0.6 | 0.54 | 0.16 |
| Example 1-9 | 95 | 40 | 20 | 1.3 | 0.6 | 0.54 | 0.16 |
| Example 1-10 | 95 | 4 | 20 | 1.3 | 0.6 | 0.54 | 0.16 |
| Example 1-11 | 95 | 4 | 20 | 1.3 | 0.6 | 0.54 | 0.16 |
| Example 1-12 | 95 | 40 | 20 | 1.3 | 0.6 | 0.54 | 0.16 |
| Example 1-13 | 95 | 40 | 20 | 1.3 | 0.6 | 0.54 | 0.16 |
| Example 1-14 | 95 | 4 | 20 | 1.3 | 0.6 | 0.54 | 0.16 |
| Example 1-15 | 95 | 40 | 20 | 1.3 | 0.6 | 0.54 | 0.16 |
| Example 1-16 | 95 | 4 | 20 | 1.3 | 0.6 | 0.54 | 0.16 |
| Example 1-17 | 95 | 4 | 20 | 1.3 | 0.6 | 0.54 | 0.16 |
| Example 1-18 | 95 | 4 | 20 | 1.3 | 0.6 | 0.54 | 0.16 |
| Example 1-19 | 95 | 40 | 20 | 1.3 | 0.6 | 0.54 | 0.16 |
| Example 1-20 | 95 | 4 | 20 | 1.3 | 0.6 | 0.54 | 0.16 |
| Example 1-21 | 95 | 40 | 20 | 1.3 | 0.6 | 0.54 | 0.16 |
| Example 1-22 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-23 | 95 | 40 | 20 | 1.3 | 0.6 | 0.54 | 0.16 |
| Example 1-24 | 95 | 4 | 20 | 1.3 | 0.6 | 0.54 | 0.16 |
| Example 1-25 | 95 | 40 | 20 | 1.3 | 0.6 | 0.54 | 0.16 |
| Example 1-26 | 95 | 4 | 20 | 1.3 | 0.6 | 0.54 | 0.16 |
| Example 1-27 | 95 | 40 | 20 | 1.3 | 0.6 | 0.54 | 0.16 |
| Example 1-28 | 95 | 4 | 20 | 1.3 | 0.6 | 0.54 | 0.16 |
| Comparative Example 1-1 | - | - | - | - | - | - | - |
| Comparative Example 1-2 | - | - | - | - | - | - | - |
| Comparative Example 1-3 | - | - | - | - | - | - | - |
| Comparative Example 1-4 | - | - | - | - | - | - | - |
| Comparative Example 1-5 | - | - | - | - | - | - | - |
| Comparative Example 1-6 | 95 | 4 | 20 | 1.3 | 0.6 | 0.54 | 0.16 |
| Example 1-29 | 95 | 40 | 20 | 1.3 | 0.6 | 0.54 | 0.16 |
| Example 1-30 | 95 | 4 | 20 | 1.3 | 0.6 | 0.54 | 0.16 |
| Example 1-31 | 95 | 4 | 20 | 1.3 | 0.6 | 0.54 | 0.16 |
| Example 1-32 | 95 | 4 | 20 | 1.3 | 0.6 | 0.54 | 0.16 |
| Example 1-33 | 95 | 4 | 20 | 1.3 | 0.6 | 0.54 | 0.16 |
| Example 1-34 | 95 | 4 | 20 | 1.3 | 0.6 | 0.54 | 0.16 |
| Example 1-35 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-36 | 95 | 4 | 20 | 1.3 | 0.6 | 0.54 | 0.16 |
| Example 1-37 | 95 | 4 | 20 | 1.3 | 0.6 | 0.54 | 0.16 |
| Example 1-38 | 95 | 190 | 20 | 1.3 | 0.6 | 0.54 | 0.16 |
| Example 1-39 | 95 | 40 | 20 | 1.3 | 0.6 | 0.54 | 0.16 |
| Example 1-40 | 95 | 4 | 20 | 1.3 | 0.6 | 0.54 | 0.16 |
| Example 1-41 | 95 | 4 | 20 | 1.3 | 0.6 | 0.54 | 0.16 |
| Example 1-42 | 95 | 4 | 20 | 1.3 | 0.6 | 0.54 | 0.16 |
| Example 1-43 | 95 | 4 | 20 | 1.3 | 0.6 | 0.54 | 0.16 |
| Example 1-44 | 95 | 4 | 20 | 1.3 | 0.6 | 0.54 | 0.16 |
| Example 1-45 | 95 | 4 | 20 | 1.3 | 0.6 | 0.54 | 0.16 |
| Example 1-46 | 95 | 4 | 20 | 1.3 | 0.6 | 0.54 | 0.16 |
| Example 1-47 | 95 | 4 | 20 | 1.3 | 0.6 | 0.54 | 0.16 |
| Example 1-48 | 95 | 40 | 20 | 1.3 | 0.6 | 0.54 | 0.16 |
| Example 1-49 | 95 | 4 | 20 | 1.3 | 0.6 | 0.54 | 0.16 |
| Example 1-50 | 95 | 4 | 20 | 1.3 | 0.6 | 0.54 | 0.16 |
| Example 1-51 | 95 | 4 | 20 | 1.3 | 0.6 | 0.54 | 0.16 |
| Example 1-52 | 95 | 40 | 20 | 1.3 | 0.6 | 0.54 | 0.16 |
| Example 1-53 | 95 | 4 | 20 | 1.3 | 0.6 | 0.54 | 0.16 |
| Example 1-54 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-55 | 95 | 4 | 20 | 1.3 | 0.6 | 0.54 | 0.16 |
| Example 1-56 | 95 | 4 | 20 | 1.3 | 0.6 | 0.54 | 0.16 |
| Example 1-57 | 95 | 4 | 20 | 1.3 | 0.6 | 0.54 | 0.16 |
| Example 1-58 | 95 | 4 | 20 | 1.3 | 0.6 | 0.54 | 0.16 |
| Example 1-59 | 95 | 4 | 20 | 1.3 | 0.6 | 0.54 | 0.16 |
| Example 1-60 | 95 | 4 | 20 | 1.3 | 0.6 | 0.54 | 0.16 |
| Example 1-61 | 95 | 190 | 20 | 1.3 | 0.6 | 0.54 | 0.16 |
| Example 1-62 | 95 | 40 | 20 | 1.3 | 0.6 | 0.54 | 0.16 |
| Example 1-63 | 95 | 4 | 20 | 1.3 | 0.6 | 0.54 | 0.16 |
| Example 1-64 | 95 | 4 | 20 | 1.3 | 0.6 | 0.54 | 0.16 |
| Example 1-65 | 95 | 4 | 20 | 1.3 | 0.6 | 0.54 | 0.16 |

[Table 1-18]

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-66 | 95 | 4 | 20 | 1.3 | 0.6 | 0.54 | 0.16 |
| Example 1-67 | 95 | 4 | 20 | 1.3 | 0.6 | 0.54 | 0.16 |
| Example 1-68 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-69 | 95 | 4 | 20 | 1.3 | 0.6 | 0.54 | 0.16 |
| Example 1-70 | 95 | 4 | 20 | 1.3 | 0.6 | 0.54 | 0.16 |
| Example 1-71 | 95 | 40 | 20 | 1.3 | 0.6 | 0.54 | 0.16 |
| Example 1-72 | 95 | 4 | 20 | 1.3 | 0.6 | 0.54 | 0.16 |
| Example 1-73 | 95 | 4 | 20 | 1.3 | 0.6 | 0.54 | 0.16 |
| Example 1-74 | 95 | 4 | 20 | 1.3 | 0.6 | 0.54 | 0.16 |
| Example 1-75 | 95 | 40 | 20 | 1.3 | 0.6 | 0.54 | 0.16 |
| Example 1-76 | 95 | 4 | 20 | 1.3 | 0.6 | 0.54 | 0.16 |
| Example 1-77 | 95 | 4 | 20 | 1.3 | 0.6 | 0.54 | 0.16 |
| Example 1-78 | 95 | 4 | 20 | 1.3 | 0.6 | 0.54 | 0.16 |
| Example 1-79 | 95 | 4 | 20 | 1.3 | 0.6 | 0.54 | 0.16 |
| Example 1-80 | 95 | 4 | 20 | 1.3 | 0.6 | 0.54 | 0.16 |
| Example 1-81 | 95 | 4 | 20 | 1.3 | 0.6 | 0.54 | 0.16 |
| Example 1-82 | 95 | 4 | 20 | 1.3 | 0.6 | 0.54 | 0.16 |
| Example 1-83 | 95 | 4 | 20 | 1.3 | 0.6 | 0.54 | 0.16 |
| Example 1-84 | 95 | 190 | 20 | 1.3 | 0.6 | 0.54 | 0.16 |
| Example 1-85 | 95 | 40 | 20 | 1.3 | 0.6 | 0.54 | 0.16 |
| Example 1-86 | 95 | 4 | 20 | 1.3 | 0.6 | 0.54 | 0.16 |
| Example 1-87 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-88 | 95 | 4 | 20 | 1.3 | 0.6 | 0.54 | 0.16 |
| Example 1-89 | 95 | 4 | 20 | 1.3 | 0.6 | 0.54 | 0.16 |
| Example 1-90 | 95 | 4 | 20 | 1.3 | 0.6 | 0.54 | 0.16 |
| Example 1-91 | 95 | 4 | 20 | 1.3 | 0.6 | 0.54 | 0.16 |
| Example 1-92 | 95 | 4 | 20 | 1.3 | 0.6 | 0.54 | 0.16 |
| Example 1-93 | 95 | 4 | 20 | 1.3 | 0.6 | 0.54 | 0.16 |
| Example 1-94 | 95 | 40 | 20 | 1.3 | 0.6 | 0.54 | 0.16 |
| Example 1-95 | 95 | 4 | 20 | 1.3 | 0.6 | 0.54 | 0.16 |
| Example 1-96 | 95 | 4 | 20 | 1.3 | 0.6 | 0.54 | 0.16 |
| Example 1-97 | 95 | 4 | 20 | 1.3 | 0.6 | 0.54 | 0.16 |
| Example 1-98 | 95 | 40 | 20 | 1.3 | 0.6 | 0.54 | 0.16 |
| Example 1-99 | 95 | 4 | 20 | 1.3 | 0.6 | 0.54 | 0.16 |
| Example 1-100 | 95 | 4 | 20 | 1.3 | 0.6 | 0.54 | 0.16 |
| Example 1-101 | 95 | 4 | 20 | 1.3 | 0.6 | 0.54 | 0.16 |
| Example 1-102 | 95 | 4 | 20 | 1.3 | 0.6 | 0.54 | 0.16 |
| Example 1-103 | 95 | 4 | 20 | 1.3 | 0.6 | 0.54 | 0.16 |
| Example 1-104 | 95 | 4 | 20 | 1.3 | 0.6 | 0.54 | 0.16 |
| Example 1-105 | 95 | 4 | 20 | 1.3 | 0.6 | 0.54 | 0.16 |
| Example 1-106 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-107 | 95 | 190 | 20 | 1.3 | 0.6 | 0.54 | 0.16 |
| Example 1-108 | 95 | 40 | 20 | 1.3 | 0.6 | 0.54 | 0.16 |
| Example 1-109 | 95 | 4 | 20 | 1.3 | 0.6 | 0.54 | 0.16 |
| Example 1-110 | 95 | 4 | 20 | 1.3 | 0.6 | 0.54 | 0.16 |
| Example 1-111 | 95 | 4 | 20 | 1.3 | 0.6 | 0.54 | 0.16 |
| Example 1-112 | 95 | 4 | 20 | 1.3 | 0.6 | 0.54 | 0.16 |
| Example 1-113 | 95 | 4 | 20 | 1.3 | 0.6 | 0.54 | 0.16 |
| Example 1-114 | 95 | 4 | 20 | 1.3 | 0.6 | 0.54 | 0.16 |
| Example 1-115 | 95 | 4 | 20 | 1.3 | 0.6 | 0.54 | 0.16 |
| Example 1-116 | 95 | 4 | 20 | 1.3 | 0.6 | 0.54 | 0.16 |
| Example 1-117 | 95 | 40 | 20 | 1.3 | 0.6 | 0.54 | 0.16 |
| Example 1-118 | 95 | 4 | 20 | 1.3 | 0.6 | 0.54 | 0.16 |
| Example 1-119 | 95 | 4 | 20 | 1.3 | 0.6 | 0.54 | 0.16 |
| Example 1-120 | 95 | 4 | 20 | 1.3 | 0.6 | 0.54 | 0.16 |
| Example 1-121 | 95 | 40 | 20 | 1.3 | 0.6 | 0.54 | 0.16 |
| Example 1-122 | 95 | 4 | 20 | 1.3 | 0.6 | 0.54 | 0.16 |
| Example 1-123 | 95 | 4 | 20 | 1.3 | 0.6 | 0.54 | 0.16 |
| Example 1-124 | 95 | 4 | 20 | 1.3 | 0.6 | 0.54 | 0.16 |
| Example 1-125 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-126 | 95 | 4 | 20 | 1.3 | 0.6 | 0.54 | 0.16 |
| Example 1-127 | 95 | 4 | 20 | 1.3 | 0.6 | 0.54 | 0.16 |
| Example 1-128 | 95 | 4 | 20 | 1.3 | 0.6 | 0.54 | 0.16 |
| Example 1-129 | 95 | 4 | 20 | 1.3 | 0.6 | 0.54 | 0.16 |
| Example 1-130 | 95 | 190 | 20 | 1.3 | 0.6 | 0.54 | 0.16 |
| Example 1-131 | 95 | 40 | 20 | 1.3 | 0.6 | 0.54 | 0.16 |
| Example 1-132 | 95 | 4 | 20 | 1.3 | 0.6 | 0.54 | 0.16 |
| Example 1-133 | 95 | 4 | 20 | 1.3 | 0.6 | 0.54 | 0.16 |
| Example 1-134 | 95 | 4 | 20 | 1.3 | 0.6 | 0.54 | 0.16 |
| Example 1-135 | 95 | 4 | 20 | 1.3 | 0.6 | 0.54 | 0.16 |
| Example 1-136 | 95 | 4 | 20 | 1.3 | 0.6 | 0.54 | 0.16 |

[Table 1-19]

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-137 | 95 | 4 | 20 | 1.3 | 0.6 | 0.54 | 0.16 |
| Example 1-138 | 95 | 4 | 20 | 1.3 | 0.6 | 0.54 | 0.16 |
| Example 1-139 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-140 | 95 | 40 | 20 | 1.3 | 0.6 | 0.54 | 0.16 |
| Example 1-141 | 95 | 4 | 20 | 1.3 | 0.6 | 0.54 | 0.16 |
| Example 1-142 | 95 | 4 | 20 | 1.3 | 0.6 | 0.54 | 0.16 |
| Example 1-143 | 95 | 4 | 20 | 1.3 | 0.6 | 0.54 | 0.16 |
| Example 1-144 | 95 | 40 | 20 | 1.3 | 0.6 | 0.54 | 0.16 |
| Example 1-145 | 95 | 4 | 20 | 1.3 | 0.6 | 0.54 | 0.16 |
| Example 1-146 | 95 | 4 | 20 | 1.3 | 0.6 | 0.54 | 0.16 |
| Example 1-147 | 95 | 4 | 20 | 1.3 | 0.6 | 0.54 | 0.16 |
| Example 1-148 | 95 | 4 | 20 | 1.3 | 0.6 | 0.54 | 0.16 |
| Example 1-149 | 95 | 4 | 20 | 1.3 | 0.6 | 0.54 | 0.16 |
| Example 1-150 | 95 | 4 | 20 | 1.3 | 0.6 | 0.54 | 0.16 |
| Example 1-151 | 95 | 4 | 20 | 1.3 | 0.6 | 0.54 | 0.16 |
| Example 1-152 | 95 | 4 | 20 | 1.3 | 0.6 | 0.54 | 0.16 |
| Example 1-153 | 95 | 190 | 20 | 1.3 | 0.6 | 0.54 | 0.16 |
| Example 1-154 | 95 | 40 | 20 | 1.3 | 0.6 | 0.54 | 0.16 |
| Example 1-155 | 95 | 4 | 20 | 1.3 | 0.6 | 0.54 | 0.16 |
| Example 1-156 | 95 | 4 | 20 | 1.3 | 0.6 | 0.54 | 0.16 |
| Example 1-157 | 95 | 4 | 20 | 1.3 | 0.6 | 0.54 | 0.16 |
| Example 1-158 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-159 | 95 | 4 | 20 | 1.3 | 0.6 | 0.54 | 0.16 |
| Example 1-160 | 95 | 4 | 20 | 1.3 | 0.6 | 0.54 | 0.16 |
| Example 1-161 | 95 | 4 | 20 | 1.3 | 0.6 | 0.54 | 0.16 |
| Example 1-162 | 95 | 4 | 20 | 1.3 | 0.6 | 0.54 | 0.16 |
| Example 1-163 | 95 | 40 | 20 | 1.3 | 0.6 | 0.54 | 0.16 |
| Example 1-164 | 95 | 4 | 20 | 1.3 | 0.6 | 0.54 | 0.16 |
| Example 1-165 | 95 | 4 | 20 | 1.3 | 0.6 | 0.54 | 0.16 |
| Example 1-166 | 95 | 4 | 20 | 1.3 | 0.6 | 0.54 | 0.16 |
| Example 1-167 | 95 | 40 | 20 | 1.3 | 0.6 | 0.54 | 0.16 |
| Example 1-168 | 95 | 4 | 20 | 1.3 | 0.6 | 0.54 | 0.16 |
| Example 1-169 | 95 | 4 | 20 | 1.3 | 0.6 | 0.54 | 0.16 |
| Example 1-170 | 95 | 4 | 20 | 1.3 | 0.6 | 0.54 | 0.16 |
| Example 1-171 | 95 | 4 | 20 | 1.3 | 0.6 | 0.54 | 0.16 |
| Example 1-172 | 95 | 4 | 20 | 1.3 | 0.6 | 0.54 | 0.16 |
| Example 1-173 | 95 | 4 | 20 | 1.3 | 0.6 | 0.54 | 0.16 |
| Example 1-174 | 95 | 4 | 20 | 1.3 | 0.6 | 0.54 | 0.16 |
| Example 1-175 | 95 | 4 | 20 | 1.3 | 0.6 | 0.54 | 0.16 |
| Example 1-176 | 95 | 190 | 20 | 1.3 | 0.6 | 0.54 | 0.16 |
| Example 1-177 | 95 | 40 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-178 | 95 | 4 | 20 | 1.3 | 0.6 | 0.54 | 0.16 |
| Example 1-179 | 95 | 4 | 20 | 1.3 | 0.6 | 0.54 | 0.16 |
| Example 1-180 | 95 | 4 | 20 | 1.3 | 0.6 | 0.54 | 0.16 |
| Example 1-181 | 95 | 4 | 20 | 1.3 | 0.6 | 0.54 | 0.16 |
| Example 1-182 | 95 | 4 | 20 | 1.3 | 0.6 | 0.54 | 0.16 |
| Example 1-183 | 95 | 4 | 20 | 1.3 | 0.6 | 0.54 | 0.16 |
| Example 1-184 | 95 | 4 | 20 | 1.3 | 0.6 | 0.54 | 0.16 |
| Example 1-185 | 95 | 4 | 20 | 1.3 | 0.6 | 0.54 | 0.16 |
| Example 1-186 | 95 | 40 | 20 | 1.3 | 0.6 | 0.54 | 0.16 |
| Example 1-187 | 95 | 4 | 20 | 1.3 | 0.6 | 0.54 | 0.16 |
| Example 1-188 | 95 | 4 | 20 | 1.3 | 0.6 | 0.54 | 0.16 |
| Example 1-189 | 95 | 4 | 20 | 1.3 | 0.6 | 0.54 | 0.16 |
| Example 1-190 | 95 | 40 | 20 | 1.3 | 0.6 | 0.54 | 0.16 |
| Example 1-191 | 95 | 4 | 20 | 1.3 | 0.6 | 0.54 | 0.16 |
| Example 1-192 | 95 | 4 | 20 | 1.3 | 0.6 | 0.54 | 0.16 |
| Example 1-193 | 95 | 4 | 20 | 1.3 | 0.6 | 0.54 | 0.16 |
| Example 1-194 | 95 | 4 | 20 | 1.3 | 0.6 | 0.54 | 0.16 |
| Example 1-195 | 95 | 4 | 20 | 1.3 | 0.6 | 0.54 | 0.16 |
| Example 1-196 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-197 | 95 | 4 | 20 | 1.3 | 0.6 | 0.54 | 0.16 |
| Example 1-198 | 95 | 4 | 20 | 1.3 | 0.6 | 0.54 | 0.16 |
| Example 1-199 | 95 | 190 | 20 | 1.3 | 0.6 | 0.54 | 0.16 |
| Example 1-200 | 95 | 40 | 20 | 1.3 | 0.6 | 0.54 | 0.16 |
| Example 1-201 | 95 | 4 | 20 | 1.3 | 0.6 | 0.54 | 0.16 |
| Example 1-202 | 95 | 4 | 20 | 1.3 | 0.6 | 0.54 | 0.16 |
| Example 1-203 | 95 | 4 | 20 | 1.3 | 0.6 | 0.54 | 0.16 |
| Example 1-204 | 95 | 4 | 20 | 1.3 | 0.6 | 0.54 | 0.16 |
| Example 1-205 | 95 | 4 | 20 | 1.3 | 0.6 | 0.54 | 0.16 |
| Example 1-206 | 95 | 4 | 20 | 1.3 | 0.6 | 0.54 | 0.16 |
| Example 1-207 | 95 | 4 | 20 | 1.3 | 0.6 | 0.54 | 0.16 |

[Table 1-20]

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-208 | 95 | 4 | 20 | 1.3 | 0.6 | 0.54 | 0.16 |
| Example 1-209 | 95 | 40 | 20 | 1.3 | 0.6 | 0.54 | 0.16 |
| Example 1-210 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-211 | 95 | 4 | 20 | 1.3 | 0.6 | 0.54 | 0.16 |
| Example 1-212 | 95 | 4 | 20 | 1.3 | 0.6 | 0.54 | 0.16 |
| Example 1-213 | 95 | 40 | 20 | 1.3 | 0.6 | 0.54 | 0.16 |
| Example 1-214 | 95 | 4 | 20 | 1.3 | 0.6 | 0.54 | 0.16 |
| Example 1-215 | 95 | 4 | 20 | 1.3 | 0.6 | 0.54 | 0.16 |
| Example 1-216 | 95 | 4 | 20 | 1.3 | 0.6 | 0.54 | 0.16 |
| Example 1-217 | 95 | 4 | 20 | 1.3 | 0.6 | 0.54 | 0.16 |
| Example 1-218 | 95 | 4 | 20 | 1.3 | 0.6 | 0.54 | 0.16 |
| Example 1-219 | 95 | 4 | 20 | 1.3 | 0.6 | 0.54 | 0.16 |
| Example 1-220 | 95 | 4 | 20 | 1.3 | 0.6 | 0.54 | 0.16 |
| Example 1-221 | 95 | 4 | 20 | 1.3 | 0.6 | 0.54 | 0.16 |
| Example 1-222 | 95 | 190 | 20 | 1.3 | 0.6 | 0.54 | 0.16 |
| Example 1-223 | 95 | 40 | 20 | 1.3 | 0.6 | 0.54 | 0.16 |
| Example 1-224 | 95 | 4 | 20 | 1.3 | 0.6 | 0.54 | 0.16 |
| Example 1-225 | 95 | 4 | 20 | 1.3 | 0.6 | 0.54 | 0.16 |
| Example 1-226 | 95 | 4 | 20 | 1.3 | 0.6 | 0.54 | 0.16 |
| Example 1-227 | 95 | 4 | 20 | 1.3 | 0.6 | 0.54 | 0.16 |
| Example 1-228 | 95 | 4 | 20 | 1.3 | 0.6 | 0.54 | 0.16 |
| Example 1-229 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-230 | 95 | 4 | 20 | 1.3 | 0.6 | 0.54 | 0.16 |
| Example 1-231 | 95 | 4 | 20 | 1.3 | 0.6 | 0.54 | 0.16 |
| Example 1-232 | 95 | 40 | 20 | 1.3 | 0.6 | 0.54 | 0.16 |
| Example 1-233 | 95 | 4 | 20 | 1.3 | 0.6 | 0.54 | 0.16 |
| Example 1-234 | 95 | 4 | 20 | 1.3 | 0.6 | 0.54 | 0.16 |
| Example 1-235 | 95 | 4 | 20 | 1.3 | 0.6 | 0.54 | 0.16 |
| Example 1-236 | 95 | 40 | 20 | 1.3 | 0.6 | 0.54 | 0.16 |
| Example 1-237 | 95 | 4 | 20 | 1.3 | 0.6 | 0.54 | 0.16 |
| Example 1-238 | 95 | 4 | 20 | 1.3 | 0.6 | 0.54 | 0.16 |
| Example 1-239 | 95 | 4 | 20 | 1.3 | 0.6 | 0.54 | 0.16 |
| Example 1-240 | 95 | 4 | 20 | 1.3 | 0.6 | 0.54 | 0.16 |
| Example 1-241 | 95 | 4 | 20 | 1.3 | 0.6 | 0.54 | 0.16 |
| Example 1-242 | 95 | 4 | 20 | 1.3 | 0.6 | 0.54 | 0.16 |
| Example 1-243 | 95 | 4 | 20 | 1.3 | 0.6 | 0.54 | 0.16 |
| Example 1-244 | 95 | 4 | 20 | 1.3 | 0.6 | 0.54 | 0.16 |
| Example 1-245 | 95 | 190 | 20 | 1.3 | 0.6 | 0.54 | 0.16 |
| Example 1-246 | 95 | 40 | 20 | 1.3 | 0.6 | 0.54 | 0.16 |
| Example 1-247 | 95 | 4 | 20 | 1.3 | 0.6 | 0.54 | 0.16 |
| Example 1-248 | 95 | 4 | 20 | 1.3 | 0.6 | 0.54 | 0.16 |

(continued)

| | Step (6) | | | | | | |
|---|---|---|---|---|---|---|---|
| | Temperature | Pressure | Average retention time | Supply rate | Recovery speed of hydroxyl compound | Recovery speed of aprotic solvent such as carbonic acid ester | Extraction speed of high-boiling-point component |
| | °C | kPa | min | kg/hr | kg/hr | kg/hr | kg/hr |
| Example 1-249 | 95 | 4 | 20 | 1.3 | 0.6 | 0.54 | 0.16 |
| Example 1-250 | 95 | 4 | 20 | 1.3 | 0.6 | 0.54 | 0.16 |
| Example 1-251 | 95 | 4 | 20 | 1.3 | 0.6 | 0.54 | 0.16 |
| Example 1-252 | 95 | 4 | 20 | 1.3 | 0.6 | 0.54 | 0.16 |
| Example 1-253 | 95 | 4 | 20 | 1.3 | 0.6 | 0.54 | 0.16 |
| Example 1-254 | 95 | 4 | 20 | 1.3 | 0.6 | 0.54 | 0.16 |
| Example 1-255 | 95 | 40 | 20 | 1.3 | 0.6 | 0.54 | 0.16 |
| Example 1-256 | 95 | 4 | 20 | 1.3 | 0.6 | 0.54 | 0.16 |
| Example 1-257 | 95 | 4 | 20 | 1.3 | 0.6 | 0.54 | 0.16 |
| Example 1-258 | 95 | 4 | 20 | 1.3 | 0.6 | 0.54 | 0.16 |
| Example 1-259 | 95 | 4 | 20 | 1.3 | 0.6 | 0.54 | 0.16 |
| Example 1-260 | 95 | 4 | 20 | 1.3 | 0.6 | 0.54 | 0.16 |
| Example 1-261 | 95 | 190 | 20 | 1.3 | 0.6 | 0.54 | 0.16 |
| Example 1-262 | 95 | 4 | 20 | 1.3 | 0.6 | 0.54 | 0.16 |
| Example 1-263 | 95 | 4 | 20 | 1.3 | 0.6 | 0.54 | 0.16 |
| Example 1-264 | 95 | 190 | 20 | 1.3 | 0.6 | 0.54 | 0.16 |
| Example 1-265 | 95 | 4 | 20 | 1.3 | 0.6 | 0.54 | 0.16 |
| Example 1-266 | 95 | 4 | 20 | 1.3 | 0.6 | 0.54 | 0.16 |
| Example 1-267 | 95 | 190 | 20 | 1.3 | 0.6 | 0.54 | 0.16 |

[Table 1-21]

| | Carbonyl compound (I) observed to be produced after step (2) | |
|---|---|---|
| | Main skeleton component | Substituent R' |
| Example 1-1, 1-3 to 1-9, 1-12 to 1-15, Comparative Example 1-2 to 1-4 | Mixture of compounds represented by General Formula (I-1) | n-Butyl group |
| Example 1-1 0, 1-11, 1-16 | | Phenyl group |
| Example 1-29, 39 | Mixture of compounds represented by General Formulae (I-5a) to (I-5c) | n-Butyl group |
| Example 1-30 to 1-37, 1-40 to 1-51 | | Phenyl group |
| Example 1-38 | | Ethyl group |
| Example 1-52, 62 | Compound represented by General Formula (I-9) | n-Butyl group |
| Example 1-53 to 1-60, 1-63 to 1-74 | | Phenyl group |
| Example 1-61 | | Ethyl group |
| Example 1-75, 85 | Compound represented by General Formula (I-10) | n-Butyl group |
| Example 1-76 to 1-83, 1-86 to 97 | | Phenyl group |
| Example 1-84 | | Ethyl group |
| Example 1-98, 108 | Compound represented by General Formula (I-2) | n-Butyl group |
| Example 1-99 to 1-106, 1-109 to 1-120 | | Phenyl group |
| Example 1-107 | | Ethyl group |
| Example 1-121, 131 | Mixture of compounds represented by General Formulae (I-8a) and (I-8b) | n-Butyl group |
| Example 1-122 to 1-129, 1-132 to 143 | | Phenyl group |
| Example 1-130 | | Ethyl group |
| Example 1-144, 154 | Mixture of compounds represented by General Formulae (I-12a) to (I-12c) | n-Butyl group |
| Example 1-145 to 1-152, 1-155 to 166 | | Phenyl group |
| Example 1-153 | | Ethyl group |
| Example 1-167, 177 | Mixture of compounds represented by General Formulae (I-13a) and (I-13b) | n-Butyl group |
| Example 1-168 to 1-175, 178-189 | | Phenyl group |
| Example 1-176 | | Ethyl group |
| Example 1-190, 200 | Compound represented by General Formula (I-14) | n-Butyl group |
| Example 1-191 to 1-198, 1-201 to 1-212 | | Phenyl group |
| Example 1-199 | | Ethyl group |
| Example 1-213, 223 | Mixture of compounds represented by General Formulae (I-15a) and (I-15b) | n-Butyl group |
| Example 1-214 to 1-221, 1-224 to 1-235 | | Phenyl group |
| Example 1-222 | | Ethyl group |
| Example 1-236, 246 | Compound represented by General Formula (I-16) | n-Butyl group |
| Example 1-237 to 1-244, 1-247 to 1-258 | | Phenyl group |
| Example 1-245 | | Ethyl group |
| Example 1-259 to 1-260 | Mixture of compounds represented by General Formulae (I-8a) and (I-8b) | Phenyl group |
| Example 1-261 | | Ethyl group |

(continued)

| | Carbonyl compound (I) observed to be produced after step (2) | |
|---|---|---|
| | Main skeleton component | Substituent R' |
| Example 1-262 to 1-263 | Mixture of compounds represented by General Formulae (I-17a) and (I-17b) | Phenyl group |
| Example 1-264 | | Ethyl group |
| Example 1-265 to 1-266 | Mixture of compounds represented by General Formulae (I-7a) and (I-7b) | Phenyl group |
| Example 1-267 | | Ethyl group |

From the above, it was clarified that the isocyanate compound can be obtained at a high yield of 99.1 % by mass by the production method according to the present embodiment. In addition, in the step (6), the hydroxy compound and the aprotic solvent were separated from the components distilled off as a gas phase in each of the step (1a) (N-substituted carbamate compound concentration step), the step (2) (isocyanate compound production step and thermal decomposition step), and the step (3) (isocyanate compound concentration step), and furthermore, in the step (5), a primary amine compound and a hydroxy compound were obtained by hydrolyzing the high-boiling-point product, and the obtained primary amine compound, hydroxy compound, and aprotic solvent were re-used in each of the step (1) (N-substituted carbamate compound production step), the step (1a) (N-substituted carbamate compound concentration step), and the step (2) (isocyanate compound production step and thermal decomposition step). It was confirmed that the isocyanate compound could be produced while suppressing the raw material cost by re-using these raw material components.

<<Second Test>>

<Analysis method>

(1) $^{1}$H-NMR analysis method

$^{1}$H-NMR analysis was performed using a JNM-A400 FT-NMR system manufactured by JEOL Ltd. as an apparatus.

(1-1) Preparation of $^{1}$H-NMR analysis sample

0.3 g of the sample solution was weighed, and a solution obtained by adding 0.7 g of deuterated chloroform and 0.05 g of dimethyldiphenylsilane as an internal standard substance to the sample solution and uniformly mixing was used as an NMR analysis sample.

(1-2) Quantitative analysis method

Each standard substance was analyzed, and a quantitative analysis of the analysis sample solution was performed based on a created calibration curve.

(2) Gas chromatography analysis method

The analysis was performed under the following conditions.

(Measurement conditions)

Device: GC-2010 manufactured by Shimadzu Corporation
Column: DB-1
Diameter: 0.25 mm, length: 30 m, film thickness: 1.0 μm
Column temperature: 60°C to 300°C
Injection port temperature: 300°C

Carrier gas: helium
Carrier gas flow rate: 40 mL/min
Detector: flame ionization detector (FID)

(2-1) Preparation of gas chromatography analysis sample

1.0 g of the sample solution was weighed, and a solution obtained by adding 10 g of acetonitrile and 0.1 g of anisole as an internal standard substance to the sample solution and uniformly mixing was used as a gas chromatography analysis sample.

(3) Liquid chromatography analysis method

The analysis was performed under the following conditions.

(Measurement conditions)

Device: LC-10AT manufactured by Shimadzu Corporation
Column: Inertsil ODS
Particle diameter: 5 μm, inner diameter: 2.1 mm, length: 250 mm
Column temperature: 40°C
Developing solvent: water/acetonitrile = 90/10
Solvent flow rate: 1 mL/min
Detector: photodiode array detector

(3-1) Preparation of liquid chromatography analysis sample

1.0 g of the sample solution was weighed, and a solution obtained by adding 10 g of acetic acid to the sample solution and uniformly mixing was used as a liquid chromatography analysis sample.

(3-2) Quantitative analysis method

Each standard substance was analyzed, and a quantitative analysis of the analysis sample solution was performed based on a created calibration curve.

[Example 2-1]

13.2 g of urea, 500 g of cresol, and 0.3 g of 7-methyl-1,5,7-triazabicyclo[4.4.0]deca-5-ene were put into a 2 L four-necked flask to which a condenser was attached, and the temperature was raised to 135°C while stirring. Nitrogen gas was blown from a pipe kept at 180°C, and the flow rate was controlled to 300 cc/min by a mass flow meter under atmospheric pressure (101.325 kPa; the same is applied hereinafter). The blown nitrogen gas was extracted from the upper part of the condenser, passed through a sulfuric acid aqueous solution, and released to the outside of the system. Next, a solution obtained by dissolving 11.6 g of hexamethylenediamine in 60 g of cresol was added thereto using a dropping funnel, and the mixture was stirred at 180°C for 1 hour under atmospheric pressure. In a case where a part of the reaction solution was extracted and analyzed by liquid chromatography, N,N'-hexamethylene-di(carbamate-cresoyl ester) was produced from hexamethylenediamine with a yield of 72.6% by mass.

[Examples 2-2 to 2-40 and Comparative Examples 2-1 to 2-8]

A corresponding carbamate was obtained by the same method, except that the type and the use amount of the primary amine compound, urea, hydroxy compound, and catalyst used in Example 2-1 were changed to those described in the following tables. The yield of the obtained carbamate with respect to the primary amine compound was as shown in the following tables. The pKa value in each table is a predicted value recorded in CAS SciFinder$^{n}$.

In addition, in the tables, the abbreviations indicate the following compounds. The same applies to the subsequent tables.

(Primary amine compound)

HDA: hexamethylenediamine
PDA: pentamethylenediamine
TTA: 4-(aminomethyl)octane-1,8-diamine
IPDA: Isophorondiamine
HMDA: 4,4'-methylenebis(cyclohexanamine)
TDA: diaminotoluene

[Table 2-1]

| | Primary amine compound | | Urea | Hydroxy compound | | Catalyst | | | Reaction time | Yield |
|---|---|---|---|---|---|---|---|---|---|---|
| | Type | Blending amount (g) | Blending amount (g) | Type | Blending amount (g) | Type | pKa | Blending amount (g) | Hour | % by mass |
| Example 2-1 | HDA | 11.6 | 13.2 | Cresol | 560 | 7-Methyl-1,5,7-triazabicyclo [4.4.0]deca-5-ene | 14.4 | 0.3 | 3 | 72.6 |
| Example 2-2 | HDA | 11.6 | 13.2 | Cresol | 560 | Pentamethyl guanidine | 13.8 | 0.26 | 3 | 71.4 |
| Example 2-3 | HDA | 11.6 | 13.2 | Cresol | 560 | Pyrazine | 1.2 | 16 | 3 | 29.5 |
| Example 2-4 | HDA | 11.6 | 13.2 | Cresol | 560 | Pyrimidine | 1.8 | 17 | 3 | 30.9 |
| Example 2-5 | HDA | 11.6 | 13.2 | Cresol | 560 | Tetramethyl-p-phenylene diamine | 5.9 | 10 | 3 | 41.2 |
| Example 2-6 | HDA | 11.6 | 13.2 | Cresol | 560 | D iazabicyclo [2.2.2] octane | 8.2 | 7 | 3 | 42.5 |
| Example 2-7 | HDA | 11.6 | 13.2 | Cresol | 560 | 1,5-Diazabicyclo-[4.3.0]nona-5-ene | 13.4 | 0.26 | 3 | 69.9 |
| Example 2-8 | HDA | 11.6 | 13.2 | Cresol | 560 | Pyridine | 5.23 | 16 | 3 | 56.6 |
| Example 2-9 | HDA | 11.6 | 13.2 | Cresol | 560 | Ethyldiisopropylamine | 11 | 7.5 | 3 | 48.7 |
| Example 2-10 | HDA | 11.6 | 13.2 | Cresol | 560 | Methylenebis(N,N'-dimethylaniline) | 5.6 | 30 | 3 | 41.2 |
| Comparative Example 2-1 | HDA | 11.6 | 13.2 | Cresol | 560 | - | - | - | 3 | 25.1 |
| Comparative Example 2-2 | HDA | 11.6 | 13.2 | Cresol | 560 | Dibutylamine | 10.7 | 7.8 | 3 | 20.3 |
| Example 2-11 | PDA | 11.6 | 15 | Phenol | 560 | Pyridine | 5.23 | 18 | 3 | 57.8 |
| Example 2-12 | PDA | 11.6 | 15 | Phenol | 560 | 1,5-Diazabicyclo-[4.3.0]nona-5-ene | 13.4 | 0.27 | 3 | 71.1 |
| Comparative Example 2-3 | PDA | 11.6 | 15 | Phenol | 560 | - | - | - | 3 | 27 |
| Example 2-13 | TTA | 4.5 | 6.9 | Phenol | 560 | Quinuclidine | 10.9 | 3.5 | 5 | 33.4 |

(continued)

| | Primary amine compound | | Urea | Hydroxy compound | | Catalyst | | | Reaction time | Yield |
|---|---|---|---|---|---|---|---|---|---|---|
| | Type | Blending amount (g) | Blending amount (g) | Type | Blending amount (g) | Type | pKa | Blending amount (g) | Hour | % by mass |
| Example 2-14 | TTA | 4.5 | 6.9 | Phenol | 560 | 1 -Methylpyrrolidine | 10.6 | 2.7 | 5 | 30.6 |
| Example 2-15 | TTA | 4.5 | 6.9 | Phenol | 560 | Bis(morpholine ethyl)ether | 6.92 | 7.5 | 5 | 42.5 |
| Example 2-16 | TTA | 4.5 | 6.9 | Phenol | 560 | 1-Phenylpyrrolidine | 5.7 | 4.6 | 5 | 43.7 |
| Example 2-17 | TTA | 4.5 | 6.9 | Phenol | 560 | 1-Methylimidazole | 7 | 8.6 | 5 | 34.4 |
| Example 2-18 | TTA | 4.5 | 6.9 | Phenol | 560 | 2-Picorine | 6 | 13 | 5 | 51 |
| Example 2-19 | TTA | 4.5 | 6.9 | Phenol | 560 | 4-Methylquinoline | 5.7 | 15 | 5 | 32.6 |
| Example 2-20 | TTA | 4.5 | 6.9 | Phenol | 560 | Dimethylaniline | 5.1 | 3.8 | 5 | 33.9 |
| Example 2-21 | TTA | 4.5 | 6.9 | Phenol | 560 | 1,5-Diazabicyclo-[4.3.0]nona-5-ene | 13.4 | 0.13 | 5 | 73.4 |
| Comparative Example 2-4 | TTA | 4.5 | 6.9 | Phenol | 560 | - | - | - | 5 | 21.3 |

[Table 2-2]

| | Primary amine compound | | Urea | Hydroxy compound | | Catalyst | | | Reaction time | Yield |
|---|---|---|---|---|---|---|---|---|---|---|
| | Type | Blending amount (g) | Blending amount (g) | Type | Blending amount (g) | Type | pKa | Blending amount (g) | Hour | % by mass |
| Example 2-22 | IPDA | 11.3 | 8.7 | Xylenol | 560 | 1-Methylpyrazole | 2.3 | 11 | 3 | 42.1 |
| Example 2-23 | IPDA | 11.3 | 8.7 | Xylenol | 560 | 1-Methylpiperidine | 9.6 | 4 | 3 | 48.6 |
| Example 2-24 | IPDA | 11.3 | 8.7 | Xylenol | 560 | N,N-Dimethylaminopyridine | 9.5 | 4.8 | 3 | 56.7 |
| Example 2-25 | IPDA | 11.3 | 8.7 | Xylenol | 560 | 4-Methylmorpholine | 7.6 | 4 | 3 | 49.3 |
| Example 2-26 | IPDA | 11.3 | 8.7 | Xylenol | 560 | 1,8-Diazabicyclo-[5.4.0] undeca-7-ene | 13.3 | 0.2 | 3 | 72.4 |
| Example 2-27 | IPDA | 11.3 | 8.7 | Xylenol | 560 | Hexahydro-1,3,5-tris(3-dimethylaminopropyl)-1,3,5-triazine | 10 | 13 | 3 | 49.8 |
| Example 2-28 | IPDA | 11.3 | 8.7 | Xylenol | 560 | Isoquinolin | 5.4 | 17 | 3 | 35.9 |
| Comparative Example 2-5 | IPDA | 11.3 | 8.7 | Xylenol | 560 | - | - | - | 3 | 24 |
| Example 2-29 | HMDA | 10 | 6.5 | 4-Ethylphenol | 560 | Dimethylpiperazine | 8 | 3.3 | 3 | 42.4 |
| Example 2-30 | HMDA | 10 | 6.5 | 4-Ethylphenol | 560 | Tributylamine | 10 | 5.3 | 3 | 55.8 |
| Example 2-31 | HMDA | 10 | 6.5 | 4-Ethylphenol | 560 | 2,6-Lutidine | 6.6 | 10.2 | 3 | 63.7 |
| Example 2-32 | HMDA | 10 | 6.5 | 4-Ethylphenol | 560 | 1-Ethylpyrrolidine | 10.6 | 2.8 | 3 | 56.6 |
| Example 2-33 | HMDA | 10 | 6.5 | 4-Ethylphenol | 560 | Pyridazine | 3.1 | 7.6 | 3 | 41.5 |

(continued)

| | Primary amine compound | | Urea | Hydroxy compound | | Catalyst | | | Reaction time | Yield |
|---|---|---|---|---|---|---|---|---|---|---|
| | Type | Blending amount (g) | Blending amount (g) | Type | Blending amount (g) | Type | pKa | Blending amount (g) | Hour | % by mass |
| Example 2-34 | HMDA | 10 | 6.5 | 4-Ethylphenol | 560 | N,N,N',N'-tetramethylethylenediamine | 8.9 | 3.3 | 3 | 56.4 |
| Example 2-35 | HMDA | 10 | 6.5 | 4-Ethylphenol | 560 | 1,8-Diazabicyclo-[5.4.0] undeca-7-ene | 13.3 | 0.15 | 3 | 71.9 |
| Comparative Example 2-6 | HMDA | 10 | 6.5 | 4-Ethylphenol | 560 | - | - | - | 3 | 22.9 |

[Table 2-3]

| | Primary amine compound | | Urea | Hydroxy compound | | Catalyst | | | Reaction time | Yield |
|---|---|---|---|---|---|---|---|---|---|---|
| | Type | Blending amount (g) | Blending amount (g) | Type | Blending amount (g) | Type | pKa | Blending amount (g) | Hour | % by mass |
| Example 2-36 | TDA | 6.3 | 6.9 | 1-Octanol | 560 | Quinoline | 5 | 13.3 | 3 | 35.5 |
| Example 2-37 | TDA | 6.3 | 6.9 | 1-Octanol | 560 | Hexamethylenetetramine | 5.3 | 4.4 | 3 | 56.1 |
| Example 2-38 | TDA | 6.3 | 6.9 | 1-Octanol | 560 | Diethylaniline | 6.7 | 4.6 | 3 | 42.6 |
| Example 2-39 | TDA | 6.3 | 6.9 | 1-Octanol | 560 | 7-Methyl-1,5,7-triazabicyclo [4.4.0]deca-5-ene | 14.4 | 0.16 | 3 | 71.7 |
| Example 2-40 | TDA | 6.3 | 6.9 | 1-Octanol | 560 | 1,8-Diazabicyclo-[5.4.0] undeca-7-ene | 13.3 | 0.15 | 3 | 71.9 |
| Comparative Example 2-7 | TDA | 6.3 | 6.9 | 1-Octanol | 560 | - | - | - | 3 | 20.3 |
| Comparative Example 2-8 | TDA | 6.3 | 6.9 | 1-Octanol | 560 | N-Methylaniline | 4.7 | 3.3 | 3 | 12.2 |

[Example 2-41]

A carbamylation step was performed using a device shown in FIG. 19. A mixture of 6.3 kg of hexamethylenediamine, 7.2 kg of urea, 180 kg of cresol, and 0.13 kg of 1,5-diazabicyclo-[4.3.0]nona-5-ene was supplied to a distillation column 101 (theoretical number of stages: 39) packed with Heli-Pak No. 4 from a line 11 at approximately 3.0 kg/hour. From the line 14, the mixed solution of cresol and urea obtained in a condenser 103 was supplied at approximately 3.5 kg/hour. The surplus condensate component was recovered in a storage tank 104. A distillation column 101 was a device for performing the carbamate synthesis step, and the column bottom temperature was set to 240°C and the column top pressure was set to 0.15 MPa by heating with a reboiler 102. For concentration adjustment, the cresol was supplied from a line 15 at the column bottom. The reaction solution was extracted from the bottom of the column and recovered in a storage tank 105 through a line 12. In a case where the recovered solution from the storage tank 105 was analyzed by liquid chromatography, N,N'-hexamethylene-di(carbamate-cresoyl ester) was produced from hexamethylenediamine with a yield of 90.4% by mass.

[Examples 2-42 to 2-122 and Comparative Examples 2-9 to 2-21]

A corresponding carbamate was obtained by the same method, except that the type and the use amount of the primary amine compound, urea, hydroxy compound, and catalyst used in Example 2-41 were changed to those described in the following tables, and the column bottom temperature was changed to a value shown in the following tables. The yield of the obtained carbamate with respect to the primary amine compound was as shown in the following tables.

[Table 2-4]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-41 | HDA | 6.3 | 7.2 | Cresol | 180 | 1,5-Diazabicyclo-[4.3.0]nona-5-ene | 0.13 | 240 | 0.15 | 90.4 |
| Example 2-42 | HDA | 6.3 | 7.2 | Cresol | 180 | 1,5-Diazabicyclo-[4.3.0]nona-5-ene | 0.65 | 240 | 0.15 | 86.9 |
| Example 2-43 | HDA | 6.3 | 7.2 | Cresol | 180 | 1,5-Diazabicyclo-[4.3.0]nona-5-ene | 1.3 | 240 | 0.15 | 81.4 |
| Example 2-44 | HDA | 6.3 | 7.2 | Cresol | 180 | 7-Methyl-1,5,7-triazabicyclo [4.4.0]deca-5-ene | 0.15 | 240 | 0.15 | 91.3 |
| Example 2-45 | HDA | 6.3 | 7.2 | Cresol | 180 | Pyridine | 0.09 | 240 | 0.15 | 36.2 |
| Example 2-46 | HDA | 6.3 | 7.2 | Cresol | 180 | Pyridine | 0.9 | 240 | 0.15 | 38.6 |
| Example 2-47 | HDA | 6.3 | 7.2 | Cresol | 180 | Pyridine | 4.5 | 240 | 0.15 | 42.3 |
| Example 2-48 | HDA | 6.3 | 7.2 | Cresol | 180 | Pyridine | 9 | 240 | 0.15 | 56.7 |
| Example 2-49 | HDA | 6.3 | 7.2 | Cresol | 180 | Pyridine | 27 | 240 | 0.15 | 86.9 |
| Example 2-50 | HDA | 6.3 | 7.2 | Cresol | 180 | Pyridine | 45 | 240 | 0.15 | 88 |
| Example 2-51 | HDA | 6.3 | 7.2 | Cresol | 180 | Diisopropylethylamine | 0.14 | 240 | 0.15 | 36.6 |
| Example 2-52 | HDA | 6.3 | 7.2 | Cresol | 180 | Diisopropylethylamine | 1.4 | 240 | 0.15 | 42.6 |
| Example 2-53 | HDA | 6.3 | 7.2 | Cresol | 180 | Diisopropylethylamine | 7 | 240 | 0.15 | 49.3 |
| Example 2-54 | HDA | 6.3 | 7.2 | Cresol | 180 | Diisopropylethylamine | 14 | 240 | 0.15 | 64.8 |
| Example 2-55 | HDA | 6.3 | 7.2 | Cresol | 180 | Diisopropylethylamine | 42 | 240 | 0.15 | 72.8 |
| Comparative Example 2-9 | HDA | 6.3 | 7.2 | Cresol | 180 | - | - | 240 | 0.15 | 36 |
| Comparative Example 2-10 | HDA | 6.3 | 7.2 | Cresol | 180 | Dibutylamine | 4.2 | 240 | 0.15 | 25.9 |
| Example 2-56 | PDA | 5.6 | 7.3 | Phenol | 180 | Pyridine | 8.7 | 240 | 0.3 | 66.4 |

(continued)

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-57 | PDA | 5.6 | 7.3 | Phenol | 180 | Pyridine | 26.1 | 240 | 0.3 | 75.7 |
| Example 2-58 | PDA | 5.6 | 7.3 | Phenol | 180 | 1,5-Diazabicyclo-[4.3.0]nona-5-ene | 0.14 | 240 | 0.3 | 92.2 |
| Comparative Example 2-11 | PDA | 5.6 | 7.3 | Phenol | 180 | 1,5-Diazabicyclo-[4.3.0]nona-5-ene | - | 240 | 0.3 | 38.3 |

[Table 2-5]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-59 | TTA | 4.5 | 5.2 | Phenol | 160 | 2-Picorine | 0.076 | 240 | 0.3 | 22.1 |
| Example 2-60 | TTA | 4.5 | 5.2 | Phenol | 160 | 2-Picorine | 0.76 | 240 | 0.3 | 23.8 |
| Example 2-61 | TTA | 4.5 | 5.2 | Phenol | 160 | 2-Picorine | 3.8 | 240 | 0.3 | 28.1 |
| Example 2-62 | TTA | 4.5 | 5.2 | Phenol | 160 | 2-Picorine | 7.6 | 240 | 0.3 | 51.2 |
| Example 2-63 | TTA | 4.5 | 5.2 | Phenol | 160 | 2-Picorine | 22.8 | 240 | 0.3 | 81.5 |
| Example 2-64 | TTA | 4.5 | 5.2 | Phenol | 160 | N,N-Dimethylaniline | 0.09 | 240 | 0.3 | 22 |
| Example 2-65 | TTA | 4.5 | 5.2 | Phenol | 160 | N,N-Dimethylaniline | 0.9 | 240 | 0.3 | 25.1 |
| Example 2-66 | TTA | 4.5 | 5.2 | Phenol | 160 | N,N-Dimethylaniline | 4.5 | 240 | 0.3 | 27.1 |
| Example 2-67 | TTA | 4.5 | 5.2 | Phenol | 160 | N,N-Dimethylaniline | 9 | 240 | 0.3 | 37.3 |
| Example 2-68 | TTA | 4.5 | 5.2 | Phenol | 160 | N,N-Dimethylaniline | 27 | 240 | 0.3 | 42.5 |
| Example 2-69 | TTA | 4.5 | 5.2 | Phenol | 160 | 1,5-Diazabicyclo-[4.3.0] nona-5-ene | 0.1 | 240 | 0.3 | 86.3 |
| Example 2-70 | TTA | 4.5 | 5.2 | Phenol | 160 | 1,5-Diazabicyclo-[4.3.0] nona-5-ene | 0.5 | 240 | 0.3 | 80.7 |
| Example 2-71 | TTA | 4.5 | 5.2 | Phenol | 160 | 1,5-Diazabicyclo-[4.3.0] nona-5-ene | 1 | 240 | 0.3 | 73.4 |
| Comparative Example 2-12 | TTA | 4.5 | 5.2 | Phenol | 160 | - | - | 240 | 0.3 | 21.7 |

[Table 2-6]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-72 | IPDA | 11.3 | 8.7 | Xylenol | 280 | N,N-Dimethylaminopyridine | 0.16 | 240 | 0.05 | 37 |
| Example 2-73 | IPDA | 11.3 | 8.7 | Xylenol | 280 | N,N-Dimethylaminopyridine | 1.6 | 240 | 0.05 | 42.1 |
| Example 2-74 | IPDA | 11.3 | 8.7 | Xylenol | 280 | N,N-Dimethylaminopyridine | 8 | 240 | 0.05 | 49 |
| Example 2-75 | IPDA | 11.3 | 8.7 | Xylenol | 280 | N,N-Dimethylaminopyridine | 16 | 240 | 0.05 | 63.8 |
| Example 2-76 | IPDA | 11.3 | 8.7 | Xylenol | 280 | N,N-Dimethylaminopyridine | 48 | 240 | 0.05 | 71.7 |
| Example 2-77 | IPDA | 11.3 | 8.7 | Xylenol | 280 | 4-Methylmorpholine | 0.13 | 240 | 0.05 | 36.6 |
| Example 2-78 | IPDA | 11.3 | 8.7 | Xylenol | 280 | 4-Methylmorpholine | 1.3 | 240 | 0.05 | 39.8 |
| Example 2-79 | IPDA | 11.3 | 8.7 | Xylenol | 280 | 4-Methylmorpholine | 6.5 | 240 | 0.05 | 42.5 |
| Example 2-80 | IPDA | 11.3 | 8.7 | Xylenol | 280 | 4-Methylmorpholine | 13 | 240 | 0.05 | 56 |
| Example 2-81 | IPDA | 11.3 | 8.7 | Xylenol | 280 | 4-Methylmorpholine | 39 | 240 | 0.05 | 53.7 |
| Example 2-82 | IPDA | 11.3 | 8.7 | Xylenol | 280 | 1 ,8-Diazabicyclo-[5.4.0] undeca-7-ene | 0.2 | 240 | 0.05 | 89.9 |
| Example 2-83 | IPDA | 11.3 | 8.7 | Xylenol | 280 | 1,8-Diazabicyclo-[5.4.0] undeca-7-ene | 1 | 240 | 0.05 | 86.5 |
| Example 2-84 | IPDA | 11.3 | 8.7 | Xylenol | 280 | 1,8-Diazabicyclo-[5.4.0] undeca-7-ene | 2 | 240 | 0.05 | 80.8 |
| Comparative Example 2-13 | IPDA | 11.3 | 8.7 | Xylenol | 280 | - | - | 240 | 0.05 | 35.5 |

[Table 2-7]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-85 | HMDA | 5 | 3.25 | 4-Ethylphenol | 100 | 2,6-Lutidine | 0.05 | 240 | 0.05 | 36.1 |
| Example 2-86 | HMDA | 5 | 3.25 | 4-Ethylphenol | 100 | 2,6-Lutidine | 0.5 | 240 | 0.05 | 38.9 |
| Example 2-87 | HMDA | 5 | 3.25 | 4-Ethylphenol | 100 | 2,6-Lutidine | 2.5 | 240 | 0.05 | 42.8 |
| Example 2-88 | HMDA | 5 | 3.25 | 4-Ethylphenol | 100 | 2,6-Lutidine | 5 | 240 | 0.05 | 64 |
| Example 2-89 | HMDA | 5 | 3.25 | 4-Ethylphenol | 100 | 2,6-Lutidine | 15 | 240 | 0.05 | 72.6 |
| Example 2-90 | HMDA | 5 | 3.25 | 4-Ethylphenol | 100 | 1,8-Diazabicyclo-[5.4.0] undeca-7-ene | 0.075 | 240 | 0.05 | 89.7 |
| Example 2-91 | HMDA | 5 | 3.25 | 4-Ethylphenol | 100 | 1,8-Diazabicyclo-[5.4.0] undeca-7-ene | 0.375 | 240 | 0.05 | 86.3 |
| Example 2-92 | HMDA | 5 | 3.25 | 4-Ethylphenol | 100 | 1,8-Diazabicyclo-[5.4.0] undeca-7-ene | 0.75 | 240 | 0.05 | 80.5 |
| Comparative Example 2-14 | HMDA | 5 | 3.25 | 4-Ethylphenol | 100 | - | - | 240 | 0.05 | 34.8 |

[Table 2-8]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-93 | TDA | 6.3 | 6.9 | 1-Octanol | 560 | Diethylaniline | 0.15 | 240 | 0.15 | 42 |
| Example 2-94 | TDA | 6.3 | 6.9 | 1-Octanol | 560 | Diethylaniline | 1.5 | 240 | 0.15 | 49.6 |
| Example 2-95 | TDA | 6.3 | 6.9 | 1-Octanol | 560 | Diethylaniline | 7.5 | 240 | 0.15 | 58.5 |
| Example 2-96 | TDA | 6.3 | 6.9 | 1-Octanol | 560 | Diethylaniline | 15 | 240 | 0.15 | 64.3 |
| Example 2-97 | TDA | 6.3 | 6.9 | 1-Octanol | 560 | Diethylaniline | 45 | 240 | 0.15 | 73.6 |
| Example 2-98 | TDA | 6.3 | 6.9 | 1-Octanol | 560 | 1,8-Diazabicyclo-[5.4.0] undeca-7-ene | 0.15 | 240 | 0.15 | 88.9 |
| Example 2-99 | TDA | 6.3 | 6.9 | 1-Octanol | 560 | 1,8-Diazabicyclo-[5.4.0] undeca-7-ene | 0.75 | 240 | 0.15 | 85.2 |
| Example 2-100 | TDA | 6.3 | 6.9 | 1-Octanol | 560 | 1,8-Diazabicyclo-[5.4.0] undeca-7-ene | 1.5 | 240 | 0.15 | 80.1 |
| Example 2-101 | TDA | 6.3 | 6.9 | 1-Octanol | 560 | 7-methyl-1,5,7-triazabicyclo [4.4.0]deca-5-ene | 0.16 | 240 | 0.15 | 89.7 |
| Comparative Example 2-15 | TDA | 6.3 | 6.9 | 1-Octanol | 560 | - | - | 240 | 0.15 | 39.4 |
| Comparative Example 2-16 | TDA | 6.3 | 6.9 | 1-Octanol | 560 | N-Methylaniline | 3.3 | 240 | 0.15 | 17.6 |

[Table 2-9]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-102 | HDA | 6.3 | 7.2 | Cresol | 180 | 1,5-Diazabicyclo-[4.3.0] nona-5-ene | 0.13 | 230 | 0.1 | 72.8 |
| Example 2-103 | HDA | 6.3 | 7.2 | Cresol | 180 | Pyridine | 9 | 230 | 0.1 | 56.4 |
| Example 2-104 | HDA | 6.3 | 7.2 | Cresol | 180 | Pyridine | 27 | 230 | 0.1 | 72.6 |
| Example 2-105 | HDA | 6.3 | 7.2 | Cresol | 180 | Diisopropylethylamine | 14 | 230 | 0.1 | 49.6 |
| Example 2-106 | HDA | 6.3 | 7.2 | Cresol | 180 | Diisopropylethylamine | 42 | 230 | 0.1 | 64.8 |
| Comparative Example 2-17 | HDA | 6.3 | 7.2 | Cresol | 180 | - | - | 230 | 0.1 | 20.3 |
| Example 2-107 | TTA | 4.5 | 6.9 | Phenol | 160 | 2-Picorine | 7.6 | 230 | 0.2 | 42.7 |
| Example 2-108 | TTA | 4.5 | 6.9 | Phenol | 160 | 2-Picorine | 22.8 | 230 | 0.2 | 61.4 |
| Example 2-109 | TTA | 4.5 | 6.9 | Phenol | 160 | N,N-Dimethylaniline | 9 | 230 | 0.2 | 34.2 |
| Example 2-110 | TTA | 4.5 | 6.9 | Phenol | 160 | N,N-Dimethylaniline | 27 | 230 | 0.2 | 51.4 |
| Example 2-111 | TTA | 4.5 | 6.9 | Phenol | 160 | 1,5-Diazabicyclo-[4.3.0] nona-5-ene | 0.1 | 230 | 0.2 | 62.3 |
| Comparative Example 2-18 | TTA | 4.5 | 6.9 | Phenol | 160 | - | - | 230 | 0.2 | 9.7 |
| Example 2-112 | IPDA | 11.3 | 8.7 | Xylenol | 280 | N,N-Dimethylaminopyridine | 16 | 230 | 0.03 | 55.8 |

(continued)

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-113 | IPDA | 11.3 | 8.7 | Xylenol | 280 | N,N-Dimethylaminopyridine | 48 | 230 | 0.03 | 71.9 |
| Example 2-114 | IPDA | 11.3 | 8.7 | Xylenol | 280 | 4-Methylmorpholine | 13 | 230 | 0.03 | 48.9 |
| Example 2-115 | IPDA | 11.3 | 8.7 | Xylenol | 280 | 4-Methylmorpholine | 39 | 230 | 0.03 | 63.7 |
| Example 2-116 | IPDA | 11.3 | 8.7 | Xylenol | 280 | 1,8-Diazabicyclo-[5.4.0] undeca-7-ene | 0.2 | 230 | 0.03 | 72.1 |
| Comparative Example 2-19 | IPDA | 11.3 | 8.7 | Xylenol | 280 | - | - | 230 | 0.03 | 18.5 |

[Table 2-10]

| | Primary amine compound | | Urea | Hydroxy compound | | Catalyst | | Column bottom temperature | Reaction time | Yield |
|---|---|---|---|---|---|---|---|---|---|---|
| | Type | Blending amount (g) | Blending amount (g) | Type | Blending amount (g) | Type | pKa | °C | Hour | % by mass |
| Example 2-117 | HMDA | 5 | 3.25 | 4-Ethylphenol | 100 | 2,6-Lutidine | 5 | 230 | 0.03 | 56.7 |
| Example 2-118 | HMDA | 5 | 3.25 | 4-Ethylphenol | 100 | 2,6-Lutidine | 15 | 230 | 0.03 | 71.4 |
| Example 2-119 | HMDA | 5 | 3.25 | 4-Ethylphenol | 100 | 1,8-Diazabicyclo-[5.4.0] undeca-7-ene | 0.075 | 230 | 0.03 | 70.9 |
| Comparative Example 2-20 | HMDA | 5 | 3.25 | 4-Ethylphenol | 100 | - | - | 230 | 0.03 | 18.5 |
| Example 2-120 | TDA | 6.3 | 6.9 | 1-Octanol | 560 | Diethylaniline | 15 | 230 | 0.1 | 11.6 |
| Example 2-121 | TDA | 6.3 | 6.9 | 1-Octanol | 560 | Diethylaniline | 45 | 230 | 0.1 | 73.1 |
| Example 2-122 | TDA | 6.3 | 6.9 | 1-Octanol | 560 | 1,8-Diazabicyclo-[5.4.0] undeca-7-ene | 0.15 | 230 | 0.1 | 78.5 |
| Comparative Example 2-21 | TDA | 6.3 | 6.9 | 1-Octanol | 560 | - | - | 230 | 0.1 | 25.2 |

[Example 2-123]

1. Carbamate synthesis step

13.2 g of urea, 500 g of phenol, and 30.0 g of 2,4-lutidine were put into a 2 L four-necked flask to which a condenser was attached, and the temperature was raised to 135°C while stirring. Nitrogen gas was blown from a pipe kept at 180°C, and the flow rate was controlled to 300 cc/min by a mass flow meter under atmospheric pressure. The blown nitrogen gas was extracted from the upper part of the condenser, passed through a sulfuric acid aqueous solution, and released to the outside of the system. Next, a solution in which 17.0 g of isophorone diamine was dissolved in 60 g of phenol was added thereto using a dropping funnel, and the mixture was stirred at 180°C for 12 hours under atmospheric pressure. In a case where a part of the reaction solution was extracted and analyzed by liquid chromatography, ((3,3,5-trimethyl-5-((phenoxycarbonyl)amino)cyclohexyl)methylcarbamate phenyl)acetic acid was produced with a yield of 85.6% by mass with respect to isophoronediamine.

2. Carbamate concentration step

The solution obtained above was depressurized at 60°C to remove a large part of the phenol and 2,4-lutidine, and 100 g of the obtained solid was dissolved in 50 g of benzyl toluene at 80°C.

3. Thermal decomposition step

350 g of benzyltoluene was put into a 1 L four-necked flask to which a condenser was attached, and heated to 250°C with stirring to set the internal pressure to 22 kPa. The total amount of the benzyltoluene solution of the carbamate obtained in the carbamate concentration step was added thereto using a dropping funnel, and thermal decomposition was carried out. The reaction was carried out for 3 hours while recovering the low-boiling compound generated by the thermal decomposition from the upper part of the condenser. Thereafter, the inside was cooled to room temperature at atmospheric pressure, and a part of the remaining reaction solution in the four-necked flask was extracted and analyzed by gas chromatography. As a result, isophorone diisocyanate was produced with a yield of 90.3% by mass with respect to ((3,3,5-trimethyl-5-((phenoxycarbonyl)amino))cyclohexyl)methylcarbamic acid phenyl. The yield with respect to the isophoronediamine charged in the carbamate synthesis step was 77.3% by mass.

[Comparative Example 2-22]

1. Carbamate synthesis step

A corresponding dicarbamate was obtained by the same method as in Example 2-123, except that 2,4-lutidine was not used. The yield of the obtained dicarbamate with respect to isophoronediamine was 56.3% by mass.

2. Carbamate concentration step

The solution obtained above was depressurized at 60°C to remove a large part of the phenol, and 100 g of the obtained solid was dissolved in 50 g of benzyl toluene at 80°C.

3. Thermal decomposition step

The thermal decomposition was carried out by the same method as in Example 2-123 to obtain isophorone diisocyanate. The yield of the obtained diisocyanate with respect to dicarbamate was 72.3% by mass. The yield with respect to the isophoronediamine charged in the carbamate synthesis step was 40.6% by mass.

[Comparative Example 2-23]

1. Carbamate synthesis step

A corresponding dicarbamate was obtained by the same method as in Example 2-123, except that 12.0 g of sodium hydroxide was used instead of 2,4-lutidine. The yield of the obtained dicarbamate with respect to isophoronediamine was 79.2% by mass.

2. Carbamate concentration step

The solution obtained above was depressurized at 60°C to remove a large part of the phenol, and 100 g of the obtained solid was dissolved in 50 g of benzyl toluene at 80°C.

3. Thermal decomposition step

The thermal decomposition was carried out by the same method as in Example 2-123 to obtain isophorone diisocyanate. The yield of the obtained diisocyanate with respect to dicarbamate was 49.0% by mass. The yield with respect to the isophoronediamine charged in the carbamate synthesis step was 38.8% by mass. In addition, in a case where the reaction solution was analyzed by NMR, an isocyanurate ring was generated from the raw material amino group in a yield of 10.3% by mass, and a gel-like deposit was precipitated on the surface of the flask.

[Example 2-124]

1. Carbamate synthesis step

A corresponding tricarbamate was obtained by the same method as in Example 2-123, except that 21.0 g of urea was used, 43.5 g of 4-picoline was used instead of 2,4-lutidine, and 18.0 g of 4-(aminomethyl)octane-1,8-diamine was used instead of isophoronediamine, and the reaction time was 18 hours. The yield of the obtained tricarbamate with respect to 4-(aminomethyl)octane-1,8-diamine was 88.5% by mass.

2. Carbamate concentration step

The solution obtained above was depressurized at 60°C to remove a large part of the phenol and 4-picoline, and 100 g of the obtained solid was dissolved in 50 g of benzyl toluene at 80°C.

3. Thermal decomposition step

The thermal decomposition was carried out by the same method as in Example 2-123 to obtain 4-(Isocyanatomethyl)octane-1,8-diisocyanate. The yield of the obtained triisocyanate with respect to tricarbamate was 85.7% by mass. The yield with respect to the 4-(aminomethyl)octane-1,8-diamine charged in the carbamate synthesis step was 75.9% by mass.

[Comparative Example 2-24]

1. Carbamate synthesis step

A corresponding tricarbamate was obtained by the same method as in Example 2-124, except that 4-picoline was not used. The yield of the obtained tricarbamate with respect to 4-(aminomethyl)octane-1,8-diamine was 47.5% by mass.

2. Carbamate concentration step

The solution obtained above was depressurized at 60°C to remove a large part of the phenol, and 100 g of the obtained solid was dissolved in 50 g of benzyl toluene at 80°C.

3. Thermal decomposition step

The thermal decomposition was carried out by the same method as in Example 2-123 to obtain 4-(Isocyanatomethyl)octane-1,8-diisocyanate. The yield of the obtained triisocyanate with respect to tricarbamate was 51.2% by mass. The yield with respect to the 4-(aminomethyl)octane-1,8-diamine charged in the carbamate synthesis step was 24.3% by mass.

[Example 2-125]

1. Carbamate synthesis step

A corresponding dicarbamate was obtained by the same method as in Example 2-123, except that 14.0 g of urea was used, 8.0 g of diisopropylethylamine was used instead of 2,4-lutidine, and 22.0 g of 4,4'-methylenebis(cyclohexylamine) was used instead of isophoronediamine. The yield of the obtained dicarbamate with respect to 4,4'-methylenebis(cyclohexylamine) was 90.3% by mass.

2. Carbamate concentration step

The solution obtained above was depressurized at 60°C to remove a large part of the cresol and diisopropylethylamine, and 100 g of the obtained solid was dissolved in 50 g of benzyl toluene at 80°C.

3. Thermal decomposition step

The thermal decomposition was carried out by the same method as in Example 2-123 to obtain bis(4-isocyanatocyclohexyl)methane. The yield of the obtained diisocyanate with respect to dicarbamate was 92.4% by mass. The yield with respect to the 4,4'-methylenebis(cyclohexylamine) charged in the carbamate synthesis step was 83.3% by mass.

[Comparative Example 2-25]

1. Carbamate synthesis step

A corresponding tricarbamate was obtained by the same method as in Example 2-125, except that diisopropylethylamine was not used. The yield of the obtained dicarbamate with respect to 4,4'-methylenebis(cyclohexylamine) was 51.8% by mass.

2. Carbamate concentration step

The solution obtained above was depressurized at 60°C to remove a large part of the cresol, and 100 g of the obtained solid was dissolved in 50 g of benzyl toluene at 80°C.

3. Thermal decomposition step

The thermal decomposition was carried out by the same method as in Example 2-123 to obtain bis(4-isocyanatocyclohexyl)methane. The yield of the obtained diisocyanate with respect to dicarbamate was 68.9% by mass. The yield with respect to the 4,4'-methylenebis(cyclohexylamine) charged in the carbamate synthesis step was 35.7% by mass.

[Example 2-126]

1. Carbamate synthesis step

A corresponding dicarbamate was obtained by the same method as in Example 2-123, except that 6.7 g of urea was used, p-cumylphenol was used instead of phenol, 0.13 g of 1,5-diazabicyclo-[4.3.0]nona-5-ene was used instead of 2,4-lutidine, and 5.8 g of hexamethylenediamine was used instead of isophoronediamine. The yield of the obtained dicarbamate with respect to hexamethylenediamine was 92.2% by mass.

2. Thermal decomposition step

The above-described reaction mixture was heated to 250°C while stirring with the same apparatus, and the internal pressure was set to 85 kPa. The reaction was carried out for 3 hours while recovering the low-boiling compound generated by the thermal decomposition from the upper part of the condenser. Thereafter, the inside was cooled to room temperature at atmospheric pressure, and a part of the solution recovered from the upper part of the condenser was analyzed by gas chromatography. As a result, 88.4% by mass of hexamethylene isocyanate was generated with respect to hexamethylene diamine. The yield with respect to the hexamethylenediamine charged in the carbamate synthesis

step was 81.4% by mass.

[Comparative Example 2-26]

1. Carbamate synthesis step

A corresponding dicarbamate was obtained in the same manner as in Example 2-126, except that 0.13 g of 1,5-diazabicyclo-[4.3.0]nona-5-ene was not used. The yield of the obtained dicarbamate with respect to hexamethylenediamine was 49.0% by mass.

2. Thermal decomposition step

The thermal decomposition was carried out by the same method as in Example 2-123 to obtain hexamethylene isocyanate. The yield of the obtained hexamethylene isocyanate with respect to hexamethylenediamine was 65.6% by mass. The yield with respect to the hexamethylenediamine charged in the carbamate synthesis step was 32.1% by mass.

<<Third Test>>

<Analysis method>

(1) $^1$H-NMR analysis method

$^1$H-NMR analysis was performed using a JNM-A400 FT-NMR system manufactured by JEOL Ltd. as an apparatus.

(1-1) Preparation of $^1$H-NMR analysis sample

0.3 g of the sample solution was weighed, and a solution obtained by adding 0.7 g of deuterated chloroform and 0.05 g of dimethyldiphenylsilane as an internal standard substance to the sample solution and uniformly mixing was used as an NMR analysis sample.

(1-2) Quantitative analysis method

Each standard substance was analyzed, and a quantitative analysis of the analysis sample solution was performed based on a created calibration curve.

(2) Gas chromatography (GC) analysis method

(2-1) Preparation of gas chromatography analysis sample

1.0 g of the sample solution was weighed, and a solution obtained by adding 10 g of acetonitrile and 0.1 g of anisole as an internal standard substance to the sample solution and uniformly mixing was used as a gas chromatography analysis sample.

(2-2) Analysis conditions

The analysis was performed under the following conditions.

(Measurement conditions)

Device: GC-2010 manufactured by Shimadzu Corporation
Column: DB-1
Diameter: 0.25 mm, length: 30 m, film thickness: 1.0 μm
Column temperature: 60°C to 300°C
Injection port temperature: 300°C
Carrier gas: helium

Carrier gas flow rate: 40 mL/min
Detector: flame ionization detector (FID)

(3) Liquid chromatography (LC) analysis method

(3-1) Preparation of liquid chromatography analysis sample

1.0 g of the sample solution was weighed, and a solution obtained by adding 10 g of acetic acid to the sample solution and uniformly mixing was used as a liquid chromatography analysis sample.

(3-2) Analysis conditions

The analysis was performed under the following conditions.

(Measurement conditions)

Device: LC-10AT manufactured by Shimadzu Corporation
Column: Inertsil ODS
Particle diameter: 5 μm, inner diameter: 2.1 mm, length: 250 mm
Column temperature: 40°C
Developing solvent: water/acetonitrile = 90/10
Solvent flow rate: 1 mL/min
Detector: photodiode array detector

(3-3) Quantitative analysis method

Each standard substance was analyzed, and a quantitative analysis of the analysis sample solution was performed based on a created calibration curve.

In addition, from $^1$H-NMR, the molar amount of nitrogen atoms included in each functional group of the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group in the liquid-phase component recovered after the production of the isocyanate compound, and the amount of hydrocarbons, included in the high-boiling-point compound, that is, the molar amount of $R^{71a}$ in General Formula (Vila) was calculated. The hydrocarbon amount (Xa) of the high-boiling-point compound can also be represented by a relational expression of Xa = YalZa.

The Ya here is the total amount of nitrogen contained in each functional group of the high-boiling-point compound, and Za is n71a in General Formula (VIIa). For example, in a case where the isocyanate group, the biuret group, the allophanate group, the isocyanurate group, the carbamate group, the urea group, the carbodiimide group, the uretonimine group, the iminotrimer group, the Fries rearrangement body, the cyclized product of the Fries rearrangement body, and the oxycarbonyl carbamate group in the liquid-phase component recovered after the production of the isocyanate compound were used, the sum represented by (isocyanate group × 1 + carbamate group × 1 + Fries rearrangement body × 1 + cyclized product of Fries rearrangement body × 1 + oxycarbonyl carbamate group × 1 + urea group × 2 + carbodiimide group × 2 + allophanate group × 2 + biuret group × 3 + isocyanurate group × 3 + uretonimine group × 3 + iminotrimer group × 3) could be calculated by dividing the sum by n71a in General Formula (Vila).

In General Formulae (IX-1) to (IX-12), which were the functional groups of the high-boiling-point compound, a wavy line represents a bonding site, and the high-boiling-point compound is converted into a compound in which $R^{71a}$ in General Formula (VIIa) is bonded to the nitrogen atom and a compound in which $R^{52c}$ in General Formula (Vc) is bonded to the oxygen atom.

In addition, the contents of the high-boiling compound and the hydroxy compound were calculated from the LC measurement value. The content and the yield of the amine compound were calculated from the measurement values of GC and LC.

[Example 3-1]

1. Recovery of liquid-phase component after separation of isocyanate compound (VIIa)

The following steps (x1) to (x4) were performed using a device shown in FIG. 20.

(1) Step (x1): production step of carbamate compound

A mixture of 9.8 kg of 2,4-toluenediamine, 10.3 kg of urea, and 261.9 kg of 4-(1,1,3,3-tetramethylbutyl)phenol was supplied to a continuous multi-stage distillation column a101 from a line a1 at 90 kg/hour.

The continuous multi-stage distillation column al01 was a device for producing a carbamate compound, and the column bottom temperature was set to 250°C and the column top pressure was set to 5 kPa by heating with a reboiler a111.

The reaction solution was extracted from the bottom portion of the continuous multi-stage distillation column a101 at 80 kg/hour.

The gas-phase component was extracted from the top portion of the continuous multi-stage distillation column a101 and introduced into a condenser a121 through a line a2. The gas-phase component introduced into the condenser a121 was cooled to 100°C in the condenser a121 to obtain a mixed solution of 4-(1,1,3,3-tetramethylbutyl)phenol and urea. From a line a3, a mixed solution of 4-(1,1,3,3-tetramethylbutyl)phenol and urea obtained by the condenser a121 was supplied to a line a1 at 9 kg/hour.

The gas-phase component not condensed in the condenser a121 was discharged from the condenser a121 through a line a8.

(2) Step (x2): thermal decomposition step of carbamate compound

The reaction solution was supplied to the thermal decomposition device a102 from the bottom portion of the continuous multi-stage distillation column a 101 through a line a4. The thermal decomposition device a102 was a tubular reactor and was a device for producing an isocyanate compound by a thermal decomposition reaction of a carbamate compound. The internal pressure was set to 1 kPa, and the device was heated to 250°C by external heating.

(3) Step (x3): purification step of isocyanate compound

The component generated in the thermal decomposition device a102 was supplied from a line a6 to a separation column a103, pentadecane was supplied by a line a15, and 4-(1,1,3,3-tetramethylbutyl)phenol and 2,4-tolylene diisocyanate were separated. The heat quantity required for the distillation separation was supplied from a reboiler A112.

4-(1,1,3,3-tetramethylbutyl)phenol was recovered from the line a11 from the bottom of the separation column a103.

The component containing 2,4-toluene diisocyanate, recovered from the top of the separation column a103, was supplied to the purification column a104 through the condenser a122 and the line a14, and distillation purification of 2,4-toluene diisocyanate was performed. The heat quantity required for the distillation purification was supplied from a reboiler a113.

2,4-toluene diisocyanate was recovered from the top of the purification column a104 through a condenser a123 and a line a18 at a rate of 2.4 kg/hour. The column bottom component of the purification column a104 was extracted from a line a20.

(4) Step (x4): recovery step of liquid-phase component

A part of the liquid-phase component recovered from the bottom portion of the thermal decomposition device a102 was recovered through the line a7, and the liquid-phase component was supplied as a liquid-phase component containing the high-boiling-point compound in the subsequent step (a1) with a liquid temperature of 180°C. The remaining liquid-phase component was supplied to the thermal decomposition device a102 again through the line a5.

In a case where the liquid-phase component recovered from the line a7 was analyzed by $^1$H-NMR, the liquid-phase component contained, per 1 kg of the liquid-phase component, 0.98 mol of carbamate groups, 0.065 mol of urea groups and carbodiimide groups in combination, 0.13 mol of biuret groups, isocyanurate groups, and uretonimine groups in combination, 0.06 mol of allophanate groups, and 0.22 mol of Fries rearrangement terminals and Fries rearrangement bodies in combination.

In addition, as a result of liquid chromatography analysis, the liquid-phase component included a urea group- and urethane group-containing compound represented by Formula (IX-9-1) and a biuret group- and urethane group-containing compound represented by Formula (IX-7-1), and 2.82 mol of 4-(1,1,3,3-tetramethylbutyl)phenol.

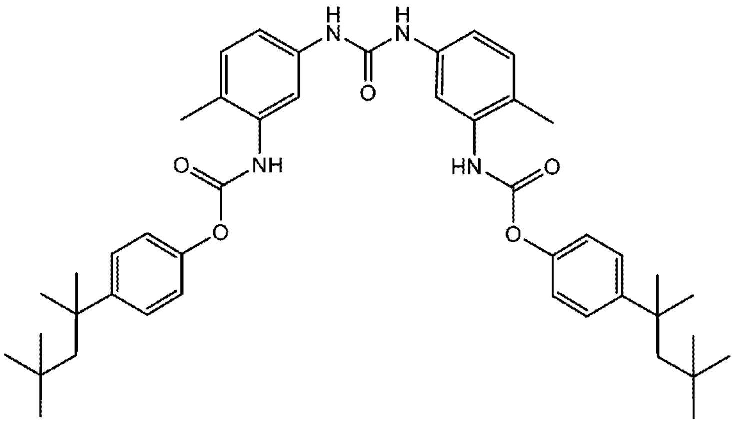

(IX-9-1)

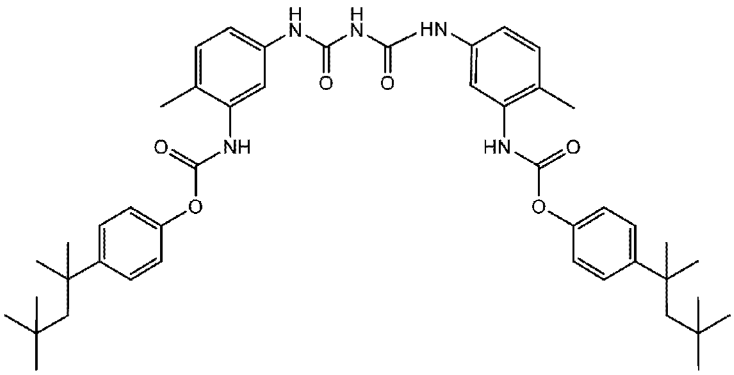

(IX-7-1)

2. Recovery (regeneration) of amine compound

The following step (a1) and step (a2) were performed using a device shown in FIG. 21.

(1) Step (a1): mixing step

50 kg of the liquid-phase component containing the high-boiling-point compound recovered through the line a7 and 50 kg of 4-(1,1,3,3-tetramethylbutyl)phenol were supplied to a pressure-resistant reactor b101 through a line b1 in a state of being held at 180°C.

(2) Step (a2): reaction step

Next, 50 kg of water (ratio to the total molar amount of nitrogen atoms included in the groups (IX-1) to (IX-12): 30 times by mole) and 1.5 kg of sodium hydroxide (ratio to the total molar amount of nitrogen atoms included in the groups (IX-1) to (IX-12): 0.41 times by mole) were supplied to the pressure-resistant reactor b101 to obtain a mixed solution. The pressure-resistant reactor b101 was heated at 280°C and a pressure of 6.6 MPa for 5 hours.

In the step (a2), the gas-phase component in the pressure-resistant reactor b101 was introduced into a condenser b121 through a line b3. The gas-phase component introduced into the condenser b121 was cooled to 10°C in the condenser b121 to obtain a condensate. The condensate was returned to the pressure-resistant container b101 through the line b2. This process was continuously performed during the step (a2). The gas-phase component not condensed was extracted as a gas component (the analysis result showed that carbon dioxide was a main component) through a line b4 from the pressure retaining valve b131. This process was continuously performed during the step (a2).

After performing the step (a2) and cooling, the reaction solution was recovered in a storage tank b102 through the line b5. In a case where the reaction solution was analyzed by gas chromatography, 46 mol of 2,4-toluenediamine was contained.

(3) Step (b): separation step of amine compound (IIa)

A step (b) was performed using a device shown in FIG. 22.

The reaction solution recovered into the storage tank b102 in the step (a2) was supplied to a storage tank c101 with a jacket through a line c1. The inside temperature was maintained at 80°C by allowing hot water to flow through the jacket of the storage tank c101, and the mixture was allowed to stand in this state, and the lower layer was recovered in a storage tank c103 through a line c3. On the other hand, from the time when the change in electrical conductivity was sensed by an electrical conductivity meter accompanying the line c3, the upper layer was recovered to a storage tank c102 through a line c2.

As a result of the analysis, the liquid-phase component recovered in the storage tank c102 was mainly 2,4-toluenediamine and 4-(1,1,3,3-tetramethylbutyl)phenol. On the other hand, as a result of the analysis, the liquid-phase component recovered in the storage tank c103 was mainly water. The reaction solution recovered in the storage tank c102 was distilled under reduced pressure to recover a crude 2,4-toluenediamine-containing liquid and a crude 4-(1,1,3,3-tetramethylbutyl)phenol-containing liquid. The yield of 2,4-toluenediamine was 88% by mass. The yield was calculated by the following expression. In the expression, "Mass of structural unit derived from 2,4-toluenediamine contained in high-boiling-point compound in liquid-phase component recovered in step (x4)" was calculated from the amount of hydrocarbon contained in the high-boiling-point compound by $^1$H-NMR analysis.

(Yield of 2,4-toluenediamine (% by mass))

= (Mass of 2,4-toluenediamine recovered in step (b)) × 100/(Mass of structural unit derived from 2,4-toluenediamine contained in high-boiling compound in liquid-phase component recovered in step (x4))

(4) Step (c): purification step of amine compound (IIa)

The crude 2,4-toluenediamine-containing liquid recovered in the step (b) was distilled and purified. The purity of the recovered 2,4-toluenediamine was 99% by mass or more (including an analysis error of gas chromatography). In addition, the metal atom content with respect to the total mass of 2,4-toluenediamine was less than 1,000 ppm by mass, and the halogen atom content was less than 1,000 ppm by mass.

(5) Step (e): separation step of hydroxy compound

The reaction solution recovered in the storage tank c 102 was distilled under reduced pressure to recover a crude 2,4-toluenediamine-containing liquid and a crude 4-(1,1,3,3-tetramethylbutyl)phenol-containing liquid. The yield was 84% by mass. The yield was calculated by the following expression. In the expression, "Mass of structural unit derived from hydroxy compound contained in high-boiling-point compound in liquid-phase component recovered in step (x4)" was the total mass of the carbamate group, the allophanate group, the Fries rearrangement terminal, the Fries rearrangement bod, and the oxycarbonyl carbamate group × 2.

(Yield of hydroxy compound (% by mass))

= (Mass of hydroxy compound recovered in step (d)) × 100/(Mass of structural unit derived from hydroxy compound contained in high-boiling compound in liquid-phase component recovered in step (x4))

(6) Step (f): purification step of hydroxy compound

The crude 4-(1,1,3,3-tetramethylbutyl)phenol-containing liquid recovered in the step (e) was distilled and purified. The purity of the recovered 4-(1,1,3,3-tetramethylbutyl)phenol was 99% by mass or more (including an analysis error of gas chromatography). In addition, the metal atom content with respect to the total mass of 4-(1,1,3,3-tetramethylbutyl)phenol was less than 1,000 ppm by mass, and the halogen atom content was less than 1,000 ppm by mass.

(7) Step (d) and step (g): production step of isocyanate compound (VIIa) by re-using amine compound (IIa) and hydroxy compound

In the device shown in FIG. 20, in a case where the 2,4-toluenediamine purified in the step (c) and the 4-(1,1,3,3-tetramethylbutyl)phenol purified in the step (f) were used, the shortage amounts of the 2,4-toluenediamine and the 4-(1,1,3,3-tetramethylbutyl)phenol were newly added and supplied to the continuous multi-stage distillation column a101 through the line a1, and the above-described steps (×1) to (x3) were performed, 2,4-tolylene diisocyanate was recovered from the line a18 at 2.4 kg/hour.

(8) Step (h): step of re-using liquid-phase components recovered from steps (b), (c), and (f) in step (a1) and step (a2)

In the device shown in FIG. 21, the liquid-phase component recovered in the tank c103 in the step (b) and the distillation residue recovered in the step (c) and the step (f) were used, the shortage amounts of water, sodium hydroxide, and 4-(1,1,3,3-tetramethylbutyl)phenol were newly added, and the recovered liquid-phase component was supplied to the pressure-resistant reactor b101 through the line b1. As a result of performing the step (a1) and the step (a2) described above, 46 mol of 2,4-toluenediamine was contained in the reaction solution recovered in the storage tank b102 through the line b5.

[Example 3-2]

1. Recovery of liquid-phase component after separation of isocyanate compound (VIIa)

(1) Step (x1): production step of carbamate compound

The following step (x1) was performed using a device shown in FIG. 23.

A mixture of 11.3 kg of 1-amino-3-aminomethyl-3,5,5-trimethylcyclohexane, 15.1 kg of urea, and 220.3 kg of 1-butanol was supplied to a continuous multi-stage distillation column a201 from a line a21 at 20 kg/hour. 1-butanol was appropriately supplied from a line a24.

The gas-phase component generated in the continuous multi-stage distillation column a201 was introduced into a condenser a221 through a line a25. The gas-phase component introduced into the condenser a221 was cooled to 0°C to obtain a mixed solution of 1-butanol and urea. A part of the mixed solution was supplied to the continuous multi-stage distillation column a201 from the line a27 at 3.5 kg/hour, and the remainder was recovered in a storage tank a202 through a26.

The continuous multi-stage distillation column a201 was a device for producing a carbamate compound, and the column bottom temperature was set to 220°C and the column top pressure was set to 1.2 MPa by heating with a reboiler a211.

The reaction solution was extracted from the bottom portion of the continuous multi-stage distillation column a201, and recovered in the storage tank a203 through the line a22.

(2) Step (xl-b): preliminary concentrating step

Next, a preliminary concentrating step was performed using a device shown in FIG. 24. The reaction solution recovered in the storage tank a203 in the carbamating step was supplied to a thin film evaporator a301 through a line a31 at approximately 21 kg/hour. In the thin film evaporator a301, the temperature of the heating and evaporation surface was set to 130°C, and the internal pressure was set to 70 kPa.

The gas-phase component generated by the thin film evaporator a301 was condensed in a condenser a321 through a line a33 and recovered in a storage tank a303. The recovered product was 1-butanol.

On the other hand, the liquid-phase component generated by the thin film evaporator a301 was recovered in the storage tank a302 at 10 kg/hour through the line a32.

(3) Step (x2): thermal decomposition step of carbamate compound

Steps (x2) to (x4) were performed using a device shown in FIG. 25.

A thermal decomposition device a401 was a multi-stage distillation column for producing isophorone diisocyanate by a thermal decomposition reaction of an N-substituted carbamate compound, and the total reflux state of the dibenzyl ether was achieved by setting the column top pressure to 25 kPa and heating with a reboiler a411. Here, the liquid-phase component recovered in the preliminary concentrating step into the storage tank a302 from the line a40 was supplied at 5 kg/hour, and dibenzyl ether was supplied from a line a49. The gas-phase component was extracted from the top of the column, recovered from the line a42b through the condenser a421, and the liquid-phase component was recovered from the line a41a.

(4) Step (x3): purification step of isocyanate compound

A fraction containing isophorone diisocyanate was recovered from a line a43 provided in the middle of the thermal decomposition device a401 and supplied to a separation column a402. The gas-phase component including 1-butanol was distilled and separated in the separation column a402, and the gas-phase component was recovered from the line a44b through the condenser a422. The heat quantity required for the distillation separation was supplied from a reboiler A412.

The liquid-phase component recovered from the bottom portion of the separation column a402 was supplied to the purification column a403 through the line a45a. In the purification column a403, the liquid-phase component was distilled and separated, and recovered from the line a47a. The heat quantity required for the distillation separation in the purification column a403 was supplied from a reboiler A413.

The gas-phase component recovered from the top of the purification column a403 was condensed in a condenser a423, and the isophorone diisocyanate was recovered from the line a46b.

(5) Step (x4): recovery step of liquid-phase component

The liquid-phase component recovered from the line a41a was used as the liquid-phase component containing the high-boiling-point compound of the present embodiment.

In a case where the liquid-phase component was analyzed by $^1$H-NMR, the liquid-phase component was a mixture containing, per 1 kg of the liquid-phase component, 2.56 mol of carbamate groups, 0.07 mol of urea groups and carbodiimide groups in combination, 0.12 mol of biuret groups, isocyanurate groups, and uretonimine groups in combination, 0.05 mol of allophanate groups, and dibenzyl ether.

In a case where the liquid-phase component was analyzed by liquid chromatography, a urea group- and urethane group-containing compound represented by Formula (IX-9-2) was contained.

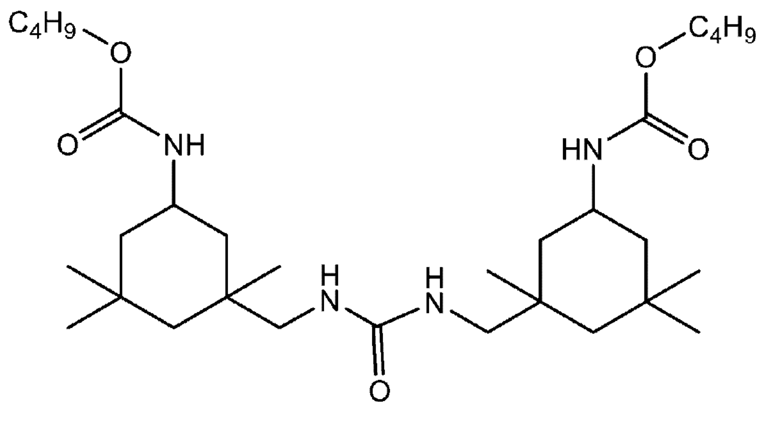

(IX-9-2)

2. Recovery (regeneration) of amine compound

The following step (a1) and step (a2) were performed using a device shown in FIG. 26.

(1) Step (a1): mixing step

50 kg of the liquid-phase component containing the high-boiling-point compound recovered through the line

a41a and 50 kg of phenol were supplied to a pressure-resistant reactor b201 through a line b20 in a state of being held at 180°C.

(2) Step (a2): reaction step

100 kg of water (ratio to the total molar amount of nitrogen atoms included in the groups (IX-1) to (IX-12): 35 times by mole), 50 kg of 1-amino-3-aminomethyl-3,5,5-trimethylcyclohexane (ratio to the total molar amount of nitrogen atoms included in the groups (IX-1) to (IX-12): 1.9 times by mole), and 3 kg of calcium hydroxide (ratio to the total molar amount of nitrogen atoms included in the groups (IX-1) to (IX-12): 0.26 times by mole) were added to the pressure-resistant reactor b201, and a reaction was carried out at 250°C and 4.1 MPa for 5 hours while circulating the liquid-phase component through the line b21 and the line b22 by means of a pump b241.

In the step (a2), the gas-phase component in the pressure-resistant reactor b201 was introduced into the condenser b221 through the line b24. The condensed liquid obtained by cooling the introduced gas-phase component to 10°C with the condenser b221 was returned to the pressure-resistant reactor b201 through the line b23. This process was continuously performed during the step (a2). The gas-phase component not condensed was extracted as a gas component (the analysis result showed that carbon dioxide was a main component) through a line b26 from the pressure retaining valve b231. This process was continuously performed during the step (a2). After performing the step (a2) and cooling, the reaction solution was recovered in a storage tank b202 through the line b25.

(3) Step (b): separation step of amine compound (IIa)

In a step (e) described later, the residual liquid obtained in a case where the hydroxy compound was recovered was distilled under reduced pressure to recover a crude 1-amino-3-aminomethyl-3,5,5-trimethylcyclohexane-containing liquid. The yield was 90% by mass.

(4) Step (c): purification step of amine compound (IIa)

The crude 1-amino-3-aminomethyl-3,5,5-trimethylcyclohexane-containing liquid recovered in the step (b) was distilled and purified. The recovered 1-amino-3-aminomethyl-3,5,5-trimethylcyclohexane had a purity of 99% by mass or more (including an analysis error of gas chromatography). In addition, the metal atom content with respect to the total mass of 1-amino-3-aminomethyl-3,5,5-trimethylcyclohexane was less than 1,000 ppm by mass, and the halogen atom content was less than 1,000 ppm by mass.

(5) Step (e): separation step of hydroxy compound

Before performing the above-described step (b), the reaction solution recovered in the storage tank b202 was distilled under reduced pressure to collect a crude 1-butanol-containing liquid. The yield was 80% by mass.

(6) Step (f): purification step of hydroxy compound

The crude 1-butanol-containing liquid recovered in the step (e) was distilled and purified. The purity of the recovered 1-butanol was 99% by mass or more (including an analysis error of gas chromatography). In addition, the metal atom content with respect to the total mass of 1-butanol was less than 1,000 ppm by mass, and the halogen atom content was less than 1,000 ppm by mass.

[Example 3-3]

1. Recovery of liquid-phase component after separation of isocyanate compound (VIIa)

(1) Step (×1): production step of carbamate compound

A step (x1) was performed using a device shown in FIG. 27.

50 kg of phenol was supplied to a stirring tank a501 through a line a51, and 38 kg of diphenyl carbonate was supplied to obtain a uniform solution. 15 kg of 4,4'-dicyclohexylmethanediamine was slowly added thereto in a state in which the stirring tank a501 was held at 40°C. After completion of the addition, stirring was continued for 5 hours, and the reaction solution was transferred to a storage tank a502 through a line a52.

(2) Step (x2): thermal decomposition step of carbamate compound

A thermal decomposition reaction of the carbonyl compound was carried out using dibenzyl toluene instead of the dibenzyl ether and using the same method as in "Step (x2): thermal decomposition step of carbamate compound" of Example 3-2, to obtain the liquid-phase component containing the high-boiling-point compound.

In a case where the liquid-phase component containing the high-boiling-point compound was analyzed by $^1$H-NMR, the liquid-phase component was a mixture containing, per 1 kg of the liquid-phase component, 2.57 mol of carbamate groups, 0.085 mol of urea groups and carbodiimide groups in combination, 0.40 mol of biuret groups, isocyanurate groups, and uretonimine groups in combination, 0.31 mol of allophanate groups, 0.02 mol of iminotrimer groups, 0.02 mol of oxycarbonylcarbamate groups, and dibenzyltoluene.

In a case where the liquid-phase component containing the high-boiling-point compound was analyzed by liquid chromatography, the liquid-phase component containing the high-boiling-point compound contained a urea group- and urethane group-containing compound represented by Formula (IX-9-3).

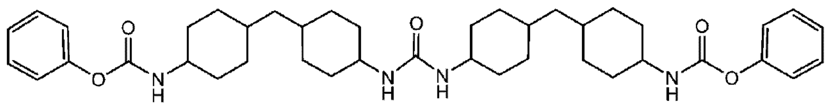

(IX-9-3)

2. Recovery (regeneration) of amine compound

The following step (a1) and step (a2) were performed using a device shown in FIG. 28.

(1) Step (a1): mixing step

In the step (x2), 25 kg of the liquid-phase component containing the high-boiling-point compound recovered through the line a41a and 50 kg of phenol were supplied to a stirring tank b301 through a line b30 in a state of being held at 180°C.

(2) Step (a2): reaction step

100 kg of water (ratio to the total molar amount of nitrogen atoms included in the groups (IX-1) to (IX-12): 24 times by mole) and 3 kg of potassium hydroxide (ratio to the total molar amount of nitrogen atoms included in the groups (IX-1) to (IX-12): 0.23 times by mole) were supplied to the stirring tank B301 to obtain a uniform solution at 250°C and 4.9 MPa. The solution was continuously supplied to a filled column b302 heated to 250°C and pressurized to 4.1 MPa through a line b33.

The gas-phase component generated in the filled column b302 was introduced into the stirring tank b301 through the line b34.

In the step (a2), the gas-phase component in the stirring tank b301 was introduced into the condenser b321 through the line b32. The gas-phase component introduced was cooled to 5°C in the condenser b321 to obtain a condensate. The obtained condensate was returned to the stirring tank b301 through the line b31. This process was continuously performed during the step (a2). The gas-phase component not condensed was extracted as a gas component (the analysis result showed that carbon dioxide was a main component) through a line b36 from the pressure retaining valve b331. This process was continuously performed during the step (a2).

In the step (a2), the reaction solution was recovered from the bottom portion of the filled column b302 through the line b35 to the storage tank b303. The average retention time was 3 hours.

In a case where the obtained reaction solution was analyzed by gas chromatography, 4,4'-dicyclohexylmethanediamine was contained.

(3) Step (b): separation step of amine compound (IIa)

In a step (e) described later, the residual liquid obtained in a case where the hydroxy compound was recovered was distilled under reduced pressure to recover a crude 4,4'-dicyclohexylmethanediamine-containing liquid. The yield was 88% by mass.

(4) Step (c): purification step of amine compound (IIa)

The crude 4,4'-dicyclohexylmethanediamine-containing liquid recovered in the step (b) was distilled and purified.

The purity of the recovered 4,4'-dicyclohexylmethanediamine was 99% by mass or more (including an analysis error of gas chromatography). In addition, the metal atom content with respect to the total mass of 4,4'-dicyclohexylmethanediamine was less than 1,000 ppm by mass, and the halogen atom content was less than 1,000 ppm by mass.

(5) Step (e): separation step of hydroxy compound

The reaction solution recovered in the storage tank b303 was distilled under reduced pressure to recover phenol. The yield was 93% by mass.

(6) Step (f): purification step of hydroxy compound

The crude phenol-containing liquid recovered in the step (e) was distilled and purified. The purity of the recovered phenol was 99% by mass or more (including an analysis error of gas chromatography). In addition, the metal atom content with respect to the total mass of phenol was less than 1,000 ppm by mass, and the halogen atom content was less than 1,000 ppm by mass.

[Example 3-4]

1. Recovery of liquid-phase component after separation of isocyanate compound (VIIa)

(1) Step (x1): production step of carbamate compound

A step (x1) was performed using a device shown in FIG. 27.

A carbonyl compound was produced by using the same method as in "Step (x1): production step of carbamate compound" of Example 3-3, except that 50 kg of phenol, 38 kg of diphenyl carbonate, and 8.2 kg of 1,6-hexamethylenediamine were used to obtain a reaction solution. In a case where the reaction solution was analyzed by liquid chromatography, a carbamate compound represented by Formula (Vc-1) was produced with a yield of 95% by mass with respect to 1,6-hexamethylenediamine.

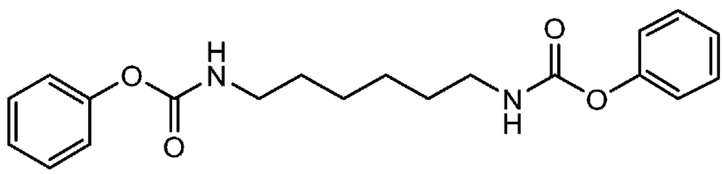

(Vc-1)

(2) Step (x1-a): transesterification reaction step

A step (x1-a) was performed using a device shown in FIG. 29.

4-(α,α-dimethylbenzyl)phenol was supplied to a multi-stage distillation column a601 through a line a65, and the multi-stage distillation column a601 was heated by a reboiler a611 to be in a total reflux state. The reaction solution obtained in the step (x1) (production step of carbamate compound) was supplied through the line a61, and the transesterification reaction of the carbamate compound represented by Formula (VI-1) was carried out. The gas-phase component containing the phenol generated by the transesterification reaction was passed through a line a63a and supplied to a condenser a621. A part of the condensate obtained by cooling to 100°C in a condenser a621 was supplied to the multi-stage distillation column a601 through a line a64, and the remaining condensate was recovered in a storage tank a602 through a line a63b. The obtained reaction solution was recovered in a storage tank a603 through a line a62a.

In a case where the reaction solution was analyzed by liquid chromatography, a carbamate compound represented by Formula (Vc-2) was produced at a yield of 95% by mass with respect to the carbamate compound represented by Formula (Vc-1).

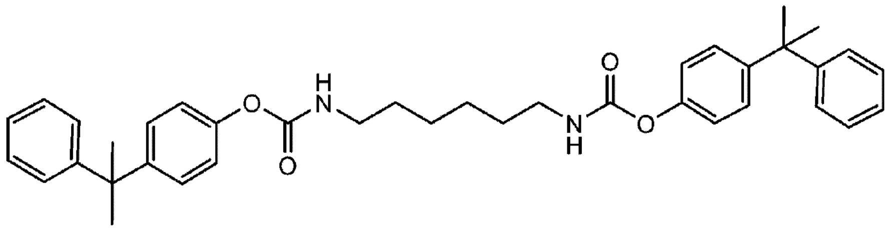

(Vc－2)

(3) Step (x2): thermal decomposition step of carbamate compound

A step (x2) was performed using a device shown in FIG. 30.

The reaction solution recovered in the storage tank a603 was supplied to a thermal decomposition device a701 through a line a71. A thermal decomposition reaction of the carbamate compound was carried out using the same method as in the step (x2) (thermal decomposition step of carbamate compound) of Example 3-1, except that pentadecane was not used, and the liquid-phase component containing a high-boiling-point compound was obtained through a line a73.

In a case where the liquid-phase component containing the high-boiling-point compound was analyzed by $^1$H-NMR, the liquid-phase component was a mixture containing, per 1 kg of the liquid-phase component, 1.55 mol of carbamate groups, 0.095 mol of urea groups and carbodiimide groups in combination, 0.34 mol of biuret groups, isocyanurate groups, and uretonimine groups in combination, 0.24 mol of allophanate groups, 0.02 mol of Fries rearrangement bodies and cyclized products of Fries rearrangement body in combination, 0.01 mol of iminotrimer groups, 0.01 mol of oxycarbonylcarbamate groups, and 1.02 mol of 4-(α,α-dimethylbenzyl)phenol.

In a case where the liquid-phase component containing the high-boiling-point compound was analyzed by liquid chromatography, the liquid-phase component containing the high-boiling-point compound contained a urea group- and urethane group-containing compound represented by Formula (IX-9-4).

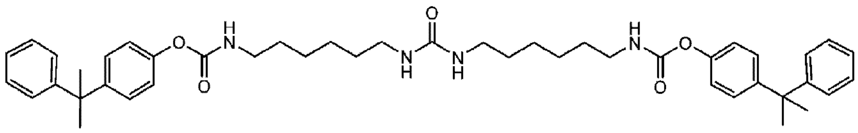

(IX－9－4)

2. Recovery (regeneration) of amine compound

The following step (a1) and step (a2) were performed using a device shown in FIG. 31.

(1) Step (a1): mixing step

50 kg of the liquid-phase component containing the high-boiling-point compound recovered in the step (x2) and 50 kg of 4-(α,α-dimethylbenzyl)phenol were heated to 280°C through a line b40 in a state of being held at 180°C, and supplied to an extruder b401 pressurized to 6.6 MPa.

(2) Step (a2): reaction step

At the same time as the step (a1), 100 kg of water (ratio to the total molar amount of nitrogen atoms included in the groups (IX-1) to (IX-12): 34 times by mole) and 2 kg of cesium carbonate (ratio to the total molar amount of nitrogen atoms included in the groups (IX-1) to (IX-12): 0.04 times by mole) were continuously added to the extruder b401 through the line b40, with the proportion.

In the step (a2), the gas-phase component of the extruder b401 was extracted from a vent opening b451 provided in the extruder b401 through the line b41 and introduced into the condenser b421. The gas-phase component introduced was cooled in the condenser b421 to obtain a condensate. The obtained condensate was returned to the extruder b401 through the line b43. This process was continuously performed during the step (a2). From a line b45, the gas-phase component not condensed was extracted as a gas component (the analysis result showed that carbon dioxide was a main component) through a line b44 from the pressure retaining valve b431. This process was continuously performed during the step (a2).

In the step (a2), the reaction solution was recovered from a discharge port of the extruder b401 through the line b42 from a pressure retaining valve b432 and through a line b46 to a storage tank b402.

In a case where the reaction solution was analyzed by gas chromatography, 1,6-hexamethylenediamine was

contained.

(3) Step (b): separation step of amine compound (IIa)

The recovered reaction solution was distilled under reduced pressure to recover a crude 1,6-hexamethylenediamine-containing liquid. The yield was 76% by mass.

(4) Step (c): purification step of amine compound (IIa)

The crude 1,6-hexamethylenediamine-containing liquid recovered in the step (b) was distilled and purified. The purity of the recovered 1,6-hexamethylenediamine was 99% by mass or more (including an analysis error of gas chromatography). In addition, the metal atom content with respect to the total mass of 1,6-hexamethylenediamine was less than 1,000 ppm by mass, and the halogen atom content was less than 1,000 ppm by mass.

(5) Step (e): separation step of hydroxy compound

In the step (b), the residual liquid after the recovery of 1,6-hexamethylenediamine was distilled under reduced pressure to recover a crude 4-(α,α-dimethylbenzyl)phenol-containing liquid. The yield was 74% by mass.

(6) Step (f): purification step of hydroxy compound

The crude 4-(α,α-dimethylbenzyl)phenol-containing liquid recovered in the step (e) was distilled and purified. The purity of 4-(α,α-dimethylbenzyl)phenol was 99% by mass or more (including an analysis error of gas chromatography). In addition, the metal atom content with respect to the total mass of 4-(α,α-dimethylbenzyl)phenol was less than 1,000 ppm by mass, and the halogen atom content was less than 1,000 ppm by mass.

[Example 3-5]

1. Recovery of liquid-phase component after separation of isocyanate compound (VIIa)

(1) Step (x1): production step of carbamate compound, and step (x1-b): preliminary concentrating step

Steps (x1) and (xl-b) were performed using devices shown in FIGS. 23 and 24.

A carbamate compound was produced by the same method as in the step (x1) (production step of carbamate compound) of Example 3-2, except that 11.3 kg of 4-aminomethyl-1,8-octanediamine, 15.1 kg of urea, and 220.3 kg of phenol were used to obtain a reaction solution containing a carbamate compound represented by Formula (Vc-3). In a case where the recovered reaction solution was analyzed by liquid chromatography, a carbamate compound represented by Formula (Vc-3) was produced with a yield of 95% by mass with respect to 4-aminomethyl-1,8-octanediamine.

[Chem. 115]

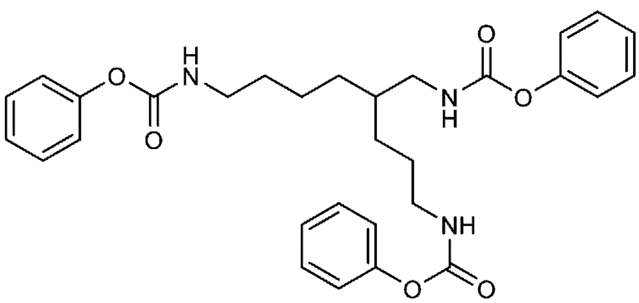

(Vc-3)

Next, using the obtained reaction solution, a preliminary concentration was performed by the step (1-a) (preliminary concentrating step) of Example 3-2.

(2) Step (x2): thermal decomposition step of carbonyl compound

A step (x2) was performed using a device shown in FIG. 32.

A thermal decomposition device a801 is a multi-stage distillation column for producing 4-isocyanatomethyl-1,8-octamethylene diisocyanate by a thermal decomposition reaction of N-substituted carbamate.

Benzophenone was supplied to the thermal decomposition device a801 through a line a85, and the thermal decomposition device a801 was heated with a reboiler a811 to be in a total reflux state. The liquid recovered in the preliminary concentration treatment into a storage tank a303 was supplied through a line a81. The gas-phase component containing the phenol generated by the thermal decomposition was passed through a line a83a and supplied to a condenser a821. A part of the condensate obtained by cooling to 100°C in a condenser a821 was supplied to the thermal decomposition device a801 through a line a84, and the remaining condensate was recovered in a storage tank a802 through a line a83b. The obtained reaction solution was recovered in a storage tank a803 through a line a82a.

In a case where the reaction solution was analyzed by liquid chromatography, 4-isocyanatomethyl-1,8-octamethylene diisocyanate was produced with a yield of 92% by mass with respect to the carbamate compound represented by Formula (Vc-3).

(3) Step (x3): purification step of isocyanate compound

(3-1) Step (x3-1): low-boiling separation step (by multi-stage distillation column)

A step (x3-1) was performed using a device shown in FIG. 33.

The reaction solution obtained in a803 of the previous operation was supplied to a separation column c201. The gas-phase component containing carbon dioxide was distilled and separated in the separation column c201, and the gas-phase component was supplied to a condenser c221 through a line c23a. A part of the condensate obtained by cooling in the condenser c221 was supplied to the separation column c201 through a line c24, and the remaining condensate was recovered in a storage tank c202 through a line c23b. The liquid phase component containing the obtained crude 4-isocyanatomethyl-1,8-octamethylene diisocyanate was recovered into a storage tank c203 through a line c22a. The heat quantity required for the distillation separation was supplied from a reboiler c211.

(3-2) Step (x3-2): high-boiling separation step (purification step using thin film evaporator)

A step (x3-2) was performed using a device shown in FIG. 34.

The crude 4-isocyanatomethyl-1,8-octamethylenediisocyanate-containing liquid obtained in the step (x3-1) was supplied to a thin film evaporator c302 through a line c31 in a state of being held at 100°C.

The supplied liquid was developed in a thin film shape on the heating and evaporation surface c301 heated to 200°C at a pressure of 100 Pa. The gas-phase component was cooled to 70°C in the condenser c321, and the condensed liquid was discharged through a line c34. The liquid-phase component not vaporized was recovered in a collection portion c303 provided at the bottom portion of the thin film evaporator, and discharged through a line c33.

In a case where the reaction solution discharged through the line c34 was analyzed by gas chromatography, the purity of the recovered 4-isocyanatomethyl-1,8-octamethylene diisocyanate was 99% by mass or more (including an analysis error of gas chromatography).

In a case where the liquid-phase component containing the high-boiling-point compound, discharged through the line c33, was analyzed by $^1$H-NMR, the liquid-phase component was a mixture containing, per 1 kg of the liquid-phase component, 4.53 mol of isocyanate groups, 0.01 mol of carbamate groups, 0.1 mol of urea groups and carbodiimide groups in combination, 1.4 mol of biuret groups, isocyanurate groups, and uretonimine groups in combination, 0.05 mol of allophanate groups, 1.35 mol of Fries rearrangement bodies and cyclized products of Fries rearrangement body in combination, and 0.05 mol of iminotrimer groups.

2. Recovery (regeneration) of amine compound

The following step (a1) and step (a2) were performed using a device shown in FIG. 26.

(1) Step (a1): mixing step

50 kg of the liquid-phase component containing the high-boiling-point compound recovered through the line c33 and 50 kg of phenol were supplied to a pressure-resistant reactor b201 through a line b20 in a state of being held at 180°C.

(2) Step (a2): reaction step

Next, a reaction was carried out using the same method as in "Step (a2): reaction step" of Example 3-2, except that 100 kg of water (ratio to the total molar amount of nitrogen atoms included in the groups (IX-1) to (IX-12): 11 times by mole) and 6 kg of triethylamine (ratio to the total molar amount of nitrogen atoms included in the groups (IX-1) to (IX-12): 0.11 times by mole) were used, and the reaction was carried out at 240°C and 3.4 MPa for 3 hours to obtain a reaction solution.

In a case where the reaction solution was analyzed by gas chromatography, 4-aminomethyl-1,8-octanediamine was contained.

(3) Step (b): separation step of amine compound (IIa)

In a step (e) described later, the residual liquid obtained in a case where the hydroxy compound was recovered was distilled under reduced pressure to recover a crude 4-aminomethyl-1,8-octanediamine-containing liquid. The yield was 89% by mass.

(4) Step (c): purification step of amine compound (IIa)

The crude 4-aminomethyl-1,8-octanediamine-containing liquid recovered in the step (b) was distilled and purified. The purity of the recovered 4-aminomethyl-1,8-octanediamine was 99% by mass or more (including an analysis error of gas chromatography). In addition, the metal atom content with respect to the total mass of 4-aminomethyl-1,8-octanediamine was less than 1,000 ppm by mass, and the halogen atom content was less than 1,000 ppm by mass.

(5) Step (e): separation step of hydroxy compound

The reaction solution obtained in the step (a2) was distilled under reduced pressure to recover a crude phenol-containing liquid. The yield was 84% by mass except for the amount added in the step (a1) and the step (a2).

(6) Step (f): purification step of hydroxy compound

The crude phenol-containing liquid recovered in the step (e) was distilled and purified. The purity of the phenol was 99% by mass or more (including an analysis error of gas chromatography). In addition, the metal atom content with respect to the total mass of phenol was less than 1,000 ppm by mass, and the halogen atom content was less than 1,000 ppm by mass.

[Example 3-6]

1. Recovery of liquid-phase component after separation of isocyanate compound (VIIa)

(1) Step (y1): production step of isocyanate compound (VIIa)

Using the devices shown in FIGS. 27 and 32 and using dichlorobenzene as a reaction solvent, a distillation residue (molten product) generated in a case of producing 1,5-pentamethylenediisocyanate by the reaction between 1,5-pentamethylenediamine and phosgene was obtained. The distillation residue was used as a composition containing a high-boiling-point compound.

The composition containing a high-boiling-point compound had a large amount of an insoluble component at the time of sample preparation, and when the composition was filtered through a filter and only a soluble component was analyzed by $^{1}$H-NMR, the result was that the composition was a mixture containing, per 1 kg of liquid-phase component, 3.88 mol of isocyanate groups, 0.51 mol of urea groups and carbodiimide groups in combination, and 2.08 mol of biuret groups, isocyanurate groups, and uretonimine groups in combination.

2. Recovery (regeneration) of amine compound

The following step (a1) and step (a2) were performed using a device shown in FIG. 35.

(1) Step (a1): mixing step

50 kg of the distillation residue obtained in the step (y1) and 100 kg of phenol were mixed in a state of being

held at 200°C and supplied to a thin film evaporator b502 through a line b50.

(2) Step (a2): reaction step

The step (a2) was performed at the same time as the step (a1). Through the line b50, 50 kg of water (ratio to the total molar amount of nitrogen atoms included in the groups (IX-1) to (IX-12): 10 times by mole) and 9 kg of zinc oxide (ratio to the total molar amount of nitrogen atoms included in the groups (IX-1) to (IX-12): 0.20 times by mole) were supplied to a thin film evaporator b502.

The supplied mixed solution was developed in a thin film shape on a heating and evaporation surface b501 heated to 280°C at 6.6 MPa. In the step (a2), the average retention time was adjusted to 1 hour.

In the step (a2), the gas-phase component by-produced in the decomposition reaction on the heating and evaporation surface b501 was extracted from the line b54 and introduced into the condenser b521. The condensate obtained by being cooled to 10°C in the condenser b521 was returned to the thin film evaporator b502 through the line b53. From a line b55, the gas-phase component not condensed was extracted as a gas component (the analysis result showed that carbon dioxide was a main component) through a line b51 from the pressure retaining valve b531. This process was continuously performed during the step (a2).

In the step (a2), the reaction solution generated by the decomposition reaction in the heating and evaporation surface B501 traveled on the surface of the heating and evaporation surface b501, and was recovered in the collection portion b503. The component recovered in the collection portion b503 was recovered in the storage tank b504 through the line b52.

In a case where the recovered reaction solution was analyzed by gas chromatography, 1,5-pentamethylenediamine was contained.

(3) Step (b): separation step of amine compound (IIa)

The recovered reaction solution was distilled under reduced pressure to recover a crude 1,5-pentamethylenediamine-containing liquid. The yield was 68% by mass.

(4) Step (c): purification step of amine compound (IIa)

The crude 1,5-pentamethylenediamine-containing liquid recovered in the step (6-3) was distilled and purified. The purity of the recovered 1,5-pentamethylenediamine was 99% by mass or more (including an analysis error of gas chromatography). In addition, the metal atom content with respect to the total mass of 1,5-pentamethylenediamine was less than 1,000 ppm by mass, and the halogen atom content was less than 1,000 ppm by mass.

[Example 3-7]

1. Recovery of liquid-phase component after separation of isocyanate compound (VIIa)

(1) Step (x1): production step of carbamate compound

Using the device shown in FIG. 23, a carbamate compound was produced by the same method as in the step (x1) (production step of carbamate compound) of Example 3-2, except that 12.7 kg of 4,4'-methylenedianiline, 15.1 kg of urea, and 253.3 kg of 4-(α,α-dimethylbenzyl)phenol were used to obtain a reaction solution containing a carbamate compound represented by Formula (Vc-4).

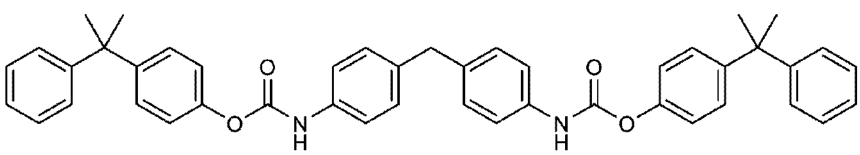

(Vc-4)

(2) Step (x2): thermal decomposition step of carbonyl compound

A step (x2) was performed using a device shown in FIG. 36.

The reaction solution obtained in the step (x1) and dibenzyltoluene were supplied to a thin film evaporator a901 through a line a91 at 7.0 kg/hour. In the thin film evaporator a901, the temperature of the heating and evaporation surface was set to 230°C, and the internal pressure was set to 2.6 kPa.

The gas-phase component generated by the thin film evaporator a901 was condensed in a condenser a921 through a line a93a, and recovered in a storage tank a903 through a line a93b. The recovered product was 4-(α,α-dimethylbenzyl)phenol and dibenzyltoluene.

On the other hand, the liquid-phase component generated by the thin film evaporator a901 was recovered in the storage tank a902 at 5.1kg/hour through the line a92. In a case where the reaction solution was analyzed by liquid chromatography, diphenylmethane diisocyanate was produced with a yield of 88% by mass with respect to the carbamate compound represented by Formula (Vc-4).

(3) Step (x3): purification step of isocyanate compound

(3-1) Step (x3-1): low-boiling separation step (by multi-stage distillation column)

Low-boiling separation by a multi-stage distillation column was performed using a device shown in FIG. 33 and the same method as in "Step (x3-1): low-boiling separation step (by multi-stage distillation column)" of Example 3-5.

The reaction solution obtained in a902 of the previous operation was supplied to the separation column c201. The gas-phase component containing dibenzyltoluene was distilled and separated in the separation column c201, and the gas-phase component was supplied to a condenser c221 through a line c23a. A part of the condensate obtained by cooling in the condenser c221 was supplied to the separation column c201 through a line c24, and the remaining condensate was recovered in a storage tank c202 through a line c23b. The obtained crude diphenylmethane diisocyanate-containing liquid was recovered into a storage tank c203 through a line c22a. The heat quantity required for the distillation separation was supplied from a reboiler c211.

(3-2) Step (x3-2): high-boiling separation step (purification step using thin film evaporator)

High-boiling separation by a multi-stage distillation column was performed using a device shown in FIG. 34 and the same method as in "Step (x3-2): high-boiling separation step (purification step using thin film evaporator)" of Example 3-5.

The obtained crude diphenylmethane diisocyanate-containing liquid obtained in the step (x3-1) was supplied to a thin film evaporator c302 through a line c31 in a state of being held at 100°C.

The supplied liquid was developed in a thin film shape on the heating and evaporation surface c301 heated to 200°C at a pressure of 100 Pa. The gas-phase component was cooled to 80°C in the condenser c321, and the condensed liquid was discharged through a line c34. The liquid-phase component not vaporized was recovered in a collection portion c303 provided at the bottom portion of the thin film evaporator, and discharged through a line c33.

In a case where the reaction solution discharged through the line c34 was analyzed by gas chromatography, the purity of the recovered diphenylmethane diisocyanate was 99% by mass or more (including an analysis error of gas chromatography).

In a case where the liquid-phase component containing the high-boiling-point compound, discharged through the line c33, was analyzed by $^1$H-NMR, the liquid-phase component was a mixture containing, per 1 kg of the liquid-phase component, 1.82 mol of isocyanate groups, 0.01 mol of carbamate groups, 0.02 mol of urea groups and carbodiimide groups in combination, 1.4 mol of biuret groups, isocyanurate groups, and uretonimine groups in combination, 0.08 mol of allophanate groups, 0.21 mol of Fries rearrangement bodies and cyclized products of Fries rearrangement body in combination, and 0.04 mol of iminotrimer groups.

2. Recovery (regeneration) of amine compound

The following step (a1) and step (a2) (step (a2-1) and step (a2-2)) were performed using the device shown in FIG. 26.

(1) Step (a1): mixing step

50 kg of the liquid-phase component containing the high-boiling-point compound recovered through the line c33 and 50 kg of 4-(α,α-dimethylbenzyl)phenol were introduced into the stirring tank b201 through the line b20, and a reaction was carried out at 180°C for 0.5 hours to obtain a reaction solution.

(2) Step (a2): reaction step

(2-1) Step (a2-1): mixing step of composition-amine compound-catalyst

Through the line b20, 200 kg of 4,4'-methylenedianiline (ratio to the total molar amount of nitrogen atoms included in the groups (IX-1) to (IX-12): 3.1 times by mole) and 9 kg of sodium hydroxide (ratio to the total molar amount of nitrogen atoms included in the groups (IX-1) to (IX-12): 0.68 times by mole) were supplied to B201. The supplied mixed solution was reacted at 240°C for 1 hour to obtain a reaction solution.

As a result of analyzing the reaction solution by $^1$H-NMR, it was a mixture containing 7.18 mol of urea groups, 0.21 mol of Fries rearrangement groups and cyclized products of Fries rearrangement group, and 0.01 mol of isocyanurate groups per 1 kg of the liquid-phase component.

(2-2) Step (a2-2): reaction step of composition obtained in step (a2-1) and water

A reaction of the composition obtained in the step (a2-1) with water was carried out using the same method as in "Step (a2): reaction step" of Example 3-2, except that 300 kg of the composition mixed in the step (a2-1) and 200 kg of water (ratio to the total molar amount of nitrogen atoms included in bases (IX-1) to (IX-12): 34 times by mole) were used, and the reaction was carried out at 190°C and 1.3 MPa for 1.5 hours to obtain a reaction solution.

(3) Step (b): separation step of amine compound (IIa)

In a step (e) described later, the residual liquid obtained in a case where the hydroxy compound was recovered was distilled under reduced pressure to recover a crude 4,4'-methylenedianiline-containing liquid. The yield was 77% by mass.

(4) Step (c): purification step of amine compound (IIa)

The crude 4,4'-methylenedianiline-containing liquid recovered in the step (b) was distilled and purified. The purity of the recovered 4,4'-methylenedianiline was 99% by mass or more (including an analysis error of gas chromatography). In addition, the metal atom content with respect to the total mass of 4,4'-methylenedianiline was less than 1,000 ppm by mass, and the halogen atom content was less than 1,000 ppm by mass.

(5) Step (e): separation step of hydroxy compound

The reaction solution obtained in the step (a2-2) was distilled under reduced pressure to recover a crude 4-(α,α-dimethylbenzyl)phenol-containing liquid. The yield was 82% by mass except for the amount added in the step (a1).

(6) Step (f): purification step of hydroxy compound

The crude 4-(α,α-dimethylbenzyl)phenol-containing liquid recovered in the step (e) was distilled and purified. The purity of 4-(α,α-dimethylbenzyl) was 99% by mass or more (including an analysis error of gas chromatography). In addition, the metal atom content with respect to the total mass of 4-(α,α-dimethylbenzyl) was less than 1,000 ppm by mass, and the halogen atom content was less than 1,000 ppm by mass.

[Examples 3-8 to 3-42]

A liquid-phase component containing a high-boiling-point compound produced in the production of the isocyanate compound (VIIa) was obtained under the conditions shown in the tables below, and the amine compound (IIa) and the hydroxy compound were recovered using the liquid-phase component. The formulation of the liquid-phase component and the results after the recovery are also shown in the following tables.

In the tables, in the step (x1) to the step (x3-2) and the step (a1) to the step (a2) (the step (a2-1) and the step (a2-2)), the same operations as those in Examples described in the column of "Method" were performed to perform the steps.

In addition, in the conditions of the step (a1) and the step (a2), "Proportion (% by mass)" means a proportion (% by mass) of the mass of the added aromatic hydroxy compound to the mass of the liquid-phase component. "Ratio (times by mole)" indicates a molar ratio of each raw material with respect to the total molar amount of nitrogen atoms included in the groups (IX-1) to (IX-12).

In addition, the aromatic hydroxy compound added to the liquid-phase component is not included in the yield

of the hydroxy compound finally recovered. Similarly, the amine compound added to the liquid-phase component is not included in the yield of the amine compound (IIa) finally recovered.

In each of the following tables, the abbreviated compound indicates the following compound.

(Amine compound)

IIa-1: 2,4-toluenediamine
IIa-2: 4,4'-dicyclohexylmethanediamine
IIa-3: 4-aminomethyl-1,8-octanediamine
IIa-4: 1,6-hexamethylenediamine
IIa-5: 1-amino-3-aminomethyl-3,5,5-trimethylcyclohexane
IIa-6: 4,4',4"-methanetrianiline
IIa-7: L-lysine aminoethyl ester
IIa-8: benzene-1,3,5-triamine
IIa-9: cyclohexyl-1,3-5-triamine
IIa-10: 4,4'-methylenedianiline

(Hydroxy compound and aromatic hydroxy compound)

VIc-1-1a: 4-(1,1,3,3-tetramethylbutyl)phenol
VIc-1-1b: ethylphenol
VIc-1-1c: phenol
VIc-1-1d: 4-($\alpha$,$\alpha$-dimethylbenzyl)phenol
VIc-1-1e: tribenzylphenol
VIc-1-1f: cresol
VIc-1-1g: p-dodecylphenol
VIc-1-1h: 3-(3,4,6-tribenzylphenyl)phenol
VIc-2-1: ethanol
VIc-2-2: 1-butanol

[Table 3-1]

| | Step (x1) | | | | | Step (x1-a) | | | Step (x1-b) | | Step (x2) | | | Step (x3-1) | | Step (x3-2) | |
|---|---|---|---|---|---|---|---|---|---|---|---|---|---|---|---|---|---|
| | Amine compound | Carbonic acid derivative | Hydroxy compound | Device | Method | Hydroxy compound | Device | Method | Device | Method | Solvent | Device | Method | Device | Method | Device | Method |
| Example 3-8 | IIa-1 | Urea | VIc-1-1c | FIG. 23 | Example 3-2 | VIc-1-1a | FIG. 29 | Example 3-4 | - | - | Dibenzyl ether | FIG. 20 | Example 3-1 | - | - | - | - |
| Example 3-9 | IIa-2 | Diethyl carbonate | VIc-2-1 | FIG. 27 | Example 3-3 | - | - | - | - | - | Dibenzyl toluene | FIG. 25 | Example 3-2 | - | - | - | - |
| Example 3-10 | IIa-3 | Ethylphenyl carbamate | VIc-1-1b | FI G . 23 | Example 3-2 | - | - | - | FIG. 24 | Example 3-2 | Dibenzyl toluene | FIG. 25 | Example 3-2 | - | - | - | - |
| Example 3-11 | IIa-1 | Urea | VIc-1-1a | FIG. 20 | Example 3-1 | - | - | - | - | - | - | FIG. 30 | Example 3-4 | - | - | - | - |
| Example 3-12 | IIa-2 | Urea | VIc-1-1a | FIG. 23 | Example 3-2 | - | - | - | FIG. 24 | Example 3-2 | Dibenzyl ether | FIG. 36 | Example 3-7 | FIG. 33 | Example 3-5 | FIG. 34 | Example 3-5 |
| Example 3-13 | IIa-4 | Urea | VIc-1-1 c | FIG. 23 | Example 3-2 | VIc-1-1d | FIG. 29 | Example 3-4 | - | - | Benzyl toluene | FIG. 20 | Example 3-1 | - | - | - | - |
| Example 3-14 | IIa-5 | Diphenyl carbonate | VIc-1-1c | FIG. 27 | Example 3-3 | - | - | - | - | - | Naphthalene | FIG. 36 | Example 3-7 | FIG. 33 | Example 3-5 | FIG. 34 | Example 3-5 |
| Example 3-15 | IIa-3 | Diphenyl carbonate | VIc-1-1c | FIG. 27 | Example-3-3 | VIc-1-1e | FIG. 29 | Example 3-4 | - | - | Dibenzyl toluene | FIG. 20 | Example 3-1 | - | - | - | - |
| Example 3-16 | IIa-4 | Diphenyl carbonate | VIe-1-1c | FIG. 27 | Example 3-3 | - | - | - | - | - | Dibenzyl toluene | FIG. 25 | Example 3-2 | - | - | - | - |
| Example 3-17 | IIa-1 | Urea | VIc-1-1f | FIG. 23 | Example 3-2 | - | - | - | FIG. 24 | Example 3-2 | Benzyl toluene | FIG. 25 | Example 3-2 | - | - | - | - |

[Table 3-2]

| | Step (x1) | | | | | Step (x1-a) | | | Step (x1-b) | | Step (x2) | | | Step (x3-1) | | Step (x3-2) | |
|---|---|---|---|---|---|---|---|---|---|---|---|---|---|---|---|---|---|
| | Amine compound | Carbonic acid derivative | Hydroxy compound | Device | Method | Hydroxy compound | Device | Method | Device | Method | Solvent | Device | Method | Device | Method | Device | Method |
| Example 3-18 | IIa-2 | Butyl carbamate | VIc-2-2 | FIG. 23 | Example 3-2 | VIc-1-1e | FIG. 29 | Example 3-4 | - | - | Di benzyl toluene | FIG. 20 | Example 3-1 | - | - | - | - |
| Example 3-19 | IIa-1 | Urea | VIc-7-1f | FIG. 23 | Example 3-2 | - | - | - | - | - | Dodecane | FIG. 32 | Example 3-5 | FIG. 33 | Example 3-5 | FIG. 34 | Example 3-5 |
| Example 3-20 | IIa-1 | Urea | VIc-1-1f | FIG. 23 | Example 3-2 | VIc-1-1g | FIG. 29 | Example 3-4 | - | - | Dibenzyl ether | FIG. 20 | Example 3-1 | - | - | - | - |
| Example 3-21 | IIa-3 | Urea | VIc-1-1c | FIG. 23 | Example 3-2 | - | - | - | FIG. 24 | Example 3-2 | Diphenyl carbonate | FIG. 32 | Example 3-5 | FIG. 33 | Example 3-5 | FIG. 34 | Example 3-5 |
| Example 3-22 | IIa-4 | Ethylphenyl carbamate | VIc-1-1b | FIG. 23 | Example 3-2 | - | - | - | - | - | Tetralin | FIG. 36 | Example 3-7 | FIG. 33 | Example 3-5 | FIG. 34 | Example 3-5 |
| Example 3-23 | IIa-5 | Urea | VIc-2-2 | FIG. 20 | Example 3-1 | VIc-1-1d | FIG. 29 | Example 3-4 | - | - | Pentadecane | FIG. 20 | Example 3-1 | - | - | - | - |
| Example 3-24 | IIa-6 | Urea | VIc-2-2 | FIG. 23 | Example 3-2 | - | - | - | FIG. 24 | Example 3-2 | Toluene | FIG. 36 | Example 3-7 | FIG. 33 | Example 3-5 | FIG. 34 | Example 3-5 |
| Example 3-25 | IIa-7 | Diphenyl carbonate | VIc-1-1c | FIG. 27 | - | - | - | - | - | - | Diphenyl carbonate | FIG. 36 | Example 3-7 | FIG. 33 | Example 3-5 | FIG. 34 | Example 3-5 |
| Example 3-26 | IIa-8 | Urea | VIc-2-2 | FIG. 23 | Example 3-2 | - | - | - | FIG. 24 | Example 3-2 | Benzophenone | FIG. 25 | Example 3-2 | - | - | - | - |
| Example 3-27 | IIa-9 | Urea | VIc-1-1c | FIG. 23 | Example 3-2 | VIc-1-1h | FIG. 29 | Example 3-4 | - | - | Benzophenone | FIG. 20 | Example 3-1 | - | - | - | - |
| Example 3-28 | IIa-4 | Diphenyl carbonate | VIc-1-1c | FIG. 27 | Example 3-3 | VIc-1-1d | FIG. 29 | Example 3-4 | - | - | - | FIG. 20 | Example 3-1 | - | - | - | - |
| Example 3-29 | IIa-4 | Diphenyl carbonate | VIc-1-1c | FIG. 27 | Example 3-3 | VIc-1-1d | FIG. 29 | Example 3-4 | - | - | - | FIG. 20 | Example 3-1 | - | - | - | - |
| Example 3-30 | IIa-4 | Diphenyl carbonate | VIc-1-1c | FIG. 27 | Example 3-3 | VIc-1-1d | FIG. 29 | Example 3-4 | - | - | - | FIG. 20 | Example 3-1 | - | - | - | - |

[Table 3-3]

| | Step (x1) | | | | | Step (x1-a) | | | Step (x1-b) | | Step (x2) | | | Step (x3-1) | | Step (x3-2) | |
|---|---|---|---|---|---|---|---|---|---|---|---|---|---|---|---|---|---|
| | Amine compound | Carbonic acid derivative | Hydroxy compound | Device | Method | Hydroxy compound | Device | Method | Device | Method | Solvent | Device | Method | Device | Method | Device | Method |
| Example 3-31 | IIa-1 | Urea | VIc-1-1a | FIG. 20 | Example 3-1 | - | - | - | - | - | Pentadecane | FIG. 20 | Example 3-1 | - | - | - | - |
| Example 3-32 | IIa-1 | Urea | VIc-1-1a | FIG. 20 | Example 3-1 | - | - | - | - | - | Pentadecane | FIG. 20 | Example 3-1 | - | - | - | - |
| Example 3-33 | IIa-2 | Diphenyl carbonate | VIc-1-1c | FIG. 27 | Example 3-3 | - | - | - | - | - | Dibenzyl ether | FIG. 25 | Example 3-2 | - | - | - | - |
| Example 3-34 | IIa-2 | Diphenyl carbonate | VIc-1-1c | FIG. 27 | Example 3-3 | - | - | - | - | - | Dibenzyl ether | FIG. 25 | Example 3-2 | - | - | - | - |
| Example 3-35 | IIa-5 | Urea | VIc-2-2 | FIG. 23 | Example 3-2 | - | - | - | FIG. 24 | Example 3-2 | Dibenzyl ether | FIG. 25 | Example 3-2 | - | - | - | - |
| Example 3-36 | IIa-5 | Urea | VIc-2-2 | FIG. 23 | Example 3-2 | - | - | - | FIG. 24 | Example 3-2 | Dibenzyl ether | FIG. 25 | Example 3-2 | - | - | - | - |
| Example 3-37 | IIa-5 | Urea | VIc-2-2 | FIG. 23 | Example 3-2 | - | - | - | FIG. 24 | Example 3-2 | Dibenzyl ether | FIG. 25 | Example 3-2 | - | - | - | - |
| Example 3-38 | IIa-3 | Urea | VIc-1-1c | FIG. 23 | Example 3-2 | - | - | - | FIG. 24 | Example 3-2 | Benzophenone | FIG. 32 | Example 3-5 | FIG. 33 | Example 3-5 | FIG. 34 | Example 3-5 |
| Example 3-39 | IIa-3 | Urea | VIc-1-1c | FIG. 23 | Example 3-2 | - | - | - | FIG. 24 | Example 3-2 | Benzophenone | FIG. 32 | Example 3-5 | FIG. 33 | Example 3-5 | FIG. 34 | Example 3-5 |
| Example 3-40 | IIa-1 | Urea | VIc-1-1c | FIG. 23 | Example 3-2 | VIc-1-1a | FIG. 29 | Example 3-4 | - | - | Dibenzyl ether | FIG. 20 | Example 3-1 | - | - | - | - |
| Example 3-41 | IIa-1 | Urea | VIc-1-1c | FIG. 23 | Example 3-2 | VIc-1-1a | FIG. 29 | Example 3-4 | - | - | Dibenzyl ether | FIG. 20 | Example 3-1 | - | - | - | - |
| Example 3-42 | IIa-6 | Urea | VIc-1-1d | FIG. 23 | Example 3-2 | - | - | - | - | - | Dibenzyltoluene | FIG. 36 | Example 3-7 | FIG. 33 | Example 3-5 | FIG. 34 | Example 3-5 |

[Table 3-4]

| | Formulation of liquid-phase component | | | | | | | | | | |
|---|---|---|---|---|---|---|---|---|---|---|---|
| | Amount of isocyanate group (mol/kg) | Amount of carbamate group (mol/kg) | Total amount of Fries rearrangement body + cyclized product of Fries rearrangement body (mol/kg) | Total amount of urea group + carbodiimide group (mol/kg) | Amount of allophanate group (mol/kg) | Total amount of biuret group + isocyanurate group + uretonimine group (mol/kg) | Amount of oxycarbonyl carbamate group (mol/kg) | Amount of iminotrimer group (mol/kg) | Amont of aromatic hydroxy compound (mol/kg) | Molar amount of nitrogen atoms in groups (IX-1) to (IX-12) (mol/kg) | Use amount (kg) |
| Example 3-8 | 0.00 | 1.45 | 0.04 | 0.04 | 0.02 | 0.06 | 0.00 | 0.00 | 1.88 | 1.81 | 50 |
| Example 3-9 | 0.00 | 2.62 | 0.00 | 0.01 | 0.39 | 0.40 | 0.00 | 0.00 | 0.00 | 4.64 | 50 |
| Example 3-10 | 0.00 | 2.44 | 0.14 | 0.31 | 0.10 | 0.41 | 0.00 | 0.00 | 0.00 | 4.64 | 50 |
| Example 3-11 | 0.00 | 2.10 | 0.04 | 0.28 | 0.01 | 0.29 | 0.00 | 0.00 | 1.10 | 3.57 | 50 |
| Example 3-12 | 1.23 | 0.02 | 0.21 | 0.20 | 0.01 | 1.35 | 0.00 | 0.02 | 0.00 | 6.00 | 50 |
| Example 3-13 | 0.00 | 1.56 | 0.02 | 0.10 | 0.24 | 0.34 | 0.00 | 0.00 | 1.28 | 3.26 | 50 |
| Example 3-14 | 1.56 | 0.16 | 0.46 | 0.01 | 0.45 | 1.34 | 0.00 | 0.00 | 0.00 | 7.19 | 50 |
| Example 3-15 | 0.00 | 1.31 | 0.07 | 0.13 | 0.11 | 0.23 | 0.00 | 0.02 | 0.45 | 2.56 | 50 |
| Example 3-16 | 0.00 | 2.51 | 0.02 | 0.09 | 0.29 | 0.37 | 0.00 | 0.00 | 0.00 | 4.39 | 50 |
| Example 3-17 | 0.00 | 2.60 | 0.06 | 0.24 | 0.21 | 0.45 | 0.00 | 0.00 | 0.00 | 4.93 | 50 |
| Example 3-18 | 0.00 | 0.63 | 0.00 | 0.12 | 0.19 | 0.24 | 0.00 | 0.00 | 0.42 | 1.96 | 50 |

(continued)

| | Formulation of liquid-phase component | | | | | | | | | | |
|---|---|---|---|---|---|---|---|---|---|---|---|
| | Amount of isocyanate group (mol/kg) | Amount of carbamate group (mol/kg) | Total amount of Fries rearrangement body + cyclized product of Fries rearrangement body (mol/kg) | Total amount of urea group + carbodiimide group (mol/kg) | Amount of allophanate group (mol/kg) | Total amount of biuret group + isocyanurate group + uretonimine group (mol/kg) | Amount of oxycarbonyl carbamate group (mol/kg) | Amount of iminotrimer group (mol/kg) | Amont of aromatic hydroxy compound (mol/kg) | Molar amount of nitrogen atoms in groups (IX-1) to (IX-12) (mol/kg) | Use amount (kg) |
| Example 3-19 | 4.54 | 0.05 | 0.14 | 0.04 | 0.10 | 0.70 | 0.00 | 0.02 | 0.00 | 7.19 | 50 |
| Example 3-20 | 0.00 | 1.39 | 0.09 | 0.15 | 0.03 | 0.18 | 0.00 | 0.00 | 1.10 | 2.37 | 50 |
| Example 3-21 | 2.77 | 0.02 | 0.20 | 0.24 | 0.44 | 1.78 | 0.02 | 0.02 | 0.00 | 9.81 | 50 |
| Example 3-22 | 3.83 | 0.02 | 0.44 | 0.24 | 0.20 | 0.90 | 0.00 | 0.02 | 0.00 | 7.97 | 50 |
| Example 3-23 | 0.00 | 1.79 | 0.05 | 0.01 | 0.15 | 0.15 | 0.00 | 0.00 | 1.03 | 2.61 | 50 |
| Example 3-24 | 2.57 | 0.09 | 0.00 | 0.03 | 0.78 | 1.05 | 0.00 | 0.03 | 0.00 | 7.54 | 50 |
| Example 3-25 | 5.45 | 0.01 | 0.33 | 0.01 | 0.07 | 1.37 | 0.02 | 0.02 | 0.00 | 10.17 | 50 |
| Example 3-26 | 0.00 | 2.58 | 0.00 | 0.05 | 0.24 | 0.39 | 0.00 | 0.00 | 0.00 | 4.32 | 50 |
| Example 3-27 | 0.00 | 1.03 | 0.05 | 0.11 | 0.13 | 0.29 | 0.00 | 0.00 | 0.66 | 2.41 | 50 |
| Example 3-28 | 0.00 | 1.55 | 0.02 | 0.10 | 0.24 | 0.34 | 0.01 | 0.01 | 1.02 | 3.30 | 50 |
| Example 3-29 | 0.00 | 1.55 | 0.02 | 0.10 | 0.24 | 0.34 | 0.01 | 0.01 | 1.02 | 3.30 | 50 |

(continued)

| | Formulation of liquid-phase component | | | | | | | | | | |
|---|---|---|---|---|---|---|---|---|---|---|---|
| | Amount of isocyanate group (mol/kg) | Amount of carbamate group (mol/kg) | Total amount of Fries rearrangement body + cyclized product of Fries rearrangement body (mol/kg) | Total amount of urea group + carbodiimide group (mol/kg) | Amount of allophanate group (mol/kg) | Total amount of biuret group + isocyanurate group + uretonimine group (mol/kg) | Amount of oxycarbonyl carbamate group (mol/kg) | Amount of iminotrimer group (mol/kg) | Amont of aromatic hydroxy compound (mol/kg) | Molar amount of nitrogen atoms in groups (IX-1) to (IX-12) (mol/kg) | Use amount (kg) |
| Example 3-30 | 0.00 | 1.55 | 0.02 | 0.10 | 0.24 | 0.34 | 0.01 | 0.01 | 1.02 | 3.30 | 50 |

[Table 3-5]

| | Formulation of liquid-phase component | | | | | | | | | | |
|---|---|---|---|---|---|---|---|---|---|---|---|
| | Amount of isocyanate group (mol/kg) | Amount of carbamate group (mol/kg) | Total amount of Fries rearrangement body + cyclized product of Fries rearrangement body (mol/kg) | Total amount of urea group + carbodiimide group (mol/kg) | Amount of allophanate group (mol/kg) | Total amount of biuret group + isocyanurate group + uretonimine group (mol/kg) | Amount of oxycarbonyl carbamate group (mol/kg) | Amount of iminotrimer group (mol/kg) | Amont of aromatic hydroxy compound (mol/kg) | Molar amount of nitrogen atoms in groups (IX-1) to (IX-12) (mol/kg) | Use amount (kg) |
| Example 3-31 | 0.00 | 0.98 | 0.22 | 0.07 | 0.06 | 0.13 | 0.00 | 0.00 | 2.82 | 1.84 | 50 |
| Example 3-32 | 0.00 | 0.98 | 0.22 | 0.07 | 0.06 | 0.13 | 0.00 | 0.00 | 2.82 | 1.84 | 50 |
| Example 3-33 | 0.00 | 2.57 | 0.00 | 0.09 | 0.31 | 0.40 | 0.02 | 0.02 | 0.00 | 4.65 | 50 |
| Example 3-34 | 0.00 | 2.57 | 0.00 | 0.09 | 0.31 | 0.40 | 0.02 | 0.02 | 0.00 | 4.65 | 50 |
| Example 3-35 | 0.00 | 2.56 | 0.00 | 0.07 | 0.05 | 0.12 | 0.00 | 0.00 | 0.00 | 3.16 | 50 |
| Example 3-36 | 0.00 | 2.56 | 0.00 | 0.07 | 0.05 | 0.12 | 0.00 | 0.00 | 0.00 | 3.16 | 50 |
| Example 3-37 | 0.00 | 2.56 | 0.00 | 0.07 | 0.05 | 0.12 | 0.00 | 0.00 | 0.00 | 3.16 | 50 |
| Example 3-38 | 4.53 | 0.01 | 1.35 | 0.01 | 0.05 | 1.40 | 0.00 | 0.05 | 0.00 | 10.41 | 50 |
| Example 3-39 | 4.53 | 0.01 | 1.35 | 0.01 | 0.05 | 1.40 | 0.00 | 0.05 | 0.00 | 10.41 | 50 |
| Example 3-40 | 0.00 | 1.45 | 0.04 | 0.04 | 0.02 | 0.06 | 0.00 | 0.00 | 1.88 | 1.81 | 50 |
| Example 3-41 | 0.00 | 1.45 | 0.04 | 0.04 | 0.02 | 0.06 | 0.00 | 0.00 | 1.88 | 1.81 | 50 |

(continued)

| | Formulation of liquid-phase component | | | | | | | | | | |
|---|---|---|---|---|---|---|---|---|---|---|---|
| | Amount of isocyanate group (mol/kg) | Amount of carbamate group (mol/kg) | Total amount of Fries rearrangement body + cyclized product of Fries rearrangement body (mol/kg) | Total amount of urea group + carbodiimide group (mol/kg) | Amount of allophanate group (mol/kg) | Total amount of biuret group + isocyanurate group + uretonimine group (mol/kg) | Amount of oxycarbonyl carbamate group (mol/kg) | Amount of iminotrimer group (mol/kg) | Amont of aromatic hydroxy compound (mol/kg) | Molar amount of nitrogen atoms in groups (IX-1) to (IX-12) (mol/kg) | Use amount (kg) |
| Example 3-42 | 1.82 | 0.01 | 0.21 | 0.02 | 0.08 | 1.40 | 0.00 | 0.04 | 0.00 | 6.60 | 50 |

[Table 3-6]

| | Step (a1): mixing step + step (a2): reaction step | | | | | | | | | | | | | |
|---|---|---|---|---|---|---|---|---|---|---|---|---|---|---|
| | Step (a1) | | | | | Step (a2) | | | | | | | | |
| | Device | Method | Aromatic hydroxy compound | | | Active hydrogen compound | | | Catalyst | | | Temperature (°C) | Pressure (MPa) | Time (hr) |
| | | | Type | Amount (kg) | Proportion(% by mass) | Type | Amount (kg) | Ratio (time by mole) | Type | Amount (kg) | Ratio (time by mole) | | | |
| Example 3-8 | FIG. 26 | Example 3-2 | VIc-1-1a | 50 | 100 | Water | 50 | 31 | NaOH | 1.2 | 0.33 | 200 | 1.5 | 2 |
| Example 3-9 | FIG. 26 | Example 3-2 | VIc-1-1c | 150 | 300 | Water/II-2 | 100/50 | 24/1.0 | $Ca(OH)_2$ | 3.4 | 0.20 | 220 | 2.4 | 2 |
| Example 3-12 | FIG. 26 | Example 3-2 | VIc-1-1a | 25 | 50 | Water | 100 | 19 | $Na_2CO_3$ | 4.4 | 0.14 | 270 | 5.8 | 2 |
| Example 3-13 | FIG. 28 | Example 3-3 | VIc-1-1d | 50 | 100 | Water/II-4 | 100/50 | 34/2.6 | NaOH | 0.9 | 0.14 | 250 | 4.1 | 3 |
| Example 3-14 | FIG. 28 | Example 3-3 | VIc-1-1c | 50 | 100 | Water | 100 | 15 | $Ca(OH)_2$ | 2.5 | 0.09 | 270 | 5.8 | 1.5 |
| Example 3-15 | FIG. 31 | Example 3-4 | VIc-1-1e | 50 | 100 | Water | 100 | 43 | KOH | 2.3 | 0.32 | 220 | 2.5 | 2.5 |
| Example 3-16 | FIG. 28 | Example 3-3 | VIc-1-1c | 50 | 100 | Water | 100 | 25 | ZnOTs | 3.0 | 0.06 | 280 | 6.9 | 1 |
| Example 3-17 | FIG. 26 | Example 3-2 | VIc-1-1f | 50 | 100 | Water | 100 | 23 | KOH | 3.0 | 0.22 | 190 | 1.3 | 4 |
| Example 3-18 | FIG. 35 | Example 3-6 | VIc-1-1e | 50 | 100 | Water | 50 | 28 | $Na_2CO_2$ | 0.3 | 0.03 | 230 | 2.8 | 1 |
| Example 3-19 | FIG. 28 | Example 3-3 | VIe-1-1f | 50 | 100 | Water/II-1 | 100/50 | 15/1.1 | NaOH | 2.4 | 0.17 | 190 | 1.3 | 5 |
| Example 3-20 | FIG. 28 | Example 3-3 | VIc-1-1g | 50 | 100 | Water | 100 | 47 | $Ca(OH)_2$ | 2.1 | 0.24 | 270 | 5.8 | 2 |
| Example 3-21 | FIG. 26 | Example 3-2 | VIc-1-1c | 50 | 100 | Water | 100 | 11 | NaOH | 0.9 | 0.05 | 210 | 1.8 | 2 |

(continued)

| | Step (a1): mixing step + step (a2): reaction step | | | | | | | | | | | | | |
|---|---|---|---|---|---|---|---|---|---|---|---|---|---|---|
| | Step (a1) | | | | | Step (a2) | | | | | | | | |
| | Device | Method | Aromatic hydroxy compound | | | Active hydrogen compound | | | Catalyst | | | Temperature (°C) | Pressure (MPa) | Time (hr) |
| | | | Type | Amount (kg) | Proportion(% by mass) | Type | Amount (kg) | Ratio (time by mole) | Type | Amount (kg) | Ratio (time by mole) | | | |
| Example 3-22 | FIG. 28 | Example 3-3 | VIc-1-1b | 50 | 100 | Water | 100 | 14 | $Ca(OH)_2$ | 0.4 | 0.01 | 220 | 2.5 | 2 |
| Example 3-23 | FIG. 26 | Example 3-2 | VIc-1-1d | 50 | 100 | Water | 100 | 43 | KOH | 0.3 | 0.04 | 280 | 6.9 | 1 |
| Example 3-24 | FIG. 26 | Example 3-2 | VIc-1-1i | 75 | 150 | Water | 125 | 18 | $Na_2CO_3$ | 4.4 | 0.11 | 270 | 5.8 | 2 |
| Example 3-25 | FIG. 28 | Example 3-3 | VIc-1-1c | 75 | 150 | Water | 125 | 14 | $Ca(OH)_2$ | 2.5 | 0.07 | 270 | 5.8 | 1.5 |
| Example 3-26 | FIG. 26 | Example 3-2 | VIc-1-1c | 50 | 100 | Water | 100 | 26 | NaOH | 3.0 | 0.35 | 250 | 4.1 | 5 |
| Example 3-27 | FIG. 28 | Example 3-3 | VIc-1-1h | 40 | 80 | Water | 80 | 37 | KOH | 2.1 | 0.31 | 270 | 5.8 | 2 |
| Example 3-28 | FIG. 31 | Example 3-4 | VIc-1-1d | 50 | 100 | Water | 100 | 34 | $Cs_2CO_3$ | 2.0 | 0.04 | 250 | 4.1 | 2 |
| Example 3-29 | FIG. 31 | Example 3-4 | VIc-1-1d | 50 | 100 | Water | 100 | 34 | $Cs_2CO_3$ | 2.0 | 0.04 | 220 | 2.4 | 2 |
| Example 3-30 | FIG. 31 | Example 3-4 | VIc-1-1d | 50 | 100 | Water | 100 | 34 | $Cs_2CO_3$ | 2.0 | 0.04 | 190 | 1.3 | 2 |

[Table 3-7]

| | Step (a1): mixing step + step (a2): reaction step | | | | | | | | | | | | | |
|---|---|---|---|---|---|---|---|---|---|---|---|---|---|---|
| | Step (a1) | | | | | Step (a2) | | | | | | | | |
| | Device | Method | Aromatic hydroxy compound | | | Active hydrogen compound | | | Catalyst | | | Temperature (°C) | Pressure (MPa) | Time (hr) |
| | | | Type | Amount (kg) | Proportion (% by mass) | Type | Amount (kg) | Ratio (time by mole) | Type | Amount (kg) | Ratio (time by mole) | | | |
| Example 3-31 | FIG. 20 | Example 3-1 | VIc-1-1a | 50 | 100 | Water | 50 | 30 | NaOH | 1.5 | 0.41 | 280 | 6.6 | 3 |
| Example 3-32 | FIG. 20 | Example 3-1 | VIc-1-1a | 50 | 100 | Water | 50 | 30 | NaOH | 1.5 | 0.41 | 280 | 6.6 | 1 |
| Example 3-33 | FIG. 28 | Example 3-3 | VIc-1-1c | 25 | 50 | Water | 100 | 24 | KOH | 9.0 | 0.71 | 250 | 4.1 | 3 |
| Example 3-34 | FIG. 28 | Example 3-3 | VIc-1-1c | 25 | 50 | Water | 100 | 24 | KOH | 27.0 | 2.12 | 250 | 4.1 | 3 |
| Example 3-35 | FIG. 26 | Example 3-2 | VIc-1-1c | 50 | 100 | Water/II-5 | 100/50 | 35/1.9 | NaOH | 3.0 | 0.47 | 250 | 4.1 | 5 |
| Example 3-36 | FIG. 26 | Example 3-2 | VIc-1-1c | 50 | 100 | Water/II-5 | 100/50 | 35/1.9 | ZnO | 3.0 | 0.23 | 250 | 4.1 | 5 |
| Example 3-37 | FIG. 26 | Example 3-2 | VIe-1-1c | 50 | 100 | Water/I I-5 | 100/50 | 35/1.9 | Triethylamine | 3.0 | 0.19 | 250 | 4.1 | 5 |
| Example 3-38 | FIG. 26 | Example 3-2 | VIc-1-1c | 50 | 100 | Water | 50 | 5 | Triethylamine | 6.0 | 0.11 | 240 | 3.4 | 3 |
| Example 3-39 | FIG. 26 | Example 3-2 | VIc-1-1c | 50 | 100 | Water | 200 | 21 | Triethylamine | 6.0 | 0.11 | 240 | 3.4 | 3 |
| Example 3-40 | FIG. 26 | Example 3-2 | VIc-1-1a | 15 | 30 | Water | 50 | 31 | NaOH | 1.2 | 0.33 | 200 | 1.5 | 2 |
| Example 3-41 | FIG. 26 | Example 3-2 | VIc-1-1a | 150 | 300 | Water | 50 | 31 | NaOH | 1.2 | 0.33 | 200 | 1.5 | 2 |

[Table 3-8]

| | Step (a1): mixing step + step (a2): reaction step | | | | | | | | | | | | | | | | | | | | | | | |
|---|---|---|---|---|---|---|---|---|---|---|---|---|---|---|---|---|---|---|---|---|---|---|---|---|
| | Step (a1) | | | | | Step (a2-1) | | | | | | | | | | | Step (a2-2) | | | | | | | |
| | Device | Method | Aromatic hydroxy compound | | | Device | Method | Active hydrogen compound | | | Catalyst | | | Temperature (°C) | Pressure (MPa) | Time (hr) | Device | Method | Active hydrogen compound | | | Temperature (°C) | Pressure (MPa) | Time (hr) |
| | | | Type | Amount (kg) | Proportion (% by mass) | | | Type | Amount (kg) | Ratio (time by mole) | Type | Amount (kg) | Ratio (time by mole) | | | | | | Type | Amount (kg) | Ratio (time by mole) | | | |
| Ex. 3-10 | FIG. 26 | Ex. 3-2 | VIc-1-1b | 25 | 50 | FIG. 21 | Ex. 3-1 | IIa-3 | 400 | 10.0 | KOH | 0.3 | 0.02 | 240 | 0.1 | 1 | FIG. 28 | Ex. 3-3 | Water | 150 | 36 | 210 | 1.8 | 1.5 |
| Ex. 3-11 | FIG. 26 | Ex. 3-2 | VIc-1-1a | 25 | 50 | FIG. 21 | Ex. 3-1 | IIa-3 | 150 | 6.9 | NaOH | 0.2 | 0.03 | 240 | 0.1 | 1 | FIG. 28 | Ex. 3-3 | Water | 150 | 47 | 210 | 1.8 | 1.5 |
| Ex. 3-42 | FIG. 26 | Ex. 3-2 | VIc-1-1d | 50 | 100 | FIG. 21 | Ex. 3-1 | IIa-10 | 200 | 3.1 | NaOH | 9.0 | 0.68 | 240 | 0 | 1 | FIG. 28 | Ex. 3-2 | Water | 100 | 17 | 190 | 1.3 | 1.5 |

[Table 3-9]

| | Step (b) | | Step (c) | Recovered product | | | | | |
|---|---|---|---|---|---|---|---|---|---|
| | | | | Amine compound (IIa) | | | Hydroxy compound | | |
| | Device 1 | Device 2 | Device | Type | Amount (kg) | Yield (% by mass) | Type | Amount (kg) | Yield (% by mass) |
| Example 3-8 | Liquid-liquid phase separation tank | Distillation column | Distillation column | IIa-1 | 4.4 | 78.5 | VIc-1-1a | 12.9 | 82.4 |
| Example 3-9 | Liquid-liquid phase separation tank | Distillation column | Distillation column | IIa-2 | 16.7 | 68.3 | VIc-2-1 | 5.9 | 84.5 |
| Example 3-10 | Liquid-liquid phase separation tank | - | Distillation column | IIa-3 | 11.7 | 87.2 | VIc-1-1b | 14.1 | 86.0 |
| Example 3-11 | Liquid-liquid phase separation tank | Distillation column | Distillation column | IIa-1 | 10.5 | 96.0 | VIc-1-1a | 20.1 | 90.7 |
| Example 3-12 | Distillation column | - | Distillation column | IIa-2 | 24.9 | 78.9 | VIc-1-1a | 2.3 | 93.4 |
| Example 3-13 | Liquid-liquid phase separation tank | Distillation column | Distillation column | IIa-4 | 9.1 | 95.8 | VIc-1-1d | 18.0 | 92.9 |
| Example 3-14 | Liquid-liquid phase separation tank | Distillation column | Distillation column | IIa-5 | 28.0 | 91.4 | VIc-1-1c | 4.1 | 81.1 |
| Example 3-15 | Distillation column | - | Distillation column | IIa-3 | 6.2 | 83.8 | VIc-1-1e | 23.3 | 85.7 |
| Example 3-16 | Liquid-liquid phase separation tank | Distillation column | Distillation column | IIa-4 | 7.8 | 60.9 | VIc-1-1c | 8.0 | 60.7 |

(continued)

| | Step (b) | | Step (c) | Recovered product | | | | | |
|---|---|---|---|---|---|---|---|---|---|
| | | | | Amine compound (IIa) | | | Hydroxy compound | | |
| | Device 1 | Device 2 | Device | Type | Amount (kg) | Yield (% by mass) | Type | Amount (kg) | Yield (% by mass) |
| Example 3-17 | Liquid-liquid phase separation tank | Distillation column | Distillation column | IIa-1 | 12.8 | 85.2 | VIc-1-1f | 10.4 | 67.0 |
| Example 3-18 | Liquid-liquid phase separation tank | Distillation column | Distillation column | IIa-2 | 9.0 | 87.4 | VIc-1-1e | 9.5 | 63.7 |
| Example 3-19 | Liquid-liquid phase separation tank | Distillation column | Distillation column | IIa-1 | 16.5 | 75.3 | VIc-1-1f | 1.2 | 75.0 |
| Example 3-20 | Liquid-liquid phase separation tank | Distillation column | Distillation column | IIa-1 | 6.3 | 87.4 | VIc-1-1g | 13.3 | 66.9 |
| Example 3-21 | Liquid-liquid phase separation tank | Distillation column | Distillation column | IIa-3 | 22.1 | 78.1 | VIc-1-1c | 2.4 | 71.0 |
| Example 3-22 | Liquid-liquid phase separation tank | Distillation column | Distillation column | IIa-4 | 18.1 | 78.3 | VIc-1-1b | 2.2 | 52.5 |
| Example 3-23 | Liquid-liquid phase separation tank | Distillation column | Distillation column | IIa-5 | 10.0 | 90.1 | VIc-1-1d | 9.8 | 46.3 |
| Example 3-24 | Distillation column | - | Distillation column | IIa-6 | 22.1 | 60.8 | VIc-2-2 | 2.4 | 74.1 |
| Example 3-25 | Liquid-liquid phase separation tank | Distillation column | Distillation column | IIa-7 | 22.1 | 69.0 | VIc-1-1c | 1.6 | 74.0 |
| Example 3-26 | Distillation column | - | Distillation column | IIa-8 | 8.1 | 91.4 | VIc-2-2 | 7.7 | 73.8 |
| Example 3-27 | Liquid-liquid phase separation tank | Distillation column | Distillation column | IIa-9 | 4.3 | 83.5 | VIc-1-1h | 16.7 | 63.1 |
| Example 3-28 | Distillation column | - | Distillation column | IIa-4 | 7.5 | 78.9 | VIc-1-1d | 16.1 | 82.8 |
| Example 3-29 | Distillation column | - | Distillation column | IIa-4 | 8.0 | 84.0 | VIc-1-1d | 17.2 | 88.6 |

(continued)

| | Step (b) | | Step (c) | Recovered product | | | | | |
|---|---|---|---|---|---|---|---|---|---|
| | | | | Amine compound (IIa) | | | Hydroxy compound | | |
| | Device 1 | Device 2 | Device | Type | Amount (kg) | Yield (% by mass) | Type | Amount (kg) | Yield (% by mass) |
| Example 3-30 | Distillation column | - | Distillation column | IIa-4 | 8.6 | 89.5 | VIc-1-1d | 18.6 | 95.9 |

[Table 3-10]

| | Step (b) | | Step (c) | Recovered product | | | | | |
|---|---|---|---|---|---|---|---|---|---|
| | | | | Amine compound (IIa) | | | Hydroxy compound | | |
| | Device 1 | Device 2 | Device | Type | Amount (kg) | Yield (% by mass) | Type | Amount (kg) | Yield (% by mass) |
| Example 3-31 | Liquid-liquid phase separation tank | Distillation column | Distillation column | IIa-1 | 5.1 | 89.9 | VIc-1-1a | 11.2 | 86.4 |
| Example 3-32 | Liquid-liquid phase separation tank | Distillation column | Distillation column | IIa-1 | 5.0 | 89.1 | VIc-1-1a | 11.1 | 85.4 |
| Example 3-33 | Distillation column | - | Distillation column | IIa-2 | 21.6 | 88.2 | VIc-1-1c | 12.6 | 91.4 |
| Example 3-34 | Distillation column | - | Distillation column | IIa-2 | 22.1 | 90.3 | VIc-1-1c | 13.3 | 97.1 |
| Example 3-35 | Distillation column | - | Distillation column | IIa-5 | 12.9 | 95.9 | VIc-2-2 | 7.9 | 81.9 |
| Example 3-36 | Distillation column | - | Distillation column | IIa-5 | 11.0 | 81.9 | VIc-2-2 | 7.5 | 77.5 |
| Example 3-37 | Distillation column | - | Distillation column | IIa-5 | 10.5 | 78.0 | VIc-2-2 | 7.6 | 78.8 |
| Example 3-38 | Distillation column | - | Distillation column | IIa-3 | 25.5 | 85.0 | VIc-1-1c | 5.4 | 80.7 |
| Example 3-39 | Distillation column | - | Distillation column | IIa-3 | 26.0 | 86.7 | VIc-1-1c | 5.7 | 85.9 |
| Example 3-40 | Liquid-liquid phase separation tank | Distillation column | Distillation column | IIa-1 | 4.0 | 72.9 | VIc-1-1a | 12.5 | 80.0 |
| Example 3-41 | Liquid-liquid phase separation tank | Distillation column | Distillation column | IIa-1 | 4.4 | 79.4 | VIc-1-1a | 12.9 | 82.8 |
| Example 3-42 | Distillation column | - | Distillation column | IIa-6 | 24.0 | 73.4 | VIc-1-1d | 2.6 | 80.2 |

[Comparative Example 3-1]

The same method as in Example 3-1 was performed, except that a catalyst was not used in the step corresponding to the step (a2) of Example 3-1, and a crude 2,4-toluenediamine-containing liquid was recovered. The yield thereof was 40% by mass. In addition, the crude 4-(1,1,3,3-tetramethylbutyl)phenol-containing liquid was recovered. The yield thereof was 39% by mass. A fixed material was generated in the distillation column after the component was distilled and separated.

[Comparative Example 3-2]

The same method as in Example 3-24 was performed, except that a catalyst was not used in the step corresponding to the step (a2) of Example 3-24. At the initial stage of the reaction, the reactor b201 was clogged, and the reactant could not be pushed out. The reaction was continued, and in a case where the reaction was further carried out for 5 hours, the clogging was eliminated, but the reaction solution was recovered as a slurry-like liquid layer component. A crude 1,6-hexamethylenediamine-containing liquid was recovered. The yield thereof was 14% by mass. In addition, the crude 1-butanol-containing liquid was recovered. The yield thereof was 31% by mass.

[Comparative Example 3-3]

The same method as in Example 3-13 was performed, except that a catalyst was not used in the step corresponding to the step (a2) of Example 3-13. At the initial stage of the reaction, the stirring tank b301 and the filled column b302 were clogged, and the reactant could not be pushed out. The reaction was continued, and in a case where the reaction was further carried out for 5 hours, the clogging was eliminated, but the reaction solution was recovered as a liquid layer component. A crude 1,6-hexamethylenediamine-containing liquid was recovered. The yield thereof was 27% by mass. In addition, a crude 4-($\alpha$,$\alpha$-dimethylbenzyl)phenol-containing liquid was recovered. The yield thereof was 47% by mass.

[Comparative Example 3-4]

The same method as in Example 8 was performed, except that the aromatic hydroxy compound was not used in the step corresponding to the step (a1) of Example 3-8, but the reactor b201 was clogged and the reactant could not be pushed out.

The reaction was continued, and in a case where the reaction was further carried out for 3 hours, the clogging was eliminated, but the reaction solution was recovered as a liquid layer component. A subsequent liquid-liquid phase separation step was attempted to be performed, but the liquid layer components were not separated into two layers. Therefore, the liquid layer components were discharged as they were into the distillation column without performing the liquid-liquid phase separation step, and the distillation separation was carried out. An attempt was made to separate the crude 2,4-toluenediamine-containing liquid and the crude 4-(1,1,3,3-tetramethylbutyl)phenol-containing liquid in the distillation column, but a fixed material was generated in the distillation column and could not be removed, so that the operation was interrupted. The yield of 2,4-toluenediamine which could be recovered was 35% by mass. In addition, the yield of the 4-(1,1,3,3-tetramethylbutyl)phenol which could be recovered was 36% by mass.

[Comparative Example 3-5]

The same method as in Example 3-5 was performed, except that water was not used in the step corresponding to the step (a2) of Example 3-5. An attempt was made to separate the crude 4-aminomethyl-1,8-octanediamine-containing liquid and the crude phenol-containing liquid in the distillation column, but a fixed material was generated in the distillation column and could not be removed, so that the operation was interrupted.

[Comparative Example 3-6]

The same method as in Example 3-2 was performed, except that 1-butanol was used instead of the aromatic hydroxy compound in the step corresponding to the step (a1) of Example 3-2.

An attempt was made to separate the crude 1-amino-3-aminomethyl-3,5,5-trimethylcyclohexane-containing liquid and the crude 1-butanol-containing liquid in the distillation column, but a fixed material was generated in the distillation column and could not be removed, so that the operation was interrupted. The yield of 1-amino-3-aminomethyl-3,5,5-trimethylcyclohexane which could be recovered was 47% by mass. In addition, the yield of 1-butanol which could be recovered was 33% by mass.

[Comparative Example 3-7]

The same method as in Examples 3-7 was performed, except that the step corresponding to the step (a1) of Example 3-7 was not performed and the aromatic hydroxy compound was not used. An attempt was made to separate the crude 4,4'-methylenedianiline-containing liquid and the crude 4-(α,α-dimethylbenzyl)phenol-containing liquid in the distillation column, but a fixed material was generated in the distillation column and could not be removed, so that the operation was interrupted. The yield of 4,4'-methylenedianiline which could be partially recovered was 38% by mass. In addition, the yield of 4-(α,α-dimethylbenzyl)phenol which could be recovered was 67% by mass.

[Comparative Example 3-8]

The same method as in Example 10 was performed, except that, in the step corresponding to the step (a2-1) of Example 3-10, the catalyst was not used, and a crude 4-aminomethyl-1,8-octanediamine-containing liquid was recovered. The yield thereof was 42% by mass. In addition, the crude ethylphenol-containing liquid was recovered. The yield thereof was 54% by mass.

[Comparative Example 3-9]

The same method as in Example 3-11 was performed, except that 2-butanol was used instead of the aromatic hydroxy compound in the step corresponding to the step (a1) of Example 3-11. An attempt was made to separate the crude 2,4-toluenediamine-containing liquid and the crude 4-(1,1,3,3-tetramethylbutyl)phenol-containing liquid in the distillation column, but a fixed material was generated in the distillation column and could not be removed, so that the operation was interrupted. The yield of 2,4-toluenediamine which could be partially recovered was 62% by mass. In addition, the yield of 4-(1,1,3,3-tetramethylbutyl)phenol which could be recovered was 37% by mass.

[Comparative Example 3-10]

The same method as in Example 3-10 was performed, except that, in the step corresponding to the step (a2-1) of Example 3-10, the active hydrogen compound was not used, and a crude 4-aminomethyl-1,8-octanediamine-containing liquid was recovered. The yield thereof was 67% by mass. In addition, the crude ethylphenol-containing liquid was recovered. The yield thereof was 68% by mass.

<<Fourth Test>>

<Analysis method>

(1) $^1$H-NMR analysis method

$^1$H-NMR analysis was performed using a JNM-A400 FT-NMR system manufactured by JEOL Ltd. as an apparatus.

(1-1) Preparation of $^1$H-NMR analysis sample

0.3 g of the sample solution was weighed, and a solution obtained by adding 0.7 g of deuterated chloroform and 0.05 g of dimethyldiphenylsilane as an internal standard substance to the sample solution and uniformly mixing was used as an NMR analysis sample.

(1-2) Quantitative analysis method

Each standard substance was analyzed, and a quantitative analysis of the analysis sample solution was performed based on a created calibration curve.

(2) Gas chromatography analysis method

The analysis was performed under the following conditions.

(Measurement conditions)

Device: GC-2010 manufactured by Shimadzu Corporation
Column: DB-1
Diameter: 0.25 mm, length: 30 m, film thickness: 1.0 μm
Column temperature: 60°C to 300°C
Injection port temperature: 300°C
Carrier gas: helium
Carrier gas flow rate: 40 mL/min
Detector: flame ionization detector (FID)

(2-1) Preparation of gas chromatography analysis sample

1.0 g of the sample solution was weighed, and a solution obtained by adding 10 g of acetonitrile and 0.1 g of anisole as an internal standard substance to the sample solution and uniformly mixing was used as a gas chromatography analysis sample.

(3) Liquid chromatography analysis method

The analysis was performed under the following conditions.

(Measurement conditions)

Device: LC-10AT manufactured by Shimadzu Corporation
Column: Inertsil ODS
Particle diameter: 5 μm, inner diameter: 2.1 mm, length: 250 mm
Column temperature: 40°C
Developing solvent: water/acetonitrile = 90/10
Solvent flow rate: 1 mL/min
Detector: photodiode array detector

(3-1) Preparation of liquid chromatography analysis sample

1.0 g of the sample solution was weighed, and a solution obtained by adding 10 g of acetic acid to the sample solution and uniformly mixing was used as a liquid chromatography analysis sample.

(3-2) Quantitative analysis method

Each standard substance was analyzed, and a quantitative analysis of the analysis sample solution was performed based on a created calibration curve.

From the measured values of $^1$H-NMR, GC, and LC, the yield of the generated isocyanate compound was calculated, and the value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) was calculated.

[Example 4-1]

Step (1-1): production of carbamate compound

A reaction was carried out using a device shown in FIG. 37. In the production of the carbamate compounds in Examples 4-1 and subsequent Examples, the device shown in FIG. 37 was also used.

In a state in which the line 14a was closed, 3.33 kg (19.2 mol) of 4-aminomethyl-1,8-octanediamine was supplied from the storage tank 101a through the line 11a to the baffle-equipped SUS reaction container 104a, and 5.50 kg (58.5 mol) of phenol was supplied from the storage tank 102a through the line 12a to the reaction container 104a, and the

mixture was stirred to be uniform. Next, in a state in which the line 16 was closed, 5.50 kg (58.5 mol) of phenol was supplied from the storage tank 102a through the line 15a to the baffle-equipped SUS reactor 105a, and 20.52 kg (95.9 mol) of diphenyl carbonate (DPC) was supplied from the storage tank 103a through the line 13a to the above-described reaction container 105a. The liquid temperature of the reaction container 105a was adjusted to 65°C, and the mixture was made uniform by stirring. Thereafter, the mixture was supplied from the reaction container 104a through the line 14a such that the internal temperature of the mixed solution of 4-aminomethyl-1,8-octanediamine and phenol did not exceed 70°C. After continuing the stirring for 2 hours, the reaction solution was heated to 120°C and the internal pressure was adjusted to approximately 1 kPa to extract 15.53 kg of phenol in the liquid to the storage tank 107 through the line 17 and the condenser X11.

As a result of analyzing the solution after the reaction (hereinafter, referred to as "reaction solution (1-1)") by liquid chromatography, a carbamate compound corresponding to 4-aminomethyl-1,8-octanediamine was produced in a yield of 99% by mass. The line 16 was opened, and the reaction solution (1-1) was transferred to the storage tank 106 through the line 16. The mass of the reaction solution (1-1) was 19.40 kg.

Step (1-2): thermal decomposition step of carbamate compound

A reaction was carried out using a device shown in FIG. 38. In the thermal decomposition of the carbamate compounds in Examples 4-1 and subsequent Examples, the device shown in FIG. 38 was also used.

In a state in which the line 24 was closed, 19.40 kg of diphenyl carbonate was supplied from the storage tank 202 through the line 22 to the baffle-equipped SUS reaction container 201. The temperature of the multi-stage distillation column 203 was raised to 170°C, and the jacket temperature of the reaction container 201 was heated to 228°C to reduce the pressure to 14 kPa. 19.40 kg of the reaction solution (1-1) recovered in the storage tank 106 in the step (1-1) was heated to 120°C, supplied to the reaction container 201 through the line 21 in approximately 15 minutes, and the carbamate compound was thermally decomposed. The pressure was adjusted to a range of 8 to 14 kPa, and the phenol generated by the thermal decomposition was separated into diphenyl carbonate and 1,8-diisocyanato-4-isocyanatomethyloctane (TTI) as products in the distillation column 203, and recovered in the storage tank 204 through the line 25, the condenser X21, and the line 27. The reflux ratio at this time was 1.2. After all of the reaction solution (1-1) was transferred, the extraction of phenol was further continued at an internal temperature of 220°C. The reaction was terminated 4 hours after the entire reaction solution (1-1) was transferred, the reaction solution was extracted from the line 28 and transferred to the storage tank 205. The mass of the reaction solution transferred to the storage tank 205 was 18.63 kg. As a result of subjecting the reaction solution (hereinafter, referred to as "reaction solution (1-2)") to $^1$H-NMR and gas chromatography analysis, TTI was produced in a yield of 70% by mass, and the value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) was 3.7. In addition, the concentration of DPC was 58% by mass and the concentration of the carbonyl compound (1) was 18% by mass with respect to the total mass of the reaction solution (1-2).

Step (1-3): low-boiling separation step

A reaction was carried out using a device shown in FIG. 39. In the low-boiling separation in Examples 4-1 and subsequent Examples, the device shown in FIG. 39 was also used.

The reaction solution (1-2) was continuously fed to the middle of the continuous multi-stage distillation column 301 at a rate of 3.73 kg/hour from the storage tank 205 through the line 31, and the distillation separation of the liquid-phase component was performed. The heat quantity required for the distillation separation was supplied by circulating the liquid in the lower part of the column through the reboiler X32 and the line 33. The liquid temperature of the bottom of the continuous multi-stage distillation column was 220°C, and the pressure of the column top was 1.5 kPa. The gas distilled from the top of the continuous multi-stage distillation column 301 was condensed by the condenser X31 through the line 32, and continuously extracted to the storage tank 302 through the line 36. In addition, the extraction speed of the line 34 was 1.49 kg/hour in a steady state, and the extraction was continuously performed into the storage tank 303. The solution recovered in the storage tank 303 (hereinafter, referred to as "reaction solution (1-3)" or "isocyanate composition (1-3)") was 7.45 kg, and as a result of subjecting the reaction solution to NMR, LC, and gas chromatography analysis, TTI was recovered in a yield of 82% by mass with respect to the supplied reaction solution (1-2), and the value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) was 4.6. In addition, the concentration of DPC was 400 ppm by mass and the concentration of the carbonyl compound (I) was 44% by mass with respect to the total mass of the reaction solution (1-3).

Step (1-4): high-boiling separation step

A reaction was carried out using a device shown in FIG. 40. In the high-boiling separation in Examples 4-1 and subsequent Examples, the device shown in FIG. 40 was also used.

A thin film distillation device 401 (manufactured by Kobe Steel, Ltd., Japan) was heated to 190°C, and the internal pressure was set to 0.3 kPa. The reaction solution (1-3) recovered in the storage tank 303 in the step (1-3) was supplied to the upper part of the thin film distillation device 401 at approximately 1.0 kg/hour through the line 41 to separate the isocyanate from the high-boiling component. The generated gas-phase component was transferred to the storage tank 402 through the line 42 and the condenser X41. The amount of the liquid recovered from the storage tank 402 was 3.35 kg, and the recovery rate of TTI was 121% by mass. The reason why the TTI recovery rate exceeded 100% by mass is that a part of the modified product of isocyanate, which had been generated in the thermal decomposition step or the low-boiling separation step, is regenerated in TTI.

[Example 4-2]

Step (2-2): thermal decomposition step of carbamate compound

A thermal decomposition reaction was performed in the same manner as in Example 4-1, except that 19.4 kg of the reaction solution (1-1) of Example 4-1 and 19.4 kg of diphenyl carbonate were used, the reaction solution (1-1) was supplied to the reactor over about 13 minutes to start the reaction, the reaction was performed at a jacket temperature of 248°C, an internal temperature of 240°C, a reflux ratio of 0.4, and a pressure in a range of 14 to 26 kPa, and after the entire reaction solution (1-1) was transferred, the extraction of phenol was continued for 3 hours. The mass of the reaction solution transferred to the storage tank 205 was 20.96 kg. As a result of analyzing the reaction solution (hereinafter, referred to as "reaction solution (2-2)") by NMR, LC, and gas chromatography, TTI was produced in a yield of 78% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 4.3. In addition, the concentration of DPC was 68% by mass and the concentration of the carbonyl compound (I) was 18% by mass with respect to the total mass of the reaction solution (2-2).

Step (2-3): low-boiling separation step

Low-boiling separation was performed in the same manner as in Example 4-1, except that the reaction solution (2-2) was continuously fed at 4.19 kg/hour and the extraction rate was 1.26 kg/hour in a steady state of the line 34. The solution recovered in the storage tank 303 (hereinafter, referred to as "reaction solution (2-3)" or "isocyanate composition (2-3)") was 6.29 kg, and as a result of subjecting the reaction solution to NMR, LC, and gas chromatography analysis, TTI was recovered in a yield of 77% by mass with respect to the supplied reaction solution (2-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 6.3. In addition, the concentration of DPC was 550 ppm by mass and the concentration of the carbonyl compound (I) was 34% by mass with respect to the total mass of the reaction solution (2-3).

Step (2-4): high-boiling separation step

High-boiling separation was performed by the same operation as in Example 4-1, except that the liquid recovered in the storage tank 303 in the step (2-3) was used. The amount of the liquid recovered from the storage tank 402 was 3.36 kg, and the recovery rate of TTI was 116% by mass.

[Example 4-3]

Step (3-2): thermal decomposition step of carbamate compound

A thermal decomposition reaction was performed in the same manner as in Example 4-1, except that 19.4 kg of the reaction solution (1-1) of Example 4-1 and 19.4 kg of diphenyl carbonate were used, the reaction solution (1-1) was supplied to the reactor over about 9 minutes to start the reaction, the reaction was performed at a jacket temperature of 258°C, an internal temperature of 250°C, a reflux ratio of 2.0, and a pressure in a range of 21 to 32 kPa, and after the entire reaction solution (1-1) was transferred, the extraction of phenol was continued for 2.5 hours. The mass of the reaction solution transferred to the storage tank 205 was 22.12 kg. As a result of analyzing the reaction solution (hereinafter, referred to as "reaction solution (3-2)") by NMR, LC, and gas chromatography, TTI was produced in a yield of 73% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 3.9. In addition, the concentration of DPC was 67% by mass and the concentration of the carbonyl compound (I) was 13% by mass with respect to the total mass of the reaction solution (3-2).

Step (3-3): low-boiling separation step

Low-boiling separation was performed in the same manner as in Example 4-1, except that the reaction solution (3-2) was continuously fed at 4.42 kg/hour and the extraction rate was 1.42 kg/hour in a steady state of the line 34. The solution recovered in the storage tank 303 (hereinafter, referred to as "reaction solution (3-3)" or "isocyanate composition (3-3)") was 7.08 kg, and as a result of subjecting the reaction solution to NMR, LC, and gas chromatography analysis, TTI was recovered in a yield of 80% by mass with respect to the supplied reaction solution (3-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 5.5. In addition, the concentration of DPC was 320 ppm by mass and the concentration of the carbonyl compound (I) was 40% by mass with respect to the total mass of the reaction solution (3-3).

Step (3-4): high-boiling separation step

High-boiling separation was performed by the same operation as in Example 4-1, except that the liquid recovered in the storage tank 303 in the step (3-3) was used. The amount of the liquid recovered from the storage tank 402 was 3.35 kg, and the recovery rate of TTI was 119% by mass.

[Example 4]

Step (4-2): thermal decomposition step of carbamate compound

A thermal decomposition reaction was performed in the same manner as in Example 4-1, except that 19.4 kg of the reaction solution (1-1) of Example 4-1 and 19.4 kg of diphenyl carbonate were used, the reaction solution (1-1) was supplied to the reactor over about 14 minutes to start the reaction, the reaction was performed at a jacket temperature of 238°C, an internal temperature of 230°C, a reflux ratio of 1.5, and a pressure in a range of 8 to 14 kPa, and after the entire reaction solution (1-1) was transferred, the extraction of phenol was continued for 3 hours. The weight of the reaction solution transferred to the storage tank 205 was 22.12 kg. As a result of analyzing the reaction solution (hereinafter, referred to as "reaction solution (4-2)") by NMR, LC, and gas chromatography, TTI was produced in a yield of 79% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 1.6. In addition, the concentration of DPC was 61% by mass and the concentration of the carbonyl compound (I) was 19% by mass with respect to the total mass of the reaction solution (4-2).

Step (4-3): low-boiling separation step

Low-boiling separation was performed in the same manner as in Example 4-1, except that the reaction solution (4-2) was continuously fed at 4.42 kg/hour and the extraction rate was 1.68 kg/hour in a steady state of the line 34. The solution recovered in the storage tank 303 (hereinafter, referred to as "reaction solution (4-3)" of "isocyanate composition (4-3)") was 8.41 kg, and as a result of subjecting the reaction solution to NMR, LC, and gas chromatography analysis, TTI was recovered in a yield of 85% by mass with respect to the supplied reaction solution (4-2), and the value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) was 2.6. In addition, the concentration of DPC was 730 ppm by mass and the concentration of the carbonyl compound (I) was 50% by mass with respect to the total mass of the reaction solution (4-3).

Step (4-4): high-boiling separation step

High-boiling separation was performed by the same operation as in Example 4-1, except that the liquid recovered in the storage tank 303 in the step (4-3) was used. The amount of the liquid recovered from the storage tank 402 was

4.05 kg, and the recovery rate of TTI was 125% by mass.

[Example 4-5]

Step (5-1): production of carbamate compound

Carbamate synthesis was performed by the same operation as in Example 4-1, except that 0.23 kg (69.2 mol) of urea was used instead of diphenyl carbonate, 25.87 kg (275.2 mol) of phenol supplied to the storage tank 102, and the reaction temperature of the reactor 104 was set to 240°C and stirred for 30 minutes. The amounts of phenol and ammonia extracted into the storage tank 107 were 24.14 kg.

As a result of analyzing the solution after the reaction (hereinafter, referred to as "reaction solution (5-1)") by liquid chromatography, a corresponding carbamate compound was produced in a yield of 99% by mass. The line 16 was opened, and the reaction solution (5-1) was transferred to the storage tank 106 through the line 16. The mass of the reaction solution (5-1) was 10.79 kg.

Step (5-2): thermal decomposition step of carbamate compound

A thermal decomposition reaction was performed in the same manner as in Example 4-1, except that 10.8 kg of the reaction solution (5-1) and 28.0 kg of diphenyl carbonate were used, the reaction solution (1-1) was supplied to the reactor over about 13 minutes to start the reaction, the reaction was performed at a jacket temperature of 238°C, an internal temperature of 230°C, a reflux ratio of 1.7, and a pressure in a range of 8 to 14 kPa, and after the entire reaction solution (5-1) was transferred, the extraction of phenol was continued for 3 hours. The mass of the reaction solution transferred to the storage tank 205 was 21.34 kg. As a result of analyzing the reaction solution (hereinafter, referred to as "reaction solution (5-2)") by NMR, LC, and gas chromatography, TTI was produced in a yield of 78% by mass. The value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) in the reaction solution was 1.5. In addition, the concentration of DPC was 58% by mass and the concentration of the carbonyl compound (I) was 21% by mass with respect to the total mass of the reaction solution (5-2).

Step (5-3): low-boiling separation step

Low-boiling separation was performed in the same manner as in Example 4-1, except that the reaction solution (5-2) was continuously fed at 4.27 kg/hour and the extraction rate was 1.75 kg/hour in a steady state of the line 34. The solution recovered in the storage tank 303 (hereinafter, referred to as "reaction solution (5-3)" or "isocyanate composition (5-3)") was 8.75 kg, and as a result of subjecting the reaction solution to NMR, LC, and gas chromatography analysis, TTI was recovered in a yield of 84% by mass with respect to the supplied reaction solution (5-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 2.5. In addition, the concentration of DPC was 510 ppm by mass and the concentration of the carbonyl compound (I) was 52% by mass with respect to the total mass of the reaction solution (5-3).

Step (5-4): high-boiling separation step

High-boiling separation was performed by the same operation as in Example 4-1, except that the liquid recovered in the storage tank 303 in the step (5-3) was used. The amount of the liquid recovered from the storage tank 402 was 4.05 kg, and the recovery rate of TTI was 128% by mass.

[Comparative Example 4-1]

Step (1'-2): thermal decomposition step of carbamate compound

A thermal decomposition reaction was performed in the same manner as in Example 4-1, except that 19.4 kg of the reaction solution (1-1) of Example 4-1 and 5.45 kg of diphenyl carbonate were used, the reaction solution (1-1) was supplied to the reactor over about 14 minutes to start the reaction, the reaction was performed at a jacket temperature of 238°C, an internal temperature of 230°C, a reflux ratio of 1.5, and a pressure in a range of 5 to 15 kPa, and after the entire reaction solution (1-1) was transferred, the extraction of phenol was continued for 1.5 hours. The mass of the reaction solution transferred to the storage tank 205 was 5.71 kg. As a result of analyzing the reaction solution (hereinafter,

referred to as "reaction solution (1'-2)") by NMR, LC, and gas chromatography, TTI was produced in a yield of 16% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 64.5. In addition, the concentration of DPC was 5% by mass and the concentration of the carbonyl compound (I) was 12% by mass with respect to the total mass of the reaction solution (1'-2).

Step (1'-3): low-boiling separation step

Low-boiling separation was performed in the same manner as in Example 4-1, except that the reaction solution (1'-2) was continuously fed at 1.14 kg/hour and the extraction rate was 1.09 kg/hour in a steady state of the line 34, but the liquid became highly viscous during the operation and it was difficult to continue the operation.

[Comparative Example 4-2]

Step (2'-2): thermal decomposition step of carbamate compound

A thermal decomposition reaction was performed in the same manner as in Example 4-1, except that 19.4 kg of the reaction solution (1-1) of Example 4-1 and 10.89 kg of diphenyl carbonate were used, the reaction solution (1-1) was supplied to the reactor over about 13 minutes to start the reaction, the reaction was performed at a jacket temperature of 238°C, an internal temperature of 230°C, a reflux ratio of 0, and a pressure in a range of 11 to 15 kPa, and after the entire reaction solution (1-1) was transferred, the extraction of phenol was continued for 4 hours. The mass of the reaction solution transferred to the storage tank 205 was 9.39 kg. As a result of analyzing the reaction solution (hereinafter, referred to as "reaction solution (2'-2)") by NMR, LC, and gas chromatography, TTI was produced in a yield of 20% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 17.7. In addition, the concentration of DPC was 27% by mass and the concentration of the carbonyl compound (I) was 23% by mass with respect to the total mass of the reaction solution (2'-2).

Step (2'-3): low-boiling separation step

Low-boiling separation was performed in the same manner as in Example 4-1, except that the reaction solution (2'-2) was continuously fed at 1.88 kg/hour and the extraction rate was 1.41 kg/hour in a steady state of the line 34, but the liquid became highly viscous during the operation and it was difficult to continue the operation.

[Comparative Example 4-3]

Step (3'-2): thermal decomposition step of carbamate compound

A thermal decomposition reaction was performed in the same manner as in Example 4-1, except that 19.4 kg of the reaction solution (1-1) of Example 4-1 and 10.89 kg of diphenyl carbonate were used, the reaction solution (1-1) was supplied to the reactor over about 11 minutes to start the reaction, the reaction was performed at a jacket temperature of 248°C, an internal temperature of 240°C, a reflux ratio of 2.5, and a pressure in a range of 30 to 32 kPa, and after the entire reaction solution (1-1) was transferred, the extraction of phenol was continued for 4 hours. The mass of the reaction solution transferred to the storage tank 205 was 20.0 kg. As a result of analyzing the reaction solution (hereinafter, referred to as "reaction solution (3'-2)") by NMR, LC, and gas chromatography, TTI was produced in a yield of 27% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 12.0. In addition, the concentration of DPC was 63% by mass and the concentration of the carbonyl compound (I) was 14% by mass with respect to the total mass of the reaction solution (3'-2).

Step (3'-3): low-boiling separation step

Low-boiling separation was performed in the same manner as in Example 4-1, except that the reaction solution (3'-2) was continuously fed at 3.99 kg/hour and the extraction rate was 1.64 kg/hour in a steady state of the line 34, but the liquid became highly viscous during the operation and it was difficult to continue the operation.

[Comparative Example 4-4]

Step (4'-2): thermal decomposition step of carbamate compound

A thermal decomposition reaction was performed in the same manner as in Example 4-1, except that 19.4 kg of the reaction solution (1-1) of Example 4-1 and 19.4 kg of diphenyl carbonate were used, the reaction solution (1-1) was supplied to the reactor over about 15 minutes to start the reaction, the reaction was performed at a jacket temperature of 238°C, an internal temperature of 230°C, a reflux ratio of 0, and a pressure in a range of 14 to 18 kPa, and after the entire reaction solution (1-1) was transferred, the extraction of phenol was continued for 4 hours. The mass of the reaction solution transferred to the storage tank 205 was 18.2 kg. As a result of analyzing the reaction solution (hereinafter, referred to as "reaction solution (4'-2)") by NMR, LC, and gas chromatography, TTI was produced in a yield of 59% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 12.8. In addition, the concentration of DPC was 66% by mass and the concentration of the carbonyl compound (I) was 8% by mass with respect to the total mass of the reaction solution (4'-2).

Step (4'-3): low-boiling separation step

Low-boiling separation was performed in the same manner as in Example 4-1, except that the reaction solution (4'-2) was continuously fed at 3.65 kg/hour and the extraction rate was 1.46 kg/hour in a steady state of the line 34, but the liquid became highly viscous during the operation and it was difficult to continue the operation.

[Comparative Example 4-5]

Step (5'-2): thermal decomposition step of carbamate compound

A thermal decomposition reaction was performed in the same manner as in Example 4-1, except that 19.4 kg of the reaction solution (1-1) of Example 4-1 and 10.89 kg of diphenyl carbonate were used, the reaction solution (1-1) was supplied to the reactor over about 10 minutes to start the reaction, the reaction was performed at a jacket temperature of 258°C, an internal temperature of 250°C, a reflux ratio of 0.5, and a pressure in a range of 18 to 32 kPa, and after the entire reaction solution (1-1) was transferred, the extraction of phenol was continued for 2.5 hours. The mass of the reaction solution transferred to the storage tank 205 was 16.1 kg. As a result of analyzing the reaction solution (hereinafter, referred to as "reaction solution (5'-2)") by NMR, LC, and gas chromatography, TTI was produced in a yield of 71% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 6.4. In addition, the concentration of DPC was 59% by mass and the concentration of the carbonyl compound (I) was 13% by mass with respect to the total mass of the reaction solution (5'-2).

Step (5'-3): low-boiling separation step

Low-boiling separation was performed in the same manner as in Example 4-1, except that the reaction solution (5'-2) was continuously fed at 3.21 kg/hour and the extraction rate was 1.22 kg/hour in a steady state of the line 34. The solution recovered in the storage tank 303 (hereinafter, may be referred to as "reaction solution (5'-3)) was 6.10 kg, and as a result of subjecting the reaction solution to NMR, LC, and gas chromatography analysis, TTI was recovered in a yield of 74% by mass with respect to the supplied reaction solution (5'-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 8.2. In addition, the concentration of DPC was 850 ppm by mass and the concentration of the carbonyl compound (I) was 33% by mass with respect to the total mass of the reaction solution (5'-3).

Step (5'-4): high-boiling separation step

High-boiling separation was performed by the same operation as in Example 4-1, except that the liquid recovered in the storage tank 303 in the step (5'-3) was used, but the residue had a high viscosity and it was difficult to continue the operation.

[Comparative Example 4-6]

Step (6'-2): thermal decomposition step of carbamate compound

A thermal decomposition reaction was performed in the same manner as in Example 1, except that 19.4 kg of the reaction solution (1-1) of Example 4-1 and 19.4 kg of diphenyl carbonate were used, the reaction solution (1-1) was supplied to the reactor over about 12 minutes to start the reaction, the reaction was performed at a jacket temperature of 238°C, an internal temperature of 230°C, a reflux ratio of 0.2, and a pressure in a range of 11 to 16 kPa, and after the entire reaction solution (1-1) was transferred, the extraction of phenol was continued for 3 hours. The mass of the reaction solution transferred to the storage tank 205 was 17.5 kg. As a result of analyzing the reaction solution (hereinafter, referred to as "reaction solution (6'-2)") by NMR, LC, and gas chromatography, TTI was produced in a yield of 79% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 5.5. In addition, the concentration of DPC was 64% by mass and the concentration of the carbonyl compound (I) was 9% by mass with respect to the total mass of the reaction solution (6'-2).

Step (6'-3): low-boiling separation step

Low-boiling separation was performed in the same manner as in Example 4-1, except that the reaction solution (6'-2) was continuously fed at 3.49 kg/hour and the extraction rate was 1.15 kg/hour in a steady state of the line 34. The solution recovered in the storage tank 303 (hereinafter, may be referred to as "reaction solution (6'-3)) was 5.76 kg, and as a result of subjecting the reaction solution to NMR, LC, and gas chromatography analysis, TTI was recovered in a yield of 74% by mass with respect to the supplied reaction solution (6'-2), and the value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) was 8.3. In addition, the concentration of DPC was 920 ppm by mass and the concentration of the carbonyl compound (I) was 29% by mass with respect to the total mass of the reaction solution (6'-3).

Step (6'-4): high-boiling separation step

High-boiling separation was performed by the same operation as in Example 4-1, except that the liquid recovered in the storage tank 303 in the step (6'-3) was used, but the residue had a high viscosity and it was difficult to continue the operation.

[Example 4-6]

Step (6-1): production of carbamate compound

In a state in which the line 14 was closed, 3.33 kg (16.8 mol) of 4,4'-diaminodiphenylmethane was supplied from the storage tank 101a through the line 1 1a to the baffle-equipped SUS reaction container 104a, and 2.53 kg (27.0 mol) of phenol was supplied from the storage tank 102a through the line 12a to the reaction container 104a, and the mixture was stirred to be uniform. Next, in a state in which the line 16 was closed, 2.53 kg (27.0 mol) of phenol was supplied from the storage tank 102a through the line 15a to the baffle-equipped SUS reaction container 105a, and 11.96 kg (55.6 mol) of diphenyl carbonate was supplied from the storage tank 103a through the line 13a to the above-described reaction container 105a. The liquid temperature of the reaction container 105a was adjusted to 65°C, and the mixture was made uniform by stirring. Thereafter, the mixture was supplied from the reaction container 104a through the line 14a such that the internal temperature of the mixed solution of 4,4'-diaminodiphenylmethane and phenol did not exceed 70°C. After continuing the stirring for 2 hours, the reaction solution was heated to 120°C and the internal pressure was adjusted to approximately 1 kPa to extract 7.58 kg of phenol in the liquid to the storage tank 107 through the line 17 and the condenser X11.

As a result of analyzing the solution after the reaction (hereinafter, referred to as "reaction solution (6-1)") by liquid chromatography, a carbamate compound corresponding to 4,4'-diaminodiphenylmethane was produced in a yield of 95% by mass. The line 16 was opened, and the reaction solution (6-1) was transferred to the storage tank 106 through the line 16. The mass of the reaction solution (6-1) was 12.77 kg.

Step (6-2): thermal decomposition step of carbamate compound

A thermal decomposition reaction was performed in the same manner as in Example 4-1, except that 12.77 kg of the reaction solution (6-1) and 12.77 kg of diphenyl carbonate were used, the reaction solution (6-1) was supplied to the reactor over about 10 minutes to start the reaction, the reaction was performed at a jacket temperature of 238°C, an internal temperature of 230°C, a reflux ratio of 0.8, and a pressure in a range of 11 to 16 kPa, and after the entire reaction solution (6-1) was transferred, the extraction of phenol was continued for 3 hours. The mass of the reaction solution transferred to the storage tank 205 was 11.75 kg. As a result of analyzing the reaction solution (hereinafter, referred to as "reaction solution (6-2)") by NMR and gas chromatography, diphenylmethane diisocyanate (MDT) was produced in a yield of 70% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 1.3. In addition, the concentration of DPC was 15% by mass and the concentration of the carbonyl compound (I) was 38% by mass with respect to the total mass of the reaction solution (6-2).

Step (6-3): low-boiling separation step

Low-boiling separation was performed in the same manner as in Example 4-1, except that the reaction solution (6-2) was continuously fed at 2.35 kg/hour and the extraction rate was 1.97 kg/hour in a steady state of the line 34. The solution recovered in the storage tank 303 (hereinafter, referred to as "reaction solution (6-3)" or "isocyanate composition (6-3)") was 9.87 kg, and as a result of subjecting the reaction solution to NMR, LC, and gas chromatography analysis, MDI was recovered in a yield of 79% by mass with respect to the supplied reaction solution (6-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 2.3. In addition, the concentration of DPC was 420 ppm by mass and the concentration of the carbonyl compound (I) was 45% by mass with respect to the total mass of the reaction solution (6-3).

Step (6-4): high-boiling separation step

High-boiling separation was performed by the same operation as in Example 4-1, except that the liquid recovered in the storage tank 303 in the step (6-3) was used, and the operating temperature was set to 170°C and the internal pressure was set to 0.3 kPa. The amount of the liquid recovered from the storage tank 402 was 4.54 kg, and the recovery rate of MDI was 130% by mass.

[Example 4-7]

Step (7-1): production of carbamate compound

In a state in which the line 14a was closed, 3.33 kg (16.8 mol) of lysine β-aminoethyl ester trihydrochloride was supplied from the storage tank 101a through the line 11a to the baffle-equipped SUS reaction container 104a, and 2.52 kg (26.7 mol) of phenol was supplied from the storage tank 102a through the line 12a to the reaction container 104a, and the mixture was stirred to be uniform. Next, in a state in which the line 16 was closed, 2.52 kg (26.7 mol) of phenol was supplied from the storage tank 102a through the line 15a to the baffle-equipped SUS reaction container 105a, and 11.91 kg (55.6 mol) of diphenyl carbonate was supplied from the storage tank 103a through the line 13a to the above-described reaction container 105a. The liquid temperature of the reaction container 105a was adjusted to 65°C, and the mixture was made uniform by stirring. Thereafter, the mixture was supplied from the reaction container 104a through the line 14a such that the internal temperature of the mixed solution of lysine β-aminoethyl ester trihydrochloride and phenol did not exceed 70°C. After continuing the stirring for 2 hours, the reaction solution was heated to 120°C and the internal pressure was adjusted to approximately 1 kPa to extract 8.22 kg of phenol in the liquid to the storage tank 107 through the line 17 and the condenser X11.

As a result of analyzing the solution after the reaction (hereinafter, referred to as "reaction solution (7-1)") by liquid chromatography, a carbamate compound corresponding to lysine β-aminoethyl ester was produced in a yield of 96% by mass. The line 16 was opened, and the reaction solution (7-1) was transferred to the storage tank 106 through the line 16. The mass of the reaction solution (7-1) was 11.45 kg.

Step (7-2): thermal decomposition step of carbamate compound

A thermal decomposition reaction was performed in the same manner as in Example 4-1, except that 11.45 kg

of the reaction solution (7-1) and 10.00 kg of diphenyl carbonate were used, the reaction solution (7-1) was supplied to the reactor over about 15 minutes to start the reaction, the reaction was performed at a jacket temperature of 238°C, an internal temperature of 230°C, a reflux ratio of 3.2, and a pressure in a range of 11 to 16 kPa, and after the entire reaction solution (7-1) was transferred, the extraction of phenol was continued for 3 hours. The mass of the reaction solution transferred to the storage tank 205 was 9.01 kg. As a result of analyzing the reaction solution (hereinafter, referred to as "reaction solution (7-2)") by NMR and gas chromatography, lysine triisocyanate (LTI) was produced in a yield of 77% by mass. The value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) in the reaction solution was 1.4. In addition, the concentration of DPC was 38% by mass and the concentration of the carbonyl compound (I) was 32% by mass with respect to the total mass of the reaction solution (7-2).

Step (7-3): low-boiling separation step

Low-boiling separation was performed in the same manner as in Example 4-1, except that the reaction solution (7-2) was continuously fed at 1.80 kg/hour and the extraction rate was 1.10 kg/hour in a steady state of the line 34. The solution recovered in the storage tank 303 (hereinafter, referred to as "reaction solution (7-3)" or "isocyanate composition (7-3)") was 5.50 kg, and as a result of subjecting the reaction solution to NMR, LC, and gas chromatography analysis, LTI was recovered in a yield of 83% by mass with respect to the supplied reaction solution (7-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 2.4. In addition, the concentration of DPC was 120 ppm by mass and the concentration of the carbonyl compound (I) was 53% by mass with respect to the total mass of the reaction solution (7-3).

Step (7-4): high-boiling separation step

High-boiling separation was performed by the same operation as in Example 4-1, except that the liquid recovered in the storage tank 303 in the step (7-3) was used, and the operating temperature was set to 190°C and the internal pressure was set to 0.1 kPa. The amount of the liquid recovered from the storage tank 402 was 2.38 kg, and the recovery rate of LTI was 125% by mass.

[Example 4-8]

Step (8-1): production of carbamate compound

In a state in which the line 14a was closed, 3.33 kg (14.3 mol) of lysine methyl ester dihydrochloride was supplied from the storage tank 101a through the line 11a to the baffle-equipped SUS reaction container 104a, and 1.91 kg (20.3 mol) of phenol was supplied from the storage tank 102a through the line 12a to the reaction container 104a, and the mixture was stirred to be uniform. Next, in a state in which the line 16 was closed, 1.91 kg (20.3 mol) of phenol was supplied from the storage tank 102a through the line 15a to the baffle-equipped SUS reaction container 105a, and 10.17 kg (47.5 mol) of diphenyl carbonate was supplied from the storage tank 103a through the line 13a to the above-described reaction container 105a. The liquid temperature of the reaction container 105a was adjusted to 65°C, and the mixture was made uniform by stirring. Thereafter, the mixture was supplied from the reaction container 104a through the line 14a such that the internal temperature of the mixed solution of lysine methyl ester dihydrochloride and phenol did not exceed 70°C. After continuing the stirring for 2 hours, the reaction solution was heated to 120°C and the internal pressure was adjusted to approximately 1 kPa to extract 5.93 kg of phenol in the liquid to the storage tank 107 through the line 17 and the condenser X11.

As a result of analyzing the solution after the reaction (hereinafter, referred to as "reaction solution (8-1)") by liquid chromatography, a carbamate compound corresponding to lysine methyl ester was produced in a yield of 97% by mass. The line 16 was opened, and the reaction solution (8-1) was transferred to the storage tank 106 through the line 16. The mass of the reaction solution (8-1) was 11.38 kg.

Step (8-2): thermal decomposition step of carbamate compound

A thermal decomposition reaction was performed in the same manner as in Example 4-1, except that 11.38 kg of the reaction solution (8-1) and 11.38 kg of diphenyl carbonate were used, the reaction solution (8-1) was supplied to the reactor over about 12 minutes to start the reaction, the reaction was performed at a jacket temperature of 238°C, an internal temperature of 230°C, a reflux ratio of 0.9, and a pressure in a range of 20 to 29 kPa, and after the entire

reaction solution (8-1) was transferred, the extraction of phenol was continued for 3 hours. The mass of the reaction solution transferred to the storage tank 205 was 19.35 kg. As a result of analyzing the reaction solution (hereinafter, referred to as "reaction solution (8-2)") by NMR and gas chromatography, lysine diisocyanate (LDI) was produced in a yield of 81% by mass. The value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) in the reaction solution was 1.4. In addition, the concentration of DPC was 61% by mass and the concentration of the carbonyl compound (I) was 13% by mass with respect to the total mass of the reaction solution (8-2).

Step (8-3): low-boiling separation step

Low-boiling separation was performed in the same manner as in Example 4-1, except that the reaction solution (8-2) was continuously fed at 3.87 kg/hour and the extraction rate was 1.47 kg/hour in a steady state of the line 34. The solution recovered in the storage tank 303 (hereinafter, referred to as "reaction solution (8-3)" or "isocyanate composition (8-3)") was 7.35 kg, and as a result of subjecting the reaction solution to NMR, LC, and gas chromatography analysis, LDI was recovered in a yield of 86% by mass with respect to the supplied reaction solution (8-2), and the value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) was 2.5. In addition, the concentration of DPC was 630 ppm by mass and the concentration of the carbonyl compound (I) was 35% by mass with respect to the total mass of the reaction solution (8-3).

Step (8-4): high-boiling separation step

High-boiling separation was performed by the same operation as in Example 4-1, except that the liquid recovered in the storage tank 303 in the step (8-3) was used, and the operating temperature was set to 180°C and the internal pressure was set to 0.1 kPa. The amount of the liquid recovered from the storage tank 402 was 4.57 kg, and the recovery rate of LDI was 122% by mass.

[Example 4-9]

Step (9-1): production of carbamate compound

In a state in which the line 14a was closed, 3.33 kg (15.8 mol) of 4,4'-methylenebis(cyclohexylamine) was supplied from the storage tank 101a through the line 11a to the baffle-equipped SUS reaction container 104a, and 2.29 kg (24.4 mol) of phenol was supplied from the storage tank 102a through the line 12a to the reaction container 104a, and the mixture was stirred to be uniform. Next, in a state in which the line 16 was closed, 2.29 kg (24.4 mol) of phenol was supplied from the storage tank 102a through the line 15a to the baffle-equipped SUS reaction container 105a, and 11.27 kg (52.7 mol) of diphenyl carbonate was supplied from the storage tank 103a through the line 13a to the above-described reaction container 105a. The liquid temperature of the reaction container 105a was adjusted to 65°C, and the mixture was made uniform by stirring. Thereafter, the mixture was supplied from the reaction container 104a through the line 14a such that the internal temperature of the mixed solution of 4,4'-methylenebis(cyclohexylamine) and phenol did not exceed 70°C. After continuing the stirring for 2 hours, the reaction solution was heated to 120°C and the internal pressure was adjusted to approximately 1 kPa to extract 6.94 kg of phenol in the liquid to the storage tank 107 through the line 17 and the condenser X11.

As a result of analyzing the solution after the reaction (hereinafter, referred to as "reaction solution (9-1)") by liquid chromatography, a carbamate compound corresponding to 4,4'-methylenebis(cyclohexylamine) was produced in a yield of 99% by mass. The line 16 was opened, and the reaction solution (9-1) was transferred to the storage tank 106 through the line 16. The mass of the reaction solution (9-1) was 12.24 kg.

Step (9-2): thermal decomposition step of carbamate compound

A thermal decomposition reaction was performed in the same manner as in Example 4-1, except that 12.24 kg of the reaction solution (9-1) and 12.24 kg of diphenyl carbonate were used, the reaction solution (9-1) was supplied to the reactor over about 10 minutes to start the reaction, the reaction was performed at a jacket temperature of 238°C, an internal temperature of 230°C, a reflux ratio of 4.5, and a pressure in a range of 11 to 16 kPa, and after the entire reaction solution (9-1) was transferred, the extraction of phenol was continued for 3 hours. The mass of the reaction solution transferred to the storage tank 205 was 9.79 kg. As a result of analyzing the reaction solution (hereinafter, referred to as "reaction solution (9-2)") by NMR, LC, gas chromatography, methylenebis(cyclohexylisocyanate) (HMDI)

was produced in a yield of 76% by mass. The value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) in the reaction solution was 2.0. In addition, the concentration of DPC was 15% by mass and the concentration of the carbonyl compound (I) was 27% by mass with respect to the total mass of the reaction solution (9-2).

Step (9-3): low-boiling separation step

Low-boiling separation was performed in the same manner as in Example 4-1, except that the reaction solution (9-2) was continuously fed at 1.96 kg/hour and the extraction rate was 1.65 kg/hour in a steady state of the line 34. The solution recovered in the storage tank 303 (hereinafter, referred to as "reaction solution (9-3)" or "isocyanate composition (9-3)") was 8.22 kg, and as a result of subjecting the reaction solution to NMR, LC, and gas chromatography analysis, HMDI was recovered in a yield of 82% by mass with respect to the supplied reaction solution (9-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 3.4. In addition, the concentration of DPC was 200 ppm by mass and the concentration of the carbonyl compound (I) was 32% by mass with respect to the total mass of the reaction solution (9-3).

Step (9-4): high-boiling separation step

High-boiling separation was performed by the same operation as in Example 4-1, except that the liquid recovered in the storage tank 303 in the step (9-3) was used, and the operating temperature was set to 190°C and the internal pressure was set to 0.3 kPa. The amount of the liquid recovered from the storage tank 402 was 4.85 kg, and the recovery rate of HMDI was 125% by mass.

[Example 4-10]

Step (10-1): production of carbamate compound

In a state in which the line 14a was closed, 3.33 kg (23.1 mol) of 1,3-di(aminomethyl)cyclohexane was supplied from the storage tank 101a through the line 11 a to the baffle-equipped SUS reaction container 104a, and 4.11 kg (43.7 mol) of phenol was supplied from the storage tank 102a through the line 12a to the reaction container 104a, and the mixture was stirred to be uniform. Next, in a state in which the line 16 was closed, 4.11 kg (43.7 mol) of phenol was supplied from the storage tank 102a through the line 15a to the baffle-equipped SUS reaction container 105a, and 16.44 kg (76.8 mol) of diphenyl carbonate was supplied from the storage tank 103a through the line 13a to the above-described reaction container 105a. The liquid temperature of the reaction container 105a was adjusted to 65°C, and the mixture was made uniform by stirring. Thereafter, the mixture was supplied from the reaction container 104a through the line 14a such that the internal temperature of the mixed solution of 1,3-di(aminomethyl)cyclohexane and phenol did not exceed 70°C. After continuing the stirring for 2 hours, the reaction solution was heated to 120°C and the internal pressure was adjusted to approximately 1 kPa to extract 11.74 kg of phenol in the liquid to the storage tank 107 through the line 17 and the condenser X11.

As a result of analyzing the solution after the reaction (hereinafter, referred to as "reaction solution (10-1)") by liquid chromatography, a carbamate compound corresponding to 1,3-di(aminomethyl)cyclohexane was produced in a yield of 99% by mass. The line 16 was opened, and the reaction solution (10-1) was transferred to the storage tank 106 through the line 16. The mass of the reaction solution (10-1) was 16.24 kg.

Step (10-2): thermal decomposition step of carbamate compound

A thermal decomposition reaction was performed in the same manner as in Example 4-1, except that 16.24 kg of the reaction solution (10-1) and 16.24 kg of diphenyl carbonate were used, the reaction solution (10-1) was supplied to the reactor over about 15 minutes to start the reaction, the reaction was performed at a jacket temperature of 238°C, an internal temperature of 230°C, a reflux ratio of 4.0, and a pressure in a range of 11 to 16 kPa, and after the entire reaction solution (10-1) was transferred, the extraction of phenol was continued for 3 hours. The mass of the reaction solution transferred to the storage tank 205 was 13.97 kg. As a result of analyzing the reaction solution (hereinafter, referred to as "reaction solution (10-2)") by NMR, LC, gas chromatography, 1,3-bis(isocyanatomethyl)cyclohexane (HXDI) was produced in a yield of 78% by mass. The value of {3 × (the molar amount of the isocyanurate group) + 2 × (the molar amount of the carbodiimide group) + 3 × (the molar amount of the uretonimine group) + 2 × (the molar amount of the allophanate group) }/(the molar amount of the carbonyl compound) in the reaction solution was 1.7. In addition,

the concentration of DPC was 31% by mass and the concentration of the carbonyl compound (I) was 25% by mass with respect to the total mass of the reaction solution (10-2).

Step (10-3): low-boiling separation step

Low-boiling separation was performed in the same manner as in Example 4-1, except that the reaction solution (10-2) was continuously fed at 2.79 kg/hour and the extraction rate was 1.90 kg/hour in a steady state of the line 34. The solution recovered in the storage tank 303 (hereinafter, referred to as "reaction solution (10-3)" or "isocyanate composition (10-3)") was 9.50 kg, and as a result of subjecting the reaction solution to NMR, LC, and gas chromatography analysis, HXDI was recovered in a yield of 77% by mass with respect to the supplied reaction solution (10-2), and the value of {3 × (the molar amount of the isocyanurate group) + 2 × (the molar amount of the carbodiimide group) + 3 × (the molar amount of the uretonimine group) + 2 × (the molar amount of the allophanate group)}/(the molar amount of the carbonyl compound) was 3.6. In addition, the concentration of DPC was 450 ppm by mass and the concentration of the carbonyl compound (I) was 37% by mass with respect to the total mass of the reaction solution (10-3).

Step (10-4): high-boiling separation step

High-boiling separation was performed by the same operation as in Example 4-1, except that the liquid recovered in the storage tank 303 in the step (10-3) was used, and the operating temperature was set to 190°C and the internal pressure was set to 0.3 kPa. The amount of the liquid recovered from the storage tank 402 was 4.93 kg, and the recovery rate of HXDI was 122% by mass.

[Industrial Applicability]

According to the method for producing an isocyanate compound of the present embodiment, the yield of the isocyanate compound is favorable, stable continuous operation can be performed, and an amount of by-products incorporated can be suppressed.

In addition, the method for producing a carbamate compound according to the present embodiment does not use a metal catalyst, is inexpensive, and has a favorable yield.

In addition, according to the recovery method of the present embodiment, it is possible to efficiently regenerate a useful component including an amine compound from a liquid-phase component remaining after production of an isocyanate compound.

In addition, according to the isocyanate composition of the present embodiment, it is possible to provide an isocyanate composition which can prevent fixation of by-products to a device and improve the yield of an isocyanate compound in a case of producing an isocyanate compound without using phosgene.

[Reference Signs List]

A21, A22, A23, A24, A25, A30, A31, A32, A33, A40, A41, A42, A43, A44, A45, A46, A47, A48, A49, A50, A51, A61, A62, A63, A64, A65, A71, A72, A73, A81, A82, A83, A84, A85, B11, B12, B13, B14, B15, B20, B21, B22, B23, B30a, B30b, B31a, B31b, B32a, B32b, B33, B34, B35, B36, B37, B40, B41, B42, B43, B44, B45, B46, B47, B50, B52, B53, B54, B55, B56, B60, B61a, B61b, B62, B63, B64, B65, B66, B67, B68, C11, C12, C13, C14, D11, D12, D13, D14, E11, E12, E13, E14, E20, E21, E22, E23, E24, E25, E26, F10, F11, F12, F13, F14, F15, F16: line
A101, A102, A204, A304, B104, C104, C104, E101, F104: tank
A201, B101, B202, B204, C101, E102: distillation column
A202, A302, B102, B203, C102: pressure gauge
A203, A404, A603, A703, A803, B103, B204, B304, B306, B404, B406, B504, B506, B604, B606, C103, E204, F103: condenser
A205, A303, A405, A406, A407, A502, A604, A605, A702, A703, A804, A805, B105, B309, C105, E206, F105: storage tank
A301: thin film evaporator
E201, E202, F101: reactor
E203, F102: vacuum distiller
E205: storage valve
A401, A501: stirring tank
A402: gas-liquid separator

A403, A601: multi-stage distillation column
A701: pre-concentrator
A801: continuous multi-stage distillation column
B201: tubular thermal decomposition reactor
B301, B302, B401, B402, B601, B602: falling film-type evaporator
B303, B307, B403, B407, B502, B603, B607: separation column
A408, A602, A802, B305, B308, B405, B408, B505, B507, B605, B608: reboiler
B501: thermal decomposition device
B503: purification column
D101: side surface portion of evaporator
D102: evaporator
D103: bottom portion of evaporator
D104: inside portion of evaporator (condensation part)
11, 12, 13, 14, 15: line
101: distillation column
102: reboiler
103: condenser
104: storage tank
105: storage tank
a1, a2, a3, a4, a5, a6, a7, a8, a9, a10, a11, a12, a13, a14, a15, a16, a17, a18, a19, a20, a21, a22, a23, a24, a25, a26, a27, a31, a32, a33, a40, a41, a42a, a42b, a43, a44a, a44b, a45a, a45b, a46a, a46b, a47a, a47b, a48, a51, a52, a61, a62a, a62b, a63a, a63b, a64, a65, a71, a72, a73, a74, a75a, a75b, a76a, a76b, a77a, a77b, a78a, a78b, a81, a82a, a82b, a83a, a83b, a84, a85, a91, a92a, a92b, a93a, a93b, b1, b2, b3, b4, b5, b20, b21, b22, b23, b24, b25, b26, b30, b31, b32, b33, b34, b35, b36, b40, b41, b42, b43, b44, b45, b46, b50, b51, b52, b53, b54, b55, c1, c2, c3, c21, c22a, c22b, c23a, c23b, c24, c31, c32, c33, c34: line
a101: continuous multi-stage distillation column
a102, a401, a701, a801: thermal decomposition device
a103, a402, a702: separation column
a104, a403, a703: purification column
a111, a112, a113, a211, a411, a412, a413, a611, a711, a712, a811, c211: reboiler
a121, a122, a123, a221, a321, a421, a422, a423, a621, a721, a722, a821, a921, b121, b221, b321, b421, b521, c221, c321: condenser
a202, a203, a302, a303, a502, a602, a603, a802, a803, a902, a903, b102, b202, b303, b402, b504, c102, c103, c202, c203: storage tank
a301, a901, b502, c302: thin film evaporator
a501, b301: stirring tank
b101, b201: pressure-resistant reactor
b131, b231, b331, b431, b432, b531: storage valve
b241: pump
b302: filled column
b401: extruder
b451: vent opening
c101: storage tank with jacket
c201: separation column
b501, c301: heating and evaporation surface
b503, c303: collection portion
11a, 12a, 13a, 14a, 15a, 16, 17, 21, 22, 23, 24, 25, 26, 27, 28, 31, 32, 33, 34, 35, 36, 41, 42, 43: line
X11, X21, X31, X41: condenser
X32: reboiler
101a, 102a, 103a, 106, 107, 202, 204, 205, 302, 303, 402, 403: storage tank
104a, 105a, 201: SUS reaction container with baffle
203, 301: multi-stage distillation column
401: thin film distillation device

**Claims**

**1.** A method for producing an isocyanate compound, comprising:

a step (1) of reacting a primary amine compound with a carbonic acid derivative to obtain an N-substituted carbamate compound while extracting a by-product compound having a boiling point lower than a boiling point of the N-substituted carbamate compound;
a step (2) of performing thermal decomposition of the N-substituted carbamate compound on a reaction solution containing the N-substituted carbamate compound, which is obtained in the step (1), in the presence of an aprotic solvent to obtain an isocyanate compound while extracting a by-product hydroxy compound;
a step (3) of separating the isocyanate compound and the aprotic solvent from a reaction solution containing the isocyanate compound, which is obtained in the step (2); and
a step (4) of removing a component having a boiling point higher than a boiling point of the isocyanate compound from a fraction containing the isocyanate compound obtained in the step (3) to purify the isocyanate compound.

**2.** The method for producing an isocyanate compound according to Claim 1, further comprising:
a step (5) of hydrolyzing a fraction containing the aprotic solvent separated in the step (3) or a component having a boiling point higher than the boiling point of the isocyanate compound removed in the step (4) in the presence of an alkali and water to obtain the primary amine compound and the hydroxy compound.

**3.** The method for producing an isocyanate compound according to Claim 1 or 2,

wherein, in the step (1), the primary amine compound is reacted with urea or a urea derivative as the carbonic acid derivative in the presence of a hydroxy compound to obtain the N-substituted carbamate compound while extracting the by-product compound having a boiling point lower than the boiling point of the N-substituted carbamate compound, or
the primary amine compound is reacted with a carbonic acid ester as the carbonic acid derivative to obtain the N-substituted carbamate compound while extracting a hydroxy compound as the by-product compound having a boiling point lower than the boiling point of the N-substituted carbamate compound.

**4.** The method for producing an isocyanate compound according to Claim 1 or 2,
wherein, in the step (2), a liquid phase containing the isocyanate compound is obtained while extracting the by-product hydroxy compound into a gas phase.

**5.** The method for producing an isocyanate compound according to Claim 1 or 2,
wherein the step (1) and the step (2) are performed using one or more reactors selected from the group consisting of a tank-type reactor, a distillation column, a tubular evaporator, a thin film evaporator, and a falling film-type evaporator.

**6.** The method for producing an isocyanate compound according to Claim 1 or 2,
wherein at least one of the step (1) or the step (2) is performed by a reactive distillation method.

**7.** The method for producing an isocyanate compound according to Claim 1 or 2,
wherein the hydroxy compound extracted in the step (2) is re-used by circulating the hydroxy compound to the step (1).

**8.** The method for producing an isocyanate compound according to Claim 1 or 2,
wherein the aprotic solvent separated in the step (3) is re-used by circulating the aprotic solvent to the step (2).

**9.** The method for producing an isocyanate compound according to Claim 1 or 2, wherein the aprotic solvent is a carbonic acid ester.

**10.** The method for producing an isocyanate compound according to Claim 1 or 2,

wherein, in the step (4), the isocyanate compound is purified in the presence of a carbonyl compound represented by General Formula (I) in an amount of 1 ppm by mass or more and 50% by mass or less with respect to a mass of the fraction containing the isocyanate compound,

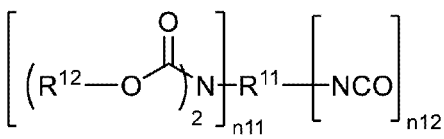

(I)

(in General Formula (1), $R^{11}$ is an (n1 1 + n1 2)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less).

**11.** The method for producing an isocyanate compound according to Claim 1 or 2,

wherein the primary amine compound is a compound represented by General Formula (II),

$$R^{21}\left(-NH_2\right)_{n21} \qquad (II)$$

(in General Formula (II), $R^{21}$ is an n21-valent organic group, and n21 is an integer of 2 or more).

**12.** The method for producing an isocyanate compound according to Claim 11, wherein n21 is 3 or more.

**13.** A method for producing a carbamate compound, comprising:
a reaction step of reacting a primary amine compound, a carbonic acid derivative, and a hydroxy compound in the presence of, as a catalyst, an amine compound having no active hydrogen to obtain a carbamate compound.

**14.** The method for producing a carbamate compound according to Claim 13,
wherein the amine compound having no active hydrogen has one or more functional groups selected from the group consisting of a tertiary amino group, a nitrogen-containing aromatic group, an amidine group, and a guanidine group.

**15.** The method for producing a carbamate compound according to Claim 14,
wherein the amine compound having no active hydrogen is a compound having 1 or more tertiary amino groups and having 3 or more and 85 or less carbon atoms.

**16.** The method for producing a carbamate compound according to Claim 15,
wherein the amine compound having no active hydrogen is a compound having 1 or more and 6 or less tertiary amino groups and having 3 or more and 30 or less carbon atoms.

**17.** The method for producing a carbamate compound according to Claim 16,
wherein the amine compound having no active hydrogen is N,N'-dimethylaniline, N,N'-diethylaniline, N-methyl-N'-ethylaniline, N,N'-dimethylaminopyridine, N,N,N',N'-tetramethylphenylenediamine, methylenebis(N,N'-dimethylaniline), triethylamine, ethyldiisopropylamine, N-methylmorpholine, N-methylpiperidine, quinuclidine, N,N'-dimethylpiperazine, triethylenediamine, N,N,N',N'-tetramethylethylenediamine, N,N,N',N'-tetramethylhexanediamine, N,N,N',N'-tetramethylxylylenediamine, pentamethyldiethylenetriamine, bis(2-morpholinoethyl)ether, hexahydro-1,3,5-tris(3-dimethylaminopropyl)-1,3,5-triazine, or hexamethylenetetramine.

**18.** The method for producing a carbamate compound according to Claim 14,
wherein the amine compound having no active hydrogen is a compound having 1 or more nitrogen-containing aromatic groups and having 3 or more and 85 or less carbon atoms.

**19.** The method for producing a carbamate compound according to Claim 18,
wherein the nitrogen-containing aromatic group is a substituted or unsubstituted pyridyl group, imidazolyl group, pyrazolyl group, quinolyl group, isoquinolyl group, oxazolyl group, thiazolyl group, pyridazyl group, pyrimidyl group, or pyrazyl group.

**20.** The method for producing a carbamate compound according to Claim 19,
wherein the amine compound having no active hydrogen is pyridine, picoline, lutidine, collidine, 1-methylimidazole, 1-methylpyrazole, quinoline, isoquinoline, methylquinoline, oxazole, thiazole, pyridazine, pyrimidine, or pyrazine.

**21.** The method for producing a carbamate compound according to Claim 14,

wherein the amine compound having no active hydrogen is an amidine group-containing compound represented by General Formula (VIII-1),

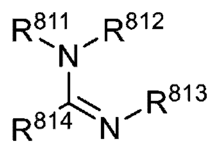

( VIII-1 )

(in General Formula (VIII-1), $R^{811}$, $R^{812}$, $R^{813}$, and $R^{814}$ are each independently a monovalent organic group, $R^{811}$ and $R^{812}$, $R^{812}$ and $R^{813}$, $R^{813}$ and $R^{814}$, or $R^{814}$ and $R^{811}$ may be each independently bonded to each other to form a ring structure, and a total number of carbon atoms in $R^{811}$, $R^{812}$, $R^{813}$, and $R^{814}$ is 5 or more and 85 or less).

**22.** The method for producing a carbamate compound according to Claim 21,

wherein the amidine group-containing compound has one or more ring structures, and
the total number of carbon atoms in $R^{811}$, $R^{812}$, $R^{813}$, and $R^{814}$ is 5 or more and 30 or less.

**23.** The method for producing a carbamate compound according to Claim 22,
wherein the amidine group-containing compound is 1,2-dimethyl-1,4,5,6-tetrahydropyrimidine, 1,8-diazabicyclo-[5.4.0]undeca-7-ene, or 1,5-diazabicyclo-[4.3.0]nona-5-ene.

**24.** The method for producing a carbamate compound according to Claim 14,

wherein the amine compound having no active hydrogen is a guanidine group-containing compound represented by General Formula (VIII-2),

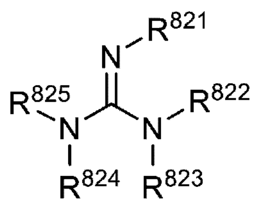

( VIII-2 )

(in General Formula (VIII-2), $R^{821}$, $R^{822}$, $R^{823}$, $R^{824}$, and $R^{825}$ are each independently a monovalent organic group, $R^{821}$ and $R^{822}$, $R^{822}$ and $R^{823}$, $R^{823}$ and $R^{824}$, $R^{824}$ and $R^{825}$, or $R^{825}$ and $R^{821}$ may be each independently bonded to each other to form a ring structure, and a total number of carbon atoms in $R^{821}$, $R^{822}$, $R^{823}$, $R^{824}$, and $R^{825}$ is 5 or more and 85 or less).

**25.** The method for producing a carbamate compound according to Claim 24,
wherein the total number of carbon atoms in $R^{821}$, $R^{822}$, $R^{823}$, $R^{824}$, and $R^{825}$ is 5 or more and 30 or less.

**26.** The method for producing a carbamate compound according to Claim 25,
wherein the guanidine group-containing compound is pentamethylguanidine or 7-methyl-1,5,7-triazabicyclo[4.4.0]deca-5-ene.

**27.** The method for producing a carbamate compound according to Claim 13,
wherein the primary amine compound is an aliphatic or aromatic primary polyamine compound.

**28.** The method for producing a carbamate compound according to Claim 13,
wherein the hydroxy compound is an aromatic hydroxy compound.

**29.** A method for producing an isocyanate compound, comprising:
a thermal decomposition step of performing thermal decomposition on a carbamate compound obtained by the method for producing a carbamate compound according to any one of Claims 13 to 28.

**30.** A method for recovering an amine compound represented by General Formula (IIa) from a liquid-phase component containing a compound having a higher boiling point than a boiling point of an isocyanate compound which is a by-product in a method for producing an isocyanate compound represented by General Formula (VIIa), the recovery

method comprising:

a step (a) of reacting the liquid-phase component, an aromatic hydroxy compound, an active hydrogen-containing compound, and a catalyst in a reactor to obtain a reaction solution containing the amine compound represented by General Formula (IIa),

$$R^{71a}\left(-NCO\right)_{n71a} \qquad (VIIa)$$

(in General Formula (VIIa), $R^{71a}$ is an n71a-valent organic group, and n71a is an integer of 2 or more and 8 or less),

$$R^{21a}\left(-NH_2\right)_{n21a} \qquad (IIa)$$

(in General Formula (IIa), $R^{21a}$ is an n21a-valent organic group, and a relational expression of $R^{21a} = R^{71a}$ is satisfied, and n21a is an integer of 2 or more and 8 or less, and a relational expression of n21a = n71a is satisfied).

**31.** The recovery method according to Claim 30,

wherein the step (a) includes the following step (a1) and step (a2),
a step (a1) of mixing the liquid-phase component with the aromatic hydroxy compound, and
a step (a2) of reacting the mixture obtained in the step (a1), the active hydrogen-containing compound, and the catalyst in the reactor to obtain the reaction solution containing the amine compound represented by General Formula (IIa).

**32.** The recovery method according to Claim 31,

wherein the step (a2) includes the following step (a2-1) and step (a2-2),
a step (a2-1) of mixing the mixture obtained in the step (a1), an amine compound as the active hydrogen-containing compound, and the catalyst, and
a step (a2-2) of reacting the mixture obtained in the step (a2-1) with water as the active hydrogen-containing compound in the reactor to obtain the reaction solution containing the amine compound represented by General Formula (IIa).

**33.** The recovery method according to any one of Claims 30 to 32,
wherein the active hydrogen-containing compound is water.

**34.** The recovery method according to any one of Claims 30 to 32,
wherein the active hydrogen-containing compound is water and a primary amine compound.

**35.** The recovery method according to Claim 34,
wherein the primary amine compound is the amine compound represented by General Formula (IIa).

**36.** The recovery method according to any one of Claims 30 to 32,
wherein, in the method for producing the isocyanate compound represented by General Formula (VIIa), a carbonic acid derivative, a hydroxy compound, and the amine compound represented by General Formula (IIa) are used as raw materials.

**37.** The recovery method according to any one of Claims 30 to 32,
wherein the liquid-phase component is a liquid-phase component which is obtained by subjecting a reaction solution containing a carbamate compound produced from a carbonic acid derivative, a hydroxy compound, and the amine compound represented by General Formula (IIa) to a thermal decomposition reaction, supplying a composition containing the produced isocyanate compound represented by General Formula (VIIa) to a distillation device, extracting the liquid-phase component from the distillation device when separating the isocyanate compound represented by General Formula (VIIa) as a gas-phase component.

**38.** The recovery method according to any one of Claims 30 to 32,

wherein the liquid-phase component includes a compound having one or more functional groups selected from the group consisting of a group represented by Formula (IX-1), a group represented by Formula (IX-2), a group represented by Formula (IX-3), a group represented by Formula (IX-4), a group represented by Formula (IX-5), a group represented by Formula (IX-6), a group represented by Formula (IX-7), a group represented by Formula (IX-8), a group represented by Formula (IX-9), a group represented by Formula (IX-10), a group represented by Formula (IX-11), and a group represented by Formula (IX-12),

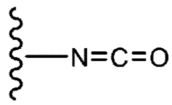

(IX-1)

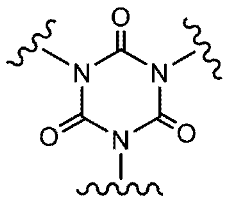

(IX-2)

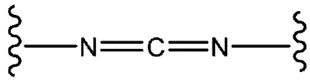

(IX-3)

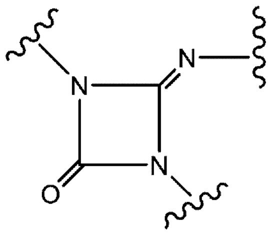

(IX-4)

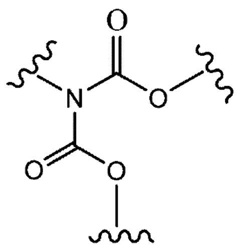

(IX-5)

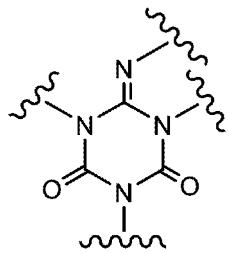

(IX-6)

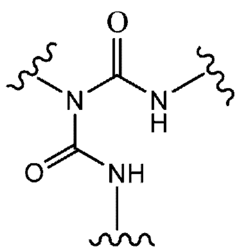

(IX-7)

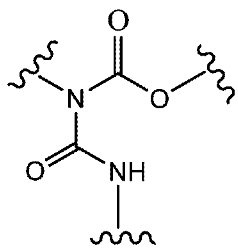

(IX-8)

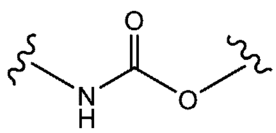

(IX-9)

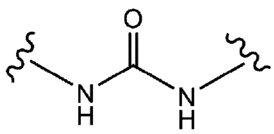

(IX-10)

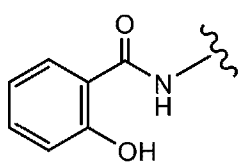

(IX-11)

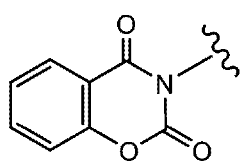

(IX-12)

(in Formulae (IX-1) to (IX-12), a wavy line represents a bonding site).

**39.** The recovery method according to Claim 38,
wherein the liquid-phase component includes a compound having two or more functional groups selected from the group consisting of the group represented by Formula (IX-1), the group represented by Formula (IX-2), the group represented by Formula (IX-3), the group represented by Formula (IX-4), the group represented by Formula (IX-5), the group represented by Formula (IX-6), the group represented by Formula (IX-7), the group represented by Formula (IX-8), the group represented by Formula (IX-9), the group represented by Formula (IX-10), the group represented by Formula (IX-11), and the group represented by Formula (IX-12).

**40.** The recovery method according to any one of Claims 30 to 32,
wherein the catalyst is one or more compounds selected from the group consisting of a hydroxide or an oxide of an alkali metal, a hydroxide or an oxide of an alkaline earth metal, a tertiary amine compound, a metal oxide of Group 12, a metal oxide of Group 13, and a metal oxide of Group 14.

**41.** The recovery method according to Claim 40,
wherein the catalyst is one or more compounds selected from the group consisting of a hydroxide or an oxide of an alkali metal, and a hydroxide or an oxide of an alkaline earth metal.

**42.** The recovery method according to Claim 41,
wherein the catalyst is a hydroxide or an oxide of an alkali metal.

**43.** The recovery method according to any one of Claims 30 to 32, further comprising:

the following step (b); and
the following step (c),
a step (b) of separating the amine compound represented by General Formula (IIa) from the reaction solution containing the amine compound represented by General Formula (IIa), and
a step (c) of purifying the amine compound represented by General Formula (IIa).

**44.** The recovery method according to Claim 43, further comprising:

the following step (d),
a step (d) of re-using the amine compound represented by General Formula (IIa), which has been purified in the step (c), as a raw material in the method for producing the isocyanate compound represented by General Formula (VIIa).

**45.** The recovery method according to any one of Claims 30 to 32, further comprising:

the following step (e); and
the following step (f),
a step (e) of separating a hydroxy compound from the reaction solution containing the amine compound represented by General Formula (IIa), and
a step (f) of purifying the hydroxy compound.

**46.** The recovery method according to Claim 45, further comprising:

the following step (g),
a step (g) of re-using the hydroxy compound purified in the step (f) as a raw material in the method for producing the isocyanate compound represented by General Formula (VIIa).

**47.** The recovery method according to Claim 45, further comprising:

the following step (h),
a step (h) of re-using, in the step (a), one or more residual liquids selected from the group consisting of a residual liquid after separating the amine compound represented by General Formula (IIa) in the step (b), a residual liquid after purifying the amine compound represented by General Formula (IIa) in the step (c), a residual liquid after separating the hydroxy compound in the step (e), and a residual liquid after purifying the hydroxy compound

in the step (f).

**48.** An isocyanate composition comprising:

an isocyanate compound;
a carbonyl compound represented by General Formula (I); and
a modified product of an isocyanate compound, in which a part of an isocyanate group of the isocyanate compound is converted into one or more functional groups selected from the group consisting of an isocyanurate group, a carbodiimide group, a uretonimine group, and an allophanate group,
wherein the isocyanate compound is a different compound from the carbonyl compound, and
a value of {3 × (a molar amount of the isocyanurate group) + 2 × (a molar amount of the carbodiimide group) + 3 × (a molar amount of the uretonimine group) + 2 × (a molar amount of the allophanate group)} ÷ (a molar amount of the carbonyl compound) is 0.001 or more and 8.0 or less,

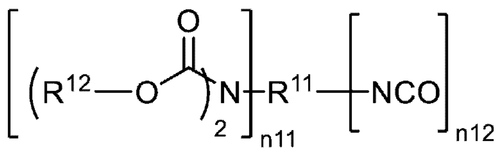

( I )

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less).

**49.** The isocyanate composition according to Claim 48,

wherein $R^{11}$ is a di- or higher and tetra- or lower valent aliphatic hydrocarbon group having 1 or more and 20 or less carbon atoms or a divalent or trivalent aromatic hydrocarbon group having 6 or more and 20 or less carbon atoms, each of which may have 1 or more and 4 or less ester groups or a nitrogen atom, and
$R^{12}$ is a monovalent aromatic hydrocarbon group having 6 or more and 20 or less carbon atoms, which may have an oxygen atom.

**50.** The isocyanate composition according to Claim 48 or 49,

wherein the isocyanate compound is a compound represented by General Formula (VIIb),

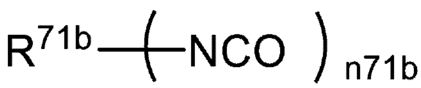

( VIIb )

(in General Formula (VIIb), $R^{71b}$ is an n71b-valent organic group, and a relational expression of $R^{71b} = R^{11}$ is satisfied, and n71b is an integer of 2 or more and 8 or less, and a relational expression of n71b = n11 + n12 is satisfied).

**51.** The isocyanate composition according to Claim 48 or 49,

wherein the isocyanurate group is a group represented by General Formula (X-1), the carbodiimide group is a group represented by General Formula (X-2), the uretonimine group is a group represented by General Formula (X-3), and the allophanate group is a group represented by General Formula (X-4).

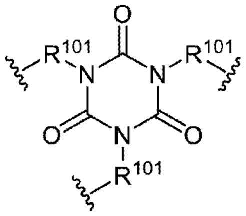
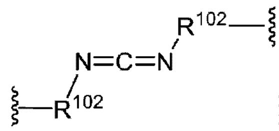
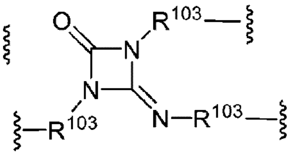
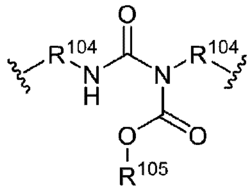

(X-1) (X-2) (X-3) (X-4)

(in General Formulae (X-1) to (X-4), $R^{101}$, $R^{102}$, $R^{103}$, and $R^{104}$ are each independently a di- or higher and octa- or lower valent organic group, and relational expressions of $R^{101} = R^{71b}$, $R^{102} = R^{71b}$, $R^{103} = R^{71b}$, and $R^{104} = R^{71b}$ are satisfied, $R^{105}$ is a monovalent organic group, and a relational expression of $R^{105} = R^{12}$ is satisfied, and a wavy line represents a bonding site).

**52.** The isocyanate composition according to Claim 48 or 49,

wherein a content of the isocyanate compound is 1 % by mass or more and 99% by mass or less with respect to a total mass of the isocyanate composition, and
a total content of the carbonyl compound and the modified product of the isocyanate compound is 1% by mass or more and 99% by mass or less with respect to the total mass of the isocyanate composition.

**53.** The isocyanate composition according to Claim 48 or 49, further comprising:

one or more compounds selected from the group consisting of a carbamate group-containing compound represented by General Formula (XI) and a carbonic acid ester represented by General Formula (III-1a), each of which is contained in an amount of 2.0 ppm by mass or more and 99% by mass or less with respect to a total mass of the isocyanate composition,

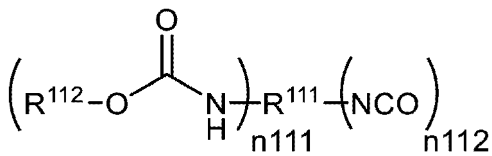

( XI )

(in General Formula (XI), $R^{111}$ is an (n111 + n112)-valent organic group, and a relational expression of $R^{111} = R^{11}$ is satisfied, $R^{112}$ is a monovalent organic group, and a relational expression of $R^{112} = R^{12}$ is satisfied, n111 is an integer of 1 or more and 8 or less, n112 is an integer of 0 or more and 7 or less, a sum of n111 and n 112 is an integer of 2 or more and 8 or less, and a relational expression of n111 + n112 = n11 + n12 is satisfied),

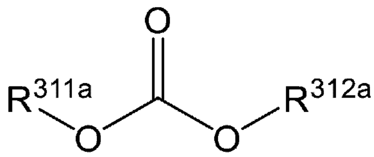

( III-1a )

(in General Formula (III-1a), $R^{311a}$ and $R^{312a}$ are each independently a monovalent organic group, and a relational expression of $R^{311a} = R^{312a} = R^{12}$ is satisfied).

**54.** The isocyanate composition according to Claim 53, further comprising:
one or more compounds selected from the group consisting of the carbamate group-containing compound represented by General Formula (XI) and the carbonic acid ester represented by General Formula (III-1a), each of which is contained in an amount of 2.0 ppm by mass or more and $1.0 \times 10^4$ ppm by mass or less with respect to the total mass of the isocyanate composition.

**55.** A method for producing an isocyanate compound, comprising:
distilling and purifying the isocyanate composition according to Claim 48 or 49 to continuously recover the isocyanate compound as a gas-phase component.

FIG. 1

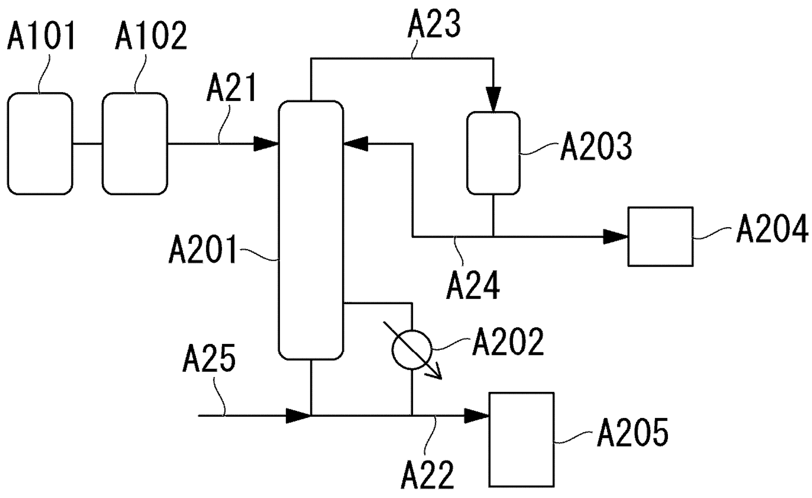


FIG. 2

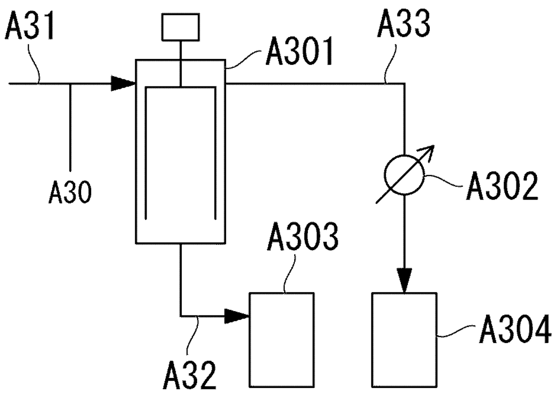


FIG. 3

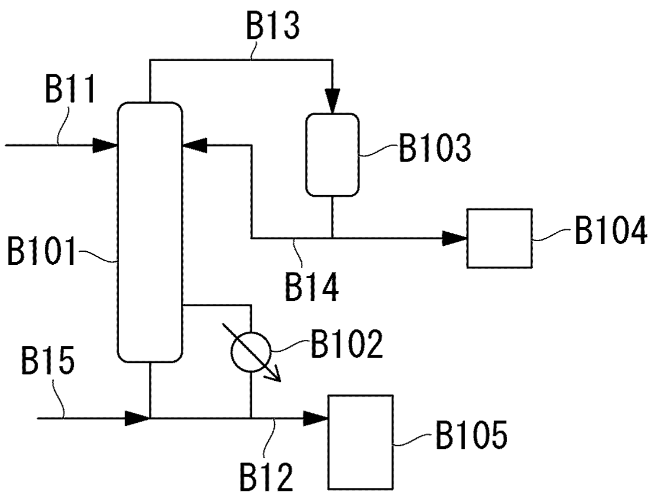

FIG. 4

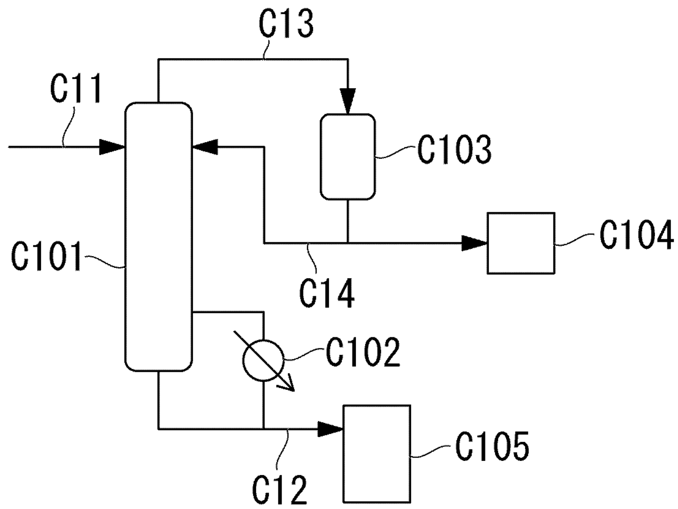
C13
C11
C103
C104
C101
C14
C102
C105
C12

FIG. 5

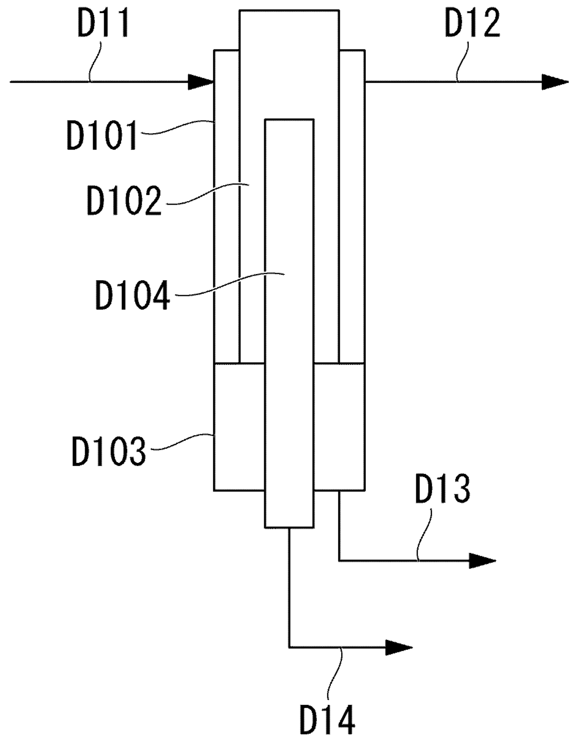
D11
D12
D101
D102
D104
D103
D13
D14

FIG. 6

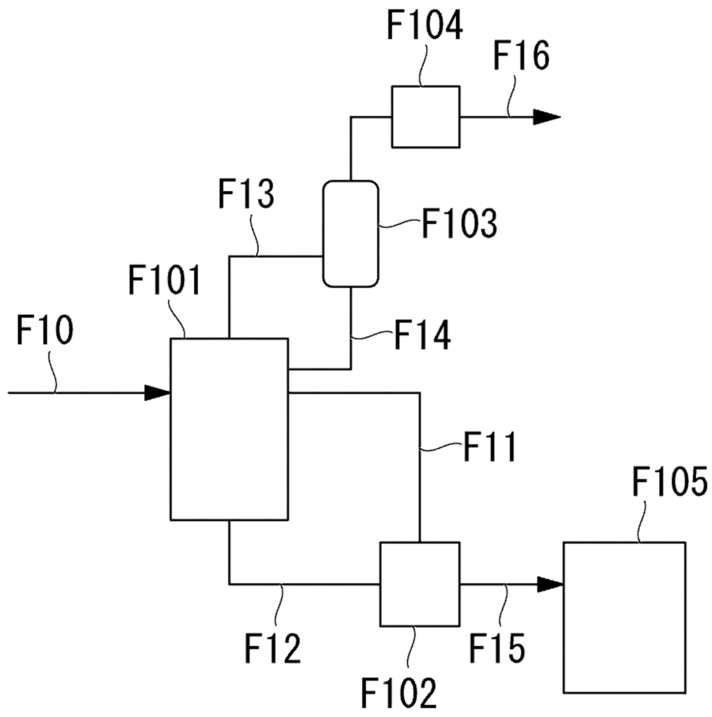
F104
F16
F13
F103
F101
F10
F14
F11
F105
F12
F102
F15

FIG. 7

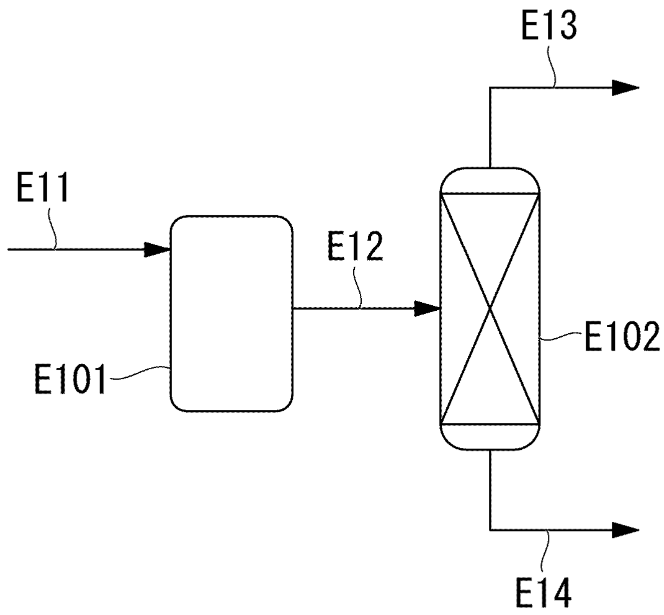
E13
E11
E12
E102
E101
E14

FIG. 8

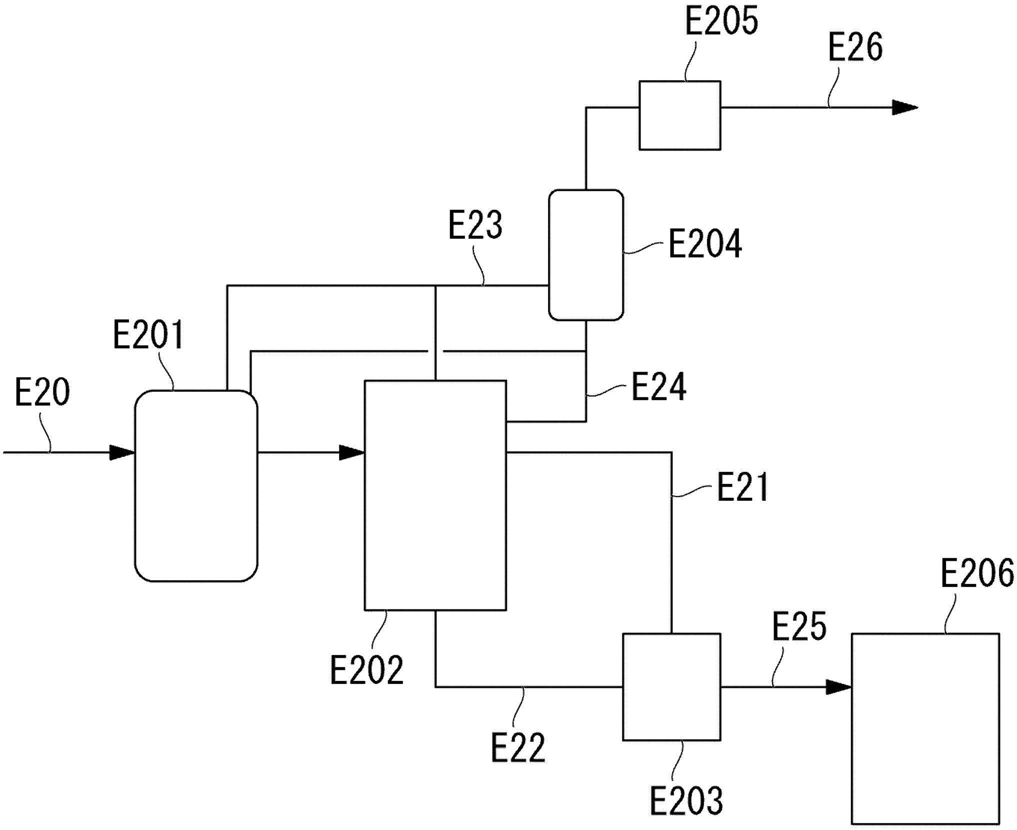
E205
E26
E23
E204
E201
E20
E24
E21
E206
E25
E202
E22
E203

FIG. 9

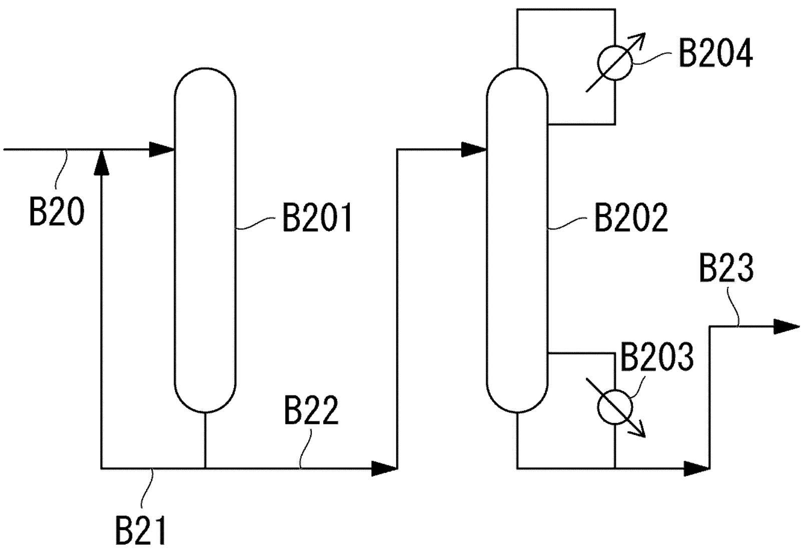
B204
B20
B201
B202
B23
B203
B22
B21

FIG. 10

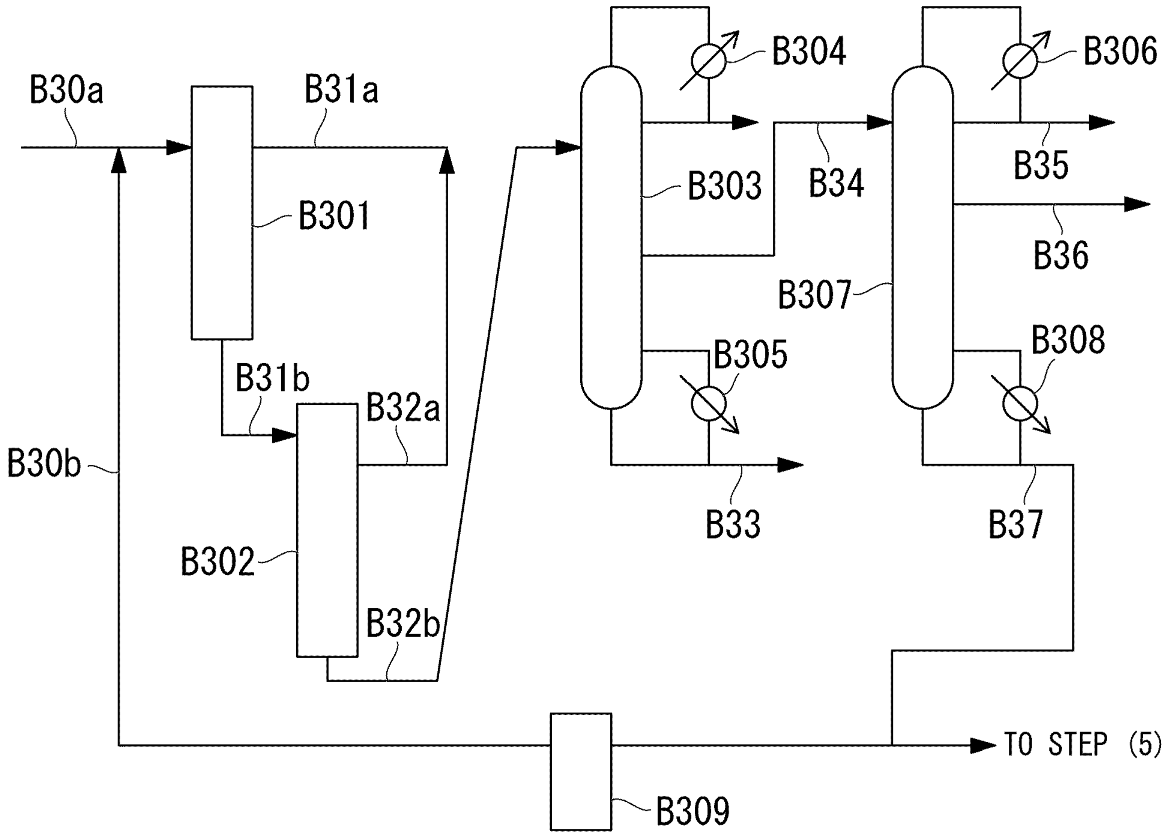
B30a
B31a
B301
B304
B303
B34
B306
B35
B36
B307
B305
B308
B31b
B32a
B30b
B33
B37
B302
B32b
TO STEP (5)
B309

FIG. 11

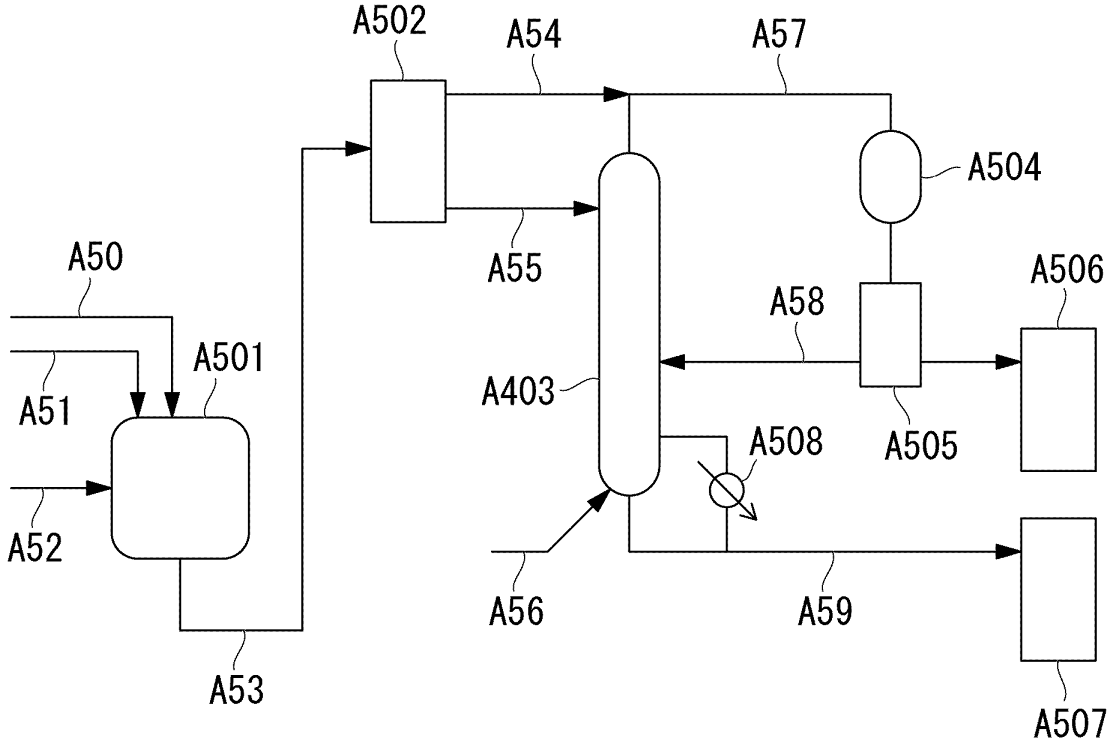
A502
A54
A57
A504
A55
A50
A58
A506
A501
A51
A403
A508
A505
A52
A56
A59
A53
A507

FIG. 12

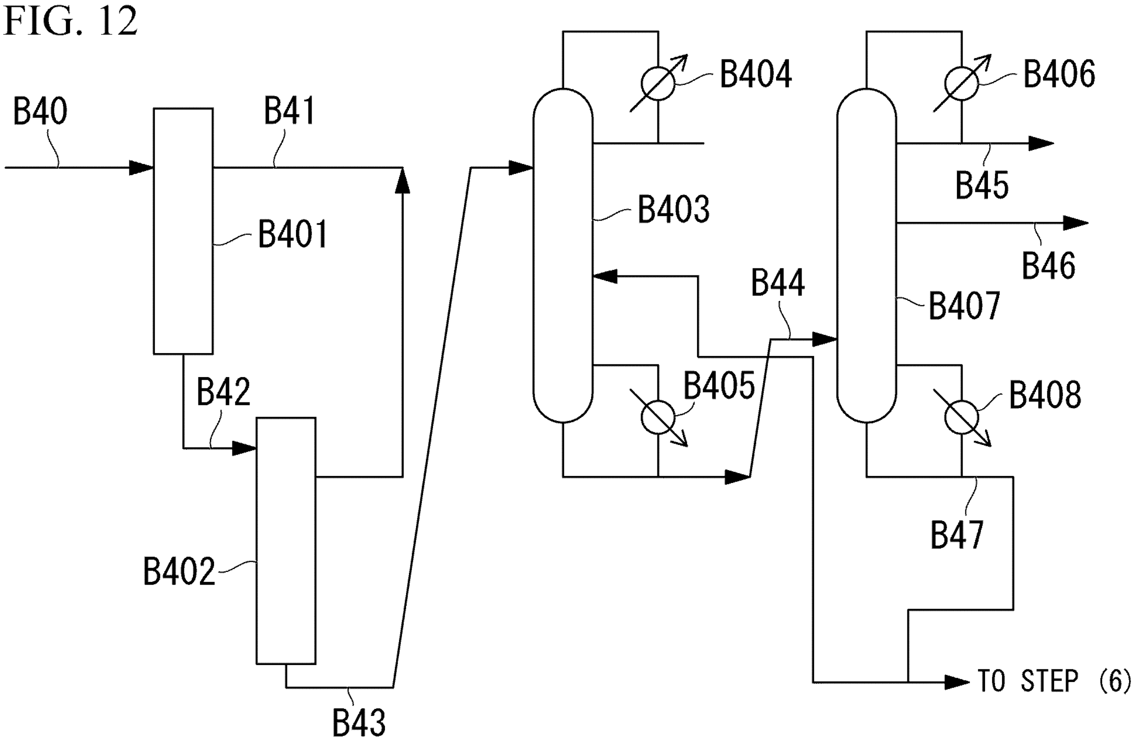


FIG. 13

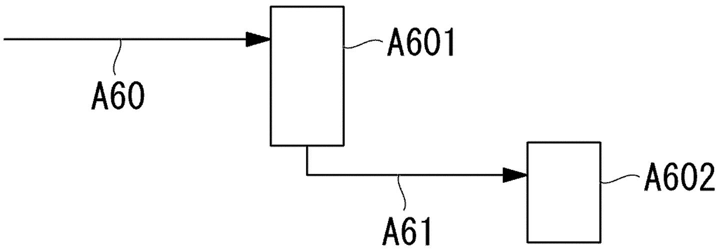


FIG. 14

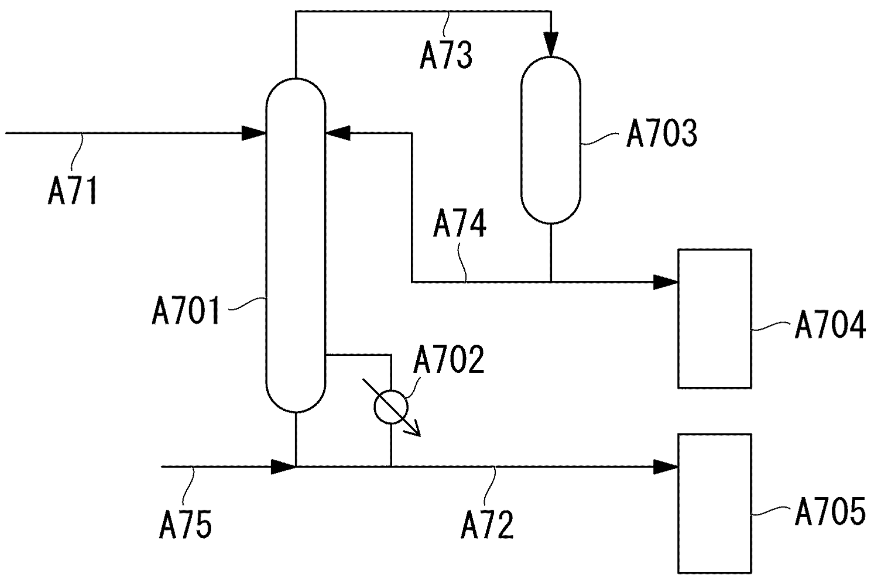

FIG. 15

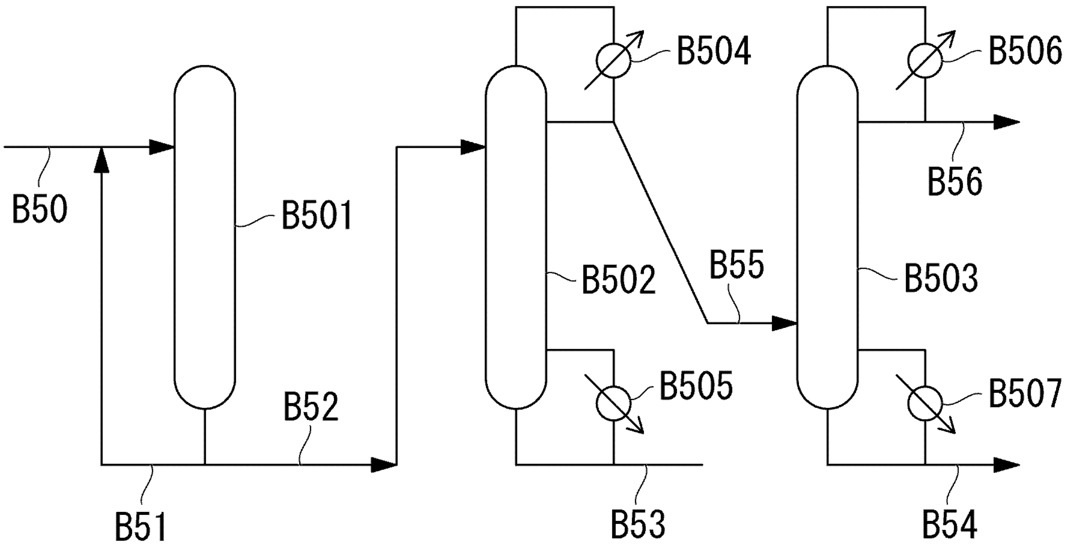
B50
B501
B52
B51
B502
B504
B505
B53
B55
B503
B506
B56
B507
B54

FIG. 16

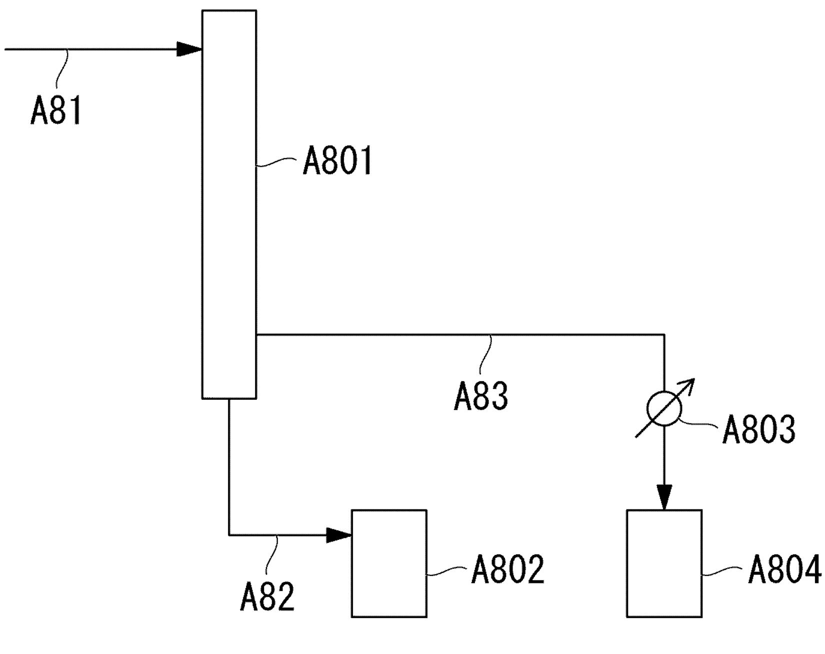
A81
A801
A83
A803
A82
A802
A804

FIG. 17

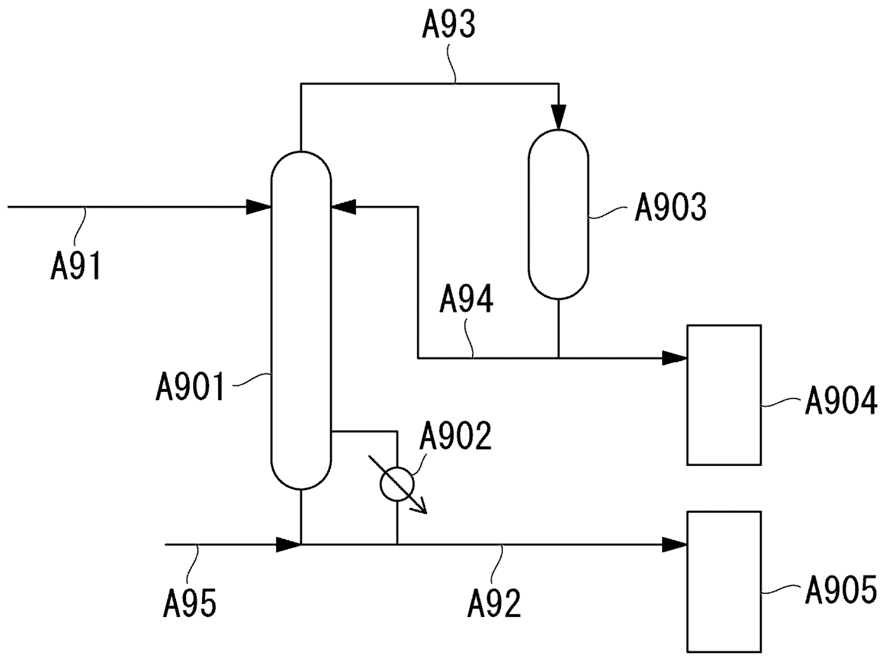
A93
A903
A91
A94
A901
A904
A902
A95
A92
A905

FIG. 18

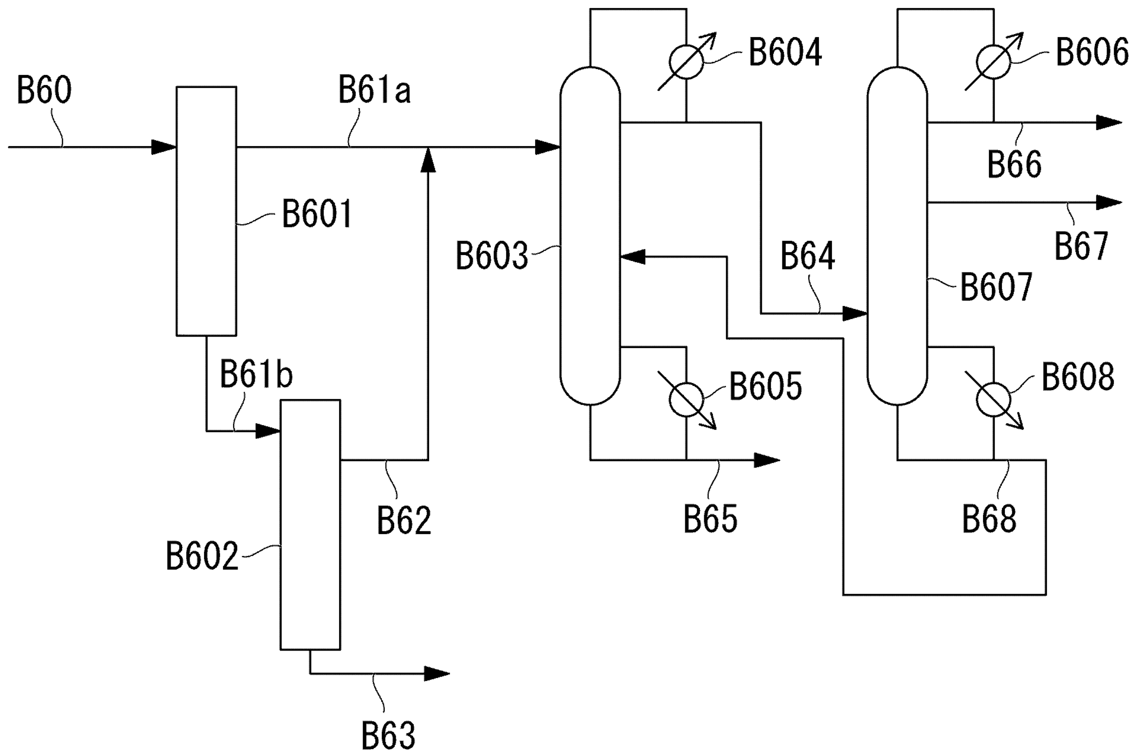
B604
B606
B60
B61a
B601
B66
B67
B603
B64
B607
B61b
B605
B608
B62
B65
B68
B602
B63

FIG. 19

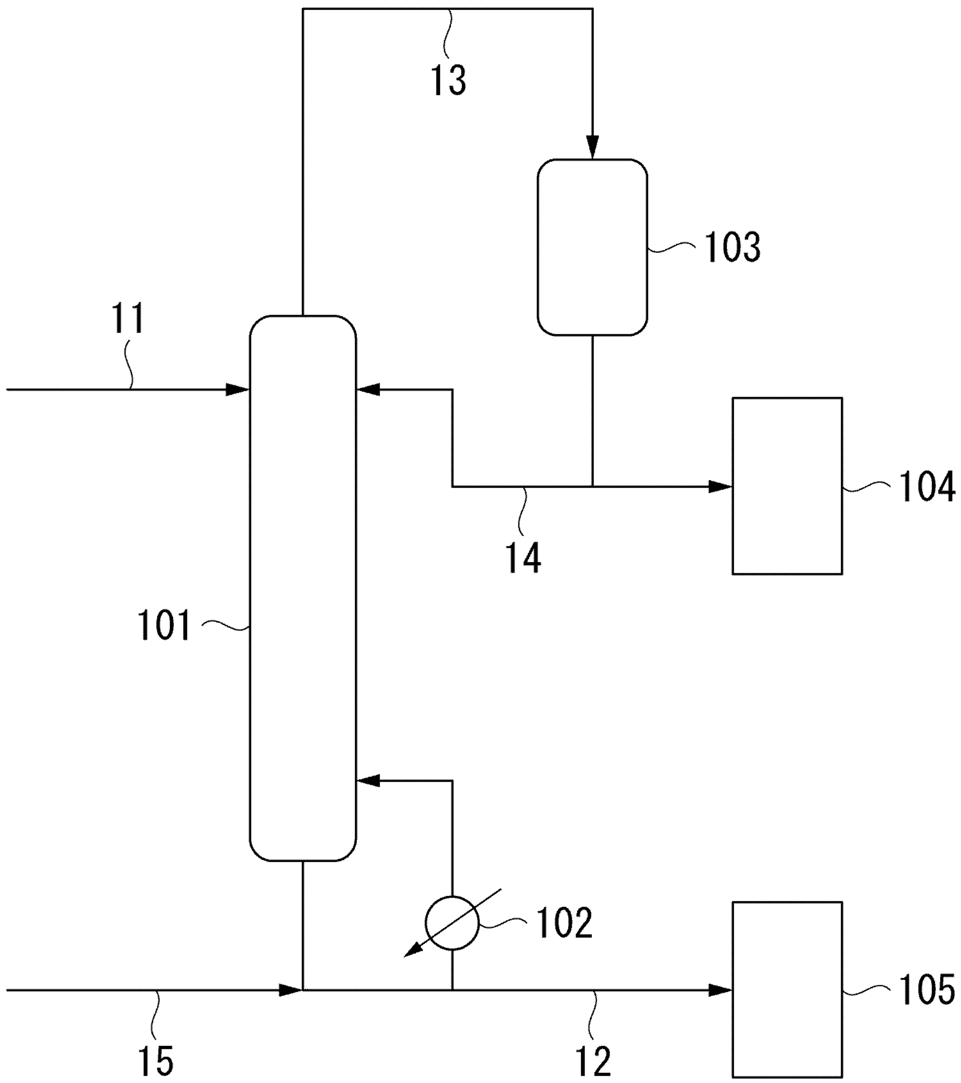

FIG. 20

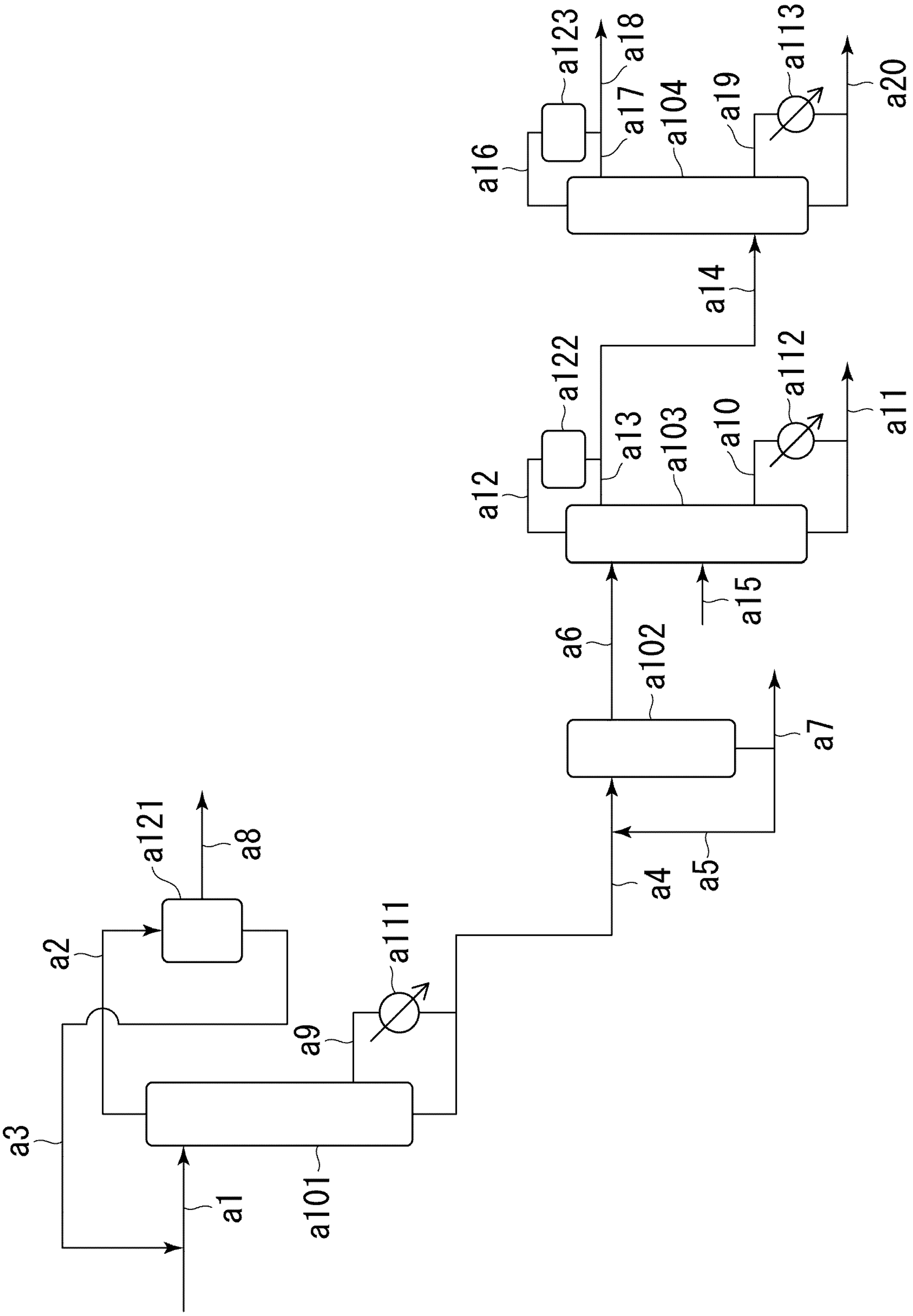
a1
a2
a3
a4
a5
a6
a7
a8
a9
a10
a11
a12
a13
a14
a15
a16
a17
a18
a19
a20
a101
a102
a103
a104
a111
a112
a113
a121
a122
a123

FIG. 21

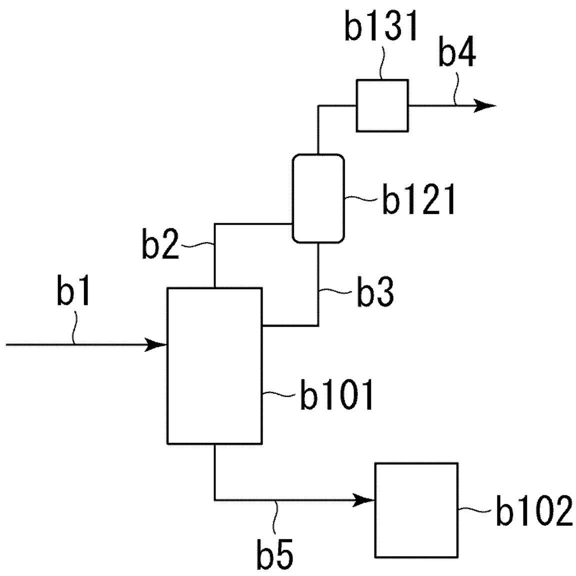


FIG. 22

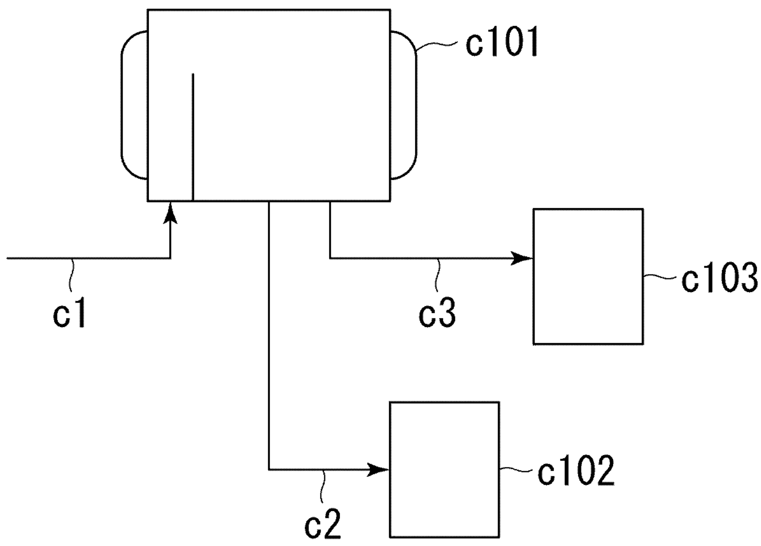

FIG. 23

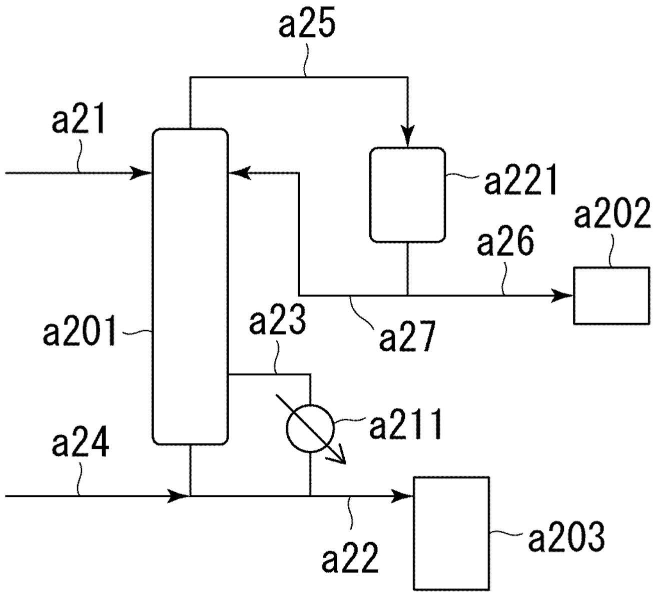
a25
a21
a221
a202
a26
a201
a23
a27
a211
a24
a22
a203

FIG. 24

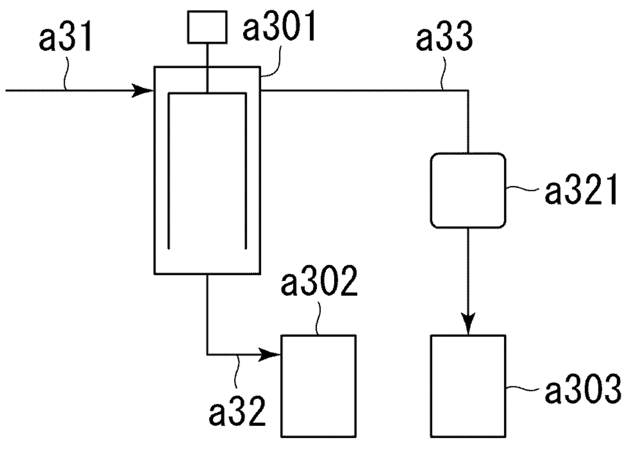
a31
a301
a33
a321
a302
a303
a32

FIG. 25

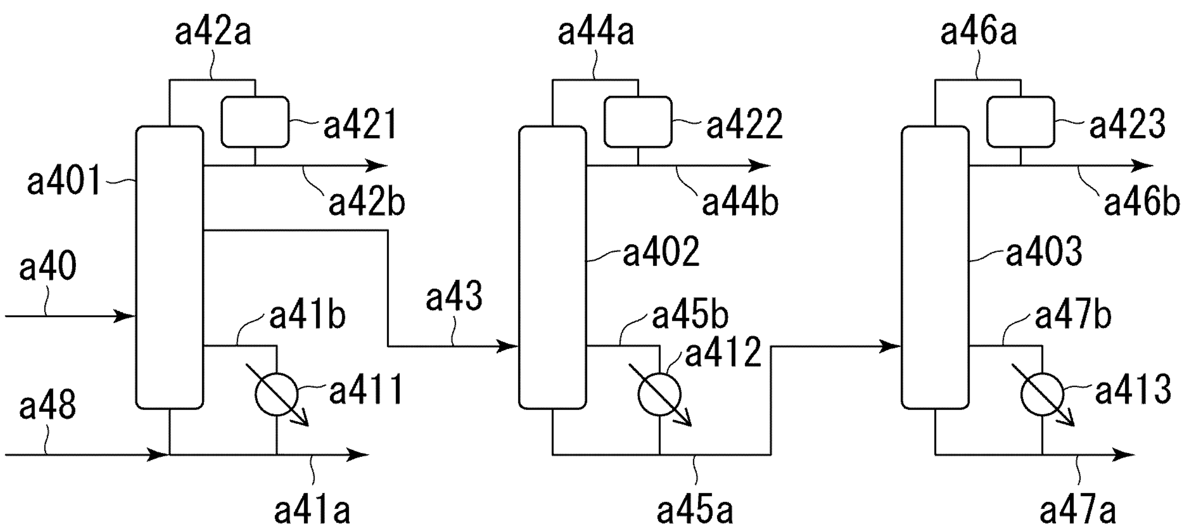
a42a
a421
a42b
a401
a40
a41b
a411
a48
a41a
a43
a44a
a422
a44b
a402
a45b
a412
a45a
a46a
a423
a46b
a403
a47b
a413
a47a

FIG. 26

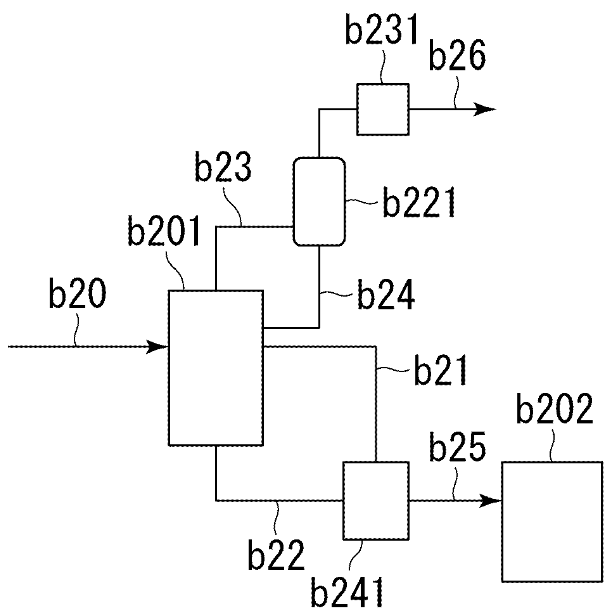
b231
b26
b23
b221
b201
b24
b20
b21
b202
b25
b22
b241

FIG. 27

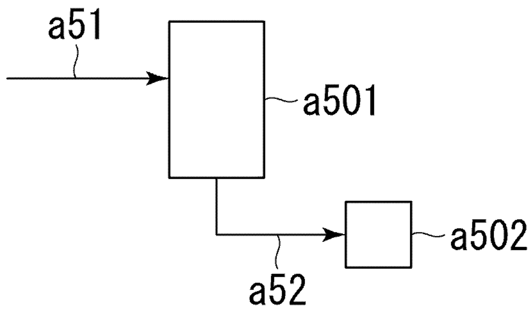
a51
a501
a502
a52

FIG. 28

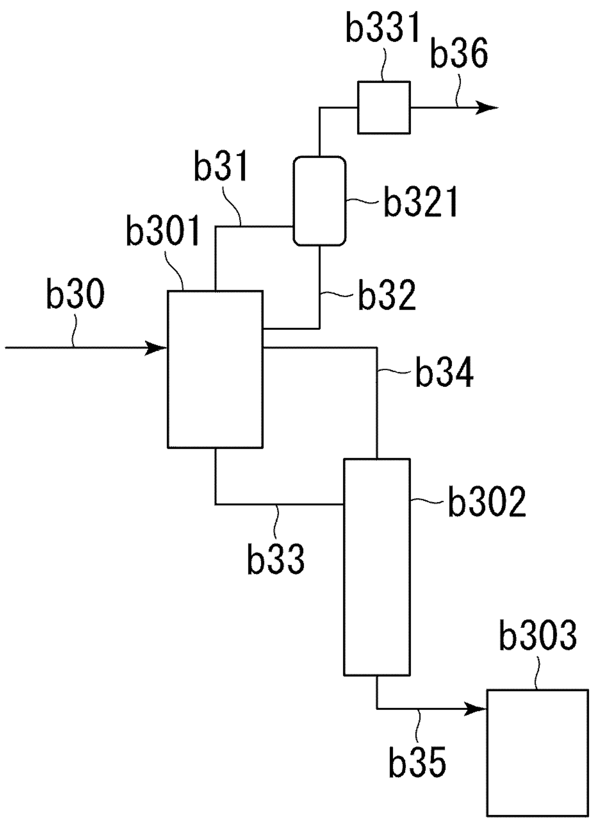
b331
b36
b31
b321
b301
b30
b32
b34
b302
b33
b303
b35

FIG. 29

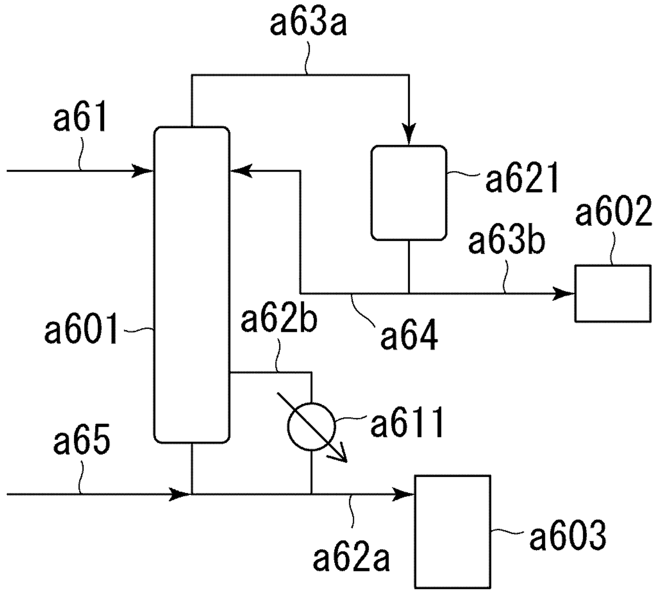
a63a
a61
a621
a602
a63b
a601
a62b
a64
a611
a65
a62a
a603

FIG. 30

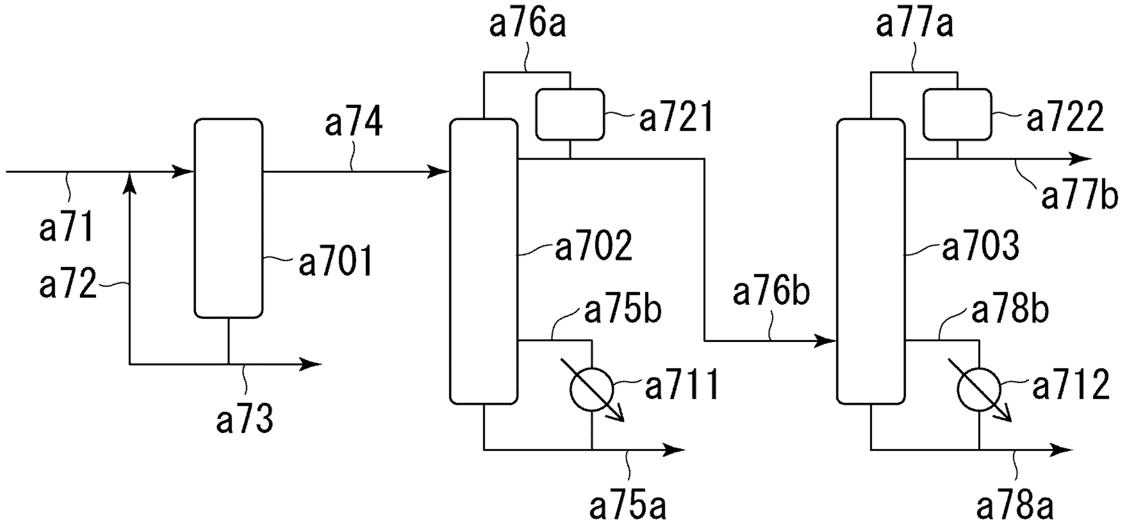
a76a
a77a
a74
a721
a722
a77b
a71
a701
a702
a703
a72
a76b
a75b
a78b
a711
a712
a73
a75a
a78a

FIG. 31

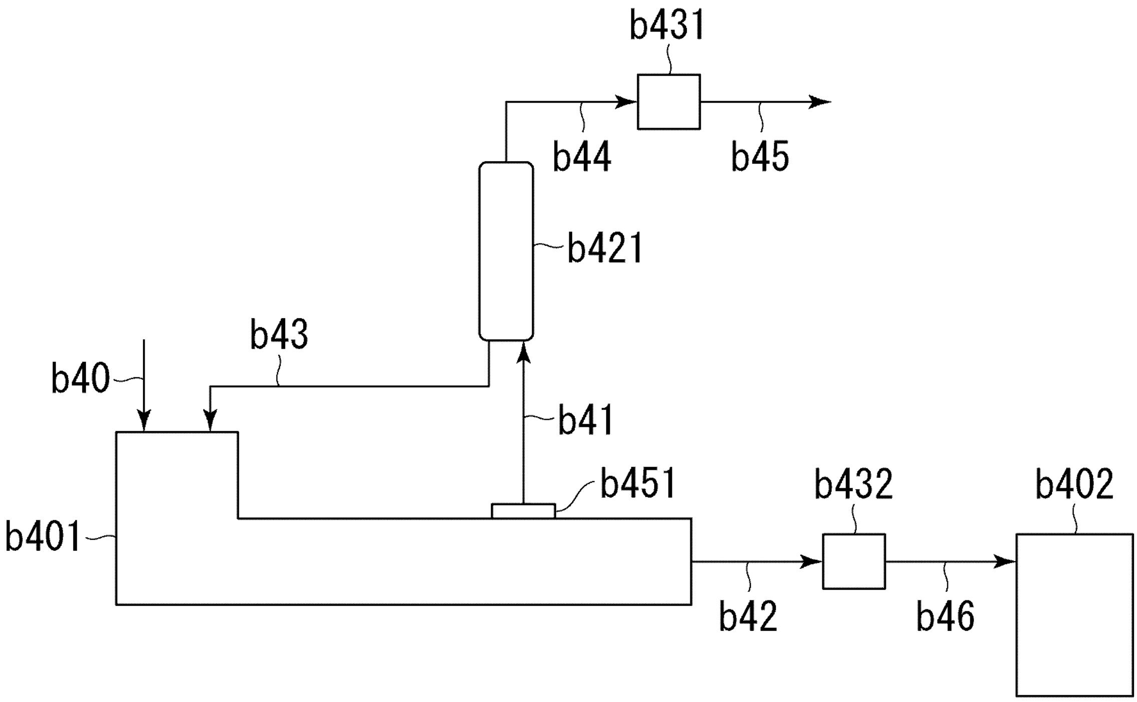
b431
b44
b45
b421
b43
b40
b41
b451
b432
b402
b401
b42
b46

FIG. 32

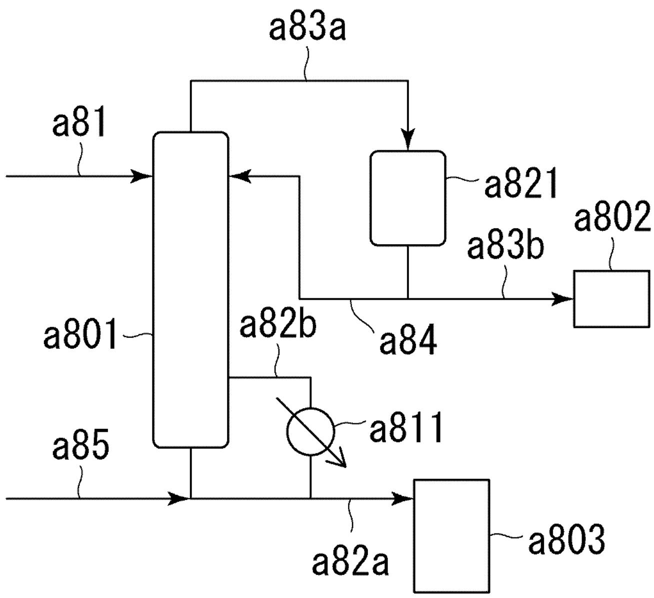
a83a
a81
a821
a802
a83b
a801
a82b
a84
a811
a85
a803
a82a

FIG. 33

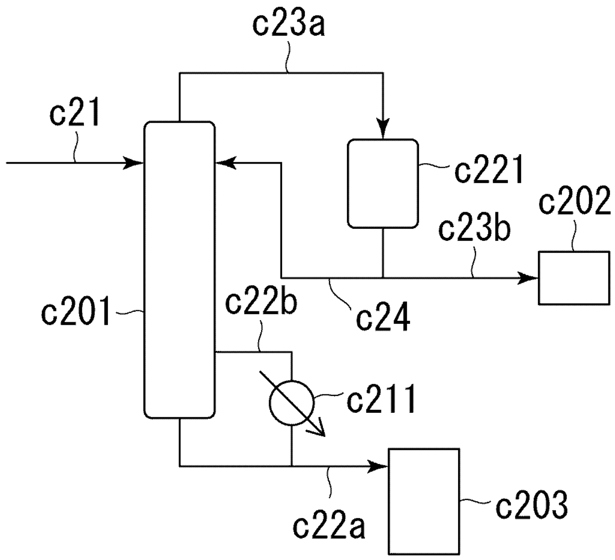
c23a
c21
c221
c202
c23b
c201
c22b
c24
c211
c22a
c203

FIG. 34

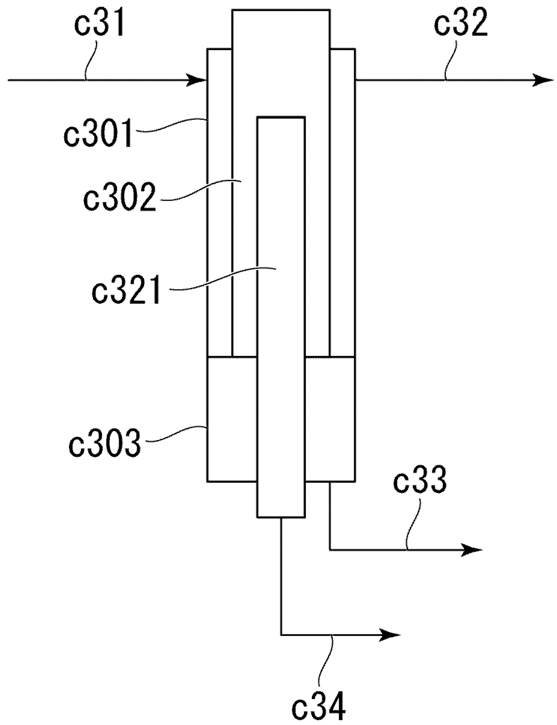
c31
c32
c301
c302
c321
c303
c33
c34

FIG. 35

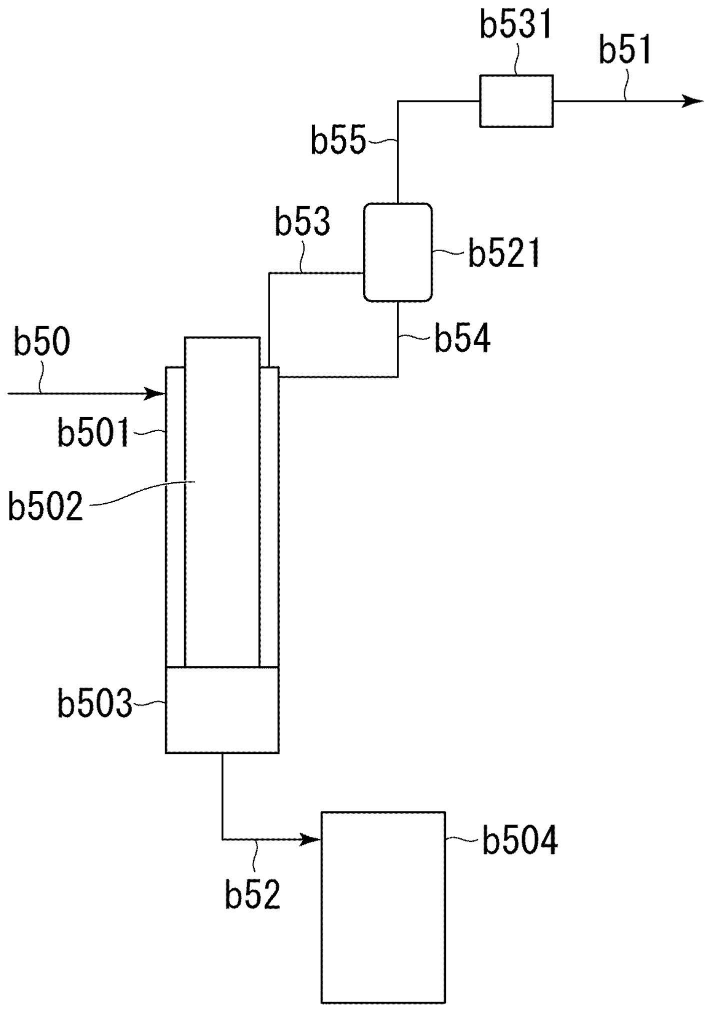
b531
b51
b55
b53
b521
b54
b50
b501
b502
b503
b504
b52

FIG. 36

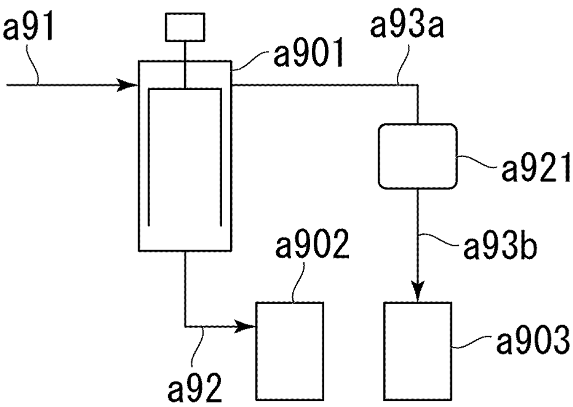
a91
a901
a93a
a921
a902
a93b
a903
a92

FIG. 37

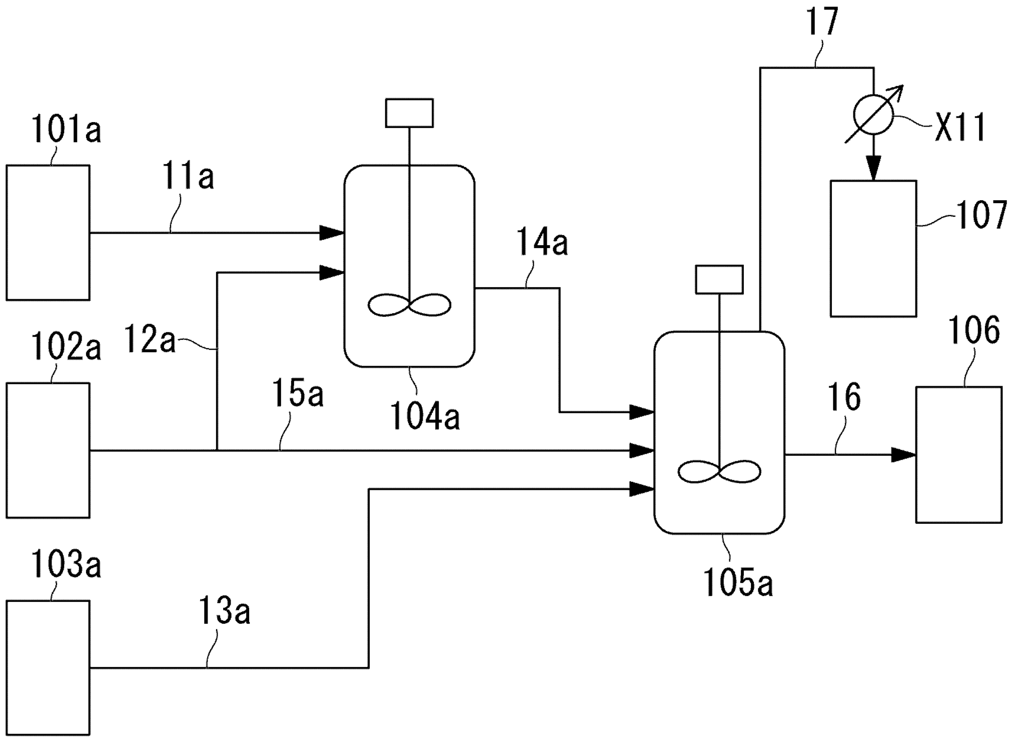
17
101a
X11
11a
107
14a
102a
12a
106
15a
16
104a
103a
105a
13a

FIG. 38

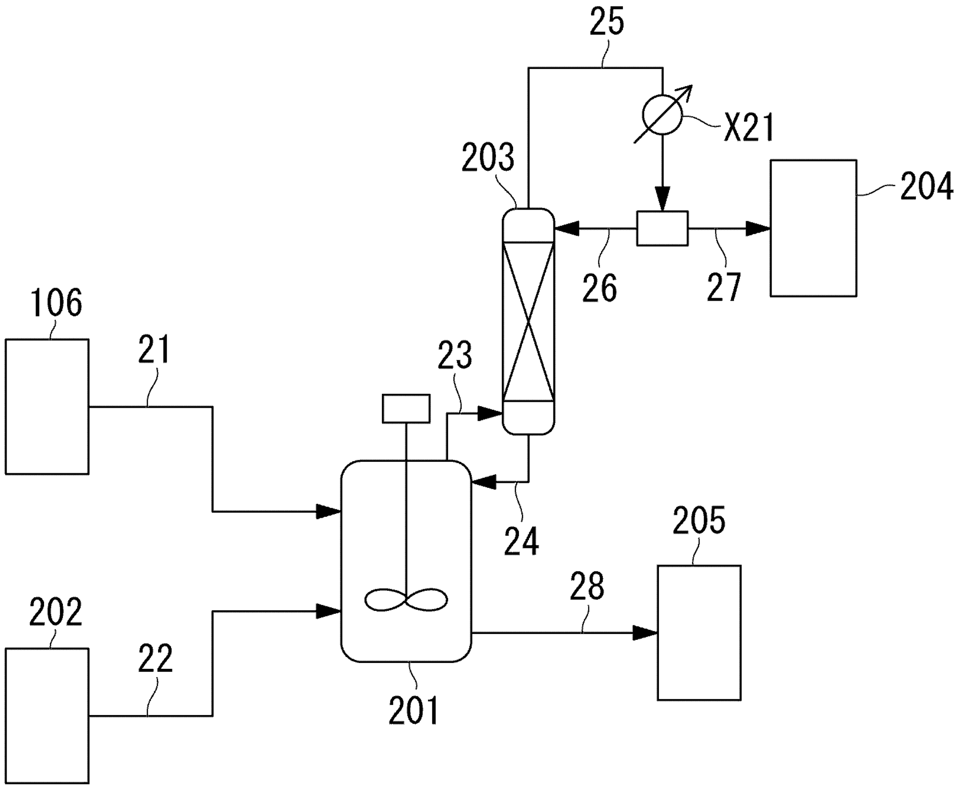
25
X21
203
204
26
27
106
21
23
24
205
202
28
22
201

FIG. 39

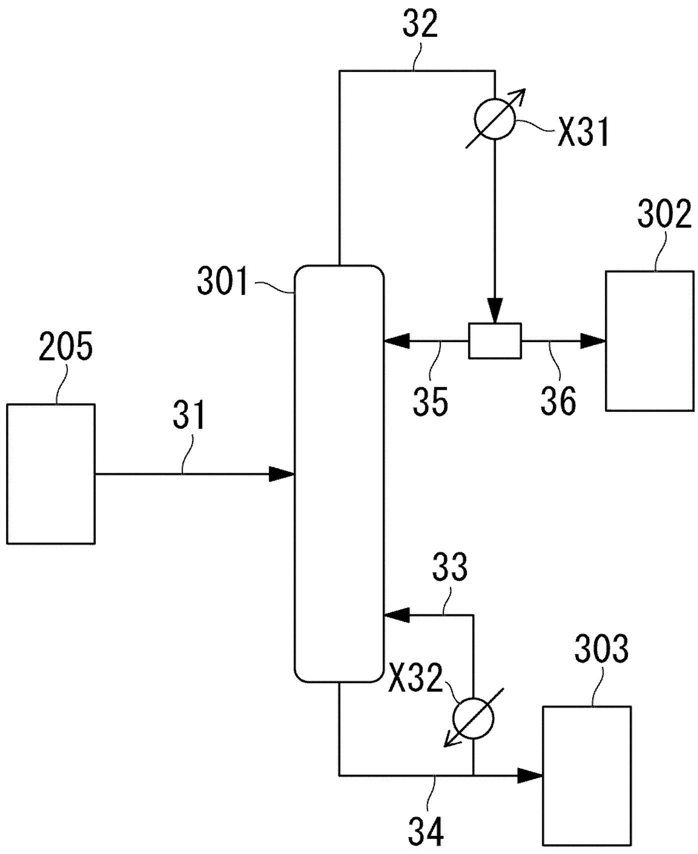
32
X31
302
301
205
35
36
31
33
303
X32
34

FIG. 40

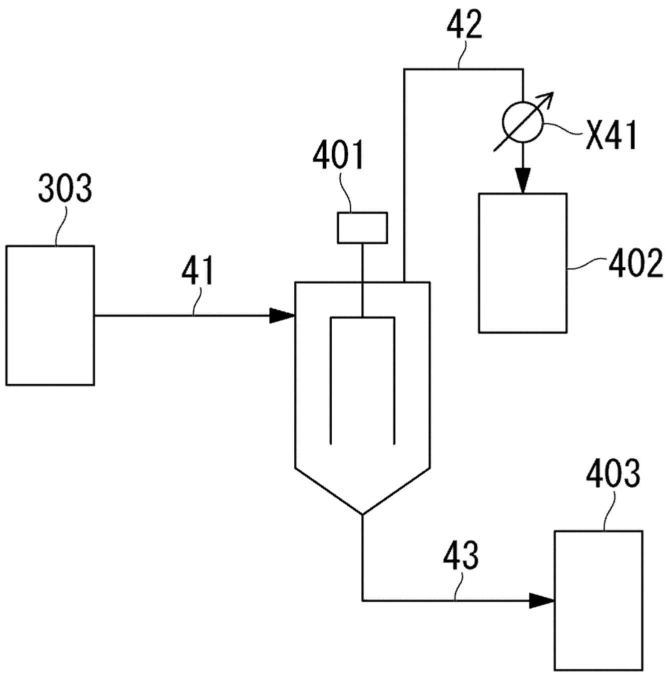
42
X41
401
303
402
41
403
43

# INTERNATIONAL SEARCH REPORT

International application No.
**PCT/JP2022/041611**

## A. CLASSIFICATION OF SUBJECT MATTER

***C07C 263/04***(2006.01)i; ***C07C 209/86***(2006.01)i; ***C07C 211/49***(2006.01)i; ***C07C 263/18***(2006.01)i; ***C07C 265/14***(2006.01)i; ***C07C 269/04***(2006.01)i; ***C07C 271/28***(2006.01)i; ***C07C 271/44***(2006.01)i; ***C07C 271/56***(2006.01)i; *C07B 61/00*(2006.01)n
FI: C07C263/04; C07C209/86; C07C211/49; C07C263/18; C07C265/14; C07C269/04; C07C271/28; C07C271/44; C07C271/56; C07B61/00 300

According to International Patent Classification (IPC) or to both national classification and IPC

## B. FIELDS SEARCHED

Minimum documentation searched (classification system followed by classification symbols)

C07C263/00; C07C209/00; C07C211/00; C07C265/00; C07C269/00; C07C271/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

## C. DOCUMENTS CONSIDERED TO BE RELEVANT

| Category* | Citation of document, with indication, where appropriate, of the relevant passages | Relevant to claim No. |
|---|---|---|
| A | WO 2011/021257 A1 (ASAHI KASEI CHEMICALS CORPORATION) 24 February 2011 (2011-02-24)<br>claims 1-47, fig. 1-20 | 1-55 |
| A | WO 2013/111353 A1 (ASAHI KASEI CHEMICALS CORPORATION) 01 August 2013 (2013-08-01)<br>claims 1-12, fig. 1-14 | 1-55 |
| A | WO 2019/131855 A1 (ASAHI KASEI CHEMICALS CORPORATION) 04 July 2019 (2019-07-04)<br>claims 1-24, fig. 1-18 | 1-55 |
| A | ENGLUND, Ethan A. et al. An Efficient Synthesis of a Probe for Protein Function: 2,3-Diaminopropionic Acid with Orthogonal Protecting Groups. Organic Letters. 2004, 6(2), pp. 213-215<br>scheme 1, compound 8 | 1-55 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2023** | **07 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2022/041611**

**C. DOCUMENTS CONSIDERED TO BE RELEVANT**

| Category* | Citation of document, with indication, where appropriate, of the relevant passages | Relevant to claim No. |
|---|---|---|
| A | WO 2015/030106 A1 (KYOTO UNIVERSITY) 05 March 2015 (2015-03-05) p. 51, compound 14a, p. 57, compound 14b | 1-55 |

Form PCT/ISA/210 (second sheet) (January 2015)

**INTERNATIONAL SEARCH REPORT**
**Information on patent family members**

International application No.
**PCT/JP2022/041611**

| Patent document cited in search report | Publication date (day/month/year) | Patent family member(s) | Publication date (day/month/year) |
|---|---|---|---|
| WO 2011/021257 A1 | 24 February 2011 | US 2011/0133121 A1<br>claims 1-55, fig. 1-20<br>EP 2439191 A1<br>CN 102471244 A<br>KR 10-2012-0036302 A | |
| WO 2013/111353 A1 | 01 August 2013 | US 2014/0332367 A1<br>claims 1-12, fig. 1-14<br>EP 2641896 A1<br>KR 10-2013-0118300 A<br>CN 104093701 A | |
| WO 2019/131855 A1 | 04 July 2019 | US 2021/0155573 A1<br>claims 1-24, fig. 1-18<br>EP 3872062 A1<br>CN 111183131 A | |
| WO 2015/030106 A1 | 05 March 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021182191 A
- JP 2021182194 A
- JP 2021182195 A
- JP 2021199645 A
- WO 2014157636 A
- JP 52071443 A
- JP H5255227 A
- US 4386033 A
- WO 2011089932 A
- WO 2010116871 A
- WO 2010110142 A
- JP S64038055 A
- JP H8217744 A
- JP H6025136 A
- JP S59205352 A
- WO 2007036479 A
- WO 2009127591 A
- WO 2019131855 A
- JP 5563886 B
- JP 5240678 B
- WO 2009130842 A
- JP S58048538 B
- JP 2002518369 W
- JP 2002173471 A
- JP S58008380 B
- WO 2013111353 A
- JP H641045 A


### Non-patent literature cited in the description


- **DRIFTER JAHRGANG.** 186. A. W. Hofmann: Ueber die aromatischen Cyanate. *Berichte der Deutschen Chemischen Gesellschaft,* vol. 3, 653-658
- **DYER E et al.** Thermal Degradation of Alkyl N-Phenylcarbomates. *Journal of American Chemical Society,* 1959, vol. 81, 2138-2143
- **ULRICH H et al.** 2+2] Cycloaddition Reactions of Unsymmetrically substituted Carbodiimides. *Journal of Heterocyclic Chemistry,* 1987, vol. 24, 1121-1123
- **SCHWETLICK K et al.** Kinetics and Catalysis of Consecutive Isocyanate Reactions. Formation of Carbamates, Allophanates and Isocyanurates. *Journal of the Chemical Society, Perkin transactions II,* 1995, vol. 2, 395-402
- Nomenclature of Organic Chemistry and Biochemistry. Nankodo Co., Ltd, 1992